(19) Europäisches Patentamt
European Patent Office
Office européen des brevets

(11) EP 3 470 089 A1

(12) EUROPEAN PATENT APPLICATION

(43) Date of publication:
**17.04.2019 Bulletin 2019/16**

(51) Int Cl.:
***A61K 48/00*** *(2006.01)* ***C12N 15/10*** *(2006.01)*
***C12N 9/22*** *(2006.01)* ***C12N 15/63*** *(2006.01)*

(21) Application number: **18199415.3**

(22) Date of filing: **12.12.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.12.2013 US 201361915215 P**
**12.12.2013 US 201361915118 P**
**12.12.2013 US 201361915148 P**
**10.06.2014 US 201462010441 P**
**24.09.2014 US 201462054490 P**

(62) Document number(s) of the earlier application(s) in accordance with Art. 76 EPC:
**14827606.6 / 3 079 726**

(71) Applicants:
- **The Broad Institute Inc.**
  **Cambridge, MA 02142 (US)**
- **Massachusetts Institute Of Technology**
  **Cambridge, MA 02139 (US)**

(72) Inventors:
- **PLATT, Randall Jeffrey**
  **4058 Basel (CH)**
- **LANGER, Robert**
  **Cambridge, MA 01238 (US)**
- **ZHANG, Feng**
  **Cambridge, MA 02139 (US)**
- **ANDERSON, Daniel**
  **Cambridge, MA 02142 (US)**
- **DAHLMAN, James**
  **Cambridge, MA 02140 (US)**

(74) Representative: **Williams, Gareth Owen**
**Marks & Clerk LLP**
**62-68 Hills Road**
**Cambridge CB2 1LA (GB)**

Remarks:
This application was filed on 09-10-2018 as a divisional application to the application mentioned under INID code 62.

# (54) DELIVERY, USE AND THERAPEUTIC APPLICATIONS OF THE CRISPR-CAS SYSTEMS AND COMPOSITIONS FOR TARGETING DISORDERS AND DISEASES USING PARTICLE DELIVERY COMPONENTS

(57) The invention provides for delivery, engineering and optimization of systems, methods, and compositions for manipulation of sequences and/or activities of target sequences. Provided are delivery particle formulations and/or systems comprising one or more components of a CRISPR-Cas system, which are means for targeting sites for delivery. The delivery particle formulations of the invention are preferably nanoparticle delivery formulations and/or systems. Also provided are vectors and vector systems some of which encode one or more components of a CRISPR complex, as well as methods for the design and use of such vectors. Also provided are methods of directing CRISPR complex formation in eukaryotic cells to ensure enhanced specificity for target recognition and avoidance of toxicity and to edit or modify a target site in a genomic locus of interest to alter or improve the status of a disease or a condition.

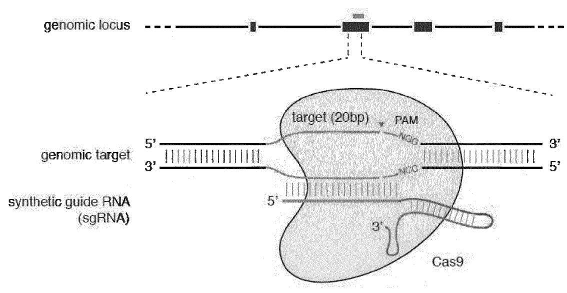


FIG. 1

EP 3 470 089 A1

**Description**

RELATED APPLICATIONS AND/OR INCORPORATION BY REFERENCE

This application claims priority from US provisional patent applications: 62/054,490, filed September 24, 2014; 62/010,441, filed June 10, 2014; and 61/915,118, 61/915,215 and 61/915,148, each filed on December 12, 2013.

The foregoing applications, and all documents cited therein or during their prosecution ("appln cited documents") and all documents cited or referenced in the appln cited documents, and all documents cited or referenced herein ("herein cited documents"), and all documents cited or referenced in herein cited documents, together with any manufacturer's instructions, descriptions, product specifications, and product sheets for any products mentioned herein or in any document incorporated by reference herein, are hereby incorporated herein by reference, and may be employed in the practice of the invention. More specifically, all referenced documents are incorporated by reference to the same extent as if each individual document was specifically and individually indicated to be incorporated by reference.

FIELD OF THE INVENTION

The present invention generally relates to the delivery, engineering, optimization and therapeutic applications of systems, methods, and compositions used for the control of gene expression involving sequence targeting, such as genome perturbation or gene-editing, that relate to Clustered Regularly Interspaced Short Palindromic Repeats (CRISPR) and components thereof.

STATEMENT AS TO FEDERALLY SPONSORED RESEARCH

This invention was made with government support under the NIH Pioneer Award (1DP1MH100706) awarded by the National Institutes of Health. The government has certain rights in the invention.

BACKGROUND OF THE INVENTION

Recent advances in genome sequencing techniques and analysis methods have significantly accelerated the ability to catalog and map genetic factors associated with a diverse range of biological functions and diseases. Precise genome targeting technologies are needed to enable systematic reverse engineering of causal genetic variations by allowing selective perturbation of individual genetic elements, as well as to advance synthetic biology, biotechnological, and medical applications. Although genome-editing techniques such as designer zinc fingers, transcription activator-like effectors (TALEs), or homing meganucleases are available for producing targeted genome perturbations, there remains a need for new genome engineering technologies that are affordable, easy to set up, scalable, and amenable to targeting multiple positions within the eukaryotic genome.

SUMMARY OF THE INVENTION

The CRISPR-Cas system does not require the generation of customized proteins to target specific sequences but rather a single Cas enzyme can be programmed by a short RNA molecule to recognize a specific DNA target. Adding the CRISPR-Cas system to the repertoire of genome sequencing techniques and analysis methods may significantly simplify the methodology and accelerate the ability to catalog and map genetic factors associated with a diverse range of biological functions and diseases. To utilize the CRISPR-Cas system effectively for genome editing without deleterious effects, it is critical to understand aspects of engineering, optimization and cell-type/tissue/organ specific delivery of these genome engineering tools, which are aspects of the claimed invention.

There exists a pressing need for alternative and robust systems and techniques for nucleic sequence targeting with a wide array of applications. Aspects of this invention address this need and provide related advantages. An exemplary CRISPR complex comprises a CRISPR enzyme complexed with a guide sequence hybridized to a target sequence within the target polynucleotide. The guide sequence is linked to a tracr mate sequence, which in turn hybridizes to a tracr sequence.

In an aspect, the invention provides methods for using one or more elements/components of a CRISPR-Cas system *via* a particle delivery formulation and/or system as a means to modify a target polynucleotide. In preferred embodiments, the particle delivery formulation and/or system are nanoparticles. The CRISPR complex of the invention provides an effective means for modifying a target polynucleotide. The CRISPR complex of the invention has a wide variety of utilities including modifying (e.g., deleting, inserting, translocating, inactivating, activating) a target polynucleotide in a multiplicity of cell types in various tissues and organs, e.g., endothelial cells, skin, heart, mucle or lung. As such the CRISPR complex of the invention has a broad spectrum of applications in, e.g., gene or genome editing, gene therapy, drug discovery, drug screening, disease diagnosis, and prognosis. The present invention contemplates uses in medicine, and gene or genome editing.

It will be appreciated that in the present methods, where the organism is an animal or a plant, the modification may occur *ex vivo* or *in vitro,* for instance in a cell culture and in some instances not *in vivo.* In other embodiments, it may occur *in vivo.*

In an aspect, the invention provides a method of modifying an organism or a non-human organism by manipulation of a target sequence in a genomic locus of interest comprising: delivering via nanoparticle complex(es) a non-naturally occurring or engineered composition comprising :

A) -

I. a CRISPR-Cas system chimeric RNA (chiRNA) polynucleotide sequence, wherein the polynucleotide sequence comprises:

(a) a guide sequence capable of hybridizing to a target sequence in a eukaryotic cell,
(b) a tracr mate sequence, and
(c) a tracr sequence, and/or

II. a polynucleotide sequence encoding a CRISPR enzyme comprising at least one or more nuclear localization sequences,
wherein (a), (b) and (c) are arranged in a 5' to 3' orientation,
wherein when transcribed, the tracr mate sequence hybridizes to the tracr sequence and the guide sequence directs sequence-specific binding of a CRISPR complex to the target sequence, and
wherein the CRISPR complex comprises the CRISPR enzyme complexed with (1) the guide sequence that is hybridizable to the target sequence, and (2) the tracr mate sequence that is hybridizable to the tracr sequence and the polynucleotide sequence encoding a CRISPR enzyme is DNA or RNA,

or
(B)

I. polynucleotides comprising:

(a) a guide sequence capable of hybridizing to a target sequence in a eukaryotic cell, and
(b) at least one or more tracr mate sequences,

II. a polynucleotide sequence encoding a CRISPR enzyme, and
III. a polynucleotide sequence comprising a tracr sequence, wherein when transcribed, the tracr mate sequence hybridizes to the tracr sequence and the guide sequence directs sequence-specific binding of a CRISPR complex to the target sequence, and wherein the CRISPR complex comprises the CRISPR enzyme complexed with (1) the guide sequence that is hybridizable to the target sequence, and (2) the tracr mate sequence that is hybridizable to the tracr sequence, and the polynucleotide sequence encoding a CRISPR enzyme is DNA or RNA.

The CRISPR enzyme is a type I or III CRISPR enzyme, preferably a type II CRISPR enzyme. This type II CRISPR enzyme may be any Cas enzyme. A preferred Cas enzyme may be identified as Cas9 as this can refer to the general class of enzymes that share homology to the biggest nuclease with multiple nuclease domains from the type II CRISPR system. Most preferably, the Cas9 enzyme is from, or is derived from, spCas9 or saCas9. It will be appreciated that SpCas9 or SaCas9 are those from or derived from *S. pyogenes* or *S. aureus* Cas9. By derived, Applicants mean that the derived enzyme is largely based, in the sense of having a high degree of sequence homology with, a wildtype enzyme, but that it has been mutated (modified) in some way as described herein It will be appreciated that the terms Cas and CRISPR enzyme are generally used herein interchangeably, unless otherwise apparent. The Cas enzyme can be for instance any naturally-occurring bacterial Cas9 as well as any chimaeras, mutants, homologs or orthologs. Many of the residue numberings used herein refer to the Cas9 enzyme from the type II CRISPR locus in *Streptococcus pyogenes* (annotated alternatively as SpCas9 or spCas9). However, it will be appreciated that this invention includes many more Cas9s from other species of microbes, such as SpCas9 derived from *S. pyogenes,* SaCas9 derived from *S. aureus,* St1Cas9 derived from *S. thermophilus* and so forth. The skilled person will be able to determine appropriate corresponding residues in Cas9 enzymes other than SpCas9 by comparison of the relevant amino acid sequences. Thus, where a specific amino acid replacement is referred to using the SpCas9 numbering, then, unless the context makes it apparent this is not intended to refer to other Cas9 enzymes, the disclosure is intended to encompass corresponding modifications in other Cas9 enzymes.

In the invention, the Cas9 enzyme can be constitutively present, or delivered via a nanoparticle, or via a vector expressing the Cas9 enzyme, e.g., by sequential or co-administration of a nanoparticle containing or vector containing nucleic acid molecule(s) for in vivio expression of the Cas9 with the nanoparticle containing RNA components of the CRISPR system. The nanoparticle can also deliver the vector,

Cas9 orthologs typically share the general organization of 3-4 RuvC domains and a HNH domain. The 5' most RuvC domain cleaves the non-complementary strand, and the HNH domain cleaves the complementary strand. All notations are in reference to the guide sequence.The catalytic residue in the 5' RuvC domain is identified through homology comparison of the Cas9 of interest with other Cas9 orthologs (from S. pyogenes type II CRISPR locus, S. thermophilus CRISPR locus 1, S. thermophilus CRISPR locus 3, and Franciscilla novicida type II CRISPR locus), and the conserved Asp residue (D10) is mutated to alanine to convert Cas9 into a complementary-strand nicking enzyme. Similarly, the conserved His and Asn residues in the HNH domains are mutated to Alanine to convert Cas9 into a non-complementary-strand nicking enzyme. In some embodiments, both sets of mutations may be made, to convert Cas9 into a non-cutting enzyme. Thus, the Cas9 may comprise one or more mutations and may be used as a generic DNA binding protein with or without fusion to a functional domain. The mutations may be artificially introduced mutations or gain- or loss-of-function mutations. The mutations may include but are not limited to mutations in one of the catalytic domains (D10 and H840) in the RuvC and HNH catalytic domains respectively. Further mutations have been characterized and may be used in one or more compositions of the invention. In one aspect of the invention, the mutated Cas9 enzyme may be fused to a protein domain, e.g., such as a transcriptional activation domain. In one aspect, the transcriptional activation domain may be VP64. In other aspects of the invention, the transcriptional repressor domain may be KRAB or SID4X. Accordingly, mutated Cas 9 enzyme can be fused to domains which include but are not limited to a transcriptional activator, repressor, a recombinase, a transposase, a histone remodeler, a demethylase, a DNA methyltransferase, a cryptochrome, a light inducible/controllable domain or a chemically inducible/controllable domain. The Cas9 in the invention may be a chimeric Cas9 proteins; e.g., a Cas9 having enhanced function by being a chimera. Chimeric Cas9 proteins may be new Cas9 containing fragments from more than one naturally occurring Cas9. These may comprise fusions of N-terminal fragment(s) of one Cas9 homolog with C-terminal fragment(s) of another Cas9 homolog. The Cas of the CRISPR-Cas system can be in a form to reducing the toxicity of Cas enzymes. For example, the Cas9 can be delivered into the cell in the form of mRNA, for transient expression of the enzyme thereby reducing toxicity. The expression of Cas9 can be under the control of an inducible promoter.

The tracrRNA and direct repeat sequences can be mutant sequences or the invention can encompass RNA of the CRISPR-Cas system that includes mutant chimeric guide sequences that allow for enhancing performance of these RNAs in cells. A suitable promoter, such as the Pol III promoter, such as a U6 promoter, can be added onto the guide RNA that is advantageously delivered via nanoparticle. Aspects of the invention also relate to the guide RNA being transcribed *in vitro* or ordered from a synthesis company and directly transfected. Expression of guide RNAs under the control of the T7 promoter driven by the expression of T7 polymerase in the cell is also envisioned. In an advantageous embodiment, the cell is a eukaryotic cell. In a preferred embodiment the eukaryotic cell is a human cell. In a more preferred embodiment the human cell is a patient specific cell.

An example of a codon optimized sequence, in this instance optimized for humans (i.e. being optimized for expression in humans) is provided herein, see the SaCas9 human codon optimized sequence. Whilst this is preferred, it will be appreciated that other examples are possible and codon optimization for a host species other than human, or for codon optimization for specific organs such as the brain, is known.

It will be appreciated that where reference is made to a polynucleotide, where that polynucleotide is RNA and is said to 'comprise' a feature such as a tracr mate sequence, the RNA sequence includes the feature. Where the polynucleotide is DNA and is said to comprise a feature such as a tracr mate sequence, the DNA sequence is or can be transcribed into the RNA that comprises the feature at issue. Where the feature is a protein, such as the CRISPR enzyme, the DNA or RNA sequence referred to is, or can be, translated (and in the case of DNA transcribed first). Furthermore, in cases where an RNA encoding the CRISPR enzyme is provided to a cell, it is understood that the RNA is capable of being translated by the cell into which it is delivered.

Accordingly, in certain embodiments the invention provides a method of modifying an organism, e.g., mammal including human or a non-human mammal or organism by manipulation of a target sequence in a genomic locus of interest comprising delivering a non-naturally occurring or engineered composition comprising a nanoparticle, wherein the composition comprises: (A) a non-naturally occurring or engineered composition comprising a vector system comprising one or more vectors comprising I. a first regulatory element operably linked to a CRISPR-Cas system chimeric RNA (chiRNA) polynucleotide sequence, wherein the polynucleotide sequence comprises (a) a guide sequence capable of hybridizing to a target sequence in a eukaryotic cell, (b) a tracr mate sequence, and (c) a tracr sequence, and II. a second regulatory element operably linked to an enzyme-coding sequence encoding a CRISPR enzyme comprising at least one or more nuclear localization sequences (or optionally at least one or more nuclear localization sequences as some embodiments can involve no NLS), wherein (a), (b) and (c) are arranged in a 5' to 3' orientation, wherein components I and II are located on the same or different vectors of the system, wherein when transcribed, the tracr mate sequence hybridizes to the tracr sequence and the guide sequence directs sequence-specific binding of a CRISPR complex to the target sequence, and wherein the CRISPR complex comprises the CRISPR enzyme complexed with (1) the guide sequence that is hybridizable to the target sequence, and (2) the tracr mate sequence that is hybridizable to the tracr sequence, or (B) a non-naturally occurring or engineered composition comprising a vector system comprising one or more vectors comprising I. a first regulatory element operably linked to (a) a guide sequence capable of hybridizing to a target sequence in a eukaryotic cell, and (b) at least one or more tracr mate sequences, II. a second regulatory element operably linked to an enzyme-coding sequence encoding a CRISPR enzyme, and III. a third regulatory element operably linked to a tracr sequence, wherein components I, II and III are located on the same or different vectors of the system, wherein when transcribed, the tracr mate sequence hybridizes to the tracr sequence and the guide sequence directs sequence-specific binding of a CRISPR complex to the target sequence, and wherein the CRISPR complex comprises the CRISPR enzyme complexed with (1) the guide sequence that is hybridizable to the target sequence, and (2) the tracr mate sequence that is hybridizable to the tracr sequence. In some embodiments, components I, II and III are delivered together. In other embodiments, components I and II are delivered separately. In other embodiments, components I and III are delivered together, while component II is delivered separately.

Accordingly, in certain embodiments the invention provides a method of modifying an organism, e.g., mammal including human or a non-human mammal or organism by manipulation of a target sequence in a genomic locus of interest, e.g., in heart, muscle or lung tissue or cells, comprising delivering a non-naturally occurring or engineered composition comprising by nanoparticle complex(es), wherein the composition comprises: (A) a non-naturally occurring or engineered composition comprising a vector system comprising one or more vectors comprising I. a first regulatory element operably linked to a CRISPR-Cas system RNA or chimeric RNA (chiRNA) polynucleotide sequence, wherein the polynucleotide sequence comprises (a) a guide sequence capable of hybridizing to a target sequence in a eukaryotic cell, (b) a tracr mate sequence, and (c) a tracr sequence, and II. a second regulatory element operably linked to nucleotide sequence encoding a CRISPR enzyme, advantageously comprising at least one or more nuclear localization sequences (or advantageiously two nuclear localization sequences), wherein (a), (b) and (c) are arranged in a 5' to 3' orientation, wherein components I and II are located on the same or different vectors of the system, wherein vector or vectors involving at least component I or II or both I and II are delivered via nanoparticles or nanoparticle complex(es); and wherein when transcribed, the tracr mate sequence is hybridzable to the tracr sequence and the guide sequence directs sequence-specific binding of a CRISPR complex to the target sequence, and wherein the CRISPR complex comprises the CRISPR enzyme complexed with (1) the guide sequence that is hybridizable to the target sequence, and (2) the tracr mate sequence that is hybridizable to the tracr sequence, or (B) a non-naturally occurring or engineered composition comprising a vector system comprising one or more vectors comprising I. a first regulatory element operably linked to (a) a guide sequence capable of hybridizing to a target sequence in a eukaryotic cell, and (b) at least one or more tracr mate sequences, II. a second regulatory element operably linked to an enzyme-coding sequence encoding a CRISPR enzyme, and III. a third regulatory element operably linked to a tracr sequence, wherein components I, II and III are located on the same or different vectors of the system, wherein vector(s) as to at least one of I, II and III, and advantageously as to all of I, II and III are delivered via nanoparticles or nanoparticle complex(es), and wherein when transcribed, the tracr mate sequence hybridizes to the tracr sequence and the guide sequence directs sequence-specific binding of a CRISPR complex to the target sequence, and wherein the CRISPR complex comprises the CRISPR enzyme complexed with (1) the guide sequence that is hybridizable to the target sequence, and (2) the tracr mate sequence that is hybridizable to the tracr sequence. In some embodiments, components I, II and III are delivered together. In other embodiments, components I and II are delivered separately. In other embodiments, components I and III are delivered together, while component II is delivered separately. In any event use of nanoparticles, especially to target heart, muscle or lung tissue or cells , are aspects of the invention.

By manipulation of a target sequence, Applicants mean the alteration of the target sequence, which may include the epigenetic manipulation of a target sequence. This epigenetic manipulation may be of the chromatin state of a target sequence, such as by modification of the methylation state of the target sequence (i.e. addition or removal of methylation or methylation patterns or CpG islands), histone modification, increasing or reducing accessibility to the target sequence, or by promoting 3D folding.

It will be appreciated that where reference is made to a method of modifying an organism or mammal including human or a non-human mammal or organism by manipulation of a target sequence in a genomic locus of interest, this may apply to the organism (or mammal) as a whole or just a single cell or population of cells from that organism (if the organism is multicellular). In the case of humans, for instance, Applicants envisage, *inter alia,* a single cell or a population of cells and these may preferably be modified *ex vivo* and then re-introduced. In this case, a biopsy or other tissue or biological fluid sample may be necessary. Stem cells are also particularly preferred in this regard. But, of course, *in vivo* embodiments are also envisaged.

In certain embodiments the invention provides a method of treating or inhibiting a condition caused by a defect in a target sequence in a genomic locus of interest in a subject (e.g., mammal or human) or a non-human subject (e.g.,

mammal) in need thereof comprising modifying the subject or a non-human subject by manipulation of the target sequence and wherein the condition is susceptible to treatment or inhibition by manipulation of the target sequence comprising providing treatment comprising: delivering a non-naturally occurring or engineered composition comprising by nanoparticle complex(es), wherein the composition comprises: (A) a non-naturally occurring or engineered composition comprising a vector system comprising one or more vectors comprising I. a first regulatory element operably linked to a CRISPR-Cas system RNA or chimeric RNA (chiRNA) polynucleotide sequence, wherein the polynucleotide sequence comprises (a) a guide sequence capable of hybridizing to a target sequence in a eukaryotic cell, (b) a tracr mate sequence, and (c) a tracr sequence, and II. a second regulatory element operably linked to nucleotide sequence encoding a CRISPR enzyme, advantageously comprising at least one or more nuclear localization sequences (or advantageiously two nuclear localization sequences), wherein (a), (b) and (c) are arranged in a 5' to 3' orientation, wherein components I and II are located on the same or different vectors of the system, wherein vector or vectors involving at least component I or II or both I and II are delivered via nanoparticles or nanoparticle complex(es); and wherein when transcribed, the tracr mate sequence is hybridzable to the tracr sequence and the guide sequence directs sequence-specific binding of a CRISPR complex to the target sequence, and wherein the CRISPR complex comprises the CRISPR enzyme complexed with (1) the guide sequence that is hybridizable to the target sequence, and (2) the tracr mate sequence that is hybridizable to the tracr sequence, or (B) a non-naturally occurring or engineered composition comprising a vector system comprising one or more vectors comprising I. a first regulatory element operably linked to (a) a guide sequence capable of hybridizing to a target sequence in a eukaryotic cell, and (b) at least one or more tracr mate sequences, II. a second regulatory element operably linked to an enzyme-coding sequence encoding a CRISPR enzyme, and III. a third regulatory element operably linked to a tracr sequence, wherein components I, II and III are located on the same or different vectors of the system, wherein vector(s) as to at least one of I, II and III, and advantageously as to all of I, II and III are delivered via nanoparticles or nanoparticle complex(es), and wherein when transcribed, the tracr mate sequence hybridizes to the tracr sequence and the guide sequence directs sequence-specific binding of a CRISPR complex to the target sequence, and wherein the CRISPR complex comprises the CRISPR enzyme complexed with (1) the guide sequence that is hybridizable to the target sequence, and (2) the tracr mate sequence that is hybridizable to the tracr sequence. In some embodiments, components I, II and III are delivered together. In other embodiments, components I and II are delivered separately. In other embodiments, components I and III are delivered together, while component II is delivered separately. In any event use of nanoparticles, especially to target heart, muscle or lung tissue or cells , are aspects of the invention.

Some methods of the invention can include inducing expression. In some methods of the invention the organism or subject is a eukaryote (including mammal including human) or a non-human eukaryote or a non-human animal or a non-human mammal. In some embodiments, the organism or subject is a non-human animal, and may be an arthropod, for example, an insect, or may be a nematode. In some methods of the invention the organism or subject is a plant. In some methods of the invention the organism or subject is a mammal or a non-human mammal. A non-human mammal may be for example a rodent (preferably a mouse or a rat), an ungulate, or a primate.

In some embodiments the invention comprehends delivering a CRISPR enzyme comprising delivering to a cell mRNA encoding the CRISPR enzyme, e.g., via nanoparticle complex(es). In some of these methods the CRISPR enzyme is a Cas9.

The invention further comprehends nanoparticle complex(es) containing CRISPR complex components or vector(s) for expression,, e.g., RNA of the CRISPR complex, or a CRISPR enzyme thereof (including or alternatively mRNA encoding the CRISPR enzyme), for use in medicine or in therapy, or more generally a method according to the invention, including *in vivo, in vitro* or *ex vivo* gene or genome editing. In some methods of the invention the CRISPR enzyme comprises one or more mutations in one of the catalytic domains.

With respect to mutations of the CRISPR enzyme, when the enzyme is not SpCas9, mutations may be made at any or all residues corresponding to positions 10, 762, 840, 854, 863 and/or 986 of SpCas9 (which may be ascertained for instance by standard sequence comparison tools). In particular, any or all of the following mutations are preferred in SpCas9: D10A, E762A, H840A, N854A, N863A and/or D986A; as well as conservative substitution for any of the replacement amino acids is also envisaged. In an aspect the invention provides as to any or each or all embodiments herein-discussed wherein the CRISPR enzyme comprises at least one or more, or at least two or more mutations, wherein the at least one or more mutation or the at least two or more mutations is as to D10, E762, H840, N854, N863, or D986 according to SpCas9 protein, e.g., D10A, E762A, H840A, N854A, N863A and/or D986A as to SpCas9, or N580 according to SaCas9, e.g., N580A as to SaCas9, or any corresponding mutation(s) in a Cas9 of an ortholog to Sp or Sa, or the CRISPR enzyme comprises at least one mutation wherein at least H840 or N863A as to Sp Cas9 or N580A as to Sa Cas9 is mutated; e.g., wherein the CRISPR enzyme comprises H840A, or D10A and H840A, or D10A and N863A, according to SpCas9 protein, or any corresponding mutation(s) in a Cas9 of an ortholog to Sp protein or Sa protein.

In some methods of the invention the CRISPR enzyme is a Cas9 nickase. The invention envisions the use of nanoparticle complex(es) containing CRISPR complex components or vector(s) for expression,, e.g., RNA of the CRISPR complex, or a CRISPR enzyme thereof (including or alternatively mRNA encoding the CRISPR enzyme), for the manufacture of a medicament for *in vivo, in vitro* or *ex vivo* gene or genome editing or for use in a method according of the invention. The invention comprehends nanoparticle complex(es) containing CRISPR complex components or vector(s) for expression,, e.g., RNA of the CRISPR complex, or a CRISPR enzyme thereof (including or alternatively mRNA encoding the CRISPR enzyme), , wherein the target sequence is flanked at its 3' end by a PAM (protospacer adjacent motif) sequence comprising 5'-motif, especially where the Cas9 is (or is derived from) S. *pyogenes* or *S. aureus* Cas9. For example, a suitable PAM is 5'-NRG or 5'-NNGRR (where N is any Nucleotide) for SpCas9 or SaCas9 enzymes (or derived enzymes).

It will be appreciated that SpCas9 or SaCas9 are those from or derived from S. *pyogenes* or *S. aureus* Cas9, and SaCas9 is presently considered advantageous.

The invention in some embodiments comprehends a method of modifying an organism or a non-human organism by minimizing off-target modifications by manipulation of a first and a second target sequence on opposite strands of a DNA duplex in a genomic locus of interest in a cell comprising delivering by at least one nanoparticle complex a non-naturally occurring or engineered composition or component thereof, said composition comprising :

I. a first CRISPR-Cas system chimeric RNA (chiRNA) polynucleotide sequence, wherein the first polynucleotide sequence comprises:

(a) a first guide sequence capable of hybridizing to the first target sequence,
(b) a first tracr mate sequence, and
(c) a first tracr sequence,

II. a second CRISPR-Cas system chiRNA polynucleotide sequence, wherein the second polynucleotide sequence comprises:

(a) a second guide sequence capable of hybridizing to the second target sequence,
(b) a second tracr mate sequence, and
(c) a second tracr sequence, and

III. a polynucleotide sequence encoding a CRISPR enzyme comprising at least one or more nuclear localization sequences and comprising one or more mutations, wherein (a), (b) and (c) are arranged in a 5' to 3' orientation, wherein when transcribed, the first and the second tracr mate sequence hybridize to the first and second tracr sequence respectively and the first and the second guide sequence directs sequence-specific binding of a first and a second CRISPR complex to the first and second target sequences respectively, wherein the first CRISPR complex comprises the CRISPR enzyme complexed with (1) the first guide sequence that is hybridizable to the first target sequence, and (2) the first tracr mate sequence that is hybridizable to the first tracr sequence, wherein the second CRISPR complex comprises the CRISPR enzyme complexed with (1) the second guide sequence that is hybridizable to the second target sequence, and (2) the second tracr mate sequence that is hybridizable to the second tracr sequence, wherein the polynucleotide sequence encoding a CRISPR enzyme is DNA or RNA, and wherein the first guide sequence directs cleavage of one strand of the DNA duplex near the first target sequence and the second guide sequence directs cleavage of the other strand near the second target sequence inducing a double strand break, thereby modifying the organism or the non-human organism by minimizing off-target modifications.

In some methods of the invention any or all of the polynucleotide sequence encoding the CRISPR enzyme, the first and the second guide sequence, the first and the second tracr mate sequence or the first and the second tracr sequence, is/are RNA; and RNA is advantageously delived via nanoparticle complex(es). In further embodiments of the invention the polynucleotides comprising the sequence encoding the CRISPR enzyme, the first and the second guide sequence, the first and the second tracr mate sequence or the first and the second tracr sequence, is/are RNA and are delivered via nanoparticles. In certain embodiments of the invention, the first and second tracr mate sequence share 100% identity and/or the first and second tracr sequence share 100% identity. In some embodiments, the polynucleotides may be comprised within a vector system comprising one or more vectors. In preferred embodiments of the invention the CRISPR enzyme is a Cas9 enzyme, e.g. SpCas9 or SaCas9. In an aspect of the invention the CRISPR enzyme comprises one or more mutations in a catalytic domain, wherein the one or more mutations are selected from the group consisting of D10A, E762A, H840A, N854A, N863A and D986A. In a highly preferred embodiment the CRISPR enzyme has the D10A mutation. In preferred embodiments, the first CRISPR enzyme has one or more mutations such that the enzyme is a complementary strand nicking enzyme, and the second CRISPR enzyme has one or more mutations such that the enzyme is a non-complementary strand nicking enzyme. Alternatively the first enzyme may be a non-complementary strand nicking enzyme, and the second enzyme may be a complementary strand nicking enzyme.

In preferred methods of the invention the first guide sequence directing cleavage of one strand of the DNA duplex near the first target sequence and the second guide sequence directing cleavage of the other strand near the second target sequence results in a 5' overhang. In embodiments of the invention the 5' overhang is at most 200 base pairs, preferably at most 100 base pairs, or more preferably at most 50 base pairs. In embodiments of the invention the 5' overhang is at least 26 base pairs, preferably at least 30 base pairs or more preferably 34-50 base pairs.

The invention in some embodiments comprehends a method of modifying an organism or a non-human organism by minimizing off-target modifications by manipulation of a first and a second target sequence on opposite strands of a DNA duplex in a genomic locus of interest in a cell comprising delivering via at least one nanoparticle complex a non-naturally occurring or engineered composition or component thereof, said composition comprising a vector system comprising one or more vectors comprising

I. a first regulatory element operably linked to

(a) a first guide sequence capable of hybridizing to the first target sequence, and
(b) at least one or more tracr mate sequences,

II. a second regulatory element operably linked to

(a) a second guide sequence capable of hybridizing to the second target sequence, and
(b) at least one or more tracr mate sequences,

III. a third regulatory element operably linked to an enzyme-coding sequence encoding a CRISPR enzyme, and
IV. a fourth regulatory element operably linked to a tracr sequence,
wherein components I, II, III and IV are located on the same or different vectors of the system, when transcribed, the tracr mate sequence hybridizes to the tracr sequence and the first and the second guide sequence direct sequence-specific binding of a first and a second CRISPR complex to the first and second target sequences respectively, wherein the first CRISPR complex comprises the CRISPR enzyme complexed with (1) the first guide sequence that is hybridizable to the first target sequence, and (2) the tracr mate sequence that is hybridizable to the tracr sequence, wherein the second CRISPR complex comprises the CRISPR enzyme complexed with (1) the second guide sequence that is hybridizable to the second target sequence, and (2) the tracr mate sequence that is hybridizable to the tracr sequence, wherein the polynucleotide sequence encoding a CRISPR enzyme is DNA or RNA, and wherein the first guide sequence directs cleavage of one strand of the DNA duplex near the first target sequence and the second guide sequence directs cleavage of the other strand near the second target sequence inducing a double strand break, thereby modifying the organism or the non-human organism by minimizing off-target modifications.

The invention also provides a vector system as described herein. The system may comprise one, two, three or four different vectors; and the system may comprise one, two, three or four different nanoparticle complex(es) delivering the component(s) of the system. Components I, II, III and IV may thus be located on one, two, three or four different vectors, and may be delivered by one, two, three or four different nanoparticle complex(es) (with other delivery means envisioned for those portions of the system not delived via nanoparticle complex(es); and all combinations for possible locations and complex(es) of the components are herein envisaged, for example: components I, II, III and IV can be located on the same vector; components I, II, III and IV can each be located on different vectors; components I, II, II I and IV may be located on a total of two or three different vectors, with all combinations of locations envisaged, etc.; and components I, II, III and IV can be delivered via the nanoparticle complex; components I, II, III and IV can be delivered via the nanoparticle complex; components I, II, II I and IV may be delivered via a total of two or three different complex(es), with all combinations of locations envisaged, etc. And complexes that target lung, heart or muscle tissue or cells are advantageous.

In some methods of the invention any or all of the polynucleotide sequence encoding the CRISPR enzyme, the first and the second guide sequence, the first and the second tracr mate sequence or the first and the second tracr sequence, is/are RNA; and advantageously delivered via nanoparticle complex(es). In further embodiments of the invention the first and second tracr mate sequence share 100% identity and/or the first and second tracr sequence share 100% identity. In preferred embodiments of the invention the CRISPR enzyme is a Cas9 enzyme, e.g. SpCas9. In an aspect of the invention the CRISPR enzyme comprises one or more mutations in a catalytic domain, wherein the one or more mutations are selected from the group consisting of D10A, E762A, H840A, N854A, N863A and D986A. In a highly preferred embodiment the CRISPR enzyme has the D10A mutation. In preferred embodiments, the first CRISPR enzyme has one or more mutations such that the enzyme is a complementary strand nicking enzyme, and the second CRISPR enzyme has one or more mutations such that the enzyme is a non-complementary strand nicking enzyme. Alternatively the first enzyme may be a non-complementary strand nicking enzyme, and the second enzyme may be a complementary strand nicking enzyme. In a further embodiment of the invention, one or more of the vectors or viral vectors are delivered via nanoparticles.

In preferred methods of the invention the first guide sequence directing cleavage of one strand of the DNA duplex near the first target sequence and the second guide sequence directing cleavage of the opposite strand near the second target sequence results in a 5' overhang. In embodiments of the invention the 5' overhang is at most 200 base pairs, preferably at most 100 base pairs, or more preferably at most 50 base pairs. In embodiments of the invention the 5' overhang is at least 26 base pairs, preferably at least 30 base pairs or more preferably 34-50 base pairs.

The invention in some embodiments comprehends a method of modifying a genomic locus of interest by minimizing off-target modifications by introducing into a cell containing and expressing a double stranded DNA molecule encoding a gene product of interest an engineered, non-naturally occurring CRISPR-Cas system, wherein the introducing is via at least one nanoparticle complex delivering at least a portion of the CRISPR-Cas system (e.g., RNA thereof, and/or the Cas9 and/or a vector encoding the Cas9 and/or a vector expressing the RNA of the system) wherein the system comprises a Cas protein having one or more mutations and two guide RNAs that target a first strand and a second strand of the DNA molecule respectively, whereby the guide RNAs target the DNA molecule encoding the gene product and the Cas protein nicks each of the first strand and the second strand of the DNA molecule encoding the gene product, whereby expression of the gene product is altered; and, wherein the Cas protein and the two guide RNAs do not naturally occur together.

The Cas protein can nick each of the first strand and the second strand of the DNA molecule encoding the gene product results in a 5' overhang. In embodiments of the invention the 5' overhang is at most 200 base pairs, preferably at most 100 base pairs, or more preferably at most 50 base pairs. In embodiments of the invention the 5' overhang is at least 26 base pairs, preferably at least 30 base pairs or more preferably 34-50 base pairs.

Embodiments of the invention also comprehend the guide RNAs comprising a guide sequence fused to a tracr mate sequence and a tracr sequence. In an aspect of the invention the Cas protein is codon optimized for expression in a eukaryotic cell, preferably a mammalian cell or a human cell. In further embodiments of the invention the Cas protein is a type II CRISPR-Cas protein, e.g. a Cas 9 protein. In a highly preferred embodiment the Cas protein is a Cas9 protein, e.g. SpCas9. In aspects of the invention the Cas protein has one or more mutations selected from the group consisting of D10A, E762A, H840A, N854A, N863A and D986A. In a highly preferred embodiment the Cas protein has the D10A mutation.

Aspects of the invention relate to the expression of the gene product being decreased or a template polynucleotide being further introduced into the DNA molecule encoding the gene product or an intervening sequence being excised precisely by allowing the two 5' overhangs to reanneal and ligate or the activity or function of the gene product being altered or the expression of the gene product being increased. In an embodiment of the invention, the gene product is a protein. The template polynucleotide can also be introduced via nanoparticle complex(es).

The invention also comprehends an engineered, non-naturally occurring CRISPR-Cas system comprising a Cas protein having one or more mutations and two guide RNAs that target a first strand and a second strand respectively of a double stranded DNA molecule encoding a gene product in a cell, whereby the guide RNAs target the DNA molecule encoding the gene product and the Cas protein nicks each of the first strand and the second strand of the DNA molecule encoding the gene product, whereby expression of the gene product is altered; and, wherein the Cas protein and the two guide RNAs do not naturally occur together; and, wherein a the system or a component thereof or that which gives rise to expression of the system or a component thereof is delivered via nanoparticle complex(es); and advantageously the complex(es) target heart, muscle or lung tissue or cells.

In aspects of the invention the guide RNAs may comprise a guide sequence fused to a tracr mate sequence and a tracr sequence. In an embodiment of the invention the Cas protein is a type II CRISPR-Cas protein. In an aspect of the invention the Cas protein is codon optimized for expression in a eukaryotic cell, preferably a mammalian cell or a human cell. In further embodiments of the invention the Cas protein is a type II CRISPR-Cas protein, e.g. a Cas 9 protein. In a highly preferred embodiment the Cas protein is a Cas9 protein, e.g. SpCas9. In aspects of the invention the Cas protein has one or more mutations selected from the group consisting of D10A, E762A, H840A, N854A, N863A and D986A. In a highly preferred embodiment the Cas protein has the D10A mutation.

Aspects of the invention relate to the expression of the gene product being decreased or a template polynucleotide being further introduced into the DNA molecule encoding the gene product or an intervening sequence being excised precisely by allowing the two 5' overhangs to reanneal and ligate or the activity or function of the gene product being altered or the expression of the gene product being increased. In an embodiment of the invention, the gene product is a protein.

The invention also comprehends an engineered, non-naturally occurring vector system comprising one or more vectors comprising:

a) a first regulatory element operably linked to each of two CRISPR-Cas system guide RNAs that target a first strand and a second strand respectively of a double stranded DNA molecule encoding a gene product,
b) a second regulatory element operably linked to a Cas protein,

wherein components (a) and (b) are located on same or different vectors of the system, wherein at least one of said vector(s) are delived via one or more nanoparticle complex(es), whereby the guide RNAs target the DNA molecule encoding the gene product and the Cas protein nicks each of the first strand and the second strand of the DNA molecule encoding the gene product, whereby expression of the gene product is altered; and, wherein the Cas protein and the two guide RNAs do not naturally occur together.

In aspects of the invention the guide RNAs may comprise a guide sequence fused to a tracr mate sequence and a tracr sequence. In an embodiment of the invention the Cas protein is a type II CRISPR-Cas protein. In an aspect of the invention the Cas protein is codon optimized for expression in a eukaryotic cell, preferably a mammalian cell or a human cell. In further embodiments of the invention the Cas protein is a type II CRISPR-Cas protein, e.g. a Cas 9 protein. In a highly preferred embodiment the Cas protein is a Cas9 protein, e.g. SpCas9. In aspects of the invention the Cas protein has one or more mutations selected from the group consisting of D10A, E762A, H840A, N854A, N863A and D986A. The Cas protein can have the D10A mutation.

Aspects of the invention relate to the expression of the gene product being decreased or a template polynucleotide being further introduced into the DNA molecule encoding the gene product or an intervening sequence being excised precisely by allowing the two 5' overhangs to reanneal and ligate or the activity or function of the gene product being altered or the expression of the gene product being increased. In an embodiment of the invention, the gene product is a protein. In preferred embodiments of the invention the vectors of the system are viral vectors. In a further embodiment, the vectors of the system are delivered via nanoparticles.

In one aspect, the invention provides a method of modifying a target polynucleotide in a eukaryotic cell. In some embodiments, the method comprises allowing a CRISPR complex to bind to the target polynucleotide to effect cleavage of said target polynucleotide thereby modifying the target polynucleotide, wherein the CRISPR complex comprises a CRISPR enzyme complexed with a guide sequence hybridized to a target sequence within said target polynucleotide, wherein said guide sequence is linked to a tracr mate sequence which in turn hybridizes to a tracr sequence; and advantageously the complex or a component thereof has been delivered via nanoparticle complex(es). In some embodiments, said cleavage comprises cleaving one or two strands at the location of the target sequence by said CRISPR enzyme. In some embodiments, said cleavage results in decreased transcription of a target gene. In some embodiments, the method further comprises repairing said cleaved target polynucleotide by homologous recombination with an exogenous template polynucleotide, wherein said repair results in a mutation comprising an insertion, deletion, or substitution of one or more nucleotides of said target polynucleotide. In some embodiments, said mutation results in one or more amino acid changes in a protein expressed from a gene comprising the target sequence. In some embodiments, the method further comprises delivering, e.g., via nanoparticle complex(es) one or more vectors to said eukaryotic cell, wherein the one or more vectors drive expression of one or more of: the CRISPR enzyme, the guide sequence linked to the tracr mate sequence, and the tracr sequence. In some embodiments, said vectors are delivered to the eukaryotic cell in a subject. In some embodiments, said modifying takes place in said eukaryotic cell in a cell culture. In some embodiments, the method further comprises isolating said eukaryotic cell from a subject prior to said modifying. In some embodiments, the method further comprises returning said eukaryotic cell and/or cells derived therefrom to said subject.

In one aspect, the invention provides a method of modifying expression of a polynucleotide in a eukaryotic cell. In some embodiments, the method comprises allowing a CRISPR complex to bind to the polynucleotide such that said binding results in increased or decreased expression of said polynucleotide; wherein the CRISPR complex comprises a CRISPR enzyme complexed with a guide sequence hybridized to a target sequence within said polynucleotide, wherein said guide sequence is linked to a tracr mate sequence which in turn hybridizes to a tracr sequence; and advantageously the complex or a component thereof has been delivered via nanoparticle complex(es). In some embodiments, the method further comprises delivering, e.g., via one or more nanoparticle complex(es0 one or more vectors to said eukaryotic cells, wherein the one or more vectors drive expression of one or more of: the CRISPR enzyme, the guide sequence linked to the tracr mate sequence, and the tracr sequence.

In one aspect, the invention provides a method of generating a model eukaryotic cell comprising a mutated disease gene. In some embodiments, a disease gene is any gene associated with an increase in the risk of having or developing a disease. In some embodiments, the method comprises (a) introducing one or more vectors into a eukaryotic cell, wherein the one or more vectors drive expression of one or more of: a CRISPR enzyme, a guide sequence linked to a tracr mate sequence, and a tracr sequence; and (b) allowing a CRISPR complex to bind to a target polynucleotide to effect cleavage of the target polynucleotide within said disease gene, wherein the CRISPR complex comprises the CRISPR enzyme complexed with (1) the guide sequence that is hybridizable to the target sequence within the target polynucleotide, and (2) the tracr mate sequence that is hybridizable to the tracr sequence, thereby generating a model eukaryotic cell comprising a mutated disease gene; and advantageously the complex or a component thereof has been delivered via nanoparticle complex(es). In some embodiments, said cleavage comprises cleaving one or two strands at the location of the target sequence by said CRISPR enzyme. In some embodiments, said cleavage results in decreased transcription of a target gene. In some embodiments, the method further comprises repairing said cleaved target polynucleotide by homologous recombination with an exogenous template polynucleotide, wherein said repair results in a mutation comprising an insertion, deletion, or substitution of one or more nucleotides of said target polynucleotide. In some embodiments, said mutation results in one or more amino acid changes in a protein expression from a gene comprising the target sequence.

In one aspect, the invention provides for methods of modifying a target polynucleotide in a eukaryotic cell. In some embodiments, the method comprises allowing a CRISPR complex to bind to the target polynucleotide to effect cleavage of said target polynucleotide thereby modifying the target polynucleotide, wherein the CRISPR complex comprises a CRISPR enzyme complexed with a guide sequence hybridized to a target sequence within said target polynucleotide, wherein said guide sequence is linked to a tracr mate sequence which in turn hybridizes to a tracr sequence; and advantageously the complex or a component thereof has been delivered via nanoparticle complex(es).

In other embodiments, this invention provides a method of modifying expression of a polynucleotide in a eukaryotic cell. The method comprises increasing or decreasing expression of a target polynucleotide by using a CRISPR complex that binds to the polynucleotide; and advantageously the complex or a component thereof has been delivered via nanoparticle complex(es).

Where desired, to effect the modification of the expression in a cell, one or more vectors comprising a tracr sequence, a guide sequence linked to the tracr mate sequence, a sequence encoding a CRISPR enzyme is delivered to a cell; and advantageously the complex or a component thereof has been delivered via nanoparticle complex(es). In some methods, the one or more vectors comprises a regulatory element operably linked to an enzyme-coding sequence encoding said CRISPR enzyme comprising a nuclear localization sequence; and a regulatory element operably linked to a tracr mate sequence and one or more insertion sites for inserting a guide sequence upstream of the tracr mate sequence; and advantageously the complex or a component thereof has been delivered via nanoparticle complex(es). When expressed, the guide sequence directs sequence-specific binding of a CRISPR complex to a target sequence in a cell. Typically, the CRISPR complex comprises a CRISPR enzyme complexed with (1) the guide sequence that is hybridizable to the target sequence, and (2) the tracr mate sequence that is hybridizable to the tracr sequence; and advantageously the complex or a component thereof has been delivered via nanoparticle complex(es).

In some methods, a target polynucleotide can be inactivated to effect the modification of the expression in a cell. For example, upon the binding of a CRISPR complex to a target sequence in a cell, the target polynucleotide is inactivated such that the sequence is not transcribed, the coded protein is not produced, or the sequence does not function as the wild-type sequence does. For example, a protein or microRNA coding sequence may be inactivated such that the protein or microRNA or pre-microRNA transcript is not produced.

In certain embodiments, the CRISPR enzyme comprises one or more mutations selected from the group consisting of D10A, E762A, H840A, N854A, N863A or D986A and/or the one or more mutations is in a RuvC1 or HNH domain of the CRISPR enzyme or is a mutation as otherwise as discussed herein. In some embodiments, the CRISPR enzyme has one or more mutations in a catalytic domain, wherein when transcribed, the tracr mate sequence hybridizes to the tracr sequence and the guide sequence directs sequence-specific binding of a CRISPR complex to the target sequence, and wherein the enzyme further comprises a functional domain. In some embodiments, , the mutated Cas9 enzyme may be fused to a protein domain, e.g., such as a transcriptional activation domain. In one aspect, the functional domain is a transcriptional activation domain, preferably VP64. In some embodiments, the functional domain is a transcription repression domain, preferably KRAB. In some embodiments, the transcription repression domain is SID, or concatemers of SID (eg SID4X). In some embodiments, the functional domain is an epigenetic modifying domain, such that an epigenetic modifying enzyme is provided. In some embodiments, the functional domain is an activation domain, which may be the P65 activation domain.

Delivery can be in the form of a vector which may be a plasmid or other nucleic acid molecule form, especially when the delivery is via a nanoparticle complex; and the vector also can be viral vector, such as a lenti- or baculo- or preferably adeno-viral/adeno-associated viral vectors, but other means of delivery are known (such as yeast systems, microvesicles, gene guns/means of attaching vectors to gold nanoparticles) and are provided, especially as to those aspects of the complex not delivered via a nanoparticle complex. A vector may mean not only a viral or yeast system (for instance, where the nucleic acids of interest may be operably linked to and under the control of (in terms of expression, such as to ultimately provide a processed RNA) a promoter), but also direct delivery of nucleic acids into a host cell; and advantageously the complex or a component thereof is delivered via nanoparticle complex(es). Also envisaged is a method of delivering the present CRISPR enzyme comprising delivering to a cell mRNA encoding the CRISPR enzyme; and advantageously the complex or a component thereof has been delivered via nanoparticle complex(es). It will be appreciated that in certain embodiments the CRISPR enzyme is truncated, and/or comprised of less than one thousand amino acids or less than four thousand amino acids, and/or is a nuclease or nickase, and/or is codon-optimized, and/or comprises one or more mutations, and/or comprises a chimeric CRISPR enzyme, and/or the other options as herein discussed. When not delivering via a nanoparticle complex, AAV and lentiviral vectors are preferred.

In certain embodiments, the target sequence is flanked or followed, at its 3' end, by a PAM suitable for the CRISPR enzyme, typically a Cas and in particular a Cas9.

For example, a suitable PAM is 5'-NRG or 5'-NNGRR for SpCas9 or SaCas9 enzymes (or derived enzymes), respectively.

In certain embodiments, the invention provides nanoparticle formulation comprising one or more guide RNAs are delivered *in vitro, ex vivo* or *in vivo* in the context of the CRISPR-Cas system.

In certain embodiments, it may be useful to deliver the guide RNA-nanoparticle formulations separately from the Cas9. In such an instance a dual-delivery system is envisaged such that the Cas 9 may be delivered *via* a vector and the guide RNAs are provided in a nanoparticle formulation, where vectors are considered in the broadest light as simply any means of delivery, rather than specifically viral vectors. It can be envisioned that separate delivery of the guide RNA-nanoparticle formulation and the Cas 9 may be sequential, for example, first Cas9 vector is delivered *via* a vector system followed by delivery of sgRNA-nanoparticle formulation) or the sgRNA-nanoparticle formulation and Cas9 may be delivered substantially contemporaneously (*i.e.*, co-delivery). Sequential delivery may be done at separate points in time, separated by days, weeks or even months.

In certain embodiments, multiple guide RNAs formulated in one or more delivery vehicles (e.g., where some guide RNAs are provided in a vector and others are formulated in nanoparticles) may be provided with a Cas9 delivery system.

In certain embodiments, the Cas9 is also delivered in a nanoparticle formulation. In such an instance the guide RNA-nanoparticle formulation and the Cas9 nanoparticle formulation may be delivered separately or may be delivered substantially contemporaneously (*i.e.,* co-delivery). Sequential delivery could be done at separate points in time, separated by days, weeks or even months.

In ceratin embodiments, nanoparticle formulations comprising one or more guide RNAs are adapted for delivery *in vitro, ex vivo* or *in vivo* in the context of the CRISPR-Cas system to different target genes, different target cells or different target different tissues/organs, e.g., heart or muscle or lung. Multiplexed gene targeting using nanoparticle formulations comprising one or more guide RNAs are also envisioned.

In an embodiment, a nanoparticle formulation comprising one or more components of the CRISPR-Cas system is provided.

In another embodiment, a gRNA-nanoparticle formulation comprising one or more guide RNAs is provided.

In certain embodiments, a composition comprising a nanoparticle formulation comprising one or more components of the CRISPR-Cas system is provided.

In certain embodiments, a pharmaceutical composition comprising a nanoparticle formulation comprising one or more components of the CRISPR-Cas system is provided.

In certain embodiments, a method for in vitro, ex vivo, and/or in vivo functional gene silencing comprising administering a composition comprising a nanoparticle formulation comprising one or more components of the CRISPR-Cas system is provided.

In certain embodiments, a method for in vitro, ex vivo, and/or in vivo functional gene silencing comprising administering a nanoparticle formulation comprising one or more components of the CRISPR-Cas system is provided.

In certain embodiments, a method for in vitro, ex vivo, and/or in vivo functional gene silencing comprising a gRNA-nanoparticle formulation comprising one or more guide RNAs is provided.

In certain embodiments, a method for in vitro, ex vivo, and/or in vivo functional gene silencing in endothelial cells comprising administering a nanoparticle formulation comprising one or more components of the CRISPR-Cas system is provided

In certain embodiments, a method for in vitro, ex vivo, and/or in vivo functional gene silencing in endothelial cells comprising administering a gRNA-nanoparticle formulation comprising one or more guide RNAs is provided.

In certain embodiments, a method for in vitro, ex vivo, and/or in vivo functional gene silencing in endothelial cells in the lung and/or heart comprising a gRNA-nanoparticle formulation comprising one or more guide RNAs is provided.

In certain embodiments a method of treating a subject suffering from a disease or disorder associated with the endothelium in any tissue or organ comprising administering a composition comprising a nanoparticle formulation comprising one or more components of the CRISPR-Cas system.

In certain embodiments a method of treating a subject suffering from a disease or disorder associated with the endothelium in any tissue or organ comprising administering a gRNA- nanoparticle formulation comprising one or more guide RNAs.

To improve activity, sgRNA may be pre-complexed with the Cas9 protein, before formulating the entire complex in a particle. Formulations may be made with a different molar ratio of different components known to promote delivery of nucleic acids into cells (e.g. 1,2-dioleoyl-3-trimethylammonium-propane (DOTAP), 1,2-ditetradecanoyl-*sn*-glycero-3-phosphocholine (DMPC), polyethylene glycol (PEG), and cholesterol) For example DOTAP : DMPC : PEG : Cholesterol Molar Ratios may be DOTAP 100, DMPC 0, PEG 0, Cholesterol 0; or DOTAP 90, DMPC 0, PEG 10, Cholesterol 0; or DOTAP 90, DMPC 0, PEG 5, Cholesterol 5. DOTAP 100, DMPC 0, PEG 0, Cholesterol 0. The invention accordingly comprehends admixing sgRNA, Cas9 protein and components that form a particle; as well as particles from such admixing.

Particles were formed using an efficient process. First, Cas9 protein and sgRNA targeting the gene EMX1 or the control gene LacZ were mixed together at a suitable, e.g.,3:1 to 1:3 or 2:1 to 1:2 or 1:1 molar ratio, at a suitable temperature, e.g., 15-30C, e.g., 20-25C, e.g., room temperature, for a suitable time, e.g., 15-45, such as 30 minutes, advantageously in sterile, nuclease free buffer, e.g., 1X PBS. Separately, particle components such as or comprising: a surfactant, e.g., cationic lipid, e.g., 1,2-dioleoyl-3-trimethylammonium-propane (DOTAP); phospholipid, e.g., dimyristoylphosphatidylcholine (DMPC); biodegradable polymer, such as an ethylene-glycol polymer or PEG, and a lipoprotein, such as a low-density lipoprotein, e.g., cholesterol were dissolved in an alcohol, advantageously a $C_{1-6}$ alkyl alcohol, such as methanol, ethanol, isopropanol, e.g., 100% ethanol. The two solutions were mixed together to form particles containing the Cas9-sgRNA complexes. Accordingly, the invention comprehends admixing sgRNA, Cas9 protein and components that form a particle, e.g., comprising admixing an sgRNA and Cas9 protein mixture with a mixture comprising or consisting essentially of or consisting of surfactant, phospholipid, biodegradable polymer, lipoprotein and alcohol, and such a method to form particles containing the sgRNA and Cas9 protein, and particles therefrom.

In a preferred embodiment, particles containing the Cas9-sgRNA complexes may be formed by mixing Cas9 protein and one or more sgRNAs together, preferably at a 1:1 molar ratio, enzyme: guide RNA. Separately, the different components known to promote delivery of nucleic acids (e.g. DOTAP, DMPC, PEG, and cholesterol) are dissolved, preferably in ethanol. The two solutions are mixed together to form particles containing the Cas9-sgRNA complexes. After the particles are formed, Cas9-sgRNA complexes may be transfected into cells (e.g. HSCs). Bar coding may be applied. The particles, the Cas-9 and/or the sgRNA may be barcoded.

In an aspect of the invention, barcoding techniques of WO/2013/138585 A1 can be adapted or integrated into the practice of the invention. WO/2013/138585 A1 provides methods for simultaneously determining the effect of a test condition on viability or proliferation of each of a plurality of genetically heterogeneous cell types. The methods include: providing a unitary sample comprising a plurality of, e.g., five, ten, twenty, twenty-five, or more, genetically heterogeneous cell types (each individual cell type is genetically homogeneous within itself, but differs from the others in the plurality), wherein each cell type further comprises: (i) an exogenous nucleic acid tag stably integrated into the genome of the cells, e.g., a tag comprising a core sequence that is unique to each cell type, and flanking amplification primer binding sequences that are the same in all of the cells of the plurality, and (ii) optionally, a marker, e.g., a selectable or detectable marker; and a known number of cells of each cell type is present in the sample; exposing the sample to a test condition for a selected time; and detecting a level of the exogenous nucleic acid tag in each cell type, wherein the level of the exogenous nucleic acid tag is proportional to the number of living cells in the sample after exposure to the test condition; and comparing the number of living cells in the sample after exposure to the test condition to a reference number of cells. The number of living cells in the sample after exposure to the test condition as compared to the reference number of cells indicates the effect of the test condition on viability or proliferation of each cell type. WO/2013/138585 A1 also provides methods for simultaneously determining the effect of a test condition on viability or proliferation of each of a plurality of genetically heterogeneous cell types, wherein the methods include providing a unitary sample comprising a plurality of, e.g., five, ten, twenty, twenty-five, or more, genetically heterogeneous cell types, wherein each cell type further comprises: (i) an exogenous nucleic acid tag stably integrated into the genome of the cells, e.g., comprising a core sequence that is unique to each cell type, and flanking amplification primer binding sequences that are the same in all of the cells of the plurality, and (ii) optionally, a selectable or detectable marker; and a known number of cells of each cell type is present in the sample; implanting the sample into a living animal; exposing the sample to a test condition for a selected time; harvesting the sample from the animal; and detecting a level of the exogenous nucleic acid tag in each cell type of the sample, wherein the level of the exogenous nucleic acid tag correlates to the number of living cells in the sample after exposure to the test condition; and comparing the number of living cells in the sample after exposure to the test condition to a reference number of cells. The number of living cells in the sample after exposure to the test condition as compared to the reference number of cells indicates the effect of the test condition on viability or proliferation of each cell type. The tag can be Cas9 or another TAG or marker that is integrated into the genome of cells to be transplanted into or onto a non-human eukaryote, e.g., animal model, or that is integrated into the genome of the non-human transgenic eukaryote, e.g., animal, mammal, primate, rodent, mouse, rat, rabbit, etc (along with coding for Cas9). The test condition can be the administration or delivery of the RNA(s) to guide the Cas9 to induce one or more or a plurality, e.g., 3-50 or more, mutations. The test condition can be the administration, delivery or contacting with a putative chemical agent treatment and/or gene therapy treatment. The tag can also be the one or more or a plurality, e.g., 3-50 or more mutations, and the test condition can be the administration, delivery or contacting with a putative chemical agent treatment and/or gene therapy treatment.

The invention in an embodiment comprehends a method of preparing an sgRNA-and-Cas9 protein containing particle comprising admixing an sgRNA and Cas9 protein mixture with a mixture comprising or consisting essentially of or consisting of surfactant, phospholipid, biodegradable polymer, lipoprotein and alcohol. An embodiment comprehends an sgRNA-and-Cas9 protein containing particle from the method. The invention in an embodiment comprehends use of the particle in a method of modifying a genomic locus of interest, or an organism or a non-human organism by manipulation of a target sequence in a genomic locus of interest, comprising contacting a cell containing the genomic locus of interest with the particle wherein the sgRNA targets the genomic locus of interest; or a method of modifying a genomic locus of interest, or an organism or a non-human organism by manipulation of a target sequence in a genomic locus of interest, comprising contacting a cell containing the genomic locus of interest with the particle wherein the sgRNA targets the genomic locus of interest.

Accordingly, it is an object of the invention to not encompass within the invention any previously known product, process of making the product, or method of using the product such that Applicants reserve the right and hereby disclose a disclaimer of any previously known product, process, or method. It is further noted that the invention does not intend to encompass within the scope of the invention any product, process, or making of the product or method of using the product, which does not meet the written description and enablement requirements of the USPTO (35 U.S.C. §112, first paragraph) or the EPO (Article 83 of the EPC), such that Applicants reserve the right and hereby disclose a disclaimer of any previously described product, process of making the product, or method of using the product.

It is noted that in this disclosure and particularly in the claims and/or paragraphs, terms such as "comprises", "comprised", "comprising" and the like can have the meaning attributed to it in U.S. Patent law; e.g., they can mean "includes", "included", "including", and the like; and that terms such as "consisting essentially of' and "consists essentially of' have the meaning ascribed to them in U.S. Patent law, e.g., they allow for elements not explicitly recited, but exclude elements that are found in the prior art or that affect a basic or novel characteristic of the invention. It may be advantageous in the practice of the invention to be in compliance with Art. 53(c) EPC and Rule 28(b) and (c) EPC. Nothing herein is a promise.

These and other embodiments are disclosed or are obvious from and encompassed by, the following Detailed Description.

## BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

Figure 1 shows a schematic model of the CRISPR system. The Cas9 nuclease from Streptococcus pyogenes (yellow) is targeted to genomic DNA by a synthetic guide RNA (sgRNA) consisting of a 20-nt guide sequence (blue) and a scaffold (red). The guide sequence base-pairs with the DNA target (blue), directly upstream of a requisite 5'-NGG protospacer adjacent motif (PAM; magenta), and Cas9 mediates a double-stranded break (DSB) ~3 bp upstream of the PAM (red triangle).

Figure 2A-F shows an exemplary CRISPR system, a possible mechanism of action, an example adaptation for expression in eukaryotic cells, and results of tests assessing nuclear localization and CRISPR activity.

Figure 3A-D shows results of an evaluation of SpCas9 specificity for an example target.

Figure 4A-G show an exemplary vector system and results for its use in directing homologous recombination in eukaryotic cells.

Figure 5 provides a table of protospacer sequences and summarizes modification efficiency results for protospacer targets designed based on exemplary S. pyogenes and S. thermophilus CRISPR systems with corresponding PAMs against loci in human and mouse genomes. Cells were transfected with Cas9 and either pre-crRNA/tracrRNA or chimeric RNA, and analyzed 72 hours after transfection. Percent indels are calculated based on Surveyor assay results from indicated cell lines (N=3 for all protospacer targets, errors are S.E.M., N.D. indicates not detectable using the Surveyor assay, and N.T. indicates not tested in this study).

Figure 6A-C shows a comparison of different tracrRNA transcripts for Cas9-mediated gene targeting.

Figure 7 shows a schematic of a surveyor nuclease assay for detection of double strand break-induced micro-insertions and -deletions.

Figure 8A-B shows exemplary bicistronic expression vectors for expression of CRISPR system elements in eukaryotic cells.

Figure 9A-C shows histograms of distances between adjacent S. pyogenes SF370 locus 1 PAM (NGG) (Figure 9A) and S. thermophilus LMD9 locus 2 PAM (NNAGAAW) (Figure 9B) in the human genome; and distances for each PAM by chromosome (Chr) (Figure 9C).

Figure 10A-D shows an exemplary CRISPR system, an example adaptation for expression in eukaryotic cells, and results of tests assessing CRISPR activity.

Figure 11A-C shows exemplary manipulations of a CRISPR system for targeting of genomic loci in mammalian cells.

Figure 12A-B shows the results of a Northern blot analysis of crRNA processing in mammalian cells.

Figure 13A-B shows an exemplary selection of protospacers in the human PVALB and mouse Th loci.

Figure 14 shows example protospacer and corresponding PAM sequence targets of the S. thermophilus CRISPR system in the human EMX1 locus.

Figure 15 provides a table of sequences for primers and probes used for Surveyor, RFLP, genomic sequencing, and Northern blot assays.

Figure 16A-D shows a circular depiction of the phylogenetic analysis revealing five families of Cas9s, including three groups of large Cas9s (~1400 amino acids) and two of small Cas9s (~1100 amino acids).

Figure 17A-F shows the linear depiction of the phylogenetic analysis revealing five families of Cas9s, including three groups of large Cas9s (~1400 amino acids) and two of small Cas9s (-1100 amino acids).

Figure 18A-D shows genome editing via homologous recombination. (a) Schematic of SpCas9 nickase, with D10A mutation in the RuvC I catalytic domain. (b) Schematic representing homologous recombination (HR) at the human EMX1 locus using either sense or antisense single stranded oligonucleotides as repair templates. Red arrow above indicates sgRNA cleavage site; PCR primers for genotyping (Tables J and K) are indicated as arrows in right panel. (c) Sequence of region modified by HR. d, SURVEYOR assay for wildtype (wt) and nickase (D10A) SpCas9-mediated indels at the EMX1 target 1 locus (n=3). Arrows indicate positions of expected fragment sizes.

Figure 19A-B shows single vector designs for SpCas9.

Figure 20 shows a graph representing the length distribution of Cas9 orthologs.

Figure 21A-M shows sequences where the mutation points are located within the SpCas9 gene.

Figure 22A shows the Conditional Cas9, Rosa26 targeting vector map.

Figure 22B shows the Constitutive Cas9, Rosa26 targeting vector map.

Figure 23 shows a schematic of the important elements in the Constitutive and Conditional Cas9 constructs.

Figure 24A-C shows RNA delivery of Cas9 and chimeric RNA into cells (A) Delivery of a GFP reporter as either DNA or mRNA into Neuro-2A cells. (B) Delivery of Cas9 and chimeric RNA against the Icam2 gene as RNA results in cutting for one of two spacers tested. (C) Delivery of Cas9 and chimeric RNA against the F7 gene as RNA results in cutting for one of two spacers tested.

Figure 25 shows how DNA double-strand break (DSB) repair promotes gene editing. In the error-prone non-homologous end joining (NHEJ) pathway, the ends of a DSB are processed by endogenous DNA repair machineries and rejoined together, which can result in random insertion/deletion (indel) mutations at the site of junction. Indel mutations occurring within the coding region of a gene can result in frame-shift and a premature stop codon, leading to gene knockout. Alternatively, a repair template in the form of a plasmid or single-stranded oligodeoxynucleotides (ssODN) can be supplied to leverage the homology-directed repair (HDR) pathway, which allows high fidelity and precise editing.

Figure 26A-C shows anticipated results for HDR in HEK and HUES9 cells. (a) Either a targeting plasmid or an ssODN (sense or antisense) with homology arms can be used to edit the sequence at a target genomic locus cleaved by Cas9 (red triangle). To assay the efficiency of HDR, we introduced a HindIII site (red bar) into the target locus, which was PCR-amplified with primers that anneal outside of the region of homology. Digestion of the PCR product with HindIII reveals the occurrence of HDR events. (b) ssODNs, oriented in either the sense or the antisense (s or a) direction relative to the locus of interest, can be used in combination with Cas9 to achieve efficient HDR-mediated editing at the target locus. A minimal homology region of 40 bp, and preferably 90 bp, is recommended on either side of the modification (red bar). (c) Example of the effect of ssODNs on HDR in the EMX1 locus is shown using both wild-type Cas9 and Cas9 nickase (D10A). Each ssODN contains homology arms of 90 bp flanking a 12-bp insertion of two restriction sites.

Figure 27A-C shows the repair strategy for Cystic Fibrosis delta F508 mutation.

Figure 28 shows a screen for efficient SpCas9 mediated targeting of Tet1-3 and Dnmt1, 3a and 3b gene loci. Surveyor assay on DNA from transfected N2A cells demonstrates efficient DNA cleavage by using different gRNAs.

Figure 29 shows a strategy of multiplex genome targeting using a 2-vector system in an AAV1/2 delivery system. Tet1-3 and Dnmt1, 3a and 3b gRNA under the control of the U6 promoter. GFP-KASH under the control of the human synapsin promoter. Restriction sides shows simple gRNA replacement strategy by subcloning. HA-tagged SpCas9 flanked by two nuclear localization signals (NLS) is shown. Both vectors are delivered into the brain by AAV1/2 virus in a 1:1 ratio.

Figure 30 shows verification of multiplex DNMT targeting vector #1 functionality using Surveyor assay. N2A cells were co-transfected with the DNMT targeting vector #1 (+) and the SpCas9 encoding vector for testing SpCas9 mediated cleavage of DNMTs genes family loci. gRNA only (-) is negative control. Cells were harvested for DNA purification and downstream processing 48 h after transfection.

Figure 31 shows verification of multiplex DNMT targeting vector #2 functionality using Surveyor assay. N2A cells were co-transfected with the DNMT targeting vector #1 (+) and the SpCas9 encoding vector for testing SpCas9 mediated cleavage of DNMTs genes family loci. gRNA only (-) is negative control. Cells were harvested for DNA purification and downstream processing 48 h after transfection.

Figure 32 shows schematic overview of short promoters and short polyA versions used for HA-SpCas9 expression in vivo. Sizes of the encoding region from L-ITR to R-ITR are shown on the right.

Figure 33 shows schematic overview of short promoters and short polyA versions used for HA-SaCas9 expression in vivo. Sizes of the encoding region from L-ITR to R-ITR are shown on the right.

Figure 34 shows expression of SpCas9 and SaCas9 in N2A cells. Representative Western blot of HA-tagged SpCas9 and SaCas9 versions under the control of different short promoters and with or short polyA (spA) sequences. Tubulin is loading control. mCherry (mCh) is a transfection control. Cells were harvested and further processed for Western blotting 48 h after transfection.

Figure 35 shows screen for efficient SaCas9 mediated targeting of Tet3 gene locus. Surveyor assay on DNA from transfected N2A cells demonstrates efficient DNA cleavage by using different gRNAs with NNGGGT PUM sequence. GFP transfected cells and cells expressing only SaCas9 are controls.

Figure 36 shows expression of HA-SaCas9 in the mouse brain. Animals were injected into dentate gyri with virus driving expression of HA-SaCas9 under the control of human Synapsin promoter. Animals were sacrificed 2 weeks after surgery. HA tag was detected using rabbit monoclonal antibody C29F4 (Cell Signaling). Cell nuclei stained in blue with DAPI stain.

Figure 37 shows expression of SpCas9 and SaCas9 in cortical primary neurons in culture 7 days after transduction. Representative Western blot of HA-tagged SpCas9 and SaCas9 versions under the control of different promoters and with bgh or short polyA (spA) sequences. Tubulin is loading control.

Figure 38 shows LIVE/DEAD stain of primary cortical neurons 7 days after transduction with AAV1 particles carrying SpCas9 with different promoters and multiplex gRNAs constructs (example shown on the last panel for DNMTs). Neurons after AAV transduction were compared with control untransduced neurons. Red nuclei indicate permeabilized, dead cells (second line of panels). Live cells are marked in green color (third line of panels).

Figure 39 shows LIVE/DEAD stain of primary cortical neurons 7 days after transduction with AAV1 particles carrying SaCas9 with different promoters. Red nuclei indicate permeabilized, dead cells (second line of panels). Live cells are marked in green color (third line of panels).

Figure 40 shows comparison of morphology of neurons after transduction with AAV1 virus carrying SpCas9 and gRNA multiplexes for TETs and DNMTs genes loci. Neurons without transduction are shown as a control.

Figure 41 shows verification of multiplex DNMT targeting vector #1 functionality using Surveyor assay in primary cortical neurons. Cells were co-transduced with the DNMT targeting vector #1 and the SpCas9 viruses with different promoters for testing SpCas9 mediated cleavage of DNMTs genes family loci.

Figure 42 shows in vivo efficiency of SpCas9 cleavage in the brain. Mice were injected with AAV1/2 virus carrying gRNA multiplex targeting DNMT family genes loci together with SpCas9 viruses under control of 2 different promoters: mouse Mecp2 and rat Map1b. Two weeks after injection brain tissue was extracted and nuclei were prepped and sorted using FACS, based on the GFP expression driven by Synapsin promoter from gRNA multiplex construct. After gDNA extraction Surveyor assay was run. + indicates GFP positive nuclei and - control, GFP-negative nuclei from the same animal. Numbers on the gel indicate assessed SpCas9 efficiency.

Figure 43 shows purification of GFP-KASH labeled cell nuclei from hippocampal neurons. The outer nuclear membrane (ONM) of the cell nuclear membrane is tagged with a fusion of GFP and the KASH protein transmembrane domain. Strong GFP expression in the brain after one week of stereotactic surgery and AAV1/2 injection. Density gradient centrifugation step to purify cell nuclei from intact brain. Purified nuclei are shown. Chromatin stain by Vybrant® DyeCycle™ Ruby Stain is shown in red, GFP labeled nuclei are green. Representative FACS profile of GFP+ and GFP- cell nuclei (Magenta: Vybrant® DyeCycle™ Ruby Stain, Green: GFP).

Figure 44 shows efficiency of SpCas9 cleavage in the mouse brain. Mice were injected with AAV1/2 virus carrying gRNA multiplex targeting TET family genes loci together with SpCas9 viruses under control of 2 different promoters: mouse Mecp2 and rat Map1b. Three weeks after injection brain tissue was extracted, nuclei were prepped and sorted using FACS, based on the GFP expression driven by Synapsin promoter from gRNA multiplex construct. After gDNA extraction Surveyor assay was run. + indicates GFP positive nuclei and - control, GFP-negative nuclei from the same animal. Numbers on the gel indicate assessed SpCas9 efficiency.

Figure 45 shows GFP-KASH expression in cortical neurons in culture. Neurons were transduced with AAV1 virus carrying gRNA multiplex constructs targeting TET genes loci. The strongest signal localize around cells nuclei due to KASH domain localization.

Figure 46A-M shows generation of the Cre-dependent Cas9 mouse and its application for *in vivo* genome editing (a) Cas9 Rosa26 targeting vector and knockin schematic. The transgene includes: (1) 5' homology arm, (2) ubiquitously expressed CAG promoter, (3) Cre-dependent loxP - 3xSV40 polyA - loxP stop cassette (LSL), (4) 3xFLAG epitope tag, (5) *Streptococcus pyogenes* Cas9 flanked by two nuclear localization signals (NLSs), (6) WPRE, (7) bovine growth hormone polyA, (8) pPGK - Neo - pA positive selection cassette, (9) 3' Rosa26 homology arm, and (10) pPGK - DTA - pA negative selection cassette. (b) Stereotactic bright field and EGFP imaging showing constitutive expression of Cas9-P2A-EGFP in constitutively Cas9 expressing mice. (c) Immunoblot of whole brain lysates showing 3xFLAG-Cas9 staining at the predicted molecular weight (167 kDa) in constitutively Cas9 expressing mice but not wild-type (WT) mice. Beta-tubulin and GAPDH were used as loading controls. (d) AAV vector schematic. (e) NeuN targeting design and representative Illumina sequencing reads showing indel formation near the predicted cleavage site (red arrow). (f) Craniotomy injections schematic showing Cre-dependent Cas9 mice were injected in the prefrontal cortex with sgNeuN and Cre by AAV1/2. (g) Immunoblot of an acutely dissected injection site showing NeuN depletion in the sgNeuN injected hemisphere and not in the non-injected, contralateral side (CTR), or WT animals. Cas9 expression (anti-3xFLAG) is only detected upon Cre delivery (anti-Cre-HA). Beta-tubulin was used as a loading control. (h) Representative immunofluorescence images of Cre-dependent Cas9 mice injected in the prefrontal cortex showing Cre expression excises the LSL cassette leading to Cas9-P2A-EGFP expression and NeuN depletion. The bottom row is a magnification of the boundary between infected and non-infected cells. Scale bars, 200 μm (top) and 50 μm (bottom). (i) Representative immunofluorescence images of Cre-dependent Cas9 mice injected bilaterally in the prefrontal cortex showing NeuN depletion only in the sgNeuN-injected and not sgLacZ-injected hemisphere. Scale bar, 200 μm. (j) Immunoblot quantification showing significant NeuN depletion in sgNeuN-injected and not sgLacZ-injected Cre-dependent Cas9 mice. Data are plotted as mean ± SEM (n=3 mice). *** p-value <0.0005. (k) Indel quantification using deep sequencing from amplified DNA from acutely dissected brain tissue (as in j) showing significant indels in sgNeuN-injected and not sgLacZ-injected Cre-dependent Cas9 mice. Data are plotted as mean ± SEM (n=3 mice). *** p-value <0.0005. (1) Single nuclei genotyping of AAV-sgRNA transduced neurons showing the majority of cells have been modified on both alleles. Data are plotted as the mean of 167 nuclei. (m) Single nuclei allele mutation analysis showing a distribution of sense (sen) and missense (mis) mutations. Data are plotted as the mean of 15 and 141 nuclei for heterozygous and homozygous nuclei, respectively.

Figure 47A-H shows cell type-specific Cas9 expression and normal neuronal electrophysiology (a) Representative immunofluorescence images of the substantia nigra in progenies from a Cre-dependent Cas9 mouse crossed with the TH-IRES-Cre driver. Double white arrows highlight a cell expressing TH and Cas9-P2A-EGFP. Single white arrow highlights a cell expressing neither TH nor Cas9-P2A-EGFP. Scale bar, 50 μm. (b) Representative immunofluorescence images of the thalamus in progenies from a Cre-dependent Cas9 mouse crossed with the PV-Cre driver. Double white arrows highlight a cell expressing PV and Cas9-P2A-EGFP. Single white arrow highlights a cell expressing neither PV nor Cas9-P2A-EGFP. Scale bar, 50 μm. (c-d) Representative current clamp recording and action potential firing from (c) wild-type (WT) and (d) constitutively Cas9 expressing neurons. Scale bar, 200 ms. (e-h) Hippocampal slice electrophysiological measurements showing no significant difference between constitutively Cas9 expressing neurons and neurons from WT mice using the metrics: (e) rheobase current, (f) input resistance, (g) whole cell capacitance, and (h) resting membrane potential. Data are plotted as mean ± SEM. n=12 neurons from 2 mice for WT and n=15 neurons from 2 mice for constitutively Cas9 expressing mice. ns, not significant. (Associated with Supplementary Table 2)

Figure 48A-H shows nanoparticle delivery of sgRNAs to endothelial cells of the constitutively Cas9 expressing mouse (a) Nanoparticle:sgRNA intravenous (i.v.) delivery and downstream assay schematic. (b) Dynamic light scattering (DLS), size distribution plot showing 7C1:sgRNA nanoparticles formed small, multilamellar structures (Dahlman, J. E. et al. Nat. Nanotechnol. 9, 648-655 (2014)). Data are plotted as mean ± SEM. Inset: CryoTEM image of 7C1:sgRNA. Scale bar, 50 μm. (c) ICAM2 locus targeting schematic and deep sequencing reads showing indels at the predicted cleavage site. (d-e) Indel analysis of sorted endothelial ($CD31^+$ and $CD45^-$) cells from lung (d) and heart (f) showing a significant indel percent in sgICAM2-1+20-injected, but not sgLacZ-injected constitutively Cas9 expressing mice. Data are plotted as mean ± SEM. *** p-value <0.0005. (f-g) Flow cytometry quantification of ICAM2 expression in endothelial ($CD31^+$ and $CD45^-$) cells dissociated from lung (f) and heart (g) showing significant protein depletion in sgICAM2-1+20-injected but not LacZ-injected constitutively Cas9 expressing mice. Data are plotted as median fluorescent intensity (MFI) ± SEM. * p-value < 0.05, ** p-value < 0.005, ***p-value<0.0005. (h) Weight change plot over the duration of the study showing no significant change. Data points are normalized based on day 0.

Figure 49 shows Cas9-P2A-EGFP expression in various organs. Stereotactic images showing Cas9-P2A-EGFP positive organs from constitutively Cas9 expressing mouse but not wild-type mice.

Figure 50 sows tightly controlled Cas9 expression. Representative fluorescence images showing lack of EGFP and FLAG staining in Cre-dependent Cas9 and wild-type mice.

Figure 51 shows normal cellular morphology of constitutively Cas9 expressing mice. Representative bright field images of HE stained tissues from the constitutively Cas9 expressing and a wild-type mouse.

Figure 52 shows no detectible DNA damage or apoptosis in constitutively Cas9 expressing mice. Representative bright field images of yH2AX, a marker for DNA damage, and cleaved caspase 3 (CC3), are marker for late stage apoptosis, stained tissues from the constitutively Cas9 expressing and a wild-type mouse.

Figure 53A-B shows *in vitro* validation of ICAM2-targeted sgRNAs. (a) ICAM2 locus schematic showing locations of sgICAM2 exon targets. SURVEYOR nuclease assay gel showing indel formation for most sgRNAs. Indel percent was quantified using relative band intensities and then plotted below. Red arrows highlight predicted cleavage products from SURVEYOR nuclease reaction. (b) SURVEYOR nuclease assay corresponding to the same samples used for indel analysis in (Fig. 48c). Red arrows highlight predicted cleavage products from SURVEYOR nuclease reaction.

The figures herein are for illustrative purposes only and are not necessarily drawn to scale.

DETAILED DESCRIPTION OF THE INVENTION

With respect to general information on CRISPR-Cas Systems, components thereof, and delivery of such components, including methods, materials, delivery vehicles, vectors, particles, viral vectors, adenovirus, AAV, lentivirus, and making and using thereof, including as to amounts and formulations, all useful in the practice of the instant invention, reference is made to: US Patents Nos. 8,697,359, 8,771,945, 8,795,965, 8,865,406, 8,871,445, 8,889,356, 8,889,418 and 8,895,308; US Patent Publications US 2014-0310830 (US APP. Ser. No. 14/105,031), US 2014-0287938 A1 (U.S. App. Ser. No. 14/213,991), US 2014-0273234 A1 (U.S. App. Ser. No. 14/293,674), US2014-0273232 A1 (U.S. App. Ser. No. 14/290,575), US 2014-0273231 (U.S. App. Ser. No. 14/259,420), US 2014-0256046 A1 (U.S. App. Ser. No. 14/226,274), US 2014-0248702 A1 (U.S. App. Ser. No. 14/258,458), US 2014-0242700 A1 (U.S. App. Ser. No. 14/222,930), US 2014-0242699 A1 (U.S. App. Ser. No. 14/183,512), US 2014-0242664 A1 (U.S. App. Ser. No. 14/104,990), US 2014-0234972 A1 (U.S. App. Ser. No. 14/183,471), US 2014-0227787 A1 (U.S. App. Ser. No. 14/256,912), US 2014-0189896 A1 (U.S. App. Ser. No. 14/105,035), US 2014-0186958 (U.S. App. Ser. No. 14/105,017), US 2014-0186919 A1 (U.S. App. Ser. No. 14/104,977), US 2014-0186843 A1 (U.S. App. Ser. No. 14/104,900), US 2014-0179770 A1 (U.S. App. Ser. No. 14/104,837) and US 2014-0179006 A1 (U.S. App. Ser. No. 14/183,486), US 2014-0170753 (US App Ser No 14/183,429); European Patent Applications EP 2 771 468 (EP13818570.7), EP 2 764 103 (EP13824232.6), and EP 2 784 162 (EP14170383.5); and PCT Patent Publications WO 2014/093661 (PCT/US2013/074743), WO 2014/093694 (PCT/US2013/074790), WO 2014/093595 (PCT/US2013/074611), WO 2014/093718 (PCT/US2013/074825), WO 2014/093709 (PCT/US2013/074812), WO 2014/093622 (PCT/US2013/074667), WO 2014/093635 (PCT/US2013/074691), WO 2014/093655 (PCT/US2013/074736), WO 2014/093712 (PCT/US2013/074819), WO2014/093701 (PCT/US2013/074800), and WO2014/018423 (PCT/US2013/051418). Reference is also made to US provisional patent applications 61/758,468; 61/802,174; 61/806,375; 61/814,263; 61/819,803 and 61/828,130, filed on January 30, 2013; March 15, 2013; March 28, 2013; April 20, 2013; May 6, 2013 and May 28, 2013 respectively. Reference is also made to US provisional patent application 61/836,123, filed on June 17, 2013. Reference is additionally made to US provisional patent applications 61/835,931, 61/835,936, 61/836,127, 61/836, 101, 61/836,080 and 61/835,973, each filed June 17, 2013. Further reference is made to US provisional patent applications 61/862,468 and 61/862,355 filed on August 5, 2013; 61/871,301 filed on August 28, 2013; 61/960,777 filed on September 25, 2013 and 61/961,980 filed on October 28, 2013. Reference is yet further made to: PCT Patent applications Nos: PCT/US2014/041803, PCT/US2014/041800, PCT/US2014/041809, PCT/US2014/041804 and PCT/US2014/041806, each filed June 10, 2014 6/10/14; PCT/US2014/041808 filed June 11, 2014; and PCT/US2014/62558 filed October 28, 2014, and US Provisional Patent Applications Serial Nos.: 61/915,251, 61/915,301 and 61/915,260, each filed December 12, 2013; 61/930,214, filed January 22, 2014; 62/010,329 and 62/010,441, each filed June 10, 2014; 61/939,228 and 61/939,242, each filed February 12, 2014; 61/980,012, filed April 15,2014; 62/038,358, filed August 17, 2014; 62/054,490, 62/055,484, 62/055,460 and 62/055,487, each filed September 25, 2014; and 62/069,243, filed October 27, 2014. And, reference is made to US provisional patent applications Serial Nos. 61/915,150, filed December 12, 2013; and 62/010,888 and 62/010,879, both filed June 11, 2014. Each of these patents, patent publications, and applications, and all documents cited therein or during their prosecution ("appln cited documents") and all documents cited or referenced in the appln cited documents, together with any instructions, descriptions, product specifications, and product sheets for any products mentioned therein or in any document therein and incorporated by reference herein, are hereby incorporated herein by reference, and may be employed in the practice of the invention. All documents (e.g., these patents, patent publications and applications and the appln cited documents) are incorporated herein by reference to the same extent as if each individual document was specifically and individually indicated to be incorporated by reference.

Also with respect to general information on CRISPR-Cas Systems, mention is made of:

- Multiplex genome engineering using CRISPR/Cas systems. Cong, L., Ran, F.A., Cox, D., Lin, S., Barretto, R., Habib, N., Hsu, P.D., Wu, X., Jiang, W., Marraffini, L.A., & Zhang, F. Science Feb 15;339(6121):819-23 (2013);
- RNA-guided editing of bacterial genomes using CRISPR-Cas systems. Jiang W., Bikard D., Cox D., Zhang F, Marraffini LA. Nat Biotechnol Mar;31(3):233-9 (2013);
- One-Step Generation of Mice Carrying Mutations in Multiple Genes by CRISPR/Cas-Mediated Genome Engineering. Wang H., Yang H., Shivalila CS., Dawlaty MM., Cheng AW., Zhang F., Jaenisch R. Cell May 9;153(4):910-8 (2013);
- Optical control of mammalian endogenous transcription and epigenetic states. Konermann S, Brigham MD, Trevino AE, Hsu PD, Heidenreich M, Cong L, Platt RJ, Scott DA, Church GM, Zhang F. Nature. 2013 Aug 22;500(7463):472-6. doi: 10.1038/Nature12466. Epub 2013 Aug 23;

- Double Nicking by RNA-Guided CRISPR Cas9 for Enhanced Genome Editing Specificity. Ran, FA., Hsu, PD., Lin, CY., Gootenberg, JS., Konermann, S., Trevino, AE., Scott, DA., Inoue, A., Matoba, S., Zhang, Y., & Zhang, F. Cell Aug 28. pii: S0092-8674(13)01015-5. (2013);
- DNA targeting specificity of RNA-guided Cas9 nucleases. Hsu, P., Scott, D., Weinstein, J., Ran, FA., Konermann, S., Agarwala, V., Li, Y., Fine, E., Wu, X., Shalem, O., Cradick, TJ., Marraffini, LA., Bao, G., & Zhang, F. Nat Biotechnol doi:10.1038/nbt.2647 (2013);
- Genome engineering using the CRISPR-Cas9 system. Ran, FA., Hsu, PD., Wright, J., Agarwala, V., Scott, DA., Zhang, F. Nature Protocols Nov;8(11):2281-308. (2013);
- Genome-Scale CRISPR-Cas9 Knockout Screening in Human Cells. Shalem, O., Sanjana, NE., Hartenian, E., Shi, X., Scott, DA., Mikkelson, T., Heckl, D., Ebert, BL., Root, DE., Doench, JG., Zhang, F. Science Dec 12. (2013). [Epub ahead of print];
- Crystal structure of cas9 in complex with guide RNA and target DNA. Nishimasu, H., Ran, FA., Hsu, PD., Konermann, S., Shehata, SI., Dohmae, N., Ishitani, R., Zhang, F., Nureki, O. Cell Feb 27. (2014). 156(5):935-49;
- Genome-wide binding of the CRISPR endonuclease Cas9 in mammalian cells. Wu X., Scott DA., Kriz AJ., Chiu AC., Hsu PD., Dadon DB., Cheng AW., Trevino AE., Konermann S., Chen S., Jaenisch R., Zhang F., Sharp PA. Nat Biotechnol. (2014) Apr 20. doi: 10.1038/nbt.2889,
- CRISPR-Cas9 Knockin Mice for Genome Editing and Cancer Modeling, Platt et al., Cell 159(2): 440-455 (2014) DOI: 10.1016/j.cell.2014.09.014,
- Development and Applications of CRISPR-Cas9 for Genome Engineering, Hsu et al, Cell 157, 1262-1278 (June 5, 2014) (Hsu 2014),
- Genetic screens in human cells using the CRISPR/Cas9 system, Wang et al., Science. 2014 January 3; 343(6166): 80-84. doi:10.1126/science.1246981,
- Rational design of highly active sgRNAs for CRISPR-Cas9-mediated gene inactivation, Doench et al., Nature Biotechnology published online 3 September 2014; doi:10.1038/nbt.3026, and
- In vivo interrogation of gene function in the mammalian brain using CRISPR-Cas9, Swiech et al, Nature Biotechnology; published online 19 October 2014; doi:10.1038/nbt.3055.

each of which is incorporated herein by reference, and discussed briefly below:

- Cong et al. engineered type II CRISPR-Cas systems for use in eukaryotic cells based on both Streptococcus thermophilus Cas9 and also Streptoccocus pyogenes Cas9 and demonstrated that Cas9 nucleases can be directed by short RNAs to induce precise cleavage of DNA in human and mouse cells. Their study further showed that Cas9 as converted into a nicking enzyme can be used to facilitate homology-directed repair in eukaryotic cells with minimal mutagenic activity. Additionally, their study demonstrated that multiple guide sequences can be encoded into a single CRISPR array to enable simultaneous editing of several at endogenous genomic loci sites within the mammalian genome, demonstrating easy programmability and wide applicability of the RNA-guided nuclease technology. This ability to use RNA to program sequence specific DNA cleavage in cells defined a new class of genome engineering tools. These studies further showed that other CRISPR loci are likely to be transplantable into mammalian cells and can also mediate mammalian genome cleavage. Importantly, it can be envisaged that several aspects of the CRISPR-Cas system can be further improved to increase its efficiency and versatility.
- Jiang et al. used the clustered, regularly interspaced, short palindromic repeats (CRISPR)-associated Cas9 endonuclease complexed with dual-RNAs to introduce precise mutations in the genomes of Streptococcus pneumoniae and Escherichia coli. The approach relied on dual-RNA:Cas9-directed cleavage at the targeted genomic site to kill unmutated cells and circumvents the need for selectable markers or counter-selection systems. The study reported reprogramming dual-RNA:Cas9 specificity by changing the sequence of short CRISPR RNA (crRNA) to make single- and multinucleotide changes carried on editing templates. The study showed that simultaneous use of two crRNAs enabled multiplex mutagenesis. Furthermore, when the approach was used in combination with recombineering, in S. pneumoniae, nearly 100% of cells that were recovered using the described approach contained the desired mutation, and in E. coli, 65% that were recovered contained the mutation.
- Konermann et al. addressed the need in the art for versatile and robust technologies that enable optical and chemical modulation of DNA-binding domains based CRISPR Cas9 enzyme and also Transcriptional Activator Like Effectors

➢ As discussed in the present specification, the Cas9 nuclease from the microbial CRISPR-Cas system is targeted to specific genomic loci by a 20 nt guide sequence, which can tolerate certain mismatches to the DNA target and thereby promote undesired off-target mutagenesis. To address this, Ran et al. described an approach that combined a Cas9 nickase mutant with paired guide RNAs to introduce targeted double-strand breaks. Because individual nicks in the genome are repaired with high fidelity, simultaneous nicking via appropriately offset guide RNAs is required for double-stranded breaks and extends the number of specifically recognized bases for target cleavage. The authors demonstrated that using paired nicking can reduce off-target activity by 50- to 1,500-fold in cell lines and to facilitate gene knockout in mouse zygotes without sacrificing on-target cleavage efficiency. This versatile strategy enables a wide variety of genome editing applications that require high specificity.

➢ Hsu et al. characterized SpCas9 targeting specificity in human cells to inform the selection of target sites and avoid off-target effects. The study evaluated >700 guide RNA variants and SpCas9-induced indel mutation levels at >100 predicted genomic off-target loci in 293T and 293FT cells. The authors that SpCas9 tolerates mismatches between guide RNA and target DNA at different positions in a sequence-dependent manner, sensitive to the number, position and distribution of mismatches. The authors further showed that SpCas9-mediated cleavage is unaffected by DNA methylation and that the dosage of SpCas9 and sgRNA can be titrated to minimize off-target modification. Additionally, to facilitate mammalian genome engineering applications, the authors reported providing a web-based software tool to guide the selection and validation of target sequences as well as off-target analyses.

➢ Ran et al. described a set of tools for Cas9-mediated genome editing via non-homologous end joining (NHEJ) or homology-directed repair (HDR) in mammalian cells, as well as generation of modified cell lines for downstream functional studies. To minimize off-target cleavage, the authors further described a double-nicking strategy using the Cas9 nickase mutant with paired guide RNAs. The protocol provided by the authors experimentally derived guidelines for the selection of target sites, evaluation of cleavage efficiency and analysis of off-target activity. The studies showed that beginning with target design, gene modifications can be achieved within as little as 1-2 weeks, and modified clonal cell lines can be derived within 2-3 weeks.

➢ Shalem et al. described a new way to interrogate gene function on a genome-wide scale. Their studies showed that delivery of a genome-scale CRISPR-Cas9 knockout (GeCKO) library targeted 18,080 genes with 64,751 unique guide sequences enabled both negative and positive selection screening in human cells. First, the authors showed use of the GeCKO library to identify genes essential for cell viability in cancer and pluripotent stem cells. Next, in a melanoma model, the authors screened for genes whose loss is involved in resistance to vemurafenib, a therapeutic that inhibits mutant protein kinase BRAF. Their studies showed that the highest-ranking candidates included previously validated genes NF1 and MED12 as well as novel hits NF2, CUL3, TADA2B, and TADA1. The authors observed a high level of consistency between independent guide RNAs targeting the same gene and a high rate of hit confirmation, and thus demonstrated the promise of genome-scale screening with Cas9.

➢ Nishimasu et al. reported the crystal structure of Streptococcus pyogenes Cas9 in complex with sgRNA and its target DNA at 2.5 A° resolution. The structure revealed a bilobed architecture composed of target recognition and nuclease lobes, accommodating the sgRNA:DNA heteroduplex in a positively charged groove at their interface. Whereas the recognition lobe is essential for binding sgRNA and DNA, the nuclease lobe contains the HNH and RuvC nuclease domains, which are properly positioned for cleavage of the complementary and non-complementary strands of the target DNA, respectively. The nuclease lobe also contains a carboxyl-terminal domain responsible for the interaction with the protospacer adjacent motif (PAM). This high-resolution structure and accompanying functional analyses have revealed the molecular mechanism of RNA-guided DNA targeting by Cas9, thus paving the way for the rational design of new, versatile genome-editing technologies.

➢ Wu et al. mapped genome-wide binding sites of a catalytically inactive Cas9 (dCas9) from Streptococcus pyogenes loaded with single guide RNAs (sgRNAs) in mouse embryonic stem cells (mESCs). The authors showed that each of the four sgRNAs tested targets dCas9 to between tens and thousands of genomic sites, frequently characterized by a 5-nucleotide seed region in the sgRNA and an NGG protospacer adjacent motif (PAM). Chromatin inaccessibility decreases dCas9 binding to other sites with matching seed sequences; thus 70% of off-target sites are associated with genes. The authors showed that targeted sequencing of 295 dCas9 binding sites in mESCs transfected with catalytically active Cas9 identified only one site mutated above background levels. The authors proposed a two-state model for Cas9 binding and cleavage, in which a seed match triggers binding but extensive pairing with target DNA is required for cleavage.

➢ Hsu 2014 is a review article that discusses generally CRISPR-Cas9 history from yogurt to genome editing, including genetic screening of cells, that is in the information, data and findings of the applications in the lineage of this specification filed prior to June 5, 2014. The general teachings of Hsu 2014 do not involve the specific models, animals of the instant specification.

In general, the CRISPR-Cas or CRISPR system is as used in the foregoing documents, such as WO 2014/093622 (PCT/US2013/074667) and refers collectively to transcripts and other elements involved in the expression of or directing the activity of CRISPR-associated ("Cas") genes, including sequences encoding a Cas gene, a tracr (trans-activating CRISPR) sequence (e.g. tracrRNA or an active partial tracrRNA), a tracr-mate sequence (encompassing a "direct repeat" and a tracrRNA-processed partial direct repeat in the context of an endogenous CRISPR system), a guide sequence (also referred to as a "spacer" in the context of an endogenous CRISPR system), or "RNA(s)" as that term is herein used (e.g., RNA(s) to guide Cas9, e.g. CRISPR RNA and transactivating (tracr) RNA or a single guide RNA (sgRNA) (chimeric RNA)) or other sequences and transcripts from a CRISPR locus. In general, a CRISPR system is characterized by elements that promote the formation of a CRISPR complex at the site of a target sequence (also referred to as a protospacer in the context of an endogenous CRISPR system). In the context of formation of a CRISPR complex, "target sequence" refers to a sequence to which a guide sequence is designed to have complementarity, where hybridization between a target sequence and a guide sequence promotes the formation of a CRISPR complex. A target sequence may comprise any polynucleotide, such as DNA or RNA polynucleotides. In some embodiments, a target sequence is located in the nucleus or cytoplasm of a cell. In some embodiments, direct repeats may be identified *in silico* by searching for repetitive motifs that fulfill any or all of the following criteria: 1. found in a 2Kb window of genomic sequence flanking the type II CRISPR locus; 2. span from 20 to 50 bp; and 3. interspaced by 20 to 50 bp. In some embodiments, 2 of these criteria may be used, for instance 1 and 2, 2 and 3, or 1 and 3. In some embodiments, all 3 criteria may be used. In some embodiments it may be preferred in a CRISPR complex that the tracr sequence has one or more hairpins and is 30 or more nucleotides in length, 40 or more nucleotides in length, or 50 or more nucleotides in length; the guide sequence is between 10 to 30 nucleotides in length, the CRISPR/Cas enzyme is a Type II Cas9 enzyme. In embodiments of the invention the terms guide sequence and guide RNA are used interchangeably as in foregoing cited documents such as WO 2014/093622 (PCT/US2013/074667). In general, a guide sequence is any polynucleotide sequence having sufficient complementarity with a target polynucleotide sequence to hybridize with the target sequence and direct sequence-specific binding of a CRISPR complex to the target sequence. In some embodiments, the degree of complementarity between a guide sequence and its corresponding target sequence, when optimally aligned using a suitable alignment algorithm, is about or more than about 50%, 60%, 75%, 80%, 85%, 90%, 95%, 97.5%, 99%, or more. Optimal alignment may be determined with the use of any suitable algorithm for aligning sequences, non-limiting example of which include the Smith-Waterman algorithm, the Needleman-Wunsch algorithm, algorithms based on the Burrows-Wheeler Transform (e.g. the Burrows Wheeler Aligner), ClustalW, Clustal X, BLAT, Novoalign (Novocraft Technologies; available at www.novocraft.com), ELAND (Illumina, San Diego, CA), SOAP (available at soap.genomics.org.cn), and Maq (available at maq.sourceforge.net). In some embodiments, a guide sequence is about or more than about 5, 10, 11, 12, 13, 14, 15, 16, 17, 18, 19, 20, 21, 22, 23, 24, 25, 26, 27, 28, 29, 30, 35, 40, 45, 50, 75, or more nucleotides in length. In some embodiments, a guide sequence is less than about 75, 50, 45, 40, 35, 30, 25, 20, 15, 12, or fewer nucleotides in length. Preferably the guide sequence is 10 - 30 nucleotides long. The ability of a guide sequence to direct sequence-specific binding of a CRISPR complex to a target sequence may be assessed by any suitable assay. For example, the components of a CRISPR system sufficient to form a CRISPR complex, including the guide sequence to be tested, may be provided to a host cell having the corresponding target sequence, such as by transfection with vectors encoding the components of the CRISPR sequence, followed by an assessment of preferential cleavage within the target sequence, such as by Surveyor assay as described herein. Similarly, cleavage of a target polynucleotide sequence may be evaluated in a test tube by providing the target sequence, components of a CRISPR complex, including the guide sequence to be tested and a control guide sequence different from the test guide sequence, and comparing binding or rate of cleavage at the target sequence between the test and control guide sequence reactions. Other assays are possible, and will occur to those skilled in the art.A guide sequence may be selected to target any target sequence. In some embodiments, the target sequence is a sequence within a genome of a cell. Exemplary target sequences include those that are unique in the target genome. For example, for the S. pyogenes Cas9, a unique target sequence in a genome may include a Cas9 target site of the form MMMMMMMMNNNNNNNNNNNNXGG where NNNNNNNNNNNNXGG (N is A, G, T, or C; and X can be anything) has a single occurrence in the genome. A unique target sequence in a genome may include an S. pyogenes Cas9 target site of the form MMMMMMMMMNNNNNNNNNNNXGG where NNNNNNNNNNNXGG (N is A, G, T, or C; and X can be anything) has a single occurrence in the genome. For the S. thermophilus CRISPR1 Cas9, a unique target sequence in a genome may include a Cas9 target site of the form MMMMMMMMNNNNNNNNNNNNXXAGAAW where NNNNNNNNNNNNXXAGAAW (N is A, G, T, or C; X can be anything; and W is A or T) has a single occurrence in the genome. A unique target sequence in a genome may include an S. thermophilus CRISPR1 Cas9 target site of the form MMMMMMMMMNNNNNNNNNNNXXAGAAW where NNNNNNNNNNNXXAGAAW (N is A, G, T, or C; X can be anything; and W is A or T) has a single occurrence in the genome. For the S. pyogenes Cas9, a unique target sequence in a genome may include a Cas9 target site of the form MMMMMMMMNNNNNNNNNNNNXGGXG where NNNNNNNNNNNNXGGXG (N is A, G, T, or C; and X can be anything) has a single occurrence in the genome. A unique target sequence in a genome may include an S. pyogenes Cas9 target site of the form MMMMMMMMMNNNNNNNNNNNNXGGXG where NNNNNNNNNNNNXGGXG (N is A, G, T, or C; and X can be anything) has a single occurrence in the genome. In each of these sequences "M" may be A, G, T, or C, and need not be considered in identifying a sequence as unique. In some embodiments, a guide sequence is selected to reduce the degree secondary structure within the guide sequence. In some embodiments, about or less than about 75%, 50%, 40%, 30%, 25%, 20%, 15%, 10%, 5%, 1%, or fewer of the nucleotides of the guide sequence participate in self-complementary base pairing when optimally folded. Optimal folding may be determined by any suitable polynucleotide folding algorithm. Some programs are based on calculating the minimal Gibbs free energy. An example of one such algorithm is mFold, as described by Zuker and Stiegler (Nucleic Acids Res. 9 (1981), 133-148). Another example folding algorithm is the online webserver RNAfold, developed at Institute for Theoretical Chemistry at the University of Vienna, using the centroid structure prediction algorithm (see e.g. A.R. Gruber et al., 2008, Cell 106(1): 23-24; and PA Carr and GM Church, 2009, Nature Biotechnology 27(12): 1151-62).

In general, a tracr mate sequence includes any sequence that has sufficient complementarity with a tracr sequence to promote one or more of: (1) excision of a guide sequence flanked by tracr mate sequences in a cell containing the corresponding tracr sequence; and (2) formation of a CRISPR complex at a target sequence, wherein the CRISPR complex comprises the tracr mate sequence hybridized to the tracr sequence. In general, degree of complementarity is with reference to the optimal alignment of the tracr mate sequence and tracr sequence, along the length of the shorter of the two sequences. Optimal alignment may be determined by any suitable alignment algorithm, and may further account for secondary structures, such as self-complementarity within either the tracr sequence or tracr mate sequence. In some embodiments, the degree of complementarity between the tracr sequence and tracr mate sequence along the length of the shorter of the two when optimally aligned is about or more than about 25%, 30%, 40%, 50%, 60%, 70%, 80%, 90%, 95%, 97.5%, 99%, or higher. In some embodiments, the tracr sequence is about or more than about 5, 6, 7, 8, 9, 10, 11, 12, 13, 14, 15, 16, 17, 18, 19, 20, 25, 30, 40, 50, or more nucleotides in length. In some embodiments, the tracr sequence and tracr mate sequence are contained within a single transcript, such that hybridization between the two produces a transcript having a secondary structure, such as a hairpin. In an embodiment of the invention, the transcript or transcribed polynucleotide sequence has at least two or more hairpins. In preferred embodiments, the transcript has two, three, four or five hairpins. In a further embodiment of the invention, the transcript has at most five hairpins. In a hairpin structure the portion of the sequence 5' of the final "N" and upstream of the loop corresponds to the tracr mate sequence, and the portion of the sequence 3' of the loop corresponds to the tracr sequence Further non-limiting examples of single polynucleotides comprising a guide sequence, a tracr mate sequence, and a tracr sequence are as follows (listed 5' to 3'), where "N" represents a base of a guide sequence, the first block of lower case letters represent the tracr mate sequence, and the second block of lower case letters represent the tracr sequence, and the final poly-T sequence represents the transcription terminator: (1) NNNNNNNNNNNNNNNNNNNNgtttttgtactctcaagatttaGAAAtaaatcttgcagaagctacaaagataa ggcttcatgccgaaatcaacaccctgtcattttatggcagggtgttttcgttatttaaTTTTTT; (2) NNNNNNNNNNNNNNNNNNNNgtttttgtactctcaGAAAtgcagaagctacaaagataaggcttcatgccg aaatcaacaccctgtcattttatggcagggtgttttcgttatttaaTTTTTT; (3) NNNNNNNNNNNNNNNNNNNNgtttttgtactctcaGAAAtgcagaagctacaaagataaggcttcatgccg aaatcaacaccctgtcattttatggcagggtgtTTTTTT; (4) NNNNNNNNNNNNNNNNNNNNgttttagagctaGAAAtagcaagttaaaataaggctagtccgttatcaactt gaaaaagtggcaccgagtcggtgcTTTTTT; (5) NNNNNNNNNNNNNNNNNNNNgttttagagctaGAAATAGcaagttaaaataaggctagtccgttatcaac ttgaaaaagtgTTTTTTT; and (6) NNNNNNNNNNNNNNNNNNNNgttttagagctagAAATAGcaagttaaaataaggctagtccgttatcaTT TTTTTT. In some embodiments, sequences (1) to (3) are used in combination with Cas9 from S. *thermophilus* CRISPR1. In some embodiments, sequences (4) to (6) are used in combination with Cas9 from *S. pyogenes.* In some embodiments, the tracr sequence is a separate transcript from a transcript comprising the tracr mate sequence.

In some embodiments, candidate tracrRNA may be subsequently predicted by sequences that fulfill any or all of the following criteria: 1. sequence homology to direct repeats (motif search in Geneious with up to 18-bp mismatches); 2. presence of a predicted Rho-independent transcriptional terminator in direction of transcription; and 3. stable hairpin secondary structure between tracrRNA and direct repeat. In some embodiments, 2 of these criteria may be used, for instance 1 and 2, 2 and 3, or 1 and 3. In some embodiments, all 3 criteria may be used.

In some embodiments, chimeric synthetic guide RNAs (sgRNAs) designs may incorporate at least 12 bp of duplex structure between the direct repeat and tracrRNA.

For minimization of toxicity and off-target effect, it will be important to control the concentration of CRISPR enzyme mRNA and guide RNA delivered. Optimal concentrations of CRISPR enzyme mRNA and guide RNA can be determined by testing different concentrations in a cellular or non-human eukaryote animal model and using deep sequencing the analyze the extent of modification at potential off-target genomic loci. For example, for the guide sequence targeting 5'-GAGTCCGAGCAGAAGAAGAA-3' in the EMX1 gene of the human genome, deep sequencing can be used to assess the level of modification at the following two off-target loci, 1: 5'-GAGTCCTAGCAGGAGAAGAA-3' and 2: 5'-GAGTCTAAGCAGAAGAAGAA-3'. The concentration that gives the highest level of on-target modification while minimizing the level of off-target modification should be chosen for in vivo delivery. Alternatively, to minimize the level of toxicity and off-target effect, CRISPR enzyme nickase mRNA (for example S. pyogenes Cas9 with the D10A mutation) can be delivered with a pair of guide RNAs targeting a site of interest. The two guide RNAs need to be spaced as follows. Guide sequences and strategies to mimize toxicity and off-target effects can be as in WO 2014/093622 (PCT/US2013/074667).

The CRISPR system is derived advantageously from a type II CRISPR system. In some embodiments, one or more elements of a CRISPR system is derived from a particular organism comprising an endogenous CRISPR system, such as *Streptococcus pyogenes.* In preferred embodiments of the invention, the CRISPR system is a type II CRISPR system and the Cas enzyme is Cas9, which catalyzes DNA cleavage. Non-limiting examples of Cas proteins include Cas1, Cas1B, Cas2, Cas3, Cas4, Cas5, Cas6, Cas7, Cas8, Cas9 (also known as Csn1 and Csx12), Cas10, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, homologues thereof, or modified versions thereof.

In some embodiments, the unmodified CRISPR enzyme has DNA cleavage activity, such as Cas9. In some embodiments, the CRISPR enzyme directs cleavage of one or both strands at the location of a target sequence, such as within the target sequence and/or within the complement of the target sequence. In some embodiments, the CRISPR enzyme directs cleavage of one or both strands within about 1, 2, 3, 4, 5, 6, 7, 8, 9, 10, 15, 20, 25, 50, 100, 200, 500, or more base pairs from the first or last nucleotide of a target sequence. In some embodiments, a vector encodes a CRISPR enzyme that is mutated to with respect to a corresponding wild-type enzyme such that the mutated CRISPR enzyme lacks the ability to cleave one or both strands of a target polynucleotide containing a target sequence. For example, an aspartate-to-alanine substitution (D10A) in the RuvC I catalytic domain of Cas9 from S. pyogenes converts Cas9 from a nuclease that cleaves both strands to a nickase (cleaves a single strand). Other examples of mutations that render Cas9 a nickase include, without limitation, H840A, N854A, and N863A. As a further example, two or more catalytic domains of Cas9 (RuvC I, RuvC II, and RuvC III or the HNH domain) may be mutated to produce a mutated Cas9 substantially lacking all DNA cleavage activity. In some embodiments, a D10A mutation is combined with one or more of H840A, N854A, or N863A mutations to produce a Cas9 enzyme substantially lacking all DNA cleavage activity. In some embodiments, a CRISPR enzyme is considered to substantially lack all DNA cleavage activity when the DNA cleavage activity of the mutated enzyme is about no more than 25%, 10%, 5%, 1%, 0.1%, 0.01%, or less of the DNA cleavage activity of the non-mutated form of the enzyme; an example can be when the DNA cleavage activity of the mutated form is nil or negligible as compared with the non-mutated form. Where the enzyme is not SpCas9, mutations may be made at any or all residues corresponding to positions 10, 762, 840, 854, 863 and/or 986 of SpCas9 (which may be ascertained for instance by standard sequence comparison tools). In particular, any or all of the following mutations are preferred in SpCas9: D10A, E762A, H840A, N854A, N863A and/or D986A; as well as conservative substitution for any of the replacement amino acids is also envisaged. The same (or conservative substitutions of these mutations) at corresponding positions in other Cas9s are also preferred. Particularly preferred are D10 and H840 in SpCas9. However, in other Cas9s, residues corresponding to SpCas9 D10 and H840 are also preferred. Orthologs of SpCas9 can be used in the practice of the invention. A Cas enzyme may be identified Cas9 as this can refer to the general class of enzymes that share homology to the biggest nuclease with multiple nuclease domains from the type II CRISPR system. Most preferably, the Cas9 enzyme is from, or is derived from, spCas9 (S. *pyogenes* Cas9) or saCas9 (*S. aureus* Cas9). StCas9" refers to wild type Cas9 from S. thermophilus, the protein sequence of which is given in the SwissProt database under accession number G3ECR1. Similarly, S pyogenes Cas9 or spCas9 is included in SwissProt under accession number Q99ZW2. By derived, Applicants mean that the derived enzyme is largely based, in the sense of having a high degree of sequence homology with, a wildtype enzyme, but that it has been mutated (modified) in some way as described herein. It will be appreciated that the terms Cas and CRISPR enzyme are generally used herein interchangeably, unless otherwise apparent. As mentioned above, many of the residue numberings used herein refer to the Cas9 enzyme from the type II CRISPR locus in *Streptococcus pyogenes.* However, it will be appreciated that this invention includes many more Cas9s from other species of microbes, such as SpCas9, SaCa9, St1Cas9 and so forth. Enzymatic action by Cas9 derived from *Streptococcus pyogenes* or any closely related Cas9 generates double stranded breaks at target site sequences which hybridize to 20 nucleotides of the guide sequence and that have a protospacer-adjacent motif (PAM) sequence (examples include NGG/NRG or a PAM that can be determined as described herein) following the 20 nucleotides of the target sequence. CRISPR activity through Cas9 for site-specific DNA recognition and cleavage is defined by the guide sequence, the tracr sequence that hybridizes in part to the guide sequence and the PAM sequence. More aspects of the CRISPR system are described in Karginov and Hannon, The CRISPR system: small RNA-guided defence in bacteria and archaea, Mole Cell 2010, January 15; 37(1): 7. The type II CRISPR locus from *Streptococcus pyogenes* SF370, which contains a cluster of four genes Cas9, Cas1, Cas2, and Csn1, as well as two non-coding RNA elements, tracrRNA and a characteristic array of repetitive sequences (direct repeats) interspaced by short stretches of non-repetitive sequences (spacers, about 30bp each). In this system, targeted DNA double-strand break (DSB) is generated in four sequential steps. First, two non-coding RNAs, the pre-crRNA array and tracrRNA, are transcribed from the CRISPR locus. Second, tracrRNA hybridizes to the direct repeats of pre-crRNA, which is then processed into mature crRNAs containing individual spacer sequences. Third, the mature crRNA:tracrRNA complex directs Cas9 to the DNA target consisting of the protospacer and the corresponding PAM via heteroduplex formation between the spacer region of the crRNA and the protospacer DNA. Finally, Cas9 mediates cleavage of target DNA upstream of PAM to create a DSB within the protospacer. A pre-crRNA array consisting of a single spacer flanked by two direct repeats (DRs) is also encompassed by the term "tracr-mate sequences"). In certain embodiments, Cas9 may be constitutively present or inducibly present or conditionally present or administered or delivered. Cas9 optimization may be used to enhance function or to develop new functions, one can generate chimeric Cas9 proteins. And Cas9 may be used as a generic DNA binding protein.

Typically, in the context of an endogenous CRISPR system, formation of a CRISPR complex (comprising a guide sequence hybridized to a target sequence and complexed with one or more Cas proteins) results in cleavage of one or both strands in or near (e.g. within 1, 2, 3, 4, 5, 6, 7, 8, 9, 10, 20, 50, or more base pairs from) the target sequence. Without wishing to be bound by theory, the tracr sequence, which may comprise or consist of all or a portion of a wild-type tracr sequence (e.g. about or more than about 20, 26, 32, 45, 48, 54, 63, 67, 85, or more nucleotides of a wild-type tracr sequence), may also form part of a CRISPR complex, such as by hybridization along at least a portion of the tracr sequence to all or a portion of a tracr mate sequence that is operably linked to the guide sequence.

An example of a codon optimized sequence, is in this instance a sequence optimized for expression in a eukaryote, e.g., humans (i.e. being optimized for expression in humans), or for another eukaryote, animal or mammal as herein discussed; see, e.g., SaCas9 human codon optimized sequence in WO 2014/093622 (PCT/US2013/074667). Whilst this is preferred, it will be appreciated that other examples are possible and codon optimization for a host species other than human, or for codon optimization for specific organs is known. In some embodiments, an enzyme coding sequence encoding a CRISPR enzyme is codon optimized for expression in particular cells, such as eukaryotic cells. The eukaryotic cells may be those of or derived from a particular organism, such as a mammal, including but not limited to human, or non-human eukaryote or animal or mammal as herein discussed, e.g., mouse, rat, rabbit, dog, livestock, or non-human mammal or primate. In some embodiments, processes for modifying the germ line genetic identity of human beings and/or processes for modifying the genetic identity of animals which are likely to cause them suffering without any substantial medical benefit to man or animal, and also animals resulting from such processes, may be excluded. In general, codon optimization refers to a process of modifying a nucleic acid sequence for enhanced expression in the host cells of interest by replacing at least one codon (e.g. about or more than about 1, 2, 3, 4, 5, 10, 15, 20, 25, 50, or more codons) of the native sequence with codons that are more frequently or most frequently used in the genes of that host cell while maintaining the native amino acid sequence. Various species exhibit particular bias for certain codons of a particular amino acid. Codon bias (differences in codon usage between organisms) often correlates with the efficiency of translation of messenger RNA (mRNA), which is in turn believed to be dependent on, among other things, the properties of the codons being translated and the availability of particular transfer RNA (tRNA) molecules. The predominance of selected tRNAs in a cell is generally a reflection of the codons used most frequently in peptide synthesis. Accordingly, genes can be tailored for optimal gene expression in a given organism based on codon optimization. Codon usage tables are readily available, for example, at the "Codon Usage Database" available at www.kazusa.orjp/codon/ and these tables can be adapted in a number of ways. See Nakamura, Y., et al. "Codon usage tabulated from the international DNA sequence databases: status for the year 2000" Nucl. Acids Res. 28:292 (2000). Computer algorithms for codon optimizing a particular sequence for expression in a particular host cell are also available, such as Gene Forge (Aptagen; Jacobus, PA), are also available. In some embodiments, one or more codons (e.g. 1, 2, 3, 4, 5, 10, 15, 20, 25, 50, or more, or all codons) in a sequence encoding a CRISPR enzyme correspond to the most frequently used codon for a particular amino acid.

In some embodiments, a vector encodes a CRISPR enzyme comprising one or more nuclear localization sequences (NLSs), such as about or more than about 1, 2, 3, 4, 5, 6, 7, 8, 9, 10, or more NLSs. In some embodiments, the CRISPR enzyme comprises about or more than about 1, 2, 3, 4, 5, 6, 7, 8, 9, 10, or more NLSs at or near the amino-terminus, about or more than about 1, 2, 3, 4, 5, 6, 7, 8, 9, 10, or more NLSs at or near the carboxy-terminus, or a combination of these (e.g. zero or at least one or more NLS at the amino-terminus and zero or at one or more NLS at the carboxy terminus). When more than one NLS is present, each may be selected independently of the others, such that a single NLS may be present in more than one copy and/or in combination with one or more other NLSs present in one or more copies. In a preferred embodiment of the invention, the CRISPR enzyme comprises at most 6 NLSs. In some embodiments, an NLS is considered near the N- or C-terminus when the nearest amino acid of the NLS is within about 1, 2, 3, 4, 5, 10, 15, 20, 25, 30, 40, 50, or more amino acids along the polypeptide chain from the N- or C-terminus. Non-limiting examples of NLSs include an NLS sequence derived from: the NLS of the SV40 virus large T-antigen, having the amino acid sequence PKKKRKV; the NLS from nucleoplasmin (e.g. the nucleoplasmin bipartite NLS with the sequence KRPAATKKAGQAKKKK); the c-myc NLS having the amino acid sequence PAAKRVKLD or RQRRNELKRSP; the hRNPA1 M9 NLS having the sequence NQSSNFGPMKGGNFGGRSSGPYGGGGQYFAKPRNQGGY; the sequence RMRIZFKNKGKDTAELRRRRVEVSVELRKAKKDEQILKRRNV of the IBB domain from importin-alpha; the sequences VSRKRPRP and PPKKARED of the myoma T protein; the sequence POPKKKPL of human p53; the sequence SALIKKKKKMAP of mouse c-abl IV; the sequences DRLRR and PKQKKRK of the influenza virus NS1; the sequence RKLKKKIKKL of the Hepatitis virus delta antigen; the sequence REKKKFLKRR of the mouse Mx1 protein; the sequence KRKGDEVDGVDEVAKKKSKK of the human poly(ADP-ribose) polymerase; and the sequence RKCLQAGMNLEARKTKK of the steroid hormone receptors (human) glucocorticoid. In general, the one or more NLSs are of sufficient strength to drive accumulation of the CRISPR enzyme in a detectable amount in the nucleus of a eukaryotic cell. In general, strength of nuclear localization activity may derive from the number of NLSs in the CRISPR enzyme, the particular NLS(s) used, or a combination of these factors. Detection of accumulation in the nucleus may be performed by any suitable technique. For example, a detectable marker may be fused to the CRISPR enzyme, such that location within a cell may be visualized, such as in combination with a means for detecting the location of the nucleus (e.g. a stain specific for the nucleus such as DAPI). Cell nuclei may also be isolated from cells, the contents of which may then be analyzed by any suitable process for detecting protein, such as immunohistochemistry, Western blot, or enzyme activity assay. Accumulation in the nucleus may also be determined indirectly, such as by an assay for the effect of CRISPR complex formation (e.g. assay for DNA cleavage or mutation at the target sequence, or assay for altered gene expression activity affected by CRISPR complex formation and/or CRISPR enzyme activity), as compared to a control no exposed to the CRISPR enzyme or complex, or exposed to a CRISPR enzyme lacking the one or more NLSs.

Aspects of the invention relate to the expression of the gene product being decreased or a template polynucleotide being further introduced into the DNA molecule encoding the gene product or an intervening sequence being excised precisely by allowing the two 5' overhangs to reanneal and ligate or the activity or function of the gene product being altered or the expression of the gene product being increased. In an embodiment of the invention, the gene product is a protein. Only sgRNA pairs creating 5' overhangs with less than 8bp overlap between the guide sequences (offset greater than -8 bp) were able to mediate detectable indel formation. Importantly, each guide used in these assays is able to efficiently induce indels when paired with wildtype Cas9, indicating that the relative positions of the guide pairs are the most important parameters in predicting double nicking activity. Since Cas9n and Cas9H840A nick opposite strands of DNA, substitution of Cas9n with Cas9H840A with a given sgRNA pair should have resulted in the inversion of the overhang type; but no indel formation is observed as with Cas9H840A indicating that Cas9H840A is a CRISPR enzyme substantially lacking all DNA cleavage activity (which is when the DNA cleavage activity of the mutated enzyme is about no more than 25%, 10%, 5%, 1%, 0.1%, 0.01%, or less of the DNA cleavage activity of the non-mutated form of the enzyme; whereby an example can be when the DNA cleavage activity of the mutated form is nil or negligible as compared with the non-mutated form, e.g., when no indel formation is observed as with Cas9H840A in the eukaryotic system in contrast to the biochemical or prokaryotic systems). Nonetheless, a pair of sgRNAs that will generate a 5' overhang with Cas9n should in principle generate the corresponding 3' overhang instead, and double nicking. Therefore, sgRNA pairs that lead to the generation of a 3' overhang with Cas9n can be used with another mutated Cas9 to generate a 5' overhang, and double nicking. Accordingly, in some embodiments, a recombination template is also provided. A recombination template may be a component of another vector as described herein, contained in a separate vector, or provided as a separate polynucleotide. In some embodiments, a recombination template is designed to serve as a template in homologous recombination, such as within or near a target sequence nicked or cleaved by a CRISPR enzyme as a part of a CRISPR complex. A template polynucleotide may be of any suitable length, such as about or more than about 10, 15, 20, 25, 50, 75, 100, 150, 200, 500, 1000, or more nucleotides in length. In some embodiments, the template polynucleotide is complementary to a portion of a polynucleotide comprising the target sequence. When optimally aligned, a template polynucleotide might overlap with one or more nucleotides of a target sequences (e.g. about or more than about 1, 5, 10, 15, 20, or more nucleotides). In some embodiments, when a template sequence and a polynucleotide comprising a target sequence are optimally aligned, the nearest nucleotide of the template polynucleotide is within about 1, 5, 10, 15, 20, 25, 50, 75, 100, 200, 300, 400, 500, 1000, 5000, 10000, or more nucleotides from the target sequence.

In some embodiments, one or more vectors driving expression of one or more elements of a CRISPR system are introduced into a host cell such that expression of the elements of the CRISPR system direct formation of a CRISPR complex at one or more target sites. For example, a Cas enzyme, a guide sequence linked to a tracr-mate sequence, and a tracr sequence could each be operably linked to separate regulatory elements on separate vectors. Or, RNA(s) of the CRISPR System can be delivered to a transgenic Cas9 animal or mammal, e.g., an animal or mammal that constitutively or inducibly or conditionally expresses Cas9; or an animal or mammal that is otherwise expressing Cas9 or has cells containing Cas9, such as by way of prior administration thereto of a vector or vectors that code for and express in vivo Cas9. Alternatively, two or more of the elements expressed from the same or different regulatory elements, may be combined in a single vector, with one or more additional vectors providing any components of the CRISPR system not included in the first vector. CRISPR system elements that are combined in a single vector may be arranged in any suitable orientation, such as one element located 5' with respect to ("upstream" of) or 3' with respect to ("downstream" of) a second element. The coding sequence of one element may be located on the same or opposite strand of the coding sequence of a second element, and oriented in the same or opposite direction. In some embodiments, a single promoter drives expression of a transcript encoding a CRISPR enzyme and one or more of the guide sequence, tracr mate sequence (optionally operably linked to the guide sequence), and a tracr sequence embedded within one or more intron sequences (e.g. each in a different intron, two or more in at least one intron, or all in a single intron). In some embodiments, the CRISPR enzyme, guide sequence, tracr mate sequence, and tracr sequence are operably linked to and expressed from the same promoter. Delivery vehicles, vectors, particles, nanoparticles, formulations and components thereof for expression of one or more elements of a CRISPR system are as used in the foregoing documents, such as WO 2014/093622 (PCT/US2013/074667). In some embodiments, a vector comprises one or more insertion sites, such as a restriction endonuclease recognition sequence (also referred to as a "cloning site"). In some embodiments, one or more insertion sites (e.g. about or more than about 1, 2, 3, 4, 5, 6, 7, 8, 9, 10, or more insertion sites) are located upstream and/or downstream of one or more sequence elements of one or more vectors. In some embodiments, a vector comprises an insertion site upstream of a tracr mate sequence, and optionally downstream of a regulatory element operably linked to the tracr mate sequence, such that following insertion of a guide sequence into the insertion site and upon expression the guide sequence directs sequence-specific binding of a CRISPR complex to a target sequence in a eukaryotic cell. In some embodiments, a vector comprises two or more insertion sites, each insertion site being located between two tracr mate sequences so as to allow insertion of a guide sequence at each site. In such an arrangement, the two or more guide sequences may comprise two or more copies of a single guide sequence, two or more different guide sequences, or combinations of these. When multiple different guide sequences are used, a single expression construct may be used to target CRISPR activity to multiple different, corresponding target sequences within a cell. For example, a single vector may comprise about or more than about 1, 2, 3, 4, 5, 6, 7, 8, 9, 10, 15, 20, or more guide sequences. In some embodiments, about or more than about 1, 2, 3, 4, 5, 6, 7, 8, 9, 10, or more such guide-sequence-containing vectors may be provided, and optionally delivered to a cell. In some embodiments, a vector comprises a regulatory element operably linked to an enzyme-coding sequence encoding a CRISPR enzyme, such as a Cas protein. CRISPR enzyme or CRISPR enzyme mRNA or CRISPR guide RNA or RNA(s) can be delivered separately; and advantageously at least one of these is delivered via a nanoparticle complex. CRISPR enzyme mRNA can be delivered prior to the guide RNA to give time for CRISPR enzyme to be expressed. CRISPR enzyme mRNA might be administered 1-12 hours (preferably around 2-6 hours) prior to the administration of guide RNA. Alternatively, CRISPR enzyme mRNA and guide RNA can be administered together. Advantageously, a second booster dose of guide RNA can be administered 1-12 hours (preferably around 2-6 hours) after the initial administration of CRISPR enzyme mRNA + guide RNA. Additional administrations of CRISPR enzyme mRNA and/or guide RNA might be useful to achieve the most efficient levels of genome modification.

In one aspect, the invention provides methods for using one or more elements of a CRISPR system. The CRISPR complex of the invention provides an effective means for modifying a target polynucleotide. The CRISPR complex of the invention has a wide variety of utility including modifying (e.g., deleting, inserting, translocating, inactivating, activating) a target polynucleotide in a multiplicity of cell types. As such the CRISPR complex of the invention has a broad spectrum of applications in, e.g., gene therapy, drug screening, disease diagnosis, and prognosis. An exemplary CRISPR complex comprises a CRISPR enzyme complexed with a guide sequence hybridized to a target sequence within the target polynucleotide. The guide sequence is linked to a tracr mate sequence, which in turn hybridizes to a tracr sequence. In one embodiment, this invention provides a method of cleaving a target polynucleotide. The method comprises modifying a target polynucleotide using a CRISPR complex that binds to the target polynucleotide and effect cleavage of said target polynucleotide. Typically, the CRISPR complex of the invention, when introduced into a cell, creates a break (e.g., a single or a double strand break) in the genome sequence. For example, the method can be used to cleave a disease gene in a cell. The break created by the CRISPR complex can be repaired by a repair processes such as the error prone non-homologous end joining (NHEJ) pathway or the high fidelity homology-directed repair (HDR). During these repair process, an exogenous polynucleotide template can be introduced into the genome sequence. In some methods, the HDR process is used modify genome sequence. For example, an exogenous polynucleotide template comprising a sequence to be integrated flanked by an upstream sequence and a downstream sequence is introduced into a cell. The upstream and downstream sequences share sequence similarity with either side of the site of integration in the chromosome. Where desired, a donor polynucleotide can be DNA, e.g., a DNA plasmid, a bacterial artificial chromosome (BAC), a yeast artificial chromosome (YAC), a viral vector, a linear piece of DNA, a PCR fragment, a naked nucleic acid, or a nucleic acid complexed with a delivery vehicle such as a liposome or poloxamer. The exogenous polynucleotide template comprises a sequence to be integrated (e.g., a mutated gene). The sequence for integration may be a sequence endogenous or exogenous to the cell. Examples of a sequence to be integrated include polynucleotides encoding a protein or a non-coding RNA (e.g., a microRNA). Thus, the sequence for integration may be operably linked to an appropriate control sequence or sequences. Alternatively, the sequence to be integrated may provide a regulatory function. The upstream and downstream sequences in the exogenous polynucleotide template are selected to promote recombination between the chromosomal sequence of interest and the donor polynucleotide. The upstream sequence is a nucleic acid sequence that shares sequence similarity with the genome sequence upstream of the targeted site for integration. Similarly, the downstream sequence is a nucleic acid sequence that shares sequence similarity with the chromosomal sequence downstream of the targeted site of integration. The upstream and downstream sequences in the exogenous polynucleotide template can have 75%, 80%, 85%, 90%, 95%, or 100% sequence identity with the targeted genome sequence. Preferably, the upstream and downstream sequences in the exogenous polynucleotide template have about 95%, 96%, 97%, 98%, 99%, or 100% sequence identity with the targeted genome sequence. In some methods, the upstream and downstream sequences in the exogenous polynucleotide template have about 99% or 100% sequence identity with the targeted genome sequence. An upstream or downstream sequence may comprise from about 20 bp to about 2500 bp, for example, about 50, 100, 200, 300, 400, 500, 600, 700, 800, 900, 1000, 1100, 1200, 1300, 1400, 1500, 1600, 1700, 1800, 1900, 2000, 2100, 2200, 2300, 2400, or 2500 bp. In some methods, the exemplary upstream or downstream sequence have about 200 bp to about 2000 bp, about 600 bp to about 1000 bp, or more particularly about 700 bp to about 1000 bp. In some methods, the exogenous polynucleotide template may further comprise a marker. Such a marker may make it easy to screen for targeted integrations. Examples of suitable markers include restriction sites, fluorescent proteins, or selectable markers. The exogenous polynucleotide template of the invention can be constructed using recombinant techniques (see, for example, Sambrook et al., 2001 and Ausubel et al., 1996). In a method for modifying a target polynucleotide by integrating an exogenous polynucleotide template, a double stranded break is introduced into the genome sequence by the CRISPR complex, the break is repaired via homologous recombination an exogenous polynucleotide template such that the template is integrated into the genome. The presence of a double-stranded break facilitates integration of the template. In other embodiments, this invention provides a method of modifying expression of a polynucleotide in a eukaryotic cell. The method comprises increasing or decreasing expression of a target polynucleotide by using a CRISPR complex that binds to the polynucleotide. In some methods, a target polynucleotide can be inactivated to effect the modification of the expression in a cell. For example, upon the binding of a CRISPR complex to a target sequence in a cell, the target polynucleotide is inactivated such that the sequence is not transcribed, the coded protein is not produced, or the sequence does not function as the wild-type sequence does. For example, a protein or microRNA coding sequence may be inactivated such that the protein or microRNA or pre-microRNA transcript is not produced. In some methods, a control sequence can be inactivated such that it no longer functions as a control sequence. As used herein, "control sequence" refers to any nucleic acid sequence that effects the transcription, translation, or accessibility of a nucleic acid sequence. Examples of a control sequence include, a promoter, a transcription terminator, and an enhancer are control sequences. The target polynucleotide of a CRISPR complex can be any polynucleotide endogenous or exogenous to the eukaryotic cell. For example, the target polynucleotide can be a polynucleotide residing in the nucleus of the eukaryotic cell. The target polynucleotide can be a sequence coding a gene product (e.g., a protein) or a non-coding sequence (e.g., a regulatory polynucleotide or a junk DNA). Examples of target polynucleotides include a sequence associated with a signaling biochemical pathway, e.g., a signaling biochemical pathway-associated gene or polynucleotide. Examples of target polynucleotides include a disease associated gene or polynucleotide. A "disease-associated" gene or polynucleotide refers to any gene or polynucleotide which is yielding transcription or translation products at an abnormal level or in an abnormal form in cells derived from a disease-affected tissues compared with tissues or cells of a non disease control. It may be a gene that becomes expressed at an abnormally high level; it may be a gene that becomes expressed at an abnormally low level, where the altered expression correlates with the occurrence and/or progression of the disease. A disease-associated gene also refers to a gene possessing mutation(s) or genetic variation that is directly responsible or is in linkage disequilibrium with a gene(s) that is responsible for the etiology of a disease. The transcribed or translated products may be known or unknown, and may be at a normal or abnormal level. The target polynucleotide of a CRISPR complex can be any polynucleotide endogenous or exogenous to the eukaryotic cell. For example, the target polynucleotide can be a polynucleotide residing in the nucleus of the eukaryotic cell. The target polynucleotide can be a sequence coding a gene product (e.g., a protein) or a non-coding sequence (e.g., a regulatory polynucleotide or a junk DNA). Without wishing to be bound by theory, it is believed that the target sequence should be associated with a PAM (protospacer adjacent motif); that is, a short sequence recognized by the CRISPR complex. The precise sequence and length requirements for the PAM differ depending on the CRISPR enzyme used, but PAMs are typically 2-5 base pair sequences adjacent the protospacer (that is, the target sequence) Examples of PAM sequences are given in the examples section below, and the skilled person will be able to identify further PAM sequences for use with a given CRISPR enzyme. In some embodiments, the method comprises allowing a CRISPR complex to bind to the target polynucleotide to effect cleavage of said target polynucleotide thereby modifying the target polynucleotide, wherein the CRISPR complex comprises a CRISPR enzyme complexed with a guide sequence hybridized to a target sequence within said target polynucleotide, wherein said guide sequence is linked to a tracr mate sequence which in turn hybridizes to a tracr sequence. In one aspect, the invention provides a method of modifying expression of a polynucleotide in a eukaryotic cell. In some embodiments, the method comprises allowing a CRISPR complex to bind to the polynucleotide such that said binding results in increased or decreased expression of said polynucleotide; wherein the CRISPR complex comprises a CRISPR enzyme complexed with a guide sequence hybridized to a target sequence within said polynucleotide, wherein said guide sequence is linked to a tracr mate sequence which in turn hybridizes to a tracr sequence. Similar considerations and conditions apply as above for methods of modifying a target polynucleotide. In fact, these sampling, culturing and re-introduction options apply across the aspects of the present invention. In one aspect, the invention provides for methods of modifying a target polynucleotide in a eukaryotic cell, which may be *in vivo, ex vivo* or *in vitro.* In some embodiments, the method comprises sampling a cell or population of cells from a human or non-human animal, and modifying the cell or cells. Culturing may occur at any stage *ex vivo.* The cell or cells may even be re-introduced into the non-human animal or plant. For re-introduced cells it is particularly preferred that the cells are stem cells.

Indeed, in any aspect of the invention, the CRISPR complex may comprise a CRISPR enzyme complexed with a guide sequence hybridized to a target sequence, wherein said guide sequence may be linked to a tracr mate sequence which in turn may hybridize to a tracr sequence.

This invention relates to the engineering and optimization of systems, methods and compositions used for the control of gene expression involving sequence targeting, such as genome perturbation or gene-editing, that relate to the CRISPR-Cas system and components thereof as delivered by nanoparticle carrier. An advantage of the present methods is that the delivery of the CRISPR system can be directed to minimize off-target delivery and its resulting side effects. This is achieved using delivery particle formulations and/or systems, preferably, nanoparticle systems.

Cas9

Cas9 optimization may be used to enhance function or to develop new functions, one can generate chimeric Cas9 proteins. Examples that the Applicants have generated are provided in Example 4. Chimeric Cas9 proteins can be made by combining fragments from different Cas9 homologs. For example, two example chimeric Cas9 proteins from the Cas9s described herein. For example, Applicants fused the N-term of St1Cas9 (fragment from this protein is in bold) with C-term of SpCas9. The benefit of making chimeric Cas9s include any or all of: reduced toxicity; improved expression in eukaryotic cells; enhanced specificity; reduced molecular weight of protein, for example, making the protein smaller by combining the smallest domains from different Cas9 homologs; and/or altering the PAM sequence requirement.

The Cas9 may be used as a generic DNA binding protein. For example, and as shown in Example 5, Applicants used Cas9 as a generic DNA binding protein by mutating the two catalytic domains (D10 and H840) responsible for cleaving both strands of the DNA target. In order to upregulate gene transcription at a target locus Applicants fused a transcriptional activation domain (VP64) to Cas9. Other transcriptional activation domains are known. As shown in Example 15, transcriptional activation is possible. As also shown in Example 15, gene repression (in this case of the beta-catenin gene) is possible using a Cas9 repressor (DNA-binding domain) that binds to the target gene sequence, thus repressing its activity.

Cas9 and one or more guide RNA can be delivered using adeno associated virus (AAV), lentivirus, adenovirus or other plasmid or viral vector types, in particular, using formulations and doses from, for example, US Patents Nos. 8,454,972 (formulations, doses for adenovirus), 8,404,658 (formulations, doses for AAV) and 5,846,946 (formulations, doses for DNA plasmids) and from clinical trials and publications regarding the clinical trials involving lentivirus, AAV and adenovirus. For examples, for AAV, the route of administration, formulation and dose can be as in US Patent No. 8,454,972 and as in clinical trials involving AAV. For Adenovirus, the route of administration, formulation and dose can be as in US Patent No. 8,404,658 and as in clinical trials involving adenovirus. For plasmid delivery, the route of administration, formulation and dose can be as in US Patent No 5,846,946 and as in clinical studies involving plasmids. Doses may be based on or extrapolated to an average 70 kg individual, and can be adjusted for patients, subjects, mammals of different weight and species. Frequency of administration is within the ambit of the medical or veterinary practitioner (e.g., physician, veterinarian), depending on usual factors including the age, sex, general health, other conditions of the patient or subject and the particular condition or symptoms being addressed.

The viral vectors can be injected into the tissue of interest. For cell-type specific genome modification, the expression of Cas9 can be driven by a cell-type specific promoter. For example, liver-specific expression might use the Albumin promoter and neuron-specific expression might use the Synapsin I promoter.

Transgenic animals and plants

Transgenic animals are also provided. Preferred examples include animals comprising Cas9, in terms of polynucleotides encoding Cas9 or the protein itself. Mice, rats and rabbits are preferred. To generate transgenic mice with the constructs, as exemplified herein one may inject pure, linear DNA into the pronucleus of a zygote from a pseudo pregnant female, e.g. a CB56 female. Founders may then be identified, genotyped, and backcrossed to CB57 mice. The constructs may then be cloned and optionally verified, for instance by Sanger sequencing. Knock outs are envisaged where for instance one or more genes are knocked out in a model. However, are knockins are also envisaged (alone or in combination). An example knockin Cas9 mouse was generated and this is exemplified, but Cas9 knockins are preferred. To generate a Cas9 knock in mice one may target the same constitutive and conditional constructs to the Rosa26 locus, as described herein (Figs. 22A-B and 23). Methods of US Patent Publication Nos. 20120017290 and 20110265198 assigned to Sangamo BioSciences, Inc. directed to targeting the Rosa locus may be modified to utilize the CRISPR Cas system of the present invention. In another embodiment, the methods of US Patent Publication No. 20130236946 assigned to Cellectis directed to targeting the Rosa locus may also be modified to utilize the CRISPR Cas system of the present invention.

Utility of the conditional Cas9 mouse: Applicants have shown in 293 cells that the Cas9 conditional expression construct can be activated by co-expression with Cre. Applicants also show that the correctly targeted R1 mESCs can have active Cas9 when Cre is expressed. Because Cas9 is followed by the P2A peptide cleavage sequence and then EGFP Applicants identify successful expression by observing EGFP. Applicants have shown Cas9 activation in mESCs. This same concept is what makes the conditional Cas9 mouse so useful. A cross of the conditional Cas9 mouse with a mouse that ubiquitously expresses Cre (ACTB-Cre line) may arrive at a mouse that expresses Cas9 in every cell. Delivery of chimeric RNA induces genome editing in embryonic or adult mice. Interestingly, if the conditional Cas9 mouse is crossed with a mouse expressing Cre under a tissue specific promoter, there is only be Cas9 in the tissues that also express Cre. This approach may be used to edit the genome in only precise tissues by delivering chimeric RNA to the same tissue.

As mentioned above, transgenic animals are also provided, as are transgenic plants, especially crops and algae. The transgenic plants may be useful in applications outside of providing a disease model. These may include food or feed production through expression of, for instance, higher protein, carbohydrate, nutrient or vitamin levels than would normally be seen in the wildtype. In this regard, transgenic plants, especially pulses and tubers, and animals, especially mammals such as livestock (cows, sheep, goats and pigs), but also poultry and edible insects, are preferred. Transgenic algae or other plants such as rape may be particularly useful in the production of vegetable oils or biofuels such as alcohols (especially methanol and ethanol), for instance. These may be engineered to express or overexpress high levels of oil or alcohols for use in the oil or biofuel industries.

Particle delivery systems and/or formulations:

Several types of particle delivery systems and/or formulations are known to be useful in a diverse spectrum of biomedical applications. In general, a particle is defined as a small object that behaves as a whole unit with respect to its transport and properties. Particles are further classified according to diameter Coarse particles cover a range between 2,500 and 10,000 nanometers. Fine particles are sized between 100 and 2,500 nanometers. Ultrafine particles, or nanoparticles, are generally between 1 and 100 nanometers in size. The basis of the 100-nm limit is the fact that novel properties that differentiate particles from the bulk material typically develop at a critical length scale of under 100 nm.

As used herein, a particle delivery system/formulation is defined as any biological delivery system/formulation which includes a particle in accordance with the present invention. A particle in accordance with the present invention is any entity having a greatest dimension (e.g. diameter) of less than 100 microns (μm). In some embodiments, inventive particles have a greatest dimension of less than 10 μm. In some embodiments, inventive particles have a greatest dimension of less than 2000 nanometers (nm). In some embodiments, inventive particles have a greatest dimension of less than 1000 nanometers (nm). In some embodiments, inventive particles have a greatest dimension of less than 900 nm, 800 nm, 700 nm, 600 nm, 500 nm, 400 nm, 300 nm, 200 nm, or 100 nm. Typically, inventive particles have a greatest dimension (e.g., diameter) of 500 nm or less. In some embodiments, inventive particles have a greatest dimension (e.g., diameter) of 250 nm or less. In some embodiments, inventive particles have a greatest dimension (e.g., diameter) of 200 nm or less. In some embodiments, inventive particles have a greatest dimension (e.g., diameter) of 150 nm or less. In some embodiments, inventive particles have a greatest dimension (e.g., diameter) of 100 nm or less. Smaller particles, e.g., having a greatest dimension of 50 nm or less are used in some embodiments of the invention. In some embodiments, inventive particles have a greatest dimension ranging between 25 nm and 200 nm.

Particle characterization (including e.g., characterizing morphology, dimension, etc.) is done using a variety of different techniques. Common techniques are electron microscopy (TEM, SEM), atomic force microscopy (AFM), dynamic light scattering (DLS), X-ray photoelectron spectroscopy (XPS), powder X-ray diffraction (XRD), Fourier transform infrared spectroscopy (FTIR), matrix-assisted laser desorption/ionization time-of-flight mass spectrometry(MALDI-TOF), ultraviolet-visible spectroscopy, dual polarisation interferometry and nuclear magnetic resonance (NMR). Characterization (dimension measurements) may be made as to native particles (i.e., preloading) or after loading of the cargo (herein cargo refers to e.g., one or more components of CRISPR-Cas system e.g., CRISPR enzyme or mRNA or guide RNA, or any combination thereof, and may include additional carriers and/or excipients) to provide particles of an optimal size for delivery for any *in vitro, ex vivo* and/or *in vivo* application of the present invention. In certain preferred embodiments, particle dimension (e.g., diameter) characterization is based on measurements using dynamic laser scattering (DLS).

Particles delivery systems within the scope of the present invention may be provided in any form, including but not limited to solid, semi-solid, emulsion, or colloidal particles. As such any of the delivery systems described herein, including but not limited to, e.g., lipid-based systems, liposomes, micelles, microvesicles, exosomes, or gene gun may be provided as particle delivery systems within the scope of the present invention.

Nanoparticles

In terms of this invention, it is preferred to have one or more components of CRISPR complex, e.g., CRISPR enzyme or mRNA or guide RNA delivered using nanoparticles or lipid envelopes. Discussion herein as to other delivery systems or vectors are because such may be used in conjunction with the nanoparticle aspects of the invention.

In general, a "nanoparticle" refers to any particle having a diameter of less than 1000 nm. In certain preferred embodiments, nanoparticles of the invention have a greatest dimension (e.g., diameter) of 500 nm or less. In other preferred embodiments, nanoparticles of the invention have a greatest dimension ranging between 25 nm and 200 nm. In other preferred embodiments, nanoparticles of the invention have a greatest dimension of 100 nm or less. In other preferred embodiments, nanoparticles of the invention have a greatest dimension ranging between 35 nm and 60 nm.

Nanoparticles encompassed in the present invention may be provided in different forms, e.g., as solid nanoparticles (e.g., metal such as silver, gold, iron, titanium), non-metal, lipid-based solids, polymers), suspensions of nanoparticles, or combinations thereof. Metal, dielectric, and semiconductor nanoparticles may be prepared, as well as hybrid structures (e.g., core-shell nanoparticles). Nanoparticles made of semiconducting material may also be labeled quantum dots if they are small enough (typically sub 10 nm) that quantization of electronic energy levels occurs. Such nanoscale particles are used in biomedical applications as drug carriers or imaging agents and may be adapted for similar purposes in the present invention.

Semi-solid and soft nanoparticles have been manufactured, and are within the scope of the present invention. A prototype nanoparticle of semi-solid nature is the liposome. Various types of liposome nanoparticles are currently used clinically as delivery systems for anticancer drugs and vaccines. Nanoparticles with one half hydrophilic and the other half hydrophobic are termed Janus particles and are particularly effective for stabilizing emulsions. They can self-assemble at water/oil interfaces and act as solid surfactants.

For example, Su X, Fricke J, Kavanagh DG, Irvine DJ ("In vitro and in vivo mRNA delivery using lipid-enveloped pH-responsive polymer nanoparticles" Mol Pharm. 2011 Jun 6;8(3):774-87. doi: 10.1021/mp100390w. Epub 2011 Apr 1) describes biodegradable core-shell structured nanoparticles with a poly(β-amino ester) (PBAE) core enveloped by a phospholipid bilayer shell. These were developed for in vivo mRNA delivery. The pH-responsive PBAE component was chosen to promote endosome disruption, while the lipid surface layer was selected to minimize toxicity of the polycation core. Such are, therefore, preferred for delivering RNA of the present invention.

In one embodiment, nanoparticles based on self assembling bioadhesive polymers are contemplated, which may be applied to oral delivery of peptides, intravenous delivery of peptides and nasal delivery of peptides, all to the brain. Other embodiments, such as oral absorption and ocular deliver of hydrophobic drugs are also contemplated. The molecular envelope technology involves an engineered polymer envelope which is protected and delivered to the site of the disease (see, e.g., Mazza, M. et al. ACSNano, 2013. 7(2): 1016-1026; Siew, A., et al. Mol Pharm, 2012. 9(1):14-28; Lalatsa, A., et al. J Contr Rel, 2012. 161(2):523-36; Lalatsa, A., et al., Mol Pharm, 2012. 9(6):1665-80; Lalatsa, A., et al. Mol Pharm, 2012. 9(6):1764-74; Garrett, N.L., et al. J Biophotonics, 2012. 5(5-6):458-68; Garrett, N.L., et al. J Raman Spect, 2012. 43(5):681-688; Ahmad, S., et al. J Royal Soc Interface 2010. 7:S423-33; Uchegbu, I.F. Expert Opin Drug Deliv, 2006. 3(5):629-40; Qu, X.,et al. Biomacromolecules, 2006. 7(12):3452-9 and Uchegbu, I.F., et al. Int J Pharm, 2001. 224:185-199). Doses of about 5 mg/kg are contemplated, with single or multiple doses, depending on the target tissue. It is mentioned herein experiments involving mice involve 20g mammals and that dosing can be scaled up to a 70 kg human.

In one embodiment, nanoparticles that can deliver RNA to a cancer cell to stop tumor growth developed by Dan Anderson's lab at MIT may be used and/or adapted to the CRISPR Cas system of the present invention. In particular, the Anderson lab developed fully automated, combinatorial systems for the synthesis, purification, characterization, and formulation of new biomaterials and nanoformulations. See, e.g., Alabi et al., Proc Natl Acad Sci U S A. 2013 Aug 6;110(32):12881-6; Zhang et al., Adv Mater. 2013 Sep 6;25(33):4641-5; Jiang et al., Nano Lett. 2013 Mar 13;13(3):1059-64; Karagiannis et al., ACS Nano. 2012 Oct 23;6(10):8484-7; Whitehead et al., ACS Nano. 2012 Aug 28;6(8):6922-9 and Lee et al., Nat Nanotechnol. 2012 Jun 3;7(6):389-93.

US patent application 20110293703 relates to lipidoid compounds are also particularly useful in the administration of polynucleotides, which may be applied to deliver the CRISPR Cas system of the present invention. In one aspect, the aminoalcohol lipidoid compounds are combined with an agent to be delivered to a cell or a subject to form nanoparticles. The agent to be delivered by the particles may be in the form of a gas, liquid, or solid, and the agent may be a polynucleotide, protein, peptide, or small molecule. The minoalcohol lipidoid compounds may be combined with other aminoalcohol lipidoid compounds, polymers (synthetic or natural), surfactants, cholesterol, carbohydrates, proteins, lipids, etc. to form the particles. These particles may then optionally be combined with a pharmaceutical excipient to form a pharmaceutical composition.

US Patent Publication No. 0110293703 also provides methods of preparing the aminoalcohol lipidoid compounds. One or more equivalents of an amine are allowed to react with one or more equivalents of an epoxide-terminated compound under suitable conditions to form an aminoalcohol lipidoid compound of the present invention. In certain embodiments, all the amino groups of the amine are fully reacted with the epoxide-terminated compound to form tertiary amines. In other embodiments, all the amino groups of the amine are not fully reacted with the epoxide-terminated compound to form tertiary amines thereby resulting in primary or secondary amines in the aminoalcohol lipidoid compound. These primary or secondary amines are left as is or may be reacted with another electrophile such as a different epoxide-terminated compound. As will be appreciated by one skilled in the art, reacting an amine with less than excess of epoxide-terminated compound will result in a plurality of different aminoalcohol lipidoid compounds with various numbers of tails. Certain amines may be fully functionalized with two epoxide-derived compound tails while other molecules will not be completely functionalized with epoxide-derived compound tails. For example, a diamine or polyamine may include one, two, three, or four epoxide-derived compound tails off the various amino moieties of the molecule resulting in primary, secondary, and tertiary amines. In certain embodiments, all the amino groups are not fully functionalized. In certain embodiments, two of the same types of epoxide-terminated compounds are used. In other embodiments, two or more different epoxide-terminated compounds are used. The synthesis of the aminoalcohol lipidoid compounds is performed with or without solvent, and the synthesis may be performed at higher temperatures ranging from 30.-100 C., preferably at approximately 50.-90 C. The prepared aminoalcohol lipidoid compounds may be optionally purified. For example, the mixture of aminoalcohol lipidoid compounds may be purified to yield an aminoalcohol lipidoid compound with a particular number of epoxide-derived compound tails. Or the mixture may be purified to yield a particular stereo- or regioisomer. The aminoalcohol lipidoid compounds may also be alkylated using an alkyl halide (e.g., methyl iodide) or other alkylating agent, and/or they may be acylated.

US Patent Publication No. 0110293703 also provides libraries of aminoalcohol lipidoid compounds prepared by the inventive methods. These aminoalcohol lipidoid compounds may be prepared and/or screened using high-throughput techniques involving liquid handlers, robots, microtiter plates, computers, etc. In certain embodiments, the aminoalcohol lipidoid compounds are screened for their ability to transfect polynucleotides or other agents (e.g., proteins, peptides, small molecules) into the cell.

US Patent Publication No. 20130302401 relates to a class of poly(beta-amino alcohols) (PBAAs) has been prepared using combinatorial polymerization. The inventive PBAAs may be used in biotechnology and biomedical applications as coatings (such as coatings of films or multilayer films for medical devices or implants), additives, materials, excipients, non-biofouling agents, micropatterning agents, and cellular encapsulation agents. When used as surface coatings, these PBAAs elicited different levels of inflammation, both in vitro and in vivo, depending on their chemical structures. The large chemical diversity of this class of materials allowed us to identify polymer coatings that inhibit macrophage activation in vitro. Furthermore, these coatings reduce the recruitment of inflammatory cells, and reduce fibrosis, following the subcutaneous implantation of carboxylated polystyrene microparticles. These polymers may be used to form polyelectrolyte complex capsules for cell encapsulation. The invention may also have many other biological applications such as antimicrobial coatings, DNA or siRNA delivery, and stem cell tissue engineering. The teachings of US Patent Publication No. 20130302401 may be applied to the CRISPR Cas system of the present invention.

In another embodiment, lipid nanoparticles (LNPs) are contemplated. In particular, an antitransthyretin small interfering RNA encapsulated in lipid nanoparticles (see, e.g., Coelho et al., N Engl J Med 2013;369:819-29) may be applied to the CRISPR Cas system of the present invention. Doses of about 0.01 to about 1 mg per kg of body weight administered intravenously are contemplated. Medications to reduce the risk of infusion-related reactions are contemplated, such as dexamethasone, acetampinophen, diphenhydramine or cetirizine, and ranitidine are contemplated. Multiple doses of about 0.3 mg per kilogram every 4 weeks for five doses are also contemplated.

LNPs have been shown to be highly effective in delivering siRNAs to the liver (see, e.g., Tabernero et al., Cancer Discovery, April 2013, Vol. 3, No. 4, pages 363-470) and are therefore contemplated for delivering CRISPR Cas to the liver. A dosage of about four doses of 6 mg/kg of the LNP every two weeks may be contemplated. Tabernero et al. demonstrated that tumor regression was observed after the first 2 cycles of LNPs dosed at 0.7 mg/kg, and by the end of 6 cycles the patient had achieved a partial response with complete regression of the lymph node metastasis and substantial shrinkage of the liver tumors. A complete response was obtained after 40 doses in this patient, who has remained in remission and completed treatment after receiving doses over 26 months. Two patients with RCC and extrahepatic sites of disease including kidney, lung, and lymph nodes that were progressing following prior therapy with VEGF pathway inhibitors had stable disease at all sites for approximately 8 to 12 months, and a patient with PNET and liver metastases continued on the extension study for 18 months (36 doses) with stable disease.

However, the charge of the LNP must be taken into consideration. As cationic lipids combined with negatively charged lipids to induce nonbilayer structures that facilitate intracellular delivery. Because charged LNPs are rapidly cleared from circulation following intravenous injection, ionizable cationic lipids with pKa values below 7 were developed (see, e.g., Rosin et al, Molecular Therapy, vol. 19, no. 12, pages 1286-2200, Dec. 2011). Negatively charged polymers such as siRNA oligonucleotides may be loaded into LNPs at low pH values (e.g., pH 4) where the ionizable lipids display a positive charge. However, at physiological pH values, the LNPs exhibit a low surface charge compatible with longer circulation times. Four species of ionizable cationic lipids have been focused upon, namely 1,2-dilineoyl-3-dimethylammonium-propane (DLinDAP), 1,2-dilinoleyloxy-3-N,N-dimethylaminopropane (DLinDMA), 1,2-dilinoleyloxy-keto-N,N-dimethyl-3-aminopropane (DLinKDMA), and 1,2-dilinoleyl-4-(2-dimethylaminoethyl)-[1,3]-dioxolane (DLinKC2-DMA). It has been shown that LNP siRNA systems containing these lipids exhibit remarkably different gene silencing properties in hepatocytes in vivo, with potencies varying according to the series DLinKC2-DMA>DLinKDMA>DLinDMA>>DLinDAP employing a Factor VII gene silencing model (see, e.g., Rosin et al, Molecular Therapy, vol. 19, no. 12, pages 1286-2200, Dec. 2011). A dosage of 1 μg/ml levels may be contemplated, especially for a formulation containing DLinKC2-DMA.

Preparation of LNPs and CRISPR Cas encapsulation may be used/and or adapted from Rosin et al, Molecular Therapy, vol. 19, no. 12, pages 1286-2200, Dec. 2011). The cationic lipids 1,2-dilineoyl-3-dimethylammonium-propane (DLinDAP), 1,2-dilinoleyloxy-3-N,N-dimethylaminopropane (DLinDMA), 1,2-dilinoleyloxyketo-N,N-dimethyl-3-aminopropane (DLinK-DMA), 1,2-dilinoleyl-4-(2-dimethylaminoethyl)-[1,3]-dioxolane (DLinKC2-DMA), (3-o-[2"-(methoxypolyethyleneglycol 2000) succinoyl]-1,2-dimyristoyl-sn-glycol (PEG-S-DMG), and R-3-[(co-methoxy-poly(ethylene glycol)2000) carbamoyl]-1,2-dimyristyloxlpropyl-3-amine (PEG-C-DOMG) may be provided by Tekmira Pharmaceuticals (Vancouver, Canada) or synthesized. Cholesterol may be purchased from Sigma (St Louis, MO). The specific CRISPR Cas RNA may be encapsulated in LNPs containing DLinDAP, DLinDMA, DLinK-DMA, and DLinKC2-DMA (cationic lipid:DSPC:CHOL: PEGS-DMG or PEG-C-DOMG at 40:10:40:10 molar ratios). When required, 0.2% SP-DiOC18 (Invitrogen, Burlington, Canada) may be incorporated to assess cellular uptake, intracellular delivery, and biodistribution. Encapsulation may be performed by dissolving lipid mixtures comprised of cationic lipid:DSPC:cholesterol:PEG-c-DOMG (40:10:40:10 molar ratio) in ethanol to a final lipid concentration of 10 mmol/l. This ethanol solution of lipid may be added drop-wise to 50 mmol/l citrate, pH 4.0 to form multilamellar vesicles to produce a final concentration of 30% ethanol vol/vol. Large unilamellar vesicles may be formed following extrusion of multilamellar vesicles through two stacked 80 nm Nuclepore polycarbonate filters using the Extruder (Northern Lipids, Vancouver, Canada). Encapsulation may be achieved by adding RNA dissolved at 2 mg/ml in 50 mmol/l citrate, pH 4.0 containing 30% ethanol vol/vol drop-wise to extruded preformed large unilamellar vesicles and incubation at 31 °C for 30 minutes with constant mixing to a final RNA/lipid weight ratio of 0.06/1 wt/wt. Removal of ethanol and neutralization of formulation buffer were performed by dialysis against phosphate-buffered saline (PBS), pH 7.4 for 16 hours using Spectra/Por 2 regenerated cellulose dialysis membranes. Nanoparticle size distribution may be determined by dynamic light scattering using a NICOMP 370 particle sizer, the vesicle/intensity modes, and Gaussian fitting (Nicomp Particle Sizing, Santa Barbara, CA). The particle size for all three LNP systems may be ~70 nm in diameter. siRNA encapsulation efficiency may be determined by removal of free siRNA using VivaPureD MiniH columns (Sartorius Stedim Biotech) from samples collected before and after dialysis. The encapsulated RNA may be extracted from the eluted nanoparticles and quantified at 260 nm. siRNA to lipid ratio was determined by measurement of cholesterol content in vesicles using the Cholesterol E enzymatic assay from Wako Chemicals USA (Richmond, VA).

Preparation of large LNPs may be used/and or adapted from Rosin et al, Molecular Therapy, vol. 19, no. 12, pages 1286-2200, Dec. 2011. A lipid premix solution (20.4 mg/ml total lipid concentration) may be prepared in ethanol containing DLinKC2-DMA, DSPC, and cholesterol at 50:10:38.5 molar ratios. Sodium acetate may be added to the lipid premix at a molar ratio of 0.75:1 (sodium acetate:DLinKC2-DMA). The lipids may be subsequently hydrated by combining the mixture with 1.85 volumes of citrate buffer (10 mmol/l, pH 3.0) with vigorous stirring, resulting in spontaneous liposome formation in aqueous buffer containing 35% ethanol. The liposome solution may be incubated at 37 °C to allow for time-dependent increase in particle size. Aliquots may be removed at various times during incubation to investigate changes in liposome size by dynamic light scattering (Zetasizer Nano ZS, Malvern Instruments, Worcestershire, UK). Once the desired particle size is achieved, an aqueous PEG lipid solution (stock = 10 mg/ml PEG-DMG in 35% (vol/vol) ethanol) may be added to the liposome mixture to yield a final PEG molar concentration of 3.5% of total lipid. Upon addition of PEG-lipids, the liposomes should their size, effectively quenching further growth. RNA may then be added to the empty liposomes at an siRNA to total lipid ratio of approximately 1:10 (wt:wt), followed by incubation for 30 minutes at 37 °C to form loaded LNPs. The mixture may be subsequently dialyzed overnight in PBS and filtered with a 0.45-μm syringe filter.

Spherical Nucleic Acid (SNA™) constructs and other nanoparticles (particularly gold nanoparticles) are also contemplate as a means to delivery CRISPR/Cas system to intended targets. Significant data show that AuraSense Therapeutics' Spherical Nucleic Acid (SNA™) constructs, based upon nucleic acid-functionalized gold nanoparticles, are superior to alternative platforms based on multiple key success factors, such as: High in vivo stability. (Due to their dense loading, a majority of cargo (DNA or siRNA) remains bound to the constructs inside cells, conferring nucleic acid stability and resistance to enzymatic degradation.) Deliverability. (For all cell types studied (e.g., neurons, tumor cell lines, etc.) the constructs demonstrate a transfection efficiency of 99% with no need for carriers or transfection agents.) Therapeutic targeting. (The unique target binding affinity and specificity of the constructs allow exquisite specificity for matched target sequences (i.e., limited off-target effects).) Superior efficacy. (The constructs significantly outperform leading conventional transfection reagents (Lipofectamine 2000 and Cytofectin).) Low toxicity. (The constructs can enter a variety of cultured cells, primary cells, and tissues with no apparent toxicity.) No significant immune response. (The constructs elicit minimal changes in global gene expression as measured by whole-genome microarray studies and cytokine-specific protein assays.) Chemical tailorability. (Any number of single or combinatorial agents (e.g., proteins, peptides, small molecules) can be used to tailor the surface of the constructs.) This platform for nucleic acid-based therapeutics may be applicable to numerous disease states, including inflammation and infectious disease, cancer, skin disorders and cardiovascular disease. Citable literature includes: Cutler et al.,. J. Am. Chem. Soc. 2011 133:9254-9257, Hao et al., Small. 2011 7:3158-3162, Zhang et al., ACS Nano. 2011 5:6962-6970, Cutler et al., J. Am. Chem. Soc. 2012 134:1376-1391, Young et al.,.Nano Lett. 2012 12:3867-71, Zheng et al., Proc. Natl. Acad. Sci. USA. 2012 109:11975-80, Mirkin, Nanomedicine 2012 7:635-638 Zhang et al., J. Am. Chem. Soc. 2012 134:16488-1691, Weintraub, Nature 2013 495:S14-S16, Choi et al., Proc. Natl. Acad. Sci. USA. 2013 110(19):7625-7630, Jensen et al., Sci. Transl. Med. 5, 209ra152 (2013) and Mirkin, et al., Small, doi.org/10.1002/smll.201302143.

Self-assembling nanoparticles with siRNA may be constructed with polyethyleneimine (PEI) that is PEGylated with an Arg-Gly-Asp (RGD) peptide ligand attached at the distal end of the polyethylene glycol (PEG), for example, as a means to target tumor neovasculature expressing integrins and used to deliver siRNA inhibiting vascular endothelial growth factor receptor-2 (VEGF R2) expression and thereby tumor angiogenesis (see, e.g., Schiffelers et al., Nucleic Acids Research, 2004, Vol. 32, No. 19). Nanoplexes may be prepared by mixing equal volumes of aqueous solutions of cationic polymer and nucleic acid to give a net molar excess of ionizable nitrogen (polymer) to phosphate (nucleic acid) over the range of 2 to 6. The electrostatic interactions between cationic polymers and nucleic acid resulted in the formation of polyplexes with average particle size distribution of about 100 nm, hence referred to here as nanoplexes. A dosage of about 100 to 200 mg of CRISPR Cas is envisioned for delivery in the self-assembling nanoparticles of Schiffelers et al.

The nanoplexes of Bartlett et al. (PNAS, September 25, 2007,vol. 104, no. 39) may also be applied to the present invention. The nanoplexes of Bartlett et al. are prepared by mixing equal volumes of aqueous solutions of cationic polymer and nucleic acid to give a net molar excess of ionizable nitrogen (polymer) to phosphate (nucleic acid) over the range of 2 to 6. The electrostatic interactions between cationic polymers and nucleic acid resulted in the formation of polyplexes with average particle size distribution of about 100 nm, hence referred to here as nanoplexes. The DOTA-siRNA of Bartlett et al. was synthesized as follows: 1,4,7,10-tetraazacyclododecane-1,4,7,10-tetraacetic acid mono(N-hydroxysuccinimide ester) (DOTA-NHSester) was ordered from Macrocyclics (Dallas, TX). The amine modified RNA sense strand with a 100-fold molar excess of DOTA-NHS-ester in carbonate buffer (pH 9) was added to a microcentrifuge tube. The contents were reacted by stirring for 4 h at room temperature. The DOTA-RNAsense conjugate was ethanol-precipitated, resuspended in water, and annealed to the unmodified antisense strand to yield DOTA-siRNA. All liquids were pretreated with Chelex-100 (Bio-Rad, Hercules, CA) to remove trace metal contaminants. Tf-targeted and nontargeted siRNA nanoparticles may be formed by using cyclodextrin-containing polycations. Typically, nanoparticles were formed in water at a charge ratio of 3 (+/-) and an siRNA concentration of 0.5 g/liter. One percent of the adamantane-PEG molecules on the surface of the targeted nanoparticles were modified with Tf (adamantane-PEG-Tf). The nanoparticles were suspended in a 5% (wt/vol) glucose carrier solution for injection.

Davis et al. (Nature, Vol 464, 15 April 2010) conducts a siRNA clinical trial that uses a targeted nanoparticle-delivery system (clinical trial registration number NCT00689065). Patients with solid cancers refractory to standard-of-care therapies are administered doses of targeted nanoparticles on days 1, 3, 8 and 10 of a 21-day cycle by a 30-min intravenous infusion. The nanoparticles consist of a synthetic delivery system containing: (1) a linear, cyclodextrin-based polymer (CDP), (2) a human transferrin protein (TF) targeting ligand displayed on the exterior of the nanoparticle to engage TF receptors (TFR) on the surface of the cancer cells, (3) a hydrophilic polymer (polyethylene glycol (PEG) used to promote nanoparticle stability in biological fluids), and (4) siRNA designed to reduce the expression of the RRM2 (sequence used in the clinic was previously denoted siR2B+5). The TFR has long been known to be upregulated in malignant cells, and RRM2 is an established anti-cancer target. These nanoparticles (clinical version denoted as CALAA-01) have been shown to be well tolerated in multi-dosing studies in non-human primates. Although a single patient with chronic myeloid leukaemia has been administered siRNAby liposomal delivery, Davis et al.'s clinical trial is the initial human trial to systemically deliver siRNA with a targeted delivery system and to treat patients with solid cancer. To ascertain whether the targeted delivery system can provide effective delivery of functional siRNA to human tumours, Davis et al. investigated biopsies from three patients from three different dosing cohorts; patients A, B and C, all of whom had metastatic melanoma and received CALAA-01 doses of 18, 24 and 30 mg $m^{-2}$ siRNA, respectively. Similar doses may also be contemplated for the CRISPR Cas system of the present invention. The delivery of the invention may be achieved with nanoparticles containing a linear, cyclodextrin-based polymer (CDP), a human transferrin protein (TF) targeting ligand displayed on the exterior of the nanoparticle to engage TF receptors (TFR) on the surface of the cancer cells and/or a hydrophilic polymer (for example, polyethylene glycol (PEG) used to promote nanoparticle stability in biological fluids).

Kanasty and Anderson (Nature Materials, Vol 12 Nov 2013), initially submitted on 11 March 2013 and published online on 23 October 2013 provides a review of delivery of RNAi. Due to the similarities between RNAi and CRISPR guide sequences, the teaching of this and other art in respect of RNAi is informative for the mechanisms of delivering the guides in the CRISPR-Cas9 system. Some of the techniques described are also be suitable for delivery of the Cas9 as well. In some instance is may be useful to deliver the guides of the CRISPR-Cas9 system separately from the Cas9. This may be as part of a dual-vector delivery system, where the vectors are considered in the broadest light as simply any means of delivery, rather than specifically viral vectors. It is envisaged that the Cas9 may be delivered via a viral vector and that guides specific to genomic targets are delivered separately. As discussed herein, the guides could be delivered via the same vector types as the Cas9, for example a dual-vector system where the Cas9 is delivered in an AAV vector and the guide(s) are delivered in a separate AAV vector. This can be done substantially contemporaneously (i.e. co-delivery), but it could also be done at separate points in time, separated even by weeks or months. For example, if a first round of CRISPR-Cas9 systems have been delivered, but then it is subsequently required to provide further guides, then the original Cas9 which is hopefully still functional in the target cells may be re-used. If the Cas9 is under the control of an inducible promoter, then induction of transcription of new CAs9 in the target cells is preferred. Equally, if a CAs9-expressing model provided for herein is used, then only delivery of guide(s) is necessary. Accordingly, where delivery of guide(s) is required separately from Cas9, then it may be delivered in much the same way as RNAi. As such, the review by Kanasty is helpful in pointing out a number of known approaches that are suitable, with particular focus on the liver, although the means of delivery are generally appropriate for a broad range of cells. Examples include:

- "Liposomal delivery system, as well as siRNA conjugated to lipophilic molecules, interact with serum lipoproteins and subsequently gain entry into hepatocytes that take up those lipoproteins;"
- PEGylation;
- Conjugates such as:
    - Dynamic Polyconjugates (DPCs, 10nm nanoparticles), which have been shown to deliver RNAi to successfully supress ApoB; and Triantennary GalNAc conjugates are "both highly effective";
- Other nanoparticles include:
    - Cyclodextrin Polymer nanoparticles (CDP), including additional formulation components such as adamantine-PEG (AD-PEG) and adamantine-PEG-transferrin (AD-PEG-Tf) ;
    - Lipid Nanoparticles (LNP), including cationic or ionisable lipids, shielding lipids, cholesterol and endogenous or exogenous targeting ligands. An example of an endogenous targeting ligand is Retinol Binding protein (RBP) useful for targeting hepatic and pancreatic stellate cells, which express the RBP receptor. An example of an exogenous targeting ligand is GalNac, which also targets the liver via the asialoglycoprotein receptor on hepatocytes. A combined approach is seen in Anlylams ALN-VSP;
- "Fenestrations in the liver endothelium allow molecules 100-200 nm in diameter to diffuse out of the bloodstream and gain access to the hepatocytes and other liver cells";
- Ligands such as GalNAc are suitable for delivery to non-parenchymal liver cells expressing the mannose receptor, and to hepatocytes where conjugation of suitable siRNA to a GalNAc ligand has been shown to successfully supress PCSK9; and
- Oligonucleotide nanoparticles (ONPs) composed of composed of complimentary DNA fragments designed to hybridise into a pre-defined 3D structure. Using suitable 3' overhand sequences, 6 siRNA strands could be attached to each particle, even at a specified position. The hydrodynamic diameter was about 29nm.

These approaches are preferred in some embodiments for delivery of at least the guides for a CRISPR-Cas9 system. Especially preferred are Dynamic Polyconjugates or the use of an endogenous targeting ligands such as Retinol Binding protein or exogenous targeting ligands such as GalNac.

US Patent No. 8,709,843, incoportated herein by reference, provides a drug delivery system for targeted delivery of therapeutic agent-containing particles to tissues, cells, and intracellular compartments. The invention provides targeted particles comprising comprising polymer conjugated to a surfactant, hydrophilic polymer or lipid. US Patent No. 6,007,845, incorporated herein by reference, provides particles which have a core of a multiblock copolymer formed by covalently linking a multifunctional compound with one or more hydrophobic polymers and one or more hydrophilic polymers, and conatin a biologically active material. US Patent No. 5,855,913, incorporated herein by reference, provides a particulate composition having aerodynamically light particles having a tap density of less than 0.4 g/cm$^3$ with a mean diameter of between 5 $\mu$m and 30 $\mu$m, incorporating a surfactant on the surface thereof for drug delivery to the pulmonary system. US Patent No. 5,985,309, incorporated herein by reference, provides particles incorporating a surfactant and/or a hydrophilic or hydrophobic complex of a positively or negatively charged therapeutic or diagnostic agent and a charged molecule of opposite charge for delivery to the pulmonary system. US. Patent No. 5,543,158, incorporated herein by reference, provides biodegradable injectable nanoparticles having a biodegradable solid core containing a biologically active material and poly(alkylene glycol) moieties on the surface. WO2012135025 (also published as US20120251560), incorporated herein by reference, describes conjugated polyethyleneimine (PEI) polymers and conjugated aza-macrocycles (collectively referred to as "conjugated lipomer" or "lipomers"). In certain embodiments, it can envisioned that such conjugated lipomers can be used in the context of the CRISPR-Cas system to achieve *in vitro, ex vivo* and *in vivo* genomic perturbations to modify gene expression, including modulation of protein expression. Techniques of these patent documents can be adapted in the practice of the instant invention.

In one embodiment, the nanoparticle may be epoxide-modified lipid-polymer, advantageously 7C1 (*see,* e.g., James E. Dahlman and Carmen Barnes et al. Nature Nanotechnology (2014) published online 11 May 2014, doi:10.1038/nnano.2014.84). C71 was synthesized by reacting C15 epoxide-terminated lipids with PEI600 at a 14:1 molar ratio, and was formulated with C14PEG2000 to produce nanoparticles (diameter between 35 and 60 nm) that were stable in PBS solution for at least 40 days. An epoxide-modified lipid-polymer may be utilized to deliver the CRISPR-Cas system of the present invention to pulmonary, cardiovascular or renal cells, however, one of skill in the art may adapt the system to deliver to other target organs. Dosage ranging from about 0.05 to about 0.6 mg/kg are envisioned. Dosages over several days or weeks are also envisioned, with a total dosage of about 2 mg/kg.

Exosomes

Exosomes are endogenous nano-vesicles that transport RNAs and proteins which can deliver short interfering (si)RNA to the brain in mice. To reduce immunogenicity, Alvarez-Erviti et al. (2011, Nat Biotechnol 29: 341) used self-derived dendritic cells for exosome production. Targeting was achieved by engineering the dendritic cells to express Lamp2b, an exosomal membrane protein, fused to the neuron-specific RVG peptide3. Purified exosomes were loaded with exogenous siRNA by electroporation. Intravenously injected RVG-targeted exosomes delivered GAPDH siRNA specifically to neurons, microglia, oligodendrocytes in the brain, resulting in a specific gene knockdown. Pre-exposure to RVG exosomes did not attenuate knockdown, and non-specific uptake in other tissues was not observed. The therapeutic potential of exosome-mediated siRNA delivery was demonstrated by the strong mRNA (60%) and protein (62%) knockdown of BACE1, a therapeutic target in Alzheimer's disease.

To obtain a pool of immunologically inert exosomes, Alvarez-Erviti et al. harvested bone marrow from inbred C57BL/6 mice with a homogenous major histocompatibility complex (MHC) haplotype. As immature dendritic cells produce large quantities of exosomes devoid of T-cell activators such as MHC-II and CD86, Alvarez-Erviti et al. selected for dendritic cells with granulocyte/macrophage-colony stimulating factor (GM-CSF) for 7 d. Exosomes were purified from the culture supernatant the following day using well-established ultracentrifugation protocols. The exosomes produced were physically homogenous, with a size distribution peaking at 80 nm in diameter as determined by nanoparticle tracking analysis (NTA) and electron microscopy. Alvarez-Erviti et al. obtained 6-12 $\mu$g of exosomes (measured based on protein concentration) per $10^6$ cells.

Next, Alvarez-Erviti et al. investigated the possibility of loading modified exosomes with exogenous cargoes using electroporation protocols adapted for nanoscale applications. As electroporation for membrane particles at the nanometer scale is not well-characterized, nonspecific Cy5-labeled siRNA was used for the empirical optimization of the electroporation protocol. The amount of encapsulated siRNA was assayed after ultracentrifugation and lysis of exosomes. Electroporation at 400 V and 125 $\mu$F resulted in the greatest retention of siRNA and was used for all subsequent experiments.

Alvarez-Erviti et al. administered 150 $\mu$g of each BACE1 siRNA encapsulated in 150 $\mu$g of RVG exosomes to normal C57BL/6 mice and compared the knockdown efficiency to four controls: untreated mice, mice injected with RVG exosomes only, mice injected with BACE1 siRNA complexed to an in vivo cationic liposome reagent and mice injected with BACE1 siRNA complexed to RVG-9R, the RVG pep tide conjugated to 9 D-arginines that electrostatically binds to the siRNA. Cortical tissue samples were analyzed 3 d after administration and a significant protein knockdown (45%, $P < 0.05$, versus 62%, $P < 0.01$) in both siRNA-RVG-9R-treated and siRNARVG exosome-treated mice was observed, resulting from a significant decrease in BACE1 mRNA levels (66% [+ or -] 15%, $P < 0.001$ and 61% [+ or -] 13% respectively, $P < 0.01$). Moreover, Applicants demonstrated a significant decrease (55%, $P < 0.05$) in the total [beta]-amyloid 1-42 levels, a main component of the amyloid plaques in Alzheimer's pathology, in the RVG-exosome-treated animals. The decrease observed was greater than the $\beta$-amyloid 1-40 decrease demonstrated in normal mice after intraventricular injection of BACE1 inhibitors. Alvarez-Erviti et al. carried out 5'-rapid amplification of cDNA ends (RACE) on BACE1 cleavage product, which provided evidence of RNAi-mediated knockdown by the siRNA.

Finally, Alvarez-Erviti et al. investigated whether siRNA-RVG exosomes induced immune responses in vivo by assessing IL-6, IP-10, TNF$\alpha$ and IFN-$\alpha$ serum concentrations. Following siRNA-RVG exosome treatment, nonsignificant changes in all cytokines were registered similar to siRNA-transfection reagent treatment in contrast to siRNA-RVG-9R, which potently stimulated IL-6 secretion, confirming the immunologically inert profile of the exosome treatment. Given that exosomes encapsulate only 20% of siRNA, delivery with RVG-exosome appears to be more efficient than RVG-9R delivery as comparable mRNA knockdown and greater protein knockdown was achieved with fivefold less siRNA without the corresponding level of immune stimulation. This experiment demonstrated the therapeutic potential of RVG-exosome technology, which is potentially suited for long-term silencing of genes related to neurodegenerative diseases. The exosome delivery system of Alvarez-Erviti et al. may be applied to deliver the CRISPR-Cas system of the present invention to therapeutic targets, especially neurodegenerative diseases. A dosage of about 100 to 1000 mg of CRISPR Cas encapsulated in about 100 to 1000 mg of RVG exosomes may be contemplated for the present invention.

El-Andaloussi et al. (Nature Protocols 7,2112-2126(2012)) discloses how exosomes derived from cultured cells can be harnessed for delivery of siRNA in vitro and in vivo. This protocol first describes the generation of targeted exosomes through transfection of an expression vector, comprising an exosomal protein fused with a peptide ligand. Next, El-Andaloussi et al. explain how to purify and characterize exosomes from transfected cell supernatant. Next, El-Andaloussi et al. detail crucial steps for loading siRNA into exosomes. Finally, El-Andaloussi et al. outline how to use exosomes to efficiently deliver siRNA in vitro and in vivo in mouse brain. Examples of anticipated results in which exosome-mediated siRNA delivery is evaluated by functional assays and imaging are also provided. The entire protocol takes ~3 weeks. Delivery or administration according to the invention may be performed using exosomes produced from self-derived dendritic cells.

In another embodiment, the plasma exosomes of Wahlgren et al. (Nucleic Acids Research, 2012, Vol. 40, No. 17 e130) are contemplated. Exosomes are nano-sized vesicles (30-90nm in size) produced by many cell types, including dendritic cells (DC), B cells, T cells, mast cells, epithelial cells and tumor cells. These vesicles are formed by inward budding of late endosomes and are then released to the extracellular environment upon fusion with the plasma membrane. Because exosomes naturally carry RNA between cells, this property might be useful in gene therapy.

Exosomes from plasma are prepared by centrifugation of buffy coat at 900g for 20 min to isolate the plasma followed by harvesting cell supernatants, centrifuging at 300g for 10 min to eliminate cells and at 16 500g for 30 min followed by filtration through a 0.22 mm filter. Exosomes are pelleted by ultracentrifugation at 120 000g for70 min. Chemical transfection of siRNA into exosomes is carried out according to the manufacturer's instructions in RNAi Human/Mouse Starter Kit (Quiagen, Hilden, Germany). siRNA is added to 100 ml PBS at a final concentration of 2 mmol/ml. After adding HiPerFect transfection reagent, the mixture is incubated for 10 min at RT. In order to remove the excess of micelles, the exosomes are re-isolated using aldehyde/sulfate latex beads. The chemical transfection of CRISPR Cas into exosomes may be conducted similarly to siRNA. The exosomes may be co-cultured with monocytes and lymphocytes isolated from the peripheral blood of healthy donors. Therefore, it may be contemplated that exosomes containing CRISPR Cas may be introduced to monocytes and lymphocytes of and autologously reintroduced into a human. Accordingly, delivery or administration according to the invention may be performed using plasma exosomes.

Liposomes

Delivery or administration according to the invention can be performed with liposomes. Liposomes are spherical vesicle structures composed of a uni- or multilamellar lipid bilayer surrounding internal aqueous compartments and a relatively impermeable outer lipophilic phospholipid bilayer. Liposomes have gained considerable attention as drug delivery carriers because they are biocompatible, nontoxic, can deliver both hydrophilic and lipophilic drug molecules, protect their cargo from degradation by plasma enzymes, and transport their load across biological membranes and the blood brain barrier (BBB) (see, e.g., Spuch and Navarro, Journal of Drug Delivery, vol. 2011, Article ID 469679, 12 pages, 2011. doi:10.1155/2011/469679 for review). Liposomes can be made from several different types of lipids; however, phospholipids are most commonly used to generate liposomes as drug carriers. Although liposome formation is spontaneous when a lipid film is mixed with an aqueous solution, it can also be expedited by applying force in the form of shaking by using a homogenizer, sonicator, or an extrusion apparatus (see, e.g., Spuch and Navarro, Journal of Drug Delivery, vol. 2011, Article ID 469679, 12 pages, 2011. doi:10.1155/2011/469679 for review).

Several other additives may be added to liposomes in order to modify their structure and properties. For instance, either cholesterol or sphingomyelin may be added to the liposomal mixture in order to help stabilize the liposomal structure and to prevent the leakage of the liposomal inner cargo. Further, liposomes are prepared from hydrogenated egg phosphatidylcholine or egg phosphatidylcholine, cholesterol, and dicetyl phosphate, and their mean vesicle sizes were adjusted to about 50 and 100 nm. (see, e.g., Spuch and Navarro, Journal of Drug Delivery, vol. 2011, Article ID 469679, 12 pages, 2011. doi:10.1155/2011/469679 for review). Conventional liposome formulation is mainly comprised of natural phospholipids and lipids such as 1,2-distearoryl-sn-glycero-3-phosphatidyl choline (DSPC), sphingomyelin, egg phosphatidylcholines and monosialoganglioside. Since this formulation is made up of phospholipids only, liposomal formulations have encountered many challenges, one of the ones being the instability in plasma. Several attempts to overcome these challenges have been made, specifically in the manipulation of the lipid membrane. One of these attempts focused on the manipulation of cholesterol. Addition of cholesterol to conventional formulations reduces rapid release of the encapsulated bioactive compound into the plasma or 1,2-dioleoyl-sn-glycero-3-phosphoethanolamine (DOPE) increases the stability (see, e.g., Spuch and Navarro, Journal of Drug Delivery, vol. 2011, Article ID 469679, 12 pages, 2011. doi:10.1155/2011/469679 for review).

In a particularly advantageous embodiment, Trojan Horse liposomes (also known as Molecular Trojan Horses) are desirable and protocols may be found at http://cshprotocols.cshlp.org/content/2010/4/pdb.prot5407.long. These particles allow delivery of a transgene to the entire brain after an intravascular injection. Without being bound by limitation, it is believed that neutral lipid particles with specific antibodies conjugated to surface allow crossing of the blood brain barrier via endocytosis. Applicant postulates utilizing Trojan Horse Liposomes to deliver the CRISPR family of nucleases to the brain via an intravascular injection, which would allow whole brain transgenic animals without the need for embryonic manipulation. About 1-5 g of DNA may be contemplated for in vivo administration in liposomes.

In another embodiment, the CRISPR Cas system may be administered in liposomes, such as a stable nucleic-acid-lipid particle (SNALP) (see, e.g., Morrissey et al., Nature Biotechnology, Vol. 23, No. 8, August 2005). Daily intravenous injections of about 1, 3 or 5 mg/kg/day of a specific CRISPR Cas targeted in a SNALP are contemplated. The daily treatment may be over about three days and then weekly for about five weeks. In another embodiment, a specific CRISPR Cas encapsulated SNALP) administered by intravenous injection to at doses of abpit 1 or 2.5 mg/kg are also contemplated (see, e.g., Zimmerman et al., Nature Letters, Vol. 441, 4 May 2006). The SNALP formulation may contain the lipids 3-N-[(wmethoxypoly(ethylene glycol) 2000) carbamoyl] -! ,2-dimyristyloxy-propylamine (PEG-C-DMA), 1,2-dilinoleyloxy-N,N-dimethyl-3-aminopropane (DLinDMA), 1,2-distearoyl-sn-glycero-3-phosphocholine (DSPC) and cholesterol, in a 2:40:10:48 molar per cent ratio (see, e.g., Zimmerman et al., Nature Letters, Vol. 441, 4 May 2006).

In another embodiment, stable nucleic-acid-lipid particles (SNALPs) have proven to be effective delivery molecules to highly vascularized HepG2-derived liver tumors but not in poorly vascularized HCT-116 derived liver tumors (see, e.g., Li, Gene Therapy (2012) 19, 775-780). The SNALP liposomes may be prepared by formulating D-Lin-DMA and PEG-C-DMA with distearoylphosphatidylcholine (DSPC), Cholesterol and siRNA using a 25:1 lipid/siRNA ratio and a 48/40/10/2 molar ratio of Cholesterol/D-Lin-DMA/DSPC/PEG-C-DMA. The resulted SNALP liposomes are about 80-100 nm in size.

In yet another embodiment, a SNALP may comprise synthetic cholesterol (Sigma-Aldrich, St Louis, MO, USA), dipalmitoylphosphatidylcholine (Avanti Polar Lipids, Alabaster, AL, USA), 3-N-[(w-methoxy poly(ethylene glycol)2000)carbamoyl]-1,2-dimyrestyloxypropylamine, and cationic 1,2-dilinoleyloxy-3-N,Ndimethylaminopropane (see, e.g., Geisbert et al., Lancet 2010; 375: 1896-905). A dosage of about 2 mg/kg total CRISPR Cas per dose administered as, for example, a bolus intravenous infusion may be contemplated. In yet another embodiment, a SNALP may comprise synthetic cholesterol (Sigma-Aldrich), 1,2-distearoyl-sn-glycero-3-phosphocholine (DSPC; Avanti Polar Lipids Inc.), PEG-cDMA, and 1,2-dilinoleyloxy-3-(N;N-dimethyl)aminopropane (DLinDMA) (see, e.g., Judge, J. Clin. Invest. 119:661-673 (2009)). Formulations used for in vivo studies may comprise a final lipid/RNA mass ratio of about 9:1.

The safety profile of RNAi nanomedicines has been reviewed by Barros and Gollob of Alnylam Pharmaceuticals (see, e.g., Advanced Drug Delivery Reviews 64 (2012) 1730-1737). The stable nucleic acid lipid particle (SNALP) is comprised of four different lipids - an ionizable lipid (DLinDMA) that is cationic at low pH, a neutral helper lipid, cholesterol, and a diffusible polyethylene glycol (PEG)-lipid. The particle is approximately 80 nm in diameter and is charge-neutral at physiologic pH. During formulation, the ionizable lipid serves to condense lipid with the anionic siRNA during particle formation. When positively charged under increasingly acidic endosomal conditions, the ionizable lipid also mediates the fusion of SNALP with the endosomal membrane enabling release of siRNA into the cytoplasm. The PEG-lipid stabilizes the particle and reduces aggregation during formulation, and subsequently provides a neutral hydrophilic exterior that improves pharmacokinetic properties.

To date, two clinical programs have been initiated using SNALPsiRNA formulations. Tekmira Pharmaceuticals recently completed a phase I single-dose study of SNALP-ApoB in adult volunteers with elevated LDL cholesterol. ApoB is predominantly expressed in the liver and jejunum and is essential for the assembly and secretion of VLDL and LDL. Seventeen subjects received a single dose of SNALP-ApoB (dose escalation across 7 dose levels). There was no evidence of liver toxicity (anticipated as the potential dose-limiting toxicity based on preclinical studies). One (of two) subjects at the highest dose experienced flu-like symptoms consistent with immune system stimulation, and the decision was made to conclude the trial.

Alnylam Pharmaceuticals has similarly advanced ALN-TTR01, which employs the SNALP technology described above and targets hepatocyte production of both mutant and wild-type TTR to treat TTR amyloidosis (ATTR). Three ATTR syndromes have been described: familial amyloidotic polyneuropathy (FAP) and familial amyloidotic cardiomyopathy (FAC) - both caused by autosomal dominant mutations in TTR; and senile systemic amyloidosis (SSA) cause by wildtype TTR. A placebo-controlled, single dose-escalation phase I trial of ALN-TTR01 was recently completed in patients with ATTR. ALN-TTR01 was administered as a 15-minute IV infusion to 31 patients (23 with study drug and 8 with placebo) within a dose range of 0.01 to 1.0 mg/kg (based on siRNA). Treatmentwaswell tolerated with no significant increases in liver function tests. Infusion-related reactions were noted in 3 of 23 patients at≥0.4 mg/kg; all responded to slowing of the infusion rate and all continued on study. Minimal and transient elevations of serum cytokines IL-6, IP-10 and IL-1ra were noted in two patients at the highest dose of 1 mg/kg (as anticipated from preclinical and NHP studies). Lowering of serum TTR, the expected pharmacodynamics effect of ALN-TTR01, was observed at 1 mg/kg.

In yet another embodiment, a SNALP may be made by solubilizing a cationic lipid, DSPC, cholesterol and PEG-lipid were solubilized in ethanol at a molar ratio of 40:10:40:10, respectively (see, Semple et al., Nature Niotechnology, Volume 28 Number 2 February 2010, pp. 172-177). The lipid mixture was added to an aqueous buffer (50 mM citrate, pH 4) with mixing to a final ethanol and lipid concentration of 30% (vol/vol) and 6.1 mg/ml, respectively, and allowed to equilibrate at 22 °C for 2 min before extrusion. The hydrated lipids were extruded through two stacked 80 nm pore-sized filters (Nuclepore) at 22 °C using a Lipex Extruder (Northern Lipids) until a vesicle diameter of 70-90 nm, as determined by dynamic light scattering analysis, was obtained. This generally required 1-3 passes. The siRNA (solubilized in a 50 mM citrate, pH 4 aqueous solution containing 30% ethanol) was added to the pre-equilibrated (35 °C) vesicles at a rate of ~5 ml/min with mixing. After a final target siRNA/lipid ratio of 0.06 (wt/wt) was reached, the mixture was incubated for a further 30 min at 35 °C to allow vesicle reorganization and encapsulation of the siRNA. The ethanol was then removed and the external buffer replaced with PBS (155 mM NaCl, 3 mM Na2HPO4, 1 mM KH2PO4, pH 7.5) by either dialysis or tangential flow diafiltration. siRNA were encapsulated in SNALP using a controlled step-wise dilution method process. The lipid constituents of KC2-SNALP were DLin-KC2-DMA (cationic lipid), dipalmitoylphosphatidylcholine (DPPC; Avanti Polar Lipids), synthetic cholesterol (Sigma) and PEG-C-DMA used at a molar ratio of 57.1:7.1:34.3:1.4. Upon formation of the loaded particles, SNALP were dialyzed against PBS and filter sterilized through a 0.2 μm filter before use. Mean particle sizes were 75-85 nm and 90-95% of the siRNA was encapsulated within the lipid particles. The final siRNA/lipid ratio in formulations used for in vivo testing was ~0.15 (wt/wt). LNP-siRNA systems containing Factor VII siRNA were diluted to the appropriate concentrations in sterile PBS immediately before use and the formulations were administered intravenously through the lateral tail vein in a total volume of 10 ml/kg. This method may be extrapolated to the CRISPR Cas system of the present invention.

Other Lipids

Other cationic lipids, such as amino lipid 2,2-dilinoleyl-4-dimethylaminoethyl-[1,3]-dioxolane (DLin-KC2-DMA) may be utilized to encapsulate CRISPR Cas similar to SiRNA (see, e.g., Jayaraman, Angew. Chem. Int. Ed. 2012, 51, 8529 -8533). A preformed vesicle with the following lipid composition may be contemplated: amino lipid, distearoylphosphatidylcholine (DSPC), cholesterol and (R)-2,3-bis(octadecyloxy) propyl-1-(methoxy poly(ethylene glycol)2000)propylcarbamate (PEG-lipid) in the molar ratio 40/10/40/10, respectively, and a FVII siRNA/total lipid ratio of approximately 0.05 (w/w). To ensure a narrow particle size distribution in the range of 70-90 nm and a low polydispersity index of 0.11_0.04 (n=56), the particles may be extruded up to three times through 80 nm membranes prior to adding the CRISPR Cas RNA. Particles containing the highly potent amino lipid 16 may be used, in which the molar ratio of the four lipid components 16, DSPC, cholesterol and PEG-lipid (50/10/38.5/1.5) which may be further optimized to enhance in vivo activity.

Michael S D Kormann et al. ("Expression of therapeutic proteins after delivery of chemically modified mRNA in mice: Nature Biotechnology, Volume:29, Pages: 154-157 (2011) Published online 09 January 2011) describes the use of lipid envelopes to deliver RNA. Use of lipid envelopes is also preferred in the present invention. In another embodiment, lipids may be formulated with the CRISPR Cas system of the present invention to form lipid nanoparticles (LNPs). Lipids include, but are not limited to, DLin-KC2-DMA4, C12-200 and colipids disteroylphosphatidyl choline, cholesterol, and PEG-DMG may be formulated with CRISPR Cas instead of siRNA (see, e.g., Novobrantseva, Molecular Therapy-Nucleic Acids (2012) 1, e4; doi:10.1038/mtna.2011.3) using a spontaneous vesicle formation procedure. The component molar ratio may be about 50/10/38.5/1.5 (DLin-KC2-DMA or C12-200/disteroylphosphatidyl choline/cholesterol/PEG-DMG). The final lipid:siRNA weight ratio may be ~12:1 and 9:1 in the case of DLin-KC2-DMA and C12-200 lipid nanoparticles (LNPs), respectively. The formulations may have mean particle diameters of ~80 nm with >90% entrapment efficiency. A 3 mg/kg dose may be contemplated. Tekmira has a portfolio of approximately 95 patent families, in the U.S. and abroad, that are directed to various aspects of LNPs and LNP formulations (see, e.g., U.S. Pat. Nos. 7,982,027; 7,799,565; 8,058,069; 8,283,333; 7,901,708; 7,745,651; 7,803,397; 8,101,741; 8,188,263; 7,915,399; 8,236,943 and 7,838,658 and European Pat. Nos .1766035; 1519714; 1781593 and 1664316), all of which may be used in and/or adapted to practice the present invention.

The CRISPR Cas system may be delivered encapsulated in PLGA Microspheres such as that further described in US published applications 20130252281 and 20130245107 and 20130244279 (assigned to Moderna Therapeutics) which relate to aspects of formulation of compositions comprising modified nucleic acid molecules which may encode a protein, a protein precursor, or a partially or fully processed form of the protein or a protein precursor. The formulation may have a molar ratio 50:10:38.5:1.5-3.0 (cationic lipid:fusogenic lipid:cholesterol:PEG lipid). The PEG lipid may be selected from, but is not limited to PEG-c-DOMG, PEG-DMG. The fusogenic lipid may be DSPC. See also, Schrum et al., Delivery and Formulation of Engineered Nucleic Acids, US published application 20120251618.

Nanomerics' technology addresses bioavailability challenges for a broad range of therapeutics, including low molecular weight hydrophobic drugs, peptides, and nucleic acid based therapeutics (plasmid, siRNA, miRNA). Specific administration routes for which the technology has demonstrated clear advantages include the oral route, transport across the blood-brain-barrier, delivery to solid tumours, as well as to the eye. See, e.g., Mazza et al., 2013, ACS Nano. 2013 Feb 26;7(2):1016-26; Uchegbu and Siew, 2013, J Pharm Sci. 102(2):305-10 and Lalatsa et al., 2012, J Control Release. 2012 Jul 20;161(2):523-36.

US Patent Publication No. 20050019923 involves cationic dendrimers for delivering bioactive molecules, such as polynucleotide molecules, peptides and polypeptides and/or pharmaceutical agents, to a mammalian body. The dendrimers are suitable for targeting the delivery of the bioactive molecules to, for example, the liver, spleen, lung, kidney or heart. Dendrimers are synthetic 3-dimensional macromolecules that are prepared in a step-wise fashion from simple branched monomer units, the nature and functionality of which can be easily controlled and varied. Dendrimers are synthesised from the repeated addition of building blocks to a multifunctional core (divergent approach to synthesis), or towards a multifunctional core (convergent approach to synthesis) and each addition of a 3-dimensional shell of building blocks leads to the formation of a higher generation of the dendrimers. Polypropylenimine dendrimers start from a diaminobutane core to which is added twice the number of amino groups by a double Michael addition of acrylonitrile to the primary amines followed by the hydrogenation of the nitriles. This results in a doubling of the amino groups. Polypropylenimine dendrimers contain 100% protonable nitrogens and up to 64 terminal amino groups (generation 5, DAB 64). Protonable groups are usually amine groups which are able to accept protons at neutral pH. The use of dendrimers as gene delivery agents has largely focused on the use of the polyamidoamine. and phosphorous containing compounds with a mixture of amine/amide or N--P($O_2$)S as the conjugating units respectively with no work being reported on the use of the lower generation polypropylenimine dendrimers for gene delivery. Polypropylenimine dendrimers have also been studied as pH sensitive controlled release systems for drug delivery and for their encapsulation of guest molecules when chemically modified by peripheral amino acid groups. The cytotoxicity and interaction of polypropylenimine dendrimers with DNA as well as the transfection efficacy of DAB 64 has also been studied. US Patent Publication No. 20050019923 is based upon the observation that, contrary to earlier reports, cationic dendrimers, such as polypropylenimine dendrimers, display suitable properties, such as specific targeting and low toxicity, for use in the targeted delivery of bioactive molecules, such as genetic material. In addition, derivatives of the cationic dendrimer also display suitable properties for the targeted delivery of bioactive molecules. See also, Bioactive Polymers, US published application 20080267903, which states: "Various polymers, including cationic polyamine polymers and dendrimeric polymers, are shown to possess anti-proliferative activity, and may therefore be useful for treatment of disorders characterised by undesirable cellular proliferation such as neoplasms and tumours, inflammatory disorders (including autoimmune disorders), psoriasis and atherosclerosis. The polymers may be used alone as active agents, or as delivery vehicles for other therapeutic agents, such as drug molecules or nucleic acids for gene therapy. In such cases, the polymers' own intrinsic anti-tumour activity may complement the activity of the agent to be delivered." These documents may be adapted in the practice of the instant invention.

CRISPR enzyme mRNA and guide RNA

CRISPR enzyme or CRISPR enzyme mRNA and CRISPR guide RNA might also be delivered separately; and advantageously at least one of these is delivered via a nanoparticle complex. CRISPR enzyme mRNA can be delivered prior to the guide RNA to give time for CRISPR enzyme to be expressed. CRISPR enzyme mRNA might be administered 1-12 hours (preferably around 2-6 hours) prior to the administration of guide RNA. Alternatively, CRISPR enzyme mRNA and guide RNA can be administered together. Advantageously, a second booster dose of guide RNA can be administered 1-12 hours (preferably around 2-6 hours) after the initial administration of CRISPR enzyme mRNA + guide RNA. Additional administrations of CRISPR enzyme mRNA and/or guide RNA might be useful to achieve the most efficient levels of genome modification. For minimization of toxicity and off-target effect, it will be important to control the concentration of CRISPR enzyme mRNA and guide RNA delivered. Optimal concentrations of CRISPR enzyme mRNA and guide RNA can be determined by testing different concentrations in a cellular or animal model and using deep sequencing the analyze the extent of modification at potential off-target genomic loci. For example, for the guide sequence targeting 5'-GAGTCCGAGCAGAAGAAGAA-3' in the EMX1 gene of the human genome, deep sequencing can be used to assess the level of modification at the following two off-target loci, 1: 5'-GAGTCCTAGCAGGAGAAGAA-3' and 2: 5'-GAGTCTAAGCAGAAGAAGAA-3'. The concentration that gives the highest level of on-target modification while minimizing the level of off-target modification should be chosen for in vivo delivery. Alternatively, to minimize the level of toxicity and off-target effect, CRISPR enzyme nickase mRNA (for example S. pyogenes Cas9 with the D10A mutation) can be delivered with a pair of guide RNAs targeting a site of interest. The two guide RNAs need to be spaced as follows. Guide sequences in red (single underline) and blue (double underline) respectively (these examples are based on the PAM requirement for *Streptococcus pyogenes* Cas9).

| Overhang length (bp) | Guide RNA design (guide sequence and PAM color coded) |
|---|---|
| 14 | 5'-NNNNNNNNNNNNNNNNNNNNCCNNNNNNNNNNNNNNNNNNNNNNGGNNNNNNNNNNNNNN-3' |

(continued)

| Overhang length (bp) | Guide RNA design (guide sequence and PAM color coded) |
|---|---|
| 13 | 3′-<br>NNNNNNNNNNNNNNNNNNNNGGNNNNNNNNNNNNNNNNNNNNNNCCNNNNNNNNNNNNN<br>N-5′ |
| 12 | 5′-<br>NNNNNNNNNNNNNNNNNNNNCCNNNNNNNNNNNNNNNNNNNNNGGNNNNNNNNNNNNNN<br>N-3′ |
| 11 | 3′-<br>NNNNNNNNNNNNNNNNNNNNGGNNNNNNNNNNNNNNNNNNNNNCCNNNNNNNNNNNNNN<br>N-5′ |
| 10 | |
| 9 | 5′-<br>NNNNNNNNNNNNNNNNNNNNCCNNNNNNNNNNNNNNNNNNNNGGNNNNNNNNNNNNNNN<br>N-3′ |
| 8 | 3′-<br>NNNNNNNNNNNNNNNNNNNNGGNNNNNNNNNNNNNNNNNNNNNCCNNNNNNNNNNNNNN<br>N-5′ |
| 7 | 5′-<br>NNNNNNNNNNNNNNNNNNNNCCNNNNNNNNNNNNNNNNNNNGGNNNNNNNNNNNNNNNN<br>N-3′ |
| 6 | 3′-<br>NNNNNNNNNNNNNNNNNNNNGGNNNNNNNNNNNNNNNNNNNCCNNNNNNNNNNNNNNNN<br>N-5′ |
| 5 | 5′-<br>NNNNNNNNNNNNNNNNNNNNCCNNNNNNNNNNNNNNNNNNGGNNNNNNNNNNNNNNNNN<br>N-3′ |
| 4 | 3′-<br>NNNNNNNNNNNNNNNNNNNNGGNNNNNNNNNNNNNNNNNNCCNNNNNNNNNNNNNNNNN<br>N-5′ |
| 3 | |
| 2 | 5′-<br>NNNNNNNNNNNNNNNNNNNNCCNNNNNNNNNNNNNNNNNGGNNNNNNNNNNNNNNNNNN<br>N-3′ |
| 1 | 3′-<br>NNNNNNNNNNNNNNNNNNNNGGNNNNNNNNNNNNNNNNNCCNNNNNNNNNNNNNNNNNN<br>N-5′ |
| blunt | 5′-<br>NNNNNNNNNNNNNNNNNNNNCCNNNNNNNNNNNNNNNNGGNNNNNNNNNNNNNNNNNNN<br>N-3′ |

(continued)

| Overhang length (bp) | Guide RNA design (guide sequence and PAM color coded) |
|---|---|
| 1 | 3′-NNNNNNNNNNNNNNNNNNNNGGNNNNNNNNNNNNNNNNCCNNNNNNNNNNNNNNNNNNNNNNN-5′ |
| 2 | 5′-NNNNNNNNNNNNNNNNNNNNCCNNNNNNNNNNNNNNNGGNNNNNNNNNNNNNNNNNNNNN-3′ |
| 3 | 3′-NNNNNNNNNNNNNNNNNNNNGGNNNNNNNNNNNNNNNCCNNNNNNNNNNNNNNNNNNNNNNN-5′ |
| 4 | |
| 5 | 5′-NNNNNNNNNNNNNNNNNNNNCCNNNNNNNNNNNNNNGGNNNNNNNNNNNNNNNNNNNNNN-3′ |
| 6 | 3′-NNNNNNNNNNNNNNNNNNNNGGNNNNNNNNNNNNNNCCNNNNNNNNNNNNNNNNNNNNNNNN-5′ |
| 7 | 5′-NNNNNNNNNNNNNNNNNNNNCCNNNNNNNNNNNNNGGNNNNNNNNNNNNNNNNNNNNNNN-3′ |
| 8 | 3′-NNNNNNNNNNNNNNNNNNNNGGNNNNNNNNNNNNNCCNNNNNNNNNNNNNNNNNNNNNNNNN-5′ |
| 12 | 5′-NNNNNNNNNNNNNNNNNNNNCCNNNNNNNNNNNNGGNNNNNNNNNNNNNNNNNNNNNNNN-3′ |
| 13 | 3′-NNNNNNNNNNNNNNNNNNNNGGNNNNNNNNNNNNCCNNNNNNNNNNNNNNNNNNNNNNNNNN-5′ |
| 14 | |
| 15 | 5′-NNNNNNNNNNNNNNNNNNNNCCNNNNNNNNNNNGGNNNNNNNNNNNNNNNNNNNNNNNNN-3′ |
| 16 | 3′-NNNNNNNNNNNNNNNNNNNNGGNNNNNNNNNNNCCNNNNNNNNNNNNNNNNNNNNNNNNNNN-5′ |
| 17 | 5′-NNNNNNNNNNNNNNNNNNNNCCNNNNNNNNNNGGNNNNNNNNNNNNNNNNNNNNNNNNNN-3′ |

(continued)

| Overhang length (bp) | Guide RNA design (guide sequence and PAM color coded) |
|---|---|
| | 3′-<br>NNNNNNNNNNNNNNNNNNNNGGNNNNNNNNNNCCNNNNNNNNNNNNNNNNNNNNNNNNN<br>N-5′ |
| | 5′-<br>NNNNNNNNNNNNNNNNNNNNCCNNNNNNNNNGGNNNNNNNNNNNNNNNNNNNNNNNNNN<br>N-3′<br>3′-<br>NNNNNNNNNNNNNNNNNNNNGGNNNNNNNNNCCNNNNNNNNNNNNNNNNNNNNNNNNNN<br>N-5′<br>5′-<br>NNNNNNNNNNNNNNNNNNNNCCNNNNNNNNGGNNNNNNNNNNNNNNNNNNNNNNNNNNN<br>N-3′<br>3′-<br>NNNNNNNNNNNNNNNNNNNNGGNNNNNNNNCCNNNNNNNNNNNNNNNNNNNNNNNNNNN<br>N-5′<br>5′-<br>NNNNNNNNNNNNNNNNNNNNCCNNNNNNNGGNNNNNNNNNNNNNNNNNNNNNNNNNNNN<br>N-3′<br>3′-<br>NNNNNNNNNNNNNNNNNNNNGGNNNNNNNCCNNNNNNNNNNNNNNNNNNNNNNNNNNNN<br>N-5′<br>5′-<br>NNNNNNNNNNNNNNNNNNNNCCNNNNNNGGNNNNNNNNNNNNNNNNNNNNNNNNNNNNN<br>N-3′<br>3′-<br>NNNNNNNNNNNNNNNNNNNNGGNNNNNNCCNNNNNNNNNNNNNNNNNNNNNNNNNNNNN<br>N-5′<br>5′-<br>NNNNNNNNNNNNNNNNNNNNCCNNNNNGGNNNNNNNNNNNNNNNNNNNNNNNNNNNNNN<br>N-3′<br>3′-<br>NNNNNNNNNNNNNNNNNNNNGGNNNNNCCNNNNNNNNNNNNNNNNNNNNNNNNNNNNNN<br>N-5′<br>5′-<br>NNNNNNNNNNNNNNNNNNNNCCNNNNGGNNNNNNNNNNNNNNNNNNNNNNNNNNNNNNN |
| | N-3′ |

(continued)

| Overhang length (bp) | Guide RNA design (guide sequence and PAM color coded) |
|---|---|
| | 3'-<br>NNNNNNNNNNNNNNNNNNNNGGNNNNCCNNNNNNNNNNNNNNNNNNNNNNNNNNNNNNN<br>N-5'<br>5'-<br>NNNNNNNNNNNNNNNNNNNNCCNNNGGNNNNNNNNNNNNNNNNNNNNNNNNNNNNNNNN<br>N-3'<br>3'-<br>NNNNNNNNNNNNNNNNNNNNGGNNNCCNNNNNNNNNNNNNNNNNNNNNNNNNNNNNNNN<br>N-5'<br>5'-<br>NNNNNNNNNNNNNNNNNNNNCCNNGGNNNNNNNNNNNNNNNNNNNNNNNNNNNNNNNNN<br>N-3'<br>3'-<br>NNNNNNNNNNNNNNNNNNNNGGNNCCNNNNNNNNNNNNNNNNNNNNNNNNNNNNNNNNN<br>N-5'<br>5'-<br>NNNNNNNNNNNNNNNNNNNNCCNGGNNNNNNNNNNNNNNNNNNNNNNNNNNNNNNNNNN<br>N-3'<br>3'-<br>NNNNNNNNNNNNNNNNNNNNGGNCCNNNNNNNNNNNNNNNNNNNNNNNNNNNNNNNNNN<br>N-5'<br>5'-<br>NNNNNNNNNNNNNNNNNNNNNCCGGNNNNNNNNNNNNNNNNNNNNNNNNNNNNNNNNNN<br>N-3'<br>3'-<br>NNNNNNNNNNNNNNNNNNNNNGGCCNNNNNNNNNNNNNNNNNNNNNNNNNNNNNNNNNN<br>N-5'<br>5'-<br>NNNNNNNNNNNNNNNNNNNNNNNGGNNNNNNNNNNNNNNNNNNNNNNNNNNNNNNNNNN<br>N-3'<br>3'-<br>NNNNNNNNNNNNNNNNNNNNNNNCCGGNNNNNNNNNNNNNNNNNNNNNNNNNNNNNNNN |
| | N-5'<br>5'-<br>NNNNNNNNNNNNNNNNNNNNNNCGGNNNNNNNNNNNNNNNNNNNNNNNNNNNNNNNNNN<br>N-3' |

(continued)

| Overhang length (bp) | Guide RNA design (guide sequence and PAM color coded) |
|---|---|
| | 3'-<br>NNNNNNNNNNNNNNNNNNNNNNNCCNGGNNNNNNNNNNNNNNNNNNNNNNNNNNNNNNN<br>N-5'<br>5'-<br>NNNNNNNNNNNNNNNNNNNNNNCGGNNNNNNNNNNNNNNNNNNNNNNNNNNNNNNNNNN<br>N-3'<br>3'-<br>NNNNNNNNNNNNNNNNNNNNNNNCCNNGGNNNNNNNNNNNNNNNNNNNNNNNNNNNNNN<br>N-5'<br>5'-<br>NNNNNNNNNNNNNNNNNNNNNNCGGNNNNNNNNNNNNNNNNNNNNNNNNNNNNNNNNNN<br>N-3'<br>3'-<br>NNNNNNNNNNNNNNNNNNNNNNNCCNNNGGNNNNNNNNNNNNNNNNNNNNNNNNNNNNN<br>N-5'<br>5'-<br>NNNNNNNNNNNNNNNNNNNNNNCGGNNNNNNNNNNNNNNNNNNNNNNNNNNNNNNNNNN<br>N-3'<br>3'-<br>NNNNNNNNNNNNNNNNNNNNNNNCCNNNNGGNNNNNNNNNNNNNNNNNNNNNNNNNNNN<br>N-5'<br>5'-<br>NNNNNNNNNNNNNNNNNNNNNNCGGNNNNNNNNNNNNNNNNNNNNNNNNNNNNNNNNNN<br>N-3'<br>3'-<br>NNNNNNNNNNNNNNNNNNNNNNNCCNNNNNGGNNNNNNNNNNNNNNNNNNNNNNNNNNN<br>N-5' |

Further interrogation of the system has given evidence of the 5' overhang (see, e.g., Ran et al., Cell. 2013 Sep 12;154(6):1380-9 and US Provisional Patent Application Serial No. 61/871,301 filed August 28, 2013). There are also parameters that relate to efficient cleavage by the Cas9 nickase mutant when combined with two guide RNAs and these parameters include but are not limited to the length of the 5' overhang. In embodiments of the invention the 5' overhang is at most 200 base pairs, preferably at most 100 base pairs, or more preferably at most 50 base pairs. In embodiments of the invention the 5' overhang is at least 26 base pairs, preferably at least 30 base pairs or more preferably 34-50 base pairs or 1-34 base pairs. In other preferred methods of the invention the first guide sequence directing cleavage of one strand of the DNA duplex near the first target sequence and the second guide sequence directing cleavage of the opposite strand near the second target sequence results in a blunt cut or a 3' overhang. In embodiments of the invention the 3' overhang is at most 150, 100 or 25 base pairs or at least 15, 10 or 1 base pairs. In preferred embodiments the 3' overhang is 1-100 base pairs (and in this instance "base pairs" can mean nucleotides or nt or nts).

Aspects of the invention relate to the expression of the gene product being decreased or a template polynucleotide being further introduced into the DNA molecule encoding the gene product or an intervening sequence being excised precisely by allowing the two 5' overhangs to reanneal and ligate or the activity or function of the gene product being altered or the expression of the gene product being increased. In an embodiment of the invention, the gene product is a protein.

Only sgRNA pairs creating 5' overhangs with less than 8bp overlap between the guide sequences (offset greater than -8 bp) were able to mediate detectable indel formation. Importantly, each guide used in these assays is able to efficiently induce indels when paired with wildtype Cas9, indicating that the relative positions of the guide pairs are the most important parameters in predicting double nicking activity.

Since Cas9n and Cas9H840A nick opposite strands of DNA, substitution of Cas9n with Cas9H840A with a given sgRNA pair should result in the inversion of the overhang type. For example, a pair of sgRNAs that will generate a 5' overhang with Cas9n should in principle generate the corresponding 3' overhang instead. Therefore, sgRNA pairs that lead to the generation of a 3' overhang with Cas9n might be used with another mutated Cas9 to generate a 5' overhang, and hence double nicking. Unexpectedly, Applicants tested Cas9H840A with a set of sgRNA pairs designed to generate both 5' and 3' overhangs (offset range from -278 to +58 bp), but were unable to observe indel formation. While one may further study, sgRNA pairing to allow double nicking by Cas9H840A, it is apparent from the data that in contrast to the biochemical or prokaryotic systems in the eukaryotic system, the Cas9H840A mutation can render Cas9 a "dead Cas9", i.e., a CRISPR enzyme substantially lacking all DNA cleavage activity (which is when the DNA cleavage activity of the mutated enzyme is about no more than 25%, 10%, 5%, 1%, 0.1%, 0.01%, or less of the DNA cleavage activity of the non-mutated form of the enzyme; whereby an example can be when the DNA cleavage activity of the mutated form is nil or negligible as compared with the non-mutated form, e.g., when no indel formation is observed as with Cas9H840A).

Liver, proprotein convertase subtilisin kexin 9 (PCSK9)

Proprotein convertase subtilisin kexin 9 (PCSK9) is a member of the subtilisin serine protease family. PCSK9 is primarily expressed by the liver and is critical for the down regulation of hepatocyte LDL receptor expression. LDL-C levels in plasma are highly elevated in humans with gain of function mutations in PCSK9, classifying them as having severe hypercholesterolemia. Therefore, PCSK9 is an attractive target for CRISPR. PCS9K-targeted CRISPR may be formulated in a lipid particle and for example administered at about 15, 45, 90, 150, 250 and 400 μg/kg intraveneously (see, e.g., http://www.alnylam.com/capella/wp-content/uploads/2013/08/ALN-PCS02-001-Protocol-Lancet.pdf).

Bailey et al. (J Mol Med (Berl). 1999 Jan;77(1):244-9) discloses insulin delivery by ex-vivo somatic cell gene therapy involves the removal of non-B-cell somatic cells (e.g. fibroblasts) from a diabetic patient, and genetically altering them in vitro to produce and secrete insulin. The cells can be grown in culture and returned to the donor as a source of insulin replacement. Cells modified in this way could be evaluated before implantation, and reserve stocks could be cryopreserved. By using the patient's own cells, the procedure should obviate the need for immunosuppression and overcome the problem of tissue supply, while avoiding a recurrence of cell destruction. Ex-vivo somatic cell gene therapy requires an accessible and robust cell type that is amenable to multiple transfections and subject to controlled proliferation. Special problems associated with the use of non-B-cell somatic cells include the processing of proinsulin to insulin, and the conferment of sensitivity to glucose-stimulated proinsulin biosynthesis and regulated insulin release. Preliminary studies using fibroblasts, pituitary cells, kidney (COS) cells and ovarian (CHO) cells show that these challenges could be met, and that ex-vivo somatic cell gene therapy offers a feasible approach to insulin replacement therapy. The system of Bailey et al. may be used/and or adapted to the CRISPR Cas system of the present invention for delivery to the liver.

The methods of Sato et al. (Nature Biotechnology Volume 26 Number 4 April 2008, Pages 431-442) may be applied to the CRISPR Cas system of the present invention for delivery to the liver. Sato et al. found that treatments with the siRNA-bearing vitamin A-coupled liposomes almost completely resolved liver fibrosis and prolonged survival in rats with otherwise lethal dimethylnitrosamine-induced liver cirrhosis in a dose- and duration-dependent manner. Cationic liposomes (Lipotrust) containing O,O'-ditetradecanoyl-N-(a-trimethylammonioacetyl) diethanolamine chloride (DC-6-14) as a cationic lipid, cholesterol and dioleoylphosphatidylethanolamine at a molar ratio of 4:3:3 (which has shown high transfection efficiency under serumcontaining conditions for in vitro and in vivo gene delivery) were purchased from Hokkaido System Science. The liposomes were manufactured using a freeze-dried empty liposomes method and prepared at a concentration of 1 mM (DC-16-4) by addition of double-distilled water (DDW) to the lyophilized lipid mixture under vortexing before use. To prepare VA-coupled liposomes, 200 nmol of vitamin A (retinol, Sigma) dissolved in DMSO was mixed with the liposome suspensions (100 nmol as DC-16-4) by vortexing in a 1.5 ml tube at 25 1C. To prepare VA-coupled liposomes carrying siRNAgp46 (VA-lip-siRNAgp46), a solution of siRNAgp46 (580 pmol/ml in DDW) was added to the retinol-coupled liposome solution with stirring at 25 C. The ratio of siRNA to DC-16-4 was 1:11.5 (mol/mol) and the siRNA to liposome ratio (wt/wt) was 1:1. Any free vitamin A or siRNA that was not taken up by liposomes were separated from liposomal preparations using a micropartition system (VIVASPIN 2 concentrator 30,000 MWCO PES, VIVASCIENCE). The liposomal suspension was added to the filters and centrifuged at 1,500g for 5 min 3 times at 25 1C. Fractions were collected and the material trapped in the filter was reconstituted with PBS to achieve the desired dose for in vitro or in vivo use. Three injections of 0.75 mg/kg siRNA were given every other day to rats. The system of Sato et al. may be used/and or adapted to the CRISPR Cas system of the present invention for delivery to the liver by delivering about 0.5 to 1 mg/kg of CRISPR Cas RNA in the liposomes as described by Sato et al. to humans.

The methods of Rozema et al. (PNAS, August 7, 2007, vol. 104, no. 32) for a vehicle for the delivery of siRNA to hepatocytes both in vitro and in vivo, which Rozema et al. have named siRNA Dynamic PolyConjugates may also be applied to the present invention. Key features of the Dynamic Poly-Conjugate technology include a membrane-active polymer, the ability to reversibly mask the activity of this polymer until it reaches the acidic environment of endosomes, and the ability to target this modified polymer and its siRNA cargo specifically to hepatocytes in vivo after simple, low-pressure i.v. injection. SATA-modified siRNAs are synthesized by reaction of 5' aminemodified siRNA with 1 weight equivalents (wt eq) of Nsuccinimidyl-S-acetylthioacetate (SATA) reagent (Pierce) and 0.36 wt eq of $NaHCO_3$ in water at 4°C for 16 h. The modified siRNAs are then precipitated by the addition of 9 vol of ethanol and incubation at 80°C for 2 h. The precipitate is resuspended in IX siRNA buffer (Dharmacon) and quantified by measuring absorbance at the 260-nm wavelength. PBAVE (30 mg/ml in 5mMTAPS, pH 9) is modified by addition of 1.5 wt % SMPT (Pierce). After a 1-h incubation, 0.8 mg of SMPT-PBAVE was added to 400 μl of isotonic glucose solution containing 5 mM TAPS (pH 9). To this solution was added 50 μg of SATA-modified siRNA. For the dose-response experiments where [PBAVE] was constant, different amounts of siRNA are added. The mixture is then incubated for 16 h. To the solution is then added 5.6 mg of Hepes free base followed by a mixture of 3.7 mg ofCDM-NAGand 1.9mg of CDM-PEG. The solution is then incubated for at least 1 h at room temperature before injection. CDM-PEG and CDM-NAG are synthesized from the acid chloride generated by using oxalyl chloride. To the acid chloride is added 1.1 molar equivalents polyethylene glycol monomethyl ether (molecular weight average of 450) to generate CDM-PEG or (aminoethoxy)ethoxy-2-(acetylamino)-2-deoxy-β-D-glucopyranoside to generate CDM-NAG. The final product is purified by using reverse-phase HPLC with a 0.1% TFA water/acetonitrile gradient. About 25 to 50 μg of siRNA was delivered to mice. The system of Rozema et al. may be applied to the CRISPR Cas system of the present invention for delivery to the liver, for example by envisioning a dosage of about 50 to about 200 mg of CRISPR Cas for delivery to a human.

## Bone

Oakes and Lieberman (Clin Orthop Relat Res. 2000 Oct;(379 Suppl):S101-12) discusses delivery of genes to the bone. By transferring genes into cells at a specific anatomic site, the osteoinductive properties of growth factors can be used at physiologic doses for a sustained period to facilitate a more significant healing response. The specific anatomic site, the quality of the bone, and the soft-tissue envelope, influences the selection of the target cells for regional gene therapy. Gene therapy vectors delivered to a treatment site in osteoconductive carriers have yielded promising results. Several investigators have shown exciting results using ex vivo and in vivo regional gene therapy in animal models. Such a system may be used/and or adapted to the CRISPR Cas system for delivery to the bone.

## Brain

Delivery options for the brain include encapsulation of CRISPR enzyme and guide RNA in the form of either DNA or RNA into liposomes and conjugating to molecular Trojan horses for trans-blood brain barrier (BBB) delivery. Molecular Trojan horses have been shown to be effective for delivery of B-gal expression vectors into the brain of non-human primates. The same approach can be used to delivery vectors containing CRISPR enzyme and guide RNA. For instance, Xia CF and Boado RJ, Pardridge WM ("Antibody-mediated targeting of siRNA via the human insulin receptor using avidin-biotin technology." Mol Pharm. 2009 May-Jun;6(3):747-51. doi: 10.1021/mp800194) describes how delivery of short interfering RNA (siRNA) to cells in culture, and in vivo, is possible with combined use of a receptor-specific monoclonal antibody (mAb) and avidin-biotin technology. The authors also report that because the bond between the targeting mAb and the siRNA is stable with avidin-biotin technology, and RNAi effects at distant sites such as brain are observed in vivo following an intravenous administration of the targeted siRNA.

Zhang et al. (Mol Ther. 2003 Jan;7(1):11-8.)) describe how expression plasmids encoding reporters such as luciferase were encapsulated in the interior of an "artificial virus" comprised of an 85 nm pegylated immunoliposome, which was targeted to the rhesus monkey brain in vivo with a monoclonal antibody (MAb) to the human insulin receptor (HIR). The HIRMAb enables the liposome carrying the exogenous gene to undergo transcytosis across the blood-brain barrier and endocytosis across the neuronal plasma membrane following intravenous injection. The level of luciferase gene expression in the brain was 50-fold higher in the rhesus monkey as compared to the rat. Widespread neuronal expression of the beta-galactosidase gene in primate brain was demonstrated by both histochemistry and confocal microscopy. The authors indicate that this approach makes feasible reversible adult transgenics in 24 hours. Accordingly, the use of immunoliposome is preferred. These may be used in conjunction with antibodies to target specific tissues or cell surface proteins.

Other means of delivery or RNA are also preferred, such as via nanoparticles (Cho, S., Goldberg, M., Son, S., Xu, Q., Yang, F., Mei, Y., Bogatyrev, S., Langer, R. and Anderson, D., Lipid-like nanoparticles for small interfering RNA delivery to endothelial cells, Advanced Functional Materials, 19: 3112-3118, 2010) or exosomes (Schroeder, A., Levins, C., Cortez, C., Langer, R., and Anderson, D., Lipid-based nanotherapeutics for siRNA delivery, Journal of Internal Medicine, 267: 9-21, 2010, PMID: 20059641).

Indeed, exosomes have been shown to be particularly useful in delivery siRNA, a system with some parallels to the CRISPR system. For instance, El-Andaloussi S, et al. ("Exosome-mediated delivery of siRNA in vitro and in vivo." Nat Protoc. 2012 Dec;7(12):2112-26. doi: 10.1038/nprot.2012.131. Epub 2012 Nov 15.) describe how exosomes are promising tools for drug delivery across different biological barriers and can be harnessed for delivery of siRNA in vitro and in vivo. Their approach is to generate targeted exosomes through transfection of an expression vector, comprising an exosomal protein fused with a peptide ligand. The exosomes are then purify and characterized from transfected cell supernatant, then siRNA is loaded into the exosomes. Delivery or administration according to the invention can be performed with exosomes, in particular but not limited to the brain.

Vitamin E ($\alpha$-tocopherol) may be conjugated with CRISPR Cas and delivered to the brain along with high density lipoprotein (HDL), for example in a similar manner as was done by Uno et al. (HUMAN GENE THERAPY 22:711-719 (June 2011)) for delivering short-interfering RNA (siRNA) to the brain. Mice were infused via Osmotic minipumps (model 1007D; Alzet, Cupertino, CA) filled with phosphate-buffered saline (PBS) or free TocsiBACE or TocsiBACE/HDL and connected with Brain Infusion Kit 3 (Alzet). A brain-infusion cannula was placed about 0.5mm posterior to the bregma at midline for infusion into the dorsal third ventricle. Uno et al. found that as little as 3 nmol of Toc-siRNA with HDL could induce a target reduction in comparable degree by the same ICV infusion method. A similar dosage of CRISPR Cas conjugated to $\alpha$-tocopherol and co-administered with HDL targeted to the brain may be contemplated for humans in the present invention, for example, about 3 nmol to about 3 $\mu$mol of CRISPR Cas targeted to the brain may becontemplated.

Zou et al. ((HUMAN GENE THERAPY 22:465-475 (April 2011)) describes a method of lentiviral-mediated delivery of short-hairpin RNAs targeting PKC$\gamma$ for in vivo gene silencing in the spinal cord of rats. Zou et al. administered about 10 $\mu$l of a recombinant lentivirus having a titer of 1 x $10^9$ transducing units (TU)/ml by an intrathecal catheter. A similar dosage of CRISPR Cas expressed in a lentiviral vector targeted to the brain may be contemplated for humans in the present invention, for example, about 10-50 ml of CRISPR Cas targeted to the brain in a lentivirus having a titer of 1 x $10^9$ transducing units (TU)/ml may becontemplated.

Targeted deletion, therapeutic applications

Targeted deletion of genes is preferred. Examples are exemplified in Example 16. Preferred are, therefore, genes involved in cholesterol biosynthesis, fatty acid biosynthesis, and other metabolic disorders, genes encoding mis-folded proteins involved in amyloid and other diseases, oncogenes leading to cellular transformation, latent viral genes, and genes leading to dominant-negative disorders, amongst other disorders. As exemplified here, Applicants prefer gene delivery of a CRISPR-Cas system to the liver, brain, ocular, epithelial, hematopoetic, or another tissue of a subject or a patient in need thereof, suffering from metabolic disorders, amyloidosis and protein-aggregation related diseases, cellular transformation arising from genetic mutations and translocations, dominant negative effects of gene mutations, latent viral infections, and other related symptoms, using either viral or nanoparticle delivery system.

Therapeutic applications of the CRISPR-Cas system include Glaucoma, Amyloidosis, and Huntington's disease. These are exemplified in Example 18 and the features described therein are preferred alone or in combination.

Another example of a polyglutamine expansion disease that may be treated by the present invention includes spinocerebellar ataxia type 1 (SCA1). Upon intracerebellar injection, recombinant adenoassociated virus (AAV) vectors expressing short hairpin RNAs profoundly improve motor coordination, restored cerebellar morphology and resolved characteristic ataxin-1 inclusions in Purkinje cells of SCA1 mice (see, e.g., Xia et al., Nature Medicine, Vol. 10, No. 8, Aug. 2004). In particular, AAV1 and AAV5 vectors are preferred and AAV titers of about 1 x $10^{12}$ vector genomes/ml are desirable.

As an example, chronic infection by HIV-1 may be treated or prevented. In order to accomplish this, one may generate CRISPR-Cas guide RNAs that target the vast majority of the HIV-1 genome while taking into account HIV-1 strain variants for maximal coverage and effectiveness. One may accomplish delivery of the CRISPR-Cas system by conventional adenoviral or lentiviral-mediated infection of the host immune system. Depending on approach, host immune cells could be a) isolated, transduced with CRISPR-Cas, selected, and re-introduced in to the host or b) transduced in vivo by systemic delivery of the CRISPR-Cas system. The first approach allows for generation of a resistant immune population whereas the second is more likely to target latent viral reservoirs within the host. This is discussed in more detail in the Examples section.

In another example, US Patent Publication No. 20130171732 assigned to Sangamo BioSciences, Inc. relates to insertion of an anti-HIV transgene into the genome, methods of which may be applied to the CRISPR Cas system of the present invention. In another embodiment, the CXCR4 gene may be targeted and the TALE system of US Patent Publication No. 20100291048 assigned to Sangamo BioSciences, Inc. may be modified to the CRISPR Cas system of the present invention. The method of US Patent Publication Nos. 20130137104 and 20130122591 assigned to Sangamo BioSciences, Inc. and US Patent Publication No. 20100146651 assigned to Cellectis may be more generally applicable for transgene expression as it involves modifying a hypoxanthine-guanine phosphoribosyltransferase (HPRT) locus for increasing the frequency of gene modification.

It is also envisaged that the present invention generates a gene knockout cell library. Each cell may have a single gene knocked out. This is exemplified in Example 21.

One may make a library of ES cells where each cell has a single gene knocked out, and the entire library of ES cells will have every single gene knocked out. This library is useful for the screening of gene function in cellular processes as well as diseases. To make this cell library, one may integrate Cas9 driven by an inducible promoter (e.g. doxycycline inducible promoter) into the ES cell. In addition, one may integrate a single guide RNA targeting a specific gene in the ES cell. To make the ES cell library, one may simply mix ES cells with a library of genes encoding guide RNAs targeting each gene in the human genome. One may first introduce a single BxB1 attB site into the AAVS1 locus of the human ES cell. Then one may use the BxB1 integrase to facilitate the integration of individual guide RNA genes into the BxB1 attB site in AAVS1 locus. To facilitate integration, each guide RNA gene may be contained on a plasmid that carries of a single attP site. This way BxB1 will recombine the attB site in the genome with the attP site on the guide RNA containing plasmid. To generate the cell library, one may take the library of cells that have single guide RNAs integrated and induce Cas9 expression. After induction, Cas9 mediates double strand break at sites specified by the guide RNA.

Chronic administration of protein therapeutics may elicit unacceptable immune responses to the specific protein. The immunogenicity of protein drugs can be ascribed to a few immunodominant helper T lymphocyte (HTL) epitopes. Reducing the MHC binding affinity of these HTL epitopes contained within these proteins can generate drugs with lower immunogenicity (Tangri S, et al. ("Rationally engineered therapeutic proteins with reduced immunogenicity" J Immunol. 2005 Mar 15;174(6):3187-96.) In the present invention, the immunogenicity of the CRISPR enzyme in particular may be reduced following the approach first set out in Tangri et al with respect to erythropoietin and subsequently developed. Accordingly, directed evolution or rational design may be used to reduce the immunogenicity of the CRISPR enzyme (for instance a Cas9) in the host species (human or other species).

In Example 26, Applicants used 3 guideRNAs of interest and able to visualize efficient DNA cleavage *in vivo* occurring only in a small subset of cells. Essentially, what Applicants have shown here is targeted *in vivo* cleavage. In particular, this provides proof of concept that specific targeting in higher organisms such as mammals can also be achieved. It also highlights multiplex aspect in that multiple guide sequences (i.e. separate targets) can be used simultaneously (in the sense of co-delivery). In other words, Applicants used a multiple approach, with several different sequences targeted at the same time, but independently.

Trinucleotide repeat disorders are preferred conditions to be treated. These are also exemplified herein.

For example, US Patent Publication No. 20110016540, describes use of zinc finger nucleases to genetically modify cells, animals and proteins associated with trinucleotide repeat expansion disorders. Trinucleotide repeat expansion disorders are complex, progressive disorders that involve developmental neurobiology and often affect cognition as well as sensori-motor functions.

Trinucleotide repeat expansion proteins are a diverse set of proteins associated with susceptibility for developing a trinucleotide repeat expansion disorder, the presence of a trinucleotide repeat expansion disorder, the severity of a trinucleotide repeat expansion disorder or any combination thereof. Trinucleotide repeat expansion disorders are divided into two categories determined by the type of repeat. The most common repeat is the triplet CAG, which, when present in the coding region of a gene, codes for the amino acid glutamine (Q). Therefore, these disorders are referred to as the polyglutamine (polyQ) disorders and comprise the following diseases: Huntington Disease (HD); Spinobulbar Muscular Atrophy (SBMA); Spinocerebellar Ataxias (SCA types 1, 2, 3, 6, 7, and 17); and Dentatorubro-Pallidoluysian Atrophy (DRPLA). The remaining trinucleotide repeat expansion disorders either do not involve the CAG triplet or the CAG triplet is not in the coding region of the gene and are, therefore, referred to as the non-polyglutamine disorders. The non-polyglutamine disorders comprise Fragile X Syndrome (FRAXA); Fragile XE Mental Retardation (FRAXE); Friedreich Ataxia (FRDA); Myotonic Dystrophy (DM); and Spinocerebellar Ataxias (SCA types 8, and 12).

The proteins associated with trinucleotide repeat expansion disorders are typically selected based on an experimental association of the protein associated with a trinucleotide repeat expansion disorder to a trinucleotide repeat expansion disorder. For example, the production rate or circulating concentration of a protein associated with a trinucleotide repeat expansion disorder may be elevated or depressed in a population having a trinucleotide repeat expansion disorder relative to a population lacking the trinucleotide repeat expansion disorder. Differences in protein levels may be assessed using proteomic techniques including but not limited to Western blot, immunohistochemical staining, enzyme linked immunosorbent assay (ELISA), and mass spectrometry. Alternatively, the proteins associated with trinucleotide repeat expansion disorders may be identified by obtaining gene expression profiles of the genes encoding the proteins using genomic techniques including but not limited to DNA microarray analysis, serial analysis of gene expression (SAGE), and quantitative real-time polymerase chain reaction (Q-PCR).

Non-limiting examples of proteins associated with trinucleotide repeat expansion disorders include AR (androgen receptor), FMR1 (fragile X mental retardation 1), HTT (huntingtin), DMPK (dystrophia myotonica-protein kinase), FXN (frataxin), ATXN2 (ataxin 2), ATN1 (atrophin 1), FEN1 (flap structure-specific endonuclease 1), TNRC6A (trinucleotide repeat containing 6A), PABPN1 (poly(A) binding protein, nuclear 1), JPH3 (junctophilin 3), MED15 (mediator complex subunit 15), ATXN1 (ataxin 1), ATXN3 (ataxin 3), TBP (TATA box binding protein), CACNA1A (calcium channel, voltage-dependent, P/Q type, alpha 1A subunit), ATXN80S (ATXN8 opposite strand (non-protein coding)), PPP2R2B (protein phosphatase 2, regulatory subunit B, beta), ATXN7 (ataxin 7), TNRC6B (trinucleotide repeat containing 6B), TNRC6C (trinucleotide repeat containing 6C), CELF3 (CUGBP, Elav-like family member 3), MAB21L1 (mab-21-like 1 (C. elegans)), MSH2 (mutS homolog 2, colon cancer, nonpolyposis type 1 (E. coli)), TMEM185A (transmembrane protein 185A), SIX5 (SIX homeobox 5), CNPY3 (canopy 3 homolog (zebrafish)), FRAXE (fragile site, folic acid type, rare, fra(X)(q28) E), GNB2 (guanine nucleotide binding protein (G protein), beta polypeptide 2), RPL14 (ribosomal protein L14), ATXN8 (ataxin 8), INSR (insulin receptor), TTR (transthyretin), EP400 (E1A binding protein p400), GIGYF2 (GRB10 interacting GYF protein 2), OGG1 (8-oxoguanine DNA glycosylase), STC1 (stanniocalcin 1), CNDP1 (carnosine dipeptidase 1 (metallopeptidase M20 family)), C10orf2 (chromosome 10 open reading frame 2), MAML3 mastermind-like 3 (Drosophila), DKC1 (dyskeratosis congenita 1, dyskerin), PAXIP1 (PAX interacting (with transcription-activation domain) protein 1), CASK (calcium/calmodulin-dependent serine protein kinase (MAGUK family)), MAPT (microtubule-associated protein tau), SP1 (Sp1 transcription factor), POLG (polymerase (DNA directed), gamma), AFF2 (AF4/FMR2 family, member 2), THBS1 (thrombospondin 1), TP53 (tumor protein p53), ESR1 (estrogen receptor 1), CGGBP1 (CGG triplet repeat binding protein 1), ABT1 (activator of basal transcription 1), KLK3 (kallikrein-related peptidase 3), PRNP (prion protein), JUN (jun oncogene), KCNN3 (potassium intermediate/small conductance calcium-activated channel, subfamily N, member 3), BAX (BCL2-associated X protein), FRAXA (fragile site, folic acid type, rare, fra(X)(q27.3) A (macroorchidism, mental retardation)), KBTBD10 (kelch repeat and BTB (POZ) domain containing 10), MBNL1 (muscleblind-like (Drosophila)), RAD51 (RAD51 homolog (RecA homolog, E. coli) (S. cerevisiae)), NCOA3 (nuclear receptor coactivator 3), ERDA1 (expanded repeat domain, CAG/CTG 1), TSC1 (tuberous sclerosis 1), COMP (cartilage oligomeric matrix protein), GCLC (glutamate-cysteine ligase, catalytic subunit), RRAD (Ras-related associated with diabetes), MSH3 (mutS homolog 3 (E. coli)), DRD2 (dopamine receptor D2), CD44 (CD44 molecule (Indian blood group)), CTCF (CCCTC-binding factor (zinc finger protein)), CCND1 (cyclin D1), CLSPN (claspin homolog (Xenopus laevis)), MEF2A (myocyte enhancer factor 2A), PTPRU (protein tyrosine phosphatase, receptor type, U), GAPDH (glyceraldehyde-3-phosphate dehydrogenase), TRIM22 (tripartite motif-containing 22), WT1 (Wilms tumor 1), AHR (aryl hydrocarbon receptor), GPX1 (glutathione peroxidase 1), TPMT (thiopurine S-methyltransferase), NDP (Norrie disease (pseudoglioma)), ARX (aristaless related homeobox), MUS81 (MUS81 endonuclease homolog (S. cerevisiae)), TYR (tyrosinase (oculocutaneous albinism IA)), EGR1 (early growth response 1), UNG (uracil-DNA glycosylase), NUMBL (numb homolog (Drosophila)-like), FABP2 (fatty acid binding protein 2, intestinal), EN2 (engrailed homeobox 2), CRYGC (crystallin, gamma C), SRP14 (signal recognition particle 14 kDa (homologous Alu RNA binding protein)), CRYGB (crystallin, gamma B), PDCD1 (programmed cell death 1), HOXA1 (homeobox A1), ATXN2L (ataxin 2-like), PMS2 (PMS2 postmeiotic segregation increased 2 (S. cerevisiae)), GLA (galactosidase, alpha), CBL (Cas-Br-M (murine) ecotropic retroviral transforming sequence), FTH1 (ferritin, heavy polypeptide 1), IL12RB2 (interleukin 12 receptor, beta 2), OTX2 (orthodenticle homeobox 2), HOXA5 (homeobox A5), POLG2 (polymerase (DNA directed), gamma 2, accessory subunit), DLX2 (distal-less homeobox 2), SIRPA (signal-regulatory protein alpha), OTX1 (orthodenticle homeobox 1), AHRR (aryl-hydrocarbon receptor repressor), MANF (mesencephalic astrocyte-derived neurotrophic factor), TMEM158 (transmembrane protein 158 (gene/pseudogene)), and ENSG00000078687.

Preferred proteins associated with trinucleotide repeat expansion disorders include HTT (Huntingtin), AR (androgen receptor), FXN (frataxin), Atxn3 (ataxin), Atxn1 (ataxin), Atxn2 (ataxin), Atxn7 (ataxin), Atxn10 (ataxin), DMPK (dystrophia myotonica-protein kinase), Atn1 (atrophin 1), CBP (creb binding protein), VLDLR (very low density lipoprotein receptor), and any combination thereof.

According to another aspect, a method of gene therapy for the treatment of a subject having a mutation in the CFTR gene is provided and comprises administering a therapeutically effective amount of a CRISPR-Cas gene therapy particle, optionally via a biocompatible pharmaceutical carrier, to the cells of a subject. Preferably, the target DNA comprises the mutation deltaF508. In general, it is of preferred that the mutation is repaired to the wildtype. In this case, the mutation is a deletion of the three nucleotides that comprise the codon for phenylalanine (F) at position 508. Accordingly, repair in this instance requires reintroduction of the missing codon into the mutant.

To implement this Gene Repair Strategy, it is preferred that an adenovirus/AAV vector system is introduced into the host cell, cells or patient. Preferably, the system comprises a Cas9 (or Cas9 nickase) and the guide RNA along with a adenovirus/AAV vector system comprising the homology repair template containing the F508 residue. This may be introduced into the subject via one of the methods of delivery discussed earlier. The CRISPR-Cas system may be guided by the CFTRdelta 508 chimeric guide RNA. It targets a specific site of the CFTR genomic locus to be nicked or cleaved. After cleavage, the repair template is inserted into the cleavage site via homologous recombination correcting the deletion that results in cystic fibrosis or causes cystic fibrosis related symptoms. This strategy to direct delivery and provide systemic introduction of CRISPR systems with appropriate guide RNAs can be employed to target genetic mutations to edit or otherwise manipulate genes that cause metabolic, liver, kidney and protein diseases and disorders such as those in Table B.

Genome editing

The CRISPR/Cas9 systems of the present invention can be used to correct genetic mutations that were previously attempted with limited success using TALEN and ZFN. For example, WO2013163628 A2, Genetic Correction of Mutated Genes, published application of Duke University describes efforts to correct, for example, a frameshift mutation which causes a premature stop codon and a truncated gene product that can be corrected via nuclease mediated non-homologous end joining such as those responsible for Duchenne Muscular Dystrophy, ("DMD") a recessive, fatal, X-linked disorder that results in muscle degeneration due to mutations in the dystrophin gene. The majority of dystrophin mutations that cause DMD are deletions of exons that disrupt the reading frame and cause premature translation termination in the dystrophin gene. Dystrophin is a cytoplasmic protein that provides structural stability to the dystroglycan complex of the cell membrane that is responsible for regulating muscle cell integrity and function. The dystrophin gene or "DMD gene" as used interchangeably herein is 2.2 megabases at locus Xp21. The primary transcription measures about 2,400 kb with the mature mRNA being about 14 kb. 79 exons code for the protein which is over 3500 amino acids. Exon 51 is frequently adjacent to frame-disrupting deletions in DMD patients and has been targeted in clinical trials for oligonucleotide-based exon skipping. A clinical trial for the exon 51 skipping compound eteplirsen recently reported a significant functional benefit across 48 weeks, with an average of 47% dystrophin positive fibers compared to baseline. Mutations in exon 51 are ideally suited for permanent correction by NHEJ-based genome editing.

The methods of US Patent Publication No. 20130145487 assigned to Cellectis, which relates to meganuclease variants to cleave a target sequence from the human dystrophin gene (DMD), may also be modified to for the CRISPR Cas system of the present invention.

Blood

The present invention also contemplates delivering the CRISPR-Cas system to the blood.

The plasma exosomes of Wahlgren et al. (Nucleic Acids Research, 2012, Vol. 40, No. 17 e130) may be modified to deliver the CRISPR Cas system to the blood.

The CRISPR Cas system of the present invention is also contemplated to treat hemoglobinopathies, such as thalassemias and sickle cell disease. See, e.g., International Patent Publication No. WO 2013/126794 for potential targets that may be targeted by the CRISPR Cas system of the present invention.

US Patent Publication Nos. 20110225664, 20110091441, 20100229252, 20090271881 and 20090222937 assigned to Cellectis, relates to CREI variants , wherein at least one of the two I-CreI monomers has at least two substitutions, one in each of the two functional subdomains of the LAGLIDADG core domain situated respectively from positions 26 to 40 and 44 to 77 of I-CreI, said variant being able to cleave a DNA target sequence from the human interleukin-2 receptor gamma chain (IL2RG) gene also named common cytokine receptor gamma chain gene or gamma C gene. The target sequences identified in US Patent Publication Nos. 20110225664, 20110091441, 20100229252, 20090271881 and 20090222937 may be utilized for the CRISPR Cas system of the present invention.

Severe Combined Immune Deficiency (SCID) results from a defect in lymphocytes T maturation, always associated with a functional defect in lymphocytes B (Cavazzana-Calvo et al., Annu. Rev. Med., 2005, 56, 585-602; Fischer et al., Immunol. Rev., 2005, 203, 98-109). Overall incidence is estimated to 1 in 75 000 births. Patients with untreated SCID are subject to multiple opportunist micro-organism infections, and do generally not live beyond one year. SCID can be treated by allogenic hematopoietic stem cell transfer, from a familial donor. Histocompatibility with the donor can vary widely. In the case of Adenosine Deaminase (ADA) deficiency, one of the SCID forms, patients can be treated by injection of recombinant Adenosine Deaminase enzyme.

Since the ADA gene has been shown to be mutated in SCID patients (Giblett et al., Lancet, 1972, 2, 1067-1069), several other genes involved in SCID have been identified (Cavazzana-Calvo et al., Annu. Rev. Med., 2005, 56, 585-602; Fischer et al., Immunol. Rev., 2005, 203, 98-109). There are four major causes for SCID: (i) the most frequent form of SCID, SCID-X1 (X-linked SCID or X-SCID), is caused by mutation in the IL2RG gene, resulting in the absence of mature T lymphocytes and NK cells. IL2RG encodes the gamma C protein (Noguchi, et al., Cell, 1993, 73, 147-157), a common component of at least five interleukin receptor complexes. These receptors activate several targets through the JAK3 kinase (Macchi et al., Nature, 1995, 377, 65-68), which inactivation results in the same syndrome as gamma C inactivation; (ii) mutation in the ADA gene results in a defect in purine metabolism that is lethal for lymphocyte precursors, which in turn results in the quasi absence of B, T and NK cells; (iii) V(D)J recombination is an essential step in the maturation of immunoglobulins and T lymphocytes receptors (TCRs). Mutations in Recombination Activating Gene 1 and 2 (RAG1 and RAG2) and Artemis, three genes involved in this process, result in the absence of mature T and B lymphocytes; and (iv) Mutations in other genes such as CD45, involved in T cell specific signaling have also been reported, although they represent a minority of cases (Cavazzana-Calvo et al., Annu. Rev. Med., 2005, 56, 585-602; Fischer et al., Immunol. Rev., 2005, 203, 98-109).

Since when their genetic bases have been identified, the different SCID forms have become a paradigm for gene therapy approaches (Fischer et al., Immunol. Rev., 2005, 203, 98-109) for two major reasons. First, as in all blood diseases, an ex vivo treatment can be envisioned. Hematopoietic Stem Cells (HSCs) can be recovered from bone marrow, and keep their pluripotent properties for a few cell divisions. Therefore, they can be treated in vitro, and then reinjected into the patient, where they repopulate the bone marrow. Second, since the maturation of lymphocytes is impaired in SCID patients, corrected cells have a selective advantage. Therefore, a small number of corrected cells can restore a functional immune system. This hypothesis was validated several times by (i) the partial restoration of immune functions associated with the reversion of mutations in SCID patients (Hirschhorn et al., Nat. Genet., 1996, 13, 290-295; Stephan et al., N. Engl. J. Med., 1996, 335, 1563-1567; Bousso et al., Proc. Natl., Acad. Sci. USA, 2000, 97, 274-278; Wada et al., Proc. Natl. Acad. Sci. USA, 2001, 98, 8697-8702; Nishikomori et al., Blood, 2004, 103, 4565-4572), (ii) the correction of SCID-X1 deficiencies in vitro in hematopoietic cells (Candotti et al., Blood, 1996, 87, 3097-3102; Cavazzana-Calvo et al., Blood, 1996, Blood, 88, 3901-3909; Taylor et al., Blood, 1996, 87, 3103-3107; Hacein-Bey et al., Blood, 1998, 92, 4090-4097), (iii) the correction of SCID-X1 (Soudais et al., Blood, 2000, 95, 3071-3077; Tsai et al., Blood, 2002, 100, 72-79), JAK-3 (Bunting et al., Nat. Med., 1998, 4, 58-64; Bunting et al., Hum. Gene Ther., 2000, 11, 2353-2364) and RAG2 (Yates et al., Blood, 2002, 100, 3942-3949) deficiencies in vivo in animal models and (iv) by the result of gene therapy clinical trials (Cavazzana-Calvo et al., Science, 2000, 288, 669-672; Aiuti et al., Nat. Med., 2002; 8, 423-425; Gaspar et al., Lancet, 2004, 364, 2181-2187).

US Patent Publication No. 20110182867 assigned to the Children's Medical Center Corporation and the President and Fellows of Harvard College relates to methods and uses of modulating fetal hemoglobin expression (HbF) in a hematopoietic progenitor cells via inhibitors of BCL11A expression or activity, such as RNAi and antibodies. The targets disclosed in US Patent Publication No. 20110182867, such as BCL11A, may be targeted by the CRISPR Cas system of the present invention for modulating fetal hemoglobin expression. See also Bauer et al. (Science 11 October 2013: Vol. 342 no. 6155 pp. 253-257) and Xu et al. (Science 18 November 2011: Vol. 334 no. 6058 pp. 993-996) for additional BCL11A targets.

Ears

The present invention also contemplates delivering the CRISPR-Cas system to one or both ears. Researchers are looking into whether gene therapy could be used to aid current deafness treatments-namely, cochlear implants. Deafness is often caused by lost or damaged hair cells that cannot relay signals to auditory neurons. In such cases, cochlear implants may be used to respond to sound and transmit electrical signals to the nerve cells. But these neurons often degenerate and retract from the cochlea as fewer growth factors are released by impaired hair cells. US patent application 20120328580 involves injection of a pharmaceutical composition into the ear (e.g., auricular administration), such as into the luminae of the cochlea (e.g., the Scala media, Sc vestibulae, and Sc tympani), e.g., using a syringe, e.g., a single-dose syringe. For example, one or more of the compounds described herein can be administered by intratympanic injection (e.g., into the middle ear), and/or injections into the outer, middle, and/or inner ear. Such methods are routinely used in the art, for example, for the administration of steroids and antibiotics into human ears. Injection can be, for example, through the round window of the ear or through the cochlear capsule. These and other inner ear administration methods known in the art (see, e.g., Salt and Plontke, Drug Discovery Today, 10:1299-1306, 2005) can also be adapted for the practice of the instant invention.

In another mode of administration, the pharmaceutical composition can be administered in situ, via a catheter or pump. A catheter or pump can, for example, direct a pharmaceutical composition into the cochlear luminae or the round window of the ear and/or the lumen of the colon. Exemplary drug delivery apparatus and methods suitable for administering one or more of the compounds described herein into an ear, e.g., a human ear, are described by McKenna et al., (U.S. Publication No. 2006/0030837) and Jacobsen et al., (U.S. Pat. No. 7,206,639). In some embodiments, a catheter or pump can be positioned, e.g., in the ear (e.g., the outer, middle, and/or inner ear) of a patient during a surgical procedure. In some embodiments, a catheter or pump can be positioned, e.g., in the ear (e.g., the outer, middle, and/or inner ear) of a patient without the need for a surgical procedure. These techniques can be adapted in the practice of the present invention.

Alternatively or in addition, one or more of the compounds described herein can be administered in combination with a mechanical device such as a cochlear implant or a hearing aid, which is worn in the outer ear. An exemplary cochlear implant that is suitable for use with the present invention is described by Edge et al., (U.S. Publication No. 2007/0093878). In some embodiments, the modes of administration described above may be combined in any order and can be simultaneous or interspersed. Alternatively or in addition, the present invention may be administered according to any of the Food and Drug Administration approved methods, for example, as described in CDER Data Standards Manual, version number 004 (which is available at fda.give/cder/dsm/DRG/drg00301.htm).

In general, the cell therapy methods described in US patent application 20120328580 can be used to promote complete or partial differentiation of a cell to or towards a mature cell type of the inner ear (e.g., a hair cell) in vitro. Cells resulting from such methods can then be transplanted or implanted into a patient in need of such treatment. The cell culture methods required to practice these methods, including methods for identifying and selecting suitable cell types, methods for promoting complete or partial differentiation of selected cells, methods for identifying complete or partially differentiated cell types, and methods for implanting complete or partially differentiated cells can be adapted in the practice of the present invention.

Cells suitable for use in the present invention include, but are not limited to, cells that are capable of differentiating completely or partially into a mature cell of the inner ear, e.g., a hair cell (e.g., an inner and/or outer hair cell), when contacted, e.g., in vitro, with one or more of the compounds described herein. Exemplary cells that are capable of differentiating into a hair cell include, but are not limited to stem cells (e.g., inner ear stem cells, adult stem cells, bone marrow derived stem cells, embryonic stem cells, mesenchymal stem cells, skin stem cells, iPS cells, and fat derived stem cells), progenitor cells (e.g., inner ear progenitor cells), support cells (e.g., Deiters' cells, pillar cells, inner phalangeal cells, tectal cells and Hensen's cells), and/or germ cells. The use of stem cells for the replacement of inner ear sensory cells is described in Li et al., (U.S. Publication No. 2005/0287127) and Li et al., (U.S. patent Ser. No. 11/953,797). The use of bone marrow derived stem cells for the replacement of inner ear sensory cells is described in Edge et al., PCT/US2007/084654. iPS cells are described, e.g., at Takahashi et al., Cell, Volume 131, Issue 5, Pages 861-872 (2007); Takahashi and Yamanaka, Cell 126, 663-76 (2006); Okita et al., Nature 448, 260-262 (2007); Yu, J. et al., Science 318(5858):1917-1920 (2007); Nakagawa et al., Nat. Biotechnol. 26:101-106 (2008); and Zaehres and Scholer, Cell 131(5):834-835 (2007).

Such suitable cells can be identified by analyzing (e.g., qualitatively or quantitatively) the presence of one or more tissue specific genes. For example, gene expression can be detected by detecting the protein product of one or more tissue-specific genes. Protein detection techniques involve staining proteins (e.g., using cell extracts or whole cells) using antibodies against the appropriate antigen. In this case, the appropriate antigen is the protein product of the tissue-specific gene expression. Although, in principle, a first antibody (i.e., the antibody that binds the antigen) can be labeled, it is more common (and improves the visualization) to use a second antibody directed against the first (e.g., an anti-IgG). This second antibody is conjugated either with fluorochromes, or appropriate enzymes for colorimetric reactions, or gold beads (for electron microscopy), or with the biotin-avidin system, so that the location of the primary antibody, and thus the antigen, can be recognized.

The CRISPR Cas molecules of the present invention may be delivered to the ear by direct application of pharmaceutical composition to the outer ear, with compositions modified from US Published application, 20110142917. In some embodiments the pharmaceutical composition is applied to the ear canal. Delivery to the ear may also be refered to as aural or otic delivery.

In some embodiments the RNA molecules of the invention are delivered in liposome or lipofectin formulations and the like and can be prepared by methods well known to those skilled in the art. Such methods are described, for example, in U.S. Pat. Nos. 5,593,972, 5,589,466, and 5,580,859, which are herein incorporated by reference.

Delivery systems aimed specifically at the enhanced and improved delivery of siRNA into mammalian cells have been developed, (see, for example, Shen et al FEBS Let. 2003, 539:111-114; Xia et al., Nat. Biotech. 2002, 20:1006-1010; Reich et al., Mol. Vision. 2003, 9: 210-216; Sorensen et al., J. Mol. Biol. 2003, 327: 761-766; Lewis et al., Nat. Gen. 2002, 32: 107-108 and Simeoni et al., NAR 2003, 31, 11: 2717-2724) and may be applied to the present invention. siRNA has recently been successfully used for inhibition of gene expression in primates (see for example. Tolentino et al., Retina 24(4):660 which may also be applied to the present invention.

Qi et al. involves methods for efficient siRNA transfection to the inner ear through the intact round window by a novel proteidic delivery technology which may be applied to the CRISPR Cas system of the present invention (see, e.g., Qi et al., Gene Therapy (2013), 1-9). In particular, a TAT double stranded RNA-binding domains (TAT-DRBDs), which can transfect Cy3-labeled siRNA into cells of the inner ear, including the inner and outer hair cells, crista ampullaris, macula utriculi and macula sacculi, through intact round-window permeation was successful for delivering double stranded siRNAs in vivo for treating various inner ear ailments and preservation of hearing function. About 40 $\mu$l of 10mM RNA may be contemplated as the dosage for administration to the ear.

According to Rejali et al. (Hear Res. 2007 Jun;228(1-2):180-7), cochlear implant function can be improved by good preservation of the spiral ganglion neurons, which are the target of electrical stimulation by the implant and brain derived neurotrophic factor (BDNF) has previously been shown to enhance spiral ganglion survival in experimentally deafened ears. Rejali et al. tested a modified design of the cochlear implant electrode that includes a coating of fibroblast cells transduced by a viral vector with a BDNF gene insert. To accomplish this type of ex vivo gene transfer, Rejali et al. transduced guinea pig fibroblasts with an adenovirus with a BDNF gene cassette insert, and determined that these cells secreted BDNF and then attached BDNF-secreting cells to the cochlear implant electrode via an agarose gel, and implanted the electrode in the scala tympani. Rejali et al. determined that the BDNF expressing electrodes were able to preserve significantly more spiral ganglion neurons in the basal turns of the cochlea after 48 days of implantation when compared to control electrodes and demonstrated the feasibility of combining cochlear implant therapy with ex vivo gene transfer for enhancing spiral ganglion neuron survival. Such a system may be applied to the CRISPR Cas system of the present invention for delivery to the ear.

Mukherjea et al. (Antioxidants & Redox Signaling, Volume 13, Number 5, 2010) document that knockdown of NOX3 using short interfering (si) RNA abrogated cisplatin ototoxicity, as evidenced by protection of OHCs from damage and reduced threshold shifts in auditory brainstem responses (ABRs). Different doses of siNOX3 (0.3, 0.6, and 0.9 μg) were administered to rats and NOX3 expression was evaluated by real time RT-PCR. The lowest dose of NOX3 siRNA used (0.3 μg) did not show any inhibition of NOX3 mRNA when compared to transtympanic administration of scrambled siRNA or untreated cochleae. However, administration of the higher doses of NOX3 siRNA (0.6 and 0.9 μg) reduced NOX3 expression compared to control scrambled siRNA. Such a system may be applied to the CRISPR Cas system of the present invention for transtympanic administration with a dosage of about 2 mg to about 4 mg of CRISPR Cas for administration to a human.

Jung et al. (Molecular Therapy, vol. 21 no. 4, 834-841 apr. 2013) demonstrate that Hes5 levels in the utricle decreased after the application of siRNA and that the number of hair cells in these utricles was significantly larger than following control treatment. The data suggest that siRNA technology may be useful for inducing repair and regeneration in the inner ear and that the Notch signaling pathway is a potentially useful target for specific gene expression inhibition. Jung et al. injected 8 μg of Hes5 siRNA in 2 μl volume, prepared by adding sterile normal saline to the lyophilized siRNA to a vestibular epithelium of the ear. Such a system may be applied to the CRISPR Cas system of the present invention for administration to the vestibular epithelium of the ear with a dosage of about 1 to about 30 mg of CRISPR Cas for administration to a human.

Eyes

The present invention also contemplates delivering the CRISPR-Cas system to one or both eyes. In yet another aspect of the invention, the CRISPR-Cas system may be used to correct ocular defects that arise from several genetic mutations further described in Genetic Diseases of the Eye, Second Edition, edited by Elias I. Traboulsi, Oxford University Press, 2012. For administration to the eye, lentiviral vectors, in particular equine infectious anemia viruses (EIAV) are particularly preferred. In another embodiment, minimal non-primate lentiviral vectors based on the equine infectious anemia virus (EIAV) are also contemplated, especially for ocular gene therapy (see, e.g., Balagaan, J Gene Med 2006; 8: 275 - 285, Published online 21 November 2005 in Wiley InterScience (www.interscience.wiley.com). DOI: 10.1002/jgm.845). The vectors are contemplated to have cytomegalovirus (CMV) promoter driving expression of the target gene. Intracameral, subretinal, intraocular and intravitreal injections are all contemplated (see, e.g., Balagaan, J Gene Med 2006; 8: 275 - 285, Published online 21 November 2005 in Wiley InterScience (www.interscience.wiley.com). DOI: 10.1002/jgm.845). Intraocular injections may be performed with the aid of an operating microscope. For subretinal and intravitreal injections, eyes may be prolapsed by gentle digital pressure and fundi visualised using a contact lens system consisting of a drop of a coupling medium solution on the cornea covered with a glass microscope slide coverslip. For subretinal injections, the tip of a 10-mm 34-gauge needle, mounted on a 5-μl Hamilton syringe may be advanced under direct visualisation through the superior equatorial sclera tangentially towards the posterior pole until the aperture of the needle was visible in the subretinal space. Then, 2 μl of vector suspension may be injected to produce a superior bullous retinal detachment, thus confirming subretinal vector administration. This approach creates a self-sealing sclerotomy allowing the vector suspension to be retained in the subretinal space until it is absorbed by the RPE, usually within 48 h of the procedure. This procedure may be repeated in the inferior hemisphere to produce an inferior retinal detachment. This technique results in the exposure of approximately 70% of neurosensory retina and RPE to the vector suspension. For intravitreal injections, the needle tip may be advanced through the sclera 1 mm posterior to the corneoscleral limbus and 2 μl of vector suspension injected into the vitreous cavity. For intracameral injections, the needle tip may be advanced through a corneoscleral limbal paracentesis, directed towards the central cornea, and 2 μl of vector suspension may be injected. For intracameral injections, the needle tip may be advanced through a corneoscleral limbal paracentesis, directed towards the central cornea, and 2 μl of vector suspension may be injected. These vectors may be injected at titres of either 1.0-1.4 $\times$ $10^{10}$ or 1.0-1.4 $\times$ $10^9$ transducing units (TU)/ml. In another embodiment, RetinoStat®, an equine infectious anemia virus-based lentiviral gene therapy vector that expresses angiostatic proteins endostain and angiostatin that is delivered via a subretinal injection for the treatment of the web form of age-related macular degeneration is also contemplated (see, e.g., Binley et al., HUMAN GENE THERAPY 23:980-991 (September 2012)). Such a vector may be modified for the CRISPR-Cas system of the present invention. Each eye may be treated with either RetinoStat® at a dose of 1.1 x $10^5$ transducing units per eye (TU/eye) in a total volume of 100 μl.

In another embodiment, an E1-, partial E3-, E4-deleted adenoviral vector may be contemplated for delivery to the eye. Twenty-eight patients with advanced neovascular age-related macular degeneration (AMD) were given a single intravitreous injection of an E1-, partial E3-, E4-deleted adenoviral vector expressing human pigment ep- ithelium-derived factor (AdPEDF.ll) (see, e.g., Campochiaro et al., Human Gene Therapy 17:167-176 (February 2006)). Doses ranging

from $10^6$ to $10^{9.5}$ particle units (PU) were investigated and there were no serious adverse events related to AdPEDF.ll and no dose-limiting toxicities (see, e.g., Campochiaro et al., Human Gene Therapy 17:167-176 (February 2006)). Adenoviral vector-mediated ocular gene transfer appears to be a viable approach for the treatment of ocular disorders and could be applied to the CRISPR Cas system.

In another embodiment, the sd-rxRNA® system of RXi Pharmaceuticals may be used/and or adapted for delivering CRISPR Cas to the eye. In this system, a single intravitreal administration of 3 $\mu$g of sd-rxRNA results in sequence-specific reduction of PPIB mRNA levels for 14 days. The the sd-rxRNA® system may be applied to the CRISPR Cas system of the present invention, contemplating a dose of about 3 to 20 mg of CRISPR administered to a human.

Millington-Ward et al. (Molecular Therapy, vol. 19 no. 4, 642-649 apr. 2011) describes adeno-associated virus (AAV) vectors to deliver an RNA interference (RNAi)-based rhodopsin suppressor and a codon-modified rhodopsin replacement gene resistant to suppression due to nucleotide alterations at degenerate positions over the RNAi target site. An injection of either 6.0 x $10^8$ vp or 1.8 x $10^{10}$ vp AAV were subretinally injected into the eyes by Millington-Ward et al. The AAV vectors of Millington-Ward et al. may be applied to the CRISPR Cas system of the present invention, contemplating a dose of about 2 x $10^{11}$ to about 6 x $10^{13}$ vp administered to a human. Dalkara et al. (Sci Transl Med 5, 189ra76 (2013)) also relates to in vivo directed evolution to fashion an AAV vector that delivers wild-type versions of defective genes throughout the retina after noninjurious injection into the eyes' vitreous humor. Dalkara describes a a 7mer peptide display library and an AAV library constructed by DNA shuffling of cap genes from AAV1, 2, 4, 5, 6, 8, and 9. The rcAAV libraries and rAAV vectors expressing GFP under a CAG or Rho promoter were packaged and and deoxyribonuclease-resistant genomic titers were obtained through quantitative PCR. The libraries were pooled, and two rounds of evolution were performed, each consisting of initial library diversification followed by three in vivo selection steps. In each such step, P30 rho-GFP mice were intravitreally injected with 2 ml of iodixanol-purified, phosphate-buffered saline (PBS)-dialyzed library with a genomic titer of about 1 $\times$ $10^{12}$ vg/ml. The AAV vectors of Dalkara et al. may be applied to the CRISPR Cas system of the present invention, contemplating a dose of about 1 x $10^{15}$ to about 1 x $10^{16}$ vg/ml administered to a human. These techniques can be adapted in the practice of the instant invention.

In another embodiment, the rhodopsin gene may be targeted for the treatment of retinitis pigmentosa (RP), wherein the system of US Patent Publication No. 20120204282 assigned to Sangamo BioSciences, Inc. may be modified in accordance of the CRISPR Cas system of the present invention. In another embodiment, the methods of US Patent Publication No. 20130183282 assigned to Cellectis, which is directed to methods of cleaving a target sequence from the human rhodopsin gene, may also be modified to the CRISPR Cas system of the present invention. US Patent Publication No. 20130202678 assigned to Academia Sinica relates to methods for treating retinopathies and sight-threatening ophthalmologic disorders relating to delivering of the Puf-A gene (which is expressed in retinal ganglion and pigmented cells of eye tissues and displays a unique anti-apoptotic activity) to the sub-retinal or intravitreal space in the eye. In particular, desirable targets are zgc: 193933, prdm1a, spata2, tex10, rbb4, ddx3, zp2.2, Blimp-1 and HtrA2, all of which may be targeted by the CRISPR Cas system of the present invention. Wu (Cell Stem Cell,13:659-62, 2013) designed a guide RNA that led Cas9 to a single base pair mutation that causes cataracts in mice, where it induced DNA cleavage. Then using either the other wild-type allele or oligos given to the zygotes repair mechanisms corrected the sequence of the broken allele and corrected the cataract-causing genetic defect in mutant mouse. This can be adapted in the practice of the instant invention. US Patent Publication No. 20120159653, describes use of zinc finger nucleases to genetically modify cells, animals and proteins associated with macular degeration (MD). Macular degeneration (MD) is the primary cause of visual impairment in the elderly, but is also a hallmark symptom of childhood diseases such as Stargardt disease, Sorsby fundus, and fatal childhood neurodegenerative diseases, with an age of onset as young as infancy. Macular degeneration results in a loss of vision in the center of the visual field (the macula) because of damage to the retina. Currently existing animal models do not recapitulate major hallmarks of the disease as it is observed in humans. The available animal models comprising mutant genes encoding proteins associated with MD also produce highly variable phenotypes, making translations to human disease and therapy development problematic. One aspect of US Patent Publication No. 20120159653 relates to editing of any chromosomal sequences that encode proteins associated with MD which may be applied to the CRISPR Cas system of the present invention. The proteins associated with MD are typically selected based on an experimental association of the protein associated with MD to an MD disorder. For example, the production rate or circulating concentration of a protein associated with MD may be elevated or depressed in a population having an MD disorder relative to a population lacking the MD disorder. Differences in protein levels may be assessed using proteomic techniques including but not limited to Western blot, immunohistochemical staining, enzyme linked immunosorbent assay (ELISA), and mass spectrometry. Alternatively, the proteins associated with MD may be identified by obtaining gene expression profiles of the genes encoding the proteins using genomic techniques including but not limited to DNA microarray analysis, serial analysis of gene expression (SAGE), and quantitative real-time polymerase chain reaction (Q-PCR). By way of non-limiting example, proteins associated with MD include but are not limited to the following proteins: (ABCA4) ATP-binding cassette, sub-family A (ABC1), member 4 ACHM1 achromatopsia (rod monochromacy) 1 ApoE Apolipoprotein E (ApoE) C1QTNF5 (CTRP5) C1q and tumor necrosis factor related protein 5 (C1QTNF5) C2 Complement component 2 (C2) C3 Complement components (C3) CCL2 Chemokine (C-C motif) Ligand 2 (CCL2) CCR2 Chemokine (C-C motif) receptor 2 (CCR2) CD36 Cluster of Differentiation 36 CFB Complement factor B CFH Complement factor CFH H CFHR1 complement factor H-related 1 CFHR3 complement factor H-related 3 CNGB3 cyclic nucleotide gated channel beta 3 CP ceruloplasmin (CP) CRP C reactive protein (CRP) CST3 cystatin C or cystatin 3 (CST3) CTSD Cathepsin D (CTSD) CX3CR1 chemokine (C-X3-C motif) receptor 1 ELOVL4 Elongation of very long chain fatty acids 4 ERCC6 excision repair cross- complementing rodent repair deficiency, complementation group 6 FBLN5 Fibulin-5 FBLN5 Fibulin 5 FBLN6 Fibulin 6 FSCN2 fascin (FSCN2) HMCN1 Hemicentrin 1 HMCN1 hemicentin 1 HTRA1 HtrA serine peptidase 1 (HTRA1) HTRA1 HtrA serine peptidase 1 IL-6 Interleukin 6 IL-8 Interleukin 8 LOC387715 Hypothetical protein PLEKHA1 Pleckstrin homology domain-containing family A member 1 (PLEKHA1) PROM1 Prominin 1(PROM1 or CD133) PRPH2 Peripherin-2 RPGR retinitis pigmentosa GTPase regulator SERPING1 serpin peptidase inhibitor, clade G, member 1 (C1- inhibitor) TCOF1 Treacle TIMP3 Metalloproteinase inhibitor 3 (TIMP3) TLR3 Toll-like receptor 3. The identity of the protein associated with MD whose chromosomal sequence is edited can and will vary. In preferred embodiments, the CRISPR-Cas system is applied as to proteins associated with MD whose chromosomal sequence is edited may be the ATP-binding cassette, sub-family A (ABC1) member 4 protein (ABCA4) encoded by the ABCR gene, the apolipoprotein E protein (APOE) encoded by the APOE gene, the chemokine (C-C motif) Ligand 2 protein (CCL2) encoded by the CCL2 gene, the chemokine (C-C motif) receptor 2 protein (CCR2) encoded by the CCR2 gene, the ceruloplasmin protein (CP) encoded by the CP gene, the cathepsin D protein (CTSD) encoded by the CTSD gene, or the metalloproteinase inhibitor 3 protein (TIMP3) encoded by the TIMP3 gene. In an exemplary embodiment, the genetically modified animal is a rat, and the edited chromosomal sequence encoding the protein associated with MD may be: (ABCA4) ATP-binding cassette, NM_000350 sub-family A (ABC1), member 4 APOE Apolipoprotein E NM_138828 (APOE) CCL2 Chemokine (C-C NM_031530 motif) Ligand 2 (CCL2) CCR2 Chemokine (C-C NM_021866 motif) receptor 2 (CCR2) CP ceruloplasmin (CP) NM_012532 CTSD Cathepsin D (CTSD) NM_134334 TIMP3 Metalloproteinase NM_012886 inhibitor 3 (TIMP3). The animal or cell may comprise 1, 2, 3, 4, 5, 6, 7 or more disrupted chromosomal sequences encoding a protein associated with MD and zero, 1, 2, 3, 4, 5, 6, 7 or more chromosomally integrated sequences encoding the disrupted protein associated with MD. The edited or integrated chromosomal sequence may be modified to encode an altered protein associated with MD. Several mutations in MD-related chromosomal sequences have been associated with MD. Non-limiting examples of mutations in chromosomal sequences associated with MD include those that may cause MD including in the ABCR protein, E471K (i.e. glutamate at position 471 is changed to lysine), R1129L (i.e. arginine at position 1129 is changed to leucine), T1428M (i.e. threonine at position 1428 is changed to methionine), R1517S (i.e. arginine at position 1517 is changed to serine), I1562T (i.e. isoleucine at position 1562 is changed to threonine), and G1578R (i.e. glycine at position 1578 is changed to arginine); in the CCR2 protein, V64I (i.e. valine at position 192 is changed to isoleucine); in CP protein, G969B (i.e. glycine at position 969 is changed to asparagine or aspartate); in TIMP3 protein, S156C (i.e. serine at position 156 is changed to cysteine), G166C (i.e. glycine at position 166 is changed to cysteine), G167C (i.e. glycine at position 167 is changed to cysteine), Y168C (i.e. tyrosine at position 168 is changed to cysteine), S170C (i.e. serine at position 170 is changed to cysteine), Y172C (i.e. tyrosine at position 172 is changed to cysteine) and S181C (i.e. serine at position 181 is changed to cysteine). Other associations of genetic variants in MD-associated genes and disease are known in the art, and can be targets as to the CRISPR-Cas system, e.g., to correct mutation.

Heart

The present invention also contemplates delivering the CRISPR-Cas system to the heart. For the heart, a myocardium tropic adena-associated virus (AAVM) is preferred, in particular AAVM41 which showed preferential gene transfer in the heart (see, e.g., Lin-Yanga et al., PNAS, March 10, 2009, vol. 106, no. 10). Administration may be systemic or local. A dosage of about 1-10 x $10^{14}$ vector genomes are contemplated for systemic administration. See also, e.g., Eulalio et al. (2012) Nature 492: 376 and Somasuntharam et al. (2013) Biomaterials 34: 7790.

For example, US Patent Publication No. 20110023139, describes use of zinc finger nucleases to genetically modify cells, animals and proteins associated with cardiovascular disease. Cardiovascular diseases generally include high blood pressure, heart attacks, heart failure, and stroke and TIA. Any chromosomal sequence involved in cardiovascular disease or the protein encoded by any chromosomal sequence involved in cardiovascular disease may be utilized in the methods described in this disclosure. The cardiovascular-related proteins are typically selected based on an experimental association of the cardiovascular-related protein to the development of cardiovascular disease. For example, the production rate or circulating concentration of a cardiovascular-related protein may be elevated or depressed in a population having a cardiovascular disorder relative to a population lacking the cardiovascular disorder. Differences in protein levels may be assessed using proteomic techniques including but not limited to Western blot, immunohistochemical staining, enzyme linked immunosorbent assay (ELISA), and mass spectrometry. Alternatively, the cardiovascular-related proteins may be identified by obtaining gene expression profiles of the genes encoding the proteins using genomic techniques including but not limited to DNA microarray analysis, serial analysis of gene expression (SAGE), and quantitative real-time polymerase chain reaction (Q-PCR). By way of example, the chromosomal sequence may comprise, but is not limited to, IL1B (interleukin 1, beta), XDH (xanthine dehydrogenase), TP53 (tumor protein p53), PTGIS (prostaglandin 12 (prostacyclin) synthase), MB (myoglobin), IL4 (interleukin 4), ANGPT1 (angiopoietin 1), ABCG8 (ATP-binding cassette, sub-family G (WHITE), member 8), CTSK (cathepsin K), PTGIR (prostaglandin 12 (prostacyclin) receptor (IP)), KCNJ11 (potassium inwardly-rectifying channel, subfamily J, member 11), INS (insulin), CRP (C-reactive protein, pentraxin-related), PDGFRB (platelet-derived growth factor receptor, beta polypeptide), CCNA2 (cyclin A2), PDGFB (platelet-derived growth factor beta polypeptide (simian sarcoma viral (v-sis) oncogene homolog)), KCNJ5 (potassium inwardly-rectifying channel, subfamily J, member 5), KCNN3 (potassium intermediate/small conductance calcium-activated channel, subfamily N, member 3), CAPN10 (calpain 10), PTGES (prostaglandin E synthase), ADRA2B (adrenergic, alpha-2B-, receptor), ABCG5 (ATP-binding cassette, sub-family G (WHITE), member 5), PRDX2 (peroxiredoxin 2), CAPN5 (calpain 5), PARP14 (poly (ADP-ribose) polymerase family, member 14), MEX3C (mex-3 homolog C (C. elegans)), ACE angiotensin I converting enzyme (peptidyl-dipeptidase A) 1), TNF (tumor necrosis factor (TNF superfamily, member 2)), IL6 (interleukin 6 (interferon, beta 2)), STN (statin), SERPINE1 (serpin peptidase inhibitor, clade E (nexin, plasminogen activator inhibitor type 1), member 1), ALB (albumin), ADIPOQ (adiponectin, C1Q and collagen domain containing), APOB (apolipoprotein B (including Ag(x) antigen)), APOE (apolipoprotein E), LEP (leptin), MTHFR (5,10-methylenetetrahydrofolate reductase (NADPH)), APOA1 (apolipoprotein A-I), EDN1 (endothelin 1), NPPB (natriuretic peptide precursor B), NOS3 (nitric oxide synthase 3 (endothelial cell)), PPARG (peroxisome proliferator-activated receptor gamma), PLAT (plasminogen activator, tissue), PTGS2 (prostaglandin-endoperoxide synthase 2 (prostaglandin G/H synthase and cyclooxygenase)), CETP (cholesteryl ester transfer protein, plasma), AGTR1 (angiotensin II receptor, type 1), HMGCR (3-hydroxy-3-methylglutaryl-Coenzyme A reductase), IGF1 (insulin-like growth factor 1 (somatomedin C)), SELE (selectin E), REN (renin), PPARA (peroxisome proliferator-activated receptor alpha), PON1 (paraoxonase 1), KNG1 (kininogen 1), CCL2 (chemokine (C-C motif) ligand 2), LPL (lipoprotein lipase), VWF (von Willebrand factor), F2 (coagulation factor II (thrombin)), ICAM1 (intercellular adhesion molecule 1), TGFB1 (transforming growth factor, beta 1), NPPA (natriuretic peptide precursor A), IL10 (interleukin 10), EPO (erythropoietin), SOD1 (superoxide dismutase 1, soluble), VCAM1 (vascular cell adhesion molecule 1), IFNG (interferon, gamma), LPA (lipoprotein, Lp(a)), MPO (myeloperoxidase), ESR1 (estrogen receptor 1), MAPK1 (mitogen-activated protein kinase 1), HP (haptoglobin), F3 (coagulation factor III (thromboplastin, tissue factor)), CST3 (cystatin C), COG2 (component of oligomeric golgi complex 2), MMP9 (matrix metallopeptidase 9 (gelatinase B, 92 kDa gelatinase, 92 kDa type IV collagenase)), SERPINC1 (serpin peptidase inhibitor, clade C (antithrombin), member 1), F8 (coagulation factor VIII, procoagulant component), HMOX1 (heme oxygenase (decycling) 1), APOC3 (apolipoprotein C-III), IL8 (interleukin 8), PROK1 (prokineticin 1), CBS (cystathionine-beta-synthase), NOS2 (nitric oxide synthase 2, inducible), TLR4 (toll-like receptor 4), SELP (selectin P (granule membrane protein 140 kDa, antigen CD62)), ABCA1 (ATP-binding cassette, sub-family A (ABC1), member 1), AGT (angiotensinogen (serpin peptidase inhibitor, clade A, member 8)), LDLR (low density lipoprotein receptor), GPT (glutamic-pyruvate transaminase (alanine aminotransferase)), VEGFA (vascular endothelial growth factor A), NR3C2 (nuclear receptor subfamily 3, group C, member 2), IL18 (interleukin 18 (interferon-gamma-inducing factor)), NOS1 (nitric oxide synthase 1 (neuronal)), NR3C1 (nuclear receptor subfamily 3, group C, member 1 (glucocorticoid receptor)), FGB (fibrinogen beta chain), HGF (hepatocyte growth factor (hepapoietin A; scatter factor)), ILIA (interleukin 1, alpha), RETN (resistin), AKT1 (v-akt murine thymoma viral oncogene homolog 1), LIPC (lipase, hepatic), HSPD1 (heat shock 60 kDa protein 1 (chaperonin)), MAPK14 (mitogen-activated protein kinase 14), SPP1 (secreted phosphoprotein 1), ITGB3 (integrin, beta 3 (platelet glycoprotein 111a, antigen CD61)), CAT (catalase), UTS2 (urotensin 2), THBD (thrombomodulin), F10 (coagulation factor X), CP (ceruloplasmin (ferroxidase)), TNFRSF11B (tumor necrosis factor receptor superfamily, member 11b), EDNRA (endothelin receptor type A), EGFR (epidermal growth factor receptor (erythroblastic leukemia viral (v-erb-b) oncogene homolog, avian)), MMP2 (matrix metallopeptidase 2 (gelatinase A, 72 kDa gelatinase, 72 kDa type IV collagenase)), PLG (plasminogen), NPY (neuropeptide Y), RHOD (ras homolog gene family, member D), MAPK8 (mitogen-activated protein kinase 8), MYC (v-myc myelocytomatosis viral oncogene homolog (avian)), FN1 (fibronectin 1), CMA1 (chymase 1, mast cell), PLAU (plasminogen activator, urokinase), GNB3 (guanine nucleotide binding protein (G protein), beta polypeptide 3), ADRB2 (adrenergic, beta-2-, receptor, surface), APOA5 (apolipoprotein A-V), SOD2 (superoxide dismutase 2, mitochondrial), F5 (coagulation factor V (proaccelerin, labile factor)), VDR (vitamin D (1,25-dihydroxyvitamin D3) receptor), ALOX5 (arachidonate 5-lipoxygenase), HLA-DRB1 (major histocompatibility complex, class II, DR beta 1), PARP1 (poly (ADP-ribose) polymerase 1), CD40LG (CD40 ligand), PON2 (paraoxonase 2), AGER (advanced glycosylation end product-specific receptor), IRS1 (insulin receptor substrate 1), PTGS1 (prostaglandin-endoperoxide synthase 1 (prostaglandin G/H synthase and cyclooxygenase)), ECE1 (endothelin converting enzyme 1), F7 (coagulation factor VII (serum prothrombin conversion accelerator)), URN (interleukin 1 receptor antagonist), EPHX2 (epoxide hydrolase 2, cytoplasmic), IGFBP1 (insulin-like growth factor binding protein 1), MAPK10 (mitogen-activated protein kinase 10), FAS (Fas (TNF receptor superfamily, member 6)), ABCB1 (ATP-binding cassette, sub-family B (MDR/TAP), member 1), JUN (jun oncogene), IGFBP3 (insulin-like growth factor binding protein 3), CD14 (CD14 molecule), PDE5A (phosphodiesterase 5A, cGMP-specific), AGTR2 (angiotensin II receptor, type 2), CD40 (CD40 molecule, TNF receptor superfamily member 5), LCAT (lecithin-cholesterol acyltransferase), CCR5 (chemokine (C-C motif) receptor 5), MMP1 (matrix metallopeptidase 1 (interstitial collagenase)), TIMP1 (TIMP metallopeptidase inhibitor 1), ADM (adrenomedullin), DYT10 (dystonia 10), STAT3 (signal transducer and activator of transcription 3 (acute-phase response factor)), MMP3 (matrix metallopeptidase 3 (stromelysin 1, progelatinase)), ELN (elastin), USF1 (upstream transcription factor 1), CFH (complement factor H), HSPA4 (heat shock 70 kDa protein 4), MMP12 (matrix metallopeptidase 12 (macrophage elastase)), MME (membrane metallo-endopeptidase), F2R (coagulation factor II (thrombin) receptor), SELL (selectin L), CTSB (cathepsin B), ANXA5 (annexin A5), ADRB1 (adrenergic, beta-1-, receptor), CYBA (cytochrome b-245, alpha polypeptide), FGA (fibrinogen alpha chain), GGT1 (gamma-glutamyltransferase 1), LIPG (lipase, endothelial), HIF1A (hypoxia inducible factor 1, alpha subunit (basic helix-loop-helix transcription factor)), CXCR4 (chemokine (C-X-C motif) receptor 4), PROC (protein C (inactivator of coagulation factors Va and VIIIa)), SCARB1 (scavenger receptor class B, member 1), CD79A (CD79a molecule, immunoglobulin-associated alpha), PLTP (phospholipid transfer protein), ADD1 (adducin 1 (alpha)), FGG (fibrinogen gamma chain), SAA1 (serum amyloid A1), KCNH2 (potassium voltage-gated channel, subfamily H (eag-related), member 2), DPP4 (dipeptidyl-peptidase 4), G6PD (glucose-6-phosphate dehydrogenase), NPR1 (natriuretic peptide receptor A/guanylate cyclase A (atrionatriuretic peptide receptor A)), VTN (vitronectin), KIAA0101 (KIAA0101), FOS (FBJ murine osteosarcoma viral oncogene homolog), TLR2 (toll-like receptor 2), PPIG (peptidylprolyl isomerase G (cyclophilin G)), IL1R1 (interleukin 1 receptor, type I), AR (androgen receptor), CYP1A1 (cytochrome P450, family 1, subfamily A, polypeptide 1), SERPINA1 (serpin peptidase inhibitor, clade A (alpha-1 antiproteinase, antitrypsin), member 1), MTR (5-methyltetrahydrofolate-homocysteine methyltransferase), RBP4 (retinol binding protein 4, plasma), APOA4 (apolipoprotein A-IV), CDKN2A (cyclin-dependent kinase inhibitor 2A (melanoma, p16, inhibits CDK4)), FGF2 (fibroblast growth factor 2 (basic)), EDNRB (endothelin receptor type B), ITGA2 (integrin, alpha 2 (CD49B, alpha 2 subunit of VLA-2 receptor)), CABIN1 (calcineurin binding protein 1), SHBG (sex hormone-binding globulin), HMGB1 (high-mobility group box 1), HSP90B2P (heat shock protein 90 kDa beta (Grp94), member 2 (pseudogene)), CYP3A4 (cytochrome P450, family 3, subfamily A, polypeptide 4), GJA1 (gap junction protein, alpha 1, 43 kDa), CAV1 (caveolin 1, caveolae protein, 22 kDa), ESR2 (estrogen receptor 2 (ER beta)), LTA (lymphotoxin alpha (TNF superfamily, member 1)), GDF15 (growth differentiation factor 15), BDNF (brain-derived neurotrophic factor), CYP2D6 (cytochrome P450, family 2, subfamily D, polypeptide 6), NGF (nerve growth factor (beta polypeptide)), SP1 (Sp1 transcription factor), TGIF1 (TGFB-induced factor homeobox 1), SRC (v-src sarcoma (Schmidt-Ruppin A-2) viral oncogene homolog (avian)), EGF (epidermal growth factor (beta-urogastrone)), PIK3CG (phosphoinositide-3-kinase, catalytic, gamma polypeptide), HLA-A (major histocompatibility complex, class I, A), KCNQ1 (potassium voltage-gated channel, KQT-like subfamily, member 1), CNR1 (cannabinoid receptor 1 (brain)), FBN1 (fibrillin 1), CHKA (choline kinase alpha), BEST1 (bestrophin 1), APP (amyloid beta (A4) precursor protein), CTNNB1 (catenin (cadherin-associated protein), beta 1, 88 kDa), IL2 (interleukin 2), CD36 (CD36 molecule (thrombospondin receptor)), PRKAB1 (protein kinase, AMP-activated, beta 1 non-catalytic subunit), TPO (thyroid peroxidase), ALDH7A1 (aldehyde dehydrogenase 7 family, member A1), CX3CR1 (chemokine (C-X3-C motif) receptor 1), TH (tyrosine hydroxylase), F9 (coagulation factor IX), GH1 (growth hormone 1), TF (transferrin), HFE (hemochromatosis), IL17A (interleukin 17A), PTEN (phosphatase and tensin homolog), GSTM1 (glutathione S-transferase mu 1), DMD (dystrophin), GATA4 (GATA binding protein 4), F13A1 (coagulation factor XIII, A1 polypeptide), TTR (transthyretin), FABP4 (fatty acid binding protein 4, adipocyte), PON3 (paraoxonase 3), APOC1 (apolipoprotein C-I), INSR (insulin receptor), TNFRSF1B (tumor necrosis factor receptor superfamily, member 1B), HTR2A (5-hydroxytryptamine (serotonin) receptor 2A), CSF3 (colony stimulating factor 3 (granulocyte)), CYP2C9 (cytochrome P450, family 2, subfamily C, polypeptide 9), TXN (thioredoxin), CYP11B2 (cytochrome P450, family 11, subfamily B, polypeptide 2), PTH (parathyroid hormone), CSF2 (colony stimulating factor 2 (granulocyte-macrophage)), KDR (kinase insert domain receptor (a type III receptor tyrosine kinase)), PLA2G2A (phospholipase A2, group IIA (platelets, synovial fluid)), B2M (beta-2-microglobulin), THBS1 (thrombospondin 1), GCG (glucagon), RHOA (ras homolog gene family, member A), ALDH2 (aldehyde dehydrogenase 2 family (mitochondrial)), TCF7L2 (transcription factor 7-like 2 (T-cell specific, HMG-box)), BDKRB2 (bradykinin receptor B2), NFE2L2 (nuclear factor (erythroid-derived 2)-like 2), NOTCH1 (Notch homolog 1, translocation-associated (Drosophila)), UGT1A1 (UDP glucuronosyltransferase 1 family, polypeptide A1), IFNA1 (interferon, alpha 1), PPARD (peroxisome proliferator-activated receptor delta), SIRT1 (sirtuin (silent mating type information regulation 2 homolog) 1 (S. cerevisiae)), GNRH1 (gonadotropin-releasing hormone 1 (luteinizing-releasing hormone)), PAPPA (pregnancy-associated plasma protein A, pappalysin 1), ARR3 (arrestin 3, retinal (X-arrestin)), NPPC (natriuretic peptide precursor C), AHSP (alpha hemoglobin stabilizing protein), PTK2 (PTK2 protein tyrosine kinase 2), IL13 (interleukin 13), MTOR (mechanistic target of rapamycin (serine/threonine kinase)), ITGB2 (integrin, beta 2 (complement component 3 receptor 3 and 4 subunit)), GSTT1 (glutathione S-transferase theta 1), IL6ST (interleukin 6 signal transducer (gp130, oncostatin M receptor)), CPB2 (carboxypeptidase B2 (plasma)), CYP1A2 (cytochrome P450, family 1, subfamily A, polypeptide 2), HNF4A (hepatocyte nuclear factor 4, alpha), SLC6A4 (solute carrier family 6 (neurotransmitter transporter, serotonin), member 4), PLA2G6 (phospholipase A2, group VI (cytosolic, calcium-independent)), TNFSF11 (tumor necrosis factor (ligand) superfamily, member 11), SLC8A1 (solute carrier family 8 (sodium/calcium exchanger), member 1), F2RL1 (coagulation factor II (thrombin) receptor-like 1), AKR1A1 (aldo-keto reductase family 1, member A1 (aldehyde reductase)), ALDH9A1 (aldehyde dehydrogenase 9 family, member A1), BGLAP (bone gamma-carboxyglutamate (gla) protein), MTTP (microsomal triglyceride transfer protein), MTRR (5-methyltetrahydrofolate-homocysteine methyltransferase reductase), SULT1A3 (sulfotransferase family, cytosolic, 1A, phenol-preferring, member 3), RAGE (renal tumor antigen), C4B (complement component 4B (Chido blood group), P2RY12 (purinergic receptor P2Y, G-protein coupled, 12), RNLS (renalase, FAD-dependent amine oxidase), CREB1 (cAMP responsive element binding protein 1), POMC (proopiomelanocortin), RAC1 (ras-related C3 botulinum toxin substrate 1 (rho family, small GTP binding protein Rac1)), LMNA (lamin NC), CD59 (CD59 molecule, complement regulatory protein), SCN5A (sodium channel, voltage-gated, type V, alpha subunit), CYP1B1 (cytochrome P450, family 1, subfamily B, polypeptide 1), MIF (macrophage migration inhibitory factor (glycosylation-inhibiting factor)), MMP13 (matrix metallopeptidase 13 (collagenase 3)), TIMP2 (TIMP metallopeptidase inhibitor 2), CYP19A1 (cytochrome P450, family 19, subfamily A, polypeptide 1), CYP21A2 (cytochrome P450, family 21, subfamily A, polypeptide 2), PTPN22 (protein tyrosine phosphatase, non-receptor type 22 (lymphoid)), MYH14 (myosin, heavy chain 14, non-muscle), MBL2 (mannose-binding lectin (protein C) 2, soluble (opsonic defect)), SELPLG (selectin P ligand), AOC3 (amine oxidase, copper containing 3 (vascular adhesion protein 1)), CTSL1 (cathepsin L1), PCNA (proliferating cell nuclear antigen), IGF2 (insulin-like growth factor 2 (somatomedin A)), ITGB1 (integrin, beta 1 (fibronectin receptor, beta polypeptide, antigen CD29 includes MDF2, MSK12)), CAST (calpastatin), CXCL12 (chemokine (C-X-C motif) ligand 12 (stromal cell-derived factor 1)), IGHE (immunoglobulin heavy constant epsilon), KCNE1 (potassium voltage-gated channel, Isk-related family, member 1), TFRC (transferrin receptor (p90, CD71)), COL1A1 (collagen, type I, alpha 1), COL1A2 (collagen, type I, alpha 2), IL2RB (interleukin 2 receptor, beta), PLA2G10 (phospholipase A2, group X), ANGPT2 (angiopoietin 2), PROCR (protein C receptor, endothelial (EPCR)), NOX4 (NADPH oxidase 4), HAMP (hepcidin antimicrobial peptide), PTPN11 (protein tyrosine phosphatase, non-receptor type 11), SLC2A1 (solute carrier family 2 (facilitated glucose transporter), member 1), IL2RA (interleukin 2 receptor, alpha), CCL5 (chemokine (C-C motif) ligand 5), IRF1 (interferon regulatory factor 1), CFLAR (CASP8 and FADD-like apoptosis regulator), CALCA (calcitonin-related polypeptide alpha), EIF4E (eukaryotic translation initiation factor 4E), GSTP1 (glutathione S-transferase pi 1), JAK2 (Janus kinase 2), CYP3A5 (cytochrome P450, family 3, subfamily A, polypeptide 5), HSPG2 (heparan sulfate proteoglycan 2), CCL3 (chemokine (C-C motif) ligand 3), MYD88 (myeloid differentiation primary response gene (88)), VIP (vasoactive intestinal peptide), SOAT1 (sterol O-acyltransferase 1), ADRBK1 (adrenergic, beta, receptor kinase 1), NR4A2 (nuclear receptor subfamily 4, group A, member 2), MMP8 (matrix metallopeptidase 8 (neutrophil collagenase)), NPR2 (natriuretic peptide receptor B/guanylate cyclase B (atrionatriuretic peptide receptor B)), GCH1 (GTP cyclohydrolase 1), EPRS (glutamyl-prolyl-tRNA synthetase), PPARGC1A (peroxisome proliferator-activated receptor gamma, coactivator 1 alpha), F12 (coagulation factor XII (Hageman factor)), PECAM1 (platelet/endothelial cell adhesion molecule), CCL4 (chemokine (C-C motif) ligand 4), SERPINA3 (serpin peptidase inhibitor, clade A (alpha-1 antiproteinase, antitrypsin), member 3), CASR (calcium-sensing receptor), GJA5 (gap junction protein, alpha 5, 40 kDa), FABP2 (fatty acid binding protein 2, intestinal), TTF2 (transcription termination factor, RNA polymerase II), PROS1 (protein S (alpha)), CTF1 (cardiotrophin 1), SGCB (sarcoglycan, beta (43 kDa dystrophin-associated glycoprotein)), YME1L1 (YME1-like 1 (S. cerevisiae)), CAMP (cathelicidin antimicrobial peptide), ZC3H12A (zinc finger CCCH-type containing 12A), AKR1B1 (aldo-keto reductase family 1, member B1 (aldose reductase)), DES (desmin), MMP7 (matrix metallopeptidase 7 (matrilysin, uterine)), AHR (aryl hydrocarbon receptor), CSF1 (colony stimulating factor 1 (macrophage)), HDAC9 (histone deacetylase 9), CTGF (connective tissue growth factor), KCNMA1 (potassium large conductance calcium-activated channel, subfamily M, alpha member 1), UGT1A (UDP glucuronosyltransferase 1 family, polypeptide A complex locus), PRKCA (protein kinase C, alpha), COMT (catechol-.beta.-methyltransferase), S100B (S100 calcium binding protein B), EGR1 (early growth response 1), PRL (prolactin), IL15 (interleukin 15), DRD4 (dopamine receptor D4), CAMK2G (calcium/calmodulin-dependent protein kinase II gamma), SLC22A2 (solute carrier family 22 (organic cation transporter), member 2), CCL11 (chemokine (C-C motif) ligand 11), PGF (B321 placental growth factor), THPO (thrombopoietin), GP6 (glycoprotein VI (platelet)), TACR1 (tachykinin receptor 1), NTS (neurotensin), HNF1A (HNF1 homeobox A), SST (somatostatin), KCND1 (potassium voltage-gated channel, Shal-related subfamily, member 1), LOC646627 (phospholipase inhibitor), TBXAS1 (thromboxane A synthase 1 (platelet)), CYP2J2 (cytochrome P450, family 2, subfamily J, polypeptide 2), TBXA2R (thromboxane A2 receptor), ADH1C (alcohol dehydrogenase 1C (class I), gamma polypeptide), ALOX12 (arachidonate 12-lipoxygenase), AHSG (alpha-2-HS-glycoprotein), BHMT (betaine-homocysteine methyltransferase), GJA4 (gap junction protein, alpha 4, 37 kDa), SLC25A4 (solute carrier family 25 (mitochondrial carrier; adenine nucleotide translocator), member 4), ACLY (ATP citrate lyase), ALOX5AP (arachidonate 5-lipoxygenase-activating protein), NUMA1 (nuclear mitotic apparatus protein 1), CYP27B1 (cytochrome P450, family 27, subfamily B, polypeptide 1), CYSLTR2 (cysteinyl leukotriene receptor 2), SOD3 (superoxide dismutase 3, extracellular), LTC4S (leukotriene C4 synthase), UCN (urocortin), GHRL (ghrelin/obestatin prepropeptide), APOC2 (apolipoprotein C-II), CLEC4A (C-type lectin domain family 4, member A), KBTBD10 (kelch repeat and BTB (POZ) domain containing 10), TNC (tenascin C), TYMS (thymidylate synthetase), SHCl (SHC (Src homology 2 domain containing) transforming protein 1), LRP1 (low density lipoprotein receptor-related protein 1), SOCS3 (suppressor of cytokine signaling 3), ADH1B (alcohol dehydrogenase 1B (class I), beta polypeptide), KLK3 (kallikrein-related peptidase 3), HSD11B1 (hydroxysteroid (11-beta) dehydrogenase 1), VKORC1 (vitamin K epoxide reductase complex, subunit 1), SERPINB2 (serpin peptidase inhibitor, clade B (ovalbumin), member 2), TNS1 (tensin 1), RNF19A (ring finger protein 19A), EPOR (erythropoietin receptor), ITGAM (integrin, alpha M (complement component 3 receptor 3 subunit)), PITX2 (paired-like homeodomain 2), MAPK7 (mitogen-activated protein kinase 7), FCGR3A (Fc fragment of IgG, low affinity 111a, receptor (CD16a)), LEPR (leptin receptor), ENG (endoglin), GPX1 (glutathione peroxidase 1), GOT2 (glutamic-oxaloacetic transaminase 2, mitochondrial (aspartate aminotransferase 2)), HRH1 (histamine receptor HI), NR112 (nuclear receptor subfamily 1, group I, member 2), CRH (corticotropin releasing hormone), HTR1A (5-hydroxytryptamine (serotonin) receptor 1A), VDAC1 (voltage-dependent anion channel 1), HPSE (heparanase), SFTPD (surfactant protein D), TAP2 (transporter 2, ATP-binding cassette, sub-family B (MDR/TAP)), RNF123 (ring finger protein 123), PTK2B (PTK2B protein tyrosine kinase 2 beta), NTRK2 (neurotrophic tyrosine kinase, receptor, type 2), IL6R (interleukin 6 receptor), ACHE (acetylcholinesterase (Yt blood group)), GLP1R (glucagon-like peptide 1 receptor), GHR (growth hormone receptor), GSR (glutathione reductase), NQO1 (NAD(P)H dehydrogenase, quinone 1), NR5A1 (nuclear receptor subfamily 5, group A, member 1), GJB2 (gap junction protein, beta 2, 26 kDa), SLC9A1 (solute carrier family 9 (sodium/hydrogen exchanger), member 1), MAOA (monoamine oxidase A), PCSK9 (proprotein convertase subtilisin/kexin type 9), FCGR2A (Fc fragment of IgG, low affinity IIa, receptor (CD32)), SERPINF1 (serpin peptidase inhibitor, clade F (alpha-2 antiplasmin, pigment epithelium derived factor), member 1), EDN3 (endothelin 3), DHFR (dihydrofolate reductase), GAS6 (growth arrest-specific 6), SMPD1 (sphingomyelin phosphodiesterase 1, acid lysosomal), UCP2 (uncoupling protein 2 (mitochondrial, proton carrier)), TFAP2A (transcription factor AP-2 alpha (activating enhancer binding protein 2 alpha)), C4BPA (complement component 4 binding protein, alpha), SERPINF2 (serpin peptidase inhibitor, clade F (alpha-2 antiplasmin, pigment epithelium derived factor), member 2), TYMP (thymidine phosphorylase), ALPP (alkaline phosphatase, placental (Regan isozyme)), CXCR2 (chemokine (C-X-C motif) receptor 2), SLC39A3 (solute carrier family 39 (zinc transporter), member 3), ABCG2 (ATP-binding cassette, sub-family G (WHITE), member 2), ADA (adenosine deaminase), JAK3 (Janus kinase 3), HSPA1A (heat shock 70 kDa protein 1A), FASN (fatty acid synthase), FGF1 (fibroblast growth factor 1 (acidic)), F11 (coagulation factor XI), ATP7A (ATPase, Cu++ transporting, alpha polypeptide), CR1 (complement component (3b/4b) receptor 1 (Knops blood group)), GFAP (glial fibrillary acidic protein), ROCK1 (Rho-associated, coiled-coil containing protein kinase 1), MECP2 (methyl CpG binding protein 2 (Rett syndrome)), MYLK (myosin light chain kinase), BCHE (butyrylcholinesterase), LIPE (lipase, hormone-sensitive), PRDX5 (peroxiredoxin 5), ADORA1 (adenosine A1 receptor), WRN (Werner syndrome, RecQ helicase-like), CXCR3 (chemokine (C-X-C motif) receptor 3), CD81 (CD81 molecule), SMAD7 (SMAD family member 7), LAMC2 (laminin, gamma 2), MAP3K5 (mitogen-activated protein kinase kinase kinase 5), CHGA (chromogranin A (parathyroid secretory protein 1)), IAPP (islet amyloid polypeptide), RHO (rhodopsin), ENPP1 (ectonucleotide pyrophosphatase/phosphodiesterase 1), PTHLH (parathyroid hormone-like hormone), NRG1 (neuregulin 1), VEGFC (vascular endothelial growth factor C), ENPEP (glutamyl aminopeptidase (aminopeptidase A)), CEBPB (CCAAT/enhancer binding protein (C/EBP), beta), NAGLU (N-acetylglucosaminidase, alpha-), F2RL3 (coagulation factor II (thrombin) receptor-like 3), CX3CL1 (chemokine (C-X3-C motif) ligand 1), BDKRB1 (bradykinin receptor B1), ADAMTS13 (ADAM metallopeptidase with thrombospondin type 1 motif, 13), ELANE (elastase, neutrophil expressed), ENPP2 (ectonucleotide pyrophosphatase/phosphodiesterase 2), CISH (cytokine inducible SH2-containing protein), GAST (gastrin), MYOC (myocilin, trabecular meshwork inducible glucocorticoid response), ATP1A2 (ATPase, Na+/K+ transporting, alpha 2 polypeptide), NF1 (neurofibromin 1), GJB1 (gap junction protein, beta 1, 32 kDa), MEF2A (myocyte enhancer factor 2A), VCL (vinculin), BMPR2 (bone morphogenetic protein receptor, type II (serine/threonine kinase)), TUBB (tubulin, beta), CDC42 (cell division cycle 42 (GTP binding protein, 25 kDa)), KRT18 (keratin 18), HSF1 (heat shock transcription factor 1), MYB (v-myb myeloblastosis viral oncogene homolog (avian)), PRKAA2 (protein kinase, AMP-activated, alpha 2 catalytic subunit), ROCK2 (Rho-associated, coiled-coil containing protein kinase 2), TFPI (tissue factor pathway inhibitor (lipoprotein-associated coagulation inhibitor)), PRKG1 (protein kinase, cGMP-dependent, type I), BMP2 (bone morphogenetic protein 2), CTNND1 (catenin (cadherin-associated protein), delta 1), CTH (cystathionase (cystathionine gamma-lyase)), CTSS (cathepsin S), VAV2 (vav 2 guanine nucleotide exchange factor), NPY2R (neuropeptide Y receptor Y2), IGFBP2 (insulin-like growth factor binding protein 2, 36 kDa), CD28 (CD28 molecule), GSTA1 (glutathione S-transferase alpha 1), PPIA (peptidylprolyl isomerase A (cyclophilin A)), APOH (apolipoprotein H (beta-2-glycoprotein I)), S100A8 (S100 calcium binding protein A8), IL11 (interleukin 11), ALOX15 (arachidonate 15-lipoxygenase), FBLN1 (fibulin 1), NR1H3 (nuclear receptor subfamily 1, group H, member 3), SCD (stearoyl-CoA desaturase (delta-9-desaturase)), GIP (gastric inhibitory polypeptide), CHGB (chromogranin B (secretogranin 1)), PRKCB (protein kinase C, beta), SRD5A1 (steroid-5-alpha-reductase, alpha polypeptide 1 (3-oxo-5 alpha-steroid delta 4-dehydrogenase alpha 1)), HSD11B2 (hydroxysteroid (11-beta) dehydrogenase 2), CALCRL (calcitonin receptor-like), GALNT2 (UDP-N-acetyl-alpha-D-galactosamine:polypeptide N-acetylgalactosaminyltransferase 2 (GalNAc-T2)), ANGPTL4 (angiopoietin-like 4), KCNN4 (potassium intermediate/small conductance calcium-activated channel, subfamily N, member 4), PIK3C2A (phosphoinositide-3-kinase, class 2, alpha polypeptide), HBEGF (heparin-binding EGF-like growth factor), CYP7A1 (cytochrome P450, family 7, subfamily A, polypeptide 1), HLA-DRB5 (major histocompatibility complex, class II, DR beta 5), BNIP3 (BCL2/adenovirus E1B 19 kDa interacting protein 3), GCKR (glucokinase (hexokinase 4) regulator), S100A12 (S100 calcium binding protein A12), PADI4 (peptidyl arginine deiminase, type IV), HSPA14 (heat shock 70 kDa protein 14), CXCR1 (chemokine (C-X-C motif) receptor 1), H19 (H19, imprinted maternally expressed transcript (non-protein coding)), KRTAP19-3 (keratin associated protein 19-3), IDDM2 (insulin-dependent diabetes mellitus 2), RAC2 (ras-related C3 botulinum toxin substrate 2 (rho family, small GTP binding protein Rac2)), RYR1 (ryanodine receptor 1 (skeletal)), CLOCK (clock homolog (mouse)), NGFR (nerve growth factor receptor (TNFR superfamily, member 16)), DBH (dopamine beta-hydroxylase (dopamine beta-monooxygenase)), CHRNA4 (cholinergic receptor, nicotinic, alpha 4), CACNA1C (calcium channel, voltage-dependent, L type, alpha 1C subunit), PRKAG2 (protein kinase, AMP-activated, gamma 2 non-catalytic subunit), CHAT (choline acetyltransferase), PTGDS (prostaglandin D2 synthase 21 kDa (brain)), NR1H2 (nuclear receptor subfamily 1, group H, member 2), TEK (TEK tyrosine kinase, endothelial), VEGFB (vascular endothelial growth factor B), MEF2C (myocyte enhancer factor 2C), MAPKAPK2 (mitogen-activated protein kinase-activated protein kinase 2), TNFRSF11A (tumor necrosis factor receptor superfamily, member 11a, NFKB activator), HSPA9 (heat shock 70 kDa protein 9 (mortalin)), CYSLTR1 (cysteinyl leukotriene receptor 1), MAT1A (methionine adenosyltransferase I, alpha), OPRL1 (opiate receptor-like 1), IMPA1 (inositol(myo)-1(or 4)-monophosphatase 1), CLCN2 (chloride channel 2), DLD (dihydrolipoamide dehydrogenase), PSMA6 (proteasome (prosome, macropain) subunit, alpha type, 6), PSMB8 (proteasome (prosome, macropain) subunit, beta type, 8 (large multifunctional peptidase 7)), CHI3L1 (chitinase 3-like 1 (cartilage glycoprotein-39)), ALDH1B1 (aldehyde dehydrogenase 1 family, member B1), PARP2 (poly (ADP-ribose) polymerase 2), STAR (steroidogenic acute regulatory protein), LBP (lipopolysaccharide binding protein), ABCC6 (ATP-binding cassette, subfamily C(CFTR/MRP), member 6), RGS2 (regulator of G-protein signaling 2, 24 kDa), EFNB2 (ephrin-B2), GJB6 (gap junction protein, beta 6, 30 kDa), APOA2 (apolipoprotein A-II), AMPD1 (adenosine monophosphate deaminase 1), DYSF (dysferlin, limb girdle muscular dystrophy 2B (autosomal recessive)), FDFT1 (farnesyl-diphosphate farnesyltransferase 1), EDN2 (endothelin 2), CCR6 (chemokine (C-C motif) receptor 6), GJB3 (gap junction protein, beta 3, 31 kDa), IL1RL1 (interleukin 1 receptor-like 1), ENTPD1 (ectonucleoside triphosphate diphosphohydrolase 1), BBS4 (Bardet-Biedl syndrome 4), CELSR2 (cadherin, EGF LAG seven-pass G-type receptor 2 (flamingo homolog, Drosophila)), F11R (F11 receptor), RAPGEF3 (Rap guanine nucleotide exchange factor (GEF) 3), HYAL1 (hyaluronoglucosaminidase 1), ZNF259 (zinc finger protein 259), ATOX1 (ATX1 antioxidant protein 1 homolog (yeast)), ATF6 (activating transcription factor 6), KHK (ketohexokinase (fructokinase)), SAT1 (spermidine/spermine N1-acetyltransferase 1), GGH (gamma-glutamyl hydrolase (conjugase, folylpolygammaglutamyl hydrolase)), TIMP4 (TIMP metallopeptidase inhibitor 4), SLC4A4 (solute carrier family 4, sodium bicarbonate cotransporter, member 4), PDE2A (phosphodiesterase 2A, cGMP-stimulated), PDE3B (phosphodiesterase 3B, cGMP-inhibited), FADS1 (fatty acid desaturase 1), FADS2 (fatty acid desaturase 2), TMSB4X (thymosin beta 4, X-linked), TXNIP (thioredoxin interacting protein), LIMS1 (LIM and senescent cell antigen-like domains 1), RHOB (ras homolog gene family, member B), LY96 (lymphocyte antigen 96), FOXO1 (forkhead box O1), PNPLA2 (patatin-like phospholipase domain containing 2), TRH (thyrotropin-releasing hormone), GJC1 (gap junction protein, gamma 1, 45 kDa), SLC17A5 (solute carrier family 17 (anion/sugar transporter), member 5), FTO (fat mass and obesity associated), GJD2 (gap junction protein, delta 2, 36 kDa), PSRC1 (proline/serine-rich coiled-coil 1), CASP12 (caspase 12 (gene/pseudogene)), GPBAR1 (G protein-coupled bile acid receptor 1), PXK (PX domain containing serine/threonine kinase), IL33 (interleukin 33), TRIB1 (tribbles homolog 1 (Drosophila)), PBX4 (pre-B-cell leukemia homeobox 4), NUPR1 (nuclear protein, transcriptional regulator, 1), 15-Sep(15 kDa selenoprotein), CILP2 (cartilage intermediate layer protein 2), TERC (telomerase RNA component), GGT2 (gamma-glutamyltransferase 2), MT-CO1 (mitochondrially encoded cytochrome c oxidase I), and UOX (urate oxidase, pseudogene). Any of these sequences, may be a target for the CRISPR-Cas system, e.g., to address mutation.

In an additional embodiment, the chromosomal sequence may further be selected from Pon1 (paraoxonase 1), LDLR (LDL receptor), ApoE (Apolipoprotein E), Apo B-100 (Apolipoprotein B-100), ApoA (Apolipoprotein(a)), ApoA1 (Apolipoprotein A1), CBS (Cystathione B-synthase), Glycoprotein IIb/IIb, MTHRF (5,10-methylenetetrahydrofolate reductase (NADPH), and combinations thereof. In one iteration, the chromosomal sequences and proteins encoded by chromosomal sequences involved in cardiovascular disease may be chosen from Cacna1C, Sod1, Pten, Ppar(alpha), Apo E, Leptin, and combinations thereof as target(s) for the CRISPR-Cas system.

<u>Kidneys</u>

The present invention also contemplates delivering the CRISPR-Cas system to the kidney. Delivery strategies to induce cellular uptake of the therapeutic nucleic acid include physical force or vector systems such as viral-, lipid- or complex- based delivery, or nanocarriers. From the initial applications with less possible clinical relevance, when nucleic acids were addressed to renal cells with hydrodynamic high pressure injection systemically, a wide range of gene therapeutic viral and non-viral carriers have been applied already to target posttranscriptional events in different animal kidney disease models in vivo (Csaba Révész and Péter Hamar (2011). Delivery Methods to Target RNAs in the Kidney, Gene Therapy Applications, Prof. Chunsheng Kang (Ed.), ISBN: 978-953-307-541-9, InTech, Available from: http://www.intechopen.com/books/gene-therapy-applications/delivery-methods-to-target-rnas-in-the-kidney). Delivery methods to the kidney are summarized as follows:

| Delivery method | Carrier | Target RNA | Disease | Model | Functional assays | Author |
|---|---|---|---|---|---|---|
| Hydrodynamic / Lipid | TransIT In Vivo Gene Delivery System, DOTAP | p85$\alpha$ | Acute renal injury | Ischemia-reperfusion | Uptake, biodistribution | Larson et al., Surgery, (Aug 2007), Vol. 142, No. 2, pp. (262-269) |
| Hydrodynamic / Lipid | Lipofectamine 2000 | Fas | Acute renal injury | Ischemia-reperfusion | Blood urea nitrogen, Fas Immunohistochem istry, apoptosis, histological scoring | Hamar et al., Proc Natl Acad Sci, (Oct 2004), Vol. 101, No. 41, pp. (14883-14888) |
| Hydrodynamic | n.a. | Apoptosis cascade elements | Acute renal injury | Ischemia-reperfusion | n.a. | Zheng et al., Am J Pathol, (Oct 2008), Vol. 173, No. 4, pp. (973-980) |
| Hydrodynamic | n.a. | Nuclear factor kappa-b (NFkB) | Acute renal injury | Ischemia-reperfusion | n.a. | Feng et al., Transplantation, (May 2009), Vol. 87, No. 9, pp. (1283-1289) |
| Hydrodynamic /Viral | Lipofectamine 2000 | Apoptosis antagonizing transcription factor (AATF) | Acute renal injury | Ischemia-reperfusion | Apoptosis, oxidative stress, caspase activation, membrane lipid peroxidation | Xie & Guo, Am Soc Nephrol, (Dec 2006), Vol. 17, No. 12, pp. (3336-3346) |
| Hydrodynamic | pBAsi mU6 Neo/ TransIT-EE Hydrodynamic Delivery System | Gremlin | Diabetic nephropathy | Streptozotozi n -induced diabetes | Proteinuria, serum creatinine, glomerular and tubular diameter, collagen type IV/BMP7 expression | Q. Zhang et al., PloS ONE, (Jul 2010), Vol. 5, No. 7, e11709, pp. (1-13) |
| Viral/Lipid | pSUPER vector/ Lipofectami ne | TGF-$\beta$ type II receptor | Interstitial renal fibrosis | Unilateral urethral obstruction | $\alpha$-SMA expression, collagen content, | Kushibikia et al., J Controlled Release, (Jul 2005), Vol. 105, No. 3, pp. (318-331) |
| Viral | Adeno-associated virus-2 | Mineral corticoid receptor | Hypertension caused renal damage | Cold-induced hypertension | blood pressure, serum albumin, serum urea nitrogen, serum creatinine, kidney weight, urinary sodium | Wang et al., Gene Therapy, (Jul 2006), Vol. 13, No. 14, pp. (1097-1103) |
| Hydrodynamic /Viral | pU6 vector | Luciferase | n.a. | n.a. | uptake | Kobayashi et al., Journal of Pharmacology and Experimental Therapeutics, (Feb 2004), Vol. 308, No. 2, pp. (688-693) |

(continued)

| Delivery method | Carrier | Target RNA | Disease | Model | Functional assays | Author |
|---|---|---|---|---|---|---|
| Lipid | Lipoproteins, albumin | apoB1, apoM | n.a. | n.a. | Uptake, binding affinity to lipoproteins and albumin | Wolfrum et al., Nature Biotechnology, (Sep 2007), Vol. 25, No. 10, pp. (1149-1157) |
| Lipid | Lipofectamine2000 | p53 | Acute renal injury | Ischemic and cisplatin-induced acute injury | Histological scoring, apoptosis | Molitoris et al., J Am Soc Nephrol, (Aug 2009), Vol. 20, No. 8, pp. (1754-1764) |
| Lipid | DOTAP/DOPE, DOTAP/DO PE/ DOPE-PEG2000 | COX-2 | Breast adenocarcinoma | MDA-MB-231 breast cancer xenograft-bearing mouse | Cell viability, uptake | Mikhaylova et al., Cancer Gene Therapy (Mar 2011), Vol. 16, No. 3, pp. (217-226) |
| Lipid | Cholesterol | 12/15-lipoxygenase | Diabetic nephro-pathy | Streptozotoci n -induced diabetes | Albuminuria, urinary creatinine, histology, type I and IV collagen, TGF-$\beta$, fibronectin, plasminogen activator inhibitor 1 | Yuan et al., Am J Physiol Renal Physiol, (Jun 2008), Vol. 295, pp. (F605-F617) |
| Lipid | Lipofectamine 2000 | Mitochondrial membrane 44 (TIM44) | Diabetic nephro-pathy | Streptozotoci n -induced diabetes | Cell proliferation and apoptosis, histology, ROS, mitochondrial import of Mn-SOD and glutathione peroxidase, cellular membrane polarization | Y. Zhang et al., J Am Soc Nephrol, (Apr 2006), Vol. 17, No. 4, pp. (1090-1101) |
| Hydrodynamic / Lipid | Proteolipo-some | RLIP76 | Renal carcinoma | Caki-2 kidney cancer xenograft-bearing mouse | uptake | Singhal et al., Cancer Res, (May 2009), Vol. 69, No. 10, pp. (4244-4251) |
| Polymer | PEGylated PEI | Luciferase pGL3 | n.a. | n.a. | Uptake, biodistribution, erythrocyte aggregation | Malek et al., Toxicology and Applied Pharmacology, (Apr 2009), Vol. 236, No. 1, pp. (97-108) |
| Polymer | PEGylated poly-L-lysine | MAPK1 | Lupus glomerulonephritis | Glomerulonephritis | Proteinuria, glomerulosclerosis TGF- $\beta$, fibronectin, plasminogen activator inhibitor 1 | Shimizu et al., J Am Soc Nephrology, (Apr 2010), Vol. 21, No. 4, pp. (622-633) |

(continued)

| Delivery method | Carrier | Target RNA | Disease | Model | Functional assays | Author |
|---|---|---|---|---|---|---|
| Polymer/Nano particle | Hyaluronic acid/ Quantum dot/PEI | VEGF | Kidney cancer/ melanoma | B16F1 melanoma tumor-bearing mouse | Biodistribution, citotoxicity, tumor volume, endocytosis | Jiang et al., Molecular Pharmaceutics, (May-Jun 2009), Vol. 6, No. 3, pp. (727-737) |
| Polymer/Nano particle | PEGylated polycapro- lactone nanofiber | GAPDH | n.a. | n.a. | cell viability, uptake | Cao et al, J Controlled Release, (Jun 2010), Vol. 144, No. 2, pp. (203-212) |
| Aptamer | Spiegelmer mNOX-E36 | CC chemokine ligand 2 | Glomerulo sclerosis | Uninephrecto - mized mouse | urinary albumin, urinary creatinine, histopathology, glomerular filtration rate, macrophage count, serum Ccl2, Mac-2+, Ki-67+ | Ninichuk et al., Am J Pathol, (Mar 2008), Vol. 172, No. 3, pp. (628-637) |
| Aptamer | Aptamer NOX-F37 | vasopressin (AVP) | Congestive heart failure | n.a. | Binding affinity to D-AVP, Inhibition of AVP Signaling, Urine osmolality and sodium concentration, | Purschke et al., Proc Natl Acad Sci, (Mar 2006), Vol 103, No. 13, pp. (5173_5178) |

Yuan et al. (Am J Physiol Renal Physiol 295: F605-F617, 2008) investigated whether in vivo delivery of small interfering RNAs (siRNAs) targeting the 12/15-lipoxygenase (12/15-LO) pathway of arachidonate acid metabolism can ameliorate renal injury and diabetic nephropathy (DN) in a streptozotocininjected mouse model of type 1 diabetes. To achieve greater in vivo access and siRNA expression in the kidney, Yuan et al. used double-stranded 12/15-LO siRNA oligonucleotides conjugated with cholesterol. About 400 μg of siRNA was injected subcutaneously into mice. The method of Yuang et al. may be applied to the CRISPR Cas system of the present invention contemplating a 1-2 g subcutaneous injection of CRISPR Cas conjugated with cholesterol to a human for delivery to the kidneys.

Molitoris et al. (J Am Soc Nephrol 20: 1754-1764, 2009) exploited proximal tubule cells (PTCs), as the site of oligonucleotide reabsorption within the kidney to test the efficacy of siRNA targeted to p53, a pivotal protein in the apoptotic pathway, to prevent kidney injury. Naked synthetic siRNA to p53 injected intravenously 4 h after ischemic injury maximally protected both PTCs and kidney function. Molitoris et al.'s data indicates that rapid delivery of siRNA to proximal tubule cells follows intravenous administration. For dose-response analysis, rats were injected with doses of siP53, 0.33; 1, 3, or 5mg/kg, given at the same four time points, resulting in cumulative doses of 1.32; 4, 12, and 20 mg/kg, respectively. All siRNA doses tested produced a SCr reducing effect on day one with higher doses being effective over approximately five days compared with PBS-treated ischemic control rats. The 12 and 20 mg/kg cumulative doses provided the best protective effect. The method of Molitoris et al. may be applied to the CRISPR Cas system of the present invention contemplating 12 and 20 mg/kg cumulative doses to a human for delivery to the kidneys.

Thompson et al. (Nucleic Acid Therapeutics, Volume 22, Number 4, 2012) reports the toxicological and pharmacokinetic properties of the synthetic, small interfering RNA I5NP following intravenous administration in rodents and nonhuman primates. I5NP is designed to act via the RNA interference (RNAi) pathway to temporarily inhibit expression of the pro-apoptotic protein p53 and is being developed to protect cells from acute ischemia/reperfusion injuries such as acute kidney injury that can occur during major cardiac surgery and delayed graft function that can occur following renal transplantation. Doses of 800mg/kg I5NP in rodents, and 1,000 mg/kg I5NP in nonhuman primates, were required to elicit adverse effects, which in the monkey were isolated to direct effects on the blood that included a sub-clinical activation of complement and slightly increased clotting times. In the rat, no additional adverse effects were observed with a rat analogue of I5NP, indicating that the effects likely represent class effects of synthetic RNA duplexes rather than toxicity related to the intended pharmacologic activity of I5NP. Taken together, these data support clinical testing of intravenous administration of I5NP for the preservation of renal function following acute ischemia/reperfusion injury. The no observed adverse effect level (NOAEL) in the monkey was 500 mg/kg. No effects on cardiovascular, respiratory, and neurologic parameters were observed in monkeys following i.v. administration at dose levels up to 25 mg/kg. Therefore, a similar dosage may be contemplated for intravenous administration of CRISPR Cas to the kidneys of a human.

Shimizu et al. (J Am Soc Nephrol 21: 622-633, 2010) developed a system to target delivery of siRNAs to glomeruli via poly(ethylene glycol)-poly(L-lysine)-based vehicles. The siRNA/nanocarrier complex was approximately 10 to 20 nm in diameter, a size that would allow it to move across the fenestrated endothelium to access to the mesangium. After intraperitoneal injection of fluorescence-labeled siRNA/nanocarrier complexes, Shimizu et al. detected siRNAs in the blood circulation for a prolonged time. Repeated intraperitoneal administration of a mitogen-activated protein kinase 1 (MAPK1) siRNA/nanocarrier complex suppressed glomerular MAPK1 mRNA and protein expression in a mouse model of glomerulonephritis. For the investigation of siRNA accumulation, Cy5-labeled siRNAs complexed with PIC nanocarriers (0.5 ml, 5 nmol of siRNA content), naked Cy5-labeled siRNAs (0.5 ml, 5 nmol), or Cy5-labeled siRNAs encapsulated in HVJ-E (0.5 ml, 5 nmol of siRNA content) were administrated to BALB-c mice. The method of Shimizu et al. may be applied to the CRISPR Cas system of the present invention contemplating a dose of about of 10-20 μmol CRISPR Cas complexed with nanocarriers in about 1-2 liters to a human for intraperitoneal administration and delivery to the kidneys.

## Lungs

The present invention also contemplates delivering the CRISPR-Cas system to oneor both lungs. Although AAV-2-based vectors were originally proposed for CFTR delivery to CF airways, other serotypes such as AAV-1, AAV-5, AAV-6, and AAV-9 exhibit improved gene transfer efficiency in a variety of models of the lung epithelium (see, e.g., Li et al., Molecular Therapy, vol. 17 no. 12, 2067-2077 Dec 2009). AAV-1 was demonstrated to be ~100-fold more efficient than AAV-2 and AAV-5 at transducing human airway epithelial cells in vitro,5 although AAV-1 transduced murine tracheal airway epithelia in vivo with an efficiency equal to that of AAV-5. Other studies have shown that AAV-5 is 50-fold more efficient than AAV-2 at gene delivery to human airway epithelium (HAE) in vitro and significantly more efficient in the mouse lung airway epithelium in vivo. AAV-6 has also been shown to be more efficient than AAV-2 in human airway epithelial cells in vitro and murine airways in vivo.8 The more recent isolate, AAV-9, was shown to display greater gene transfer efficiency than AAV-5 in murine nasal and alveolar epithelia in vivo with gene expression detected for over 9 months showing AAV may enable long-term gene expression in vivo, a desirable property for a CFTR gene delivery vector. Furthermore, it was demonstrated that AAV-9 could be readministered to the murine lung with no loss of CFTR expression and minimal immune consequences. CF and non-CF HAE cultures may be inoculated on the apical surface with 100 μl of AAV vectors for hours (see, e.g., Li et al., Molecular Therapy, vol. 17 no. 12, 2067-2077 Dec 2009). The MOI may vary from $1 \times 10^3$ to $4 \times 10^5$ vector genomes/cell, depending on virus concentration and purposes of the experiments. The above cited vectors are contemplated for the delivery and/or administration of the invention.

Zamora et al. (Am J Respir Crit Care Med Vol 183. pp 531-538, 2011) reported an example of the application of an RNA interference therapeutic to the treatment of human infectious disease and also a randomized trial of an antiviral drug in respiratory syncytial virus (RSV)-infected lung transplant recipients. Zamora et al. performed a randomized, double-blind, placebocontrolled trial in LTX recipients with RSV respiratory tract infection. Patients were permitted to receive standard of care for RSV. Aerosolized ALN-RSV01 (0.6 mg/kg) or placebo was administered daily for 3 days. This study demonstrates that an RNAi therapeutic targeting RSV can be safely administered to LTX recipients with RSV infection. Three daily doses of ALN-RSV01 did not result in any exacerbation of respiratory tract symptoms or impairment of lung function and did not exhibit any systemic proinflammatory effects, such as induction of cytokines or CRP. Pharmacokinetics showed only low, transient systemic exposure after inhalation, consistent with preclinical animal data showing that ALN-RSV01, administered intravenously or by inhalation, is rapidly cleared from the circulation through exonucleasemediated digestion and renal excretion. The method of Zamora et al. may be applied to the CRISPR Cas system of the present invention and an aerosolized CRISPR Cas, for example with a dosage of 0.6 mg/kg, may be contemplated for the present invention.

For an example of CFTRdelta508 chimeric guide RNA, see Example 20 which demonstrates gene transfer or gene delivery of a CRISPR-Cas system in airways of subject or a patient in need thereof, suffering from cystic fibrosis or from cystic fibrosis (CF) related symptoms, using adeno-associated virus (AAV) particles. In particular, they exemplify a repair strategy for Cystic Fibrosis delta F508 mutation. This type of strategy should apply across all organisms. With particular reference to CF, suitable patients may include: Human, non-primate human, canine, feline, bovine, equine and other domestic animals. In this instance, Applicants utilized a CRISPR-Cas system comprising a Cas9 enzyme to target deltaF508 or other CFTR-inducing mutations.

The treated subjects in this instance receive pharmaceutically effective amount of aerosolized AAV vector system per lung endobronchially delivered while spontaneously breathing. As such, aerosolized delivery is preferred for AAV delivery in general. An adenovirus or an AAV particle may be used for delivery. Suitable gene constructs, each operably linked to one or more regulatory sequences, may be cloned into the delivery vector. In this instance, the following constructs are provided as examples: Cbh or EF1a promoter for Cas9, U6 or H1 promoter for chimeric guide RNA),: A preferred arrangement is to use a CFTRdelta508 targeting chimeric guide, a repair template for deltaF508 mutation and a codon optimized Cas9 enzyme (preferred Cas9s are those with nuclease or nickase activity) with optionally one or more nuclear localization signal or sequence(s) (NLS(s)), e.g., two (2) NLSs. Constructs without NLS are also envisaged.

In order to identify the Cas9 target site, Applicants analyzed the human CFTR genomic locus and identified the Cas9 target site. Preferably, in general and in this CF case, the PAM may contain a NGG or a NNAGAAW motif.

Accordingly, in the case of CF, the present method comprises manipulation of a target sequence in a genomic locus of interest comprising

delivering via at least one nanoparticle complex a non-naturally occurring or engineered composition comprising a viral vector system comprising one or more viral vectors operably encoding a composition for expression thereof, wherein the composition comprises:

a non-naturally occurring or engineered composition comprising a vector system comprising one or more vectors comprising

I. a first regulatory element operably linked to a CRISPR-Cas system chimeric RNA (chiRNA) polynucleotide sequence, wherein the polynucleotide sequence comprises

(a) a guide sequence capable of hybridizing to the CF target sequence in a suitable mammalian cell,
(b) a tracr mate sequence, and
(c) a tracr sequence, and

II. a second regulatory element operably linked to an enzyme-coding sequence encoding a CRISPR enzyme comprising at least one or more nuclear localization sequences,
wherein (a), (b) and (c) are arranged in a 5' to 3' orientation,
wherein components I and II are located on the same or different vectors of the system,
wherein when transcribed, the tracr mate sequence hybridizes to the tracr sequence and the guide sequence directs sequence-specific binding of a CRISPR complex to the target sequence, and
wherein the CRISPR complex comprises the CRISPR enzyme complexed with (1) the guide sequence that is hybridizable to the target sequence, and (2) the tracr mate sequence that is hybridizable to the tracr sequence. In respect of CF, preferred target DNA sequences comprise the CFTRdelta508 mutation. A preferred PAM is described above. A preferred CRISPR enzyme is any Cas (described herein, but particularly that described in Example 20).

Alternatives to CF include any genetic disorder and examples of these are well known. Another preferred method or use of the invention is for correcting defects in the EMP2A and EMP2B genes that have been identified to be associated with Lafora disease.

In some embodiments, a "guide sequence" may be distinct from "guide RNA". A guide sequence may refer to an approx. 20bp sequence, within the guide RNA, that specifies the target site.

In some embodiments, the Cas9 is (or is derived from) SpCas9. In such embodiments, preferred mutations are at any or all or positions 10, 762, 840, 854, 863 and/or 986 of SpCas9 or corresponding positions in other Cas9s (which may be ascertained for instance by standard sequence comparison tools. In particular, any or all of the following mutations are preferred in SpCas9: D10A, E762A, H840A, N854A, N863A and/or D986A; as well as conservative substitution for any of the replacement amino acids is also envisaged. The same (or conservative substitutions of these mutations) at corresponding positions in other Cas9s are also preferred. Particularly preferred are D10 and H840 in SpCas9. However, in other Cas9s, residues corresponding to SpCas9 D10 and H840 are also preferred. These are advantageous as they provide nickase activity. Such mutations may be applied to all aspects of the present invention, not only treatment of CF.

Schwank et al. (Cell Stem Cell, 13:653-58, 2013) used CRISPR/Cas9 to correct a defect associated with cystic fibrosis in human stem cells. The team's target was the gene for an ion channel, cystic fibrosis transmembrane conductor receptor (CFTR). A deletion in CFTR causes the protein to misfold in cystic fibrosis patients. Using cultured intestinal stem cells developed from cell samples from two children with cystic fibrosis, Schwank et al. were able to correct the defect using CRISPR along with a donor plasmid containing the reparative sequence to be inserted. The researchers then grew the cells into intestinal "organoids," or miniature guts, and showed that they functioned normally. In this case, about half of clonal organoids underwent the proper genetic correction.

Muscles

The present invention also contemplates delivering the CRISPR-Cas system to muscle(s). Bortolanza et al. (Molecular Therapy vol. 19 no. 11, 2055-2064 Nov. 2011) shows that systemic delivery of RNA interference expression cassettes in the FRG1 mouse, after the onset of facioscapulohumeral muscular dystrophy (FSHD), led to a dose-dependent long-term FRG1 knockdown without signs of toxicity. Bortolanza et al. found that a single intravenous injection of $5 \times 10^{12}$ vg of rAAV6-sh1FRG1 rescues muscle histopathology and muscle function of FRG1 mice. In detail, 200 $\mu$l containing $2 \times 10^{12}$ or $5 \times 10^{12}$ vg of vector in physiological solution were injected into the tail vein using a 25-gauge Terumo syringe. The method of Bortolanza et al. may be applied to an AAV expressing CRISPR Cas and injected into humans at a dosage of about $2 \times 10^{15}$ or $2 \times 10^{16}$ vg of vector.

Dumonceaux et al. (Molecular Therapy vol. 18 no. 5, 881-887 May 2010) inhibit the myostatin pathway using the technique of RNA interference directed against the myostatin receptor AcvRIIb mRNA (sh-AcvRIIb). The restoration of a quasi-dystrophin was mediated by the vectorized U7 exon-skipping technique (U7-DYS). Adeno-associated vectors carrying either the sh-AcvrIIb construct alone, the U7-DYS construct alone, or a combination of both constructs were injected in the tibialis anterior (TA) muscle of dystrophic mdx mice. The injections were performed with $10^{11}$ AAV viral genomes. The method of Dumonceaux et al. may be applied to an AAV expressing CRISPR Cas and injected into humans, for example, at a dosage of about $10^{14}$ to about $10^{15}$ vg of vector.

Kinouchi et al. (Gene Therapy (2008) 15, 1126-1130) report the effectiveness of in vivo siRNA delivery into skeletal muscles of normal or diseased mice through nanoparticle formation of chemically unmodified siRNAs with atelocollagen (ATCOL). ATCOL-mediated local application of siRNA targeting myostatin, a negative regulator of skeletal muscle growth, in mouse skeletal muscles or intravenously, caused a marked increase in the muscle mass within a few weeks after application. These results imply that ATCOL-mediated application of siRNAs is a powerful tool for future therapeutic use for diseases including muscular atrophy. Mst-siRNAs (final concentration, 10 mM) were mixed with ATCOL (final concentration for local administration, 0.5%) (AteloGene, Kohken, Tokyo, Japan) according to the manufacturer's instructions. After anesthesia of mice (20-week-old male C57BL/6) by Nembutal (25 mg/kg, i.p.), the Mst-siRNA/ATCOL complex was injected into the masseter and biceps femoris muscles. The method of Kinouchi et al. may be applied to CRISPR Cas and injected into a human, for example, at a dosage of about 500 to 1000 ml of a 40 $\mu$M solution into the muscle.

Hagstrom et al. (Molecular Therapy Vol. 10, No. 2, August 2004) describe an intravascular, nonviral methodology that enables efficient and repeatable delivery of nucleic acids to muscle cells (myofibers) throughout the limb muscles of mammals. The procedure involves the injection of naked plasmid DNA or siRNA into a distal vein of a limb that is transiently isolated by a tourniquet or blood pressure cuff. Nucleic acid delivery to myofibers is facilitated by its rapid injection in sufficient volume to enable extravasation of the nucleic acid solution into muscle tissue. High levels of transgene expression in skeletal muscle were achieved in both small and large animals with minimal toxicity. Evidence of siRNA delivery to limb muscle was also obtained. For plasmid DNA intravenous injection into a rhesus monkey, a threeway stopcock was connected to two syringe pumps (Model PHD 2000; Harvard Instruments), each loaded with a single syringe. Five minutes after a papaverine injection, pDNA (15.5 to 25.7 mg in 40 -100 ml saline) was injected at a rate of 1.7 or 2.0 ml/s. This could be scaled up for plasmid DNA expressing CRISPR Cas of the present invention with an injection of about 300 to 500 mg in 800 to 2000 ml saline for a human. For adenoviral vector injections into a rat, 2 x $10^9$ infectious particles were injected in 3 ml of normal saline solution (NSS). This could be scaled up for an adenoviral vector expressing CRISPR Cas of the present invention with an injection of about 1 x $10^{13}$ infectious particles were injected in 10 liters of NSS for a human. For siRNA, a rat was injected into the great saphenous vein with 12.5 μg of a siRNA and a primate was injected injected into the great saphenous vein with 750 μg of a siRNA. This could be scaled up for a CRISPR Cas of the present invention, for example, with an injection of about 15 to about 50 mg into the great saphenous vein of a human.

Skin

The present invention also contemplates delivering the CRISPR-Cas system to the skin. Hickerson et al. (Molecular Therapy-Nucleic Acids (2013) 2, e129) relates to a motorized microneedle array skin delivery device for delivering self-delivery (sd)-siRNA to human and murine skin. The primary challenge to translating siRNA-based skin therapeutics to the clinic is the development of effective delivery systems. Substantial effort has been invested in a variety of skin delivery technologies with limited success. In a clinical study in which skin was treated with siRNA, the exquisite pain associated with the hypodermic needle injection precluded enrollment of additional patients in the trial, highlighting the need for improved, more "patient-friendly" (i.e., little or no pain) delivery approaches. Microneedles represent an efficient way to deliver large charged cargos including siRNAs across the primary barrier, the stratum corneum, and are generally regarded as less painful than conventional hypodermic needles. Motorized "stamp type" microneedle devices, including the motorized microneedle array (MMNA) device used by Hickerson et al., have been shown to be safe in hairless mice studies and cause little or no pain as evidenced by (i) widespread use in the cosmetic industry and (ii) limited testing in which nearly all volunteers found use of the device to be much less painful than a flushot, suggesting siRNA delivery using this device will result in much less pain than was experienced in the previous clinical trial using hypodermic needle injections. The MMNA device (marketed as Triple-M or Tri-M by Bomtech Electronic Co, Seoul, South Korea) was adapted for delivery of siRNA to mouse and human skin. sd-siRNA solution (up to 300 μl of 0.1 mg/ml RNA) was introduced into the chamber of the disposable Tri-M needle cartridge (Bomtech), which was set to a depth of 0.1 mm. For treating human skin, deidentified skin (obtained immediately following surgical procedures) was manually stretched and pinned to a cork platform before treatment. All intradermal injections were performed using an insulin syringe with a 28-gauge 0.5-inch needle. The MMNA device and method of Hickerson et al. could be used and/or adapted to deliver the CRISPR Cas of the present invention, for example, at a dosage of up to 300 μl of 0.1 mg/ml CRISPR Cas to the skin. Leachman et al. (Molecular Therapy, vol. 18 no. 2, 442-446 Feb. 2010) relates to a phase Ib clinical trial for treatment of a rare skin disorder pachyonychia congenita (PC), an autosomal dominant syndrome that includes a disabling plantar keratoderma, utilizing the first short-interfering RNA (siRNA)-based therapeutic for skin. This siRNA, called TD101, specifically and potently targets the keratin 6a (K6a) N171K mutant mRNA without affecting wild-type K6a mRNA. The dose-escalation schedule is presented below:

| Week | Dose no. | Days | Volume (ml) | Concentration of TD101 (mg/ml) | Total dose TD101 (mg) |
|---|---|---|---|---|---|
| 1 | 1-2 | 1-7 | 0.1 | 1.0 | 0.10 |
| 2 | 3-4 | 8-14 | 0.25 | 1.0 | 0.25 |
| 3 | 5-6 | 15-21 | 0.50 | 1.0 | 0.50 |
| 4 | 7-8 | 22-28 | 1.0 | 1.0 | 1.0 |
| 5 | 9-10 | 29-35 | 1.5 | 1.0 | 1.5 |
| 6 | 11-12 | 36-42 | 2.0 | 1.0 | 2.0 |
| 7 | 13-14 | 43-49 | 2.0 | 1.5 | 3.0 |
| 8 | 15-16 | 50-56 | 2.0 | 2.0 | 4.0 |
| 9 | 17-18 | 57-63 | 2.0 | 2.5 | 5.0 |
| 10 | 19-20 | 64-70 | 2.0 | 3.0 | 6.0 |
| 11 | 21-22 | 71-77 | 2.0 | 3.5 | 7.0 |
| 12 | 23-24 | 78-84 | 2.0 | 4.0 | 8.0 |
| 13 | 25-26 | 85-91 | 2.0 | 4.5 | 9.0 |
| 14 | 27-28 | 92-98 | 2.0 | 5.0 | 10.0 |

(continued)

| Week | Dose no. | Days | Volume (ml) | Concentration of TD101 (mg/ml) | Total dose TD101 (mg) |
|---|---|---|---|---|---|
| 15 | 29-30 | 99-105 | 2.0 | 6.0 | 12.0 |
| 16 | 31-32 | 106-112 | 2.0 | 7.0 | 14.0 |
| 17 | 33 | 113-119 | 2.0 | 8.5 | 17.0 |

Initially, 0.1 ml of a 1.0 mg/ml solution of TD101 or vehicle alone (Dulbecco's phosphate-buffered saline without calcium or magnesium) was administered to symmetric calluses. Six rising dose-volumes were completed without an adverse reaction to the increases: 0.1, 0.25, 0.5, 1.0, 1.5, and 2.0 ml of a 1.0 mg/ml solution of TD101 solution per injection. As the highest planned volume (2.0 ml) was well tolerated, the concentration of TD101 was then increased each week from 1 mg/ml up to a final concentration of 8.5 mg/ml. Similar dosages are contemplated for the administration of a CRISPR Cas that specifically and potently targets the keratin 6a (K6a) N171K mutant mRNA.

Zheng et al. (PNAS, July 24, 2012, vol. 109, no. 30, 11975-11980) show that spherical nucleic acid nanoparticle conjugates (SNA-NCs), gold cores surrounded by a dense shell of highly oriented, covalently immobilized siRNA, freely penetrate almost 100% of keratinocytes in vitro, mouse skin, and human epidermis within hours after application. Zheng et al. demonstrated that a single application of 25 nM epidermal growth factor receptor (EGFR) SNA-NCs for 60 h demonstrate effective gene knockdown in human skin. A similar dosage may be contemplated for CRISPR Cas immobilized in SNA-NCs for administration to the skin.

Hepatitis viruses

The present invention may also be applied to treat hepatitis B virus (HBV). However, the CRISPR Cas system must be adapted to avoid the shortcomings of RNAi, such as the risk of oversatring endogenous small RNA pathways, by for example, optimizing dose and sequence (see, e.g., Grimm et al., Nature vol. 441, 26 May 2006). For example, low doses, such as about 1-10 x $10^{14}$ particles per humane are contemplated. In another embodiment, the CRISPR Cas system directed against HBV may be administered in liposomes, such as a stable nucleic-acid-lipid particle (SNALP) (see, e.g., Morrissey et al., Nature Biotechnology, Vol. 23, No. 8, August 2005). Daily intravenous injections of about 1, 3 or 5 mg/kg/day of CRISPR Cas targeted to HBV RNA in a SNALP are contemplated. The daily treatment may be over about three days and then weekly for about five weeks. In another embodiment, the system of Chen et al. (Gene Therapy (2007) 14, 11-19) may be used/and or adapted for the CRISPR Cas system of the present invention. Chen et al. use a double-stranded adenoassociated virus 8-pseudotyped vector (dsAAV2/8) to deliver shRNA. A single administration of dsAAV2/8 vector (1 x $10^{12}$ vector genomes per mouse), carrying HBV-specific shRNA, effectively suppressed the steady level of HBV protein, mRNA and replicative DNA in liver of HBV transgenic mice, leading to up to 2-3 $\log_{10}$ decrease in HBV load in the circulation. Significant HBV suppression sustained for at least 120 days after vector administration. The therapeutic effect of shRNA was target sequence dependent and did not involve activation of interferon. For the present invention, a CRISPR Cas system directed to HBV may be cloned into an AAV vector, such as a dsAAV2/8 vector and administered to a human, for example, at a dosage of about 1 x $10^{15}$ vector genomes to about 1 x $10^{16}$ vector genomes per human. In another embodiment, the method of Wooddell et al. (Molecular Therapy vol. 21 no. 5, 973-985 May 2013) may be used/and or adapted to the CRISPR Cas system of the present invention. Woodell et al. show that simple coinjection of a hepatocyte-targeted, N-acetylgalactosamine-conjugated melittin-like peptide (NAG-MLP) with a liver-tropic cholesterol-conjugated siRNA (chol-siRNA) targeting coagulation factor VII (F7) results in efficient F7 knockdown in mice and nonhuman primates without changes in clinical chemistry or induction of cytokines. Using transient and transgenic mouse models of HBV infection, Wooddell et al. show that a single coinjection of NAG-MLP with potent chol-siRNAs targeting conserved HBV sequences resulted in multilog repression of viral RNA, proteins, and viral DNA with long duration of effect. Intraveinous coinjections, for example, of about 6 mg/kg of NAG-MLP and 6 mg/kg of HBV specific CRISPR Cas may be envisioned for the present invention. In the alternative, about 3 mg/kg of NAG-MLP and 3 mg/kg of HBV specific CRISPR Cas may be delivered on day one, followed by administration of about about 2-3 mg/kg of NAG-MLP and 2-3 mg/kg of HBV specific CRISPR Cas two weeks later.

The present invention may also be applied to treat hepatitis C virus (HCV). The methods of Roelvinki et al. (Molecular Therapy vol. 20 no. 9, 1737-1749 Sep 2012) may be applied to the CRISPR Cas system. For example, an AAV vector such as AAV8 may be a contemplated vector and for example a dosage of about $1.25 \times 10^{11}$ to $1.25 \times 10^{13}$ vector genomes per kilogram body weight (vg/kg) may be contemplated.

It will be readily apparent that a host of other diseases can be treated in a similar fashion. Some examples of genetic diseases caused by mutations are provided herein, but many more are known. The above strategy can be applied to these diseases.

Huntington's Disease (HP)

RNA interference (RNAi) offers therapeutic potential for this disorder by reducing the expression of *HTT,* the disease-causing gene of Huntington's disease (see, e.g., McBride et al., Molecular Therapy vol. 19 no. 12 Dec. 2011, pp. 2152-2162), therefore Applicant postulates that it may be used/and or adapted to the CRISPR-Cas system. The CRISPR-Cas system may be generated using an algorithm to reduce the off-targeting potential of antisense sequences. The CRISPR-Cas sequences may target either a sequence in exon 52 of mouse, rhesus or human huntingtin and expressed in a viral vector, such as AAV. Animals, including humans, may be injected with about three microinjections per hemisphere (six injections total): the first 1 mm rostral to the anterior commissure (12 μl) and the two remaining injections (12 μl and 10 μl, respectively) spaced 3 and 6 mm caudal to the first injection with 1e12 vg/ml of AAV at a rate of about 1 μl/minute, and the needle was left in place for an additional 5 minutes to allow the injectate to diffuse from the needle tip. DiFiglia et al. (PNAS, October 23, 2007, vol. 104, no. 43, 17204-17209) observed that single administration into the adult striatum of an siRNA targeting Htt can silence mutant Htt, attenuate neuronal pathology, and delay the abnormal behavioral phenotype observed in a rapid-onset, viral transgenic mouse model of HD. DiFiglia injected mice intrastriatally with 2 μl of Cy3-labeled cc-siRNA-Htt or unconjugated siRNA-Htt at 10 μM. A similar dosage of CRISPR Cas targeted to Htt may be contemplated for humans in the present invention, for example, about 5-10 ml of 10 μM CRISPR Cas targeted to Htt may be injected intrastriatally.

In another example, Boudreau et al. (Molecular Therapy vol. 17 no. 6 june 2009) injects 5 μl of recombinant AAV serotype 2/1 vectors expressing htt-specific RNAi virus (at 4 x $10^{12}$ viral genomes/ml) into the straiatum. A similar dosage of CRISPR Cas targeted to Htt may be contemplated for humans in the present invention, for example, about 10-20 ml of 4 x $10^{12}$ viral genomes/ml) CRISPR Cas targeted to Htt may be injected intrastriatally. In another example, a CRISPR Cas targetd to HTT may be administered continuously (see, e.g., Yu et al., Cell 150, 895-908, August 31, 2012). Yu et al. utilizes osmotic pumps delivering 0.25 ml/hr (Model 2004) to deliver 300 mg/day of ss-siRNA or phosphate-buffered saline (PBS) (Sigma Aldrich) for 28 days, and pumps designed to deliver 0.5 μl/hr (Model 2002) were used to deliver 75 mg/day of the positive control MOE ASO for 14 days. Pumps (Durect Corporation) were filled with ss-siRNA or MOE diluted in sterile PBS and then incubated at 37 C for 24 or 48 (Model 2004) hours prior to implantation. Mice were anesthetized with 2.5% isofluorane, and a midline incision was made at the base of the skull. Using stereotaxic guides, a cannula was implanted into the right lateral ventricle and secured with Loctite adhesive. A catheter attached to an Alzet osmotic mini pump was attached to the cannula, and the pump was placed subcutaneously in the midscapular area. The incision was closed with 5.0 nylon sutures. A similar dosage of CRISPR Cas targeted to Htt may be contemplated for humans in the present invention, for example, about 500 to 1000 g/day CRISPR Cas targeted to Htt may be administered. In another example of continuous infusion, Stiles et al. (Experimental Neurology 233 (2012) 463-471) implanted an intraparenchymal catheter with a titanium needle tip into the right putamen. The catheter was connected to a SynchroMed® II Pump (Medtronic Neurological, Minneapolis, MN) subcutaneously implanted in the abdomen. After a 7 day infusion of phosphate buffered saline at 6 μL/day, pumps were re-filled with test article and programmed for continuous delivery for 7 days. About 2.3 to 11.52 mg/d of siRNA were infused at varying infusion rates of about 0.1 to 0.5 μL/min. A similar dosage of CRISPR Cas targeted to Htt may be contemplated for humans in the present invention, for example, about 20 to 200 mg/day CRISPR Cas targeted to Htt may be administered. In another example, the methods of US Patent Publication No. 20130253040 assigned to Sangamo may also be also be adapted from TALES to the CRISPR Cas system of the present invention for treating Huntington's Disease.

Nucleic acids, amino acids and proteins

The invention uses nucleic acids to bind target DNA sequences. This is advantageous as nucleic acids are much easier and cheaper to produce than proteins, and the specificity can be varied according to the length of the stretch where homology is sought. Complex 3-D positioning of multiple fingers, for example is not required.

The terms "polynucleotide", "nucleotide", "nucleotide sequence", "nucleic acid" and "oligonucleotide" are used interchangeably. They refer to a polymeric form of nucleotides of any length, either deoxyribonucleotides or ribonucleotides, or analogs thereof. Polynucleotides may have any three dimensional structure, and may perform any function, known or unknown. The following are non-limiting examples of polynucleotides: coding or non-coding regions of a gene or gene fragment, loci (locus) defined from linkage analysis, exons, introns, messenger RNA (mRNA), transfer RNA, ribosomal RNA, short interfering RNA (siRNA), short-hairpin RNA (shRNA), micro-RNA (miRNA), ribozymes, cDNA, recombinant polynucleotides, branched polynucleotides, plasmids, vectors, isolated DNA of any sequence, isolated RNA of any sequence, nucleic acid probes, and primers. The term also encompasses nucleic-acid-like structures with synthetic backbones, see, e.g., Eckstein, 1991; Baserga et al., 1992; Milligan, 1993; WO 97/03211; WO 96/39154; Mata, 1997; Strauss-Soukup, 1997; and Samstag, 1996. A polynucleotide may comprise one or more modified nucleotides, such as methylated nucleotides and nucleotide analogs. If present, modifications to the nucleotide structure may be imparted before or after assembly of the polymer. The sequence of nucleotides may be interrupted by non-nucleotide components. A polynucleotide may be further modified after polymerization, such as by conjugation with a labeling component.

As used herein the term "wild type" is a term of the art understood by skilled persons and means the typical form of an organism, strain, gene or characteristic as it occurs in nature as distinguished from mutant or variant forms.

As used herein the term "variant" should be taken to mean the exhibition of qualities that have a pattern that deviates from what occurs in nature.

The terms "non-naturally occurring" or "engineered" are used interchangeably and indicate the involvement of the hand of man. The terms, when referring to nucleic acid molecules or polypeptides mean that the nucleic acid molecule or the polypeptide is at least substantially free from at least one other component with which they are naturally associated in nature and as found in nature.

"Complementarity" refers to the ability of a nucleic acid to form hydrogen bond(s) with another nucleic acid sequence by either traditional Watson-Crick base pairing or other non-traditional types. A percent complementarity indicates the percentage of residues in a nucleic acid molecule which can form hydrogen bonds (e.g., Watson-Crick base pairing) with a second nucleic acid sequence (e.g., 5, 6, 7, 8, 9, 10 out of 10 being 50%, 60%, 70%, 80%, 90%, and 100% complementary). "Perfectly complementary" means that all the contiguous residues of a nucleic acid sequence will hydrogen bond with the same number of contiguous residues in a second nucleic acid sequence. "Substantially complementary" as used herein refers to a degree of complementarity that is at least 60%, 65%, 70%, 75%, 80%, 85%, 90%, 95%, 97%, 98%, 99%, or 100% over a region of 8, 9, 10, 11, 12, 13, 14, 15, 16, 17, 18, 19, 20, 21, 22, 23, 24, 25, 30, 35, 40, 45, 50, or more nucleotides, or refers to two nucleic acids that hybridize under stringent conditions.

As used herein, "stringent conditions" for hybridization refer to conditions under which a nucleic acid having complementarity to a target sequence predominantly hybridizes with the target sequence, and substantially does not hybridize to non-target sequences. Stringent conditions are generally sequence-dependent, and vary depending on a number of factors. In general, the longer the sequence, the higher the temperature at which the sequence specifically hybridizes to its target sequence. Non-limiting examples of stringent conditions are described in detail in Tijssen (1993), Laboratory Techniques In Biochemistry And Molecular Biology-Hybridization With Nucleic Acid Probes Part I, Second Chapter "Overview of principles of hybridization and the strategy of nucleic acid probe assay", Elsevier, N.Y. Where reference is made to a polynucleotide sequence, then complementary or partially complementary sequences are also envisaged. These are preferably capable of hybridising to the reference sequence under highly stringent conditions. Generally, in order to maximize the hybridization rate, relatively low-stringency hybridization conditions are selected: about 20 to 25° C lower than the thermal melting point ($T_m$). The $T_m$ is the temperature at which 50% of specific target sequence hybridizes to a perfectly complementary probe in solution at a defined ionic strength and pH. Generally, in order to require at least about 85% nucleotide complementarity of hybridized sequences, highly stringent washing conditions are selected to be about 5 to 15° C lower than the $T_m$. In order to require at least about 70% nucleotide complementarity of hybridized sequences, moderately-stringent washing conditions are selected to be about 15 to 30° C lower than the $T_m$. Highly permissive (very low stringency) washing conditions may be as low as 50° C below the $T_m$, allowing a high level of mis-matching between hybridized sequences. Those skilled in the art will recognize that other physical and chemical parameters in the hybridization and wash stages can also be altered to affect the outcome of a detectable hybridization signal from a specific level of homology between target and probe sequences. Preferred highly stringent conditions comprise incubation in 50% formamide, 5×SSC, and 1% SDS at 42° C, or incubation in 5×SSC and 1% SDS at 65° C, with wash in 0.2×SSC and 0.1% SDS at 65° C.

"Hybridization" refers to a reaction in which one or more polynucleotides react to form a complex that is stabilized via hydrogen bonding between the bases of the nucleotide residues. The hydrogen bonding may occur by Watson Crick base pairing, Hoogstein binding, or in any other sequence specific manner. The complex may comprise two strands forming a duplex structure, three or more strands forming a multi stranded complex, a single self-hybridizing strand, or any combination of these. A hybridization reaction may constitute a step in a more extensive process, such as the initiation of PCR, or the cleavage of a polynucleotide by an enzyme. A sequence capable of hybridizing with a given sequence is referred to as the "complement" of the given sequence.

As used herein, the term "genomic locus" or "locus" (plural loci) is the specific location of a gene or DNA sequence on a chromosome. A "gene" refers to stretches of DNA or RNA that encode a polypeptide or an RNA chain that has functional role to play in an organism and hence is the molecular unit of heredity in living organisms. For the purpose of this invention it may be considered that genes include regions which regulate the production of the gene product, whether or not such regulatory sequences are adjacent to coding and/or transcribed sequences. Accordingly, a gene includes, but is not necessarily limited to, promoter sequences, terminators, translational regulatory sequences such as ribosome binding sites and internal ribosome entry sites, enhancers, silencers, insulators, boundary elements, replication origins, matrix attachment sites and locus control regions.

As used herein, "expression of a genomic locus" or "gene expression" is the process by which information from a gene is used in the synthesis of a functional gene product. The products of gene expression are often proteins, but in non-protein coding genes such as rRNA genes or tRNA genes, the product is functional RNA. The process of gene expression is used by all known life - eukaryotes (including multicellular organisms), prokaryotes (bacteria and archaea) and viruses to generate functional products to survive. As used herein "expression" of a gene or nucleic acid encompasses not only cellular gene expression, but also the transcription and translation of nucleic acid(s) in cloning systems and in any other context. As used herein, "expression" also refers to the process by which a polynucleotide is transcribed from a DNA template (such as into and mRNA or other RNA transcript) and/or the process by which a transcribed mRNA is subsequently translated into peptides, polypeptides, or proteins. Transcripts and encoded polypeptides may be collectively referred to as "gene product." If the polynucleotide is derived from genomic DNA, expression may include splicing of the mRNA in a eukaryotic cell.

The terms "polypeptide", "peptide" and "protein" are used interchangeably herein to refer to polymers of amino acids of any length. The polymer may be linear or branched, it may comprise modified amino acids, and it may be interrupted by non amino acids. The terms also encompass an amino acid polymer that has been modified; for example, disulfide bond formation, glycosylation, lipidation, acetylation, phosphorylation, or any other manipulation, such as conjugation with a labeling component. As used herein the term "amino acid" includes natural and/or unnatural or synthetic amino acids, including glycine and both the D or L optical isomers, and amino acid analogs and peptidomimetics.

As used herein, the term "domain" or "protein domain" refers to a part of a protein sequence that may exist and function independently of the rest of the protein chain.

As described in aspects of the invention, sequence identity is related to sequence homology. Homology comparisons may be conducted by eye, or more usually, with the aid of readily available sequence comparison programs. These commercially available computer programs may calculate percent (%) homology between two or more sequences and may also calculate the sequence identity shared by two or more amino acid or nucleic acid sequences. In some preferred embodiments, the capping region of the dTALEs described herein have sequences that are at least 95% identical or share identity to the capping region amino acid sequences provided herein.

Sequence homologies may be generated by any of a number of computer programs known in the art, for example BLAST or FASTA, etc. A suitable computer program for carrying out such an alignment is the GCG Wisconsin Bestfit package (University of Wisconsin, U.S.A; Devereux et al., 1984, Nucleic Acids Research 12:387). Examples of other software than may perform sequence comparisons include, but are not limited to, the BLAST package (see Ausubel et al., 1999 ibid - Chapter 18), FASTA (Atschul et al., 1990, J. Mol. Biol., 403-410) and the GENEWORKS suite of comparison tools. Both BLAST and FASTA are available for offline and online searching (see Ausubel et al., 1999 ibid, pages 7-58 to 7-60). However it is preferred to use the GCG Bestfit program.

Percentage (%) sequence homology may be calculated over contiguous sequences, i.e., one sequence is aligned with the other sequence and each amino acid or nucleotide in one sequence is directly compared with the corresponding amino acid or nucleotide in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion may cause the following amino acid residues to be put out of alignment, thus potentially resulting in a large reduction in % homology when a global alignment is performed. Consequently, most sequence comparison methods are designed to produce optimal alignments that take into consideration possible insertions and deletions without unduly penalizing the overall homology or identity score. This is achieved by inserting "gaps" in the sequence alignment to try to maximize local homology or identity.

However, these more complex methods assign "gap penalties" to each gap that occurs in the alignment so that, for the same number of identical amino acids, a sequence alignment with as few gaps as possible - reflecting higher relatedness between the two compared sequences - may achieve a higher score than one with many gaps. "Affinity gap costs" are typically used that charge a relatively high cost for the existence of a gap and a smaller penalty for each subsequent residue in the gap. This is the most commonly used gap scoring system. High gap penalties may, of course, produce optimized alignments with fewer gaps. Most alignment programs allow the gap penalties to be modified. However, it is preferred to use the default values when using such software for sequence comparisons. For example, when using the GCG Wisconsin Bestfit package the default gap penalty for amino acid sequences is -12 for a gap and -4 for each extension.

Calculation of maximum % homology therefore first requires the production of an optimal alignment, taking into consideration gap penalties. A suitable computer program for carrying out such an alignment is the GCG Wisconsin Bestfit package (Devereux et al., 1984 Nuc. Acids Research 12 p387). Examples of other software than may perform sequence comparisons include, but are not limited to, the BLAST package (see Ausubel et al., 1999 Short Protocols in Molecular Biology, 4th Ed. - Chapter 18), FASTA (Altschul et al., 1990 J. Mol. Biol. 403-410) and the GENEWORKS suite of comparison tools. Both BLAST and FASTA are available for offline and online searching (see Ausubel et al., 1999, Short Protocols in Molecular Biology, pages 7-58 to 7-60). However, for some applications, it is preferred to use the GCG Bestfit program. A new tool, called BLAST 2 Sequences is also available for comparing protein and nucleotide sequences (see FEMS Microbiol Lett. 1999 174(2): 247-50; FEMS Microbiol Lett. 1999 177(1): 187-8 and the website of the National Center for Biotechnology information at the website of the National Institutes for Health).

Although the final % homology may be measured in terms of identity, the alignment process itself is typically not based on an all-or-nothing pair comparison. Instead, a scaled similarity score matrix is generally used that assigns scores to each pair-wise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table, if supplied (see user manual for further details). For some applications, it is preferred to use the public default values for the GCG package, or in the case of other software, the default matrix, such as BLOSUM62.

Alternatively, percentage homologies may be calculated using the multiple alignment feature in DNASIS™ (Hitachi Software), based on an algorithm, analogous to CLUSTAL (Higgins DG & Sharp PM (1988), Gene 73(1), 237-244). Once the software has produced an optimal alignment, it is possible to calculate % homology, preferably % sequence identity. The software typically does this as part of the sequence comparison and generates a numerical result.

The sequences may also have deletions, insertions or substitutions of amino acid residues which produce a silent change and result in a functionally equivalent substance. Deliberate amino acid substitutions may be made on the basis of similarity in amino acid properties (such as polarity, charge, solubility, hydrophobicity, hydrophilicity, and/or the amphipathic nature of the residues) and it is therefore useful to group amino acids together in functional groups. Amino acids may be grouped together based on the properties of their side chains alone. However, it is more useful to include mutation data as well. The sets of amino acids thus derived are likely to be conserved for structural reasons. These sets may be described in the form of a Venn diagram (Livingstone C.D. and Barton G.J. (1993) "Protein sequence alignments: a strategy for the hierarchical analysis of residue conservation" Comput. Appl. Biosci. 9: 745-756) (Taylor W.R. (1986) "The classification of amino acid conservation" J. Theor. Biol. 119; 205-218). Conservative substitutions may be made, for example according to the table below which describes a generally accepted Venn diagram grouping of amino acids.

| Set | | Sub-set | |
|---|---|---|---|
| Hydrophobic | F W Y H K M I L V A G C | Aromatic | F W Y H |
| | | Aliphatic | I L V |
| Polar | W Y H K R E D C S T N Q | Charged | H K R E D |
| | | Positively charged | H K R |
| | | Negatively charged | E D |
| Small | V C A G S P T N D | Tiny | A G S |

Embodiments of the invention include sequences (both polynucleotide or polypeptide) which may comprise homologous substitution (substitution and replacement are both used herein to mean the interchange of an existing amino acid residue or nucleotide, with an alternative residue or nucleotide) that may occur i.e., like-for-like substitution in the case of amino acids such as basic for basic, acidic for acidic, polar for polar, etc. Non-homologous substitution may also occur i.e., from one class of residue to another or alternatively involving the inclusion of unnatural amino acids such as ornithine (hereinafter referred to as Z), diaminobutyric acid ornithine (hereinafter referred to as B), norleucine ornithine (hereinafter referred to as O), pyriylalanine, thienylalanine, naphthylalanine and phenylglycine.

Variant amino acid sequences may include suitable spacer groups that may be inserted between any two amino acid residues of the sequence including alkyl groups such as methyl, ethyl or propyl groups in addition to amino acid spacers such as glycine or β-alanine residues. A further form of variation, which involves the presence of one or more amino acid residues in peptoid form, may be well understood by those skilled in the art. For the avoidance of doubt, "the peptoid form" is used to refer to variant amino acid residues wherein the α-carbon substituent group is on the residue's nitrogen atom rather than the α-carbon. Processes for preparing peptides in the peptoid form are known in the art, for example Simon RJ et al., PNAS (1992) 89(20), 9367-9371 and Horwell DC, Trends Biotechnol. (1995) 13(4), 132-134.

The practice of the present invention employs, unless otherwise indicated, conventional techniques of immunology, biochemistry, chemistry, molecular biology, microbiology, cell biology, genomics and recombinant DNA, which are within the skill of the art. See Sambrook, Fritsch and Maniatis, MOLECULAR CLONING: A LABORATORY MANUAL, 2nd edition (1989); CURRENT PROTOCOLS IN MOLECULAR BIOLOGY (F. M. Ausubel, et al. eds., (1987)); the series METHODS IN ENZYMOLOGY (Academic Press, Inc.): PCR 2: A PRACTICAL APPROACH (M.J. MacPherson, B.D. Hames and G.R. Taylor eds. (1995)), Harlow and Lane, eds. (1988) ANTIBODIES, A LABORATORY MANUAL, and ANIMAL CELL CULTURE (R.I. Freshney, ed. (1987)).

Vectors

In one aspect, the invention provides for vectors that are used in the engineering and optimization of CRISPR-Cas systems.

A used herein, a "vector" is a tool that allows or facilitates the transfer of an entity from one environment to another. It is a replicon, such as a plasmid, phage, or cosmid, into which another DNA segment may be inserted so as to bring about the replication of the inserted segment. Generally, a vector is capable of replication when associated with the proper control elements. In general, the term "vector" refers to a nucleic acid molecule capable of transporting another nucleic acid to which it has been linked. Vectors include, but are not limited to, nucleic acid molecules that are single-stranded, double-stranded, or partially double-stranded; nucleic acid molecules that comprise one or more free ends, no free ends (e.g. circular); nucleic acid molecules that comprise DNA, RNA, or both; and other varieties of polynucleotides known in the art. One type of vector is a "plasmid," which refers to a circular double stranded DNA loop into which additional DNA segments can be inserted, such as by standard molecular cloning techniques. Another type of vector is a viral vector, wherein virally-derived DNA or RNA sequences are present in the vector for packaging into a virus (e.g. retroviruses, replication defective retroviruses, adenoviruses, replication defective adenoviruses, and adeno-associated viruses (AAVs)). Viral vectors also include polynucleotides carried by a virus for transfection into a host cell. Certain vectors are capable of autonomous replication in a host cell into which they are introduced (e.g. bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors are capable of directing the expression of genes to which they are operatively-linked. Such vectors are referred to herein as "expression vectors." Common expression vectors of utility in recombinant DNA techniques are often in the form of plasmids.

Recombinant expression vectors can comprise a nucleic acid of the invention in a form suitable for expression of the nucleic acid in a host cell, which means that the recombinant expression vectors include one or more regulatory elements, which may be selected on the basis of the host cells to be used for expression, that is operatively-linked to the nucleic acid sequence to be expressed. Within a recombinant expression vector, "operably linked" is intended to mean that the nucleotide sequence of interest is linked to the regulatory element(s) in a manner that allows for expression of the nucleotide sequence (e.g. in an in vitro transcription/translation system or in a host cell when the vector is introduced into the host cell). With regards to recombination and cloning methods, mention is made of U.S. patent application 10/815,730, published September 2, 2004 as US 2004-0171156 A1, the contents of which are herein incorporated by reference in their entirety.

Aspects of the invention relate to bicistronic vectors for chimeric RNA and Cas9. Bicistronic expression vectors for chimeric RNA and Cas9 are preferred. In general and particularly in this embodiment Cas9 is preferably driven by the CBh promoter. The chimeric RNA may preferably be driven by a Pol III promoter, such as a U6 promoter. Ideally the two are combined. The chimeric guide RNA typically consists of a 20bp guide sequence (Ns) and this may be joined to the tracr sequence (running from the first "U" of the lower strand to the end of the transcript). The tracr sequence may be truncated at various positions as indicated. The guide and tracr sequences are separated by the tracr-mate sequence, which may be GUUUUAGAGCUA. This may be followed by the loop sequence GAAA as shown. Both of these are preferred examples. Applicants have demonstrated Cas9-mediated indels at the human *EMX1* and *PVALB* loci by SURVEYOR assays. ChiRNAs are indicated by their "+n" designation, and crRNA refers to a hybrid RNA where guide and tracr sequences are expressed as separate transcripts. Throughout this application, chimeric RNA may also be called single guide, or synthetic guide RNA (sgRNA). The loop is preferably GAAA, but it is not limited to this sequence or indeed to being only 4bp in length. Indeed, preferred loop forming sequences for use in hairpin structures are four nucleotides in length, and most preferably have the sequence GAAA. However, longer or shorter loop sequences may be used, as may alternative sequences. The sequences preferably include a nucleotide triplet (for example, AAA), and an additional nucleotide (for example C or G). Examples of loop forming sequences include CAAA and AAAG.

The term "regulatory element" is intended to include promoters, enhancers, internal ribosomal entry sites (IRES), and other expression control elements (e.g. transcription termination signals, such as polyadenylation signals and poly-U sequences). Such regulatory elements are described, for example, in Goeddel, GENE EXPRESSION TECHNOLOGY: METHODS IN ENZYMOLOGY 185, Academic Press, San Diego, Calif. (1990). Regulatory elements include those that direct constitutive expression of a nucleotide sequence in many types of host cell and those that direct expression of the nucleotide sequence only in certain host cells (e.g., tissue-specific regulatory sequences). A tissue-specific promoter may direct expression primarily in a desired tissue of interest, such as muscle, neuron, bone, skin, blood, specific organs (e.g. liver, pancreas), or particular cell types (e.g. lymphocytes). Regulatory elements may also direct expression in a temporal-dependent manner, such as in a cell-cycle dependent or developmental stage-dependent manner, which may or may not also be tissue or cell-type specific. In some embodiments, a vector comprises one or more pol III promoter (e.g. 1, 2, 3, 4, 5, or more pol III promoters), one or more pol II promoters (e.g. 1, 2, 3, 4, 5, or more pol II promoters), one or more pol I promoters (e.g. 1, 2, 3, 4, 5, or more pol I promoters), or combinations thereof. Examples of pol III promoters include, but are not limited to, U6 and H1 promoters. Examples of pol II promoters include, but are not limited to, the retroviral Rous sarcoma virus (RSV) LTR promoter (optionally with the RSV enhancer), the cytomegalovirus (CMV) promoter (optionally with the CMV enhancer) [see, e.g., Boshart et al, Cell, 41:521-530 (1985)], the SV40 promoter, the dihydrofolate reductase promoter, the β-actin promoter, the phosphoglycerol kinase (PGK) promoter, and the EF1α promoter. Also encompassed by the term "regulatory element" are enhancer elements, such as WPRE; CMV enhancers; the R-U5' segment in LTR of HTLV-I (Mol. Cell. Biol., Vol. 8(1), p. 466-472, 1988); SV40 enhancer; and the intron sequence between exons 2 and 3 of rabbit β-globin (Proc. Natl. Acad. Sci. USA., Vol. 78(3), p. 1527-31, 1981). It will be appreciated by those skilled in the art that the design of the expression vector can depend on such factors as the choice of the host cell to be transformed, the level of expression desired, etc. A vector can be introduced into host cells to thereby produce transcripts, proteins, or peptides, including fusion proteins or peptides, encoded by nucleic acids as described herein (e.g., clustered regularly interspersed short palindromic repeats (CRISPR) transcripts, proteins, enzymes, mutant forms thereof, fusion proteins thereof, etc.). With regards to regulatory sequences, mention is made of U.S. patent application 10/491,026, the contents of which are incorporated by reference herein in their entirety. With regards to promoters, mention is made of PCT publication WO 2011/028929 and U.S. application 12/511,940, the contents of which are incorporated by reference herein in their entirety.

Vectors can be designed for expression of CRISPR transcripts (e.g. nucleic acid transcripts, proteins, or enzymes) in prokaryotic or eukaryotic cells. For example, CRISPR transcripts can be expressed in bacterial cells such as Escherichia coli, insect cells (using baculovirus expression vectors), yeast cells, or mammalian cells. Suitable host cells are discussed further in Goeddel, GENE EXPRESSION TECHNOLOGY: METHODS IN ENZYMOLOGY 185, Academic Press, San Diego, Calif. (1990). Alternatively, the recombinant expression vector can be transcribed and translated in vitro, for example using T7 promoter regulatory sequences and T7 polymerase.

Vectors may be introduced and propagated in a prokaryote or prokaryotic cell. In some embodiments, a prokaryote is used to amplify copies of a vector to be introduced into a eukaryotic cell or as an intermediate vector in the production of a vector to be introduced into a eukaryotic cell (e.g. amplifying a plasmid as part of a viral vector packaging system). In some embodiments, a prokaryote is used to amplify copies of a vector and express one or more nucleic acids, such as to provide a source of one or more proteins for delivery to a host cell or host organism. Expression of proteins in prokaryotes is most often carried out in Escherichia coli with vectors containing constitutive or inducible promoters directing the expression of either fusion or non-fusion proteins. Fusion vectors add a number of amino acids to a protein encoded therein, such as to the amino terminus of the recombinant protein. Such fusion vectors may serve one or more purposes, such as: (i) to increase expression of recombinant protein; (ii) to increase the solubility of the recombinant protein; and (iii) to aid in the purification of the recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Example fusion expression vectors include pGEX (Pharmacia Biotech Inc; Smith and Johnson, 1988. Gene 67: 31-40), pMAL (New England Biolabs, Beverly, Mass.) and pRIT5 (Pharmacia, Piscataway, N.J.) that fuse glutathione S-transferase (GST), maltose E binding protein, or protein A, respectively, to the target recombinant protein.

Examples of suitable inducible non-fusion E. coli expression vectors include pTrc (Amrann et al., (1988) Gene 69:301-315) and pET 11d (Studier et al., GENE EXPRESSION TECHNOLOGY: METHODS IN ENZYMOLOGY 185, Academic Press, San Diego, Calif. (1990) 60-89). In some embodiments, a vector is a yeast expression vector. Examples of vectors for expression in yeast Saccharomyces cerivisae include pYepSec1 (Baldari, et al., 1987. EMBO J. 6: 229-234), pMFa (Kuijan and Herskowitz, 1982. Cell 30: 933-943), pJRY88 (Schultz et al., 1987. Gene 54: 113-123), pYES2 (Invitrogen Corporation, San Diego, Calif.), and picZ (InVitrogen Corp, San Diego, Calif.). In some embodiments, a vector drives protein expression in insect cells using baculovirus expression vectors. Baculovirus vectors available for expression of proteins in cultured insect cells (e.g., SF9 cells) include the pAc series (Smith, et al., 1983. Mol. Cell. Biol. 3: 2156-2165) and the pVL series (Lucklow and Summers, 1989. Virology 170: 31-39).

In some embodiments, a vector is capable of driving expression of one or more sequences in mammalian cells using a mammalian expression vector. Examples of mammalian expression vectors include pCDM8 (Seed, 1987. Nature 329: 840) and pMT2PC (Kaufman, et al., 1987. EMBO J. 6: 187-195). When used in mammalian cells, the expression vector's control functions are typically provided by one or more regulatory elements. For example, commonly used promoters are derived from polyoma, adenovirus 2, cytomegalovirus, simian virus 40, and others disclosed herein and known in the art. For other suitable expression systems for both prokaryotic and eukaryotic cells see, e.g., Chapters 16 and 17 of Sambrook, et al., MOLECULAR CLONING: A LABORATORY MANUAL. 2nd ed., Cold Spring Harbor Laboratory, Cold Spring Harbor Laboratory Press, Cold Spring Harbor, N.Y., 1989.

In some embodiments, the recombinant mammalian expression vector is capable of directing expression of the nucleic acid preferentially in a particular cell type (e.g., tissue-specific regulatory elements are used to express the nucleic acid). Tissue-specific regulatory elements are known in the art. Non-limiting examples of suitable tissue-specific promoters include the albumin promoter (liver-specific; Pinkert, et al., 1987. Genes Dev. 1: 268-277), lymphoid-specific promoters (Calame and Eaton, 1988. Adv. Immunol. 43: 235-275), in particular promoters of T cell receptors (Winoto and Baltimore, 1989. EMBO J. 8: 729-733) and immunoglobulins (Baneiji, et al., 1983. Cell 33: 729-740; Queen and Baltimore, 1983. Cell 33: 741-748), neuron-specific promoters (e.g., the neurofilament promoter; Byrne and Ruddle, 1989. Proc. Natl. Acad. Sci. USA 86: 5473-5477), pancreas-specific promoters (Edlund, et al., 1985. Science 230: 912-916), and mammary gland-specific promoters (e.g., milk whey promoter; U.S. Pat. No. 4,873,316 and European Application Publication No. 264,166). Developmentally-regulated promoters are also encompassed, e.g., the murine hox promoters (Kessel and Gruss, 1990. Science 249: 374-379) and the $\alpha$-fetoprotein promoter (Campes and Tilghman, 1989. Genes Dev. 3: 537-546). With regards to these prokaryotic and eukaryotic vectors, mention is made of U.S. Patent 6,750,059, the contents of which are incorporated by reference herein in their entirety. Other embodiments of the invention may relate to the use of viral vectors, with regards to which mention is made of U.S. Patent application 13/092,085, the contents of which are incorporated by reference herein in their entirety. Tissue-specific regulatory elements are known in the art and in this regard, mention is made of U.S. Patent 7,776,321, the contents of which are incorporated by reference herein in their entirety.

Regulatory elements

In some embodiments, a regulatory element is operably linked to one or more elements of a CRISPR system so as to drive expression of the one or more elements of the CRISPR system. In general, CRISPRs (Clustered Regularly Interspaced Short Palindromic Repeats), also known as SPIDRs (SPacer Interspersed Direct Repeats), constitute a family of DNA loci that are usually specific to a particular bacterial species. The CRISPR locus comprises a distinct class of interspersed short sequence repeats (SSRs) that were recognized in E. coli (Ishino et al., J. Bacteriol., 169:5429-5433 [1987]; and Nakata et al., J. Bacteriol., 171:3553-3556 [1989]), and associated genes. Similar interspersed SSRs have been identified in Haloferax mediterranei, Streptococcus pyogenes, Anabaena, and Mycobacterium tuberculosis (See, Groenen et al., Mol. Microbiol., 10:1057-1065 [1993]; Hoe et al., Emerg. Infect. Dis., 5:254-263 [1999]; Masepohl et al., Biochim. Biophys. Acta 1307:26-30 [1996]; and Mojica et al., Mol. Microbiol., 17:85-93 [1995]). The CRISPR loci typically differ from other SSRs by the structure of the repeats, which have been termed short regularly spaced repeats (SRSRs) (Janssen et al., OMICS J. Integ. Biol., 6:23-33 [2002]; and Mojica et al., Mol. Microbiol., 36:244-246 [2000]). In general, the repeats are short elements that occur in clusters that are regularly spaced by unique intervening sequences with a substantially constant length (Mojica et al., [2000], supra). Although the repeat sequences are highly conserved between strains, the number of interspersed repeats and the sequences of the spacer regions typically differ from strain to strain (van Embden et al., J. Bacteriol., 182:2393-2401 [2000]). CRISPR loci have been identified in more than 40 prokaryotes (See e.g., Jansen et al., Mol. Microbiol., 43:1565-1575 [2002]; and Mojica et al., [2005]) including, but not limited to Aeropyrum, Pyrobaculum, Sulfolobus, Archaeoglobus, Halocarcula, Methanobacterium, Methanococcus, Methanosarcina, Methanopyrus, Pyrococcus, Picrophilus, Thermoplasma, Corynebacterium, Mycobacterium, Streptomyces, Aquifex, Porphyromonas, Chlorobium, Thermus, Bacillus, Listeria, Staphylococcus, Clostridium, Thermoanaerobacter, Mycoplasma, Fusobacterium, Azarcus, Chromobacterium, Neisseria, Nitrosomonas, Desulfovibrio, Geobacter, Myxococcus, Campylobacter, Wolinella, Acinetobacter, Erwinia, Escherichia, Legionella, Methylococcus, Pasteurella, Photobacterium, Salmonella, Xanthomonas, Yersinia, Treponema, and Thermotoga.

In general, "CRISPR system" refers collectively to transcripts and other elements involved in the expression of or directing the activity of CRISPR-associated ("Cas") genes, including sequences encoding a Cas gene, a tracr (trans-activating CRISPR) sequence (e.g. tracrRNA or an active partial tracrRNA), a tracr-mate sequence (encompassing a "direct repeat" and a tracrRNA-processed partial direct repeat in the context of an endogenous CRISPR system), a guide sequence (also referred to as a "spacer" in the context of an endogenous CRISPR system), or other sequences and transcripts from a CRISPR locus. In embodiments of the invention the terms guide sequence and guide RNA are used interchangeably. In some embodiments, one or more elements of a CRISPR system is derived from a type I, type II, or type III CRISPR system. In some embodiments, one or more elements of a CRISPR system is derived from a particular organism comprising an endogenous CRISPR system, such as Streptococcus pyogenes. In general, a CRISPR system is characterized by elements that promote the formation of a CRISPR complex at the site of a target sequence (also referred to as a protospacer in the context of an endogenous CRISPR system). In the context of formation of a CRISPR complex, "target sequence" refers to a sequence to which a guide sequence is designed to have complementarity, where hybridization between a target sequence and a guide sequence promotes the formation of a CRISPR complex. A target sequence may comprise any polynucleotide, such as DNA or RNA polynucleotides. In some embodiments, a target sequence is located in the nucleus or cytoplasm of a cell.

In some embodiments, direct repeats may be identified *in silico* by searching for repetitive motifs that fulfill any or all of the following criteria: 1. found in a 2Kb window of genomic sequence flanking the type II CRISPR locus; 2. span from 20 to 50 bp; and 3. interspaced by 20 to 50 bp. In some embodiments, 2 of these criteria may be used, for instance 1 and 2, 2 and 3, or 1 and 3. In some embodiments, all 3 criteria may be used.

In some embodiments, candidate tracrRNA may be subsequently predicted by sequences that fulfill any or all of the following criteria: 1. sequence homology to direct repeats (motif search in Geneious with up to 18-bp mismatches); 2. presence of a predicted Rho-independent transcriptional terminator in direction of transcription; and 3. stable hairpin secondary structure between tracrRNA and direct repeat. In some embodiments, 2 of these criteria may be used, for instance 1 and 2, 2 and 3, or 1 and 3. In some embodiments, all 3 criteria may be used.

In some embodiments, chimeric synthetic guide RNAs (sgRNAs) designs may incorporate at least 12 bp of duplex structure between the direct repeat and tracrRNA.

In preferred embodiments of the invention, the CRISPR system is a type II CRISPR system and the Cas enzyme is Cas9, which catalyzes DNA cleavage. Enzymatic action by Cas9 derived from Streptococcus pyogenes or any closely related Cas9 generates double stranded breaks at target site sequences which hybridize to 20 nucleotides of the guide sequence and that have a protospacer-adjacent motif (PAM) sequence (examples include NGG/NRG or a PAM that can be determined as described herein) following the 20 nucleotides of the target sequence. CRISPR activity through Cas9 for site-specific DNA recognition and cleavage is defined by the guide sequence, the tracr sequence that hybridizes in part to the guide sequence and the PAM sequence. More aspects of the CRISPR system are described in Karginov and Hannon, The CRISPR system: small RNA-guided defence in bacteria and archaea, Mole Cell 2010, January 15; 37(1): 7.

The type II CRISPR locus from Streptococcus pyogenes SF370, which contains a cluster of four genes Cas9, Cas1, Cas2, and Csn1, as well as two non-coding RNA elements, tracrRNA and a characteristic array of repetitive sequences (direct repeats) interspaced by short stretches of non-repetitive sequences (spacers, about 30bp each). In this system, targeted DNA double-strand break (DSB) is generated in four sequential steps (Fig. 2A). First, two non-coding RNAs, the pre-crRNA array and tracrRNA, are transcribed from the CRISPR locus. Second, tracrRNA hybridizes to the direct repeats of pre-crRNA, which is then processed into mature crRNAs containing individual spacer sequences. Third, the mature crRNA:tracrRNA complex directs Cas9 to the DNA target consisting of the protospacer and the corresponding PAM via heteroduplex formation between the spacer region of the crRNA and the protospacer DNA. Finally, Cas9 mediates cleavage of target DNA upstream of PAM to create a DSB within the protospacer (Fig. 2A). Fig. 2B demonstrates the nuclear localization of the codon optimized Cas9. To promote precise transcriptional initiation, the RNA polymerase III-based U6 promoter was selected to drive the expression of tracrRNA (Fig. 2C). Similarly, a U6 promoter-based construct was developed to express a pre-crRNA array consisting of a single spacer flanked by two direct repeats (DRs, also encompassed by the term "tracr-mate sequences"; Fig. 2C). The initial spacer was designed to target a 33-base-pair (bp) target site (30-bp protospacer plus a 3-bp CRISPR motif (PAM) sequence satisfying the NGG recognition motif of Cas9) in the human EMX1 locus (Fig. 2C), a key gene in the development of the cerebral cortex.

Typically, in the context of an endogenous CRISPR system, formation of a CRISPR complex (comprising a guide sequence hybridized to a target sequence and complexed with one or more Cas proteins) results in cleavage of one or both strands in or near (e.g. within 1, 2, 3, 4, 5, 6, 7, 8, 9, 10, 20, 50, or more base pairs from) the target sequence. Without wishing to be bound by theory, the tracr sequence, which may comprise or consist of all or a portion of a wild-type tracr sequence (e.g. about or more than about 20, 26, 32, 45, 48, 54, 63, 67, 85, or more nucleotides of a wild-type tracr sequence), may also form part of a CRISPR complex, such as by hybridization along at least a portion of the tracr sequence to all or a portion of a tracr mate sequence that is operably linked to the guide sequence. In some embodiments, one or more vectors driving expression of one or more elements of a CRISPR system are introduced into a host cell such that expression of the elements of the CRISPR system direct formation of a CRISPR complex at one or more target sites. For example, a Cas enzyme, a guide sequence linked to a tracr-mate sequence, and a tracr sequence could each be operably linked to separate regulatory elements on separate vectors. Alternatively, two or more of the elements expressed from the same or different regulatory elements, may be combined in a single vector, with one or more additional vectors providing any components of the CRISPR system not included in the first vector. CRISPR system elements that are combined in a single vector may be arranged in any suitable orientation, such as one element located 5' with respect to ("upstream" of) or 3' with respect to ("downstream" of) a second element. The coding sequence of one element may be located on the same or opposite strand of the coding sequence of a second element, and oriented in the same or opposite direction. In some embodiments, a single promoter drives expression of a transcript encoding a CRISPR enzyme and one or more of the guide sequence, tracr mate sequence (optionally operably linked to the guide sequence), and a tracr sequence embedded within one or more intron sequences (e.g. each in a different intron, two or more in at least one intron, or all in a single intron). In some embodiments, the CRISPR enzyme, guide sequence, tracr mate sequence, and tracr sequence are operably linked to and expressed from the same promoter.

In some embodiments, a vector comprises one or more insertion sites, such as a restriction endonuclease recognition sequence (also referred to as a "cloning site"). In some embodiments, one or more insertion sites (e.g. about or more than about 1, 2, 3, 4, 5, 6, 7, 8, 9, 10, or more insertion sites) are located upstream and/or downstream of one or more sequence elements of one or more vectors. In some embodiments, a vector comprises an insertion site upstream of a tracr mate sequence, and optionally downstream of a regulatory element operably linked to the tracr mate sequence, such that following insertion of a guide sequence into the insertion site and upon expression the guide sequence directs sequence-specific binding of a CRISPR complex to a target sequence in a eukaryotic cell. In some embodiments, a vector comprises two or more insertion sites, each insertion site being located between two tracr mate sequences so as to allow insertion of a guide sequence at each site. In such an arrangement, the two or more guide sequences may comprise two or more copies of a single guide sequence, two or more different guide sequences, or combinations of these. When multiple different guide sequences are used, a single expression construct may be used to target CRISPR activity to multiple different, corresponding target sequences within a cell. For example, a single vector may comprise about or more than about 1, 2, 3, 4, 5, 6, 7, 8, 9, 10, 15, 20, or more guide sequences. In some embodiments, about or more than about 1, 2, 3, 4, 5, 6, 7, 8, 9, 10, or more such guide-sequence-containing vectors may be provided, and optionally delivered to a cell.

In some embodiments, a vector comprises a regulatory element operably linked to an enzyme-coding sequence encoding a CRISPR enzyme, such as a Cas protein. Non-limiting examples of Cas proteins include Cas1, Cas1B, Cas2, Cas3, Cas4, Cas5, Cas6, Cas7, Cas8, Cas9 (also known as Csn1 and Csx12), Cas10, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, homologues thereof, or modified versions thereof. In some embodiments, the unmodified CRISPR enzyme has DNA cleavage activity, such as Cas9. In some embodiments, the CRISPR enzyme directs cleavage of one or both strands at the location of a target sequence, such as within the target sequence and/or within the complement of the target sequence. In some embodiments, the CRISPR enzyme directs cleavage of one or both strands within about 1, 2, 3, 4, 5, 6, 7, 8, 9, 10, 15, 20, 25, 50, 100, 200, 500, or more base pairs from the first or last nucleotide of a target sequence. In some embodiments, a vector encodes a CRISPR enzyme that is mutated to with respect to a corresponding wild-type enzyme such that the mutated CRISPR enzyme lacks the ability to cleave one or both strands of a target polynucleotide containing a target sequence. For example, an aspartate-to-alanine substitution (D10A) in the RuvC I catalytic domain of Cas9 from S. pyogenes converts Cas9 from a nuclease that cleaves both strands to a nickase (cleaves a single strand). Other examples of mutations that render Cas9 a nickase include, without limitation, H840A, N854A, and N863A. As a further example, two or more catalytic domains of Cas9 (RuvC I, RuvC II, and RuvC III or the HNH domain) may be mutated to produce a mutated Cas9 substantially lacking all DNA cleavage activity. In some embodiments, a D10A mutation is combined with one or more of H840A, N854A, or N863A mutations to produce a Cas9 enzyme substantially lacking all DNA cleavage activity. In some embodiments, a CRISPR enzyme is considered to substantially lack all DNA cleavage activity when the DNA cleavage activity of the mutated enzyme is less than about 25%, 10%, 5%, 1%, 0.1%, 0.01%, or lower of the DNA cleavage activity of its non-mutated form; e.g., when the DNA cleavage activity of the mutated enzyme is about no more than 25%, 10%, 5%, 1%, 0.1%, 0.01%, or less of the DNA cleavage activity of the non-mutated form of the enzyme; whereby an example can be when the DNA cleavage activity of the mutated form is nil or negligible as compared with the non-mutated form, e.g., when no indel formation is observed as with Cas9H840A, as herein discussed. Where the enzyme is not SpCas9, mutations may be made at any or all residues corresponding to positions 10, 762, 840, 854, 863 and/or 986 of SpCas9 (which may be ascertained for instance by standard sequence comparison tools . In particular, any or all of the following mutations are preferred in SpCas9: D10A, E762A, H840A, N854A, N863A and/or D986A; as well as conservative substitution for any of the replacement amino acids is also envisaged. The same (or conservative substitutions of these mutations) at corresponding positions in other Cas9s are also preferred. Particularly preferred are D10 and H840 in SpCas9 . However, in other Cas9s, residues corresponding to SpCas9 D10 and H840 are also preferred.

An aspartate-to-alanine substitution (D10A) in the RuvC I catalytic domain of SpCas9 was engineered to convert the nuclease into a nickase (SpCas9n) (see e.g. Sapranauskas et al., 2011, Nucleic Acis Research, 39: 9275; Gasiunas et al., 2012, Proc. Natl. Acad. Sci. USA, 109:E2579), such that nicked genomic DNA undergoes the high-fidelity homology-directed repair (HDR). Surveyor assay confirmed that SpCas9n does not generate indels at the EMX1 protospacer target. Co-expression of EMX1-targeting chimeric crRNA (having the tracrRNA component as well) with SpCas9 produced indels in the target site, whereas co-expression with SpCas9n did not (n=3). Moreover, sequencing of 327 amplicons did not detect any indels induced by SpCas9n. The same locus was selected to test CRISPR-mediated HR by co-transfecting HEK 293FT cells with the chimeric RNA targeting EMX1, hSpCas9 or hSpCas9n, as well as a HR template to introduce a pair of restriction sites (HindIII and NheI) near the protospacer.

Preferred orthologs are discussed herein. A Cas enzyme may be identified Cas9 as this can refer to the general class of enzymes that share homology to the biggest nuclease with multiple nuclease domains from the type II CRISPR system. Most preferably, the Cas9 enzyme is from, or is derived from, spCas9 (S. pyogenes Cas9) or saCas9 (S. aureus Cas9). By derived, Applicants mean that the derived enzyme is largely based, in the sense of having a high degree of sequence homology with, a wildtype enzyme, but that it has been mutated (modified) in some way as described herein.

It will be appreciated that the terms Cas and CRISPR enzyme are generally used herein interchangeably, unless otherwise apparent. As mentioned above, many of the residue numberings used herein refer to the Cas9 enzyme from the type II CRISPR locus in *Streptococcus pyogenes.* However, it will be appreciated that this invention includes many more Cas9s from other species of microbes, such as SpCas9, SaCa9, St1Cas9 and so forth.

Codon optimization

An example of a codon optimized sequence, in this instance optimized for humans (i.e. being optimized for expression in humans) is provided herein, see the SaCas9 human codon optimized sequence. Whilst this is preferred, it will be appreciated that other examples are possible and codon optimization for a host species other than human, or for codon optimization for specific organs such as the brain, is known. In some embodiments, an enzyme coding sequence encoding a CRISPR enzyme is codon optimized for expression in particular cells, such as eukaryotic cells. The eukaryotic cells may be those of or derived from a particular organism, such as a mammal, including but not limited to human, mouse, rat, rabbit, dog, or non-human mammal or primate. In some embodiments, processes for modifying the germ line genetic identity of human beings and/or processes for modifying the genetic identity of animals which are likely to cause them suffering without any substantial medical benefit to man or animal, and also animals resulting from such processes, may be excluded.

In general, codon optimization refers to a process of modifying a nucleic acid sequence for enhanced expression in the host cells of interest by replacing at least one codon (e.g. about or more than about 1, 2, 3, 4, 5, 10, 15, 20, 25, 50, or more codons) of the native sequence with codons that are more frequently or most frequently used in the genes of that host cell while maintaining the native amino acid sequence. Various species exhibit particular bias for certain codons of a particular amino acid. Codon bias (differences in codon usage between organisms) often correlates with the efficiency of translation of messenger RNA (mRNA), which is in turn believed to be dependent on, among other things, the properties of the codons being translated and the availability of particular transfer RNA (tRNA) molecules. The predominance of selected tRNAs in a cell is generally a reflection of the codons used most frequently in peptide synthesis. Accordingly, genes can be tailored for optimal gene expression in a given organism based on codon optimization. Codon usage tables are readily available, for example, at the "Codon Usage Database" available at www.kazusa.orjp/codon/ (visited Jul. 9, 2002), and these tables can be adapted in a number of ways. See Nakamura, Y., et al. "Codon usage tabulated from the international DNA sequence databases: status for the year 2000" Nucl. Acids Res. 28:292 (2000). Computer algorithms for codon optimizing a particular sequence for expression in a particular host cell are also available, such as Gene Forge (Aptagen; Jacobus, PA), are also available. In some embodiments, one or more codons (e.g. 1, 2, 3, 4, 5, 10, 15, 20, 25, 50, or more, or all codons) in a sequence encoding a CRISPR enzyme correspond to the most frequently used codon for a particular amino acid.

Nuclear localization sequences (NLSs)

In some embodiments, a vector encodes a CRISPR enzyme comprising one or more nuclear localization sequences (NLSs), such as about or more than about 1, 2, 3, 4, 5, 6, 7, 8, 9, 10, or more NLSs. In some embodiments, the CRISPR enzyme comprises about or more than about 1, 2, 3, 4, 5, 6, 7, 8, 9, 10, or more NLSs at or near the amino-terminus, about or more than about 1, 2, 3, 4, 5, 6, 7, 8, 9, 10, or more NLSs at or near the carboxy-terminus, or a combination of these (e.g. zero or at least one or more NLS at the amino-terminus and zero or at one or more NLS at the carboxy terminus). When more than one NLS is present, each may be selected independently of the others, such that a single NLS may be present in more than one copy and/or in combination with one or more other NLSs present in one or more copies. In a preferred embodiment of the invention, the CRISPR enzyme comprises at most 6 NLSs. In some embodiments, an NLS is considered near the N- or C-terminus when the nearest amino acid of the NLS is within about 1, 2, 3, 4, 5, 10, 15, 20, 25, 30, 40, 50, or more amino acids along the polypeptide chain from the N- or C-terminus. Non-limiting examples of NLSs include an NLS sequence derived from: the NLS of the SV40 virus large T-antigen, having the amino acid sequence PKKKRKV; the NLS from nucleoplasmin (e.g. the nucleoplasmin bipartite NLS with the sequence KRPAATKKAGQAKKKK); the c-myc NLS having the amino acid sequence PAAKRVKLD or RQRRNELKRSP; the hRNPA1 M9 NLS having the sequence NQSSNFGPMKGGNFGGRSSGPYGGGGQYFAKPRNQGGY; the sequence RMRIZFKNKGKDTAELRRRRVEVSVELRKAKKDEQILKRRNV of the IBB domain from importin-alpha; the sequences VSRKRPRP and PPKKARED of the myoma T protein; the sequence POPKKKPL of human p53; the sequence SALIKKKKKMAP of mouse c-abl IV; the sequences DRLRR and PKQKKRK of the influenza virus NS1; the sequence RKLKKKIKKL of the Hepatitis virus delta antigen; the sequence REKKKFLKRR of the mouse Mx1 protein; the sequence KRKGDEVDGVDEVAKKKSKK of the human poly(ADP-ribose) polymerase; and the sequence RKCLQAGMNLEARKTKK of the steroid hormone receptors (human) glucocorticoid.

In general, the one or more NLSs are of sufficient strength to drive accumulation of the CRISPR enzyme in a detectable amount in the nucleus of a eukaryotic cell. In general, strength of nuclear localization activity may derive from the number of NLSs in the CRISPR enzyme, the particular NLS(s) used, or a combination of these factors. Detection of accumulation in the nucleus may be performed by any suitable technique. For example, a detectable marker may be fused to the CRISPR enzyme, such that location within a cell may be visualized, such as in combination with a means for detecting the location of the nucleus (e.g. a stain specific for the nucleus such as DAPI). Cell nuclei may also be isolated from cells, the contents of which may then be analyzed by any suitable process for detecting protein, such as immunohistochemistry, Western blot, or enzyme activity assay. Accumulation in the nucleus may also be determined indirectly, such as by an assay for the effect of CRISPR complex formation (e.g. assay for DNA cleavage or mutation at the target sequence, or assay for altered gene expression activity affected by CRISPR complex formation and/or CRISPR enzyme activity), as compared to a control no exposed to the CRISPR enzyme or complex, or exposed to a CRISPR enzyme lacking the one or more NLSs.

Guide sequence

In general, a guide sequence is any polynucleotide sequence having sufficient complementarity with a target polynucleotide sequence to hybridize with the target sequence and direct sequence-specific binding of a CRISPR complex to the target sequence. In some embodiments, the degree of complementarity between a guide sequence and its corresponding target sequence, when optimally aligned using a suitable alignment algorithm, is about or more than about 50%, 60%, 75%, 80%, 85%, 90%, 95%, 97.5%, 99%, or more. Optimal alignment may be determined with the use of any suitable algorithm for aligning sequences, non-limiting example of which include the Smith-Waterman algorithm, the Needleman-Wunsch algorithm, algorithms based on the Burrows-Wheeler Transform (e.g. the Burrows Wheeler Aligner), ClustalW, Clustal X, BLAT, Novoalign (Novocraft Technologies; available at www.novocraft.com), ELAND (Illumina, San Diego, CA), SOAP (available at soap.genomics.org.cn), and Maq (available at maq.sourceforge.net). In some embodiments, a guide sequence is about or more than about 5, 10, 11, 12, 13, 14, 15, 16, 17, 18, 19, 20, 21, 22, 23, 24, 25, 26, 27, 28, 29, 30, 35, 40, 45, 50, 75, or more nucleotides in length. In some embodiments, a guide sequence is less than about 75, 50, 45, 40, 35, 30, 25, 20, 15, 12, or fewer nucleotides in length. The ability of a guide sequence to direct sequence-specific binding of a CRISPR complex to a target sequence may be assessed by any suitable assay. For example, the components of a CRISPR system sufficient to form a CRISPR complex, including the guide sequence to be tested, may be provided to a host cell having the corresponding target sequence, such as by transfection with vectors encoding the components of the CRISPR sequence, followed by an assessment of preferential cleavage within the target sequence, such as by Surveyor assay as described herein. Similarly, cleavage of a target polynucleotide sequence may be evaluated in a test tube by providing the target sequence, components of a CRISPR complex, including the guide sequence to be tested and a control guide sequence different from the test guide sequence, and comparing binding or rate of cleavage at the target sequence between the test and control guide sequence reactions. Other assays are possible, and will occur to those skilled in the art.

A guide sequence may be selected to target any target sequence. In some embodiments, the target sequence is a sequence within a genome of a cell. Exemplary target sequences include those that are unique in the target genome. For example, for the *S. pyogenes* Cas9, a unique target sequence in a genome may include a Cas9 target site of the form MMMMMMMMNNNNNNNNNNNNXGG where NNNNNNNNNNNNXGG (N is A, G, T, or C; and X can be anything) has a single occurrence in the genome. A unique target sequence in a genome may include an *S. pyogenes* Cas9 target site of the form MMMMMMMMMNNNNNNNNNNNXGG where NNNNNNNNNNNXGG (N is A, G, T, or C; and X can be anything) has a single occurrence in the genome. For the *S. thermophilus* CRISPR1 Cas9, a unique target sequence in a genome may include a Cas9 target site of the form MMMMMMMMNNNNNNNNNNNNXXAGAAW where NNNNNNNNNNNNXXAGAAW (N is A, G, T, or C; X can be anything; and W is A or T) has a single occurrence in the genome. A unique target sequence in a genome may include an *S. thermophilus* CRISPR1 Cas9 target site of the form MMMMMMMMMNNNNNNNNNNNXXAGAAW where NNNNNNNNNNNXXAGAAW (N is A, G, T, or C; X can be anything; and W is A or T) has a single occurrence in the genome. For the *S. pyogenes* Cas9, a unique target sequence in a genome may include a Cas9 target site of the form MMMMMMMMNNNNNNNNNNNNXGGXG where NNNNNNNNNNNNXGGXG (N is A, G, T, or C; and X can be anything) has a single occurrence in the genome. A unique target sequence in a genome may include an *S. pyogenes* Cas9 target site of the form MMMMMMMMMNNNNNNNNNNNXGGXG where NNNNNNNNNNNXGGXG (N is A, G, T, or C; and X can be anything) has a single occurrence in the genome. In each of these sequences "M" may be A, G, T, or C, and need not be considered in identifying a sequence as unique.

In some embodiments, a guide sequence is selected to reduce the degree secondary structure within the guide sequence. In some embodiments, about or less than about 75%, 50%, 40%, 30%, 25%, 20%, 15%, 10%, 5%, 1%, or fewer of the nucleotides of the guide sequence participate in self-complementary base pairing when optimally folded. Optimal folding may be determined by any suitable polynucleotide folding algorithm. Some programs are based on calculating the minimal Gibbs free energy. An example of one such algorithm is mFold, as described by Zuker and Stiegler (Nucleic Acids Res. 9 (1981), 133-148). Another example folding algorithm is the online webserver RNAfold, developed at Institute for Theoretical Chemistry at the University of Vienna, using the centroid structure prediction algorithm (see e.g. A.R. Gruber et al., 2008, Cell 106(1): 23-24; and PA Carr and GM Church, 2009, Nature Biotechnology 27(12): 1151-62).

Tracr mate sequence

In general, a tracr mate sequence includes any sequence that has sufficient complementarity with a tracr sequence to promote one or more of: (1) excision of a guide sequence flanked by tracr mate sequences in a cell containing the corresponding tracr sequence; and (2) formation of a CRISPR complex at a target sequence, wherein the CRISPR complex comprises the tracr mate sequence hybridized to the tracr sequence. In general, degree of complementarity is with reference to the optimal alignment of the tracr mate sequence and tracr sequence, along the length of the shorter of the two sequences. Optimal alignment may be determined by any suitable alignment algorithm, and may further account for secondary structures, such as self-complementarity within either the tracr sequence or tracr mate sequence. In some embodiments, the degree of complementarity between the tracr sequence and tracr mate sequence along the length of the shorter of the two when optimally aligned is about or more than about 25%, 30%, 40%, 50%, 60%, 70%, 80%, 90%, 95%, 97.5%, 99%, or higher. In some embodiments, the tracr sequence is about or more than about 5, 6, 7, 8, 9, 10, 11, 12, 13, 14, 15, 16, 17, 18, 19, 20, 25, 30, 40, 50, or more nucleotides in length. In some embodiments, the tracr sequence and tracr mate sequence are contained within a single transcript, such that hybridization between the two produces a transcript having a secondary structure, such as a hairpin. In an embodiment of the invention, the transcript or transcribed polynucleotide sequence has at least two or more hairpins. In preferred embodiments, the transcript has two, three, four or five hairpins. In a further embodiment of the invention, the transcript has at most five hairpins. In a hairpin structure the portion of the sequence 5' of the final "N" and upstream of the loop corresponds to the tracr mate sequence, and the portion of the sequence 3' of the loop corresponds to the tracr sequence Further non-limiting examples of single polynucleotides comprising a guide sequence, a tracr mate sequence, and a tracr sequence are as follows (listed 5' to 3'), where "N" represents a base of a guide sequence, the first block of lower case letters represent the tracr mate sequence, and the second block of lower case letters represent the tracr sequence, and the final poly-T sequence represents the transcription terminator: (1) NNNNNNNNNNNNNNNNNNNNgtttttgtactctcaagatttaGAAAtaaatcttgcagaagctacaaagataa ggcttcatgccgaaatcaacaccctgtcattttatggcagggtgttttcgttatttaaTTTTTT; (2) NNNNNNNNNNNNNNNNNNNNgtttttgtactctcaGAAAtgcagaagctacaaagataaggcttcatgccg aaatcaacaccctgtcattttatggcagggtgttttcgttatttaaTTTTTT; (3) NNNNNNNNNNNNNNNNNNNNgtttttgtactctcaGAAAtgcagaagctacaaagataaggcttcatgccg aaatcaacaccctgtcattttatggcagggtgtTTTTTT; (4) NNNNNNNNNNNNNNNNNNNNgttttagagctaGAAAtagcaagttaaaataaggctagtccgttatcaactt gaaaaagtggcaccgagtcggtgcTTTTTT; (5) NNNNNNNNNNNNNNNNNNNNgttttagagctaGAAATAGcaagttaaaataaggctagtccgttatcaac ttgaaaaagtgTTTTTTT; and (6) NNNNNNNNNNNNNNNNNNNNgttttagagctagAAATAGcaagttaaaataaggctagtccgttatcaTT TTTTTT. In some embodiments, sequences (1) to (3) are used in combination with Cas9 from *S. thermophilus* CRISPR1. In some embodiments, sequences (4) to (6) are used in combination with Cas9 from *S. pyogenes.* In some embodiments, the tracr sequence is a separate transcript from a transcript comprising the tracr mate sequence.

Recombination template

In some embodiments, a recombination template is also provided. A recombination template may be a component of another vector as described herein, contained in a separate vector, or provided as a separate polynucleotide. In some embodiments, a recombination template is designed to serve as a template in homologous recombination, such as within or near a target sequence nicked or cleaved by a CRISPR enzyme as a part of a CRISPR complex. A template polynucleotide may be of any suitable length, such as about or more than about 10, 15, 20, 25, 50, 75, 100, 150, 200, 500, 1000, or more nucleotides in length. In some embodiments, the template polynucleotide is complementary to a portion of a polynucleotide comprising the target sequence. When optimally aligned, a template polynucleotide might overlap with one or more nucleotides of a target sequences (e.g. about or more than about 1, 5, 10, 15, 20, or more nucleotides). In some embodiments, when a template sequence and a polynucleotide comprising a target sequence are optimally aligned, the nearest nucleotide of the template polynucleotide is within about 1, 5, 10, 15, 20, 25, 50, 75, 100, 200, 300, 400, 500, 1000, 5000, 10000, or more nucleotides from the target sequence.

Fusion protein

In some embodiments, the CRISPR enzyme is part of a fusion protein comprising one or more heterologous protein domains (e.g. about or more than about 1, 2, 3, 4, 5, 6, 7, 8, 9, 10, or more domains in addition to the CRISPR enzyme). A CRISPR enzyme fusion protein may comprise any additional protein sequence, and optionally a linker sequence between any two domains. Examples of protein domains that may be fused to a CRISPR enzyme include, without limitation, epitope tags, reporter gene sequences, and protein domains having one or more of the following activities: methylase activity, demethylase activity, transcription activation activity, transcription repression activity, transcription release factor activity, histone modification activity, RNA cleavage activity and nucleic acid binding activity. Non-limiting examples of epitope tags include histidine (His) tags, V5 tags, FLAG tags, influenza hemagglutinin (HA) tags, Myc tags, VSV-G tags, and thioredoxin (Trx) tags. Examples of reporter genes include, but are not limited to, glutathione-S-transferase (GST), horseradish peroxidase (HRP), chloramphenicol acetyltransferase (CAT) beta-galactosidase, beta-glucuronidase, luciferase, green fluorescent protein (GFP), HcRed, DsRed, cyan fluorescent protein (CFP), yellow fluorescent protein (YFP), and autofluorescent proteins including blue fluorescent protein (BFP). A CRISPR enzyme may be fused to a gene sequence encoding a protein or a fragment of a protein that bind DNA molecules or bind other cellular molecules, including but not limited to maltose binding protein (MBP), S-tag, Lex A DNA binding domain (DBD) fusions, GAL4 DNA binding domain fusions, and herpes simplex virus (HSV) BP16 protein fusions. Additional domains that may form part of a fusion protein comprising a CRISPR enzyme are described in US20110059502, incorporated herein by reference. In some embodiments, a tagged CRISPR enzyme is used to identify the location of a target sequence.

Inducible system

In some embodiments, a CRISPR enzyme may form a component of an inducible system. The inducible nature of the system would allow for spatiotemporal control of gene editing or gene expression using a form of energy. The form of energy may include but is not limited to electromagnetic radiation, sound energy, chemical energy and thermal energy. Examples of inducible system include tetracycline inducible promoters (Tet-On or Tet-Off), small molecule two-hybrid transcription activations systems (FKBP, ABA, etc), or light inducible systems (Phytochrome, LOV domains, or cryptochrome).In one embodiment, the CRISPR enzyme may be a part of a Light Inducible Transcriptional Effector (LITE) to direct changes in transcriptional activity in a sequence-specific manner. The components of a light may include a CRISPR enzyme, a light-responsive cytochrome heterodimer (e.g. from Arabidopsis thaliana), and a transcriptional activation/repression domain. Further examples of inducible DNA binding proteins and methods for their use are provided in US 61/736465 and US 61/721,283, which is hereby incorporated by reference in its entirety.

Delivery

The invention involves at least one component of the CRISPR complex, e.g., RNA, delivered via at least one nanoparticle complex. In some aspects, the invention provides methods comprising delivering one or more polynucleotides, such as or one or more vectors as described herein, one or more transcripts thereof, and/or one or proteins transcribed therefrom, to a host cell. In some aspects, the invention further provides cells produced by such methods, and animals comprising or produced from such cells. In some embodiments, a CRISPR enzyme in combination with (and optionally complexed with) a guide sequence is delivered to a cell. Conventional viral and non-viral based gene transfer methods can be used to introduce nucleic acids in mammalian cells or target tissues. Such methods can be used to administer nucleic acids encoding components of a CRISPR system to cells in culture, or in a host organism. Non-viral vector delivery systems include DNA plasmids, RNA (e.g. a transcript of a vector described herein), naked nucleic acid, and nucleic acid complexed with a delivery vehicle, such as a liposome. Viral vector delivery systems include DNA and RNA viruses, which have either episomal or integrated genomes after delivery to the cell. For a review of gene therapy procedures, see Anderson, Science 256:808-813 (1992); Nabel & Felgner, TIBTECH 11:211-217 (1993); Mitani & Caskey, TIBTECH 11:162-166 (1993); Dillon, TIBTECH 11:167-175 (1993); Miller, Nature 357:455-460 (1992); Van Brunt, Biotechnology 6(10):1149-1154 (1988); Vigne, Restorative Neurology and Neuroscience 8:35-36 (1995); Kremer & Perricaudet, British Medical Bulletin 51(1):31-44 (1995); Haddada et al., in Current Topics in Microbiology and Immunology Doerfler and Böhm (eds) (1995); and Yu et al., Gene Therapy 1:13-26 (1994).

Methods of non-viral delivery of nucleic acids include lipofection, microinjection, biolistics, virosomes, liposomes, immunoliposomes, polycation or lipid:nucleic acid conjugates, naked DNA, artificial virions, and agent-enhanced uptake of DNA. Lipofection is described in e.g., U.S. Pat. Nos. 5,049,386, 4,946,787; and 4,897,355) and lipofection reagents are sold commercially (e.g., Transfectam™ and Lipofectin™). Cationic and neutral lipids that are suitable for efficient receptor-recognition lipofection of polynucleotides include those of Felgner, WO 91/17424; WO 91/16024. Delivery can be to cells (e.g. in vitro or ex vivo administration) or target tissues (e.g. in vivo administration).

The preparation of lipid:nucleic acid complexes, including targeted liposomes such as immunolipid complexes, is well known to one of skill in the art (see, e.g., Crystal, Science 270:404-410 (1995); Blaese et al., Cancer Gene Ther. 2:291-297 (1995); Behr et al., Bioconjugate Chem. 5:382-389 (1994); Remy et al., Bioconjugate Chem. 5:647-654 (1994); Gao et al., Gene Therapy 2:710-722 (1995); Ahmad et al., Cancer Res. 52:4817-4820 (1992); U.S. Pat. Nos. 4,186,183, 4,217,344, 4,235,871, 4,261,975, 4,485,054, 4,501,728, 4,774,085, 4,837,028, and 4,946,787).

The use of RNA or DNA viral based systems for the delivery of nucleic acids take advantage of highly evolved processes for targeting a virus to specific cells in the body and trafficking the viral payload to the nucleus. Viral vectors can be administered directly to patients (in vivo) or they can be used to treat cells in vitro, and the modified cells may optionally be administered to patients (ex vivo). Conventional viral based systems could include retroviral, lentivirus, adenoviral, adeno-associated and herpes simplex virus vectors for gene transfer. Integration in the host genome is possible with the retrovirus, lentivirus, and adeno-associated virus gene transfer methods, often resulting in long term expression of the inserted transgene. Additionally, high transduction efficiencies have been observed in many different cell types and target tissues.

The tropism of a retrovirus can be altered by incorporating foreign envelope proteins, expanding the potential target population of target cells. Lentiviral vectors are retroviral vectors that are able to transduce or infect non-dividing cells and typically produce high viral titers. Selection of a retroviral gene transfer system would therefore depend on the target tissue. Retroviral vectors are comprised of cis-acting long terminal repeats with packaging capacity for up to 6-10 kb of foreign sequence. The minimum cis-acting LTRs are sufficient for replication and packaging of the vectors, which are then used to integrate the therapeutic gene into the target cell to provide permanent transgene expression. Widely used retroviral vectors include those based upon murine leukemia virus (MuLV), gibbon ape leukemia virus (GaLV), Simian Immuno deficiency virus (SIV), human immuno deficiency virus (HIV), and combinations thereof (see, e.g., Buchscher et al., J. Virol. 66:2731-2739 (1992); Johann et al., J. Virol. 66:1635-1640 (1992); Sommnerfelt et al., Virol. 176:58-59 (1990); Wilson et al., J. Virol. 63:2374-2378 (1989); Miller et al., J. Virol. 65:2220-2224 (1991); PCT/US94/05700).

In another embodiment, Cocal vesiculovirus envelope pseudotyped retroviral vector particles are contemplated (see, e.g., US Patent Publication No. 20120164118 assigned to the Fred Hutchinson Cancer Research Center). Cocal virus is in the Vesiculovirus genus, and is a causative agent of vesicular stomatitis in mammals. Cocal virus was originally isolated from mites in Trinidad (Jonkers et al., Am. J. Vet. Res. 25:236-242 (1964)), and infections have been identified in Trinidad, Brazil, and Argentina from insects, cattle, and horses. Many of the vesiculoviruses that infect mammals have been isolated from naturally infected arthropods, suggesting that they are vector-borne. Antibodies to vesiculoviruses are common among people living in rural areas where the viruses are endemic and laboratory-acquired; infections in humans usually result in influenza-like symptoms. The Cocal virus envelope glycoprotein shares 71.5% identity at the amino acid level with VSV-G Indiana, and phylogenetic comparison of the envelope gene of vesiculoviruses shows that Cocal virus is serologically distinct from, but most closely related to, VSV-G Indiana strains among the vesiculoviruses. Jonkers et al., Am. J. Vet. Res. 25:236-242 (1964) and Travassos da Rosa et al., Am. J. Tropical Med. & Hygiene 33:999-1006 (1984). The Cocal vesiculovirus envelope pseudotyped retroviral vector particles may include for example, lentiviral, alpharetroviral, betaretroviral, gammaretroviral, deltaretroviral, and epsilonretroviral vector particles that may comprise retroviral Gag, Pol, and/or one or more accessory protein(s) and a Cocal vesiculovirus envelope protein. Within certain aspects of these embodiments, the Gag, Pol, and accessory proteins are lentiviral and/or gammaretroviral.

In applications where transient expression is preferred, adenoviral based systems may be used. Adenoviral based vectors are capable of very high transduction efficiency in many cell types and do not require cell division. With such vectors, high titer and levels of expression have been obtained. This vector can be produced in large quantities in a relatively simple system.

Adeno-associated virus ("AAV") vectors may also be used to transduce cells with target nucleic acids, e.g., in the in vitro production of nucleic acids and peptides, and for in vivo and ex vivo gene therapy procedures (see, e.g., West et al., Virology 160:38-47 (1987); U.S. Pat. No. 4,797,368; WO 93/24641; Kotin, Human Gene Therapy 5:793-801 (1994); Muzyczka, J. Clin. Invest. 94:1351 (1994). Construction of recombinant AAV vectors are described in a number of publications, including U.S. Pat. No. 5,173,414; Tratschin et al., Mol. Cell. Biol. 5:3251-3260 (1985); Tratschin, et al., Mol. Cell. Biol. 4:2072-2081 (1984); Hermonat & Muzyczka, PNAS 81:6466-6470 (1984); and Samulski et al., J. Virol. 63:03822-3828 (1989).

Packaging cells are typically used to form virus particles that are capable of infecting a host cell. Such cells include 293 cells, which package adenovirus, and ψ2 cells or PA317 cells, which package retrovirus. Viral vectors used in gene therapy are usually generated by producer a cell line that packages a nucleic acid vector into a viral particle. The vectors typically contain the minimal viral sequences required for packaging and subsequent integration into a host, other viral sequences being replaced by an expression cassette for the polynucleotide(s) to be expressed. The missing viral functions are typically supplied in trans by the packaging cell line. For example, AAV vectors used in gene therapy typically only possess ITR sequences from the AAV genome which are required for packaging and integration into the host genome. Viral DNA is packaged in a cell line, which contains a helper plasmid encoding the other AAV genes, namely rep and cap, but lacking ITR sequences. The cell line may also infected with adenovirus as a helper. The helper virus promotes replication of the AAV vector and expression of AAV genes from the helper plasmid. The helper plasmid is not packaged in significant amounts due to a lack of ITR sequences. Contamination with adenovirus can be reduced by, e.g., heat treatment to which adenovirus is more sensitive than AAV.

Accordingly, AAV is considered an ideal candidate for use as a transducing vector. Such AAV transducing vectors can comprise sufficient cis-acting functions to replicate in the presence of adenovirus or herpesvirus or poxvirus (e.g., vaccinia virus) helper functions provided in trans. Recombinant AAV (rAAV) can be used to carry exogenous genes into cells of a variety of lineages. In these vectors, the AAV cap and/or rep genes are deleted from the viral genome and replaced with a DNA segment of choice. Current AAV vectors may accommodate up to 4300 bases of inserted DNA.

There are a number of ways to produce rAAV, and the invention provides rAAV and methods for preparing rAAV. For example, plasmid(s) containing or consisting essentially of the desired viral construct are transfected into AAV-infected cells. In addition, a second or additional helper plasmid is cotransfected into these cells to provide the AAV rep and/or cap genes which are obligatory for replication and packaging of the recombinant viral construct. Under these conditions, the rep and/or cap proteins of AAV act in trans to stimulate replication and packaging of the rAAV construct. Two to Three days after transfection, rAAV is harvested. Traditionally rAAV is harvested from the cells along with adenovirus. The contaminating adenovirus is then inactivated by heat treatment. In the instant invention, rAAV is advantageously harvested not from the cells themselves, but from cell supernatant. Accordingly, in an initial aspect the invention provides for preparing rAAV, and in addition to the foregoing, rAAV can be prepared by a method that comprises or consists essentially of: infecting susceptible cells with a rAAV containing exogenous DNA including DNA for expression, and helper virus (e.g., adenovirus, herpesvirus, poxvirus such as vaccinia virus) wherein the rAAV lacks functioning cap and/or rep (and the helper virus (e.g., adenovirus, herpesvirus, poxvirus such as vaccinia virus) provides the cap and/or rev function that the rAAV lacks); or infecting susceptible cells with a rAAV containing exogenous DNA including DNA for expression, wherein the recombinant lacks functioning cap and/or rep, and transfecting said cells with a plasmid supplying cap and/or rep function that the rAAV lacks; or infecting susceptible cells with a rAAV containing exogenous DNA including DNA for expression, wherein the recombinant lacks functioning cap and/or rep, wherein said cells supply cap and/or rep function that the recombinant lacks; or transfecting the susceptible cells with an AAV lacking functioning cap and/or rep and plasmids for inserting exogenous DNA into the recombinant so that the exogenous DNA is expressed by the recombinant and for supplying rep and/or cap functions whereby transfection results in an rAAV containing the exogenous DNA including DNA for expression that lacks functioning cap and/or rep.

The rAAV can be from an AAV as herein described, and advantageously can be an rAAV1, rAAV2, AAV5 or rAAV having hybrid or capsid which may comprise AAV1, AAV2, AAV5 or any combination thereof. One can select the AAV of the rAAV with regard to the cells to be targeted by the rAAV; e.g., one can select AAV serotypes 1, 2, 5 or a hybrid or capsid AAV1, AAV2, AAV5 or any combination thereof for targeting brain or neuronal cells; and one can select AAV4 for targeting cardiac tissue. In addition to 293 cells, other cells that can be used in the practice of the invention and the relative infectivity of certain AAV serotypes *in vitro* as to these cells (see Grimm, D. et al, J. Virol. 82: 5887-5911 (2008)) are as follows:

| Cell Line | AAV-1 | AAV-2 | AAV-3 | AAV-4 | AAV-5 | AAV-6 | AAV-8 | AAV-9 |
|---|---|---|---|---|---|---|---|---|
| Huh-7 | 13 | 100 | 2.5 | 0.0 | 0.1 | 10 | 0.7 | 0.0 |
| HEK293 | 25 | 100 | 2.5 | 0.1 | 0.1 | 5 | 0.7 | 0.1 |
| HeLa | 3 | 100 | 2.0 | 0.1 | 6.7 | 1 | 0.2 | 0.1 |
| HepG2 | 3 | 100 | 16.7 | 0.3 | 1.7 | 5 | 0.3 | ND |
| Hep1A | 20 | 100 | 0.2 | 1.0 | 0.1 | 1 | 0.2 | 0.0 |
| 911 | 17 | 100 | 11 | 0.2 | 0.1 | 17 | 0.1 | ND |
| CHO | 100 | 100 | 14 | 1.4 | 333 | 50 | 10 | 1.0 |
| COS | 33 | 100 | 33 | 3.3 | 5.0 | 14 | 2.0 | 0.5 |
| MeWo | 10 | 100 | 20 | 0.3 | 6.7 | 10 | 1.0 | 0.2 |
| NIH3T3 | 10 | 100 | 2.9 | 2.9 | 0.3 | 10 | 0.3 | ND |
| A549 | 14 | 100 | 20 | ND | 0.5 | 10 | 0.5 | 0.1 |
| HT1180 | 20 | 100 | 10 | 0.1 | 0.3 | 33 | 0.5 | 0.1 |
| Monocytes | 1111 | 100 | ND | ND | 125 | 1429 | ND | ND |
| Immature DC | 2500 | 100 | ND | ND | 222 | 2857 | ND | ND |
| Mature DC | 2222 | 100 | ND | ND | 333 | 3333 | ND | ND |

The invention provides rAAV that contains or consists essentially of an exogenous nucleic acid molecule encoding a CRISPR (Clustered Regularly Interspaced Short Palindromic Repeats) system, e.g., a plurality of cassettes comprising or consisting a first cassette comprising or consisting essentially of a promoter, a nucleic acid molecule encoding a CRISPR-associated (Cas) protein (putative nuclease or helicase proteins), e.g., Cas9 and a terminator, and a two, or more, advantageously up to the packaging size limit of the vector, e.g., in total (including the first cassette) five, cassettes comprising or consisting essentially of a promoter, nucleic acid molecule encoding guide RNA (gRNA) and a terminator (e.g., each cassette schematically represented as Promoter-gRNAl-terminator, Promoter-gRNA2-terminator ... Promoter-gRNA(N)-terminator (where N is a number that can be inserted that is at an upper limit of the packaging size limit of the vector), or two or more individual rAAVs, each containing one or more than one cassette of a CRISPR system, e.g., a first rAAV containing the first cassette comprising or consisting essentially of a promoter, a nucleic acid molecule encoding Cas, e.g., Cas9 and a terminator, and a second rAAV containing a plurality, four, cassettes comprising or consisting essentially of a promoter, nucleic acid molecule encoding guide RNA (gRNA) and a terminator (e.g., each cassette schematically represented as Promoter-gRNAl-terminator, Promoter-gRNA2-terminator ... Promoter-gRNA(N)-terminator (where N is a number that can be inserted that is at an upper limit of the packaging size limit of the vector). As rAAV is a DNA virus, the nucleic acid molecules in the herein discussion concerning AAV or rAAV are advantageously DNA. The promoter is in some embodiments advantageously human Synapsin I promoter (hSyn). Additional methods for the delivery of nucleic acids to cells are known to those skilled in the art. See, for example, US20030087817, incorporated herein by reference.

In some embodiments, a host cell is transiently or non-transiently transfected with one or more vectors described herein. In some embodiments, a cell is transfected as it naturally occurs in a subject. In some embodiments, a cell that is transfected is taken from a subject. In some embodiments, the cell is derived from cells taken from a subject, such as a cell line. A wide variety of cell lines for tissue culture are known in the art. Examples of cell lines include, but are not limited to, C8161, CCRF-CEM, MOLT, mIMCD-3, NHDF, HeLa-S3, Huh1, Huh4, Huh7, HUVEC, HASMC, HEKn, HEKa, MiaPaCell, Panc1, PC-3, TF1, CTLL-2, C1R, Rat6, CV1, RPTE, A10, T24, J82, A375, ARH-77, Calu1, SW480, SW620, SKOV3, SK-UT, CaCo2, P388D1, SEM-K2, WEHI-231, HB56, TIB55, Jurkat, J45.01, LRMB, Bcl-1, BC-3, IC21, DLD2, Raw264.7, NRK, NRK-52E, MRC5, MEF, Hep G2, HeLa B, HeLa T4, COS, COS-1, COS-6, COS-M6A, BS-C-1 monkey kidney epithelial, BALB/ 3T3 mouse embryo fibroblast, 3T3 Swiss, 3T3-L1, 132-d5 human fetal fibroblasts; 10.1 mouse fibroblasts, 293-T, 3T3, 721, 9L, A2780, A2780ADR, A2780cis, A172, A20, A253, A431, A-549, ALC, B16, B35, BCP-1 cells, BEAS-2B, bEnd.3, BHK-21, BR 293, BxPC3, C3H-10T1/2, C6/36, Cal-27, CHO, CHO-7, CHO-IR, CHO-K1, CHO-K2, CHO-T, CHO Dhfr -/-, COR-L23, COR-L23/CPR, COR-L23/5010, COR-L23/R23, COS-7, COV-434, CML T1, CMT, CT26, D17, DH82, DU145, DuCaP, EL4, EM2, EM3, EMT6/AR1, EMT6/AR10.0, FM3, H1299, H69, HB54, HB55, HCA2, HEK-293, HeLa, Hepalclc7, HL-60, HMEC, HT-29, Jurkat, JY cells, K562 cells, Ku812, KCL22, KG1, KYO1, LNCap, Ma-Mel 1-48, MC-38, MCF-7, MCF-10A, MDA-MB-231, MDA-MB-468, MDA-MB-435, MDCK II, MDCK II, MOR/0.2R, MONO-MAC 6, MTD-1A, MyEnd, NCI-H69/CPR, NCI-H69/LX10, NCI-H69/LX20, NCI-H69/LX4, NIH-3T3, NALM-1, NW-145, OPCN / OPCT cell lines, Peer, PNT-1A / PNT 2, RenCa, RIN-5F, RMA/RMAS, Saos-2 cells, Sf-9, SkBr3, T2, T-47D, T84, THP1 cell line, U373, U87, U937, VCaP, Vero cells, WM39, WT-49, X63, YAC-1, YAR, and transgenic varieties thereof. Cell lines are available from a variety of sources known to those with skill in the art (see, e.g., the American Type Culture Collection (ATCC) (Manassus, Va.)). In some embodiments, a cell transfected with one or more vectors described herein is used to establish a new cell line comprising one or more vector-derived sequences. In some embodiments, a cell transiently transfected with the components of a CRISPR system as described herein (such as by transient transfection of one or more vectors, or transfection with RNA), and modified through the activity of a CRISPR complex, is used to establish a new cell line comprising cells containing the modification but lacking any other exogenous sequence. In some embodiments, cells transiently or non-transiently transfected with one or more vectors described herein, or cell lines derived from such cells are used in assessing one or more test compounds.

In some embodiments, one or more vectors described herein are used to produce a non-human transgenic animal or transgenic plant. In some embodiments, the transgenic animal is a mammal, such as a mouse, rat, or rabbit. Methods for producing transgenic animals and plants are known in the art, and generally begin with a method of cell transfection, such as described herein. In another embodiment, a fluid delivery device with an array of needles (see, e.g., US Patent Publication No. 20110230839 assigned to the Fred Hutchinson Cancer Research Center) may be contemplated for delivery of CRISPR Cas to solid tissue. A device of US Patent Publication No. 20110230839 for delivery of a fluid to a solid tissue may comprise a plurality of needles arranged in an array; a plurality of reservoirs, each in fluid communication with a respective one of the plurality of needles; and a plurality of actuators operatively coupled to respective ones of the plurality of reservoirs and configured to control a fluid pressure within the reservoir. In certain embodiments each of the plurality of actuators may comprise one of a plurality of plungers, a first end of each of the plurality of plungers being received in a respective one of the plurality of reservoirs, and in certain further embodiments the plungers of the plurality of plungers are operatively coupled together at respective second ends so as to be simultaneously depressable. Certain still further embodiments may comprise a plunger driver configured to depress all of the plurality of plungers at a selectively variable rate. In other embodiments each of the plurality of actuators may comprise one of a plurality of fluid transmission lines having first and second ends, a first end of each of the plurality of fluid transmission lines being coupled to a respective one of the plurality of reservoirs. In other embodiments the device may comprise a fluid pressure source, and each of the plurality of actuators comprises a fluid coupling between the fluid pressure source and a respective one of the plurality of reservoirs. In further embodiments the fluid pressure source may comprise at least one of a compressor, a vacuum accumulator, a peristaltic pump, a master cylinder, a microfluidic pump, and a valve. In another embodiment, each of the plurality of needles may comprise a plurality of ports distributed along its length.

Modifying a target

In some embodiments, the method comprises allowing a CRISPR complex to bind to the target polynucleotide to effect cleavage of said target polynucleotide thereby modifying the target polynucleotide, wherein the CRISPR complex comprises a CRISPR enzyme complexed with a guide sequence hybridized to a target sequence within said target polynucleotide, wherein said guide sequence is linked to a tracr mate sequence which in turn hybridizes to a tracr sequence. In one aspect, the invention provides a method of modifying expression of a polynucleotide in a eukaryotic cell. In some embodiments, the method comprises allowing a CRISPR complex to bind to the polynucleotide such that said binding results in increased or decreased expression of said polynucleotide; wherein the CRISPR complex comprises a CRISPR enzyme complexed with a guide sequence hybridized to a target sequence within said polynucleotide, wherein said guide sequence is linked to a tracr mate sequence which in turn hybridizes to a tracr sequence. Similar considerations and conditions apply as above for methods of modifying a target polynucleotide. In fact, these sampling, culturing and re-introduction options apply across the aspects of the present invention. In one aspect, the invention provides for methods of modifying a target polynucleotide in a eukaryotic cell, which may be *in vivo, ex vivo* or *in vitro.* In some embodiments, the method comprises sampling a cell or population of cells from a human or non-human animal, or a plant, and modifying the cell or cells. Culturing may occur at any stage *ex vivo.* The cell or cells may even be re-introduced into the non-human animal or plant. For re-introduced cells it is particularly preferred that the cells are stem cells. Indeed, in any aspect of the invention, the CRISPR complex may comprise a CRISPR enzyme complexed with a guide sequence hybridized to a target sequence, wherein said guide sequence may be linked to a tracr mate sequence which in turn may hybridize to a tracr sequence. Similar considerations and conditions apply as above for methods of modifying a target polynucleotide.

Kits

In one aspect, the invention provides kits containing any one or more of the elements disclosed in the above methods and compositions. Elements may be provided individually or in combinations, and may be provided in any suitable container, such as a vial, a bottle, or a tube. In some embodiments, the kit includes instructions in one or more languages, for example in more than one language.

In some embodiments, a kit comprises one or more reagents for use in a process utilizing one or more of the elements described herein. Reagents may be provided in any suitable container. For example, a kit may provide one or more reaction or storage buffers. Reagents may be provided in a form that is usable in a particular assay, or in a form that requires addition of one or more other components before use (e.g. in concentrate or lyophilized form). A buffer can be any buffer, including but not limited to a sodium carbonate buffer, a sodium bicarbonate buffer, a borate buffer, a Tris buffer, a MOPS buffer, a HEPES buffer, and combinations thereof. In some embodiments, the buffer is alkaline. In some embodiments, the buffer has a pH from about 7 to about 10. In some embodiments, the kit comprises one or more oligonucleotides corresponding to a guide sequence for insertion into a vector so as to operably link the guide sequence and a regulatory element. In some embodiments, the kit comprises a homologous recombination template polynucleotide. In some embodiments, the kit comprises one or more of the vectors and/or one or more of the polynucleotides described herein. The kit may advantageously allows to provide all elements of the systems of the invention.

CRISPR complex

In one aspect, the invention provides methods for using one or more elements of a CRISPR system. The CRISPR complex of the invention provides an effective means for modifying a target polynucleotide. The CRISPR complex of the invention has a wide variety of utility including modifying (e.g., deleting, inserting, translocating, inactivating, activating) a target polynucleotide in a multiplicity of cell types. As such the CRISPR complex of the invention has a broad spectrum of applications in, e.g., gene therapy, drug screening, disease diagnosis, and prognosis. An exemplary CRISPR complex comprises a CRISPR enzyme complexed with a guide sequence hybridized to a target sequence within the target polynucleotide. The guide sequence is linked to a tracr mate sequence, which in turn hybridizes to a tracr sequence.

In one embodiment, this invention provides a method of cleaving a target polynucleotide. The method comprises modifying a target polynucleotide using a CRISPR complex that binds to the target polynucleotide and effect cleavage of said target polynucleotide. Typically, the CRISPR complex of the invention, when introduced into a cell, creates a break (e.g., a single or a double strand break) in the genome sequence. For example, the method can be used to cleave a disease gene in a cell.

The break created by the CRISPR complex can be repaired by a repair processes such as the error prone non-homologous end joining (NHEJ) pathway or the high fidelity homology-directed repair (HDR) (Fig. 25). During these repair process, an exogenous polynucleotide template can be introduced into the genome sequence. In some methods, the HDR process is used modify genome sequence. For example, an exogenous polynucleotide template comprising a sequence to be integrated flanked by an upstream sequence and a downstream sequence is introduced into a cell. The upstream and downstream sequences share sequence similarity with either side of the site of integration in the chromosome.

Where desired, a donor polynucleotide can be DNA, e.g., a DNA plasmid, a bacterial artificial chromosome (BAC), a yeast artificial chromosome (YAC), a viral vector, a linear piece of DNA, a PCR fragment, a naked nucleic acid, or a nucleic acid complexed with a delivery vehicle such as a liposome or poloxamer.

The exogenous polynucleotide template comprises a sequence to be integrated (e.g., a mutated gene). The sequence for integration may be a sequence endogenous or exogenous to the cell. Examples of a sequence to be integrated include polynucleotides encoding a protein or a non-coding RNA (e.g., a microRNA). Thus, the sequence for integration may be operably linked to an appropriate control sequence or sequences. Alternatively, the sequence to be integrated may provide a regulatory function.

The upstream and downstream sequences in the exogenous polynucleotide template are selected to promote recombination between the chromosomal sequence of interest and the donor polynucleotide. The upstream sequence is a nucleic acid sequence that shares sequence similarity with the genome sequence upstream of the targeted site for integration. Similarly, the downstream sequence is a nucleic acid sequence that shares sequence similarity with the chromosomal sequence downstream of the targeted site of integration. The upstream and downstream sequences in the exogenous polynucleotide template can have 75%, 80%, 85%, 90%, 95%, or 100% sequence identity with the targeted genome sequence. Preferably, the upstream and downstream sequences in the exogenous polynucleotide template have about 95%, 96%, 97%, 98%, 99%, or 100% sequence identity with the targeted genome sequence. In some methods, the upstream and downstream sequences in the exogenous polynucleotide template have about 99% or 100% sequence identity with the targeted genome sequence.

An upstream or downstream sequence may comprise from about 20 bp to about 2500 bp, for example, about 50, 100, 200, 300, 400, 500, 600, 700, 800, 900, 1000, 1100, 1200, 1300, 1400, 1500, 1600, 1700, 1800, 1900, 2000, 2100, 2200, 2300, 2400, or 2500 bp. In some methods, the exemplary upstream or downstream sequence have about 200 bp to about 2000 bp, about 600 bp to about 1000 bp, or more particularly about 700 bp to about 1000 bp.

In some methods, the exogenous polynucleotide template may further comprise a marker. Such a marker may make it easy to screen for targeted integrations. Examples of suitable markers include restriction sites, fluorescent proteins, or selectable markers. The exogenous polynucleotide template of the invention can be constructed using recombinant techniques (see, for example, Sambrook et al., 2001 and Ausubel et al., 1996).

In an exemplary method for modifying a target polynucleotide by integrating an exogenous polynucleotide template, a double stranded break is introduced into the genome sequence by the CRISPR complex, the break is repaired via homologous recombination an exogenous polynucleotide template such that the template is integrated into the genome. The presence of a double-stranded break facilitates integration of the template.

In other embodiments, this invention provides a method of modifying expression of a polynucleotide in a eukaryotic cell. The method comprises increasing or decreasing expression of a target polynucleotide by using a CRISPR complex that binds to the polynucleotide.

In some methods, a target polynucleotide can be inactivated to effect the modification of the expression in a cell. For example, upon the binding of a CRISPR complex to a target sequence in a cell, the target polynucleotide is inactivated such that the sequence is not transcribed, the coded protein is not produced, or the sequence does not function as the wild-type sequence does. For example, a protein or microRNA coding sequence may be inactivated such that the protein or microRNA or pre-microRNA transcript is not produced.

In some methods, a control sequence can be inactivated such that it no longer functions as a control sequence. As used herein, "control sequence" refers to any nucleic acid sequence that effects the transcription, translation, or accessibility of a nucleic acid sequence. Examples of a control sequence include, a promoter, a transcription terminator, and an enhancer are control sequences.

The inactivated target sequence may include a deletion mutation (i.e., deletion of one or more nucleotides), an insertion mutation (i.e., insertion of one or more nucleotides), or a nonsense mutation (i.e., substitution of a single nucleotide for another nucleotide such that a stop codon is introduced). In some methods, the inactivation of a target sequence results in "knockout" of the target sequence.

Disease models

A method of the invention may be used to create a plant, an animal or cell that may be used as a disease model. As used herein, "disease" refers to a disease, disorder, or indication in a subject. For example, a method of the invention may be used to create an animal or cell that comprises a modification in one or more nucleic acid sequences associated with a disease, or a plant, animal or cell in which the expression of one or more nucleic acid sequences associated with a disease are altered. Such a nucleic acid sequence may encode a disease associated protein sequence or may be a disease associated control sequence. Accordingly, it is understood that in embodiments of the invention, a plant, subject, patient, organism or cell can be a non-human subject, patient, organism or cell. Thus, the invention provides a plant, animal or cell, produced by the present methods, or a progeny thereof. The progeny may be a clone of the produced plant or animal, or may result from sexual reproduction by crossing with other individuals of the same species to introgress further desirable traits into their offspring. The cell may be *in vivo* or *ex vivo* in the cases of multicellular organisms, particularly animals or plants. In the instance where the cell is in cultured, a cell line may be established if appropriate culturing conditions are met and preferably if the cell is suitably adapted for this purpose (for instance a stem cell). Bacterial cell lines produced by the invention are also envisaged. Hence, cell lines are also envisaged.

In some methods, the disease model can be used to study the effects of mutations on the animal or cell and development and/or progression of the disease using measures commonly used in the study of the disease. Alternatively, such a disease model is useful for studying the effect of a pharmaceutically active compound on the disease.

In some methods, the disease model can be used to assess the efficacy of a potential gene therapy strategy. That is, a disease-associated gene or polynucleotide can be modified such that the disease development and/or progression is inhibited or reduced. In particular, the method comprises modifying a disease-associated gene or polynucleotide such that an altered protein is produced and, as a result, the animal or cell has an altered response. Accordingly, in some methods, a genetically modified animal may be compared with an animal predisposed to development of the disease such that the effect of the gene therapy event may be assessed.

In another embodiment, this invention provides a method of developing a biologically active agent that modulates a cell signaling event associated with a disease gene. The method comprises contacting a test compound with a cell comprising one or more vectors that drive expression of one or more of a CRISPR enzyme, a guide sequence linked to a tracr mate sequence, and a tracr sequence; and detecting a change in a readout that is indicative of a reduction or an augmentation of a cell signaling event associated with, e.g., a mutation in a disease gene contained in the cell.

A cell model or animal model can be constructed in combination with the method of the invention for screening a cellular function change. Such a model may be used to study the effects of a genome sequence modified by the CRISPR complex of the invention on a cellular function of interest. For example, a cellular function model may be used to study the effect of a modified genome sequence on intracellular signaling or extracellular signaling. Alternatively, a cellular function model may be used to study the effects of a modified genome sequence on sensory perception. In some such models, one or more genome sequences associated with a signaling biochemical pathway in the model are modified.

Several disease models have been specifically investigated. These include *de novo* autism risk genes CHD8, KATNAL2, and SCN2A; and the syndromic autism (Angelman Syndrome) gene UBE3A. These genes and resulting autism models are of course preferred, but serve to show the broad applicability of the invention across genes and corresponding models.

An altered expression of one or more genome sequences associated with a signaling biochemical pathway can be determined by assaying for a difference in the mRNA levels of the corresponding genes between the test model cell and a control cell, when they are contacted with a candidate agent. Alternatively, the differential expression of the sequences associated with a signaling biochemical pathway is determined by detecting a difference in the level of the encoded polypeptide or gene product.

To assay for an agent-induced alteration in the level of mRNA transcripts or corresponding polynucleotides, nucleic acid contained in a sample is first extracted according to standard methods in the art. For instance, mRNA can be isolated using various lytic enzymes or chemical solutions according to the procedures set forth in Sambrook et al. (1989), or extracted by nucleic-acid-binding resins following the accompanying instructions provided by the manufacturers. The mRNA contained in the extracted nucleic acid sample is then detected by amplification procedures or conventional hybridization assays (e.g. Northern blot analysis) according to methods widely known in the art or based on the methods exemplified herein.

For purpose of this invention, amplification means any method employing a primer and a polymerase capable of replicating a target sequence with reasonable fidelity. Amplification may be carried out by natural or recombinant DNA polymerases such as TaqGold™, T7 DNA polymerase, Klenow fragment of E.coli DNA polymerase, and reverse transcriptase. A preferred amplification method is PCR. In particular, the isolated RNA can be subjected to a reverse transcription assay that is coupled with a quantitative polymerase chain reaction (RT-PCR) in order to quantify the expression level of a sequence associated with a signaling biochemical pathway.

Detection of the gene expression level can be conducted in real time in an amplification assay. In one aspect, the amplified products can be directly visualized with fluorescent DNA-binding agents including but not limited to DNA intercalators and DNA groove binders. Because the amount of the intercalators incorporated into the double-stranded DNA molecules is typically proportional to the amount of the amplified DNA products, one can conveniently determine the amount of the amplified products by quantifying the fluorescence of the intercalated dye using conventional optical systems in the art. DNA-binding dye suitable for this application include SYBR green, SYBR blue, DAPI, propidium iodine, Hoeste, SYBR gold, ethidium bromide, acridines, proflavine, acridine orange, acriflavine, fluorcoumanin, ellipticine, daunomycin, chloroquine, distamycin D, chromomycin, homidium, mithramycin, ruthenium polypyridyls, anthramycin, and the like.

In another aspect, other fluorescent labels such as sequence specific probes can be employed in the amplification reaction to facilitate the detection and quantification of the amplified products. Probe-based quantitative amplification relies on the sequence-specific detection of a desired amplified product. It utilizes fluorescent, target-specific probes (e.g., TaqMan® probes) resulting in increased specificity and sensitivity. Methods for performing probe-based quantitative amplification are well established in the art and are taught in U.S. Patent No. 5,210,015.

In yet another aspect, conventional hybridization assays using hybridization probes that share sequence homology with sequences associated with a signaling biochemical pathway can be performed. Typically, probes are allowed to form stable complexes with the sequences associated with a signaling biochemical pathway contained within the biological sample derived from the test subject in a hybridization reaction. It will be appreciated by one of skill in the art that where antisense is used as the probe nucleic acid, the target polynucleotides provided in the sample are chosen to be complementary to sequences of the antisense nucleic acids. Conversely, where the nucleotide probe is a sense nucleic acid, the target polynucleotide is selected to be complementary to sequences of the sense nucleic acid.

Hybridization can be performed under conditions of various stringency. Suitable hybridization conditions for the practice of the present invention are such that the recognition interaction between the probe and sequences associated with a signaling biochemical pathway is both sufficiently specific and sufficiently stable. Conditions that increase the stringency of a hybridization reaction are widely known and published in the art. See, for example, (Sambrook, et al., (1989); Nonradioactive In Situ Hybridization Application Manual, Boehringer Mannheim, second edition). The hybridization assay can be formed using probes immobilized on any solid support, including but are not limited to nitrocellulose, glass, silicon, and a variety of gene arrays. A preferred hybridization assay is conducted on high-density gene chips as described in U.S. Patent No. 5,445,934.

For a convenient detection of the probe-target complexes formed during the hybridization assay, the nucleotide probes are conjugated to a detectable label. Detectable labels suitable for use in the present invention include any composition detectable by photochemical, biochemical, spectroscopic, immunochemical, electrical, optical or chemical means. A wide variety of appropriate detectable labels are known in the art, which include fluorescent or chemiluminescent labels, radioactive isotope labels, enzymatic or other ligands. In preferred embodiments, one will likely desire to employ a fluorescent label or an enzyme tag, such as digoxigenin, β-galactosidase, urease, alkaline phosphatase or peroxidase, avidin/biotin complex.

The detection methods used to detect or quantify the hybridization intensity will typically depend upon the label selected above. For example, radiolabels may be detected using photographic film or a phosphoimager. Fluorescent markers may be detected and quantified using a photodetector to detect emitted light. Enzymatic labels are typically detected by providing the enzyme with a substrate and measuring the reaction product produced by the action of the enzyme on the substrate; and finally colorimetric labels are detected by simply visualizing the colored label.

An agent-induced change in expression of sequences associated with a signaling biochemical pathway can also be determined by examining the corresponding gene products. Determining the protein level typically involves a) contacting the protein contained in a biological sample with an agent that specifically bind to a protein associated with a signaling biochemical pathway; and (b) identifying any agent:protein complex so formed. In one aspect of this embodiment, the agent that specifically binds a protein associated with a signaling biochemical pathway is an antibody, preferably a monoclonal antibody.

The reaction is performed by contacting the agent with a sample of the proteins associated with a signaling biochemical pathway derived from the test samples under conditions that will allow a complex to form between the agent and the proteins associated with a signaling biochemical pathway. The formation of the complex can be detected directly or indirectly according to standard procedures in the art. In the direct detection method, the agents are supplied with a detectable label and unreacted agents may be removed from the complex; the amount of remaining label thereby indicating the amount of complex formed. For such method, it is preferable to select labels that remain attached to the agents even during stringent washing conditions. It is preferable that the label does not interfere with the binding reaction. In the alternative, an indirect detection procedure may use an agent that contains a label introduced either chemically or enzymatically. A desirable label generally does not interfere with binding or the stability of the resulting agent:polypeptide complex. However, the label is typically designed to be accessible to an antibody for an effective binding and hence generating a detectable signal.

A wide variety of labels suitable for detecting protein levels are known in the art. Non-limiting examples include radioisotopes, enzymes, colloidal metals, fluorescent compounds, bioluminescent compounds, and chemiluminescent compounds.

The amount of agent:polypeptide complexes formed during the binding reaction can be quantified by standard quantitative assays. As illustrated above, the formation of agent:polypeptide complex can be measured directly by the amount of label remained at the site of binding. In an alternative, the protein associated with a signaling biochemical pathway is tested for its ability to compete with a labeled analog for binding sites on the specific agent. In this competitive assay, the amount of label captured is inversely proportional to the amount of protein sequences associated with a signaling biochemical pathway present in a test sample.

A number of techniques for protein analysis based on the general principles outlined above are available in the art. They include but are not limited to radioimmunoassays, ELISA (enzyme linked immunoradiometric assays), "sandwich" immunoassays, immunoradiometric assays, in situ immunoassays (using e.g., colloidal gold, enzyme or radioisotope labels), western blot analysis, immunoprecipitation assays, immunofluorescent assays, and SDS-PAGE.

Antibodies that specifically recognize or bind to proteins associated with a signaling biochemical pathway are preferable for conducting the aforementioned protein analyses. Where desired, antibodies that recognize a specific type of post-translational modifications (e.g., signaling biochemical pathway inducible modifications) can be used. Post-translational modifications include but are not limited to glycosylation, lipidation, acetylation, and phosphorylation. These antibodies may be purchased from commercial vendors. For example, anti-phosphotyrosine antibodies that specifically recognize tyrosine-phosphorylated proteins are available from a number of vendors including Invitrogen and Perkin Elmer. Anti-phosphotyrosine antibodies are particularly useful in detecting proteins that are differentially phosphorylated on their tyrosine residues in response to an ER stress. Such proteins include but are not limited to eukaryotic translation initiation factor 2 alpha (eIF-2$\alpha$). Alternatively, these antibodies can be generated using conventional polyclonal or monoclonal antibody technologies by immunizing a host animal or an antibody-producing cell with a target protein that exhibits the desired post-translational modification.

In practicing the subject method, it may be desirable to discern the expression pattern of an protein associated with a signaling biochemical pathway in different bodily tissue, in different cell types, and/or in different subcellular structures. These studies can be performed with the use of tissue-specific, cell-specific or subcellular structure specific antibodies capable of binding to protein markers that are preferentially expressed in certain tissues, cell types, or subcellular structures.

An altered expression of a gene associated with a signaling biochemical pathway can also be determined by examining a change in activity of the gene product relative to a control cell. The assay for an agent-induced change in the activity of a protein associated with a signaling biochemical pathway will dependent on the biological activity and/or the signal transduction pathway that is under investigation. For example, where the protein is a kinase, a change in its ability to phosphorylate the downstream substrate(s) can be determined by a variety of assays known in the art. Representative assays include but are not limited to immunoblotting and immunoprecipitation with antibodies such as anti-phosphotyrosine antibodies that recognize phosphorylated proteins. In addition, kinase activity can be detected by high throughput chemiluminescent assays such as AlphaScreen™ (available from Perkin Elmer) and eTag™ assay (Chan-Hui, et al. (2003) Clinical Immunology 111: 162-174).

Where the protein associated with a signaling biochemical pathway is part of a signaling cascade leading to a fluctuation of intracellular pH condition, pH sensitive molecules such as fluorescent pH dyes can be used as the reporter molecules. In another example where the protein associated with a signaling biochemical pathway is an ion channel, fluctuations in membrane potential and/or intracellular ion concentration can be monitored. A number of commercial kits and high-throughput devices are particularly suited for a rapid and robust screening for modulators of ion channels. Representative instruments include FLIPRTM (Molecular Devices, Inc.) and VIPR (Aurora Biosciences). These instruments are capable of detecting reactions in over 1000 sample wells of a microplate simultaneously, and providing real-time measurement and functional data within a second or even a minisecond.

In practicing any of the methods disclosed herein, a suitable vector can be introduced to a cell or an embryo via one or more methods known in the art, including without limitation, microinjection, electroporation, sonoporation, biolistics, calcium phosphate-mediated transfection, cationic transfection, liposome transfection, dendrimer transfection, heat shock transfection, nucleofection transfection, magnetofection, lipofection, impalefection, optical transfection, proprietary agent-enhanced uptake of nucleic acids, and delivery via liposomes, immunoliposomes, virosomes, or artificial virions. In some methods, the vector is introduced into an embryo by microinjection. The vector or vectors may be microinjected into the nucleus or the cytoplasm of the embryo. In some methods, the vector or vectors may be introduced into a cell by nucleofection.

The target polynucleotide of a CRISPR complex can be any polynucleotide endogenous or exogenous to the eukaryotic cell. For example, the target polynucleotide can be a polynucleotide residing in the nucleus of the eukaryotic cell. The target polynucleotide can be a sequence coding a gene product (e.g., a protein) or a non-coding sequence (e.g., a regulatory polynucleotide or a junk DNA).

Examples of target polynucleotides include a sequence associated with a signaling biochemical pathway, e.g., a signaling biochemical pathway-associated gene or polynucleotide. Examples of target polynucleotides include a disease associated gene or polynucleotide. A "disease-associated" gene or polynucleotide refers to any gene or polynucleotide which is yielding transcription or translation products at an abnormal level or in an abnormal form in cells derived from a disease-affected tissues compared with tissues or cells of a non disease control. It may be a gene that becomes expressed at an abnormally high level; it may be a gene that becomes expressed at an abnormally low level, where the altered expression correlates with the occurrence and/or progression of the disease. A disease-associated gene also refers to a gene possessing mutation(s) or genetic variation that is directly responsible or is in linkage disequilibrium with a gene(s) that is responsible for the etiology of a disease. The transcribed or translated products may be known or unknown, and may be at a normal or abnormal level.

The target polynucleotide of a CRISPR complex can be any polynucleotide endogenous or exogenous to the eukaryotic cell. For example, the target polynucleotide can be a polynucleotide residing in the nucleus of the eukaryotic cell. The target polynucleotide can be a sequence coding a gene product (e.g., a protein) or a non-coding sequence (e.g., a regulatory polynucleotide or a junk DNA). Without wishing to be bound by theory, it is believed that the target sequence should be associated with a PAM (protospacer adjacent motif); that is, a short sequence recognized by the CRISPR complex. The precise sequence and length requirements for the PAM differ depending on the CRISPR enzyme used, but PAMs are typically 2-5 base pair sequences adjacent the protospacer (that is, the target sequence) Examples of PAM sequences are given in the examples section below, and the skilled person will be able to identify further PAM sequences for use with a given CRISPR enzyme.

The target polynucleotide of a CRISPR complex may include a number of disease-associated genes and polynucleotides as well as signaling biochemical pathway-associated genes and polynucleotides as listed in US provisional patent applications 61/736,527 and 61/748,427 having Broad reference BI-2011/008/WSGR Docket No. 44063-701.101 and BI-2011/008/WSGR Docket No. 44063-701.102 respectively, both entitled SYSTEMS METHODS AND COMPOSITIONS FOR SEQUENCE MANIPULATION filed on December 12, 2012 and January 2, 2013, respectively, the contents of all of which are herein incorporated by reference in their entirety.

Examples of target polynucleotides include a sequence associated with a signaling biochemical pathway, e.g., a signaling biochemical pathway-associated gene or polynucleotide. Examples of target polynucleotides include a disease associated gene or polynucleotide. A "disease-associated" gene or polynucleotide refers to any gene or polynucleotide which is yielding transcription or translation products at an abnormal level or in an abnormal form in cells derived from a disease-affected tissues compared with tissues or cells of a non disease control. It may be a gene that becomes expressed at an abnormally high level; it may be a gene that becomes expressed at an abnormally low level, where the altered expression correlates with the occurrence and/or progression of the disease. A disease-associated gene also refers to a gene possessing mutation(s) or genetic variation that is directly responsible or is in linkage disequilibrium with a gene(s) that is responsible for the etiology of a disease. The transcribed or translated products may be known or unknown, and may be at a normal or abnormal level.

Examples of disease-associated genes and polynucleotides are listed in Tables A and B. Disease specific information is available from McKusick-Nathans Institute of Genetic Medicine, Johns Hopkins University (Baltimore, Md.) and National Center for Biotechnology Information, National Library of Medicine (Bethesda, Md.), available on the World Wide Web. Examples of signaling biochemical pathway-associated genes and polynucleotides are listed in Table C.

Mutations in these genes and pathways can result in production of improper proteins or proteins in improper amounts which affect function. Further examples of genes, diseases and proteins are hereby incorporated by reference from US Provisional application 61/736,527 filed December 12, 2012. Such genes, proteins and pathways may be the target polynucleotide of a CRISPR complex.

**Table A**

| DISEASE/DISORDER S | GENE(S) |
|---|---|
| Neoplasia | PTEN; ATM; ATR; EGFR; ERBB2; ERBB3; ERBB4; |
| | Notch1; Notch2; Notch3; Notch4; AKT; AKT2; AKT3; HIF; |
| | HIF1a; HIF3a; Met; HRG; Bcl2; PPAR alpha; PPAR |
| | gamma; WT1 (Wilms Tumor); FGF Receptor Family |
| | members (5 members: 1, 2, 3, 4, 5); CDKN2a; APC; RB |
| | (retinoblastoma); MEN1; VHL; BRCA1; BRCA2; AR |
| | (Androgen Receptor); TSG101; IGF; IGF Receptor; Igf1 (4 |
| | variants); Igf2 (3 variants); Igf 1 Receptor; Igf 2 Receptor; |
| | Bax; Bcl2; caspases family (9 members: |
| | 1, 2, 3, 4, 6, 7, 8, 9, 12); Kras; Apc |
| Age-related Macular | Abcr; Ccl2; Cc2; cp (ceruloplasmin); Timp3; cathepsinD; |

(continued)

| DISEASE/DISORDER S | GENE(S) |
|---|---|
| Degeneration | Vldlr; Ccr2 |
| Schizophrenia | Neuregulin1 (Nrg1); Erb4 (receptor for Neuregulin); |
| | Complexin1 (Cplx1); Tph1 Tryptophan hydroxylase; Tph2 |
| | Tryptophan hydroxylase 2; Neurexin 1; GSK3; GSK3a; |
| | GSK3b |
| Disorders | 5-HTT (Slc6a4); COMT; DRD (Drdla); SLC6A3; DAOA; |
| | DTNBP1; Dao (Dao1) |
| Trinucleotide Repeat | HTT (Huntington's Dx); SBMA/SMAX1/AR (Kennedy's |
| Disorders | Dx); FXN/X25 (Friedrich's Ataxia); ATX3 (Machado- |
| | Joseph's Dx); ATXN1 and ATXN2 (spinocerebellar |
| | ataxias); DMPK (myotonic dystrophy); Atrophin-1 and Atn1 |
| | (DRPLA Dx); CBP (Creb-BP - global instability); VLDLR |
| | (Alzheimer's); Atxn7; Atxn10 |
| Fragile X Syndrome | FMR2; FXR1; FXR2; mGLUR5 |
| Secretase Related | APH-1 (alpha and beta); Presenilin (Psen1); nicastrin |
| Disorders | (Ncstn); PEN-2 |
| Others | Nos1; Parp1; Natl; Nat2 |
| Prion - related disorders | Prp |
| ALS | SOD1; ALS2; STEX; FUS; TARDBP; VEGF (VEGF-a; |
| | VEGF-b; VEGF-c) |
| Drug addiction | Prkce (alcohol); Drd2; Drd4; ABAT (alcohol); GRIA2; |
| | Grm5; Grin1; Htr1b; Grin2a; Drd3; Pdyn; Grial (alcohol) |
| Autism | Mecp2; BZRAP1; MDGA2; Sema5A; Neurexin 1; Fragile X |
| | (FMR2 (AFF2); FXR1; FXR2; Mglur5) |
| Alzheimer's Disease | E1; CHIP; UCH; UBB; Tau; LRP; PICALM; Clusterin; PS1; |
| | SORL1; CR1; Vldlr; Uba1; Uba3; CHIP28 (Aqp1, |
| | Aquaporin 1); Uchl1; Uchl3; APP |
| Inflammation | IL-10; IL-1 (IL-1a; IL-1b); IL-13; IL-17 (IL-17a (CTLA8); IL- |
| | 17b; IL-17c; IL-17d; IL-17f); II-23; Cx3cr1; ptpn22; TNFa; |
| | NOD2/CARD15 for IBD; IL-6; IL-12 (IL-12a; IL-12b); |
| | CTLA4; Cx3cl1 |
| Parkinson's Disease | x-Synuclein; DJ-1; LRRK2; Parkin; PINK1 |

**Table B:**

| | |
|---|---|
| Blood and coagulation diseases and disorders | Anemia (CDAN1, CDA1, RPS19, DBA, PKLR, PK1, NT5C3, UMPH1, PSN1, RHAG, RH50A, NRAMP2, SPTB, ALAS2, ANH1, ASB, ABCB7, ABC7, ASAT); Bare lymphocyte syndrome (TAPBP, TPSN, TAP2, ABCB3, PSF2, RING11, MHC2TA, C2TA, RFX5, RFXAP, RFX5), Bleeding disorders (TBXA2R, P2RX1, P2X1); Factor H and factor H-like 1 (HF1, CFH, HUS); Factor V and factor VIII (MCFD2); Factor VII deficiency (F7); Factor X deficiency (F10); Factor XI deficiency (F11); Factor XII deficiency (F12, HAF); Factor XIIIA deficiency (F13A1, F13A); Factor XIIIB deficiency (F13B); Fanconi anemia (FANCA, FACA, FA1, FA, FAA, FAAP95, FAAP90, FLJ34064, FANCB, FANCC, FACC, BRCA2, FANCD1, FANCD2, FANCD, FACD, FAD, FANCE, FACE, FANCF, XRCC9, FANCG, BRIP1, BACH1, FANCJ, PHF9, FANCL, FANCM, KIAA1596); Hemophagocytic lymphohistiocytosis disorders (PRF1, HPLH2, UNC13D, MUNC13-4, HPLH3, HLH3, FHL3); Hemophilia A (F8, F8C, HEMA); Hemophilia B (F9, HEMB), Hemorrhagic disorders (PI, ATT, F5); Leukocyde deficiencies and disorders (ITGB2, CD18, LCAMB, LAD, EIF2B1, EIF2BA, EIF2B2, EIF2B3, EIF2B5, LVWM, CACH, CLE, EIF2B4); Sickle cell anemia (HBB); Thalassemia (HBA2, HBB, HBD, LCRB, HBA1). |
| | |
| Cell dysregulationand oncology diseases and disorders | B-cell non-Hodgkin lymphoma (BCL7A, BCL7); Leukemia (TALI, TCL5, SCL, TAL2, FLT3, NBS1, NBS, ZNFN1A1, IK1, LYF1, HOXD4, HOX4B, BCR, CML, PHL, ALL, ARNT, KRAS2, RASK2, GMPS, AF10, ARHGEF12, LARG, KIAA0382, CALM, CLTH, CEBPA, CEBP, CHIC2, BTL, FLT3, KIT, PBT, LPP, NPM1, NUP214, D9S46E, CAN, CAIN, RUNX1, CBFA2, AML1, WHSC1L1, NSD3, FLT3, AF1Q, NPM1, NUMA1, ZNF145, PLZF, PML, MYL, STAT5B, AF10, CALM, CLTH, ARL11, ARLTS1, P2RX7, P2X7, BCR, CML, PHL, ALL, GRAF, NF1, VRNF, WSS, NFNS, PTPN11, PTP2C, SHP2, NS1, BCL2, CCND1, PRAD1, BCL1, TCRA, GATA1, GF1, ERYF1, |
| | NFE1, ABL1, NQO1, DIA4, NMOR1, NUP214, D9S46E, CAN, CAIN). |
| | |
| Inflammation and immune related diseases and disorders | AIDS (KIR3DL1, NKAT3, NKB1, AMB11, KIR3DS1, IFNG, CXCL12, SDF1); Autoimmune lymphoproliferative syndrome (TNFRSF6, APT1, FAS, CD95, ALPS1A); Combined immunodeficiency, (IL2RG, SCIDX1, SCIDX, IMD4); HIV-1 (CCL5, SCYA5, D17S136E, TCP228), HIV susceptibility or infection (IL10, CSIF, CMKBR2, CCR2, CMKBR5, CCCKR5 (CCR5)); Immunodeficiencies (CD3E, CD3G, AICDA, AID, HIGM2, TNFRSF5, CD40, UNG, DGU, HIGM4, TNFSF5, CD40LG, HIGM1, IGM, FOXP3, IPEX, AIID, XPID, PIDX, TNFRSF14B, TACI); Inflammation (IL-10, IL-1 (IL-1a, IL-1b), IL-13, IL-17 (IL-17a (CTLA8), IL-17b, IL-17c, IL-17d, IL-17f), II-23, Cx3cr1, ptpn22, TNFa, NOD2/CARD15 for IBD, IL-6, IL-12 (IL-12a, IL-12b), CTLA4, Cx3cl1); Severe combined immunodeficiencies (SCIDs)(JAK3, JAKL, DCLRE1C, ARTEMIS, SCIDA, RAG1, RAG2, ADA, PTPRC, CD45, LCA, IL7R, CD3D, T3D, IL2RG, SCIDX1, SCIDX, IMD4). |
| | |
| Metabolic, liver, kidney and protein diseases and disorders | Amyloid neuropathy (TTR, PALB); Amyloidosis (APOA1, APP, AAA, CVAP, AD1, GSN, FGA, LYZ, TTR, PALB); Cirrhosis (KRT18, KRT8, CIRH1A, NAIC, TEX292, KIAA1988); Cystic fibrosis (CFTR, ABCC7, CF, MRP7); Glycogen storage diseases (SLC2A2, GLUT2, G6PC, G6PT, G6PT1, GAA, LAMP2, LAMPB, AGL, GDE, GBE1, GYS2, PYGL, PFKM); Hepatic adenoma, 142330 (TCF1, HNF1A, MODY3), Hepatic failure, early onset, and neurologic disorder (SCOD1, SCO1), Hepatic lipase deficiency (LIPC), Hepatoblastoma, cancer and carcinomas (CTNNB1, PDGFRL, PDGRL, PRLTS, AXIN1, AXIN, CTNNB1, TP53, P53, LFS1, IGF2R, MPRI, MET, CASP8, MCH5; Medullary cystic kidney disease (UMOD, HNFJ, FJHN, MCKD2, ADMCKD2); Phenylketonuria (PAH, PKU1, QDPR, DHPR, PTS); Polycystic kidney and hepatic disease (FCYT, PKHD1, ARPKD, PKD1, PKD2, PKD4, PKDTS, PRKCSH, G19P1, PCLD, SEC63). |
| | |

(continued)

| | |
|---|---|
| Muscular / Skeletal diseases and disorders | Becker muscular dystrophy (DMD, BMD, MYF6), Duchenne Muscular Dystrophy (DMD, BMD); Emery-Dreifuss muscular dystrophy (LMNA, LMN1, EMD2, FPLD, CMD1A, HGPS, LGMD1B, LMNA, LMN1, EMD2, FPLD, CMD1A); Facioscapulohumeral muscular dystrophy (FSHMD1A, FSHD1A); Muscular dystrophy (FKRP, MDC1C, LGMD2I, LAMA2, LAMM, LARGE, KIAA0609, MDC1D, FCMD, TTID, MYOT, CAPN3, CANP3, DYSF, LGMD2B, SGCG, LGMD2C, DMDA1, SCG3, SGCA, ADL, DAG2, LGMD2D, DMDA2, SGCB, LGMD2E, SGCD, SGD, LGMD2F, CMD1L, TCAP, LGMD2G, CMD1N, TRIM32, HT2A, LGMD2H, FKRP, MDC1C, LGMD2I, TTN, CMD1G, TMD, LGMD2J, POMT1, CAV3, LGMD1C, SEPN1, SELN, RSMD1, PLEC1, PLTN, EBS1); Osteopetrosis (LRP5, BMND1, LRP7, LR3, OPPG, VBCH2, CLCN7, CLC7, OPTA2, OSTM1, GL, TCIRG1, TIRC7, OC116, OPTB1); Muscular atrophy (VAPB, VAPC, ALS8, |
| | SMN1, SMA1, SMA2, SMA3, SMA4, BSCL2, SPG17, GARS, SMAD1, CMT2D, HEXB, IGHMBP2, SMUBP2, CATF1, SMARD1). |
| | |
| Neurological and neuronal diseases and disorders | ALS (SOD1, ALS2, STEX, FUS, TARDBP, VEGF (VEGF-a, VEGF-b, VEGF-c); Alzheimer disease (APP, AAA, CVAP, AD1, APOE, AD2, PSEN2, AD4, STM2, APBB2, FE65L1, NOS3, PLAU, URK, ACE, DCP1, ACE1, MPO, PACIP1, PAXIP1L, PTIP, A2M, BLMH, BMH, PSEN1, AD3); Autism (Mecp2, BZRAP1, MDGA2, Sema5A, Neurexin 1, GLO1, MECP2, RTT, PPMX, MRX16, MRX79, NLGN3, NLGN4, KIAA1260, AUTSX2); Fragile X Syndrome (FMR2, FXR1, FXR2, mGLUR5); Huntington's disease and disease like disorders (HD, IT15, PRNP, PRIP, JPH3, JP3, HDL2, TBP, SCA17); Parkinson disease (NR4A2, NURR1, NOT, TINUR, SNCAIP, TBP, SCA17, SNCA, NACP, PARK1, PARK4, DJ1, PARK7, LRRK2, PARK8, PINK1, PARK6, UCHL1, PARK5, SNCA, NACP, PARK1, PARK4, PRKN, PARK2, PDJ, DBH, NDUFV2); Rett syndrome (MECP2, RTT, PPMX, MRX16, MRX79, CDKL5, STK9, MECP2, RTT, PPMX, MRX16, MRX79, x-Synuclein, DJ-1); Schizophrenia (Neuregulin1 (Nrg1), Erb4 (receptor for Neuregulin), Complexin1 (Cplx1), Tph1 Tryptophan hydroxylase, Tph2, Tryptophan hydroxylase 2, Neurexin 1, GSK3, GSK3a, GSK3b, 5-HTT (Slc6a4), COMT, DRD (Drdla), SLC6A3, DAOA, DTNBP1, Dao (Dao1)); Secretase Related Disorders (APH-1 (alpha and beta), Presenilin (Psen1), nicastrin, (Ncstn), PEN-2, Nos1, Parp1, Natl, Nat2); Trinucleotide Repeat Disorders (HTT (Huntington's Dx), SBMA/SMAX1/AR (Kennedy's Dx), FXN/X25 (Friedrich's Ataxia), ATX3 (Machado-Joseph's Dx), ATXN1 and ATXN2 (spinocerebellar ataxias), DMPK (myotonic dystrophy), Atrophin-1 and Atn1 (DRPLA Dx), CBP (Creb-BP - global instability), VLDLR (Alzheimer's), Atxn7, Atxn10). |
| | |
| Occular diseases and disorders | Age-related macular degeneration (Abcr, Ccl2, Cc2, cp (ceruloplasmin), Timp3, cathepsinD, Vldlr, Ccr2); Cataract (CRYAA, CRYA1, CRYBB2, CRYB2, PITX3, BFSP2, CP49, CP47, CRYAA, CRYA1, PAX6, AN2, MGDA, CRYBA1, CRYB1, CRYGC, CRYG3, CCL, LIM2, MP19, CRYGD, CRYG4, BFSP2, CP49, CP47, HSF4, CTM, HSF4, CTM, MIP, AQP0, CRYAB, CRYA2, CTPP2, CRYBB1, CRYGD, CRYG4, CRYBB2, CRYB2, CRYGC, CRYG3, CCL, CRYAA, CRYA1, GJA8, CX50, CAE1, GJA3, CX46, CZP3, CAE3, CCM1, CAM, KRIT1); Corneal clouding and dystrophy (APOA1, TGFBI, CSD2, CDGG1, CSD, BIGH3, CDG2, TACSTD2, TROP2, M1S1, VSX1, RINX, PPCD, PPD, KTCN, COL8A2, FECD, PPCD2, PIP5K3, CFD); Cornea plana congenital (KERA, CNA2); Glaucoma (MYOC, TIGR, GLC1A, JOAG, GPOA, OPTN, GLC1E, FIP2, HYPL, NRP, CYP1B1, GLC3A, OPA1, NTG, NPG, CYP1B1, GLC3A); Leber congenital amaurosis (CRB1, RP12, CRX, CORD2, CRD, RPGRIP1, LCA6, CORD9, RPE65, RP20, AIPL1, LCA4, GUCY2D, GUC2D, LCA1, CORD6, RDH12, LCA3); |
| | Macular dystrophy (ELOVL4, ADMD, STGD2, STGD3, RDS, RP7, PRPH2, PRPH, AVMD, AOFMD, VMD2). |

**Table C:**

| CELLULAR FUNCTION | GENES |
|---|---|
| PI3K/AKT Signaling | PRKCE; ITGAM; ITGA5; IRAK1; PRKAA2; EIF2AK2; |

(continued)

| CELLULAR FUNCTION | GENES |
|---|---|
| | PTEN; EIF4E; PRKCZ; GRK6; MAPK1; TSC1; PLK1; |
| | AKT2; IKBKB; PIK3CA; CDK8; CDKN1B; NFKB2; BCL2; |
| | PIK3CB; PPP2R1A; MAPK8; BCL2L1; MAPK3; TSC2; |
| | ITGA1; KRAS; EIF4EBP1; RELA; PRKCD; NOS3; |
| | PRKAA1; MAPK9; CDK2; PPP2CA; PIM1; ITGB7; |
| | YWHAZ; ILK; TP53; RAF1; IKBKG; RELB; DYRK1A; |
| | CDKN1A; ITGB1; MAP2K2; JAK1; AKT1; JAK2; PIK3R1; |
| | CHUK; PDPK1; PPP2R5C; CTNNB1; MAP2K1; NFKB1; |
| | PAK3; ITGB3; CCND1; GSK3A; FRAP1; SFN; ITGA2; |
| | TTK; CSNK1A1; BRAF; GSK3B; AKT3; FOXO1; SGK; |
| | HSP90AA1; RPS6KB1 |
| ERK/MAPK Signaling | PRKCE; ITGAM; ITGA5; HSPB1; IRAK1; PRKAA2; |
| | EIF2AK2; RAC1; RAP1A; TLN1; EIF4E; ELK1; GRK6; |
| | MAPK1; RAC2; PLK1; AKT2; PIK3CA; CDK8; CREB1; |
| | PRKCI; PTK2; FOS; RPS6KA4; PIK3CB; PPP2R1A; |
| | PIK3C3; MAPK8; MAPK3; ITGA1; ETS1; KRAS; MYCN; |
| | EIF4EBP1; PPARG; PRKCD; PRKAA1; MAPK9; SRC; |
| | CDK2; PPP2CA; PIM1; PIK3C2A; ITGB7; YWHAZ; |
| | PPP1CC; KSR1; PXN; RAF1; FYN; DYRK1A; ITGB1; |
| | MAP2K2; PAK4; PIK3R1; STAT3; PPP2R5C; MAP2K1; |
| | PAK3; ITGB3; ESR1; ITGA2; MYC; TTK; CSNK1A1; |
| | CRKL; BRAF; ATF4; PRKCA; SRF; STAT1; SGK |
| Glucocorticoid Receptor | RAC1; TAF4B; EP300; SMAD2; TRAF6; PCAF; ELK1; |
| Signaling | MAPK1; SMAD3; AKT2; IKBKB; NCOR2; UBE2I; |
| | PIK3CA; CREB1; FOS; HSPA5; NFKB2; BCL2; |
| | MAP3K14; STAT5B; PIK3CB; PIK3C3; MAPK8; BCL2L1; |
| | MAPK3; TSC22D3; MAPK10; NRIP1; KRAS; MAPK13; |
| | RELA; STAT5A; MAPK9; NOS2A; PBX1; NR3C1; |
| | PIK3C2A; CDKN1C; TRAF2; SERPINE1; NCOA3; |
| | MAPK14; TNF; RAF1; IKBKG; MAP3K7; CREBBP; |
| | CDKN1A; MAP2K2; JAK1; IL8; NCOA2; AKT1; JAK2; |
| | PIK3R1; CHUK; STAT3; MAP2K1; NFKB1; TGFBR1; |
| | ESR1; SMAD4; CEBPB; JUN; AR; AKT3; CCL2; MMP1; |
| | STAT1; IL6; HSP90AA1 |
| Axonal Guidance Signaling | PRKCE; ITGAM; ROCK1; ITGA5; CXCR4; ADAM12; |
| | IGF1; RAC1; RAP1A; EIF4E; PRKCZ; NRP1; NTRK2; |
| | ARHGEF7; SMO; ROCK2; MAPK1; PGF; RAC2; |
| | PTPN11; GNAS; AKT2; PIK3CA; ERBB2; PRKCI; PTK2; |

(continued)

| CELLULAR FUNCTION | GENES |
|---|---|
| | CFL1; GNAQ; PIK3CB; CXCL12; PIK3C3; WNT11; |
| | PRKD1; GNB2L1; ABL1; MAPK3; ITGA1; KRAS; RHOA; |
| | PRKCD; PIK3C2A; ITGB7; GLI2; PXN; VASP; RAF1; |
| | FYN; ITGB1; MAP2K2; PAK4; ADAM17; AKT1; PIK3R1; |
| | GLI1; WNT5A; ADAM10; MAP2K1; PAK3; ITGB3; |
| | CDC42; VEGFA; ITGA2; EPHA8; CRKL; RND1; GSK3B; |
| | AKT3; PRKCA |
| Ephrin Receptor Signaling | PRKCE; ITGAM; ROCK1; ITGA5; CXCR4; IRAK1; |
| | PRKAA2; EIF2AK2; RAC1; RAP1A; GRK6; ROCK2; |
| | MAPK1; PGF; RAC2; PTPN11; GNAS; PLK1; AKT2; |
| | DOK1; CDK8; CREB1; PTK2; CFL1; GNAQ; MAP3K14; |
| | CXCL12; MAPK8; GNB2L1; ABL1; MAPK3; ITGA1; |
| | KRAS; RHOA; PRKCD; PRKAA1; MAPK9; SRC; CDK2; |
| | PIM1; ITGB7; PXN; RAF1; FYN; DYRK1A; ITGB1; |
| | MAP2K2; PAK4; AKT1; JAK2; STAT3; ADAM10; |
| | MAP2K1; PAK3; ITGB3; CDC42; VEGFA; ITGA2; |
| | EPHA8; TTK; CSNK1A1; CRKL; BRAF; PTPN13; ATF4; |
| | AKT3; SGK |
| Actin Cytoskeleton | ACTN4; PRKCE; ITGAM; ROCK1; ITGA5; IRAK1; |
| Signaling | PRKAA2; EIF2AK2; RAC1; INS; ARHGEF7; GRK6; |
| | ROCK2; MAPK1; RAC2; PLK1; AKT2; PIK3CA; CDK8; |
| | PTK2; CFL1; PIK3CB; MYH9; DIAPH1; PIK3C3; MAPK8; |
| | F2R; MAPK3; SLC9A1; ITGA1; KRAS; RHOA; PRKCD; |
| | PRKAA1; MAPK9; CDK2; PIM1; PIK3C2A; ITGB7; |
| | PPP1CC; PXN; VIL2; RAF1; GSN; DYRK1A; ITGB1; |
| | MAP2K2; PAK4; PIP5K1A; PIK3R1; MAP2K1; PAK3; |
| | ITGB3; CDC42; APC; ITGA2; TTK; CSNK1A1; CRKL; |
| | BRAF; VAV3; SGK |
| Huntington's Disease | PRKCE; IGF1; EP300; RCOR1; PRKCZ; HDAC4; TGM2; |
| Signaling | MAPK1; CAPNS1; AKT2; EGFR; NCOR2; SP1; CAPN2; |
| | PIK3CA; HDAC5; CREB1; PRKCI; HSPA5; REST; |
| | GNAQ; PIK3CB; PIK3C3; MAPK8; IGF1R; PRKD1; |
| | GNB2L1; BCL2L1; CAPN1; MAPK3; CASP8; HDAC2; |
| | HDAC7A; PRKCD; HDAC11; MAPK9; HDAC9; PIK3C2A; |
| | HDAC3; TP53; CASP9; CREBBP; AKT1; PIK3R1; |
| | PDPK1; CASP1; APAF1; FRAP1; CASP2; JUN; BAX; |
| | ATF4; AKT3; PRKCA; CLTC; SGK; HDAC6; CASP3 |
| Apoptosis Signaling | PRKCE; ROCK1; BID; IRAK1; PRKAA2; EIF2AK2; BAK1; |

(continued)

| CELLULAR FUNCTION | GENES |
|---|---|
| | BIRC4; GRK6; MAPK1; CAPNS1; PLK1; AKT2; IKBKB; |
| | CAPN2; CDK8; FAS; NFKB2; BCL2; MAP3K14; MAPK8; |
| | BCL2L1; CAPN1; MAPK3; CASP8; KRAS; RELA; |
| | PRKCD; PRKAA1; MAPK9; CDK2; PIM1; TP53; TNF; |
| | RAF1; IKBKG; RELB; CASP9; DYRK1A; MAP2K2; |
| | CHUK; APAF1; MAP2K1; NFKB1; PAK3; LMNA; CASP2; |
| | BIRC2; TTK; CSNK1A1; BRAF; BAX; PRKCA; SGK; |
| | CASP3; BIRC3; PARP1 |
| B Cell Receptor Signaling | RAC1; PTEN; LYN; ELK1; MAPK1; RAC2; PTPN11; |
| | AKT2; IKBKB; PIK3CA; CREB1; SYK; NFKB2; CAMK2A; |
| | MAP3K14; PIK3CB; PIK3C3; MAPK8; BCL2L1; ABL1; |
| | MAPK3; ETS1; KRAS; MAPK13; RELA; PTPN6; MAPK9; |
| | EGR1; PIK3C2A; BTK; MAPK14; RAF1; IKBKG; RELB; |
| | MAP3K7; MAP2K2; AKT1; PIK3R1; CHUK; MAP2K1; |
| | NFKB1; CDC42; GSK3A; FRAP1; BCL6; BCL10; JUN; |
| | GSK3B; ATF4; AKT3; VAV3; RPS6KB1 |
| Leukocyte Extravasation | ACTN4; CD44; PRKCE; ITGAM; ROCK1; CXCR4; CYBA; |
| Signaling | RAC1; RAP1A; PRKCZ; ROCK2; RAC2; PTPN11; |
| | MMP14; PIK3CA; PRKCI; PTK2; PIK3CB; CXCL12; |
| | PIK3C3; MAPK8; PRKD1; ABL1; MAPK10; CYBB; |
| | MAPK13; RHOA; PRKCD; MAPK9; SRC; PIK3C2A; BTK; |
| | MAPK14; NOX1; PXN; VIL2; VASP; ITGB1; MAP2K2; |
| | CTNND1; PIK3R1; CTNNB1; CLDN1; CDC42; F11R; ITK; |
| | CRKL; VAV3; CTTN; PRKCA; MMP1; MMP9 |
| Integrin Signaling | ACTN4; ITGAM; ROCK1; ITGA5; RAC1; PTEN; RAP1A; |
| | TLN1; ARHGEF7; MAPK1; RAC2; CAPNS1; AKT2; |
| | CAPN2; PIK3CA; PTK2; PIK3CB; PIK3C3; MAPK8; |
| | CAV1; CAPN1; ABL1; MAPK3; ITGA1; KRAS; RHOA; |
| | SRC; PIK3C2A; ITGB7; PPP1CC; ILK; PXN; VASP; |
| | RAF1; FYN; ITGB1; MAP2K2; PAK4; AKT1; PIK3R1; |
| | TNK2; MAP2K1; PAK3; ITGB3; CDC42; RND3; ITGA2; |
| | CRKL; BRAF; GSK3B; AKT3 |
| Acute Phase Response | IRAK1; SOD2; MYD88; TRAF6; ELK1; MAPK1; PTPN11; |
| Signaling | AKT2; IKBKB; PIK3CA; FOS; NFKB2; MAP3K14; |
| | PIK3CB; MAPK8; RIPK1; MAPK3; IL6ST; KRAS; |
| | MAPK13; IL6R; RELA; SOCS1; MAPK9; FTL; NR3C1; |
| | TRAF2; SERPINE1; MAPK14; TNF; RAF1; PDK1; |
| | IKBKG; RELB; MAP3K7; MAP2K2; AKT1; JAK2; PIK3R1; |

(continued)

| CELLULAR FUNCTION | GENES |
|---|---|
| | CHUK; STAT3; MAP2K1; NFKB1; FRAP1; CEBPB; JUN; |
| | AKT3; IL1R1; IL6 |
| PTEN Signaling | ITGAM; ITGA5; RAC1; PTEN; PRKCZ; BCL2L11; |
| | MAPK1; RAC2; AKT2; EGFR; IKBKB; CBL; PIK3CA; |
| | CDKN1B; PTK2; NFKB2; BCL2; PIK3CB; BCL2L1; |
| | MAPK3; ITGA1; KRAS; ITGB7; ILK; PDGFRB; INSR; |
| | RAF1; IKBKG; CASP9; CDKN1A; ITGB1; MAP2K2; |
| | AKT1; PIK3R1; CHUK; PDGFRA; PDPK1; MAP2K1; |
| | NFKB1; ITGB3; CDC42; CCND1; GSK3A; ITGA2; |
| | GSK3B; AKT3; FOXO1; CASP3; RPS6KB1 |
| p53 Signaling | PTEN; EP300; BBC3; PCAF; FASN; BRCA1; GADD45A; |
| | BIRC5; AKT2; PIK3CA; CHEK1; TP53INP1; BCL2; |
| | PIK3CB; PIK3C3; MAPK8; THBS1; ATR; BCL2L1; E2F1; |
| | PMAIP1; CHEK2; TNFRSF10B; TP73; RB1; HDAC9; |
| | CDK2; PIK3C2A; MAPK14; TP53; LRDD; CDKN1A; |
| | HIPK2; AKT1; PIK3R1; RRM2B; APAF1; CTNNB1; |
| | SIRT1; CCND1; PRKDC; ATM; SFN; CDKN2A; JUN; |
| | SNAI2; GSK3B; BAX; AKT3 |
| Aryl Hydrocarbon Receptor | HSPB1; EP300; FASN; TGM2; RXRA; MAPK1; NQO1; |
| Signaling | NCOR2; SP1; ARNT; CDKN1B; FOS; CHEK1; |
| | SMARCA4; NFKB2; MAPK8; ALDH1A1; ATR; E2F1; |
| | MAPK3; NRIP1; CHEK2; RELA; TP73; GSTP1; RB1; |
| | SRC; CDK2; AHR; NFE2L2; NCOA3; TP53; TNF; |
| | CDKN1A; NCOA2; APAF1; NFKB1; CCND1; ATM; ESR1; |
| | CDKN2A; MYC; JUN; ESR2; BAX; IL6; CYP1B1; |
| | HSP90AA1 |
| Xenobiotic Metabolism | PRKCE; EP300; PRKCZ; RXRA; MAPK1; NQO1; |
| Signaling | NCOR2; PIK3CA; ARNT; PRKCI; NFKB2; CAMK2A; |
| | PIK3CB; PPP2R1A; PIK3C3; MAPK8; PRKD1; |
| | ALDH1A1; MAPK3; NRIP1; KRAS; MAPK13; PRKCD; |
| | GSTP1; MAPK9; NOS2A; ABCB1; AHR; PPP2CA; FTL; |
| | NFE2L2; PIK3C2A; PPARGC1A; MAPK14; TNF; RAF1; |
| | CREBBP; MAP2K2; PIK3R1; PPP2R5C; MAP2K1; |
| | NFKB1; KEAP1; PRKCA; EIF2AK3; IL6; CYP1B1; |
| | HSP90AA1 |
| SAPK/JNK Signaling | PRKCE; IRAK1; PRKAA2; EIF2AK2; RAC1; ELK1; |
| | GRK6; MAPK1; GADD45A; RAC2; PLK1; AKT2; PIK3CA; |
| | FADD; CDK8; PIK3CB; PIK3C3; MAPK8; RIPK1; |

(continued)

| CELLULAR FUNCTION | GENES |
|---|---|
| | GNB2L1; IRS1; MAPK3; MAPK10; DAXX; KRAS; |
| | PRKCD; PRKAA1; MAPK9; CDK2; PIM1; PIK3C2A; |
| | TRAF2; TP53; LCK; MAP3K7; DYRK1A; MAP2K2; |
| | PIK3R1; MAP2K1; PAK3; CDC42; JUN; TTK; CSNK1A1; |
| | CRKL; BRAF; SGK |
| PPAr/RXR Signaling | PRKAA2; EP300; INS; SMAD2; TRAF6; PPARA; FASN; |
| | RXRA; MAPK1; SMAD3; GNAS; IKBKB; NCOR2; |
| | ABCA1; GNAQ; NFKB2; MAP3K14; STAT5B; MAPK8; |
| | IRS1; MAPK3; KRAS; RELA; PRKAA1; PPARGC1A; |
| | NCOA3; MAPK14; INSR; RAF1; IKBKG; RELB; MAP3K7; |
| | CREBBP; MAP2K2; JAK2; CHUK; MAP2K1; NFKB1; |
| | TGFBR1; SMAD4; JUN; IL1R1; PRKCA; IL6; HSP90AA1; |
| | ADIPOQ |
| NF-KB Signaling | IRAK1; EIF2AK2; EP300; INS; MYD88; PRKCZ; TRAF6; |
| | TBK1; AKT2; EGFR; IKBKB; PIK3CA; BTRC; NFKB2; |
| | MAP3K14; PIK3CB; PIK3C3; MAPK8; RIPK1; HDAC2; |
| | KRAS; RELA; PIK3C2A; TRAF2; TLR4; PDGFRB; TNF; |
| | INSR; LCK; IKBKG; RELB; MAP3K7; CREBBP; AKT1; |
| | PIK3R1; CHUK; PDGFRA; NFKB1; TLR2; BCL10; |
| | GSK3B; AKT3; TNFAIP3; IL1R1 |
| Neuregulin Signaling | ERBB4; PRKCE; ITGAM; ITGA5; PTEN; PRKCZ; ELK1; |
| | MAPK1; PTPN11; AKT2; EGFR; ERBB2; PRKCI; |
| | CDKN1B; STAT5B; PRKD1; MAPK3; ITGA1; KRAS; |
| | PRKCD; STAT5A; SRC; ITGB7; RAF1; ITGB1; MAP2K2; |
| | ADAM17; AKT1; PIK3R1; PDPK1; MAP2K1; ITGB3; |
| | EREG; FRAP1; PSEN1; ITGA2; MYC; NRG1; CRKL; |
| | AKT3; PRKCA; HSP90AA1; RPS6KB1 |
| Wnt & Beta catenin | CD44; EP300; LRP6; DVL3; CSNK1E; GJA1; SMO; |
| Signaling | AKT2; PIN1; CDH1; BTRC; GNAQ; MARK2; PPP2R1A; |
| | WNT11; SRC; DKK1; PPP2CA; SOX6; SFRP2; ILK; |
| | LEF1; SOX9; TP53; MAP3K7; CREBBP; TCF7L2; AKT1; |
| | PPP2R5C; WNT5A; LRP5; CTNNB1; TGFBR1; CCND1; |
| | GSK3A; DVL1; APC; CDKN2A; MYC; CSNK1A1; GSK3B; |
| | AKT3; SOX2 |
| Insulin Receptor Signaling | PTEN; INS; EIF4E; PTPN1; PRKCZ; MAPK1; TSC1; |
| | PTPN11; AKT2; CBL; PIK3CA; PRKCI; PIK3CB; PIK3C3; |
| | MAPK8; IRS1; MAPK3; TSC2; KRAS; EIF4EBP1; |
| | SLC2A4; PIK3C2A; PPP1CC; INSR; RAF1; FYN; |

(continued)

| CELLULAR FUNCTION | GENES |
|---|---|
| | MAP2K2; JAK1; AKT1; JAK2; PIK3R1; PDPK1; MAP2K1; |
| | GSK3A; FRAP1; CRKL; GSK3B; AKT3; FOXO1; SGK; |
| | RPS6KB1 |
| IL-6 Signaling | HSPB1; TRAF6; MAPKAPK2; ELK1; MAPK1; PTPN11; |
| | IKBKB; FOS; NFKB2; MAP3K14; MAPK8; MAPK3; |
| | MAPK10; IL6ST; KRAS; MAPK13; IL6R; RELA; SOCS1; |
| | MAPK9; ABCB1; TRAF2; MAPK14; TNF; RAF1; IKBKG; |
| | RELB; MAP3K7; MAP2K2; IL8; JAK2; CHUK; STAT3; |
| | MAP2K1; NFKB1; CEBPB; JUN; IL1R1; SRF; IL6 |
| Hepatic Cholestasis | PRKCE; IRAK1; INS; MYD88; PRKCZ; TRAF6; PPARA; |
| | RXRA; IKBKB; PRKCI; NFKB2; MAP3K14; MAPK8; |
| | PRKD1; MAPK10; RELA; PRKCD; MAPK9; ABCB1; |
| | TRAF2; TLR4; TNF; INSR; IKBKG; RELB; MAP3K7; IL8; |
| | CHUK; NR1H2; TJP2; NFKB1; ESR1; SREBF1; FGFR4; |
| | JUN; IL1R1; PRKCA; IL6 |
| IGF-1 Signaling | IGF1; PRKCZ; ELK1; MAPK1; PTPN11; NEDD4; AKT2; |
| | PIK3CA; PRKCI; PTK2; FOS; PIK3CB; PIK3C3; MAPK8; |
| | IGF1R; IRS1; MAPK3; IGFBP7; KRAS; PIK3C2A; |
| | YWHAZ; PXN; RAF1; CASP9; MAP2K2; AKT1; PIK3R1; |
| | PDPK1; MAP2K1; IGFBP2; SFN; JUN; CYR61; AKT3; |
| | FOXO1; SRF; CTGF; RPS6KB1 |
| NRF2-mediated | PRKCE; EP300; SOD2; PRKCZ; MAPK1; SQSTM1; |
| Oxidative | |
| Stress Response | NQO1; PIK3CA; PRKCI; FOS; PIK3CB; PIK3C3; MAPK8; |
| | PRKD1; MAPK3; KRAS; PRKCD; GSTP1; MAPK9; FTL; |
| | NFE2L2; PIK3C2A; MAPK14; RAF1; MAP3K7; CREBBP; |
| | MAP2K2; AKT1; PIK3R1; MAP2K1; PPIB; JUN; KEAP1; |
| | GSK3B; ATF4; PRKCA; EIF2AK3; HSP90AA1 |
| Hepatic Fibrosis/Hepatic | EDN1; IGF1; KDR; FLT1; SMAD2; FGFR1; MET; PGF; |
| Stellate Cell Activation | SMAD3; EGFR; FAS; CSF1; NFKB2; BCL2; MYH9; |
| | IGF1R; IL6R; RELA; TLR4; PDGFRB; TNF; RELB; IL8; |
| | PDGFRA; NFKB1; TGFBR1; SMAD4; VEGFA; BAX; |
| | IL1R1; CCL2; HGF; MMP1; STAT1; IL6; CTGF; MMP9 |
| PPAR Signaling | EP300; INS; TRAF6; PPARA; RXRA; MAPK1; IKBKB; |
| | NCOR2; FOS; NFKB2; MAP3K14; STAT5B; MAPK3; |
| | NRIP1; KRAS; PPARG; RELA; STAT5A; TRAF2; |
| | PPARGC1A; PDGFRB; TNF; INSR; RAF1; IKBKG; |
| | RELB; MAP3K7; CREBBP; MAP2K2; CHUK; PDGFRA; |

(continued)

| CELLULAR FUNCTION | GENES |
|---|---|
| | MAP2K1; NFKB1; JUN; IL1R1; HSP90AA1 |
| Fc Epsilon RI Signaling | PRKCE; RAC1; PRKCZ; LYN; MAPK1; RAC2; PTPN11; |
| | AKT2; PIK3CA; SYK; PRKCI; PIK3CB; PIK3C3; MAPK8; |
| | PRKD1; MAPK3; MAPK10; KRAS; MAPK13; PRKCD; |
| | MAPK9; PIK3C2A; BTK; MAPK14; TNF; RAF1; FYN; |
| | MAP2K2; AKT1; PIK3R1; PDPK1; MAP2K1; AKT3; |
| | VAV3; PRKCA |
| G-Protein Coupled | PRKCE; RAP1A; RGS16; MAPK1; GNAS; AKT2; IKBKB; |
| Receptor Signaling | PIK3CA; CREB1; GNAQ; NFKB2; CAMK2A; PIK3CB; |
| | PIK3C3; MAPK3; KRAS; RELA; SRC; PIK3C2A; RAF1; |
| | IKBKG; RELB; FYN; MAP2K2; AKT1; PIK3R1; CHUK; |
| | PDPK1; STAT3; MAP2K1; NFKB1; BRAF; ATF4; AKT3; |
| | PRKCA |
| Inositol Phosphate | PRKCE; IRAK1; PRKAA2; EIF2AK2; PTEN; GRK6; |
| Metabolism | MAPK1; PLK1; AKT2; PIK3CA; CDK8; PIK3CB; PIK3C3; |
| | MAPK8; MAPK3; PRKCD; PRKAA1; MAPK9; CDK2; |
| | PIM1; PIK3C2A; DYRK1A; MAP2K2; PIP5K1A; PIK3R1; |
| | MAP2K1; PAK3; ATM; TTK; CSNK1A1; BRAF; SGK |
| PDGF Signaling | EIF2AK2; ELK1; ABL2; MAPK1; PIK3CA; FOS; PIK3CB; |
| | PIK3C3; MAPK8; CAV1; ABL1; MAPK3; KRAS; SRC; |
| | PIK3C2A; PDGFRB; RAF1; MAP2K2; JAK1; JAK2; |
| | PIK3R1; PDGFRA; STAT3; SPHK1; MAP2K1; MYC; |
| | JUN; CRKL; PRKCA; SRF; STAT1; SPHK2 |
| VEGF Signaling | ACTN4; ROCK1; KDR; FLT1; ROCK2; MAPK1; PGF; |
| | AKT2; PIK3CA; ARNT; PTK2; BCL2; PIK3CB; PIK3C3; |
| | BCL2L1; MAPK3; KRAS; HIF1A; NOS3; PIK3C2A; PXN; |
| | RAF1; MAP2K2; ELAVL1; AKT1; PIK3R1; MAP2K1; SFN; |
| | VEGFA; AKT3; FOXO1; PRKCA |
| Natural Killer Cell | PRKCE; RAC1; PRKCZ; MAPK1; RAC2; PTPN11; |
| Signaling | |
| | KIR2DL3; AKT2; PIK3CA; SYK; PRKCI; PIK3CB; |
| | PIK3C3; PRKD1; MAPK3; KRAS; PRKCD; PTPN6; |
| | PIK3C2A; LCK; RAF1; FYN; MAP2K2; PAK4; AKT1; |
| | PIK3R1; MAP2K1; PAK3; AKT3; VAV3; PRKCA |
| Cell Cycle: G1/S | HDAC4; SMAD3; SUV39H1; HDAC5; CDKN1B; BTRC; |
| Checkpoint Regulation | ATR; ABL1; E2F1; HDAC2; HDAC7A; RB1; HDAC11; |
| | HDAC9; CDK2; E2F2; HDAC3; TP53; CDKN1A; CCND1; |
| | E2F4; ATM; RBL2; SMAD4; CDKN2A; MYC; NRG1; |

(continued)

| CELLULAR FUNCTION | GENES |
|---|---|
| | GSK3B; RBL1; HDAC6 |
| T Cell Receptor Signaling | RAC1; ELK1; MAPK1; IKBKB; CBL; PIK3CA; FOS; |
| | NFKB2; PIK3CB; PIK3C3; MAPK8; MAPK3; KRAS; |
| | RELA; PIK3C2A; BTK; LCK; RAF1; IKBKG; RELB; FYN; |
| | MAP2K2; PIK3R1; CHUK; MAP2K1; NFKB1; ITK; BCL10; |
| | JUN; VAV3 |
| Death Receptor Signaling | CRADD; HSPB1; BID; BIRC4; TBK1; IKBKB; FADD; |
| | FAS; NFKB2; BCL2; MAP3K14; MAPK8; RIPK1; CASP8; |
| | DAXX; TNFRSF10B; RELA; TRAF2; TNF; IKBKG; RELB; |
| | CASP9; CHUK; APAF1; NFKB1; CASP2; BIRC2; CASP3; |
| | BIRC3 |
| FGF Signaling | RAC1; FGFR1; MET; MAPKAPK2; MAPK1; PTPN11; |
| | AKT2; PIK3CA; CREB1; PIK3CB; PIK3C3; MAPK8; |
| | MAPK3; MAPK13; PTPN6; PIK3C2A; MAPK14; RAF1; |
| | AKT1; PIK3R1; STAT3; MAP2K1; FGFR4; CRKL; ATF4; |
| | AKT3; PRKCA; HGF |
| GM-CSF Signaling | LYN; ELK1; MAPK1; PTPN11; AKT2; PIK3CA; CAMK2A; |
| | STAT5B; PIK3CB; PIK3C3; GNB2L1; BCL2L1; MAPK3; |
| | ETS1; KRAS; RUNX1; PIM1; PIK3C2A; RAF1; MAP2K2; |
| | AKT1; JAK2; PIK3R1; STAT3; MAP2K1; CCND1; AKT3; |
| | STAT1 |
| Amyotrophic Lateral | BID; IGF1; RAC1; BIRC4; PGF; CAPNS1; CAPN2; |
| Sclerosis Signaling | PIK3CA; BCL2; PIK3CB; PIK3C3; BCL2L1; CAPN1; |
| | PIK3C2A; TP53; CASP9; PIK3R1; RAB5A; CASP1; |
| | APAF1; VEGFA; BIRC2; BAX; AKT3; CASP3; BIRC3 |
| JAK/Stat Signaling | PTPN1; MAPK1; PTPN11; AKT2; PIK3CA; STAT5B; |
| | PIK3CB; PIK3C3; MAPK3; KRAS; SOCS1; STAT5A; |
| | PTPN6; PIK3C2A; RAF1; CDKN1A; MAP2K2; JAK1; |
| | AKT1; JAK2; PIK3R1; STAT3; MAP2K1; FRAP1; AKT3; |
| | STAT1 |
| Nicotinate and Nicotinamide | PRKCE; IRAK1; PRKAA2; EIF2AK2; GRK6; MAPK1; |
| Metabolism | PLK1; AKT2; CDK8; MAPK8; MAPK3; PRKCD; PRKAA1; |
| | PBEF1; MAPK9; CDK2; PIM1; DYRK1A; MAP2K2; |
| | MAP2K1; PAK3; NT5E; TTK; CSNK1A1; BRAF; SGK |
| Chemokine Signaling | CXCR4; ROCK2; MAPK1; PTK2; FOS; CFL1; GNAQ; |
| | CAMK2A; CXCL12; MAPK8; MAPK3; KRAS; MAPK13; |
| | RHOA; CCR3; SRC; PPP1CC; MAPK14; NOX1; RAF1; |
| | MAP2K2; MAP2K1; JUN; CCL2; PRKCA |

(continued)

| CELLULAR FUNCTION | GENES |
|---|---|
| IL-2 Signaling | ELK1; MAPK1; PTPN11; AKT2; PIK3CA; SYK; FOS; |
| | STAT5B; PIK3CB; PIK3C3; MAPK8; MAPK3; KRAS; |
| | SOCS1; STAT5A; PIK3C2A; LCK; RAF1; MAP2K2; |
| | JAK1; AKT1; PIK3R1; MAP2K1; JUN; AKT3 |
| Synaptic Long Term | PRKCE; IGF1; PRKCZ; PRDX6; LYN; MAPK1; GNAS; |
| Depression | PRKCI; GNAQ; PPP2R1A; IGF1R; PRKD1; MAPK3; |
| | KRAS; GRN; PRKCD; NOS3; NOS2A; PPP2CA; |
| | YWHAZ; RAF1; MAP2K2; PPP2R5C; MAP2K1; PRKCA |
| Estrogen Receptor | TAF4B; EP300; CARM1; PCAF; MAPK1; NCOR2; |
| Signaling | SMARCA4; MAPK3; NRIP1; KRAS; SRC; NR3C1; |
| | HDAC3; PPARGC1A; RBM9; NCOA3; RAF1; CREBBP; |
| | MAP2K2; NCOA2; MAP2K1; PRKDC; ESR1; ESR2 |
| Protein Ubiquitination | TRAF6; SMURF1; BIRC4; BRCA1; UCHL1; NEDD4; |
| Pathway | CBL; UBE2I; BTRC; HSPA5; USP7; USP10; FBXW7; |
| | USP9X; STUB1; USP22; B2M; BIRC2; PARK2; USP8; |
| | USP1; VHL; HSP90AA1; BIRC3 |
| IL-10 Signaling | TRAF6; CCR1; ELK1; IKBKB; SP1; FOS; NFKB2; |
| | MAP3K14; MAPK8; MAPK13; RELA; MAPK14; TNF; |
| | IKBKG; RELB; MAP3K7; JAK1; CHUK; STAT3; NFKB1; |
| | JUN; IL1R1; IL6 |
| VDR/RXR Activation | PRKCE; EP300; PRKCZ; RXRA; GADD45A; HES1; |
| | NCOR2; SP1; PRKCI; CDKN1B; PRKD1; PRKCD; |
| | RUNX2; KLF4; YY1; NCOA3; CDKN1A; NCOA2; SPP1; |
| | LRP5; CEBPB; FOXO1; PRKCA |
| TGF-beta Signaling | EP300; SMAD2; SMURF1; MAPK1; SMAD3; SMAD1; |
| | FOS; MAPK8; MAPK3; KRAS; MAPK9; RUNX2; |
| | SERPINE1; RAF1; MAP3K7; CREBBP; MAP2K2; |
| | MAP2K1; TGFBR1; SMAD4; JUN; SMAD5 |
| Toll-like Receptor Signaling | IRAK1; EIF2AK2; MYD88; TRAF6; PPARA; ELK1; |
| | IKBKB; FOS; NFKB2; MAP3K14; MAPK8; MAPK13; |
| | RELA; TLR4; MAPK14; IKBKG; RELB; MAP3K7; CHUK; |
| | NFKB1; TLR2; JUN |
| p38 MAPK Signaling | HSPB1; IRAK1; TRAF6; MAPKAPK2; ELK1; FADD; FAS; |
| | CREB1; DDIT3; RPS6KA4; DAXX; MAPK13; TRAF2; |
| | MAPK14; TNF; MAP3K7; TGFBR1; MYC; ATF4; IL1R1; |
| | SRF; STAT1 |
| Neurotrophin/TRK Signaling | NTRK2; MAPK1; PTPN11; PIK3CA; CREB1; FOS; |
| | PIK3CB; PIK3C3; MAPK8; MAPK3; KRAS; PIK3C2A; |

(continued)

| CELLULAR FUNCTION | GENES |
|---|---|
| | RAF1; MAP2K2; AKT1; PIK3R1; PDPK1; MAP2K1; |
| | CDC42; JUN; ATF4 |
| FXR/RXR Activation | INS; PPARA; FASN; RXRA; AKT2; SDC1; MAPK8; |
| | APOB; MAPK10; PPARG; MTTP; MAPK9; PPARGC1A; |
| | TNF; CREBBP; AKT1; SREBF1; FGFR4; AKT3; FOXO1 |
| Synaptic Long Term | PRKCE; RAP1A; EP300; PRKCZ; MAPK1; CREB1; |
| Potentiation | PRKCI; GNAQ; CAMK2A; PRKD1; MAPK3; KRAS; |
| | PRKCD; PPP1CC; RAF1; CREBBP; MAP2K2; MAP2K1; |
| | ATF4; PRKCA |
| Calcium Signaling | RAP1A; EP300; HDAC4; MAPK1; HDAC5; CREB1; |
| | CAMK2A; MYH9; MAPK3; HDAC2; HDAC7A; HDAC11; |
| | HDAC9; HDAC3; CREBBP; CALR; CAMKK2; ATF4; |
| | HDAC6 |
| EGF Signaling | ELK1; MAPK1; EGFR; PIK3CA; FOS; PIK3CB; PIK3C3; |
| | MAPK8; MAPK3; PIK3C2A; RAF1; JAK1; PIK3R1; |
| | STAT3; MAP2K1; JUN; PRKCA; SRF; STAT1 |
| Hypoxia Signaling in the | EDN1; PTEN; EP300; NQO1; UBE2I; CREB1; ARNT; |
| Cardiovascular System | HIF1A; SLC2A4; NOS3; TP53; LDHA; AKT1; ATM; |
| | VEGFA; JUN; ATF4; VHL; HSP90AA1 |
| LPS/IL-1 Mediated Inhibition | IRAK1; MYD88; TRAF6; PPARA; RXRA; ABCA1; |
| of RXR Function | MAPK8; ALDH1A1; GSTP1; MAPK9; ABCB1; TRAF2; |
| | TLR4; TNF; MAP3K7; NR1H2; SREBF1; JUN; IL1R1 |
| LXR/RXR Activation | FASN; RXRA; NCOR2; ABCA1; NFKB2; IRF3; RELA; |
| | NOS2A; TLR4; TNF; RELB; LDLR; NR1H2; NFKB1; |
| | SREBF1; IL1R1; CCL2; IL6; MMP9 |
| Amyloid Processing | PRKCE; CSNK1E; MAPK1; CAPNS1; AKT2; CAPN2; |
| | CAPN1; MAPK3; MAPK13; MAPT; MAPK14; AKT1; |
| | PSEN1; CSNK1A1; GSK3B; AKT3; APP |
| IL-4 Signaling | AKT2; PIK3CA; PIK3CB; PIK3C3; IRS1; KRAS; SOCS1; |
| | PTPN6; NR3C1; PIK3C2A; JAK1; AKT1; JAK2; PIK3R1; |
| | FRAP1; AKT3; RPS6KB1 |
| Cell Cycle: G2/M DNA | EP300; PCAF; BRCA1; GADD45A; PLK1; BTRC; |
| Damage Checkpoint | CHEK1; ATR; CHEK2; YWHAZ; TP53; CDKN1A; |
| Regulation | PRKDC; ATM; SFN; CDKN2A |
| Nitric Oxide Signaling in the | KDR; FLT1; PGF; AKT2; PIK3CA; PIK3CB; PIK3C3; |
| Cardiovascular System | CAV1; PRKCD; NOS3; PIK3C2A; AKT1; PIK3R1; |
| | VEGFA; AKT3; HSP90AA1 |
| Purine Metabolism | NME2; SMARCA4; MYH9; RRM2; ADAR; EIF2AK4; |

(continued)

| CELLULAR FUNCTION | GENES |
|---|---|
| | PKM2; ENTPD1; RAD51; RRM2B; TJP2; RAD51C; |
| | NT5E; POLD1; NME1 |
| cAMP-mediated Signaling | RAP1A; MAPK1; GNAS; CREB1; CAMK2A; MAPK3; |
| | SRC; RAF1; MAP2K2; STAT3; MAP2K1; BRAF; ATF4 |
| Mitochondrial | SOD2; MAPK8; CASP8; MAPK10; MAPK9; CASP9; |
| Dysfunction | |
| | PARK7; PSEN1; PARK2; APP; CASP3 |
| Notch Signaling | HES1; JAG1; NUMB; NOTCH4; ADAM17; NOTCH2; |
| | PSEN1; NOTCH3; NOTCH1; DLL4 |
| Endoplasmic Reticulum | HSPA5; MAPK8; XBP1; TRAF2; ATF6; CASP9; ATF4; |
| Stress Pathway | EIF2AK3; CASP3 |
| Pyrimidine Metabolism | NME2; AICDA; RRM2; EIF2AK4; ENTPD1; RRM2B; |
| | NT5E; POLD1; NME1 |
| Parkinson's Signaling | UCHL1; MAPK8; MAPK13; MAPK14; CASP9; PARK7; |
| | PARK2; CASP3 |
| Cardiac & Beta Adrenergic | GNAS; GNAQ; PPP2R1A; GNB2L1; PPP2CA; PPP1CC; |
| Signaling | PPP2R5C |
| Glycolysis/Gluconeogene sis | HK2; GCK; GPI; ALDH1A1; PKM2; LDHA; HK1 |
| Interferon Signaling | IRF1; SOCS1; JAK1; JAK2; IFITM1; STAT1; IFIT3 |
| Sonic Hedgehog Signaling | ARRB2; SMO; GLI2; DYRK1A; GLI1; GSK3B; DYRK1B |
| Glycerophospholipid | PLD1; GRN; GPAM; YWHAZ; SPHK1; SPHK2 |
| Metabolism | |
| Phospholipid Degradation | PRDX6; PLD1; GRN; YWHAZ; SPHK1; SPHK2 |
| Tryptophan Metabolism | SIAH2; PRMT5; NEDD4; ALDH1A1; CYP1B1; SIAH1 |
| Lysine Degradation | SUV39H1; EHMT2; NSD1; SETD7; PPP2R5C |
| Nucleotide Excision Repair | ERCC5; ERCC4; XPA; XPC; ERCC1 |
| Pathway | |
| Starch and Sucrose | UCHL1; HK2; GCK; GPI; HK1 |
| Metabolism | |
| Aminosugars Metabolism | NQO1; HK2; GCK; HK1 |
| Arachidonic Acid | PRDX6; GRN; YWHAZ; CYP1B1 |
| Metabolism | |
| Circadian Rhythm Signaling | CSNK1E; CREB1; ATF4; NR1D1 |
| Coagulation System | BDKRB1; F2R; SERPINE1; F3 |
| Dopamine Receptor | PPP2R1A; PPP2CA; PPP1CC; PPP2R5C |
| Signaling | |
| Glutathione Metabolism | IDH2; GSTP1; ANPEP; IDH1 |
| Glycerolipid Metabolism | ALDH1A1; GPAM; SPHK1; SPHK2 |

(continued)

| CELLULAR FUNCTION | GENES |
|---|---|
| Linoleic Acid Metabolism | PRDX6; GRN; YWHAZ; CYP1B1 |
| Methionine Metabolism | DNMT1; DNMT3B; AHCY; DNMT3A |
| Pyruvate Metabolism | GLO1; ALDH1A1; PKM2; LDHA |
| Arginine and Proline | ALDH1A1; NOS3; NOS2A |
| Metabolism | |
| Eicosanoid Signaling | PRDX6; GRN; YWHAZ |
| Fructose and Mannose | HK2; GCK; HK1 |
| Metabolism | |
| Galactose Metabolism | HK2; GCK; HK1 |
| Stilbene, Coumarine and | PRDX6; PRDX1; TYR |
| Lignin Biosynthesis | |
| Antigen Presentation | CALR; B2M |
| Pathway | |
| Biosynthesis of Steroids | NQO1; DHCR7 |
| Butanoate Metabolism | ALDH1A1; NLGN1 |
| Citrate Cycle | IDH2; IDH1 |
| Fatty Acid Metabolism | ALDH1A1; CYP1B1 |
| Glycerophospholipid | PRDX6; CHKA |
| Metabolism | |
| Histidine Metabolism | PRMT5; ALDH1A1 |
| Inositol Metabolism | ERO1L; APEX1 |
| Metabolism of Xenobiotics | GSTP1; CYP1B1 |
| by Cytochrome p450 | |
| Methane Metabolism | PRDX6; PRDX1 |
| Phenylalanine Metabolism | PRDX6; PRDX1 |
| Propanoate Metabolism | ALDH1A1; LDHA |
| Selenoamino Acid | PRMT5; AHCY |
| Metabolism | |
| Sphingolipid Metabolism | SPHK1; SPHK2 |
| Aminophosphonate | PRMT5 |
| Metabolism | |
| Androgen and Estrogen | PRMT5 |
| Metabolism | |
| Ascorbate and Aldarate | ALDH1A1 |
| Metabolism | |
| Bile Acid Biosynthesis | ALDH1A1 |
| Cysteine Metabolism | LDHA |
| Fatty Acid Biosynthesis | FASN |

(continued)

| CELLULAR FUNCTION | GENES |
|---|---|
| Glutamate Receptor | GNB2L1 |
| Signaling | |
| NRF2-mediated Oxidative | PRDX1 |
| Stress Response | |
| Pentose Phosphate | GPI |
| Pathway | |
| Pentose and Glucuronate | UCHL1 |
| Interconversions | |
| Retinol Metabolism | ALDH1A1 |
| Riboflavin Metabolism | TYR |
| Tyrosine Metabolism | PRMT5, TYR |
| Ubiquinone Biosynthesis | PRMT5 |
| Valine, Leucine and | ALDH1A1 |
| Isoleucine Degradation | |
| Glycine, Serine and | CHKA |
| Threonine Metabolism | |
| Lysine Degradation | ALDH1A1 |
| Pain/Taste | TRPM5; TRPA1 |
| Pain | TRPM7; TRPC5; TRPC6; TRPC1; Cnr1; cnr2; Grk2; |
| | Trpa1; Pomc; Cgrp; Crf; Pka; Era; Nr2b; TRPM5; Prkaca; |
| | Prkacb; Prkar1a; Prkar2a |
| Mitochondrial Function | AIF; CytC; SMAC (Diablo); Aifm-1; Aifm-2 |
| Developmental Neurology | BMP-4; Chordin (Chrd); Noggin (Nog); WNT (Wnt2; |
| | Wnt2b; Wnt3a; Wnt4; Wnt5a; Wnt6; Wnt7b; Wnt8b; |
| | Wnt9a; Wnt9b; Wnt10a; Wnt10b; Wnt16); beta-catenin; |
| | Dkk-1; Frizzled related proteins; Otx-2; Gbx2; FGF-8; |
| | Reelin; Dab1; unc-86 (Pou4f1 or Brn3a); Numb; Rein |

Embodiments of the invention also relate to methods and compositions related to knocking out genes, amplifying genes and repairing particular mutations associated with DNA repeat instability and neurological disorders (Robert D. Wells, Tetsuo Ashizawa, Genetic Instabilities and Neurological Diseases, Second Edition, Academic Press, Oct 13, 2011 - Medical). Specific aspects of tandem repeat sequences have been found to be responsible for more than twenty human diseases (New insights into repeat instability: role of RNA•DNA hybrids. McIvor EI, Polak U, Napierala M. RNA Biol. 2010 Sep-Oct;7(5):551-8). The CRISPR-Cas system may be harnessed to correct these defects of genomic instability.

A further aspect of the invention relates to utilizing the CRISPR-Cas system for correcting defects in the EMP2A and EMP2B genes that have been identified to be associated with Lafora disease. Lafora disease is an autosomal recessive condition which is characterized by progressive myoclonus epilepsy which may start as epileptic seizures in adolescence. A few cases of the disease may be caused by mutations in genes yet to be identified. The disease causes seizures, muscle spasms, difficulty walking, dementia, and eventually death. There is currently no therapy that has proven effective against disease progression. Other genetic abnormalities associated with epilepsy may also be targeted by the CRISPR-Cas system and the underlying genetics is further described in Genetics of Epilepsy and Genetic Epilepsies, edited by Giuliano Avanzini, Jeffrey L. Noebels, Mariani Foundation Paediatric Neurology:20; 2009).

The methods of US Patent Publication No. 20110158957 assigned to Sangamo BioSciences, Inc. involved in inactivating T cell receptor (TCR) genes may also be modified to the CRISPR Cas system of the present invention. In another example, the methods of US Patent Publication No. 20100311124 assigned to Sangamo BioSciences, Inc. and US Patent Publication No. 20110225664 assigned to Cellectis, which are both involved in inactivating glutamine synthetase gene expression genes may also be modified to the CRISPR Cas system of the present invention.

Several further aspects of the invention relate to correcting defects associated with a wide range of genetic diseases which are further described on the website of the National Institutes of Health under the topic subsection Genetic Disorders (website at health.nih.gov/topic/GeneticDisorders). The genetic brain diseases may include but are not limited to Adrenoleukodystrophy, Agenesis of the Corpus Callosum, Aicardi Syndrome, Alpers' Disease, Alzheimer's Disease, Barth Syndrome, Batten Disease, CADASIL, Cerebellar Degeneration, Fabry's Disease, Gerstmann-Straussler-Scheinker Disease, Huntington's Disease and other Triplet Repeat Disorders, Leigh's Disease, Lesch-Nyhan Syndrome, Menkes Disease, Mitochondrial Myopathies and NINDS Colpocephaly. These diseases are further described on the website of the National Institutes of Health under the subsection Genetic Brain Disorders.

In some embodiments, the condition may be neoplasia. In some embodiments, where the condition is neoplasia, the genes to be targeted are any of those listed in Table A (in this case PTEN and so forth). In some embodiments, the condition may be Age-related Macular Degeneration. In some embodiments, the condition may be a Schizophrenic Disorder. In some embodiments, the condition may be a Trinucleotide Repeat Disorder. In some embodiments, the condition may be Fragile X Syndrome. In some embodiments, the condition may be a Secretase Related Disorder. In some embodiments, the condition may be a Prion - related disorder. In some embodiments, the condition may be ALS. In some embodiments, the condition may be a drug addiction. In some embodiments, the condition may be Autism. In some embodiments, the condition may be Alzheimer's Disease. In some embodiments, the condition may be inflammation. In some embodiments, the condition may be Parkinson's Disease.

For example, US Patent Publication No. 20110023145, describes use of zinc finger nucleases to genetically modify cells, animals and proteins associated with autism spectrum disorders (ASD). Autism spectrum disorders (ASDs) are a group of disorders characterized by qualitative impairment in social interaction and communication, and restricted repetitive and stereotyped patterns of behavior, interests, and activities. The three disorders, autism, Asperger syndrome (AS) and pervasive developmental disorder-not otherwise specified (PDD-NOS) are a continuum of the same disorder with varying degrees of severity, associated intellectual functioning and medical conditions. ASDs are predominantly genetically determined disorders with a heritability of around 90%.

US Patent Publication No. 20110023145 comprises editing of any chromosomal sequences that encode proteins associated with ASD which may be applied to the CRISPR Cas system of the present invention. The proteins associated with ASD are typically selected based on an experimental association of the protein associated with ASD to an incidence or indication of an ASD. For example, the production rate or circulating concentration of a protein associated with ASD may be elevated or depressed in a population having an ASD relative to a population lacking the ASD. Differences in protein levels may be assessed using proteomic techniques including but not limited to Western blot, immunohistochemical staining, enzyme linked immunosorbent assay (ELISA), and mass spectrometry. Alternatively, the proteins associated with ASD may be identified by obtaining gene expression profiles of the genes encoding the proteins using genomic techniques including but not limited to DNA microarray analysis, serial analysis of gene expression (SAGE), and quantitative real-time polymerase chain reaction (Q-PCR).

Non limiting examples of disease states or disorders that may be associated with proteins associated with ASD include autism, Asperger syndrome (AS), pervasive developmental disorder-not otherwise specified (PDD-NOS), Rett's syndrome, tuberous sclerosis, phenylketonuria, Smith-Lemli-Opitz syndrome and fragile X syndrome. By way of non-limiting example, proteins associated with ASD include but are not limited to the following proteins: ATP10C aminophospholipid- MET MET receptor transporting ATPase tyrosine kinase (ATP10C) BZRAP1 MGLUR5 (GRM5) Metabotropic glutamate receptor 5 (MGLUR5) CDH10 Cadherin-10 MGLUR6 (GRM6) Metabotropic glutamate receptor 6 (MGLUR6) CDH9 Cadherin-9 NLGN1 Neuroligin-1 CNTN4 Contactin-4 NLGN2 Neuroligin-2 CNTNAP2 Contactin-associated SEMA5A Neuroligin-3 protein-like 2 (CNTNAP2) DHCR7 7-dehydrocholesterol NLGN4X Neuroligin-4 X- reductase (DHCR7) linked DOC2A Double C2-like domain- NLGN4Y Neuroligin-4 Y- containing protein alpha linked DPP6 Dipeptidyl NLGN5 Neuroligin-5 aminopeptidase-like protein 6 EN2 engrailed 2 (EN2) NRCAM Neuronal cell adhesion molecule (NRCAM) MDGA2 fragile X mental retardation NRXN1 Neurexin-1 1 (MDGA2) FMR2 (AFF2) AF4/FMR2 family member 2 OR4M2 Olfactory receptor (AFF2) 4M2 FOXP2 Forkhead box protein P2 OR4N4 Olfactory receptor (FOXP2) 4N4 FXR1 Fragile X mental OXTR oxytocin receptor retardation, autosomal (OXTR) homolog 1 (FXR1) FXR2 Fragile X mental PAH phenylalanine retardation, autosomal hydroxylase (PAH) homolog 2 (FXR2) GABRA1 Gamma-aminobutyric acid PTEN Phosphatase and receptor subunit alpha-1 tensin homologue (GABRA1) (PTEN) GABRA5 GABAA (.gamma.-aminobutyric PTPRZ1 Receptor-type acid) receptor alpha 5 tyrosine-protein subunit (GABRA5) phosphatase zeta (PTPRZ1) GABRB1 Gamma-aminobutyric acid RELN Reelin receptor subunit beta-1 (GABRB1) GABRB3 GABAA (.gamma.-aminobutyric RPL10 60S ribosomal acid) receptor .beta.3 subunit protein L10 (GABRB3) GABRG1 Gamma-aminobutyric acid SEMA5A Semaphorin-5A receptor subunit gamma-1 (SEMA5A) (GABRG1) HIRIP3 HIRA-interacting protein 3 SEZ6L2 seizure related 6 homolog (mouse)- like 2 HOXA1 Homeobox protein Hox-A1 SHANK3 SH3 and multiple (HOXA1) ankyrin repeat domains 3 (SHANK3) IL6 Interleukin-6 SHBZRAP1 SH3 and multiple ankyrin repeat domains 3 (SHBZRAP1) LAMB1 Laminin subunit beta-1 SLC6A4 Serotonin (LAMB1) transporter (SERT) MAPK3 Mitogen-activated protein TAS2R1 Taste receptor kinase 3 type 2 member 1 TAS2R1 MAZ Myc-associated zinc finger TSC1 Tuberous sclerosis protein protein 1 MDGA2 MAM domain containing TSC2 Tuberous sclerosis glycosylphosphatidylinositol protein 2 anchor 2 (MDGA2) MECP2 Methyl CpG binding UBE3A Ubiquitin protein protein 2 (MECP2) ligase E3A (UBE3A) MECP2 methyl CpG binding WNT2 Wingless-type protein 2 (MECP2) MMTV integration site family, member 2 (WNT2)

The identity of the protein associated with ASD whose chromosomal sequence is edited can and will vary. In preferred embodiments, the proteins associated with ASD whose chromosomal sequence is edited may be the benzodiazapine receptor (peripheral) associated protein 1 (BZRAP1) encoded by the BZRAP1 gene, the AF4/FMR2 family member 2 protein (AFF2) encoded by the AFF2 gene (also termed MFR2), the fragile X mental retardation autosomal homolog 1 protein (FXR1) encoded by the FXR1 gene, the fragile X mental retardation autosomal homolog 2 protein (FXR2) encoded by the FXR2 gene, the MAM domain containing glycosylphosphatidylinositol anchor 2 protein (MDGA2) encoded by the MDGA2 gene, the methyl CpG binding protein 2 (MECP2) encoded by the MECP2 gene, the metabotropic glutamate receptor 5 (MGLUR5) encoded by the MGLUR5-1 gene (also termed GRM5), the neurexin 1 protein encoded by the NRXN1 gene, or the semaphorin-5A protein (SEMA5A) encoded by the SEMA5A gene. In an exemplary embodiment, the genetically modified animal is a rat, and the edited chromosomal sequence encoding the protein associated with ASD is as listed below: BZRAP1 benzodiazapine receptor XM_002727789, (peripheral) associated XM_213427, protein 1 (BZRAP1) XM_002724533, XM_001081125 AFF2 (FMR2) AF4/FMR2 family member 2 XM_219832, (AFF2) XM_001054673 FXR1 Fragile X mental NM_001012179 retardation, autosomal homolog 1 (FXR1) FXR2 Fragile X mental NM_001100647 retardation, autosomal homolog 2 (FXR2) MDGA2 MAM domain containing NM_199269 glycosylphosphatidylinositol anchor 2 (MDGA2) MECP2 Methyl CpG binding NM_022673 protein 2 (MECP2) MGLUR5 Metabotropic glutamate NM_017012 (GRM5) receptor 5 (MGLUR5) NRXN1 Neurexin-1 NM_021767 SEMA5A Semaphorin-5A (SEMA5A) NM_001107659

Exemplary animals or cells may comprise one, two, three, four, five, six, seven, eight, or nine or more inactivated chromosomal sequences encoding a protein associated with ASD, and zero, one, two, three, four, five, six, seven, eight, nine or more chromosomally integrated sequences encoding proteins associated with ASD. The edited or integrated chromosomal sequence may be modified to encode an altered protein associated with ASD. Non-limiting examples of mutations in proteins associated with ASD include the L18Q mutation in neurexin 1 where the leucine at position 18 is replaced with a glutamine, the R451C mutation in neuroligin 3 where the arginine at position 451 is replaced with a cysteine, the R87W mutation in neuroligin 4 where the arginine at position 87 is replaced with a tryptophan, and the I425V mutation in serotonin transporter where the isoleucine at position 425 is replaced with a valine. A number of other mutations and chromosomal rearrangements in ASD-related chromosomal sequences have been associated with ASD and are known in the art. See, for example, Freitag et al. (2010) Eur. Child. Adolesc. Psychiatry 19:169-178, and Bucan et al. (2009) PLoS Genetics 5: e1000536, the disclosure of which is incorporated by reference herein in its entirety.

Examples of proteins associated with Parkinson's disease include but are not limited to α-synuclein, DJ-1, LRRK2, PINK1, Parkin, UCHL1, Synphilin-1, and NURR1.

Examples of addiction-related proteins may include ABAT for example.

Examples of inflammation-related proteins may include the monocyte chemoattractant protein-1 (MCP1) encoded by the Ccr2 gene, the C-C chemokine receptor type 5 (CCR5) encoded by the Ccr5 gene, the IgG receptor IIB (FCGR2b, also termed CD32) encoded by the Fcgr2b gene, or the Fc epsilon R1g (FCER1g) protein encoded by the Fcerlg gene, for example.

Examples of cardiovascular diseases associated proteins may include IL1B (interleukin 1, beta), XDH (xanthine dehydrogenase), TP53 (tumor protein p53), PTGIS (prostaglandin 12 (prostacyclin) synthase), MB (myoglobin), IL4 (interleukin 4), ANGPT1 (angiopoietin 1), ABCG8 (ATP-binding cassette, sub-family G (WHITE), member 8), or CTSK (cathepsin K), for example.

For example, US Patent Publication No. 20110023153, describes use of zinc finger nucleases to genetically modify cells, animals and proteins associated with Alzheimer's Disease. Once modified cells and animals may be further tested using known methods to study the effects of the targeted mutations on the development and/or progression of AD using measures commonly used in the study of AD - such as, without limitation, learning and memory, anxiety, depression, addiction, and sensory motor functions as well as assays that measure behavioral, functional, pathological, metaboloic and biochemical function.

The present disclosure comprises editing of any chromosomal sequences that encode proteins associated with AD. The AD-related proteins are typically selected based on an experimental association of the AD-related protein to an AD disorder. For example, the production rate or circulating concentration of an AD-related protein may be elevated or depressed in a population having an AD disorder relative to a population lacking the AD disorder. Differences in protein levels may be assessed using proteomic techniques including but not limited to Western blot, immunohistochemical staining, enzyme linked immunosorbent assay (ELISA), and mass spectrometry. Alternatively, the AD-related proteins may be identified by obtaining gene expression profiles of the genes encoding the proteins using genomic techniques including but not limited to DNA microarray analysis, serial analysis of gene expression (SAGE), and quantitative real-time polymerase chain reaction (Q-PCR).

Examples of Alzheimer's disease associated proteins may include the very low density lipoprotein receptor protein (VLDLR) encoded by the VLDLR gene, the ubiquitin-like modifier activating enzyme 1 (UBA1) encoded by the UBA1 gene, or the NEDD8-activating enzyme E1 catalytic subunit protein (UBE1C) encoded by the UBA3 gene, for example.

By way of non-limiting example, proteins associated with AD include but are not limited to the proteins listed as follows: Chromosomal Sequence Encoded Protein ALAS2 Delta-aminolevulinate synthase 2 (ALAS2) ABCA1 ATP-binding cassette transporter (ABCA1) ACE Angiotensin I-converting enzyme (ACE) APOE Apolipoprotein E precursor (APOE) APP amyloid precursor protein (APP) AQP1 aquaporin 1 protein (AQP1) BIN1 Myc box-dependent-interacting protein 1 or bridging integrator 1 protein (BIN1) BDNF brain-derived neurotrophic factor (BDNF) BTNL8 Butyrophilin-like protein 8 (BTNL8) C1ORF49 chromosome 1 open reading frame 49 CDH4 Cadherin-4 CHRNB2 Neuronal acetylcholine receptor subunit beta-2 CKLFSF2 CKLF-like MARVEL transmembrane domain- containing protein 2 (CKLFSF2) CLEC4E C-type lectin domain family 4, member e (CLEC4E) CLU clusterin protein (also known as apoplipoprotein J) CR1 Erythrocyte complement receptor 1 (CR1, also known as CD35, C3b/C4b receptor and immune adherence receptor) CR1L Erythrocyte complement receptor 1 (CR1L) CSF3R granulocyte colony-stimulating factor 3 receptor (CSF3R) CST3 Cystatin C or cystatin 3 CYP2C Cytochrome P450 2C DAPK1 Death-associated protein kinase 1 (DAPK1) ESR1 Estrogen receptor 1 FCAR Fc fragment of IgA receptor (FCAR, also known as CD89) FCGR3B Fc fragment of IgG, low affinity IIIb, receptor (FCGR3B or CD16b) FFA2 Free fatty acid receptor 2 (FFA2) FGA Fibrinogen (Factor I) GAB2 GRB2-associated-binding protein 2 (GAB2) GAB2 GRB2-associated-binding protein 2 (GAB2) GALP Galanin-like peptide GAPDHS Glyceraldehyde-3-phosphate dehydrogenase, spermatogenic (GAPDHS) GMPB GMBP HP Haptoglobin (HP) HTR7 5-hydroxytryptamine (serotonin) receptor 7 (adenylate cyclase-coupled) IDE Insulin degrading enzyme IF127 IF127 IFI6 Interferon, alpha-inducible protein 6 (IFI6) IFIT2 Interferon-induced protein with tetratricopeptide repeats 2 (IFIT2) IL1RN interleukin-1 receptor antagonist (IL-1RA) IL8RA Interleukin 8 receptor, alpha (IL8RA or CD181) IL8RB Interleukin 8 receptor, beta (IL8RB) JAG1 Jagged 1 (JAG1) KCNJ15 Potassium inwardly-rectifying channel, subfamily J, member 15 (KCNJ15) LRP6 Low-density lipoprotein receptor-related protein 6 (LRP6) MAPT microtubule-associated protein tau (MAPT) MARK4 MAP/microtubule affinity-regulating kinase 4 (MARK4) MPHOSPH1 M-phase phosphoprotein 1 MTHFR 5,10-methylenetetrahydrofolate reductase MX2 Interferon-induced GTP-binding protein Mx2 NBN Nibrin, also known as NBN NCSTN Nicastrin NIACR2 Niacin receptor 2 (NIACR2, also known as GPR109B) NMNAT3 nicotinamide nucleotide adenylyltransferase 3 NTM Neurotrimin (or HNT) ORM1 Orosmucoid 1 (ORM1) or Alpha-1-acid glycoprotein 1 P2RY13 P2Y purinoceptor 13 (P2RY13) PBEF1 Nicotinamide phosphoribosyltransferase (NAmPRTase or Nampt) also known as pre-B-cell colony-enhancing factor 1 (PBEF1) or visfatin PCK1 Phosphoenolpyruvate carboxykinase PICALM phosphatidylinositol binding clathrin assembly protein (PICALM) PLAU Urokinase-type plasminogen activator (PLAU) PLXNC1 Plexin C1 (PLXNC1) PRNP Prion protein PSEN1 presenilin 1 protein (PSEN1) PSEN2 presenilin 2 protein (PSEN2) PTPRA protein tyrosine phosphatase receptor type A protein (PTPRA) RALGPS2 Ral GEF with PH domain and SH3 binding motif 2 (RALGPS2) RGSL2 regulator of G-protein signaling like 2 (RGSL2) SELENBP1 Selenium binding protein 1 (SELNBP1) SLC25A37 Mitoferrin-1 SORL1 sortilin-related receptor L(DLR class) A repeats-containing protein (SORL1) TF Transferrin TFAM Mitochondrial transcription factor A TNF Tumor necrosis factor TNFRSF10C Tumor necrosis factor receptor superfamily member 10C (TNFRSF10C) TNFSF10 Tumor necrosis factor receptor superfamily, (TRAIL) member 10a (TNFSF10) UBA1 ubiquitin-like modifier activating enzyme 1 (UBA1) UBA3 NEDD8-activating enzyme E1 catalytic subunit protein (UBE1C) UBB ubiquitin B protein (UBB) UBQLN1 Ubiquilin-1 UCHL1 ubiquitin carboxyl-terminal esterase L1 protein (UCHL1) UCHL3 ubiquitin carboxyl-terminal hydrolase isozyme L3 protein (UCHL3) VLDLR very low density lipoprotein receptor protein (VLDLR)

In exemplary embodiments, the proteins associated with AD whose chromosomal sequence is edited may be the very low density lipoprotein receptor protein (VLDLR) encoded by the VLDLR gene, the ubiquitin-like modifier activating enzyme 1 (UBA1) encoded by the UBA1 gene, the NEDD8-activating enzyme E1 catalytic subunit protein (UBE1C) encoded by the UBA3 gene, the aquaporin 1 protein (AQP1) encoded by the AQP1 gene, the ubiquitin carboxyl-terminal esterase L1 protein (UCHL1) encoded by the UCHL1 gene, the ubiquitin carboxyl-terminal hydrolase isozyme L3 protein (UCHL3) encoded by the UCHL3 gene, the ubiquitin B protein (UBB) encoded by the UBB gene, the microtubule-associated protein tau (MAPT) encoded by the MAPT gene, the protein tyrosine phosphatase receptor type A protein (PTPRA) encoded by the PTPRA gene, the phosphatidylinositol binding clathrin assembly protein (PICALM) encoded by the PICALM gene, the clusterin protein (also known as apoplipoprotein J) encoded by the CLU gene, the presenilin 1 protein encoded by the PSEN1 gene, the presenilin 2 protein encoded by the PSEN2 gene, the sortilin-related receptor L(DLR class) A repeats-containing protein (SORL1) protein encoded by the SORL1 gene, the amyloid precursor protein (APP) encoded by the APP gene, the Apolipoprotein E precursor (APOE) encoded by the APOE gene, or the brain-derived neurotrophic factor (BDNF) encoded by the BDNF gene. In an exemplary embodiment, the genetically modified animal is a rat, and the edited chromosomal sequence encoding the protein associated with AD is as as follows: APP amyloid precursor protein (APP) NM_019288 AQP1 aquaporin 1 protein (AQP1) NM_012778 BDNF Brain-derived neurotrophic factor NM_012513 CLU clusterin protein (also known as NM_053021 apoplipoprotein J) MAPT microtubule-associated protein NM_017212 tau (MAPT) PICALM phosphatidylinositol binding NM_053554 clathrin assembly protein (PICALM) PSEN1 presenilin 1 protein (PSEN1) NM_019163 PSEN2 presenilin 2 protein (PSEN2) NM_031087 PTPRA protein tyrosine phosphatase NM_012763 receptor type A protein (PTPRA) SORL1 sortilin-related receptor L(DLR NM_053519, class) A repeats-containing XM_001065506, protein (SORL1) XM_217115 UBA1 ubiquitin-like modifier activating NM_001014080 enzyme 1 (UBA1) UBA3 NEDD8-activating enzyme E1 NM_057205 catalytic subunit protein (UBE1C) UBB ubiquitin B protein (UBB) NM_138895 UCHL1 ubiquitin carboxyl-terminal NM_017237 esterase L1 protein (UCHL1) UCHL3 ubiquitin carboxyl-terminal NM_001110165 hydrolase isozyme L3 protein (UCHL3) VLDLR very low density lipoprotein NM_013155 receptor protein (VLDLR)

The animal or cell may comprise 1, 2, 3, 4, 5, 6, 7, 8, 9,10, 11, 12, 13, 14, 15 or more disrupted chromosomal sequences encoding a protein associated with AD and zero, 1, 2, 3, 4, 5, 6, 7, 8, 9, 10, 11, 12, 13, 14, 15 or more chromosomally integrated sequences encoding a protein associated with AD.

The edited or integrated chromosomal sequence may be modified to encode an altered protein associated with AD. A number of mutations in AD-related chromosomal sequences have been associated with AD. For instance, the V7171 (i.e. valine at position 717 is changed to isoleucine) missense mutation in APP causes familial AD. Multiple mutations in the presenilin-1 protein, such as H163R (i.e. histidine at position 163 is changed to arginine), A246E (i.e. alanine at position 246 is changed to glutamate), L286V (i.e. leucine at position 286 is changed to valine) and C410Y (i.e. cysteine at position 410 is changed to tyrosine) cause familial Alzheimer's type 3. Mutations in the presenilin-2 protein, such as N141 I (i.e. asparagine at position 141 is changed to isoleucine), M239V (i.e. methionine at position 239 is changed to valine), and D439A (i.e. aspartate at position 439 is changed to alanine) cause familial Alzheimer's type 4. Other associations of genetic variants in AD-associated genes and disease are known in the art. See, for example, Waring et al. (2008) Arch. Neurol. 65:329-334, the disclosure of which is incorporated by reference herein in its entirety.

Examples of proteins associated Autism Spectrum Disorder may include the benzodiazapine receptor (peripheral) associated protein 1 (BZRAP1) encoded by the BZRAP1 gene, the AF4/FMR2 family member 2 protein (AFF2) encoded by the AFF2 gene (also termed MFR2), the fragile X mental retardation autosomal homolog 1 protein (FXR1) encoded by the FXR1 gene, or the fragile X mental retardation autosomal homolog 2 protein (FXR2) encoded by the FXR2 gene, for example.

Examples of proteins associated Macular Degeneration may include the ATP-binding cassette, sub-family A (ABC1) member 4 protein (ABCA4) encoded by the ABCR gene, the apolipoprotein E protein (APOE) encoded by the APOE gene, or the chemokine (C-C motif) Ligand 2 protein (CCL2) encoded by the CCL2 gene, for example.

Examples of proteins associated Schizophrenia may include NRG1, ErbB4, CPLX1, TPH1, TPH2, NRXN1, GSK3A, BDNF, DISCI, GSK3B, and combinations thereof.

Examples of proteins involved in tumor suppression may include ATM (ataxia telangiectasia mutated), ATR (ataxia telangiectasia and Rad3 related), EGFR (epidermal growth factor receptor), ERBB2 (v-erb-b2 erythroblastic leukemia viral oncogene homolog 2), ERBB3 (v-erb-b2 erythroblastic leukemia viral oncogene homolog 3), ERBB4 (v-erb-b2 erythroblastic leukemia viral oncogene homolog 4), Notch 1, Notch2, Notch 3, or Notch 4, for example.

Examples of proteins associated with a secretase disorder may include PSENEN (presenilin enhancer 2 homolog (C. elegans)), CTSB (cathepsin B), PSEN1 (presenilin 1), APP (amyloid beta (A4) precursor protein), APH1B (anterior pharynx defective 1 homolog B (C. elegans)), PSEN2 (presenilin 2 (Alzheimer disease 4)), or BACE1 (beta-site APP-cleaving enzyme 1), for example.

For example, US Patent Publication No. 20110023146, describes use of zinc finger nucleases to genetically modify cells, animals and proteins associated with secretase-associated disorders. Secretases are essential for processing pre-proteins into their biologically active forms. Defects in various components of the secretase pathways contribute to many disorders, particularly those with hallmark amyloidogenesis or amyloid plaques, such as Alzheimer's disease (AD).

A secretase disorder and the proteins associated with these disorders are a diverse set of proteins that effect susceptibility for numerous disorders, the presence of the disorder, the severity of the disorder, or any combination thereof. The present disclosure comprises editing of any chromosomal sequences that encode proteins associated with a secretase disorder. The proteins associated with a secretase disorder are typically selected based on an experimental association of the secretase--related proteins with the development of a secretase disorder. For example, the production rate or circulating concentration of a protein associated with a secretase disorder may be elevated or depressed in a population with a secretase disorder relative to a population without a secretase disorder. Differences in protein levels may be assessed using proteomic techniques including but not limited to Western blot, immunohistochemical staining, enzyme linked immunosorbent assay (ELISA), and mass spectrometry. Alternatively, the protein associated with a secretase disorder may be identified by obtaining gene expression profiles of the genes encoding the proteins using genomic techniques including but not limited to DNA microarray analysis, serial analysis of gene expression (SAGE), and quantitative real-time polymerase chain reaction (Q-PCR).

By way of non-limiting example, proteins associated with a secretase disorder include PSENEN (presenilin enhancer 2 homolog (C. elegans)), CTSB (cathepsin B), PSEN1 (presenilin 1), APP (amyloid beta (A4) precursor protein), APH1B (anterior pharynx defective 1 homolog B (C. elegans)), PSEN2 (presenilin 2 (Alzheimer disease 4)), BACE1 (beta-site APP-cleaving enzyme 1), ITM2B (integral membrane protein 2B), CTSD (cathepsin D), NOTCH1 (Notch homolog 1, translocation-associated (Drosophila)), TNF (tumor necrosis factor (TNF superfamily, member 2)), INS (insulin), DYT10 (dystonia 10), ADAM17 (ADAM metallopeptidase domain 17), APOE (apolipoprotein E), ACE (angiotensin I converting enzyme (peptidyl-dipeptidase A) 1), STN (statin), TP53 (tumor protein p53), IL6 (interleukin 6 (interferon, beta 2)), NGFR (nerve growth factor receptor (TNFR superfamily, member 16)), IL1B (interleukin 1, beta), ACHE (acetylcholinesterase (Yt blood group)), CTNNB1 (catenin (cadherin-associated protein), beta 1, 88kDa), IGF1 (insulin-like growth factor 1 (somatomedin C)), IFNG (interferon, gamma), NRG1 (neuregulin 1), CASP3 (caspase 3, apoptosis-related cysteine peptidase), MAPK1 (mitogen-activated protein kinase 1), CDH1 (cadherin 1, type 1, E-cadherin (epithelial)), APBB1 (amyloid beta (A4) precursor protein-binding, family B, member 1 (Fe65)), HMGCR (3-hydroxy-3-methylglutaryl-Coenzyme A reductase), CREB1 (cAMP responsive element binding protein 1), PTGS2 (prostaglandin-endoperoxide synthase 2 (prostaglandin G/H synthase and cyclooxygenase)), HES1 (hairy and enhancer of split 1, (Drosophila)), CAT (catalase), TGFB1 (transforming growth factor, beta 1), ENO2 (enolase 2 (gamma, neuronal)), ERBB4 (v-erb-a erythroblastic leukemia viral oncogene homolog 4 (avian)), TRAPPC10 (trafficking protein particle complex 10), MAOB (monoamine oxidase B), NGF (nerve growth factor (beta polypeptide)), MMP12 (matrix metallopeptidase 12 (macrophage elastase)), JAG1 (jagged 1 (Alagille syndrome)), CD40LG (CD40 ligand), PPARG (peroxisome proliferator-activated receptor gamma), FGF2 (fibroblast growth factor 2 (basic)), IL3 (interleukin 3 (colony-stimulating factor, multiple)), LRP1 (low density lipoprotein receptor-related protein 1), NOTCH4 (Notch homolog 4 (Drosophila)), MAPK8 (mitogen-activated protein kinase 8), PREP (prolyl endopeptidase), NOTCH3 (Notch homolog 3 (Drosophila)), PRNP (prion protein), CTSG (cathepsin G), EGF (epidermal growth factor (beta-urogastrone)), REN (renin), CD44 (CD44 molecule (Indian blood group)), SELP (selectin P (granule membrane protein 140 kDa, antigen CD62)), GHR (growth hormone receptor), ADCYAP1 (adenylate cyclase activating polypeptide 1 (pituitary)), INSR (insulin receptor), GFAP (glial fibrillary acidic protein), MMP3 (matrix metallopeptidase 3 (stromelysin 1, progelatinase)), MAPK10 (mitogen-activated protein kinase 10), SP1 (Sp1 transcription factor), MYC (v-myc myelocytomatosis viral oncogene homolog (avian)), CTSE (cathepsin E), PPARA (peroxisome proliferator-activated receptor alpha), JUN (jun oncogene), TIMP1 (TIMP metallopeptidase inhibitor 1), IL5 (interleukin 5 (colony-stimulating factor, eosinophil)), ILIA (interleukin 1, alpha), MMP9 (matrix metallopeptidase 9 (gelatinase B, 92 kDa gelatinase, 92 kDa type IV collagenase)), HTR4 (5-hydroxytryptamine (serotonin) receptor 4), HSPG2 (heparan sulfate proteoglycan 2), KRAS (v-Ki-ras2 Kirsten rat sarcoma viral oncogene homolog), CYCS (cytochrome c, somatic), SMG1 (SMG1 homolog, phosphatidylinositol 3-kinase-related kinase (C. elegans)), IL1R1 (interleukin 1 receptor, type I), PROK1 (prokineticin 1), MAPK3 (mitogen-activated protein kinase 3), NTRK1 (neurotrophic tyrosine kinase, receptor, type 1), IL13 (interleukin 13), MME (membrane metallo-endopeptidase), TKT (transketolase), CXCR2 (chemokine (C-X-C motif) receptor 2), IGF1R (insulin-like growth factor 1 receptor), RARA (retinoic acid receptor, alpha), CREBBP (CREB binding protein), PTGS1 (prostaglandin-endoperoxide synthase 1 (prostaglandin G/H synthase and cyclooxygenase)), GALT (galactose-1-phosphate uridylyltransferase), CHRM1 (cholinergic receptor, muscarinic 1), ATXN1 (ataxin 1), PAWR (PRKC, apoptosis, WT1, regulator), NOTCH2 (Notch homolog 2 (Drosophila)), M6PR (mannose-6-phosphate receptor (cation dependent)), CYP46A1 (cytochrome P450, family 46, subfamily A, polypeptide 1), CSNK1 D (casein kinase 1, delta), MAPK14 (mitogen-activated protein kinase 14), PRG2 (proteoglycan 2, bone marrow (natural killer cell activator, eosinophil granule major basic protein)), PRKCA (protein kinase C, alpha), L1 CAM (LI cell adhesion molecule), CD40 (CD40 molecule, TNF receptor superfamily member 5), NR1I2 (nuclear receptor subfamily 1, group I, member 2), JAG2 (jagged 2), CTNND1 (catenin (cadherin-associated protein), delta 1), CDH2 (cadherin 2, type 1, N-cadherin (neuronal)), CMA1 (chymase 1, mast cell), SORT1 (sortilin 1), DLK1 (delta-like 1 homolog (Drosophila)), THEM4 (thioesterase superfamily member 4), JUP (junction plakoglobin), CD46 (CD46 molecule, complement regulatory protein), CCL11 (chemokine (C-C motif) ligand 11), CAV3 (caveolin 3), RNASE3 (ribonuclease, RNase A family, 3 (eosinophil cationic protein)), HSPA8 (heat shock 70kDa protein 8), CASP9 (caspase 9, apoptosis-related cysteine peptidase), CYP3A4 (cytochrome P450, family 3, subfamily A, polypeptide 4), CCR3 (chemokine (C-C motif) receptor 3), TFAP2A (transcription factor AP-2 alpha (activating enhancer binding protein 2 alpha)), SCP2 (sterol carrier protein 2), CDK4 (cyclin-dependent kinase 4), HIF1A (hypoxia inducible factor 1, alpha subunit (basic helix-loop-helix transcription factor)), TCF7L2 (transcription factor 7-like 2 (T-cell specific, HMG-box)), IL1R2 (interleukin 1 receptor, type II), B3GALTL (beta 1,3-galactosyltransferase-like), MDM2 (Mdm2 p53 binding protein homolog (mouse)), RELA (v-rel reticuloendotheliosis viral oncogene homolog A (avian)), CASP7 (caspase 7, apoptosis-related cysteine peptidase), IDE (insulin-degrading enzyme), FABP4 (fatty acid binding protein 4, adipocyte), CASK (calcium/calmodulin-dependent serine protein kinase (MAGUK family)), ADCYAP1R1 (adenylate cyclase activating polypeptide 1 (pituitary) receptor type I), ATF4 (activating transcription factor 4 (tax-responsive enhancer element B67)), PDGFA (platelet-derived growth factor alpha polypeptide), C21 or f33 (chromosome 21 open reading frame 33), SCG5 (secretogranin V (7B2 protein)), RNF123 (ring finger protein 123), NFKB1 (nuclear factor of kappa light polypeptide gene enhancer in B-cells 1), ERBB2 (v-erb-b2 erythroblastic leukemia viral oncogene homolog 2, neuro/glioblastoma derived oncogene homolog (avian)), CAV1 (caveolin 1, caveolae protein, 22 kDa), MMP7 (matrix metallopeptidase 7 (matrilysin, uterine)), TGFA (transforming growth factor, alpha), RXRA (retinoid X receptor, alpha), STX1A (syntaxin 1A (brain)), PSMC4 (proteasome (prosome, macropain) 26S subunit, ATPase, 4), P2RY2 (purinergic receptor P2Y, G-protein coupled, 2), TNFRSF21 (tumor necrosis factor receptor superfamily, member 21), DLG1 (discs, large homolog 1 (Drosophila)), NUMBL (numb homolog (Drosophila)-like), SPN (sialophorin), PLSCR1 (phospholipid scramblase 1), UBQLN2 (ubiquilin 2), UBQLN1 (ubiquilin 1), PCSK7 (proprotein convertase subtilisin/kexin type 7), SPON1 (spondin 1, extracellular matrix protein), SILV (silver homolog (mouse)), QPCT (glutaminyl-peptide cyclotransferase), HESS (hairy and enhancer of split 5 (Drosophila)), GCC1 (GRIP and coiled-coil domain containing 1), and any combination thereof.

The genetically modified animal or cell may comprise 1, 2, 3, 4, 5, 6, 7, 8, 9, 10 or more disrupted chromosomal sequences encoding a protein associated with a secretase disorder and zero, 1, 2, 3, 4, 5, 6, 7, 8, 9, 10 or more chromosomally integrated sequences encoding a disrupted protein associated with a secretase disorder.

Examples of proteins associated with Amyotrophic Lateral Sclerosis may include SOD1 (superoxide dismutase 1), ALS2 (amyotrophic lateral sclerosis 2), FUS (fused in sarcoma), TARDBP (TAR DNA binding protein), VAGFA (vascular endothelial growth factor A), VAGFB (vascular endothelial growth factor B), and VAGFC (vascular endothelial growth factor C), and any combination thereof.

For example, US Patent Publication No. 20110023144, describes use of zinc finger nucleases to genetically modify cells, animals and proteins associated with amyotrophyic lateral sclerosis (ALS) disease. ALS is characterized by the gradual steady degeneration of certain nerve cells in the brain cortex, brain stem, and spinal cord involved in voluntary movement.

Motor neuron disorders and the proteins associated with these disorders are a diverse set of proteins that effect susceptibility for developing a motor neuron disorder, the presence of the motor neuron disorder, the severity of the motor neuron disorder or any combination thereof. The present disclosure comprises editing of any chromosomal sequences that encode proteins associated with ALS disease, a specific motor neuron disorder. The proteins associated with ALS are typically selected based on an experimental association of ALS--related proteins to ALS. For example, the production rate or circulating concentration of a protein associated with ALS may be elevated or depressed in a population with ALS relative to a population without ALS. Differences in protein levels may be assessed using proteomic techniques including but not limited to Western blot, immunohistochemical staining, enzyme linked immunosorbent assay (ELISA), and mass spectrometry. Alternatively, the proteins associated with ALS may be identified by obtaining gene expression profiles of the genes encoding the proteins using genomic techniques including but not limited to DNA microarray analysis, serial analysis of gene expression (SAGE), and quantitative real-time polymerase chain reaction (Q-PCR).

By way of non-limiting example, proteins associated with ALS include but are not limited to the following proteins: SOD1 superoxide dismutase 1, ALS3 amyotrophic lateral soluble sclerosis 3 SETX senataxin ALS5 amyotrophic lateral sclerosis 5 FUS fused in sarcoma ALS7 amyotrophic lateral sclerosis 7 ALS2 amyotrophic lateral DPP6 Dipeptidyl-peptidase 6 sclerosis 2 NEFH neurofilament, heavy PTGS1 prostaglandin- polypeptide endoperoxide synthase 1 SLC1A2 solute carrier family 1 TNFRSF10B tumor necrosis factor (glial high affinity receptor superfamily, glutamate transporter), member 10b member 2 PRPH peripherin HSP90AA1 heat shock protein 90 kDa alpha (cytosolic), class A member 1 GRIA2 glutamate receptor, IFNG interferon, gamma ionotropic, AMPA 2 S100B S100 calcium binding FGF2 fibroblast growth factor 2 protein B AOX1 aldehyde oxidase 1 CS citrate synthase TARDBP TAR DNA binding protein TXN thioredoxin RAPH1 Ras association MAP3K5 mitogen-activated protein (RaIGDS/AF-6) and kinase 5 pleckstrin homology domains 1 NBEAL1 neurobeachin-like 1 GPX1 glutathione peroxidase 1 ICA1L islet cell autoantigen RAC1 ras-related C3 botulinum 1.69 kDa-like toxin substrate 1 MAPT microtubule-associated ITPR2 inositol 1,4,5- protein tau triphosphate receptor, type 2 ALS2CR4 amyotrophic lateral GLS glutaminase sclerosis 2 (juvenile) chromosome region, candidate 4 ALS2CR8 amyotrophic lateral CNTFR ciliary neurotrophic factor sclerosis 2 (juvenile) receptor chromosome region, candidate 8 ALS2CR11 amyotrophic lateral FOLH1 folate hydrolase 1 sclerosis 2 (juvenile) chromosome region, candidate 11 FAM117B family with sequence P4HB prolyl 4-hydroxylase, similarity 117, member B beta polypeptide CNTF ciliary neurotrophic factor SQSTM1 sequestosome 1 STRADB STE20-related kinase NAIP NLR family, apoptosis adaptor beta inhibitory protein YWHAQ tyrosine 3- SLC33A1 solute carrier family 33 monooxygenase/tryptoph (acetyl-CoA transporter), an 5-monooxygenase member 1 activation protein, theta polypeptide TRAK2 trafficking protein, FIG. 4 FIG. 4 homolog, SAC1 kinesin binding 2 lipid phosphatase domain containing NIF3L1 NIF3 NGG1 interacting INA internexin neuronal factor 3-like 1 intermediate filament protein, alpha PARD3B par-3 partitioning COX8A cytochrome c oxidase defective 3 homolog B subunit VIIIA CDK15 cyclin-dependent kinase HECW1 HECT, C2 and WW 15 domain containing E3 ubiquitin protein ligase 1 NOS1 nitric oxide synthase 1 MET met proto-oncogene SOD2 superoxide dismutase 2, HSPB1 heat shock 27 kDa mitochondrial protein 1 NEFL neurofilament, light CTSB cathepsin B polypeptide ANG angiogenin, HSPA8 heat shock 70 kDa ribonuclease, RNase A protein 8 family, 5 VAPB VAMP (vesicle- ESR1 estrogen receptor 1 associated membrane protein)-associated protein B and C SNCA synuclein, alpha HGF hepatocyte growth factor CAT catalase ACTB actin, beta NEFM neurofilament, medium TH tyrosine hydroxylase polypeptide BCL2 B-cell CLL/lymphoma 2 FAS Fas (TNF receptor superfamily, member 6) CASP3 caspase 3, apoptosis- CLU clusterin related cysteine peptidase SMN1 survival of motor neuron G6PD glucose-6-phosphate 1, telomeric dehydrogenase BAX BCL2-associated X HSF1 heat shock transcription protein factor 1 RNF19A ring finger protein 19A JUN jun oncogene ALS2CR12 amyotrophic lateral HSPA5 heat shock 70 kDa sclerosis 2 (juvenile) protein 5 chromosome region, candidate 12 MAPK14 mitogen-activated protein IL10 interleukin 10 kinase 14 APEX1 APEX nuclease TXNRD1 thioredoxin reductase 1 (multifunctional DNA repair enzyme) 1 NOS2 nitric oxide synthase 2, TIMP1 TIMP metallopeptidase inducible inhibitor 1 CASP9 caspase 9, apoptosis- XIAP X-linked inhibitor of related cysteine apoptosis peptidase GLG1 golgi glycoprotein 1 EPO erythropoietin VEGFA vascular endothelial ELN elastin growth factor A GDNF glial cell derived NFE2L2 nuclear factor (erythroid- neurotrophic factor derived 2)-like 2 SLC6A3 solute carrier family 6 HSPA4 heat shock 70 kDa (neurotransmitter protein 4 transporter, dopamine), member 3 APOE apolipoprotein E PSMB8 proteasome (prosome, macropain) subunit, beta type, 8 DCTN1 dynactin 1 TIMP3 TIMP metallopeptidase inhibitor 3 KIFAP3 kinesin-associated SLC1A1 solute carrier family 1 protein 3 (neuronal/epithelial high affinity glutamate transporter, system Xag), member 1 SMN2 survival of motor neuron CCNC cyclin C 2, centromeric MPP4 membrane protein, STUB1 STIP1 homology and U- palmitoylated 4 box containing protein 1 ALS2 amyloid beta (A4) PRDX6 peroxiredoxin 6 precursor protein SYP synaptophysin CABIN 1 calcineurin binding protein 1 CASP1 caspase 1, apoptosis- GART phosphoribosylglycinami related cysteine de formyltransferase, peptidase phosphoribosylglycinami de synthetase, phosphoribosylaminoimi dazole synthetase CDK5 cyclin-dependent kinase 5 ATXN3 ataxin 3 RTN4 reticulon 4 C1QB complement component 1, q subcomponent, B chain VEGFC nerve growth factor HTT huntingtin receptor PARK7 Parkinson disease 7 XDH xanthine dehydrogenase GFAP glial fibrillary acidic MAP2 microtubule-associated protein protein 2 CYCS cytochrome c, somatic FCGR3B Fc fragment of IgG, low affinity IIIb, CCS copper chaperone for UBL5 ubiquitin-like 5 superoxide dismutase MMP9 matrix metallopeptidase SLC18A3 solute carrier family 18 9 ((vesicular acetylcholine), member 3 TRPM7 transient receptor HSPB2 heat shock 27 kDa potential cation channel, protein 2 subfamily M, member 7 AKT1 v-akt murine thymoma DERL1 Der1-like domain family, viral oncogene homolog 1 member 1 CCL2 chemokine (C--C motif) NGRN neugrin, neurite ligand 2 outgrowth associated GSR glutathione reductase TPPP3 tubulin polymerization- promoting protein family member 3 APAF1 apoptotic peptidase BTBD10 BTB (POZ) domain activating factor 1 containing 10 GLUD1 glutamate CXCR4 chemokine (C--X--C motif) dehydrogenase 1 receptor 4 SLC1A3 solute carrier family 1 FLT1 fms-related tyrosine (glial high affinity glutamate transporter), member 3 kinase 1 PON1 paraoxonase 1 AR androgen receptor LIF leukemia inhibitory factor ERBB3 v-erb-b2 erythroblastic leukemia viral oncogene homolog 3 LGALS1 lectin, galactoside- CD44 CD44 molecule binding, soluble, 1 TP53 tumor protein p53 TLR3 toll-like receptor 3 GRIA1 glutamate receptor, GAPDH glyceraldehyde-3- ionotropic, AMPA 1 phosphate dehydrogenase GRIK1 glutamate receptor, DES desmin ionotropic, kainate 1 CHAT choline acetyltransferase FLT4 fms-related tyrosine kinase 4 CHMP2B chromatin modifying BAG1 BCL2-associated protein 2B athanogene MT3 metallothionein 3 CHRNA4 cholinergic receptor, nicotinic, alpha 4 GSS glutathione synthetase BAK1 BCL2-antagonist/killer 1 KDR kinase insert domain GSTP1 glutathione S-transferase receptor (a type III pi 1 receptor tyrosine kinase) OGG1 8-oxoguanine DNA IL6 interleukin 6 (interferon, glycosylase beta 2).

The animal or cell may comprise 1, 2, 3, 4, 5, 6, 7, 8, 9, 10 or more disrupted chromosomal sequences encoding a protein associated with ALS and zero, 1, 2, 3, 4, 5, 6, 7, 8, 9, 10 or more chromosomally integrated sequences encoding the disrupted protein associated with ALS. Preferred proteins associated with ALS include SOD1 (superoxide dismutase 1), ALS2 (amyotrophic lateral sclerosis 2), FUS (fused in sarcoma), TARDBP (TAR DNA binding protein), VAGFA (vascular endothelial growth factor A), VAGFB (vascular endothelial growth factor B), and VAGFC (vascular endothelial growth factor C), and any combination thereof.

Examples of proteins associated with prion diseases may include SOD1 (superoxide dismutase 1), ALS2 (amyotrophic lateral sclerosis 2), FUS (fused in sarcoma), TARDBP (TAR DNA binding protein), VAGFA (vascular endothelial growth factor A), VAGFB (vascular endothelial growth factor B), and VAGFC (vascular endothelial growth factor C), and any combination thereof.

Examples of proteins related to neurodegenerative conditions in prion disorders may include A2M (Alpha-2-Macroglobulin), AATF (Apoptosis antagonizing transcription factor), ACPP (Acid phosphatase prostate), ACTA2 (Actin alpha 2 smooth muscle aorta), ADAM22 (ADAM metallopeptidase domain), ADORA3 (Adenosine A3 receptor), or ADRA1D (Alpha-ID adrenergic receptor for Alpha-ID adrenoreceptor), for example.

Examples of proteins associated with Immunodeficiency may include A2M [alpha-2-macroglobulin]; AANAT [arylalkylamine N-acetyltransferase]; ABCA1 [ATP-binding cassette, sub-family A (ABC1), member 1]; ABCA2 [ATP-binding cassette, sub-family A (ABC1), member 2]; or ABCA3 [ATP-binding cassette, sub-family A (ABC1), member 3]; for example.

Examples of proteins associated with Trinucleotide Repeat Disorders include AR (androgen receptor), FMR1 (fragile X mental retardation 1), HTT (huntingtin), or DMPK (dystrophia myotonica-protein kinase), FXN (frataxin), ATXN2 (ataxin 2), for example.

Examples of proteins associated with Neurotransmission Disorders include SST (somatostatin), NOS1 (nitric oxide synthase 1 (neuronal)), ADRA2A (adrenergic, alpha-2A-, receptor), ADRA2C (adrenergic, alpha-2C-, receptor), TACR1 (tachykinin receptor 1), or HTR2c (5-hydroxytryptamine (serotonin) receptor 2C), for example.

Examples of neurodevelopmental-associated sequences include A2BP1 [ataxin 2-binding protein 1], AADAT [aminoadipate aminotransferase], AANAT [arylalkylamine N-acetyltransferase], ABAT [4-aminobutyrate aminotransferase], ABCA1 [ATP-binding cassette, sub-family A (ABC1), member 1], or ABCA13 [ATP-binding cassette, sub-family A (ABC1), member 13], for example.

Further examples of preferred conditions treatable with the present system include may be selected from: Aicardi-Goutières Syndrome; Alexander Disease; Allan-Herndon-Dudley Syndrome; POLG-Related Disorders; Alpha-Mannosidosis (Type II and III); Alström Syndrome; Angelman; Syndrome; Ataxia-Telangiectasia; Neuronal Ceroid-Lipofuscinoses; Beta-Thalassemia; Bilateral Optic Atrophy and (Infantile) Optic Atrophy Type 1; Retinoblastoma (bilateral); Canavan Disease; Cerebrooculofacioskeletal Syndrome 1 [COFS1]; Cerebrotendinous Xanthomatosis; Cornelia de Lange Syndrome; MAPT-Related Disorders; Genetic Prion Diseases; Dravet Syndrome; Early-Onset Familial Alzheimer Disease; Friedreich Ataxia [FRDA]; Fryns Syndrome; Fucosidosis; Fukuyama Congenital Muscular Dystrophy; Galactosialidosis; Gaucher Disease; Organic Acidemias; Hemophagocytic Lymphohistiocytosis; Hutchinson-Gilford Progeria Syndrome; Mucolipidosis II; Infantile Free Sialic Acid Storage Disease; PLA2G6-Associated Neurodegeneration; Jervell and Lange-Nielsen Syndrome; Junctional Epidermolysis Bullosa; Huntington Disease; Krabbe Disease (Infantile); Mitochondrial DNA-Associated Leigh Syndrome and NARP; Lesch-Nyhan Syndrome; LIS1-Associated Lissencephaly; Lowe Syndrome; Maple Syrup Urine Disease; MECP2 Duplication Syndrome; ATP7A-Related Copper Transport Disorders; LAMA2-Related Muscular Dystrophy; Arylsulfatase A Deficiency; Mucopolysaccharidosis Types I, II or III; Peroxisome Biogenesis Disorders, Zellweger Syndrome Spectrum; Neurodegeneration with Brain Iron Accumulation Disorders; Acid Sphingomyelinase Deficiency; Niemann-Pick Disease Type C; Glycine Encephalopathy; ARX-Related Disorders; Urea Cycle Disorders; COL1A1/2-Related Osteogenesis Imperfecta; Mitochondrial DNA Deletion Syndromes; PLP1-Related Disorders; Perry Syndrome; Phelan-McDermid Syndrome; Glycogen Storage Disease Type II (Pompe Disease) (Infantile); MAPT-Related Disorders; MECP2-Related Disorders; Rhizomelic Chondrodysplasia Punctata Type 1; Roberts Syndrome; Sandhoff Disease; Schindler Disease - Type 1; Adenosine Deaminase Deficiency; Smith-Lemli-Opitz Syndrome; Spinal Muscular Atrophy; Infantile-Onset Spinocerebellar Ataxia; Hexosaminidase A Deficiency; Thanatophoric Dysplasia Type 1; Collagen Type VI-Related Disorders; Usher Syndrome Type I; Congenital Muscular Dystrophy; Wolf-Hirschhorn Syndrome; Lysosomal Acid Lipase Deficiency; and Xeroderma Pigmentosum.

As will be apparent, it is envisaged that the present system can be used to target any polynucleotide sequence of interest. Some examples of conditions or diseases that might be usefully treated using the present system are included in the Tables above and examples of genes currently associated with those conditions are also provided there. However, the genes exemplified are not exhaustive.

For example, "wild type StCas9" refers to wild type Cas9 from S thermophilus, the protein sequence of which is given in the SwissProt database under accession number G3ECR1. Similarly, S pyogenes Cas9 is included in SwissProt under accession number Q99ZW2.

The ability to use CRISPR-Cas systems to perform efficient and cost effective gene editing and manipulation will allow the rapid selection and comparison of single and and multiplexed genetic manipulations to transform such genomes for improved production and enhanced traits. The CRISPR-cas system may be expressed in plants with a tobacco mosaic virus-derived system or an Agrobacterium Ti or Ri plasmid. In this regard reference is made to US patents and publications: US Patent No. 6,603,061 - Agrobacterium-Mediated Plant Transformation Method; US Patent No. 7,868,149 - Plant Genome Sequences and Uses Thereof and US 2009/0100536 - Transgenic Plants with Enhanced Agronomic Traits, all the contents and disclosure of each of which are herein incorporated by reference in their entirety. In the practice of the invention, the contents and disclosure of Morrell et al "Crop genomics:advances and applications" Nat Rev Genet. 2011 Dec 29;13(2):85-96 are also herein incorporated by reference in their entirety.

## EXAMPLES

The following examples are given for the purpose of illustrating various embodiments of the invention and are not meant to limit the present invention in any fashion. The present examples, along with the methods described herein are presently representative of preferred embodiments, are exemplary, and are not intended as limitations on the scope of the invention. Changes therein and other uses which are encompassed within the spirit of the invention as defined by the scope of the claims will occur to those skilled in the art.

*Example 1: CRISPR Complex Activity in the Nucleus of a Eukaryotic Cell*

An example type II CRISPR system is the type II CRISPR locus from *Streptococcus pyogenes* SF370, which contains a cluster of four genes Cas9, Cas1, Cas2, and Csn1, as well as two non-coding RNA elements, tracrRNA and a characteristic array of repetitive sequences (direct repeats) interspaced by short stretches of non-repetitive sequences (spacers, about 30bp each). In this system, targeted DNA double-strand break (DSB) is generated in four sequential steps (Fig. 2A). First, two non-coding RNAs, the pre-crRNA array and tracrRNA, are transcribed from the CRISPR locus. Second, tracrRNA hybridizes to the direct repeats of pre-crRNA, which is then processed into mature crRNAs containing individual spacer sequences. Third, the mature crRNA:tracrRNA complex directs Cas9 to the DNA target consisting of the protospacer and the corresponding PAM via heteroduplex formation between the spacer region of the crRNA and the protospacer DNA. Finally, Cas9 mediates cleavage of target DNA upstream of PAM to create a DSB within the protospacer (Fig. 2A). This example describes an example process for adapting this RNA-programmable nuclease system to direct CRISPR complex activity in the nuclei of eukaryotic cells.

Cell culture and transfection

Human embryonic kidney (HEK) cell line HEK 293FT (Life Technologies) was maintained in Dulbecco's modified Eagle's Medium (DMEM) supplemented with 10% fetal bovine serum (HyClone), 2mM GlutaMAX (Life Technologies), 100U/mL penicillin, and 100μg/mL streptomycin at 37°C with 5% $CO_2$ incubation. Mouse neuro2A (N2A) cell line (ATCC) was maintained with DMEM supplemented with 5% fetal bovine serum (HyClone), 2mM GlutaMAX (Life Technologies), 100U/mL penicillin, and 100μg/mL streptomycin at 37°C with 5% $CO_2$.

HEK 293FT or N2A cells were seeded into 24-well plates (Corning) one day prior to transfection at a density of 200,000 cells per well. Cells were transfected using Lipofectamine 2000 (Life Technologies) following the manufacturer's recommended protocol. For each well of a 24-well plate a total of 800ng of plasmids were used.

Surveyor assay and sequencing analysis for genome modification

HEK 293FT or N2A cells were transfected with plasmid DNA as described above. After transfection, the cells were incubated at 37°C for 72 hours before genomic DNA extraction. Genomic DNA was extracted using the QuickExtract DNA extraction kit (Epicentre) following the manufacturer's protocol. Briefly, cells were resuspended in QuickExtract solution and incubated at 65°C for 15 minutes and 98°C for 10 minutes. Extracted genomic DNA was immediately processed or stored at -20°C.

The genomic region surrounding a CRISPR target site for each gene was PCR amplified, and products were purified using QiaQuick Spin Column (Qiagen) following manufacturer's protocol. A total of 400ng of the purified PCR products were mixed with 2μl 10X Taq polymerase PCR buffer (Enzymatics) and ultrapure water to a final volume of 20μl, and subjected to a re-annealing process to enable heteroduplex formation: 95°C for 10min, 95°C to 85°C ramping at - 2°C/s, 85°C to 25°C at - 0.25°C/s, and 25°C hold for 1 minute. After re-annealing, products were treated with Surveyor nuclease and Surveyor enhancer S (Transgenomics) following the manufacturer's recommended protocol, and analyzed on 4-20% Novex TBE poly-acrylamide gels (Life Technologies). Gels were stained with SYBR Gold DNA stain (Life Technologies) for 30 minutes and imaged with a Gel Doc gel imaging system (Bio-rad). Quantification was based on relative band intensities, as a measure of the fraction of cleaved DNA. Fig. 7 provides a schematic illustration of this Surveyor assay.

Restriction fragment length polymorphism assay for detection of homologous recombination.

HEK 293FT and N2A cells were transfected with plasmid DNA, and incubated at 37°C for 72 hours before genomic DNA extraction as described above. The target genomic region was PCR amplified using primers outside the homology arms of the homologous recombination (HR) template. PCR products were separated on a 1% agarose gel and extracted with MinElute GelExtraction Kit (Qiagen). Purified products were digested with HindIII (Fermentas) and analyzed on a 6% Novex TBE poly-acrylamide gel (Life Technologies).

RNA secondary structure prediction and analysis

RNA secondary structure prediction was performed using the online webserver RNAfold developed at Institute for Theoretical Chemistry at the University of Vienna, using the centroid structure prediction algorithm (see e.g. A.R. Gruber et al., 2008, Cell 106(1): 23-24; and PA Carr and GM Church, 2009, Nature Biotechnology 27(12): 1151-62).

RNA purification

HEK 293FT cells were maintained and transfected as stated above. Cells were harvested by trypsinization followed by washing in phosphate buffered saline (PBS). Total cell RNA was extracted with TRI reagent (Sigma) following manufacturer's protocol. Extracted total RNA was quantified using Naonodrop (Thermo Scientific) and normalized to same concentration.

Northern blot analysis of crRNA and tracrRNA expression in mammalian cells

RNAs were mixed with equal volumes of 2X loading buffer (Ambion), heated to 95°C for 5 min, chilled on ice for 1 min, and then loaded onto 8% denaturing polyacrylamide gels (SequaGel, National Diagnostics) after pre-running the gel for at least 30 minutes. The samples were electrophoresed for 1.5 hours at 40W limit. Afterwards, the RNA was transferred to Hybond N+ membrane (GE Healthcare) at 300 mA in a semi-dry transfer apparatus (Bio-rad) at room temperature for 1.5 hours. The RNA was crosslinked to the membrane using autocrosslink button on Stratagene UV Crosslinker the Stratalinker (Stratagene). The membrane was pre-hybridized in ULTRAhyb-Oligo Hybridization Buffer (Ambion) for 30 min with rotation at 42°C, and probes were then added and hybridized overnight. Probes were ordered from IDT and labeled with [gamma-$^{32}$P] ATP (Perkin Elmer) with T4 polynucleotide kinase (New England Biolabs). The membrane was washed once with pre-warmed (42°C) 2xSSC, 0.5% SDS for 1 min followed by two 30 minute washes at 42°C. The membrane was exposed to a phosphor screen for one hour or overnight at room temperature and then scanned with a phosphorimager (Typhoon).

Bacterial CRISPR system construction and evaluation

CRISPR locus elements, including tracrRNA, *Cas9,* and leader were PCR amplified from *Streptococcus pyogenes* SF370 genomic DNA with flanking homology arms for Gibson Assembly. Two *Bsa*I type IIS sites were introduced in between two direct repeats to facilitate easy insertion of spacers (Fig. 8). PCR products were cloned into EcoRV-digested pACYC184 downstream of the tet promoter using Gibson Assembly Master Mix (NEB). Other endogenous CRISPR system elements were omitted, with the exception of the last 50bp of Csn2. Oligos (Integrated DNA Technology) encoding spacers with complimentary overhangs were cloned into the *Bsa*I-digested vector pDC000 (NEB) and then ligated with T7 ligase (Enzymatics) to generate pCRISPR plasmids. Challenge plasmids containing spacers with PAM

expression in mammalian cells (expression constructs illustrated in Fig. 6A, with functionality as determined by results of the Surveyor assay shown in Fig. 6B). Transcription start sites are marked as +1, and transcription terminator and the sequence probed by northern blot are also indicated. Expression of processed tracrRNA was also confirmed by Northern blot. Fig. 6C shows results of a Northern blot analysis of total RNA extracted from 293FT cells transfected with U6 expression constructs carrying long or short tracrRNA, as well as SpCas9 and DR-EMX1(1)-DR. Left and right panels are from 293FT cells transfected without or with SpRNase III, respectively. U6 indicate loading control blotted with a probe targeting human U6 snRNA. Transfection of the short tracrRNA expression construct led to abundant levels of the processed form of tracrRNA (~75bp). Very low amounts of long tracrRNA are detected on the Northern blot.

To promote precise transcriptional initiation, the RNA polymerase III-based U6 promoter was selected to drive the expression of tracrRNA (Fig. 2C). Similarly, a U6 promoter-based construct was developed to express a pre-crRNA array consisting of a single spacer flanked by two direct repeats (DRs, also encompassed by the term "tracr-mate sequences"; Fig. 2C). The initial spacer was designed to target a 33-base-pair (bp) target site (30-bp protospacer plus a 3-bp CRISPR motif (PAM) sequence satisfying the NGG recognition motif of Cas9) in the human *EMX1* locus (Fig. 2C), a key gene in the development of the cerebral cortex.

To test whether heterologous expression of the CRISPR system (SpCas9, SpRNase III, tracrRNA, and pre-crRNA) in mammalian cells can achieve targeted cleavage of mammalian chromosomes, HEK 293FT cells were transfected with combinations of CRISPR components. Since DSBs in mammalian nuclei are partially repaired by the non-homologous end joining (NHEJ) pathway, which leads to the formation of indels, the Surveyor assay was used to detect potential cleavage activity at the target *EMX1* locus (Fig. 7) (see e.g. Guschin *et al.,* 2010, Methods Mol Biol 649: 247). Co-transfection of all four CRISPR components was able to induce up to 5.0% cleavage in the protospacer (see Fig. 2D). Co-transfection of all CRISPR components minus SpRNase III also induced up to 4.7% indel in the protospacer; there may be endogenous mammalian RNases that are capable of assisting with crRNA maturation, such as for example the related Dicer and Drosha enzymes. Removing any of the remaining three components abolished the genome cleavage activity of the CRISPR system (Fig. 2D). Sanger sequencing of amplicons containing the target locus verified the cleavage activity: in 43 sequenced clones, 5 mutated alleles (11.6%) were found. Similar experiments using a variety of guide sequences produced indel percentages as high as 29% (see Figs. 3-6, 10, and 11). These results define a three-component system for efficient CRISPR-mediated genome modification in mammalian cells. To optimize the cleavage efficiency, Applicants also tested whether different isoforms of tracrRNA affected the cleavage efficiency and found that, in this example system, only the short (89-bp) transcript form was able to mediate cleavage of the human *EMX1* genomic locus (Fig. 6B).

Fig. 12 provides an additional Northern blot analysis of crRNA processing in mammalian cells. Fig. 12A illustrates a schematic showing the expression vector for a single spacer flanked by two direct repeats (DR-EMX1(1)-DR). The 30bp spacer targeting the human EMX1 locus protospacer 1 (see Fig. 6) and the direct repeat sequences are shown in the sequence beneath Fig. 12A. The line indicates the region whose reverse-complement sequence was used to generate Northern blot probes for EMX1(1) crRNA detection. Fig. 12B shows a Northern blot analysis of total RNA extracted from 293FT cells transfected with U6 expression constructs carrying DR-EMX1(1)-DR. Left and right panels are from 293FT cells transfected without or with SpRNase III respectively. DR-EMX1(1)-DR was processed into mature crRNAs only in the presence of SpCas9 and short tracrRNA and was not dependent on the presence of SpRNase III. The mature crRNA detected from transfected 293FT total RNA is ~33bp and is shorter than the 39-42bp mature crRNA from *S. pyogenes.* These results demonstrate that a CRISPR system can be transplanted into eukaryotic cells and reprogrammed to facilitate cleavage of endogenous mammalian target polynucleotides.

Fig. 2 illustrates the bacterial CRISPR system described in this example. Fig. 2A illustrates a schematic showing the CRISPR locus 1 from *Streptococcus pyogenes* SF370 and a proposed mechanism of CRISPR-mediated DNA cleavage by this system. Mature crRNA processed from the direct repeat-spacer array directs Cas9 to genomic targets consisting of complimentary protospacers and a protospacer-adjacent motif (PAM). Upon target-spacer base pairing, Cas9 mediates a double-strand break in the target DNA. Fig. 2B illustrates engineering of *S. pyogenes* Cas9 (SpCas9) and RNase III (SpRNase III) with nuclear localization signals (NLSs) to enable import into the mammalian nucleus. Fig. 2C illustrates mammalian expression of SpCas9 and SpRNase III driven by the constitutive EF1a promoter and tracrRNA and pre-crRNA array (DR-Spacer-DR) driven by the RNA Pol3 promoter U6 to promote precise transcription initiation and termination. A protospacer from the human *EMX1* locus with a satisfactory PAM sequence is used as the spacer in the pre-crRNA array. Fig. 2D illustrates surveyor nuclease assay for SpCas9-mediated minor insertions and deletions. SpCas9 was expressed with and without SpRNase III, tracrRNA, and a pre-crRNA array carrying the *EMX1*-target spacer. Fig. 2E illustrates a schematic representation of base pairing between target locus and *EMX1*-targeting crRNA, as well as an example chromatogram showing a micro deletion adjacent to the SpCas9 cleavage site. Fig. 2F illustrates mutated alleles identified from sequencing analysis of 43 clonal amplicons showing a variety of micro insertions and deletions. Dashes indicate deleted bases, and non-aligned or mismatched bases indicate insertions or mutations. Scale bar = 10$\mu$m.

To further simplify the three-component system, a chimeric crRNA-tracrRNA hybrid design was adapted, where a mature crRNA (comprising a guide sequence) may be fused to a partial tracrRNA via a stem-loop to mimic the natural crRNA:tracrRNA duplex. To increase co-delivery efficiency, a bicistronic expression vector was created to drive co-expression of a chimeric RNA and SpCas9 in transfected cells. In parallel, the bicistronic vectors were used to express a pre-crRNA (DR-guide sequence-DR) with SpCas9, to induce processing into crRNA with a separately expressed tracrRNA (compare Fig. 11B top and bottom). Fig. 8 provides schematic illustrations of bicistronic expression vectors for pre-crRNA array (Figure 8A) or chimeric crRNA (represented by the short line downstream of the guide sequence insertion site and upstream of the EF1$\alpha$ promoter in Fig. 8B) with hSpCas9, showing location of various elements and the point of guide sequence insertion. The expanded sequence around the location of the guide sequence insertion site in Fig. 8B also shows a partial DR sequence (GTTTAGAGCTA) and a partial tracrRNA sequence (TAGCAAGTTAAAATAAGGCTAGTCCGTTTTT). Guide sequences can be inserted between BbsI sites using annealed oligonucleotides. Sequence design for the oligonucleotides are shown below the schematic illustrations in Fig. 8, with appropriate ligation adapters indicated. WPRE represents the Woodchuck hepatitis virus post-transcriptional regulatory element. The efficiency of chimeric RNA-mediated cleavage was tested by targeting the same *EMX1* locus described above. Using both Surveyor assay and Sanger sequencing of amplicons, Applicants confirmed that the chimeric RNA design facilitates cleavage of human *EMX1* locus with approximately a 4.7% modification rate (Fig. 3).

Generalizability of CRISPR-mediated cleavage in eukaryotic cells was tested by targeting additional genomic loci in both human and mouse cells by designing chimeric RNA targeting multiple sites in the human *EMX1* and *PVALB,* as well as the mouse *Th* loci. Fig. 13 illustrates the selection of some additional targeted protospacers in human *PVALB* (Fig. 13A) and mouse *Th* (Fig. 13B) loci. Schematics of the gene loci and the location of three protospacers within the last exon of each are provided. The underlined sequences include 30bp of protospacer sequence and 3bp at the 3' end corresponding to the PAM sequences. Protospacers on the sense and anti-sense strands are indicated above and below the DNA sequences, respectively. A modification rate of 6.3% and 0.75% was achieved for the human *PVALB* and mouse *Th* loci respectively, demonstrating the broad applicability of the CRISPR system in modifying different loci across multiple organisms (Fig. 5). While cleavage was only detected with one out of three spacers for each locus using the chimeric constructs, all target sequences were cleaved with efficiency of indel production reaching 27% when using the co-expressed pre-crRNA arrangement (Figs. 6 and 13).

Fig. 11 provides a further illustration that SpCas9 can be reprogrammed to target multiple genomic loci in mammalian cells. Fig. 11A provides a schematic of the human *EMX1* locus showing the location of five protospacers, indicated by the underlined sequences. Fig. 11B provides a schematic of the pre-crRNA/trcrRNA complex showing hybridization between the direct repeat region of the pre-crRNA and tracrRNA (top), and a schematic of a chimeric RNA design comprising a 20bp guide sequence, and tracr mate and tracr sequences consisting of partial direct repeat and tracrRNA sequences hybridized in a hairpin structure (bottom). Results of a Surveyor assay comparing the efficacy of Cas9-mediated cleavage at five protospacers in the human *EMX1* locus is illustrated in Fig. 11C. Each protospacer is targeted using either processed pre-crRNA/tracrRNA complex (crRNA) or chimeric RNA (chiRNA).

Since the secondary structure of RNA can be crucial for intermolecular interactions, a structure prediction algorithm based on minimum free energy and Boltzmann-weighted structure ensemble was used to compare the putative secondary structure of all guide sequences used in the genome targeting experiment (see e.g. Gruber et al., 2008, Nucleic Acids Research, 36: W70). Analysis revealed that in most cases, the effective guide sequences in the chimeric crRNA context were substantially free of secondary structure motifs, whereas the ineffective guide sequences were more likely to form internal secondary structures that could prevent base pairing with the target protospacer DNA. It is thus possible that variability in the spacer secondary structure might impact the efficiency of CRISPR-mediated interference when using a chimeric crRNA.

Further vector designs for SpCas9 are shown in Fig. 19, which illustrates single expression vectors incorporating a U6 promoter linked to an insertion site for a guide oligo, and a Cbh promoter linked to SpCas9 coding sequence. The vector shown in Fig. 19b includes a tracrRNA coding sequence linked to an H1 promoter.

In the bacterial assay, all spacers facilitated efficient CRISPR interference (Fig. 3C). These results suggest that there may be additional factors affecting the efficiency of CRISPR activity in mammalian cells.

To investigate the specificity of CRISPR-mediated cleavage, the effect of single-nucleotide mutations in the guide sequence on protospacer cleavage in the mammalian genome was analyzed using a series of *EMX1*-targeting chimeric crRNAs with single point mutations (Fig. 3A). Fig. 3B illustrates results of a Surveyor nuclease assay comparing the cleavage efficiency of Cas9 when paired with different mutant chimeric RNAs. Single-base mismatch up to 12-bp 5' of the PAM substantially abrogated genomic cleavage by SpCas9, whereas spacers with mutations at farther upstream positions retained activity against the original protospacer target (Fig. 3B). In addition to the PAM, SpCas9 has single-base specificity within the last 12-bp of the spacer. Furthermore, CRISPR is able to mediate genomic cleavage as efficiently as a pair of TALE nucleases (TALEN) targeting the same *EMX1* protospacer. Fig. 3C provides a schematic showing the design of TALENs targeting *EMX1,* and Fig. 3D shows a Surveyor gel comparing the efficiency of TALEN and Cas9 (n=3).

Having established a set of components for achieving CRISPR-mediated gene editing in mammalian cells through the error-prone NHEJ mechanism, the ability of CRISPR to stimulate homologous recombination (HR), a high fidelity gene repair pathway for making precise edits in the genome, was tested. The wild type SpCas9 is able to mediate site-specific DSBs, which can be repaired through both NHEJ and HR. In addition, an aspartate-to-alanine substitution (D10A) in the RuvC I catalytic domain of SpCas9 was engineered to convert the nuclease into a nickase (SpCas9n; illustrated in Fig. 4A) (see e.g. Sapranausaks et al., 2011, Nucleic Acids Resch, 39: 9275; Gasiunas et al., 2012, Proc. Natl. Acad. Sci. USA, 109:E2579), such that nicked genomic DNA undergoes the high-fidelity homology-directed repair (HDR). Surveyor assay confirmed that SpCas9n does not generate indels at the *EMX1* protospacer target. As illustrated in Fig. 4B, co-expression of *EMX1*-targeting chimeric crRNA with SpCas9 produced indels in the target site, whereas co-expression with SpCas9n did not (n=3). Moreover, sequencing of 327 amplicons did not detect any indels induced by SpCas9n. The same locus was selected to test CRISPR-mediated HR by co-transfecting HEK 293FT cells with the chimeric RNA targeting *EMX1*, hSpCas9 or hSpCas9n, as well as a HR template to introduce a pair of restriction sites (*Hind*III and *Nhe*I) near the protospacer. Fig. 4C provides a schematic illustration of the HR strategy, with relative locations of recombination points and primer annealing sequences (arrows). SpCas9 and SpCas9n indeed catalyzed integration of the HR template into the *EMX1* locus. PCR amplification of the target region followed by restriction digest with *Hind*III revealed cleavage products corresponding to expected fragment sizes (arrows in restriction fragment length polymorphism gel analysis shown in Fig. 4D), with SpCas9 and SpCas9n mediating similar levels of HR efficiencies. Applicants further verified HR using Sanger sequencing of genomic amplicons (Fig. 4E). These results demonstrate the utility of CRISPR for facilitating targeted gene insertion in the mammalian genome. Given the 14-bp (12-bp from the spacer and 2-bp from the PAM) target specificity of the wild type SpCas9, the availability of a nickase can significantly reduce the likelihood of off-target modifications, since single strand breaks are not substrates for the error-prone NHEJ pathway.

Expression constructs mimicking the natural architecture of CRISPR loci with arrayed spacers (Fig. 2A) were constructed to test the possibility of multiplexed sequence targeting. Using a single CRISPR array encoding a pair of *EMX1*- and PVALB-targeting spacers, efficient cleavage at both loci was detected (Fig. 4F, showing both a schematic design of the crRNA array and a Surveyor blot showing efficient mediation of cleavage). Targeted deletion of larger genomic regions through concurrent DSBs using spacers against two targets within *EMX1* spaced by 119bp was also tested, and a 1.6% deletion efficacy (3 out of 182 amplicons; Fig. 4G) was detected. This demonstrates that the CRISPR system can mediate multiplexed editing within a single genome.

*Example 2: CRISPR system modifications and alternatives*

The ability to use RNA to program sequence-specific DNA cleavage defines a new class of genome engineering tools for a variety of research and industrial applications. Several aspects of the CRISPR system can be further improved to increase the efficiency and versatility of CRISPR targeting. Optimal Cas9 activity may depend on the availability of free $Mg^{2+}$ at levels higher than that present in the mammalian nucleus (see e.g. Jinek et al., 2012, Science, 337:816), and the preference for an NGG motif immediately downstream of the protospacer restricts the ability to target on average every 12-bp in the human genome (Fig. 9, evaluating both plus and minus strands of human chromosomal sequences). Some of these constraints can be overcome by exploring the diversity of CRISPR loci across the microbial metagenome (see e.g. Makarova et al., 2011, Nat Rev Microbiol, 9:467). Other CRISPR loci may be transplanted into the mammalian cellular milieu by a process similar to that described in Example 1. For example, Fig. 10 illustrates adaptation of the Type II CRISPR system from CRISPR 1 of *Streptococcus thermophilus* LMD-9 for heterologous expression in mammalian cells to achieve CRISPR-mediated genome editing. Fig. 10A provides a Schematic illustration of CRISPR 1 from S. *thermophilus* LMD-9. Figure 10B illustrates the design of an expression system for the *S. thermophilus* CRISPR system. Human codon-optimized *hStCas9* is expressed using a constitutive EF1$\alpha$ promoter. Mature versions of tracrRNA and crRNA are expressed using the U6 promoter to promote precise transcription initiation. Sequences from the mature crRNA and tracrRNA are illustrated. A single base indicated by the lower case "a" in the crRNA sequence is used to remove the polyU sequence, which serves as a RNA polIII transcriptional terminator. Fig. 10C provides a schematic showing guide sequences targeting the human *EMX1* locus. Fig. 10D shows the results of hStCas9-mediated cleavage in the target locus using the Surveyor assay. RNA guide spacers 1 and 2 induced 14% and 6.4%, respectively. Statistical analysis of cleavage activity across biological replica at these two protospacer sites is also provided in Fig. 5. Fig. 14 provides a schematic of additional protospacer and corresponding PAM sequence targets of the *S. thermophilus* CRISPR system in the human *EMX1* locus. Two protospacer sequences are highlighted and their corresponding PAM sequences satisfying NNAGAAW motif are indicated by underlining 3' with respect to the corresponding highlighted sequence. Both protospacers target the anti-sense strand.

*Example 3: Cas9 diversity*

The CRISPR-Cas system is an adaptive immune mechanism against invading exogenous DNA employed by diverse species across bacteria and archaea. The type II CRISPR-Cas9 system consists of a set of genes encoding proteins responsible for the "acquisition" of foreign DNA into the CRISPR locus, as well as a set of genes encoding the "execution" of the DNA cleavage mechanism; these include the DNA nuclease (Cas9), a non-coding transactivating cr-RNA (tracrRNA), and an array of foreign DNA-derived spacers flanked by direct repeats (crRNAs). Upon maturation by Cas9, the tracRNA and crRNA duplex guide the Cas9 nuclease to a target DNA sequence specified by the spacer guide sequences, and mediates double-stranded breaks in the DNA near a short sequence motif in the target DNA that is required for cleavage and specific to each CRISPR-Cas system. The type II CRISPR-Cas systems are found throughout the bacterial kingdom and highly diverse in in Cas9 protein sequence and size, tracrRNA and crRNA direct repeat sequence, genome organization of these elements, and the motif requirement for target cleavage. One species may have multiple distinct CRISPR-Cas systems.

Applicants evaluated 207 putative Cas9s from bacterial species identified based on sequence homology to known Cas9s and structures orthologous to known subdomains, including the HNH endonuclease domain and the RuvC endonuclease domains [information from the Eugene Koonin and Kira Makarova]. Phylogenetic analysis based on the protein sequence conservation of this set revealed five families of Cas9s, including three groups of large Cas9s (~1400 amino acids) and two of small Cas9s (~1100 amino acids) (see Figs. 16 and 17A-F).

Further details of Cas9s and mutations of the Cas9 enzyme to convert into a nickase or DNA binding protein and use of same with altered functionality can be found in U.S. application Serial Nos 61/836,101 and 61/835,936 (Attorney docket 44790.09.2022 and 4790.07.2022 and Broad Reference BI-2013/004E and BI-2013/004F respectively) incorporated herein by reference.

*Example 4: Cas9 orthologs*

Applicants analyzed Cas9 orthologs to identify the relevant PAM sequences and the corresponding chimeric guide RNA. Having an expanded set of PAMs provides broader targeting across the genome and also significantly increases the number of unique target sites and provides potential for identifying novel Cas9s with increased levels of specificity in the genome.

The specificity of Cas9 orthologs can be evaluated by testing the ability of each Cas9 to tolerate mismatches between the guide RNA and its DNA target. For example, the specificity of SpCas9 has been characterized by testing the effect of mutations in the guide RNA on cleavage efficiency. Libraries of guide RNAs were made with single or multiple mismatches between the guide sequence and the target DNA. Based on these findings, target sites for SpCas9 can be selected based on the following guidelines:

To maximize SpCas9 specificity for editing a particular gene, one should choose a target site within the locus of interest such that potential 'off-target' genomic sequences abide by the following four constraints: First and foremost, they should not be followed by a PAM with either 5'-NGG or NAG sequences. Second, their global sequence similarity to the target sequence should be minimized. Third, a maximal number of mismatches should lie within the PAM-proximal region of the off-target site. Finally, a maximal number of mismatches should be consecutive or spaced less than four bases apart.

Similar methods can be used to evaluate the specificity of other Cas9 orthologs and to establish criteria for the selection of specific target sites within the genomes of target species. As mentioned previously phylogenetic analysis based on the protein sequence conservation of this set revealed five families of Cas9s, including three groups of large Cas9s (~1400 amino acids) and two of small Cas9s (~1100 amino acids) (see Figs. 16 and 17A-F). Further details on Cas orthologs can be found in U.S. application Serial Nos 61/836,101 and 61/835,936 (Attorney docket 44790.09.2022 and 4790.07.2022 and Broad Reference BI-2013/004E and BI-2013/004F respectively) incorporated herein by reference.

*Example 5: Methodological improvement to simplify cloning and delivery.*

Rather than encoding the U6-promoter and guide RNA on a plasmid, Applicants amplified the U6 promoter with a DNA oligo to add on the guide RNA. The resulting PCR product may be transfected into cells to drive expression of the guide RNA.

Example primer pair that allows the generation a PCR product consisting of U6-promoter::guideRNA targeting human Emx1 locus:

Forward Primer: AAACTCTAGAgagggcctatttcccatgattc
Reverse Primer (carrying the guide RNA, which is underlined):

acctctagAAAAAAAGCACCGACTCGGTGCCACTTTTTCAAGTTGATAACGGACTAGC
CTTATTTTAACTTGCTATGCTGTTTTGTTTCCAAAACAGCATAGCTCTAAAACCCC
TAGTCATTGGAGGTGACGGTGTTTCGTCCTTTCCACaag

*Example 6: Methodological improvement to improve activity:*

Rather than use pol3 promoters, in particular RNA polymerase III (e.g. U6 or HI promoters), to express guide RNAs in eukaryotic cells, Applicants express the T7 polymerase in eukaryotic cells to drive expression of guide RNAs using the T7 promoter.

One example of this system may involve introduction of three pieces of DNA:

1. expression vector for Cas9
2. expression vector for T7 polymerase
3. expression vector containing guideRNA fused to the T7 promoter

*Example 7: Methodological improvement to reduce toxicity of Cas9: Delivery of Cas9 in the form of mRNA.*

Delivery of Cas9 in the form of mRNA enables transient expression of Cas9 in cells, to reduce toxicity. For example, humanized SpCas9 may be amplified using the following primer pair:

Forward Primer (to add on T7 promoter for in vitro transcription): TAATACGACTCACTATAGGAAGTGCGCCACCATGGCCCCAAAGAAGAAGCGG
Reverse Primer (to add on polyA tail): GGTTTTTTTTTTTTTTTTTTTTTTTTTTTTTTttcttaCTTTTTCTTTTTTGCCTGGCCG
Applicants transfect the Cas9 mRNA into cells with either guide RNA in the form of RNA or DNA cassettes to drive guide RNA expression in eukaryotic cells.

*Example 8: Methodological improvement to reduce toxicity of Cas9: Use of an inducible promoter*

Applicants transiently turn on Cas9 expression only when it is needed for carrying out genome modification. Examples of inducible system include tetracycline inducible promoters (Tet-On or Tet-Off), small molecule two-hybrid transcription activations systems (FKBP, ABA, etc), or light inducible systems (Phytochrome, LOV domains, or cryptochrome).

*Example 9: Improvement of the Cas9 system for in vivo application*

Applicants conducted a Metagenomic search for a Cas9 with small molecular weight. Most Cas9 homologs are fairly large. For example the SpCas9 is around 1368aa long, which is too large to be easily packaged into viral vectors for delivery. A graph representing the length distribution of Cas9 homologs is generated from sequences deposited in GenBank (Fig. 20). Some of the sequences may have been mis-annotated and therefore the exact frequency for each length may not necessarily be accurate. Nevertheless it provides a glimpse at distribution of Cas9 proteins and suggest that there are shorter Cas9 homologs.

Through computational analysis, Applicants found that in the bacterial strain *Campylobacter,* there are two Cas9 proteins with less than 1000 amino acids. The sequence for one Cas9 from *Campylobacter jejuni* is presented below. At this length, CjCas9 can be easily packaged into AAV, lentiviruses, Adenoviruses, and other viral vectors for robust delivery into primary cells and *in vivo* in animal models. In a preferred embodiment of the invention, the Cas9 protein from *S. aureus* is used.

>Campylobacterjejuni Cas9 (CjCas9)

MARILAFDIGISSIGWAFSENDELKDCGVRIFTKVENPKTGESLALPRRLAR SARKRLARRKARLNHLKHLIANEFKLNYEDYQSFDESLAKAYKGSLISPYELRFRALN ELLSKQDFARVILHIAKRRGYDDIKNSDDKEKGAILKAIKQNEEKLANYQSVGEYLYK EYFQKFKENSKEFTNVRNKKESYERCIAQSFLKDELKLIFKKQREFGFSFSKKFEEEVL SVAFYKRALKDFSHLVGNCSFFTDEKRAPKNSPLAFMFVALTRIINLLNNLKNTEGIL YTKDDLNALLNEVLKNGTLTYKQTKKLLGLSDDYEFKGEKGTYFIEFKKYKEFIKAL GEHNLSQDDLNEIAKDITLIKDEIKLKKALAKYDLNQNQIDSLSKLEFKDHLNISFKAL KLVTPLMLEGKKYDEACNELNLKVAINEDKKDFLPAFNETYYKDEVTNPVVLRAIKE YRKVLNALLKKYGKVHKINIELAREVGKNHSQRAKIEKEQNENYKAKKDAELECEK LGLKINSKNILKLRLFKEQKEFCAYSGEKIKISDLQDEKMLEIDHIYPYSRSFDDSYMN KVLVFTKQNQEKLNQTPFEAFGNDSAKWQKIEVLAKNLPTKKQKRILDKNYKDKEQ KNFKDRNLNDTRYIARLVLNYTKDYLDFLPLSDDENTKLNDTQKGSKVHVEAKSGM LTSALRHTWGFSAKDRNNHLHHAIDAVIIAYANNSIVKAFSDFKKEQESNSAELYAK KISELDYKNKRKFFEPFSGFRQKVLDKIDEIFVSKPERKKPSGALHEETFRKEEEFYQS YGGKEGVLKALELGKIRKVNGKIVKNGDMFRVDIFKHKKTNKFYAVPIYTMDFALK VLPNKAVARSKKGEIKDWILMDENYEFCFSLYKDSLILIQTKDMQEPEFVYYNAFTSS TVSLIVSKHDNKFETLSKNQKILFKNANEKEVIAKSIGIQNLKVFEKYIVSALGEVTKA EFRQREDFKK.

The putative tracrRNA element for this CjCas9 is:

TATAATCTCATAAGAAATTTAAAAAGGGACTAAAATAAAGAGTTTGCG GGACTCTGCGGGGTTACAATCCCCTAAAACCGCTTTTAAAATT

The Direct Repeat sequence is:

ATTTTACCATAAAGAAATTTAAAAAGGGACTAAAAC

An example of a chimeric guideRNA for CjCas9 is:

NNNNNNNNNNNNNNNNNNNNGUUUUAGUCCCGAAAGGGACUAAAAU
AAAGAGUUUGCGGGACUCUGCGGGGUUACAAUCCCCUAAAACCGCUUUU

*Example 10: Cas9 optimization*

For enhanced function or to develop new functions, Applicants generate chimeric Cas9 proteins by combining fragments from different Cas9 homologs. For example, two example chimeric Cas9 proteins:
For example, Applicants fused the N-term of St1Cas9 (fragment from this protein is in bold) with C-term of SpCas9 (fragment from this protein is underlined).

>St1(N)Sp(C)Cas9

MSDLVLGLDIGIGSVGVGILNKVTGEIIHKNSRIFPAAQAENNLVRRTN
RQGRRLARRKKHRRVRLNRLFEESGLITDFTKISINLNPYQLRVKGLTDELSNEE
LFIALKNMVKHRGISYLDDASDDGNSSVGDYAQIVKENSKQLETKTPGQIQLERY
QTYGQLRGDFTVEKDGKKHRLINVFPTSAYRSEALRILQTQQEFNPQITDEFINR
YLEILTGKRKYYHGPGNEKSRTDYGRYRTSGETLDNIFGILIGKCTFYPDEFRAAK
ASYTAQEFNLLNDLNNLTVPTETKKLSKEQKNQIINYVKNEKAMGPAKLFKYIAK
LLSCDVADIKGYRIDKSGKAEIHTFEAYRKMKTLETLDIEQMDRETLDKLAYVLT
LNTEREGIQEALEHEFADGSFSQKQVDELVQFRKANSSIFGKGWHNFSVKLMME
LIPELYETSEEQMTILTRLGKQKTTSSSNKTKYIDEKLLTEEIYNPVVAKSVRQAIK
IVNAAIKEYGDFDNIVIEMARENQTTQKGQKNSRERMKRIEEGIKELGSQILKEHPVE
NTQLQNEKLYLYYLQNGRDMYVDQELDINRLSDYDVDHIVPQSFLKDDSIDNKVLT
RSDKNRGKSDNVPSEEVVKKMKNYWRQLLNAKLITQRKFDNLTKAERGGLSELDK
AGFIKRQLVETRQITKHVAQILDSRMNTKYDENDKLIREVKVITLKSKLVSDFRKDFQ
FYKVREINNYHHAHDAYLNAVVGTALIKKYPKLESEFVYGDYKVYDVRKMIAKSEQ
EIGKATAKYFFYSNIMNFFKTEITLANGEIRKRPLIETNGETGEIVWDKGRDFATVRKV
LSMPQVNIVKKTEVQTGGFSKESILPKRNSDKLIARKKDWDPKKYGGFDSPTVAYSV
LVVAKVEKGKSKKLKSVKELLGITIMERSSFEKNPIDFLEAKGYKEVKKDLIIKLPKYS
LFELENGRKRMLASAGELQKGNELALPSKYVNFLYLASHYEKLKGSPEDNEQKQLF
VEQHKHYLDEIIEQISEFSKRVILADANLDKVLSAYNKHRDKPIREQAENIIHLFTLTNL
GAPAAFKYFDTTIDRKRYTSTKEVLDATLIHQSITGLYETRIDLSQLGGD

>Sp(N)St1(C)Cas9

MDKKYSIGLDIGTNSVGWAVITDEYKVPSKKFKVLGNTDRHSIKKNLIGA LLFDSGETAEATRLKRTARRRYTRRKNRICYLQEIFSNEMAKVDDSFFHRLEESFLVE EDKKHERHPIFGNIVDEVAYHEKYPTIYHLRKKLVDSTDKADLRLIYLALAHMIKFRG HFLIEGDLNPDNSDVDKLFIQLVQTYNQLFEENPINASGVDAKAILSARLSKSRRLENL IAQLPGEKKNGLFGNLIALSLGLTPNFKSNFDLAEDAKLQLSKDTYDDDLDNLLAQIG DQYADLFLAAKNLSDAILLSDILRVNTEITKAPLSASMIKRYDEHHQDLTLLKALVRQ QLPEKYKEIFFDQSKNGYAGYIDGGASQEEFYKFIKPILEKMDGTEELLVKLNREDLL

RKQRTFDNGSIPHQIHLGELHAILRRQEDFYPFLKDNREKIEKILTFRIPYYVGPLARGN SRFAWMTRKSEETITPWNFEEVVDKGASAQSFIERMTNFDKNLPNEKVLPKHSLLYE YFTVYNELTKVKYVTEGMRKPAFLSGEQKKAIVDLLFKTNRKVTVKQLKEDYFKKIE CFDSVEISGVEDRFNASLGTYHDLLKIIKDKDFLDNEENEDILEDIVLTLTLFEDREMIE ERLKTYAHLFDDKVMKQLKRRRYTGWGRLSRKLINGIRDKQSGKTILDFLKSDGFAN RNFMQLIHDDSLTFKEDIQKAQVSGQGDSLHEHIANLAGSPAIKKGILQTVKVVDELV KVMGRHKPENIVIEMARE**TNEDDEKKAIQKIQKANKDEKDAAMLKAANQYNGKA ELPHSVFHGHKQLATKIRLWHQQGERCLYTGKTISIHDLINNSNQFEVDHILPLSI TFDDSLANKVLVYATANQEKGQRTPYQALDSMDDAWSFRELKAFVRESKTLSNK KKEYLLTEEDISKFDVRKKFIERNLVDTRYASRVVLNALQEHFRAHKIDTKVSVVR GQFTSQLRRHWGIEKTRDTYHHHAVDALIIAASSQLNLWKKQKNTLVSYSEDQL LDIETGELISDDEYKESVFKAPYQHFVDTLKSKEFEDSILFSYQVDSKFNRKISDATI YATRQAKVGKDKADETYVLGKIKDIYTQDGYDAFMKIYKKDKSKFLMYRHDPQT FEKVIEPILENYPNKQINEKGKEVPCNPFLKYKEEHGYIRKYSKKGNGPEIKSLKYY DSKLGNHIDITPKDSNNKVVLQSVSPWRADVYFNKTTGKYEILGLKYADLQFEKG TGTYKISQEKYNDIKKKEGVDSDSEFKFTLYKNDLLLVKDTETKEQQLFRFLSRT MPKQKHYVELKPYDKQKFEGGEALIKVLGNVANSGQCKKGLGKSNISIYKVRTD VLGNQHIIKNEGDKPKLDF**

The benefit of making chimeric Cas9 include:

reduce toxicity
improve expression in eukaryotic cells
enhance specificity
reduce molecular weight of protein, make protein smaller by combining the smallest domains from different Cas9 homologs.

Altering the PAM sequence requirement

*Example 11: Utilization of Cas9 as a generic DNA binding protein*

Applicants used Cas9 as a generic DNA binding protein by mutating the two catalytic domains (D10 and H840) responsible for cleaving both strands of the DNA target. In order to upregulate gene transcription at a target locus Applicants fused the transcriptional activation domain (VP64) to Cas9. Applicants hypothesized that it would be important to see strong nuclear localization of the Cas9-VP64 fusion protein because transcription factor activation strength is a function of time spent at the target. Therefore, Applicants cloned a set of Cas9-VP64-GFP constructs, transfected them into 293 cells and assessed their localization under a fluorescent microscope 12 hours post-transfection.

The same constructs were cloned as a 2A-GFP rather than a direct fusion in order to functionally test the constructs without a bulky GFP present to interfere. Applicants elected to target the Sox2 locus with the Cas9 transactivator because it could be useful for cellular reprogram and the locus has already been validated as a target for TALE-TF mediated transcriptional activation. For the Sox2 locus Applicants chose eight targets near the transcriptional start site (TSS). Each target was 20bp long with a neighboring NGG protospacer adjacent motif (PAM). Each Cas9-VP64 construct was co-transfected with each PCR generated chimeric crispr RNA (chiRNA) in 293 cells. 72 hours post transfection the transcriptional activation was assessed using RT-qPCR.

To further optimize the transcriptional activator, Applicants titrated the ratio of chiRNA (Sox2.1 and Sox2.5) to Cas9 (NLS-VP64-NLS-hSpCas9-NLS-VP64-NLS), transfected into 293 cells, and quantified using RT-qPCR. These results indicate that Cas9 can be used as a generic DNA binding domain to upregulate gene transcription at a target locus.

Applicants designed a second generation of constructs. (Table below).

| |
|---|
| pLenti-EF 1a-GFP-2A-6xHis-NLS-VP64-NLS-hSpCsn1(D10A, H840A)-NLS |
| pLenti-EF 1a-GFP-2A-6xHis-NLS-VP64-NLS-hSpCsn1(D10A, H840A) |
| pLenti-EF 1a-GFP-2A-6xHis-NLS-VP64-NLS-NLS-hSpCsn1(D10A, H840A) |
| pLenti-EF1a-GFP-2A-6xHis-NLS-hSpCsn1(D10A, H840A)-NLS |
| pLenti-EF1a-GFP-2A-6xHis-NLS-hSpCsn1(D10A, H840A) |
| pLenti-EF1a-GFP-2A-6xHis-NLS-NLS-hSpCsn1(D10A, H840A) |

Applicants use these constructs to assess transcriptional activation (VP64 fused constructs) and repression (Cas9 only) by RT-qPCR. Applicants assess the cellular localization of each construct using anti-His antibody, nuclease activity using a Surveyor nuclease assay, and DNA binding affinity using a gel shift assay. In a preferred embodiment of the invention, the gel shift assay is an EMSA gel shift assay.

*Example 12: Cas9 transgenic and knock in mice*

To generate a mouse that expresses the Cas9 nuclease Applicants submit two general strategies, transgenic and knock in. These strategies may be applied to generate any other model organism of interest, for e.g. Rat. For each of the general strategies Applicants made a constitutively active Cas9 and a Cas9 that is conditionally expressed (Cre recombinase dependent). The constitutively active Cas9 nuclease is expressed in the following context: pCAG-NLS-Cas9-NLS-P2A-EGFP-WPRE-bGHpolyA. pCAG is the promoter, NLS is a nuclear localization signal, P2A is the peptide cleavage sequence, EGFP is enhanced green fluorescent protein, WPRE is the woodchuck hepatitis virus posttranscriptional regulatory element, and bGHpolyA is the bovine growth hormone poly-A signal sequence (Figs. 22A-B). The conditional version has one additional stop cassette element, loxP-SV40 polyA x3-loxP, after the promoter and before NLS-Cas9-NLS (i.e. pCAG-loxP-SV40polyAx3-loxP-NLS-Cas9-NLS-P2A-EGFP-WPRE-bGHpolyA). The important expression elements can be visualized as in Fig. 23. The constitutive construct should be expressed in all cell types throughout development, whereas, the conditional construct will only allow Cas9 expression when the same cell is expressing the Cre recombinase. This latter version will allow for tissue specific expression of Cas9 when Cre is under the expression of a tissue specific promoter. Moreover, Cas9 expression could be induced in adult mice by putting Cre under the expression of an inducible promoter such as the TET on or off system.

Validation of Cas9 constructs: Each plasmid was functionally validated in three ways: 1) transient transfection in 293 cells followed by confirmation of GFP expression; 2) transient transfection in 293 cells followed by immunofluorescence using an antibody recognizing the P2A sequence; and 3) transient transfection followed by Surveyor nuclease assay. The 293 cells may be 293FT or 293 T cells depending on the cells that are of interest. In a preferred embodiment the cells are 293FT cells. The results of the Surveyor were run out on the top and bottom row of the gel for the conditional and constitutive constructs, respectively. Each was tested in the presence and absence of chimeric RNA targeted to the hEMX1 locus (chimeric RNA hEMX1.1). The results indicate that the construct can successfully target the hEMX1 locus only in the presence of chimeric RNA (and Cre in the conditional case). The gel was quantified and the results are presented as average cutting efficiency and standard deviation for three samples.

Transgenic Cas9 mouse: To generate transgenic mice with constructs, Applicants inject pure, linear DNA into the pronucleus of a zygote from a pseudo pregnant CB56 female. Founders are identified, genotyped, and backcrossed to CB57 mice. The constructs were successfully cloned and verified by Sanger sequencing.

Knock in Cas9 mouse: To generate Cas9 knock in mice Applicants target the same constitutive and conditional constructs to the Rosa26 locus. Applicants did this by cloning each into a Rosa26 targeting vector with the following elements: Rosa26 short homology arm - constitutive/conditional Cas9 expression cassette - pPGK-Neo-Rosa26 long homology arm - pPGK-DTA. pPGK is the promoter for the positive selection marker Neo, which confers resistance to neomycin, a 1 kb short arm, a 4.3 kb long arm, and a negative selection diphtheria toxin (DTA) driven by PGK.

The two constructs were electroporated into R1 mESCs and allowed to grow for 2 days before neomycin selection was applied. Individual colonies that had survived by days 5-7 were picked and grown in individual wells. 5-7 days later the colonies were harvested, half were frozen and the other half were used for genotyping. Genotyping was done by genomic PCR, where one primer annealed within the donor plasmid (AttpF) and the other outside of the short homology arm (Rosa26-R) Of the 22 colonies harvested for the conditional case, 7 were positive (Left). Of the 27 colonies harvested for the constitutive case, zero were positive (Right). It is likely that Cas9 causes some level of toxicity in the mESC and for this reason there were no positive clones. To test this Applicants introduced a Cre expression plasmid into correctly targeted conditional Cas9 cells and found very low toxicity after many days in culture. The reduced copy number of Cas9 in correctly targeted conditional Cas9 cells (1-2 copies per cell) is enough to allow stable expression and relatively no cytotoxicity. Moreover, this data indicates that the Cas9 copy number determines toxicity. After electroporation each cell should get several copies of Cas9 and this is likely why no positive colonies were found in the case of the constitutive Cas9 construct. This provides strong evidence that utilizing a conditional, Cre-dependent strategy should show reduced toxicity. Applicants inject correctly targeted cells into a blastocyst and implant into a female mouse. Chimerics are identified and backcrossed. Founders are identified and genotyped.

Utility of the conditional Cas9 mouse: Applicants have shown in 293 cells that the Cas9 conditional expression construct can be activated by co-expression with Cre. Applicants also show that the correctly targeted R1 mESCs can have active Cas9 when Cre is expressed. Because Cas9 is followed by the P2A peptide cleavage sequence and then EGFP Applicants identify successful expression by observing EGFP. This same concept is what makes the conditional Cas9 mouse so useful. Applicants may cross their conditional Cas9 mouse with a mouse that ubiquitously expresses Cre (ACTB-Cre line) and may arrive at a mouse that expresses Cas9 in every cell. It should only take the delivery of chimeric RNA to induce genome editing in embryonic or adult mice. Interestingly, if the conditional Cas9 mouse is crossed with a mouse expressing Cre under a tissue specific promoter, there should only be Cas9 in the tissues that also express Cre. This approach may be used to edit the genome in only precise tissues by delivering chimeric RNA to the same tissue.

*Example 13: Cas9 diversity and chimeric RNAs*

The CRISPR-Cas system is an adaptive immune mechanism against invading exogenous DNA employed by diverse species across bacteria and archaea. The type II CRISPR-Cas system consists of a set of genes encoding proteins responsible for the "acquisition" of foreign DNA into the CRISPR locus, as well as a set of genes encoding the "execution" of the DNA cleavage mechanism; these include the DNA nuclease (Cas9), a non-coding transactivating cr-RNA (tracrRNA), and an array of foreign DNA-derived spacers flanked by direct repeats (crRNAs). Upon maturation by Cas9, the tracrRNA and crRNA duplex guide the Cas9 nuclease to a target DNA sequence specified by the spacer guide sequences, and mediates double-stranded breaks in the DNA near a short sequence motif in the target DNA that is required for cleavage and specific to each CRISPR-Cas system. The type II CRISPR-Cas systems are found throughout the bacterial kingdom and highly diverse in in Cas9 protein sequence and size, tracrRNA and crRNA direct repeat sequence, genome organization of these elements, and the motif requirement for target cleavage. One species may have multiple distinct CRISPR-Cas systems.

Applicants evaluated 207 putative Cas9s from bacterial species identified based on sequence homology to known Cas9s and structures orthologous to known subdomains, including the HNH endonuclease domain and the RuvC endonuclease domains [information from the Eugene Koonin and Kira Makarova]. Phylogenetic analysis based on the protein sequence conservation of this set revealed five families of Cas9s, including three groups of large Cas9s (~1400 amino acids) and two of small Cas9s (~1100 amino acids) (Figs.16A-D and 17A-F).

Applicants have also optimized Cas9 guide RNA using *in vitro* methods.

*Example 14: Cas9 mutations*

In this example, Applicants show that the following mutations can convert SpCas9 into a nicking enzyme: D10A, E762A, H840A, N854A, N863A, D986A.

Applicants provide sequences showing where the mutation points are located within the SpCas9 gene (Fig. 21A-M). Applicants also show that the nickases are still able to mediate homologous recombination. Furthermore, Applicants show that SpCas9 with these mutations (individually) do not induce double strand break.

Cas9 orthologs all share the general organization of 3-4 RuvC domains and a HNH domain. The 5' most RuvC domain cleaves the non-complementary strand, and the HNH domain cleaves the complementary strand. All notations are in reference to the guide sequence.

The catalytic residue in the 5' RuvC domain is identified through homology comparison of the Cas9 of interest with other Cas9 orthologs (from S. pyogenes type II CRISPR locus, S. thermophilus CRISPR locus 1, S. thermophilus CRISPR locus 3, and Franciscilla novicida type II CRISPR locus), and the conserved Asp residue is mutated to alanine to convert Cas9 into a complementary-strand nicking enzyme. Similarly, the conserved His and Asn residues in the HNH domains are mutated to Alanine to convert Cas9 into a non-complementary-strand nicking enzyme.

*Example 15: Cas9 Transcriptional Activation and Cas9 Repressor*

Cas9 Transcriptional Activation

A second generation of constructs were designed and tested (Table 1). These constructs are used to assess transcriptional activation (VP64 fused constructs) and repression (Cas9 only) by RT-qPCR. Applicants assess the cellular localization of each construct using anti-His antibody, nuclease activity using a Surveyor nuclease assay, and DNA binding affinity using a gel shift assay.

Cas Repressor

It has been shown previously that dCas9 can be used as a generic DNA binding domain to repress gene expression. Applicants report an improved dCas9 design as well as dCas9 fusions to the repressor domains KRAB and SID4x. From the plasmid library created for modulating transcription using Cas9 in Table 1, the following repressor plasmids were functionally characterized by qPCR: pXRP27, pXRP28, pXRP29, pXRP48, pXRP49, pXRP50, pXRP51, pXRP52, pXRP53, pXRP56, pXRP58, pXRP59, pXRP61, and pXRP62.

Each dCas9 repressor plasmid was co-transfected with two guide RNAs targeted to the coding strand of the beta-catenin gene. RNA was isolated 72 hours after transfection and gene expression was quantified by RT-qPCR. The endogenous control gene was GAPDH. Two validated shRNAs were used as positive controls. Negative controls were certain plasmids transfected without gRNA, these are denoted as "pXRP## control". The plasmids pXRP28, pXRP29, pXRP48, and pXRP49 could repress the beta-catenin gene when using the specified targeting strategy. These plasmids correspond to dCas9 without a functional domain (pXRP28 and pXRP28) and dCas9 fused to SID4x (pXRP48 and pXRP49).

Further work investigates: repeating the above experiment, targeting different genes, utilizing other gRNAs to determine the optimal targeting position, and multiplexed repression.

Table 1

| |
|---|
| pXRP024-pLenti2-EF1a-VP64-NLS-FLAG-Linker-dCas9-NLS-gLuc-2A-GFP-WPRE |
| pXRP025-pLenti2-EF1a-VP64-NLS-$GGGGS_3$Linker-dCas9-NLS-gLuc-2A-GFP-WPRE |
| pXRP026-pLenti2-EF1a-VP64-NLS-$EAAAK_3$Linker-dCas9-NLS-gLuc-2A-GFP-WPRE |
| pXRP027-pLenti2-EF1a-NLS-FLAG-Linker-dCas9-NLS-gLuc-2A-GFP-WPRE |
| pXRP028-pLenti2-EF1a-NLS-$GGGGS_3$Linker-dCas9-NLS-gLuc-2A-GFP-WPRE |
| pXRP029-pLenti2-EF1a-NLS-$EAAAK_3$Linker-dCas9-NLS-gLuc-2A-GFP-WPRE |
| pXRP030-pLenti2-pSV40-VP64-NLS-FLAG-Linker-dCas9-NLS-gLuc-2A-GFP-WPRE |
| pXRP031-pLenti2-pPGK-VP64-NLS-FLAG-Linker-dCas9-NLS-gLuc-2A-GFP-WPRE |
| pXRP032-pLenti2-LTR-VP64-NLS-FLAG-Linker-dCas9-NLS-gLuc-2A-GFP-WPRE |
| pXRP033-pLenti2-pSV40-VP64-NLS-$GGGGS_3$Linker-dCas9-NLS-gLuc-2A-GFP-WPRE |
| pXRP034-pLenti2-pPGK-VP64-NLS-$GGGGS_3$Linker-dCas9-NLS-gLuc-2A-GFP-WPRE |
| pXRP035-pLenti2-LTR-VP64-NLS-$GGGGS_3$Linker-dCas9-NLS-gLuc-2A-GFP-WPRE |
| pXRP036-pLenti2-pSV40-VP64-NLS-$EAAAK_3$Linker-dCas9-NLS-gLuc-2A-GFP-WPRE |

(continued)

| |
|---|
| pXRP037-pLenti2-pPGK-VP64-NLS-EAAA$K_3$Linker-dCas9-NLS-gLuc-2A-GFP-WPRE |
| pXRP038-pLenti2-LTR-VP64-NLS-EAAA$K_3$Linker-dCas9-NLS-gLuc-2A-GFP-WPRE |
| pXRP048-pLenti2-EF1a-SID4x-NLS-FLAG-Linker-dCas9-NLS-gLuc-2A-GFP-WPRE |
| pXRP049-pLenti2-EF1a-SID4X-NLS-GGGG$S_3$Linker-dCas9-NLS-gLuc-2A-GFP-WPRE |
| pXRP050-pLenti2-EF1a-SID4X-NLS-EAAA$K_3$Linker-dCas9-NLS-gLuc-2A-GFP-WPRE |
| pXRP051-pLenti2-EF1a-KRAB-NLS-FLAG-Linker-dCas9-NLS-gLuc-2A-GFP-WPRE |
| pXRP052-pLenti2-EF1a-KRAB-NLS-GGGG$S_3$Linker-dCas9-NLS-gLuc-2A-GFP-WPRE |
| pXRP053-pLenti2-EF1a-KRAB-NLS-EAAA$K_3$Linker-dCas9-NLS-gLuc-2A-GFP-WPRE |
| pXRP054-pLenti2-EF1a-dCas9-Linker-FLAG-NLS-VP64-gLuc-2A-GFP-WPRE |
| pXRP055-pLenti2-EF1a-dCas9-Linker-FLAG-NLS-SID4X-gLuc-2A-GFP-WPRE |
| pXRP056-pLenti2-EF 1a-dCas9-Linker-FLAG-NLS-KRAB-gLuc-2A-GFP-WPRE |
| pXRP057-pLenti2-EF1a-dCas9-GGGGG$S_3$-NLS-VP64-gLuc-2A-GFP-WPRE |
| pXRP058-pLenti2-EF1a-dCas9-GGGGG$S_3$-NLS-SID4X-gLuc-2A-GFP-WPRE |
| pXRP059-pLenti2-EF1a-dCas9-GGGGG$S_3$-NLS-KRAB-gLuc-2A-GFP-WPRE |
| pXRP060-pLenti2-EF1a-dCas9-EAAA$K_3$-NLS-VP64-gLuc-2A-GFP-WPRE |
| pXRP061-pLenti2-EF1a-dCas9-EAAA$K_3$-NLS-SID4X-gLuc-2A-GFP-WPRE |
| pXRP062-pLenti2-EF1a-dCas9-EAAA$K_3$-NLS-KRAB-gLuc-2A-GFP-WPRE |
| pXRP024-pLenti2-EF1a-VP64-NLS-FLAG-Linker-Cas9-NLS-gLuc-2A-GFP-WPRE |
| pXRP025-pLenti2-EF1a-VP64-NLS-GGGG$S_3$Linker-Cas9-NLS-gLuc-2A-GFP-WPRE |
| pXRP026-pLenti2-EF1a-VP64-NLS-EAAA$K_3$Linker-Cas9-NLS-gLuc-2A-GFP-WPRE |
| pXRP027-pLenti2-EF1a-NLS-FLAG-Linker-Cas9-NLS-gLuc-2A-GFP-WPRE |
| pXRP028-pLenti2-EF1a-NLS-GGGG$S_3$Linker-Cas9-NLS-gLuc-2A-GFP-WPRE |
| pXRP029-pLenti2-EF1a-NLS-EAAA$K_3$Linker-Cas9-NLS-gLuc-2A-GFP-WPRE |
| pXRP030-pLenti2-pSV40-VP64-NLS-FLAG-Linker-Cas9-NLS-gLuc-2A-GFP-WPRE |
| pXRP031-pLenti2-pPGK-VP64-NLS-FLAG-Linker-Cas9-NLS-gLuc-2A-GFP-WPRE |
| pXRP032-pLenti2-LTR-VP64-NLS-FLAG-Linker-Cas9-NLS-gLuc-2A-GFP-WPRE |
| pXRP033-pLenti2-pSV40-VP64-NLS-GGGG$S_3$Linker-Cas9-NLS-gLuc-2A-GFP-WPRE |
| pXRP034-pLenti2-pPGK-VP64-NLS-GGGG$S_3$Linker-Cas9-NLS-gLuc-2A-GFP-WPRE |
| pXRP035-pLenti2-LTR-VP64-NLS-GGGG$S_3$Linker-Cas9-NLS-gLuc-2A-GFP-WPRE |
| pXRP036-pLenti2-pSV40-VP64-NLS-EAAA$K_3$Linker-Cas9-NLS-gLuc-2A-GFP-WPRE |
| pXRP037-pLenti2-pPGK-VP64-NLS-EAAA$K_3$Linker-Cas9-NLS-gLuc-2A-GFP-WPRE |
| pXRP038-pLenti2-LTR-VP64-NLS-EAAA$K_3$Linker-Cas9-NLS-gLuc-2A-GFP-WPRE |
| pXRP048-pLenti2-EF1a-SID4x-NLS-FLAG-Linker-Cas9-NLS-gLuc-2A-GFP-WPRE |
| pXRP049-pLenti2-EF1a-SID4X-NLS-GGGG$S_3$Linker-Cas9-NLS-gLuc-2A-GFP-WPRE |
| pXRP050-pLenti2-EF1a-SID4X-NLS-EAAA$K_3$Linker-Cas9-NLS-gLuc-2A-GFP-WPRE |
| pXRP051-pLenti2-EF1a-KRAB-NLS-FLAG-Linker-Cas9-NLS-gLuc-2A-GFP-WPRE |
| pXRP052-pLenti2-EF1a-KRAB-NLS-GGGG$S_3$Linker-Cas9-NLS-gLuc-2A-GFP-WPRE |
| pXRP053-pLenti2-EF1a-KRAB-NLS-EAAA$K_3$Linker-Cas9-NLS-gLuc-2A-GFP-WPRE |
| pXRP054-pLenti2-EF1a-Cas9-Linker-FLAG-NLS-VP64-gLuc-2A-GFP-WPRE |

(continued)

| |
|---|
| pXRP055-pLenti2-EF1a-Cas9-Linker-FLAG-NLS-SID4X-gLuc-2A-GFP-WPRE |
| pXRP056-pLenti2-EF1a-Cas9-Linker-FLAG-NLS-KRAB-gLuc-2A-GFP-WPRE |
| pXRP057-pLenti2-EF1a-Cas9-$GGGGGS_3$-NLS-VP64-gLuc-2A-GFP-WPRE |
| pXRP058-pLenti2-EF1a-Cas9-$GGGGGS_3$-NLS-SID4X-gLuc-2A-GFP-WPRE |
| pXRP059-pLenti2-EF1a-Cas9-$GGGGGS_3$-NLS-KRAB-gLuc-2A-GFP-WPRE |
| pXRP060-pLenti2-EF1a-Cas9-$EAAAK_3$-NLS-VP64-gLuc-2A-GFP-WPRE |
| pXRP061-pLenti2-EF1a-Cas9-$EAAAK_3$-NLS-SID4X-gLuc-2A-GFP-WPRE |
| pXRP062-pLenti2-EF1a-Cas9-$EAAAK_3$-NLS-KRAB-gLuc-2A-GFP-WPRE |

*Example 16: Targeted deletion of genes involved in cholesterol biosynthesis, fatty acid biosynthesis, and other metabolic disorders, genes encoding mis-folded proteins involved in amyloid and other diseases, oncogenes leading to cellular transformation, latent viral genes, and genes leading to dominant-negative disorders, amongst other disorders.*

Applicants demonstrate gene delivery of a CRISPR-Cas system in the liver, brain, ocular, epithelial, hematopoetic, or another tissue of a subject or a patient in need thereof, suffering from metabolic disorders, amyloidosis and protein-aggregation related diseases, cellular transformation arising from genetic mutations and translocations, dominant negative effects of gene mutations, latent viral infections, and other related symptoms, using either viral or nanoparticle delivery system.

**Study Design:** Subjects or patients in need thereof suffering from metabolic disorders, amyloidosis and protein aggregation related disease which include but are not limited to human, non-primate human, canine, feline, bovine, equine, other domestic animals and related mammals. The CRISPR-Cas system is guided by a chimeric guide RNA and targets a specific site of the human genomic loci to be cleaved. After cleavage and non-homologous end-joining mediated repair, frame-shift mutation results in knock out of genes.

Applicants select guide-RNAs targeting genes involved in above-mentioned disorders to be specific to endogenous loci with minimal off-target activity. Two or more guide RNAs may be encoded into a single CRISPR array to induce simultaneous double-stranded breaks in DNA leading to micro-deletions of affected genes or chromosomal regions.

Identification and design of gene targets

For each candidate disease gene, Applicants select DNA sequences of interest include protein-coding exons, sequences including and flanking known dominant negative mutation sites, sequences including and flanking pathological repetitive sequences. For gene-knockout approaches, early coding exons closest to the start codon offer best options for achieving complete knockout and minimize possibility of truncated protein products retaining partial function.

Applicants analyze sequences of interest for all possible targetable 20-bp sequences immediately 5' to a NGG motif (for SpCas9 system) or a NNAGAAW (for St1Cas9 system). Applicants choose sequences for unique, single RNA-guided Cas9 recognition in the genome to minimize off-target effects based on computational algorithm to determine specificity.

Cloning of guide sequences into a delivery system

Guide sequences are synthesized as double-stranded 20-24 bp oligonucleotides. After 5'-phosphorylation treatment of oligos and annealing to form duplexes, oligos are ligated into suitable vector depending on the delivery method:

Virus-based delivery methods

AAV-based vectors (PX260, 330, 334, 335) have been described elsewhere

Lentiviral-based vectors use a similar cloning strategy of directly ligating guide sequences into a single vector carrying a U6 promoter-driven chimeric RNA scaffold and a EF1a promoter-driven Cas9 or Cas9 nickase.

Virus production is described elsewhere.

Nanoparticle-based RNA delivery methods

1. Guide sequences are synthesized as an oligonucleotide duplex encoding T7 promoter-guide sequence-chimeric RNA. A T7 promoter is added 5' of Cas9 by PCR method.
2. T7-driven Cas9 and guide-chimeric RNAs are transcribed *in vitro,* and Cas9 mRNA is further capped and A-tailed using commercial kits. RNA products are purified per kit instructions.

Hydrodynamic tail vein delivery methods (for mouse)

Guide sequences are cloned into AAV plasmids as described above and elsewhere in this application.

*In vitro* validation on cell lines

Transfection

1. DNA plasmid transfection

Plasmids carrying guide sequences are transfected into human embryonic kidney (HEK293T) or human embryonic stem (hES) cells, other relevant cell types using lipid-, chemical-, or electroporation-based methods. For a 24-well transfection of HEK293T cells (~260,000 cells), 500ng of total DNA is transfected into each single well using Lipofectamine 2000. For a 12-well transfection of hES cells, 1ug of total DNA is transfected into a single well using Fugene HD.

2. RNA transfection

Purified RNA described above is used for transfection into HEK293T cells. 1-2ug of RNA may be transfected into ~260,000 using Lipofectamine 2000 per manufacturer's instruction. RNA delivery of Cas9 and chimeric RNA is shown in Fig. 24.

Assay of indel formation *in vitro*

Cells are harvested 72-hours post-transfection and assayed for indel formation as an indication of double-stranded breaks.

Briefly, genomic region around target sequence is PCR amplified (~400-600 bp amplicon size) using high-fidelity polymerase. Products are purified, normalized to equal concentration, and slowly annealed from 95°C to 4°C to allow formation of DNA heteroduplexes. Post annealing, the Cel-I enzyme is used to cleave heteroduplexes, and resulting products are separated on a polyacrylamide gel and indel efficiency calculated.

In vivo proof of principle in animal

Delivery mechanisms

AAV or Lentivirus production is described elsewhere.

Nanoparticle formulation: RNA mixed into nanoparticle formulation

Hydrodynamic tail vein injections with DNA plasmids in mice are conducted using a commercial kit

Cas9 and guide sequences are delivered as virus, nanoparticle-coated RNA mixture, or DNA plasmids, and injected into subject animals. A parallel set of control animals is injected with sterile saline, Cas9 and GFP, or guide sequence and GFP alone.

Three weeks after injection, animals are tested for amelioration of symptoms and sacrificed. Relevant organ systems analyzed for indel formation. Phenotypic assays include blood levels of HDL, LDL, lipids,

Assay for indel formation

DNA is extracted from tissue using commercial kits; indel assay will be performed as described for *in vitro* demonstration.

Therapeutic applications of the CRISPR-Cas system are amenable for achieving tissue-specific and temporally controlled targeted deletion of candidate disease genes. Examples include genes involved in cholesterol and fatty acid metabolism, amyloid diseases, dominant negative diseases, latent viral infections, among other disorders.

Examples of a single guide-RNA to introduce targeted indels at a gene locus

| Disease | GENE | SPACER | PAM | Mechanism | References |
|---|---|---|---|---|---|
| Hypercholesterolemia | HMGCR | GCCAAATTGGACGACCCTCG | CGG | Knockout | Fluvastatin: a review of its pharmacology and use in the management of hypercholesterolaemia.(Plosker GL et al. Drugs 1996, 51(3):433-459) |
| Hypercholesterolemia | SQLE | CGAGGAGACCCCCGTTTCGG | TGG | Knockout | Potential role of nonstatin cholesterol lowering agents (Trapani et al. IUBMB Life, Volume 63, Issue 11, pages 964-971, November 2011) |
| Hyperlipidemia | DGAT 1 | CCCGCCGCCGCCGTGGCTCG | AGG | Knockout | DGAT1 inhibitors as anti-obesity and anti-diabetic agents. (Birch AM et al. Current Opinion in Drug Discovery & Development [2010, 13(4):489-496) |
| Leukemia | BCR-ABL | TGAGCTCTACGAGATCCACA | AGG | Knockout | Killing of leukemic cells with a BCR/ABL fusion gene by RNA interference (RNAi).( Fuchs et al. Oncogene 2002, 21(37):5716-5724) |

Examples of a pair of guide-RNA to introduce chromosomal microdeletion at a gene locus

| Disease | GENE | SPACER | PAM | Mechanism | References |
|---|---|---|---|---|---|
| Hyperlipidemia | PLIN2 guide1 | CTCAAAATTCATACCGGTTG | TGG | Microdeletion | Perilipin-2 Null Mice are Protected Against Diet-Induced Obesity, Adipose Inflammation and Fatty Liver Disease (McManaman JL et al. The Journal of Lipid Research, jlr.M035063. First Published on February 12, 2013) |
| Hyperlipidemia | PLIN2 guide2 | CGTTAAACAACAACCGGACT | TGG | Microdeletion | |
| Hyperlipidemia | SREBP guide1 | TTCACCCCGCGGCGCTGAAT | ggg | Microdeletion | Inhibition of SREBP by a Small Molecule, Betulin, Improves Hyperlipidemia and Insulin Resistance and Reduces Atherosclerotic Plaques (Tang J et al. Cell Metabolism, Volume 13, Issue 1, 44-56, 5 January 2011) |
| Hyperlipidemia | SREBP guide2 | ACCACTACCAGTCCGTCCAC | agg | Microdeletion | |

*Example 17: Targeted integration of repair for genes carrying disease-causing mutations; reconstitution of enzyme deficiencies and other related diseases.*

Study design

I. Identification and design of gene targets

- Described in Example 20

II. Cloning of guide sequences and repair templates into a delivery system

- Described above in Example 20
- Applicants clone DNA repair templates to include homology arms with diseased allele as well a wild-type repair template

III. *In vitro* validation on cell lines

a. Transfection is described above in Example 20; Cas9, guide RNAs, and repair template are co-transfected into relevant cell types.

b. Assay for repair *in vitro*

i. Applicants harvest cells 72-hours post-transfection and assay for repair

ii. Briefly, Applicants amplify genomic region around repair template PCR using high-fidelity polymerase. Applicants sequence products for decreased incidence of mutant allele.

IV. *In vivo* proof of principle in animal

a. Delivery mechanisms are described herein.

b. Assay for repair *in vivo*

i. Applicants perform the repair assay as described in the *in vitro* demonstration.

V. Therapeutic applications

The CRISPR-Cas system is amenable for achieving tissue-specific and temporally controlled targeted deletion of candidate disease genes. Examples include genes involved in cholesterol and fatty acid metabolism, amyloid diseases, dominant negative diseases, latent viral infections, among other disorders.

Example of one single missense mutation with repair template:

| **Disease** | **GENE** | **SPACER** | **PAM** |
|---|---|---|---|
| Familial amyloid polyneuropathy | TTR | AGCCTTTCTGAACACATGCA | CGG |

| **Mechanism** | **References** |
|---|---|
| V30M repair | Transthyretin mutations in health and disease (Joao et al. Human Mutation, Volume 5, Issue 3, pages 191-196, 1995) |
| V30M allele | CCTGCCATCAATGTGGCC**A**TGCATGTGTTCAGAAAGGCT |
| WT allele | CCTGCCATCAATGTGGCC**G**TGCATGTGTTCAGAAAGGCT |

*Example 18: Therapeutic application of the CRISPR-Cas system in Glaucoma, Amyloidosis, and Huntington's disease*

Glaucoma: Applicants design guide RNAs to target the first exon of the mycilin (MYOC) gene. Applicants use adenovirus vectors (Ad5) to package both Cas9 as well as a guide RNA targeting the MYOC gene. Applicants inject adenoviral vectors into the trabecular meshwork where cells have been implicated in the pathophysiology of glaucoma. Applicants initially test this out in mouse models carrying the mutated MYOC gene to see whether they improve visual acuity and decrease pressure in the eyes. Therapeutic application in humans employ a similar strategy.

Amyloidosis: Applicants design guide RNAs to target the first exon of the transthyretin (TTR) gene in the liver. Applicants use AAV8 to package Cas9 as well as guide RNA targeting the first exon of the TTR gene. AAV8 has been shown to have efficient targeting of the liver and will be administered intravenously. Cas9 can be driven either using liver specific promoters such as the albumin promoter, or using a constitutive promoter. A pol3 promoter drives the guide RNA.

Alternatively, Applicants utilize hydrodynamic delivery of plasmid DNA to knockout the TTR gene. Applicants deliver a plasmid encoding Cas9 and the guideRNA targeting Exon1 of TTR.

As a further alternative approach, Applicants administer a combination of RNA (mRNA for Cas9, and guide RNA). RNA can be packaged using liposomes such as Invivofectamine from Life Technologies and delivered intravenously. To reduce RNA-induced immunogenicity, increase the level of Cas9 expression and guide RNA stability, Applicants modify the Cas9 mRNA using 5' capping. Applicants also incorporate modified RNA nucleotides into Cas9 mRNA and guide RNA to increase their stability and reduce immunogenicity (e.g. activation of TLR). To increase efficiency, Applicants administer multiple doses of the virus, DNA, or RNA.

Huntington's Disease: Applicants design guide RNA based on allele specific mutations in the HTT gene of patients. For example, in a patient who is heterozygous for HTT with expanded CAG repeat, Applicants identify nucleotide sequences unique to the mutant HTT allele and use it to design guideRNA. Applicants ensure that the mutant base is located within the last 9 bp of the guide RNA (which Applicants have ascertained has the ability to discriminate between single DNA base mismatches between the target size and the guide RNA).

Applicants package the mutant HTT allele specific guide RNA and Cas9 into AAV9 and deliver into the striatum of Huntington's patients. Virus is injected into the striatum stereotactically via a craniotomy. AAV9 is known to transduce neurons efficiently. Applicants drive Cas9 using a neuron specific promoter such as human Synapsin I.

Example 19: Therapeutic application of the CRISPR-Cas system in HIV

Chronic viral infection is a source of significant morbidity and mortality. While there exists for many of these viruses conventional antiviral therapies that effectively target various aspects of viral replication, current therapeutic modalities are usually non-curative in nature due to "viral latency." By its nature, viral latency is characterized by a dormant phase in the viral life cycle without active viral production. During this period, the virus is largely able to evade both immune surveillance and conventional therapeutics allowing for it to establish long-standing viral reservoirs within the host from which subsequent re-activation can permit continued propagation and transmission of virus. Key to viral latency is the ability to stably maintain the viral genome, accomplished either through episomal or proviral latency, which stores the viral genome in the cytoplasm or integrates it into the host genome, respectively. In the absence of effective vaccinations which would prevent primary infection, chronic viral infections characterized by latent reservoirs and episodes of lytic activity can have significant consequences: human papilloma virus (HPV) can result in cervical cancer, hepatitis C virus (HCV) predisposes to hepatocellular carcinoma, and human immunodeficiency virus eventually destroys the host immune system resulting in susceptibility to opportunistic infections. As such, these infections require life-long use of currently available antiviral therapeutics. Further complicating matters is the high mutability of many of these viral genomes which lead to the evolution of resistant strains for which there exists no effective therapy.

The CRISPR-Cas system is a bacterial adaptive immune system able to induce doublestranded DNA breaks (DSB) in a multiplex-able, sequence-specific manner and has been recently re-constituted within mammalian cell systems. It has been shown that targeting DNA with one or numerous guide-RNAs can result in both indels and deletions of the intervening sequences, respectively. As such, this new technology represents a means by which targeted and multiplexed DNA mutagenesis can be accomplished within a single cell with high efficiency and specificity. Consequently, delivery of the CRISPR-Cas system directed against viral DNA sequences could allow for targeted disruption and deletion of latent viral genomes even in the absence of ongoing viral production.

As an example, chronic infection by HIV-1 represents a global health issue with 33 million individuals infected and an annual incidence of 2.6 million infections. The use of the multimodal highly active antiretroviral therapy (HAART), which simultaneously targets multiple aspects of viral replication, has allowed HIV infection to be largely managed as a chronic, not terminal, illness. Without treatment, progression of HIV to AIDS occurs usually within 9-10 years resulting in depletion of the host immune system and occurrence of opportunistic infections usually leading to death soon thereafter. Secondary to viral latency, discontinuation of HAART invariably leads to viral rebound. Moreover, even temporary disruptions in therapy can select for resistant strains of HIV uncontrollable by available means. Additionally, the costs of HAART therapy are significant: within the US $10,000-15,0000 per person per year. As such, treatment approaches directly targeting the HIV genome rather than the process of viral replication represents a means by which eradication of latent reservoirs could allow for a curative therapeutic option.

Development and delivery of an HIV-1 targeted CRISPR-Cas system represents a unique approach differentiable from existing means of targeted DNA mutagenesis, i.e. ZFN and TALENs, with numerous therapeutic implications.

Targeted disruption and deletion of the HIV-1 genome by CRISPR-mediated DSB and indels in conjunction with HAART could allow for simultaneous prevention of active viral production as well as depletion of latent viral reservoirs within the host.

Once integrated within the host immune system, the CRISPR-Cas system allows for generation of a HIV-1 resistant sub-population that, even in the absence of complete viral eradication, could allow for maintenance and reconstitution of host immune activity. This could potentially prevent primary infection by disruption of the viral genome preventing viral production and integration, representing a means to "vaccination". Multiplexed nature of the CRISPR-Cas system allows targeting of multiple aspects of the genome simultaneously within individual cells.

As in HAART, viral escape by mutagenesis is minimized by requiring acquisition of multiple adaptive mutations concurrently. Multiple strains of HIV-1 can be targeted simultaneously which minimizes the chance of super-infection and prevents subsequent creation of new recombinants strains. Nucleotide, rather than protein, mediated sequence-specificity of the CRISPR-Cas system allows for rapid generation of therapeutics without need for significantly altering delivery mechanism.

In order to accomplish this, Applicants generate CRISPR-Cas guide RNAs that target the vast majority of the HIV-1 genome while taking into account HIV-1 strain variants for maximal coverage and effectiveness. Sequence analyses of genomic conservation between HIV-1 subtypes and variants should allow for targeting of flanking conserved regions of the genome with the aims of deleting intervening viral sequences or induction of frame-shift mutations which would disrupt viral gene functions.

Applicants accomplish delivery of the CRISPR-Cas system by conventional adenoviral or lentiviral-mediated infection of the host immune system. Depending on approach, host immune cells could be a) isolated, transduced with CRISPR-Cas, selected, and re-introduced in to the host or b) transduced in vivo by systemic delivery of the CRISPR-Cas system. The first approach allows for generation of a resistant immune population whereas the second is more likely to target latent viral reservoirs within the host.

| Examples of potential HIV-1 targeted spacers adapted from Mcintyre *et al,* which generated shRNAs against HIV-1 optimized for maximal coverage of HIV-1 variants. |
|---|
| CACTGCTTAAGCCTCGCTCGAGG<br>TCACCAGCAATATTCGCTCGAGG<br>CACCAGCAATATTCCGCTCGAGG<br>TAGCAACAGACATACGCTCGAGG<br>GGGCAGTAGTAATACGCTCGAGG<br>CCAATTCCCATACATTATTGTAC |

*Example 20: Targeted correction of deltaF508 or other mutations in cystic fibrosis*

An aspect of the invention provides for a pharmaceutical composition that may comprise an CRISPR-Cas gene therapy particle and a biocompatible pharmaceutical carrier. According to another aspect, a method of gene therapy for the treatment of a subject having a mutation in the CFTR gene comprises administering a therapeutically effective amount of a CRISPR-Cas gene therapy particle to the cells of a subject.

This Example demonstrates gene transfer or gene delivery of a CRISPR-Cas system in airways of subject or a patient in need thereof, suffering from cystic fibrosis or from cystic fibrosis related symptoms, using adeno-associated virus (AAV) particles.

Study Design: Subjects or patients in need there of: Human, non-primate human, canine, feline, bovine, equine and other domestic animals, related. This study tests efficacy of gene transfer of a CRISPR-Cas system by a AAV vector. Applicants determine transgene levels sufficient for gene expression and utilize a CRISPR-Cas system comprising a Cas9 enzyme to target deltaF508 or other CFTR-inducing mutations.

The treated subjects receive pharmaceutically effective amount of aerosolized AAV vector system per lung endobronchially delivered while spontaneously breathing. The control subjects receive equivalent amount of a pseudotyped AAV vector system with an internal control gene. The vector system may be delivered along with a pharmaceutically acceptable or biocompatible pharmaceutical carrier. Three weeks or an appropriate time interval following vector administration, treated subjects are tested for amelioration of cystic fibrosis related symptoms.

Applicants use an adenovirus or an AAV particle.

Applicants clone the following gene constructs, each operably linked to one or more regulatory sequences (Cbh or EF1a promoter for Cas9, U6 or HI promoter for chimeric guide RNA), into one or more adenovirus or AAV vectors or any other compatible vector: A CFTRdelta508 targeting chimeric guide RNA (Fig. 27B), a repair template for deltaF508 mutation (Fig. 27C) and a codon optimized Cas9 enzyme with optionally one or more nuclear localization signal or sequence(s) (NLS(s)), e.g., two (2) NLSs.

Identification of Cas9 target site

Applicants analyzed the human CFTR genomic locus and identified the Cas9 target site (Fig. 27A). (PAM may contain a NGG or a NNAGAAW motif).

Gene Repair Strategy

Applicants introduce an adenovirus/AAV vector system comprising a Cas9 (or Cas9 nickase) and the guide RNA along with a adenovirus/AAV vector system comprising the homology repair template containing the F508 residue into the subject via one of the methods of delivery discussed earlier. The CRISPR-Cas system is guided by the CFTRdelta 508 chimeric guide RNA and targets a specific site of the CFTR genomic locus to be nicked or cleaved. After cleavage, the repair template is inserted into the cleavage site via homologous recombination correcting the deletion that results in cystic fibrosis or causes cystic fibrosis related symptoms. This strategy to direct delivery and provide systemic introduction of CRISPR systems with appropriate guide RNAs can be employed to target genetic mutations to edit or otherwise manipulate genes that cause metabolic, liver, kidney and protein diseases and disorders such as those in Table B.

*Example 21: Use of Cas9 to target a variety of disease types*

Diseases that involve mutations in protein coding sequence:
Dominant disorders may be targeted by inactivating the dominant negative allele. Applicants use Cas9 to target a unique sequence in the dominant negative allele and introduce a mutation via NHEJ. The NHEJ-induced indel may be able to introduce a frame-shift mutation in the dominant negative allele and eliminate the dominant negative protein. This may work if the gene is haplo-sufficient (e.g. MYOC mutation induced glaucoma and Huntington's disease).
Recessive disorders may be targeted by repairing the disease mutation in both alleles. For dividing cells, Applicants use Cas9 to introduce double strand breaks near the mutation site and increase the rate of homologous recombination using an exogenous recombination template. For dividing cells, this may be achieved using multiplexed nickase activity to catalyze the replacement of the mutant sequence in both alleles via NHEJ-mediated ligation of an exogenous DNA fragment carrying complementary overhangs.
Applicants also use Cas9 to introduce protective mutations (e.g. inactivation of CCR5 to prevent HIV infection, inactivation of PCSK9 for cholesterol reduction, or introduction of the A673T into APP to reduce the likelihood of Alzheimer's disease).

**Diseases that involve non-coding sequences**

Applicants use Cas9 to disrupt non-coding sequences in the promoter region, to alter transcription factor binding sites and alter enhancer or repressor elements. For example, Cas9 may be used to excise out the Klf1 enhancer EHS1 in hematopoietic stem cells to reduce BCL11a levels and reactivate fetal globin gene expression in differentiated erythrocytes
Applicants also use Cas9 to disrupt functional motifs in the 5' or 3' untranslated regions. For example, for the treatment of myotonic dystrophy, Cas9 may be used to remove CTG repeat expansions in the DMPK gene.

*Example 22: Multiplexed Nickase*

Aspects of optimization and the teachings of Cas9 detailed in this application may also be used to generate Cas9 nickases. Applicants use Cas9 nickases in combination with pairs of guide RNAs to generate DNA double strand breaks with defined overhangs. When two pairs of guide RNAs are used, it is possible to excise an intervening DNA fragment. If an exogenous piece of DNA is cleaved by the two pairs of guide RNAs to generate compatible overhangs with the genomic DNA, then the exogenous DNA fragment may be ligated into the genomic DNA to replace the excised fragment. For example, this may be used to remove trinucleotide repeat expansion in the huntintin (HTT) gene to treat Huntington's Disease.
If an exogenous DNA that bears fewer number of CAG repeats is provided, then it may be able to generate a fragment of DNA that bears the same overhangs and can be ligated into the HTT genomic locus and replace the excised fragment.

| | | | |
|---|---|---|---|
| HTT locus with fragment excised by Cas9 nickase and two pairs of guide RNAs | ...CCGTGCCGGGCGGGAGACCGCCATGG<br>...GGCACGGCCCGCCCTCTGGC | + | GGCCCGGCTGTGGCTGAGGAGC...<br>TGGGCCGGGCCGACACCGACTCCTCG... |
| exogenous DNA fragment with fewer number of CAG repeats also cleaved by Cas9 nicakse and the two pairs of guide RNAs | CGACCCTGGAAA....<br>GGTACCGCΓGGGACCTTT..... | reduced number of CAG repeats | .....CCCCGCCGCCACCC<br>.....GGGGCGGCGG |

The ligation of the exogenous DNA fragment into the genome does not require homologous recombination machineries and therefore this method may be used in post-mitotic cells such as neurons.

*Example 23: Delivery of CRISPR System*

While an aspect of the invention is delivery of the CRISPR complex via one or more nanoparticle complex(es) or delivery of at least one component of the CRISPR complex, e.g., RNA thereof, via at least one nanoparticle complex, other delivery systems can be used in conjunction with the nanoparticle delivery of the system; e.g., to deliver Cas9 or have it expressed. Cas9 and its chimeric guide RNA, or combination of tracrRNA and crRNA, can be delivered either as DNA or RNA. Delivery of Cas9 and guide RNA both as RNA (normal or containing base or backbone modifications) molecules can be used to reduce the amount of time that Cas9 protein persist in the cell. This may reduce the level of off-target cleavage activity in the target cell. Since delivery of Cas9 as mRNA takes time to be translated into protein, it might be advantageous to deliver the guide RNA several hours following the delivery of Cas9 mRNA, to maximize the level of guide RNA available for interaction with Cas9 protein.

In situations where guide RNA amount is limiting, it may be desirable to introduce Cas9 as mRNA and guide RNA in the form of a DNA expression cassette with a promoter driving the expression of the guide RNA. This way the amount of guide RNA available will be amplified via transcription.

A variety of delivery systems can be introduced to introduce Cas9 (DNA or RNA) and guide RNA (DNA or RNA) into the host cell. These include the use of liposomes, viral vectors, electroporation, nanoparticles , nanowires (Shalek et al., Nano Letters, 2012), exosomes. Molecular trojan horses liposomes (Pardridge et al., Cold Spring Harb Protoc; 2010; doi:10.1101/pdb.prot5407) may be used to deliver Cas9 and guide RNA across the blood brain barrier.

*Example 24: CRISPR-Cas for rapid, multiplex genome editing*

Aspects of the invention relate to protocols and methods by which efficiency and specificity of gene modification may be tested within 3-4 days after target design, and modified clonal cell lines may be derived within 2-3 weeks.

Programmable nucleases are powerful technologies for mediating genome alteration with high precision. The RNA-guided Cas9 nuclease from the microbial CRISPR adaptive immune system can be used to facilitate efficient genome editing in eukaryotic cells by simply specifying a 20-nt targeting sequence in its guide RNA. Applicants describe a set of protocols for applying Cas9 to facilitate efficient genome editing in mammalian cells and generate cell lines for downstream functional studies. Beginning with target design, efficient and specific gene modification can be achieved within 3-4 days, and modified clonal cell lines can be derived within 2-3 weeks.

The ability to engineer biological systems and organisms holds enormous potential for applications across basic science, medicine, and biotechnology. Programmable sequence-specific endonucleases that facilitate precise editing of endogenous genomic loci are now enabling systematic interrogation of genetic elements and causal genetic variations in a broad range of species, including those that have not been genetically tractable previously. A number of genome editing technologies have emerged in recent years, including zinc finger nucleases (ZFNs), transcription activator-like effector nucleases (TALENs), and the RNA-guided CRISPR-Cas nuclease system. The first two technologies use a common strategy of tethering endonuclease catalytic domains to modular DNA-binding proteins for inducing targeted DNA double stranded breaks (DSB) at specific genomic loci. By contrast, Cas9 is a nuclease guided by small RNAs through Watson-Crick base-pairing with target DNA, presenting a system that is easy to design, efficient, and well-suited for high-throughput and multiplexed gene editing for a variety of cell types and organisms. Here Applicants describe a set of protocols for applying the recently developed Cas9 nuclease to facilitate efficient genome editing in mammalian cells and generate cell lines for downstream functional studies.

Like ZFNs and TALENs, Cas9 promotes genome editing by stimulating DSB at the target genomic loci. Upon cleavage by Cas9, the target locus undergoes one of two major pathways for DNA damage repair, the error-prone non-homologous end joining (NHEJ) or the high-fidelity homology directed repair (HDR) pathway. Both pathways may be utilized to achieve the desired editing outcome.

NHEJ: In the absence of a repair template, the NHEJ process re-ligates DSBs, which may leave a scar in the form of indel mutations. This process can be harnessed to achieve gene knockouts, as indels occurring within a coding exon may lead to frameshift mutations and a premature stop codon. Multiple DSBs may also be exploited to mediate larger deletions in the genome.

HDR: Homology directed repair is an alternate major DNA repair pathway to NHEJ. Although HDR typically occurs at lower frequencies than NHEJ, it may be harnessed to generate precise, defined modifications at a target locus in the presence of an exogenously introduced repair template. The repair template may be either in the form of double stranded DNA, designed similarly to conventional DNA targeting constructs with homology arms flanking the insertion sequence, or single-stranded DNA oligonucleotides (ssODNs). The latter provides an effective and simple method for making small edits in the genome, such as the introduction of single nucleotide mutations for probing causal genetic variations. Unlike NHEJ, HDR is generally active only in dividing cells and its efficiency varies depending on the cell type and state.

Overview of CRISPR: The CRISPR-Cas system, by contrast, is at minimum a two-component system consisting of the Cas9 nuclease and a short guide RNA. Re-targeting of Cas9 to different loci or simultaneous editing of multiple genes simply requires cloning a different 20-bp oligonucleotide. Although specificity of the Cas9 nuclease has yet to be thoroughly elucidated, the simple Watson-Crick base-pairing of the CRISPR-Cas system is likely more predictable than that of ZFN or TALEN domains.

The type II CRISPR-Cas (clustered regularly interspaced short palindromic repeats) is a bacterial adaptive immune system that uses Cas9, to cleave foreign genetic elements. Cas9 is guided by a pair of non-coding RNAs, a variable crRNA and a required auxiliary tracrRNA. The crRNA contains a 20-nt guide sequence determines specificity by locating the target DNA via Watson-Crick base-pairing. In the native bacterial system, multiple crRNAs are co-transcribed to direct Cas9 against various targets. In the CRISPR-Cas system derived from *Streptococcus pyogenes,* the target DNA must immediately precede a 5'-NGG/NRG protospacer adjacent motif (PAM), which can vary for other CRISPR systems.

CRISPR-Cas is reconstituted in mammalian cells through the heterologous expression of human codon-optimized Cas9 and the requisite RNA components. Furthermore, the crRNA and tracrRNA can be fused to create a chimeric, synthetic guide RNA (sgRNA). Cas9 can thus be redirected toward any target of interest by altering the 20-nt guide sequence within the sgRNA.

Given its ease of implementation and multiplex capability, Cas9 has been used to generate engineered eukaryotic cells carrying specific mutations via both NHEJ and HDR. In addition, direct injection of sgRNA and mRNA encoding Cas9 into embryos has enabled the rapid generation of transgenic mice with multiple modified alleles; these results hold promise for editing organisms that are otherwise genetically intractable.

A mutant Cas9 carrying a disruption in one of its catalytic domains has been engineered to nick rather than cleave DNA, allowing for single-stranded breaks and preferential repair through HDR, potentially ameliorating unwanted indel mutations from off-target DSBs. Additionally, a Cas9 mutant with both DNA-cleaving catalytic residues mutated has been adapted to enable transcriptional regulation in *E. coli,* demonstrating the potential of functionalizing Cas9 for diverse applications. Certain aspects of the invention relate to the construction and application of Cas9 for multiplexed editing of human cells.

Applicants have provided a human codon-optimized, nuclear localization sequence-flanked Cas9 to facilitate eukaryotic gene editing. Applicants describe considerations for designing the 20-nt guide sequence, protocols for rapid construction and functional validation of sgRNAs, and finally use of the Cas9 nuclease to mediate both NHEJ- and HDR-based genome modifications in human embryonic kidney (HEK-293FT) and human stem cell (HUES9) lines. This protocol can likewise be applied to other cell types and organisms.

Target selection for sgRNA: There are two main considerations in the selection of the 20-nt guide sequence for gene targeting: 1) the target sequence should precede the 5'-NGG PAM for S. pyogenes Cas9, and 2) guide sequences should be chosen to minimize off-target activity. Applicants provided an online Cas9 targeting design tool that takes an input sequence of interest and identifies suitable target sites. To experimentally assess off-target modifications for each sgRNA, Applicants also provide computationally predicted off-target sites for each intended target, ranked according to Applicants' quantitative specificity analysis on the effects of base-pairing mismatch identity, position, and distribution.

The detailed information on computationally predicted off-target sites is as follows:

Considerations for Off-target Cleavage Activities: Similar to other nucleases, Cas9 can cleave off-target DNA targets in the genome at reduced frequencies. The extent to which a given guide sequence exhibit off-target activity depends on a combination of factors including enzyme concentration, thermodynamics of the specific guide sequence employed, and the abundance of similar sequences in the target genome. For routine application of Cas9, it is important to consider ways to minimize the degree of off-target cleavage and also to be able to detect the presence of off-target cleavage.

Minimizing off-target activity: For application in cell lines, Applicants recommend following two steps to reduce the degree of off-target genome modification. First, using our online CRISPR target selection tool, it is possible to computationally assess the likelihood of a given guide sequence to have off-target sites. These analyses are performed through an exhaustive search in the genome for off-target sequences that are similar sequences as the guide sequence. Comprehensive experimental investigation of the effect of mismatching bases between the sgRNA and its target DNA revealed that mismatch tolerance is 1) position dependent - the 8-14 bp on the 3' end of the guide sequence are less tolerant of mismatches than the 5' bases, 2) quantity dependent - in general more than 3 mismatches are not tolerated, 3) guide sequence dependent - some guide sequences are less tolerant of mismatches than others, and 4) concentration dependent - off-target cleavage is highly sensitive to the amount of transfected DNA. The Applicants' target site analysis web tool (available at the website genome-engineering.org/tools) integrates these criteria to provide predictions for likely off-target sites in the target genome. Second, Applicants recommend titrating the amount of Cas9 and sgRNA expression plasmid to minimize off-target activity.

Detection of off-target activities: Using Applicants' CRISPR targeting web tool, it is possible to generate a list of most likely off-target sites as well as primers performing SURVEYOR or sequencing analysis of those sites. For isogenic clones generated using Cas9, Applicants strongly recommend sequencing these candidate off-target sites to check for any undesired mutations. It is worth noting that there may be off target modifications in sites that are not included in the predicted candidate list and full genome sequence should be performed to completely verify the absence of off-target sites. Furthermore, in multiplex assays where several DSBs are induced within the same genome, there may be low rates of translocation events and can be evaluated using a variety of techniques such as deep sequencing.

The online tool provides the sequences for all oligos and primers necessary for 1) preparing the sgRNA constructs, 2) assaying target modification efficiency, and 3) assessing cleavage at potential off-target sites. It is worth noting that because the U6 RNA polymerase III promoter used to express the sgRNA prefers a guanine (G) nucleotide as the first base of its transcript, an extra G is appended at the 5' of the sgRNA where the 20-nt guide sequence does not begin with G.

Approaches for sgRNA construction and delivery: Depending on the desired application, sgRNAs may be delivered as either 1) PCR amplicons containing an expression cassette or 2) sgRNA-expressing plasmids. PCR-based sgRNA delivery appends the custom sgRNA sequence onto the reverse PCR primer used to amplify a U6 promoter template. The resulting amplicon may be co-transfected with a plasmid containing Cas9 (PX165). This method is optimal for rapid screening of multiple candidate sgRNAs, as cell transfections for functional testing can be performed mere hours after obtaining the sgRNA-encoding primers. Because this simple method obviates the need for plasmid-based cloning and sequence verification, it is well suited for testing or co-transfecting a large number of sgRNAs for generating large knockout libraries or other scale-sensitive applications. Note that the sgRNA-encoding primers are over 100-bp, compared to the ~20-bp oligos required for plasmid-based sgRNA delivery.

Construction of an expression plasmid for sgRNA is also simple and rapid, involving a single cloning step with a pair of partially complementary oligonucleotides. After annealing the oligo pairs, the resulting guide sequences may be inserted into a plasmid bearing both Cas9 and an invariant scaffold bearing the remainder of the sgRNA sequence (PX330). The transfection plasmids may also be modified to enable virus production for *in vivo* delivery.

In addition to PCR and plasmid-based delivery methods, both Cas9 and sgRNA can be introduced into cells as RNA.

Design of repair template: Traditionally, targeted DNA modifications have required use of plasmid-based donor repair templates that contain homology arms flanking the site of alteration. The homology arms on each side can vary in length, but are typically longer than 500 bp. This method can be used to generate large modifications, including insertion of reporter genes such as fluorescent proteins or antibiotic resistance markers. The design and construction of targeting plasmids has been described elsewhere.

More recently, single-stranded DNA oligonucleotides (ssODNs) have been used in place of targeting plasmids for short modifications within a defined locus without cloning. To achieve high HDR efficiencies, ssODNs contain flanking sequences of at least 40 bp on each side that are homologous to the target region, and can be oriented in either the sense or antisense direction relative to the target locus.

Functional testing

SURVEYOR nuclease assay: Applicants detected indel mutations either by the SURVEYOR nuclease assay (or PCR amplicon sequencing. Applicants online CRISPR target design tool provides recommended primers for both approaches. However, SURVEYOR or sequencing primers may also be designed manually to amplify the region of interest from genomic DNA and to avoid non-specific amplicons using NCBI Primer-BLAST. SURVEYOR primers should be designed to amplify 300-400 bp (for a 600-800 bp total amplicon) on either side of the Cas9 target for allowing clear visualization of cleavage bands by gel electrophoresis. To prevent excessive primer dimer formation, SURVEYOR primers should be designed to be typically under 25-nt long with melting temperatures of ~60°C. Applicants recommend testing each pair of candidate primers for specific PCR amplicons as well as for the absence of non-specific cleavage during the SURVEYOR nuclease digestion process.

Plasmid- or ssODN-mediated HDR: HDR can be detected via PCR-amplification and sequencing of the modified region. PCR primers for this purpose should anneal outside the region spanned by the homology arms to avoid false detection of residual repair template (HDR Fwd and Rev, Fig. 26). For ssODN-mediated HDR, SURVEYOR PCR primers can be used.

Detection of indels or HDR by sequencing: Applicants detected targeted genome modifications by either Sanger or next-generation deep sequencing (NGS). For the former, genomic DNA from modified region can be amplified using either SURVEYOR or HDR primers. Amplicons should be subcloned into a plasmid such as pUC19 for transformation; individual colonies can be sequenced to reveal clonal genotype.

Applicants designed next-generation sequencing (NGS) primers for shorter amplicons, typically in the 100-200 bp size range. For detecting NHEJ mutations, it is important to design primers with at least 10-20 bp between the priming regions and the Cas9 target site to allow detection of longer indels. Applicants provide guidelines for a two-step PCR method to attach barcoded adapters for multiplex deep sequencing. Applicants recommend the Illumina platform, due to its generally low levels of false positive indels. Off-target analysis (described previously) can then be performed through read alignment programs such as ClustalW, Geneious, or simple sequence analysis scripts.

Materials and Reagents

sgRNA **preparation:**

UltraPure DNaseRNase-free distilled water (Life Technologies, cat. no. 10977-023)
Herculase II fusion polymerase (Agilent Technologies, cat. no. 600679)
CRITICAL. Standard Taq polymerase, which lacks 3'-5' exonuclease proofreading activity, has lower fidelity and can lead to amplification errors. Herculase II is a high-fidelity polymerase (equivalent fidelity to Pfu) that produces high yields of PCR product with minimal optimization. Other high-fidelity polymerases may be substituted.

Herculase II reaction buffer (5x; Agilent Technologies, included with polymerase)
dNTP solution mix (25 mM each; Enzymatics, cat. no. N205L)
MgC12 (25mM; ThermoScientific, cat. no. R0971)
QIAquick gel extraction kit (Qiagen, cat. no. 28704)
QIAprep spin miniprep kit (Qiagen, cat. no. 27106)
UltraPure TBE buffer (10X; Life Technologies, cat. no. 15581-028)
SeaKem LE agarose (Lonza, cat. no. 50004)
SYBR Safe DNA stain (10,000x; Life Technologies, cat. no. S33102)
1-kb Plus DNA ladder (Life Technologies, cat. no. 10787-018)
TrackIt CyanOrange loading buffer (Life Technologies, cat. no. 10482-028)
FastDigest BbsI (Bpil) (Fermentas/ThermoScientific, cat. no. FD1014)
Fermentas Tango Buffer (Fermentas/ThermoScientific, cat. no. BY5)
DL-dithiothreitol (DTT; Fermentas/ThermoScientific, cat. no. R0862)
T7 DNA ligase (Enzymatics, cat. no. L602L)
Critical:Do not substitute the more commonly used T4 ligase. T7 ligase has 1,000-fold higher activity on the sticky ends than on the blunt ends and higher overall activity than commercially available concentrated T4 ligases.
T7 2X Rapid Ligation Buffer (included with T7 DNA ligase, Enzymatics, cat. no. L602L)
T4 Polynucleotide Kinase (New England Biolabs, cat. no M0201S)
T4 DNA Ligase Reaction Buffer (10X; New England Biolabs, cat. no B0202S)
Adenosine 5'-triphosphate (10 mM; New England Biolabs, cat. no. P0756S)
PlasmidSafe ATP-dependent DNase (Epicentre, cat. no. E3101K)
One Shot Stbl3 chemically competent Escherichia coli (E. coli) (Life Technologies, cat. no. C7373-03)
SOC medium (New England Biolabs, cat. no. B9020S)
LB medium (Sigma, cat. no. L3022)
LB agar medium (Sigma, cat. no. L2897)
Ampicillin, sterile filtered (100 mg ml-1; Sigma, cat. no. A5354)

**Mammalian cell culture:**

HEK293FT cells (Life Technologies, cat. no. R700-07)
Dulbecco's minimum Eagle's medium (DMEM, IX, high glucose; Life Technologies, cat. no. 10313-039)
Dulbecco's minimum Eagle's medium (DMEM, IX, high glucose, no phenol red; Life Technologies, cat. no. 31053-028)
Dulbecco's phosphate-buffered saline (DPBS, 1X; Life Technologies, cat. no. 14190-250)
Fetal bovine serum, qualified and heat inactivated (Life Technologies, cat. no. 10438-034)
Opti-MEM I reduced-serum medium (FBS; Life Technologies, cat. no. 11058-021)
Penicillin-streptomycin (100x; Life Technologies, cat. no. 15140-163)
TrypLE™ Express (IX, no Phenol Red; Life Technologies, cat. no. 12604-013)
Lipofectamine 2000 transfection reagent (Life Technologies, cat. no. 11668027)
Amaxa SF Cell Line 4D-Nucleofector® X Kit S (32 RCT; Lonza, cat. no V4XC-2032)
HUES 9 cell line (HARVARD STEM CELL SCIENCE)
Geltrex LDEV-Free Reduced Growth Factor Basement Membrane Matrix (Life Technologies, cat. no. A1413201)
mTeSR1 medium (Stemcell Technologies, cat. no. 05850)
Accutase cell detachment solution (Stemcell Technologies, cat. no. 07920)
ROCK Inhibitor (Y-27632; Millipore, cat. no. SCM075)

Amaxa P3 Primary Cell 4D-Nucleofector® X Kit S (32 RCT; Lonza cat. no. V4XP-3032)

**Genotyping analysis:**

QuickExtract DNA extraction solution (Epicentre, cat. no. QE09050)
PCR primers for SURVEYOR, RFLP analysis, or sequencing (see Primer table)
Herculase II fusion polymerase (Agilent Technologies, cat. no. 600679)
CRITICAL. As Surveyor assay is sensitive to single-base mismatches, it is particularly important to use a high-fidelity polymerase. Other high-fidelity polymerases may be substituted.
Herculase II reaction buffer (5x; Agilent Technologies, included with polymerase)
dNTP solution mix (25 mM each; Enzymatics, cat. no. N205L)
QIAquick gel extraction kit (Qiagen, cat. no. 28704)
Taq Buffer (10x; Genscript, cat. no. B0005)
SURVEYOR mutation detection kit for standard gel electrophoresis (Transgenomic, cat. no. 706025)
UltraPure TBE buffer (10x; Life Technologies, cat. no. 15581-028)
SeaKem LE agarose (Lonza, cat. no. 50004)
4-20% TBE Gels 1.0 mm, 15 Well (Life Technologies, cat. no. EC62255BOX)
Novex® Hi-Density TBE Sample Buffer (5X; Life Technologies, cat. no. LC6678)
SYBR Gold Nucleic Acid Gel Stain (10,000X; Life Technologies, cat. no. S-11494)
1-kb Plus DNA ladder (Life Technologies, cat. no. 10787-018)
TrackIt CyanOrange loading buffer (Life Technologies, cat. no. 10482-028)
FastDigest HindIII (Fermentas/ThermoScientific, cat. no. FD0504)

**Equipment**

Filtered sterile pipette tips (Corning)
Standard 1.5ml microcentrifuge tubes (Eppendorf, cat. no. 0030 125.150)
Axygen 96-well PCR plates (VWR, cat. no. PCR-96M2-HSC)
Axygen 8-Strip PCR tubes (Fischer Scientific, cat. no. 14-222-250)
Falcon tubes, polypropylene, 15 ml (BD Falcon, cat. no. 352097)
Falcon tubes, polypropylene, 50 ml (BD Falcon, cat. no. 352070)
Round-bottom Tube with cell strainer cap, 5ml (BD Falcon, cat. no. 352235)
Petri dishes (60 mm × 15 mm; BD Biosciences, cat. no. 351007)
Tissue culture plate (24 well; BD Falcon, cat. no. 353047)
Tissue culture plate (96 well, flat bottom; BD Falcon, cat. no. 353075)
Tissue culture dish (100 mm; BD Falcon, 353003)
96-well thermocycler with programmable temperature stepping functionality (Applied Biosystems Veriti, cat. no. 4375786).
Desktop microcentrifuges 5424, 5804 (Eppendorf)
Gel electrophoresis system (PowerPac basic power supply, Bio-Rad, cat. no. 164-5050, and Sub-Cell GT System gel tray, Bio-Rad, cat. no. 170-4401)
Novex XCell SureLock Mini-Cell (Life Technologies, cat. no. EI0001)
Digital gel imaging system (GelDoc EZ, Bio-Rad, cat. no. 170-8270, and blue sample tray, Bio-Rad, cat. no. 170-8273)
Blue light transilluminator and orange filter goggles (SafeImager 2.0; Invitrogen, cat. no. G6600)
Gel quantification software (Bio-Rad, ImageLab, included with GelDoc EZ,or open-source ImageJ from the National Institutes of Health, available at the website rsbweb.nih.gov/ij/) UV spectrophotometer (NanoDrop 2000c, Thermo Scientific)

**Reagent Setup**

Tris-borate EDTA (TBE) electrophoresis solution Dilute TBE buffer in distilled water to 1X working solution for casting agarose gels and for use as a buffer for gel electrophoresis. Buffer may be stored at room temperature (18 - 22 °C) for at least 1 year.

- ATP, 10 mM Divide 10 mM ATP into 50-μl aliquots and store at - 20 °C for up to 1 year; avoid repeated freeze-thaw cycles.
- DTT, 10 mM Prepare 10 mM DTT solution in distilled water and store in 20-μl aliquots at - 70 °C for up to 2 years; for each reaction, use a new aliquot, as DTT is easily oxidized.

- D10 culture medium For culture of HEK293FT cells, prepare D10 culture medium by supplementing DMEM with 1X GlutaMAX and 10% (vol/vol) fetal bovine serum. As indicated in the protocol, this medium can also be supplemented with 1X penicillin-streptomycin . D10 medium can be made in advance and stored at 4 °C for up to 1 month.
- mTeSR1 culture medium For culture of human embryonic stem cells, prepare mTeSR1 medium by supplementing the 5X supplement (included with mTeSR1 basal medium), and 100 ug/ml Normocin.

**Procedure**

Design of targeting components and use of the online tool • Timing 1 d

1| Input target genomic DNA sequence. Applicants provide an online Cas9 targeting design tool that takes an input sequence of interest, identifies and ranks suitable target sites, and computationally predicts off-target sites for each intended target. Alternatively, one can manually select guide sequence by identifying the 20-bp sequence directly upstream of any 5'-NGG.
2| Order necessary oligos and primers as specified by the online tool. If the site is chosen manually, the oligos and primers should be designed.

Preparation of sgRNA expression construct

3| To generate the sgRNA expression construct, either the PCR- or plasmid-based protocol can be used.

(A) via PCR amplification • Timing 2 h

(i) Applicants prepare diluted U6 PCR template. Applicants recommend using PX330 as a PCR template, but any U6-containing plasmid may likewise be used as the PCR template. Applicants diluted template with $ddH_2O$ to a concentration of 10 ng/ul. Note that if a plasmid or cassette already containing an U6-driven sgRNA is used as a template, a gel extraction needs to be performed to ensure that the product contains only the intended sgRNA and no trace sgRNA carryover from template.
(ii) Applicants prepared diluted PCR oligos. U6-Fwd and U6-sgRNA-Rev primers are diluted to a final concentration of 10 uM in $ddH_2O$ (add 10 ul of 100 uM primer to 90 ul $ddH_2O$).
(iii) U6-sgRNA PCR reaction. Applicants set up the following reaction for each U6-sgRNA-Rev primer and mastermix as needed:

| Component: | Amount (ul) | Final concentration |
|---|---|---|
| Herculase II PCR buffer, 5X | 10 | 1X |
| dNTP, 100mM (25mM each) | 0.5 | 1 mM |
| U6 template (PX330) | 1 | 0.2 ng/ul |
| U6-Fwd primer | 1 | 0.2 uM |
| U6-sgRNA-Rev primer (variable) | 1 | 0.2 uM |
| Herculase II Fusion polymerase | 0.5 | |
| Distilled water | 36 | |
| Total | 50 | |

(iv) Applicants performed PCR reaction on the reactions from step (iii) using the following cycling conditions:

| Cycle number | Denature | Anneal | Extend |
|---|---|---|---|
| 1 | 95°C, 2 m | | |
| 2-31 | 95°C, 20 s | 60°C, 20 s | 72°C, 20 s |
| 32 | | | 72°C, 3 m |

(v) After the reaction is completed, Applicants ran the product on a gel to verify successful, single-band amplification. Cast a 2% (wt/vol) agarose gel in 1X TBE buffer with 1X SYBR Safe dye. Run 5 ul of the PCR product in the gel at 15 V cm-1 for 20-30 min. Successful amplicons should yield one single 370-bp product and the template should be invisible.

It should not be necessary to gel extract the PCR amplicon.
(vi) Applicants purified the PCR product using the QIAquick PCR purification kit according to the manufacturer's directions. Elute the DNA in 35 ul of Buffer EB or water. Purified PCR products may be stored at 4°C or -20°C.

**(B) Cloning sgRNA into Cas9-containing bicistronic expression vector • Timing 3 d**

(i) *Prepare the sgRNA oligo inserts.* Applicants resuspended the top and bottom strands of oligos for each sgRNA design to a final concentration of 100 uM. Phosphorylate and anneal the oligo as follows:

| | |
|---|---|
| Oligo 1 (100 uM) | 1 ul |
| Oligo 2 (100 uM) | 1 ul |
| T4 Ligation Buffer, 10X | 1 ul |
| T4 PNK | 1 ul |
| $ddH_2O$ | 6 ul |
| Total | 10 ul |

(ii) Anneal in a thermocycler using the following parameters:

37°C for 30 m
95°C for 5 m
Ramp down to 25°C at 5°C per m

(iii) Applicants diluted phosphorylated and annealed oligos 1:200 by add 1ul of oligo to 199 ul room temperature $ddH_2O$.
(iv) *Clone sgRNA oligo into PX330.* Applicants set up Golden Gate reaction for each sgRNA. Applicants recommend also setting up a no-insert, PX330 only negative control.

| | |
|---|---|
| PX330 (100 ng) | x ul |
| Diluted oligo duplex from step (iii) | 2 ul |
| Tango Buffer, 10X | 2 ul |
| DTT, 10mM | 1 ul |
| ATP, 10mM | 1 ul |
| FastDigest BbsI | 1 ul |
| T7 Ligase | 0.5 ul |
| $ddH_2O$ | x ul |
| Total | 20 ul |

(v) Incubate the Golden Gate reaction for a total of 1 h:

| Cycle number | Condition |
|---|---|
| 1-6 | 37°C for 5 m, 21°C for 5 m |

(vi) Applicants treated Golden Gate reaction with PlasmidSafe exonuclease to digest any residual linearized DNA. This step is optional but highly recommended.

| | |
|---|---|
| Golden Gate reaction from step 4 | 11 ul |
| 10X PlasmidSafe Buffer | 1.5 ul |
| ATP, 10 mM | 1.5 ul |
| PlasmidSafe exonuclease | 1 ul |
| Total | 15 ul |

(vii) Applicants incubated the PlasmidSafe reaction at 37°C for 30 min, followed by inactivation at 70°C for 30 min. **Pause point:** after completion, the reaction may be frozen and continued later. The circular DNA should be stable for at least 1 week.

(viii) *Transformation.* Applicants transformed the PlasmidSafe-treated plasmid into a competent *E. coli* strain, according to the protocol supplied with the cells. Applicants recommend Stbl3 for quick transformation. Briefly, Applicants added 5ul of the product from step (vii) into 20ul of ice-cold chemically competent Stbl3 cells. This is then incubated on ice for 10 m, heat shocked at 42°C for 30 s, returned immediately to ice for 2 m, 100 ul of SOC medium is added, and this is plated onto an LB plate containing 100 ug/ml ampicillin with incubation overnight at 37°C.

(ix) Day 2: Applicants inspected plates for colony growth. Typically, there are no colonies on the negative control plates (ligation of BbsI-digested PX330 only, no annealed sgRNA oligo), and tens to hundreds of colonies on the PX330-sgRNA cloning plates.

(x) From each plate, Applicants picked 2-3 colonies to check correct insertion of sgRNA. Applicants used a sterile pipette tip to inoculate a single colony into a 3 ml culture of LB medium with 100 ug/ml ampicillin. Incubate and shake at 37°C overnight.

(xi) Day 3: Applicants isolated plasmid DNA from overnight cultures using a QiAprep Spin miniprep kit according to the manufacturer's instructions.

(xii) *Sequence validate CRISPR plasmid.* Applicants verified the sequence of each colony by sequencing from the U6 promoter using the U6-Fwd primer. Optional: sequence the Cas9 gene using primers listed in the following Primer table.

| Primer | Sequence (5' to 3') | Purpose |
|---|---|---|
| U6-For | GAGGGCCTATTTCCCATGATTCC | Amplify U6-sgRNA |
| U6-Rev | AAAAAAAGCACCGACTCGGTGCCACTTTTTCAAGTTGA TAACGGACTAGCCTTATTTTAACTTGCTATTTCTAG CTCTAAAACNNNNNNNNNNNNNNNNNNNNCCGGTGTTTC GTCCTTTCCACAAG | Amplify U6-sgRNA; N is reverse complement of target |
| sgRNA-top | CACCGNNNNNNNNNNNNNNNNNNNN | Clone sgRNA into PX330 |
| sgRNA-bottom | AAACNNNNNNNNNNNNNNNNNNNNC | Clone sgRNA into PX330 |
| U6-*EMX1*-Rev | AAAAAAAGCACCGACTCGGTGCCACTTTTTCAAGTTGA TAACGGACTAGCCTTATTTTAACTTGCTATTTCTAG CTCTAAAACCCCTAGTCATTGGAGGTGACCGGTGTTTCG TCCTTTCCACAAG | Amplify U6-EMX1 sgRNA |
| *EMX1*-top | CACCGTCACCTCCAATGACTAGGG | Clone EMX1 sgRNA into PX330 |
| *EMX1*-bottom | AAACCCCTAGTCATTGGAGGTGAC | Clone EMX1 sgRNA into PX330 |
| ssODN-sense | CAGAAGAAGAAGGGCTCCCATCACATCAACCGGTGGCG CATTGCCACGAAGCAGGCCAATGGGGAGGACATC GATGTCACCTCCAATGACAAGCTTGCTAGCGGTGGGCA ACCACAAACCCACGAGGGCAGAGTGCTGCTTGCTG CTGGCCAGGCCCCTGCGTGGGCCCAAGCTGGACTCTGG CCACTCCCT | EMX1 HDR (sense; insertion underlined) |
| ssODN-antisense | AGGGAGTGGCCAGAGTCCAGCTTGGGCCCACGCAGGGG CCTGGCCAGCAGCAAGCAGCACTCTGCCCTCGTG GGTTTGTGGTTGCCCACCGCTAGCAAGCTTGTCATTGGA GGTGACATCGATGTCCTCCCCATTGGCCTGCTTCG TGGCAATGCGCCACCGGTTGATGTGATGGGAGCCCTTC TTCTTCTG | EMX1 HDR (antisense; insertion underlined) |
| EMX1-SURV-F | CCATCCCCTTCTGTGAATGT | EMX1 SURVEYOR assay PCR, sequencing |
| EMX1-SURV-R | GGAGATTGGAGACACGGAGA | EMX1 SURVEYOR assay PCR, sequencing |
| EMX1-HDR-F | GGCTCCCTGGGTTCAAAGTA | EMX1 RFLP analysis PCR, sequencing |

(continued)

| Primer | Sequence (5' to 3') | Purpose |
|---|---|---|
| EMX1-HDR-R | AGAGGGGTCTGGATGTCGTAA | EMX1 RFLP analysis PCR, sequencing |
| pUC19-F | CGCCAGGGTTTTCCCAGTCACGAC | pUC19 multiple cloning site F primer, for Sanger sequencing |

Applicants referenced the sequencing results against the PX330 cloning vector sequence to check that the 20 bp guide sequence was inserted between the U6 promoter and the remainder of the sgRNA scaffold. Details and sequence of the PX330 map in GenBank vector map format (*.gb file) can be found at the website crispr.genome-engineering.org.

**(Optional) Design of ssODN template • Timing 3 d planning ahead**

3| *Design and order ssODN.* Either the sense or antisense ssODN can be purchased directly from supplier. Applicants recommend designing homology arms of at least 40 bp on either side and 90 bp for optimal HDR efficiency. In Applicants' experience, antisense oligos have slightly higher modification efficiencies.
4| Applicants resuspended and diluted ssODN ultramers to a final concentration of 10 uM. Do not combine or anneal the sense and antisense ssODNs. Store at -20°C.
5| Note for HDR applications, Applicants recommend cloning sgRNA into the PX330 plasmid.

**Functional validation of sgRNAs: cell culture and transfections • Timing 3-4 d**

The CRISPR-Cas system has been used in a number of mammalian cell lines. Conditions may vary for each cell line. The protocols below details transfection conditions for HEK239FT cells. Note for ssODN-mediated HDR transfections, the Amaxa SF Cell Line Nucleofector Kit is used for optimal delivery of ssODNs. This is described in the next section.
7| *HEK293FT maintenance.* Cells are maintained according to the manufacturer's recommendations. Briefly, Applicants cultured cells in D10 medium (GlutaMax DMEM supplemented with 10% Fetal Bovine Serum), at 37°C and 5% CO2.
8| To passage, Applicants removed medium and rinsed once by gently adding DPBS to side of vessel, so as not to dislodge cells. Applicants added 2 ml of TrypLE to a T75 flask and incubated for 5 m at 37°C. 10 ml of warm D10 medium is added to inactivate and transferred to a 50 ml Falcon tube. Applicants dissociated cells by triturating gently, and re-seeded new flasks as necessary. Applicants typically passage cells every 2-3 d at a split ratio of 1:4 or 1:8, never allowing cells to reach more than 70% confluency. Cell lines are restarted upon reaching passage number 15.
9| *Prepare cells for transfection.* Applicants plated well-dissociated cells onto 24-well plates in D10 medium without antibiotics 16-24 h before transfection at a seeding density of 1.3 x $10^5$ cells per well and a seeding volume of 500 ul. Scale up or down according to the manufacturer's manual as needed. It is suggested to not plate more cells than recommended density as doing so may reduce transfection efficiency.
10| On the day of transfection, cells are optimal at 70-90% confluency. Cells may be transfected with Lipofectamine 2000 or Amaxa SF Cell Line Nucleofector Kit according to the manufacturers' protocols.
(A) For sgRNAs cloned into PX330, Applicants transfected 500 ng of sequence-verified CRISPR plasmid; if transfecting more than one plasmid, mix at equimolar ratio and no more than 500 ng total.
(B) For sgRNA amplified by PCR, Applicants mixed the following:

| | |
|---|---|
| PX165 (Cas9 only) | 200 ng |
| sgRNA amplicon (each) | 40 ng |
| pUC19 | fill up total DNA to 500 ng |

Applicants recommend transfecting in technical triplicates for reliable quantification and including transfection controls (e.g. GFP plasmid) to monitor transfection efficiency. In addition, PX330 cloning plasmid and/or sgRNA amplicon may be transfected alone as a negative control for downstream functional assays.

111 Applicants added Lipofectamine complex to cells gently as HEK293FT cells may detach easily from plate easily and result in lower transfection efficiency.
12| Applicants checked cells 24 h after transfection for efficiency by estimating the fraction of fluorescent cells in the control (e.g., GFP) transfection using a fluorescence microscope. Typically cells are more than 70% transfected.
13| Applicants supplemented the culture medium with an additional 500 ul of warm D10 medium. Add D10 very slowly to the side of the well and do not use cold medium, as cells can detach easily.
14| Cells are incubated for a total of 48-72 h post-transfection before harvested for indel analysis. Indel efficiency does not increase noticeably after 48 h.

**(Optional) Co-transfection of CRISPR plasmids and ssODNs or targeting plasmids for HR • Timing 3-4 d**

15| *Linearize targeting plasmid.* Targeting vector is linearized if possible by cutting once at a restriction site in the vector backbone near one of the homology arms or at the distal end of either homology arm.
16| Applicants ran a small amount of the linearized plasmid alongside uncut plasmid on a 0.8-1% agarose gel to check successful linearization. Linearized plasmid should run above the supercoiled plasmid.
17| Applicants purified linearized plasmid with the QIAQuick PCR Purification kit.
18| Prepare cells for transfection. Applicants cultured HEK293FT in T75 or T225 flasks. Sufficient cell count before day of transfection is planned for. For the Amaxa strip-cuvette format, 2 x $10^6$ cells are used per transfection.
19| Prepare plates for transfection. Applicants added 1 ml of warm D10 medium into each well of a 12 well plate. Plates are placed into the incubator to keep medium warm.
20| Nucleofection. Applicants transfected HEK293FT cells according to the Amaxa SF Cell Line Nucleofector 4D Kit manufacturer's instructions, adapted in the steps below.
a. For ssODN and CRISPR cotransfection, pre-mix the following DNA in PCR tubes:

| | |
|---|---|
| pCRISPR plasmid (Cas9 + sgRNA) | 500 ng |
| ssODN template (10uM) | 1 ul |

b. For HDR targeting plasmid and CRISPR cotransfection, pre-mix the following DNA in PCR tubes:

| | |
|---|---|
| CRISPR plasmid (Cas9 + sgRNA) | 500 ng |
| Linearized targeting plasmid | 500 ng |

For transfection controls, see previous section. In addition, Applicants recommend transfecting ssODN or targeting plasmid alone as a negative control.

21| Dissociate to single cells. Applicants removed medium and rinsed once gently with DPBS, taking care not to dislodge cells. 2 ml of TrypLE is added to a T75 flask and incubated for 5 m at 37°C. 10 ml of warm D10 medium is added to inactivate and triturated gently in a 50 ml Falcon tube. It is recommended that cells are triturated gently and dissociated to single cells. Large clumps will reduce transfection efficiency. Applicants took a 10 ul aliquot from the suspension and diluted into 90 ul of D10 medium for counting. Applicants counted cells and calculated the number of cells and volume of suspension needed for transfection. Applicants typically transfected 2 x $10^5$ cells per condition using the Amaxa Nucleocuvette strips, and recommend calculating for 20% more cells than required to adjust for volume loss in subsequent pipetting steps. The volume needed is transferred into a new Falcon tube.
23| Applicants spun down the new tube at 200 x g for 5 m.

Applicants prepared the transfection solution by mixing the SF solution and S1 supplement as recommended by Amaxa. For Amaxa strip-cuvettes, a total of 20 ul of supplemented SF solution is needed per transfection. Likewise, Applicants recommend calculating for 20% more volume than required.

25| Applicants removed medium completely from pelleted cells from step 23 and gently resuspended in appropriate volume (20 ul per 2 x $10^5$ cells) of S1-supplemented SF solution. Do not leave cells in SF solution for extended period of time.
26| 20 ul of resuspended cells is pipetted into each DNA pre-mix from step 20. Pipette gently to mix and transfer to Nucleocuvette strip chamber. This is repeated for each transfection condition.

Electroporate cells using the Nucleofector 4D program recommended by Amaxa, CM-130.

28| Applicants gently and slowly pipetted 100 ul of warm D10 medium into each Nucleocuvette strip chamber, and transferred all volume into the pre-warmed plate from step 19. CRITICAL. Cells are very fragile at this stage and harsh pipetting can cause cell death. Incubate for 24 h. At this point, transfection efficiency can be estimated from fraction of fluorescent cells in positive transfection control. Nucleofection typically results in greater than 70-80% transfection efficiency. Applicants slowly added 1 ml warm D10 medium to each well without dislodging the cells. Incubate cells for a total of 72 h.

**Human embryonic stem cell (HUES 9) culture and transfection • Timing 3-4 d**

Maintaining hESC (HUES9) line. Applicants routinely maintain HUES9 cell line in feeder-free conditions with mTesR1 medium. Applicants prepared mTeSR1 medium by adding the 5X supplement included with basal medium and 100 ug/ml Normocin. Applicants prepared a 10 ml aliquot of mTeSR1 medium supplemented further with 10 uM Rock Inhibitor. Coat tissue culture plate. Dilute cold GelTrex 1:100 in cold DMEM and coat the entire surface of a 100 mm tissue culture plate.

Place plate in incubator for at least 30 m at 37°C. Thaw out a vial of cells at 37°C in a 15 ml Falcon tube, add 5 ml of mTeSR1 medium, and pellet at 200 x g for 5 m. Aspirate off GelTrex coating and seed ~1 x 106 cells with 10 ml mTeSR1 medium containing Rock Inhibitor. Change to normal mTeSR1 medium 24 h after transfection and re-feed daily. Passaging cells. Re-feed cells with fresh mTeSR1 medium daily and passage before reaching 70% confluency. Aspirate off mTeSR1 medium and wash cells once with DPBS. Dissociate cells by adding 2 ml Accutase and incubating at 37°C for 3 - 5 m. Add 10 ml mTeSR1 medium to detached cells, transfer to 15 ml Falcon tube and resuspend gently. Re-plate onto GelTrex-coated plates in mTeSR1 medium with 10 uM Rock Inhibitor. Change to normal mTeSR1 medium 24 h after plating.

Transfection. Applicants recommend culturing cells for at least 1 week post-thaw before transfecting using the Amaxa P3 Primary Cell 4-D Nucleofector Kit (Lonza). Re-feed log-phase growing cells with fresh medium 2 h before transfection. Dissociate to single cells or small clusters of no more than 10 cells with accutase and gentle resuspension. Count the number of cells needed for nucleofection and spin down at 200 x g for 5 m. Remove medium completely and resuspend in recommended volume of S1-supplemented P3 nucleofection solution. Gently plate electroporated cells into coated plates in presence of 1X Rock Inhibitor.

Check transfection success and re-feed daily with regular mTeSR1 medium beginning 24 h after nucleofection. Typically, Applicants observe greater than 70% transfection efficiency with Amaxa Nucleofection. Harvest DNA. 48-72 h post transfection, dissociate cells using accutase and inactivate by adding 5 x volume of mTeSR1. Spin cells down at 200 x g for 5 m. Pelleted cells can be directed processed for DNA extraction with QuickExtract solution. It is recommended to not mechanically dissociate cells without accutase. It is recommended to not spin cells down without inactivating accutase or above the recommended speed; doing so may cause cells to lyse.

**Isolation of clonal cell lines by FACS. Timing • 2-3 h hands-on; 2-3 weeks expansion**

Clonal isolation may be performed 24 h post-transfection by FACS or by serial dilution.

54| *Prepare FACS buffer.* Cells that do not need sorting using colored fluorescence may be sorted in regular D10 medium supplemented with 1X penicillin/streptinomycin. If colored fluorescence sorting is also required, a phenol-free DMEM or DPBS is substituted for normal DMEM. Supplement with 1X penicillin/streptinomycin and filter through a .22 um Steriflip filter.

55| *Prepare 96 well plates.* Applicants added 100 ul of D10 media supplemented with 1X penicillin/streptinomycin per well and prepared the number of plates as needed for the desired number of clones.

56| *Prepare cells for FACS.* Applicants dissociated cells by aspirating the medium completely and adding 100 ul TrypLE per well of a 24-well plate. Incubate for 5 m and add 400 ul warm D10 media.

57| Resuspended cells are transferred into a 15 ml Falcon tube and gently triturated 20 times. Recommended to check under the microscope to ensure dissociation to single cells.

58| Spin down cells at 200 x g for 5 minutes.

59| Applicants aspirated the media, and resuspended the cells in 200 ul of FACS media.

60| Cells are filtered through a 35 um mesh filter into labeled FACS tubes. Applicants recommend using the BD Falcon 12 x 75 mm Tube with Cell Strainer cap. Place cells on ice until sorting.

61| Applicants sorted single cells into 96-well plates prepared from step 55. Applicants recommend that in one single designated well on each plate, sort 100 cells as a positive control.

NOTE. The remainder of the cells may be kept and used for genotyping at the population level to gauge overall modification efficiency.

62| Applicants returned cells into the incubator and allowed them to expand for 2-3 weeks. 100 ul of warm D10 medium is added 5 d post sorting. Change 100 ul of medium every 3-5 d as necessary.

63| Colonies are inspected for "clonal" appearance 1 week post sorting: rounded colonies radiating from a central point. Mark off wells that are empty or may have been seeded with doublets or multiplets.

64| When cells are more than 60% confluent, Applicants prepared a set of replica plates for passaging. 100 ul of D10 medium is added to each well in the replica plates. Applicants dissociated cells directly by pipetting up and down vigorously 20 times. 20% of the resuspended volume was plated into the prepared replica plates to keep the clonal lines. Change the medium every 2-3 d thereafter and passage accordingly.

65| Use the remainder 80% of cells for DNA isolation and genotyping.

**Optional: Isolation of clonal cell lines by dilution. Timing • 2-3 h hands-on; 2-3 weeks expansion**

66| Applicants dissociated cells from 24-well plates as described above. Make sure to dissociate to single cells. A cell strainer can be used to prevent clumping of cells.

67| The number of cells are counted in each condition. Serially dilute each condition in D10 medium to a final concentration of 0.5 cells per 100 ul. For each 96 well plate, Applicants recommend diluting to a final count of 60 cells in 12 ml of D10. Accurate count of cell number is recommended for appropriate clonal dilution. Cells may be recounted at an intermediate serial dilution stage to ensure accuracy.

68| Multichannel pipette was used to pipette 100 ul of diluted cells to each well of a 96 well plate.

NOTE. The remainder of the cells may be kept and used for genotyping at the population level to gauge overall modification efficiency.

69| Applicants inspected colonies for "clonal" appearance ~1 week post plating: rounded colonies radiating from a central point. Mark off wells that may have seeded with doublets or multiplets.

70| Applicants returned cells to the incubator and allowed them to expand for 2-3 weeks. Re-feed cells as needed as detailed in previous section.

**SURVEYOR assay for CRISPR cleavage efficiency. Timing • 5-6 h**

71| *Harvest cells for DNA.* Dissociate cells and spin down at 200 x g for 5 m. NOTE. Replica plate at this stage as needed to keep transfected cell lines.

72| Aspirate the supernatant completely.

Before assaying cleavage efficiency of transfected cells, Applicants recommend testing each new SURVEYOR primer on negative (untransfected) control samples through the step of SURVEYOR nuclease digestion using the protocol described below. Occasionally, even single-band clean SURVEYOR PCR products can yield non-specific SURVEYOR nuclease cleavage bands and potentially interfere with accurate indel analysis.

73| Applicants used QuickExtract DNA extraction solution according to the manufacturer's instructions. Applicants typically used 50 ul of the solution for each well of a 24 well plate and 10 ul for a 96 well plate.

74| Applicants normalized extracted DNA to a final concentration of 100-200 ng/ul with ddH2O. **Pause point:** Extracted DNA may be stored at -20°C for several months.

75| *Set up the SURVEYOR PCR.* Master mix the following using SURVEYOR primers provided by Applicants online/computer algorithm tool:

| Component: | Amount (ul) | Final concentration |
|---|---|---|
| Herculase II PCR buffer, 5X | 10 | 1X |
| dNTP, 100mM (25mM each) | 1 | 1 mM |
| SURVEYOR Fwd primer (10uM) | 1 | 0.2 uM |
| SURVEYOR Rev primer (10uM) | 1 | 0.2 uM |
| Herculase II Fusion polymerase | 1 | |
| $MgCl_2$ (25mM) | 2 | 1 mM |
| Distilled water | 33 | |
| Total | 49 (for each reaction) | |

76| Applicants added 100-200 ng of normalized genomic DNA template from step 74 for each reaction.

77| PCR reaction was performed using the following cycling conditions, for no more than 30 amplification cycles:

| Cycle number | Denature | Anneal | Extend |
|---|---|---|---|
| 1 | 95°C, 2 min | | |

(continued)

| Cycle number | Denature | Anneal | Extend |
|---|---|---|---|
| 2-31 | 95°C, 20 s | 60°C, 20 s | 72°C, 30 s |
| 32 | | | 72°C, 3 min |

78| Applicants ran 2-5 ul of PCR product on a 1% gel to check for single-band product. Although these PCR conditions are designed to work with most pairs of SURVEYOR primers, some primers may need additional optimization by adjusting the template concentration, $MgCl_2$ concentration, and/or the annealing temperature.
79| Applicants purified the PCR reactions using the QIAQuick PCR purification kit and normalized eluant to 20 ng/ul. **Pause point:** Purified PCR product may be stored at -20°C.
80| *DNA heteroduplex formation.* The annealing reaction was set up as follows:

| | |
|---|---|
| Taq PCR buffer, 10X | 2 ul |
| Normalized DNA (20 ng/ul) | 18 ul |
| Total volume | 20 ul |

81| Anneal the reaction using the following conditions:

| Cycle number | Condition |
|---|---|
| 1 | 95°C, 10 mn |
| 2 | 95°C-85°C, -2°C/s |
| 3 | 85°C, 1 min |
| 4 | 85°C-75°C, -0.3°C/s |
| 5 | 75°C, 1 min |
| 6 | 75°C-65°C, -0.3°C/s |
| 7 | 65°C, 1 min |
| 8 | 65°C-55°C, -0.3°C/s |
| 9 | 55°C, 1 min |
| 10 | 55°C-45°C, -0.3°C/s |
| 11 | 45°C, 1 min |
| 12 | 45°C-35°C, -0.3°C/s |
| 13 | 35°C, 1 min |
| 14 | 35°C-25°C, -0.3°C/s |
| 15 | 25°C, 1 min |

82| *SURVEYOR nuclease S digestion.* Applicants prepared master-mix and added the following components on ice to annealed heteroduplexes from step 81 for a total final volume of 25 ul:

| Component | Amount (ul) | Final Concentration |
|---|---|---|
| $MgCl_2$ solution, 0.15M | 2.5 | 15mM |
| $ddH_2O$ | 0.5 | |
| SURVEYOR nuclease S | 1 | 1X |
| SURVEYOR enhancer S | 1 | 1X |
| Total | 5 | |

83| Vortex well and spin down. Incubate the reaction at 42°C for 1 h.
84| Optional: 2 ul of the Stop Solution from the SURVEYOR kit may be added. **Pause point.** The digested product may be stored at -20°C for analysis at a later time.
85| *Visualize the SURVEYOR reaction.* SURVEYOR nuclease digestion products may be visualized on a 2% agarose gel. For better resolution, products may be run on a 4-20% gradient Polyacrylamide TBE gel. Applicants loaded 10 ul of product with the recommended loading buffer and ran the gel according to manufacturer's instructions. Typically, Applicants run until the bromophenol blue dye has migrated to the bottom of the gel. Include DNA ladder and negative controls on the same gel.

86| Applicants stained the gel with 1X SYBR Gold dye diluted in TBE. The gel was gently rocked for 15 m.

87| Applicants imaged the gel using a quantitative imaging system without overexposing the bands. The negative controls should have only one band corresponding to the size of the PCR product, but may have occasionally non-specific cleavage bands of other sizes. These will not interfere with analysis if they are different in size from target cleavage bands. The sum of target cleavage band sizes, provided by Applicants online/computer algorithm tool, should be equal to the size of the PCR product.

88| *Estimate the cleavage intensity.* Applicants quantified the integrated intensity of each band using ImageJ or other gel quantification software.

89| For each lane, Applicants calculated the fraction of the PCR product cleaved ($f_{cut}$) using the following formula: $f_{cut} = (b + c) / (a + b + c)$, where a is the integrated intensity of the undigested PCR product and b and c are the integrated intensities of each cleavage product. 90| Cleavage efficiency may be estimated using the following formula, based on the binomial probability distribution of duplex formation:

91|

$$indel\,(\%) = 100 \times (1 - \sqrt{(1 - f_{cut})})$$

**Sanger sequencing for assessing CRISPR cleavage efficiency. Timing • 3 d**

Initial steps are identical to Steps 71-79 of the SURVEYOR assay. Note: SURVEYOR primers may be used for Sanger sequencing if appropriate restriction sites are appended to the Forward and Reverse primers. For cloning into the recommended pUC19 backbone, EcoRI may be used for the Fwd primer and HindIII for the Rev primer.

92| *Amplicon digestion.* Set up the digestion reaction as follows:

| Component | Amount (ul) |
|---|---|
| Fast Digest buffer, 10X | 3 |
| FastDigest EcoRI | 1 |
| FastDigest HindIII | 1 |
| Normalized DNA (20 ng/ul) | 10 |
| $ddH_2O$ | 15 |
| Total volume | 30 |

93| pUC19 backbone digestion. Set up the digestion reaction as follows:

| Component | Amount (ul) |
|---|---|
| Fast Digest buffer, 10X | 3 |
| FastDigest EcoRI | 1 |
| FastDigest HindIII | 1 |
| FastAP Alkaline Phosphatase | 1 |
| pUC19 vector (200 ng/ul) | 5 |
| $ddH_2O$ | 20 |
| Total volume | 30 |

94| Applicants purified the digestion reactions using the QIAQuick PCR purification kit. **Pause point:** Purified PCR product may be stored at -20°C.

95| Applicants ligated the digested pUC19 backbone and Sanger amplicons at a 1:3 vector:insert ratio as follows:

| Component | Amount (ul) |
|---|---|
| Digested pUC19 | x (50 ng) |
| Digested insert | x (1:3 vector:insert molar ratio) |
| T7 ligase | 1 |
| 2X Rapid Ligation Buffer | 10 |

(continued)

| Component | Amount (ul) |
|---|---|
| $ddH_2O$ | x |
| Total volume | 20 |

96| *Transformation.* Applicants transformed the PlasmidSafe-treated plasmid into a competent *E. coli* strain, according to the protocol supplied with the cells. Applicants recommend Stbl3 for quick transformation. Briefly, 5ul of the product from step 95 is added into 20ul of ice-cold chemically competent Stbl3 cells, incubated on ice for 10 m, heat shocked at 42°C for 30 s, returned immediately to ice for 2 m, 100 ul of SOC medium is added, and plated onto an LB plate containing 100 ug/ml ampicillin. This is incubated overnight at 37°C.
97| Day 2: Applicants inspected plates for colony growth. Typically, there are no colonies on the negative control plates (ligation of EcoRI-HindIII digested pUC19 only, no Sanger amplicon insert), and tens to hundreds of colonies on the pUC19-Sanger amplicon cloning plates.
98| Day 3: Applicants isolated plasmid DNA from overnight cultures using a QIAprep Spin miniprep kit according to the manufacturer's instructions.
99| *Sanger sequencing.* Applicants verified the sequence of each colony by sequencing from the pUC19 backbone using the pUC19-For primer. Applicants referenced the sequencing results against the expected genomic DNA sequence to check for the presence of Cas9-induced NHEJ mutations. % editing efficiency = (# modified clones)/(# total clones). It is important to pick a reasonable number of clones (>24) to generate accurate modification efficiencies.

**Genotyping for microdeletion. Timing • 2-3 d hands on; 2-3 weeks expansion**

100| Cells were transfected as described above with a pair of sgRNAs targeting the region to be deleted.
101| 24 h post-transfection, clonal lines are isolated by FACS or serial dilution as described above.
102| Cells are expanded for 2-3 weeks.
103| Applicants harvested DNA from clonal lines as described above using 10 ul QuickExtract solution and normalized genomic DNA with $ddH_2O$ to a final concentration of 50-100 ng/ul.
104| *PCR Amplify the modified region.* The PCR reaction is set up as follows:

| Component: | Amount (ul) | Final concentration |
|---|---|---|
| Herculase II PCR buffer, 5X | 10 | 1X |
| dNTP, 100mM (25mM each) | 1 | 1 mM |
| Out Fwd primer (10uM) | 1 | 0.2 uM |
| Out Rev primer (10uM) | 1 | 0.2 uM |
| Herculase II Fusion polymerase | 1 | |
| MgCl2 (25mM) | 2 | 1 mM |
| $ddH_2O$ | 32 | |
| Total | 48 (for each reaction) | |

Note: if deletion size is more than 1 kb, set up a parallel set of PCR reactions with In-Fwd and In-Rev primers to screen for the presence of the wt allele.

105| To screen for inversions, a PCR reaction is set up as follows:

| Component: | Amount (ul) | Final concentration |
|---|---|---|
| Herculase II PCR buffer, 5X | 10 | 1X |
| dNTP, 100mM (25mM each) | 1 | 1 mM |
| Out Fwd *or* Out-Rev primer (10uM) | 1 | 0.2 uM |
| In Fwd *or* In-Rev primer (10uM) | 1 | 0.2 uM |
| Herculase II Fusion polymerase | 1 | |
| $MgCl_2$ (25mM) | 2 | 1 mM |
| $ddH_2O$ | 32 | |
| Total | 48 (for each reaction) | |

Note: primers are paired either as Out-Fwd + In Fwd, or Out-Rev + In-Rev.

106| Applicants added 100-200 ng of normalized genomic DNA template from step 103 for each reaction.
107| PCR reaction was performed using the following cycling conditions:

| Cycle number | Denature | Anneal | Extend |
|---|---|---|---|
| 1 | 95°C, 2 min | | |
| 2-31 | 95°C, 20 s | 60°C, 20 s | 72°C, 30 s |
| 32 | | | 72°C, 3 m |

108| Applicants run 2-5 ul of PCR product on a 1-2% gel to check for product. Although these PCR conditions are designed to work with most primers, some primers may need additional optimization by adjusting the template concentration, $MgCl_2$ concentration, and/or the annealing temperature.

**Genotyping for targeted modifications via HDR. Timing • 2-3 d, 2-3 h hands on**

109| Applicants harvested DNA as described above using QuickExtract solution and normalized genomic DNA with TE to a final concentration of 100-200 ng/ul.
110| *PCR Amplify the modified region.* The PCR reaction is set up as follows:

| Component: | Amount (ul) | Final concentration |
|---|---|---|
| Herculase II PCR buffer, 5X | 10 | 1X |
| dNTP, 100 mM (25 mM each) | 1 | 1 mM |
| HDR Fwd primer (10 uM) | 1 | 0.2 uM |
| HDR Rev primer (10 uM) | 1 | 0.2 uM |
| Herculase II Fusion polymerase | 1 | |
| $MgCl_2$ (25mM) | 2 | 1 mM |
| $ddH_2O$ | 33 | |
| Total | 49 (for each reaction) | |

111| Applicants added 100-200 ng of genomic DNA template from step 109 for each reaction and run the following program.

| Cycle number | Denature | Anneal | Extend |
|---|---|---|---|
| 1 | 95°C, 2 min | | |
| 2-31 | 95°C, 20 s | 60°C, 20 s | 72°C, *30-60 s per kb* |
| 32 | | | 72°C, 3 min |

112| Applicants ran 5 ul of PCR product on a 0.8-1% gel to check for single-band product. Primers may need additional optimization by adjusting the template concentration, $MgCl_2$ concentration, and/or the annealing temperature.
1131 Applicants purified the PCR reactions using the QIAQuick PCR purification kit.
114| In the HDR example, a *Hind*III restriction site is inserted into the EMX1 gene. These are detected by a restriction digest of the PCR amplicon:

| Component | Amount (ul) |
|---|---|
| Purified PCR amplicon (200-300ng) | x |
| F.D. buffer, Green | 1 |
| HindIII | 0.5 |
| ddH2O | x |
| Total | 10 |

i. The DNA is digested for 10 m at 37°C:
ii. Applicants ran 10 ul of the digested product with loading dye on a 4-20% gradient polyacrylamide TBE gel until the xylene cyanol band had migrated to the bottom of the gel.
iii. Applicants stained the gel with 1X SYBR Gold dye while rocking for 15 m.
iv. The cleavage products are imaged and quantified as described above in the SURVEYOR assay section. HDR efficiency is estimated by the formula: (b + c)/(a + b + c), where a is the integrated intensity for the undigested HDR PCR product, and b and c are the integrated intensities for the HindIII-cut fragments.

115| Alternatively, purified PCR amplicons from step 113 may be cloned and genotyped using Sanger sequencing or NGS.

**Deep sequencing and off-target analysis • Timing 1 - 2 d**

The online CRISPR target design tool generates candidate genomic off-target sites for each identified target site. Off-target analysis at these sites can be performed by SURVEYOR nuclease assay, Sanger sequencing, or next-generation deep sequencing. Given the likelihood of low or undetectable modification rates at many of these sites, Applicants recommend deep sequencing with the Illumina Miseq platform for high sensitivity and accuracy. Protocols will vary with sequencing platform; here, Applicants briefly describe a fusion PCR method for attaching sequencing adapters.
116| *Design deep sequencing primers.* Next-generation sequencing (NGS) primers are designed for shorter amplicons, typically in the 100-200 bp size range. Primers may be manually designed using NCBI Primer-Blast or generated with online CRISPR target design tools (website at genome-engineering.org/tools).
117| Harvest genomic DNA from Cas9-targeted cells. Normalize QuickExtract genomic DNA to 100-200 ng/ul with ddH2O.
118| *Initial library preparation PCR.* Using the NGS primers from step 116, prepare the initial library preparation PCR

| Component: | Amount (ul) | Final concentration |
|---|---|---|
| Herculase II PCR buffer, 5X | 10 | 1X |
| dNTP, 100mM (25mM each) | 1 | 1 mM |
| NGS Fwd primer (10uM) | 1 | 0.2 uM |
| NGS Rev primer (10uM) | 1 | 0.2 uM |
| Herculase II Fusion polymerase | 1 | |
| MgCl2 (25mM) | 2 | 1 mM |
| ddH2O | 33 | |
| Total | 49 (for each reaction) | |

119| Add 100-200 ng of normalized genomic DNA template for each reaction.
120| Perform PCR reaction using the following cycling conditions, for no more than **20** amplification cycles:

| Cycle number | Denature | Anneal | Extend |
|---|---|---|---|
| 1 | 95°C, 2 min | | |
| 2-21 | 95°C, 20 s | 60°C, 20 s | 72°C, 15 s |
| 22 | | | 72°C, 3 min |

121| Run 2-5 ul of PCR product on a 1% gel to check for single-band product. As with all genomic DNA PCRs, NGS primers may require additional optimization by adjusting the template concentration, $MgCl_2$ concentration, and/or the annealing temperature.
122| Purify the PCR reactions using the QIAQuick PCR purification kit and normalize eluant to 20 ng/ul. **Pause point:** Purified PCR product may be stored at -20°C.
123| *Nextera XT DNA Sample Preparation Kit.* Following the manufacturer's protocol, generate Miseq sequencing-ready libraries with unique barcodes for each sample.
124| *Analyze sequencing data.* Off-target analysis may be performed through read alignment programs such as ClustalW, Geneious, or simple sequence analysis scripts.

**Timing**

Steps 1 - 2 Design and synthesis of sgRNA oligos and ssODNs: 1-5 d, variable depending on supplier

Steps 3 - 5 Construction of CRISPR plasmid or PCR expression cassette: 2 h to 3 d

Steps 6 - 53 Transfection into cell lines: 3 d (1 h hands-on time)

Steps 54 - 70 Optional derivation of clonal lines: 1-3 weeks, variable depending on cell type

Steps 71 - 91 Functional validation of NHEJ via SURVEYOR: 5-6 h

Steps 92 - 124 Genotyping via Sanger or next-gen deep sequencing: 2-3 d (3-4 h hands on time)

**Addressing Situations Concerning Herein Examples**

| Situation | Solution |
|---|---|
| No amplification of sgRNA | Titrate U6-template concentration |
| SURVEYOR or HDR PCR dirty or no amplification | Titrate MgCl2; normalize and titrate template concentration; annealing temp gradient; redesign primers |
| Unequal amplification of alleles in microdeletion PCRs | Set up separate PCRs to detect wildtype and deletion alleles; Redesign primers with similar sized amplicons |
| Colonies on negative control plate | Increase *Bbs*I; increase Golden Gate reaction cycle number, cut PX330 separately with Antarctic Phosphate treatment |
| No sgRNA sequences or wrong sequences | Screen additional colonies |
| Low lipofectamine transfection efficiency | Check cell health and density; titrate DNA; add GFP transfection control |
| Low nucleofection transfection efficiency | Check cell health and density; titrate DNA; suspend to single cell |
| Clumps or no cells after FACS | Filter cells before FACS; dissociate to single cells; resuspend in appropriate density |
| Clumps or no cells in serial dilution | Recount cells; dissociate to single cells and filter through strainer; check serial dilution |
| High SURVEYOR background on negative sample | Redesign primers to prime from different locations |
| Dirty SURVEYOR result on gel | Purify PCR product; reduce input DNA; reduce 42°C incubation to 30 m |
| No SURVEYOR cleavage | Purify and normalize PCR product; re-anneal with TaqB buffer; Redesign sgRNAs; sequence verify Cas9 on px330 backbone |
| Samples do not sink in TBE acrylamide gel | Supplement with MgCl2 to a final concentration of 15mM or add loading buffer containing glycerol |

*Example 25: NLSs*

Cas9 Transcriptional Modulator: Applicants set out to turn the Cas9/gRNA CRISPR system into a generalized DNA binding system in which functions beyond DNA cleavage can be executed. For instance, by fusing functional domain(s) onto a catalytically inactive Cas9 Applicants have imparted novel functions, such as transcriptional activation/repression, methylation/demethylation, or chromatin modifications. To accomplish this goal Applicants made a catalytically inactive Cas9 mutant by changing two residues essential for nuclease activity, D10 and H840, to alanine. By mutating these two residues the nuclease activity of Cas9 is abolished while maintaining the ability to bind target DNA.

The functional domains Applicants decided to focus on to test Applicants' hypothesis are the transcriptional activator VP64 and the transcriptional repressors SID and KRAB.

CRISPR-Cas may be easily multiplexed to facilitate simultaneous modification of several genes and mediate chromosomal microdeletions at high efficiencies. Applicants used two sgRNAs to demonstrate simultaneous targeting of the human *GRIN2B* and *DYRK1A* loci at efficiencies of up to 68% in HEK293FT cells. Likewise, a pair of sgRNAs may be used to mediate microdeletions, such as excision of an exon, which can be genotyped by PCR on a clonal level. Note that the precise location of exon junctions can vary. Applicants also demonstrated the use of ssODNs and targeting vector to mediate HDR with both wildtype and nickase mutant of Cas9 in HEK 293FT and HUES9 cells (Fig. 26). Note that Applicants have not been able to detect HDR in HUES9 cells using the Cas9 nickase, which may be due to low efficiency or a potential difference in repair activities in HUES9 cells. Although these values are typical, there is some variability in the cleavage efficiency of a given sgRNA, and on rare occasions certain sgRNAs may not work for reasons yet unknown. Applicants recommend designing two sgRNAs for each locus, and testing their efficiencies in the intended cell type.

Cas9 Nuclear localization: Applicants hypothesized that the most effective Cas9 transcriptional modulator would be strongly localized to the nucleus where it would have its greatest influence on transcription. Moreover, any residual Cas9 in the cytoplasm could have unwanted effects. Applicants determined that wild-type Cas9 does not localize into the nucleus without including multiple nuclear localization signals (NLSs) (although a CRISPR system need not have one or more NLSs but advantageously has at zero or least one or more NLS(s)). Because multiple NLS sequences were required it was reasoned that it is difficult to get Cas9 into the nucleus and any additional domain that is fused to Cas9 could disrupt the nuclear localization. Therefore, Applicants made four Cas9-VP64-GFP fusion constructs with different NLS sequences (pXRP02- pLenti2-EF1a-NLS-hSpCsn1(10A,840A)-NLS-VP64-EGFP, pXRP04- pLenti2-EF1a-NLS-hSpCsn1(10A,840A)-NLS-VP64-2A-EGFP-NLS, pXRP06-pLenti2-EF1a-NLS-EGFP-VP64-NLS-hSpCsn1(10A,840A)-NLS, pXRP08- pLenti2-EF1a-NLS-VP64-NLS-hSpCsn1(10A,840A)-NLS-VP64-EGFP-NLS). These constructs were cloned into a lenti backbone under the expression of the human EF 1a promoter. The WPRE element was also added for more robust protein expression. Each construct was transfected into HEK 293FT cells using Lipofectame 2000 and imaged 24 hours post-transfection. The best nuclear localization is obtained when the fusion proteins have NLS sequences on both the N- and C-term of the fusion protein. The highest observed nuclear localization occurred in the construct with four NLS elements.

To more robustly understand the influence of NLS elements on Cas9 Applicants made 16 Cas9-GFP fusions by adding the same alpha importin NLS sequence on either the N- or C-term looking at zero to three tandem repeats. Each construct was transfected into HEK 293FT cells using Lipofectame 2000 and imaged 24 hours post-transfection. Notably, the number of NLS elements does not directly correlate with the extent of nuclear localization. Adding an NLS on the C-term has a greater influence on nuclear localization than adding on the N-term.

Cas9 Transcriptional Activator: Applicants functionally tested the Cas9-VP64 protein by targeting the Sox2 locus and quantifying transcriptional activation by RT-qPCR. Eight DNA target sites were chosen to span the promoter of Sox2. Each construct was transfected into HEK 293FT cells using Lipofectame 2000 and 72 hours post-transfection total RNA was extracted from the cells. 1 ug of RNA was reverse transcribed into cDNA (qScript Supermix) in a 40 ul reaction. 2 ul of reaction product was added into a single 20 ul TaqMan assay qPCR reaction. Each experiment was performed in biological and technical triplicates. No RT control and no template control reactions showed no amplification. Constructs that do not show strong nuclear localization, pXRP02 and pXRP04, result in no activation. For the construct that did show strong nuclear localization, pXRP08, moderate activation was observed. Statistically significant activation was observed in the case of guide RNAs Sox2.4 and Sox2.5.

*Example 26: In Vivo Mouse Data*

This example demonstrates that CRISPR complex delived *in vivo* functions as intended.

**Material and reagents**

Herculase II fusion polymerase (Agilent Technologies, cat. no. 600679)
10x NEBuffer 4 (NEB, cat. No. B7004S)
BsaI HF (NEB, cat. No. R3535S)
T7 DNA ligase (Enzymatics, cat. no. L602L)
Fast Digest buffer, 10X (ThermoScientific, cat. No. B64)
FastDigest NotI (ThermoScientific, cat. No. FD0594)
FastAP Alkaline Phosphatase (ThermoScientific, cat. No. EF0651)
Lipofectamine2000 (Life Technologies, cat. No. 11668-019)
Trypsin (Life Technologies, cat. No. 15400054)

Forceps #4 (Sigma, cat. No. Z168777-1EA)
Forceps #5 (Sigma, cat. No. F6521-1EA)
10x Hank's Balanced Salt Solution (Sigma, cat. No. H4641-500ML)
Penicillin/Streptomycin solution (Life Technologies, cat. No. P4333)
Neurobasal (Life Technologies, cat. No. 21103049)
B27 Supplement (Life Technologies, cat. No. 17504044)
L-glutamine (Life Technologies, cat. No. 25030081)
Glutamate (Sigma, cat. No. RES5063G-A7)
β-mercaptoethanol (Sigma, cat. No. M6250-100ML)
HA rabbit antibody (Cell Signaling, cat. No. 3724S)
LIVE/DEAD® Cell Imaging Kit (Life Technologies, cat. No. R37601)
30G World Precision Instrument syringe (World Precision Instruments, cat. No. NANOFIL)
Stereotaxic apparatus (Kopf Instruments)
UltraMicroPump3 (World Precision Instruments, cat. No. UMP3-4)
Sucrose (Sigma, cat. No. S7903)
Calcium chloride (Sigma, cat. No. C1016)
Magnesium acetate (Sigma, cat. No. M0631)
Tris-HCl (Sigma, cat. no T5941)
EDTA (Sigma, cat. No. E6758)
NP-40 (Sigma, cat. No. NP40)
Phenylmethanesulfonyl fluoride (Sigma, cat. No. 78830)
Magnesium chloride (Sigma, cat. No. M8266)
Potassium chloride (Sigma, cat. No. P9333)
β-glycerophosphate (Sigma, cat. No. G9422)
Glycerol (Sigma, cat. No. G9012)
Vybrant® DyeCycle™ Ruby Stain (Life technologies, cat. No. S4942)
FACS Aria Flu-act-cell sorter (Koch Institute of MIT, Cambridge US)
DNAeasy Blood & Tissue Kit (Qiagen, cat. No. 69504)

**Procedure**

***Constructing gRNA multiplexes for use in vivo in the brain***

Applicants designed and PCR amplified single gRNAs targeting mouse TET and DNMT family members (as described herein) Targeting efficiency was assessed in N2a cell line (Fig. 28). To obtain simultaneous modification of several genes in vivo, efficient gRNA was multiplexed in AAV-packaging vector (Fig. 29). To facilitate further analysis of system efficiency applicants added to the system expression cassette consistent of GFP-KASH domain fusion protein under control of human Synapsin I promoter (Fig. 29). This modification allows for further analysis of system efficiency in neuronal population (more detail procedure in section ***Sorting nuclei and in vivo results***).

All 4 parts of the system were PCR amplified using Herculase II Fusion polymerase using following primers:

1st U6 Fw:

gagggtctcgtccttgcggccgcgctagcgagggcctatttcccatgattc 1st gRNA Rv:

ctcggtctcggtAAAAAAgcaccgactcggtgccactttttcaagttgataacggactagc
cttattttaacttgctaTTTCtagctctaaaacNNNNNNNNNNNNNNNNNNNNGGTGTTTC
GTCCTTTCCAC

2nd U6 Fw:

gagggtctcTTTaccggtgagggcctatttcccatgattcc 2nd gRNA Rv:

ctcggtctcctcAAAAAAgcaccgactcggtgccactttttcaagttgataacggacta gc cttattttaacttgctaTTTCtagctctaaaacNNNNNNNNNNNNNNNNNNNNGGTGTTTC GTCCTTTCCAC

3rd U6 Fw:

gagggtctcTTTgagctcgagggcctatttcccatgattc 3rd gRNA Rv:

ctcggtctcgcgtAAAAAAgcaccgactcggtgccactttttcaagttgataacggact ag ccttattttaacttgctaTTTCtagctctaaaacNNNNNNNNNNNNNNNNNNNNGGTGTTT CGTCCTTTCCA

hSyn_GFP-kash Fw: gagggtctcTTacgcgtgtgtctagac
hSyn_GFP-kash Rv:

ctcggtctcAaggaCAGGGAAGGGAGCAGTGGTTCACGCCTGTAATCCCAGCAATTTGG GA GGCCAAGGTGGGTAGATCACCTGAGATTAGGAGTTGC

(NNNNNNNNNNNNNNNNNNNN is a reverse compliment targeted genomic sequence)

Applicants used Golden Gate strategy to assemble all parts (1:1 molecular ratio) of the system in a single step reaction:

| | |
|---|---|
| 1stU6_gRNA | 18 ng |
| 2nd U6_gRNA | 18 ng |
| 3rd U6_gRNA | 18 ng |
| Syn_GFP-kash | 100 ng |
| 10x NEBuffer 4 | 1.0 μl |
| 10x BSA | 1.0 μl |
| 10 mM ATP | 1.0 μl |
| BsaI HF | 0.75 μl |
| T7 ligase | 0.25 μl |
| $ddH_2O$ | 10 μl |

| Cycle number | Condition |
|---|---|
| 1-50 | 37°C for 5 m, 21°C for 5 m |

Golden Gate reaction product was PCR amplified using Herculase II fusion polymerase and following primers:

| | |
|---|---|
| Fw | 5'cctgtccttgcggccgcgctagcgagggcc |
| Rv | 5'cacgcggccgcaaggacagggaagggagcag |

PCR product was cloned into AAV backbone, between ITR sequences using NotI restriction sites:
PCR product digestion:

| | |
|---|---|
| Fast Digest buffer, 10X | 3 μl |
| FastDigest NotI | 1 μl |
| DNA | 1 μg |
| $ddH_2O$ | up to 30 μl |

AAV backbone digestion:

| | |
|---|---|
| Fast Digest buffer, 10X | 3 μl |
| FastDigest NotI | 1 μl |
| FastAP Alkaline Phosphatase | 1 μl |
| AAV backbone | 1 μg |
| $ddH_2O$ | up to 30 μl |

After 20 min incubation in 37°C samples were purified using QIAQuick PCR purification kit. Standardized samples were ligated at a 1:3 vector:insert ratio as follows:

| | |
|---|---|
| Digested pUC19 | 50 ng |
| Digested insert | 1:3 vector:insert molar ratio |
| T7 ligase | 1 μl |
| 2X Rapid Ligation Buffer | 5 μl |
| $ddH_2O$ | up to 10 μl |

After transformation of bacteria with ligation reaction product, applicants confirmed obtained clones with Sanger sequencing.

Positive DNA clones were tested in N2a cells after co-transfection with Cas9 construct (Figs. 31 and 32).

***Design of new Cas9 constructs for vector delivery, e.g., reduced size to accommodate packaging of AAV or other vectors (such as plasmids that can be delivered via nanoparticle complex(es)***

AAV delivery system despite its unique features has packing limitation - to successfully deliver expressing cassette in vivo it has to be in size < then 4.7 kb. To decrease the size of SpCas9 expressing cassette and facilitate delivery applicants tested several alteration: different promoters, shorter polyA signal and finally a smaller version of Cas9 from *Staphylococcus aureus* (SaCas9) (Figs. 33 and 34). All tested promoters were previously tested and published to be active in neurons, including mouse *Mecp2* (Gray et al., 2011), rat *Map1b* and truncated rat *Map1b* (Liu and Fischer, 1996). Alternative synthetic polyA sequence was previously shown to be functional as well (Levitt et al., 1989; Gray et al., 2011). All cloned constructs were expressed in N2a cells after transfection with Lipofectamine 2000, and tested with Western blotting method (Fig. 35).

***Testing AAV multiplex system in primary neurons***

To confirm functionality of developed system in neurons, Applicants use primary neuronal cultures *in vitro.* Mouse cortical neurons was prepared according to the protocol published previously by Banker and Goslin (Banker and Goslin, 1988).

Neuronal cells are obtained from embryonic day 16. Embryos are extracted from the euthanized pregnant female and decapitated, and the heads are placed in ice-cold HBSS. The brains are then extracted from the skulls with forceps (#4 and #5) and transferred to another change of ice-cold HBSS. Further steps are performed with the aid of a stereoscopic microscope in a Petri dish filled with ice-cold HBSS and #5 forceps. The hemispheres are separated from each other and the brainstem and cleared of meninges. The hippocampi are then very carefully dissected and placed in a 15 ml conical tube filled with ice-cold HBSS. Cortices that remain after hippocampal dissection can be used for further cell isolation using an analogous protocol after removing the brain steam residuals and olfactory bulbs. Isolated hippocampi are washed three times with 10 ml ice-cold HBSS and dissociated by 15 min incubation with trypsin in HBSS (4 ml HBSS with the addition of 10 μl 2.5% trypsin per hippocampus) at 37°C. After trypsinization, the hippocampi are very carefully washed three times to remove any traces of trypsin with HBSS preheated to 37°C and dissociated in warm HBSS. Applicants usually dissociate cells obtained from 10-12 embryos in 1 ml HBSS using 1 ml pipette tips and dilute dissociated cells up to 4 ml. Cells are plated at a density of 250 cells/mm2 and cultured at 37°C and 5% CO2 for up to 3 week

**HBSS**

435 ml H2O
50 ml 10x Hank's Balanced Salt Solution
16.5 ml 0.3M HEPES pH 7.3

5 ml penicillin-streptomycin solution
Filter (0.2 μm) and store 4°C

**Neuron Plating Medium** (100 ml)

97 ml Neurobasal
2 ml B27 Supplement
1 ml penicillin-streptomycin solution
250 μl glutamine
125 μl glutamate

Neurons are transduced with concentrated AAV1/2 virus or AAV1 virus from filtered medium of HEK293FT cells, between 4-7 days in culture and keep for at least one week in culture after transduction to allow for delivered gene expression.

*AAV-driven expression of the system*

Applicants confirmed expression of SpCas9 and SaCas9 in neuronal cultures after AAV delivery using Western blot method (Fig. 37). One week after transduction neurons were collected in NuPage SDS loading buffer with β-mercaptoethanol to denaturate proteins in 95°C for 5 min. Samples were separated on SDS PAGE gel and transferred on PVDF membrane for WB protein detection. Cas9 proteins were detected with HA antibody.

Expression of *Syn*-GFP-kash from gRNA multiplex AAV was confirmed with fluorescent microscopy (Fig. 45).

*Toxicity*

To assess the toxicity of AAV with CRISPR system Applicants tested overall morphology of neurons one week after virus transduction (Fig. 40). Additionally, Applicants tested potential toxicity of designed system with the LIVE/DEAD® Cell Imaging Kit, which allows to distinguish live and dead cells in culture. It is based on the presence of intracellular esterase activity (as determined by the enzymatic conversion of the non-fluorescent calcein AM to the intensely green fluorescent calcein). On the other hand, the red, cell-impermeant component of the Kit enters cells with damaged membranes only and bind to DNA generating fluorescence in dead cells. Both flourophores can be easily visualized in living cells with fluorescent microscopy. AAV-driven expression of Cas9 proteins and multiplex gRNA constructs in the primary cortical neurons was well tolerated and not toxic (Figs. 38 and 39), what indicates that designed AAV system is suitable for in vivo tests.

***Virus production***

Concentrated virus was produced according to the methods described in McClure et al., 2011. Supernatant virus production occurred in HEK293FT cells.

***Brain surgeries***

For viral vector injections 10-15 week old male C57BL/6N mice were anesthetized with a Ketamine/Xylazine cocktail (Ketamine dose of 100 mg/kg and Xylazine dose of 10 mg/kg) by intraperitoneal injection. Intraperitonial administration of Buprenex was used as a pre-emptive analgesic (1 mg/kg). Animals were immobilized in a Kopf stereotaxic apparatus using intra-aural positioning studs and tooth bar to maintain an immobile skull. Using a hand-held drill, a hole (1-2mm) at -3.0 mm posterior to Bregma and 3.5 mm lateral for injection in the CA1 region of the hippocampus was made. Using 30G World Precision Instrument syringe at a depth of 2.5 mm, the solution of AAV viral particles in a total volume of 1 ul was injected. The injection was monitored by a 'World Precision Instruments UltraMicroPump3' injection pump at a flow rate of 0.5 ul/min to prevent tissue damage. When the injection was complete, the injection needle was removed slowly, at a rate of 0.5 mm/min. After injection, the skin was sealed with 6-0 Ethilon sutures. Animals were postoperatively hydrated with 1 mL lactated Ringer's (subcutaneous) and housed in a temperature controlled (37°C) environment until achieving an ambulatory recovery. 3 weeks after surgery animals were euthanized by deep anesthesia followed by tissue removal for nuclei sorting or with 4% paraformaldehyde perfusion for immunochemistry.

***Sorting nuclei and in vivo results***

Applicants designed a method to specifically genetically tag the gRNA targeted neuronal cell nuclei with GFP for Fluorescent Activated Cell Sorting (FACS) of the labeled cell nuclei and downstream processing of DNA, RNA and nuclear proteins. To that purpose the applicants' multiplex targeting vector was designed to express both a fusion protein between GFP and the mouse nuclear membrane protein domain KASH (Starr DA, 2011, Current biology) and the 3 gRNAs to target specific gene loci of interest (Fig. 39). GFP-KASH was expressed under the control of the human Synapsin promoter to specifically label neurons. The amino acid of the fusion protein GFP-KASH was:

MVSKGEELFTGVVPILVELDGDVNGHKFSVSGEGEGDATYGKLTLKFICT
TGKLPVPWPTLVTTLTYGVQCFSRYPDHMKQHDFFKSAMPEGYVQERTIFFKDDGN
YKTRAEVKFEGDTLVNRIELKGIDFKEDGNILGHKLEYNYNSHNVYIMADKQKNGIK
VNFKIRHNIEDGSVQLADHYQQNTPIGDGPVLLPDNHYLSTQSALSKDPNEKRDHMV
LLEFVTAAGITLGMDELYKSGLRSREEEEETDSRMPHLDSPGSSQPRRSFLSRVIRAAL
PLQLLLLLLLLLACLLPASEDDYSCTQANNFARSFYPMLRYTNGPPPT

One week after AAV1/2 mediated delivery into the brain a robust expression of GFP-KASH was observed. For FACS and downstream processing of labeled nuclei, the hippocampi were dissected 3 weeks after surgery and processed for cell nuclei purification using a gradient centrifugation step. For that purpose the tissue was homogenized in 320 mM Sucrose, 5 mM CaCl, 3 mM Mg(Ac)2, 10 mM Tris pH 7.8, 0.1 mM EDTA, 0.1% NP40, 0.1 mM Phenylmethanesulfonyl fluoride (PMSF), 1 mM β-mercaptoethanol using 2ml Dounce homogenizer (Sigma) The homogenisate was centrifuged on a 25% to 29% Optiprep® gradient according to the manufacture's protocol for 30 min at 3.500 rpm at 4 °C. The nuclear pellet was resuspended in 340 mM Sucrose, 2 mM MgCl2, 25 mM KCl, 65 mM glycerophosphate, 5% glycerol, 0.1 mM PMSF, 1 mM β-mercaptoethanol and Vybrant® DyeCycle™ Ruby Stain (Life technologies) was added to label cell nuclei (offers near-infrared emission for DNA). The labeled and purified nuclei were sorted by FACS using an Aria Flu-act-cell sorter and BDFACS Diva software. The sorted GFP+ and GFP- nuclei were finally used to purify genomic DNA using DNAeasy Blood & Tissue Kit (Qiagen) for Surveyor assay analysis of the targeted genomic regions. The same approach can be easily used to purify nuclear RNA or protein from targeted cells for downstream processing. Due to the 2-vector system (Fig. 39) the applicants using in this approach efficient Cas9 mediated DNA cleavage was expected to occur only in a small subset of cells in the brain (cells which were co-infected with both the multiplex targeting vector and the Cas9 encoding vector). The method described here enables the applicants to specifically purify DNA, RNA and nuclear proteins from the cell population expressing the 3 gRNAs of interest and therefore are supposed to undergo Cas9 mediated DNA cleavage. By using this method the applicants were able to visualize efficient DNA cleavage *in vivo* occurring only in a small subset of cells.

Essentially, what Applicants have shown here is targeted *in vivo* cleavage. Furthermore, Applicants used a multiple approach, with several different sequences targeted at the same time, but independently. Presented system can be applied for studying brain pathologic conditions (gene knock out, e.g. Parkinson disease) and also open a field for further development of genome editing tools in the brain. By replacing nuclease activity with gene transcription regulators or epigenetic regulators it will be possible to answer whole spectrum of scientific question about role of gene regulation and epigenetic changes in the brain in not only in the pathologic conditions but also in physiological process as learning and memory formation. Finally, presented technology can be applied in more complex mammalian system as primates, what allows to overcome current technology limitations.

*Example 27: A Cre-dependent CRISPR-Cas9 knockin transgenic mouse for efficient ex vivo and in vivo genome editing*

CRISPR-Cas9 is a powerful technology for genome editing and is being widely adopted due to its efficiency and versatility. While Cas9-mediated genome editing applications are compelling, applying them *in vivo* and *ex vivo* is challenging, since commonly used delivery systems are inefficient and limit accessible cell types. To broaden the application of Cas9 Applicants established a Cre-dependent, Rosa26 *Streptococcus pyogenes* Cas9 knockin mouse and demonstrate its utility in diverse applications, which include gene editing *in vivo* in the brain by AAV-mediated delivery of an sgRNA expression cassettes and *in vivo* in endothelial cells by nanoparticle-mediated sgRNA delivery. Applicants also demonstrate efficient editing of key regulators *ex vivo* in hard to transfect/transduce primary immune cells resulting in an altered immune response. The ability to efficiently edit genes in diverse experimental systems *ex vivo* and *in vivo* in the conditional Cas9 mouse can be a valuable resource for many areas of biology.

A major potential benefit of genome engineering is the ability to directly perturb gene function *in vivo* or in primary cells *ex vivo* (Xue, W. et al. Nature advance on, (2014); Yin, H. et al. Nat. Biotechnol. 32, 551-3 (2014); Heckl, D. et al. Nat. Biotechnol. advance on, (2014)). However, before genetically perturbed cells can be investigated in many cell types *in vivo* or in primary cells *ex vivo* genome modifications must first be made in the germline, which can then be used to create a stable transgenic strain for experimentation. This process is time consuming and technically challenging, which increases exponentially as the number and complexity of genome modifications increases. Zinc finger nuclease (ZFN) and transcription activator-like effector nuclease (TALEN)-mediated genome engineering, mainly by viral-mediated delivery, have been used successfully to edit genes *in vivo* and *ex vivo* and are even being applied therapeutically (Perez, E. E. et al. Nat. Biotechnol. 26, 808-16 (2008); Miller, J. C. et al. Nat. Biotechnol. 29, 143-8 (2011)). However, these technologies are mostly inefficient and require the generation and validation of entirely new protein constructs for each desired target.

More recently the CRISPR (clustered regularly interspaced short palindromic repeat)-associated endonuclease Cas9 from *Streptococcus pyogenes* (*Sp*Cas9, abbreviated as Cas9 throughout this paper) has been applied for editing mammalian genomes by efficiently facilitating DNA double strand breaks (DSBs) (Cong, L. et al. Science (2013); doi:10.1126/science.1231143; Mali, P. et al. Science 339, 823-6 (2013)). Targeted DSBs provide an effective means for knocking out genes via non-homologous end joining (NHEJ)-mediated repair and for stimulating homologous recombination through homology directed repair (HDR) in the presence of a repair template. The Cas9 endonuclease is targeted to specific sequences by either a pair of crRNA and tracrRNA or a single guide RNA (sgRNA) (Deltcheva, E. et al. Nature 471, 602-7 (2011); Jinek, M. et al. Science 337, 816-21 (2012); Gasiunas, G. et al. Proc. Natl. Acad. Sci. U. S. A. 109, E2579-86 (2012)). A significant advantage of Cas9 over ZFNs and TALENs is that targeting a new sequence only requires designing an sgRNA and not a new protein. Moreover, Cas9 can be combined with multiple sgRNAs to perturb multiple genes simultaneously (Cong, L. et al. Science (2013); doi:10.1126/science.1231143; Mali, P. et al. Science 339, 823-6 (2013); Xiao, A. et al. Nucleic Acids Res. 41, e141 (2013)) or with a library of sgRNAs for functional genomic screens (Shalem, O. et al. Science 343, 84-7 (2014); Sanjana, N. E. et al. Nat. Methods 11, 783-784 (2014)).

While Cas9-mediated genome editing applications are compelling, applying them *in vivo* and *ex vivo* is challenging, mainly due to its large transgene size (4.1 kb). Commonly used delivery systems, such as viral vectors, have strict packaging limits and therefore as transgene size increases the available options for delivery decreases (Kumar, M. et al. Hum. Gene Ther. 12, 1893-905 (2001)). Moreover, as transgene size increases the ability to generate high titer virus, which is essential for efficiently transducing cells *in vivo* and *ex vivo,* is also significantly reduced (Kumar, M. et al. Hum. Gene Ther. 12, 1893-905 (2001)). Consequently, Cas9-containing viral vectors have a limited capacity for including additional genetic elements (i.e. sgRNAs, homolous recombination donor templates, fluorescent proteins, modulators, etc.) in a single vector and suffer from low titer, which together limit the flexibility of gene editing in a range of cell types.

To facilitate the broader application of Cas9, Applicants generated a Cre-dependent Rosa26 3xFLAG tagged Cas9-P2A-EGFP knockin transgenic mouse (**Fig. 46a**), which removes the significant Cas9 delivery burden and only requires the delivery of Cre and an sgRNA to achieve gene editing. However, a significant concern was that Cas9 expression, once induced, may cause toxicity due to non-specific nuclease activity. Therefore, as a broad way to test this Applicants generated a sub strain with constitutive Cas9 expression in all cells by crossing the Cre-dependent Cas9 mouse to a beta-actin Cre driver strain (Lewandoski, M. et al. Cold Spring Harb. Symp. Quant. Biol. 62, 159-68 (1997)). Resulting progenies of this cross were viable and Cas9 expression was observed at the whole-organ-level (**Figures 46b and 49**) as well as the protein-level in whole-brain lysates (**Fig. 46c**). This was not observed in the Cre-dependent Cas9 mice (**Figure 50**) or wild-type controls (**Fig. 46b,c and 49, 50**). Additionally, the constitutively Cas9 expressing animals were fertile, had normal litter sizes, presented no morphological abnormalities, and could be bred to homozygosity. At the cellular-level Applicants also found no morphological abnormalities or upregulation in DNA damage and apoptosis markers (**Figures 51 and 52**). Taken together these data suggest that Cas9 expression is tightly controlled in Cre-dependent Cas9 mice, and constitutive expression can be maintained without causing detectible toxicity using several metrics.

To determine whether the Cas9 transgene within the genome was functional Applicants tested whether adeno-associated virus (AAV)-mediated delivery of HA-tagged Cre and sgRNA could mediate indel formation in the brain *in vivo.* Applicants constructed an AAV-U6-sgRNA-Cre vector (AAV-sgRNA) (**Fig. 46d**) containing an sgRNA targeted to a highly expressed neuronal-specific RNA-splicing factor, NeuN (sgNeuN) (**Fig. 46e**) and packaged the vector using AAV serotypes 1 and 2 (AAV1/2), for delivery by stereotactic injection into the prefrontal cortex of Cre-dependent Cas9 mice (**Fig. 46f**). Three weeks post injection Applicants acutely dissected the injected brain region and used the tissue for downstream assays. Deep sequencing of the NeuN locus showed indel formation near the predicted cleavage site (**Fig. 46e**); Cas9 was functional and facilitating DSBs. Furthermore, in these same samples Applicants observed NeuN protein depleted only in the injected region (**Fig. 46g,h**) and not in controls (non-injected and LacZ-targeted sgRNA, termed sgLacZ) (**Fig. 46i**). To quantify this effect Applicants performed immunoblots and deep sequencing on tissue from three mice and found that NeuN protein expression was reduced by nearly 80% (**Fig. 46j**), whereas indels were found at the target site in 85% of sequencing reads (**Fig. 46k**).

While immunoblot and whole-tissue indel analysis provides a global view of genome editing by Cas9, it does not capture the degree of modification at the single-cell-level. For example, without isolating single cells Applicants cannot identify whether one or both alleles are being edited within individual cells, which is important for understanding the genetic heterogeneity of this strategy. However, due to their complex morphology, isolation of individual intact neurons within the brain is challenging. To overcome this and facilitate single-cell genetic analysis Applicants set out to isolate individual neuronal nuclei, which has been shown previously (Okada, S. et al. J. Cell. Physiol. 226, 552-8 (2011)). Cytosolic expression of EGFP, originating from the Cas9-P2A-EGFP transgene, for isolating individual nuclei was insufficient so Applicants generated a single vector (AAV-sgRNA-hSyn-Cre-P2A-EGFP-KASH) and expressed sgRNA, Cre, and EGFP fused to the KASH (Klarsicht, ANC-1, Syne Homology) nuclear transmembrane domain. For downstream analysis Applicants used fluorescence activated cell sorting (FACS) to isolate single EGFP positive nuclei. Sequencing of 167 single nuclei showed that 84% (n=141) of transduced cells have both alleles edited (homozygous), while only 9% (n=15) of nuclei had single allele modification and 7% (n=11) remained unmodified (**Fig. 46l**). Of the 15 heterozygous nuclei all of the modified alleles contained missense (mis) mutations, whereas 141 homozygous nuclei contained both missense (mis) and sense (sen) mutations (**Fig. 46m**).

Viral-mediated expression of Cas9 is efficient for targeting heterogeneous cells within an injected tissue and is useful for a range of applications. However, a large diversity of cellular subtypes play fundamental roles in biological processes and need to be perturbed individually, which often cannot be done using viral-vectors. While tissue- and cell type-specific promoters for heterologous expression do exist they are often large or not tightly controlled, leading to reduced specificity (Lewandoski, M. et al. Cold Spring Harb. Symp. Quant. Biol. 62, 159-68 (1997)). A powerful way to achieve tissue- or cell type-specific expression is through the use of Cre driver strains. Combining Cre-dependent Cas9 mice with the wide variety of available Cre driver strains would provide a powerful means to achieve Cas9 expression in specific cell types and subsequent genome editing in these cells *in vivo.* Therefore, Applicants crossed the Cre-dependent Cas9 mouse with two Cre driver strains to determine if Applicants could achieve neuronal subtype-specific Cas9 expression, namely the tyrosine hydroxylase (TH-IRES-Cre) driver for dopaminergic neurons and the parvalbumin (PV-Cre) driver for a subtype of inhibitory interneurons. As predicted, Cas9 expression was restricted to TH or PV positive cells (**Fig. 47a,b**).

While the morphology of Cas9 expressing neurons were no different than wild-type controls, neurons are particularly sensitive to transgene expression and thus may have abnormal physiological properties. Therefore, to ensure that constitutive Cas9 expression does not adversely affect cellular health and physiology Applicants used a panel of electrophysiological measurements to check the basal function of Cas9 expressing neurons. Applicants performed whole-cell patch clamp recordings in CA1 pyramidal neurons from acute hippocampal slices to examine firing threshold (**Fig. 47c,d**) and rheobase current (**Fig. 47e**) and found no significant changes between control and constitutively Cas9 expressing mice (**Supplementary Table 2**). In addition, there was no significant change in input resistance (**Fig. 47f**) whole cell capacitance (**Fig. 47g**) or membrane resting potential (**Fig**. **47h**). Taken together these data suggest that fundamental properties of neurons, a particularly sensitive cell type, are not altered by the heterologous expression of Cas9.

Cell type-specificity achieved through the use of a Cre driver is valuable but only possible when a cell type-specific promoter has been identified. Indeed, without a specific promoter genetic perturbation in specific cells is challenging. Therefore, to determine if Applicants could achieve cell type specificity without performing a genetic cross or viral delivery Applicants set out to test whether Applicants could combine constitutively expressing Cas9 mice with other targeting methods for *in vivo* sgRNA delivery.

Applicants adapted an endothelial-specific nanoparticle (Dahlman, J. E. et al. Nat. Nanotechnol. 9, 648-655 (2014)), 7C1, which was developed to mediate small interfering RNA (siRNA) delivery specifically to endothelial cells *in vivo.* 7C1, like most nanoparticles, was optimized to deliver specific cargo, such as siRNAs, which are over five times smaller than sgRNAs. To determine if 7C1 could be formulated with sgRNA Applicants combined 7C1 nanoparticles with sgRNA and observed multilamellar structures (**Fig. 48b**) (Dahlman, J. E. et al. Nat. Nanotechnol. 9, 648-655 (2014)). Surprisingly, the larger size of sgRNAs compared to siRNAs did not significantly influence nanoparticle formulation. Then to test the possibility of nanoparticle-mediated delivery of sgRNAs *in vivo,* Applicants designed 20 unique sgRNAs targeting the endothelial-specific gene intercellular adhesion molecule 2 (ICAM2). After determining the two most potent sgRNAs in an *in vitro* cleavage assay in the mouse-derived N2a cell line (**Figure 53**), the two most potent sgRNAs, named sgICAM2-1 and sgICAM2-20, were then formulated together with 7C1 nanoparticles (7C1:sgICAM2-1+20). These were subsequently administered to constitutively Cas9 expressing mice at 1.5 mg/kg via tail vein injection on days 0, 3, 7, 10, and 13.

To test the Cas9-mediated genome editing efficiency in targeted endothelial cells, the mice were sacrificed on day 21 and pulmonary and cardiovascular endothelial cells ($CD31^+$ and $CD45^-$) were isolated using FACS. For mice treated with sgICAM2-1+20 Applicants observed indel formation at both target sites in both pulmonary and cardiovascular endothelial cells (**Fig. 48c,d,e)** but not in controls. Consistent with these results Applicants also observed reduced levels of ICAM2 protein expression in pulmonary (60%) (**Fig. 48f**) and cardiovascular (70%) (**Fig. 48g**) endothelial cells. As a gross measure of general health (Dahlman, J. E. et al. Nat. Nanotechnol. 9, 648-655 (2014)), Applicants also tracked the weights of injected animals over the course of Applicants' study and saw no significant change (**Fig. 48h**). These data broadly demonstrate that delivery vehicles optimized for siRNA delivery can also be utilized for sgRNA delivery to constitutively Cas9 expressing mice to enable non-viral cell type-specific genome editing, which opens up new cell types for editing by Cas9 *in vivo.*

To determine if Applicants could broaden the utility of Cas9 even further Applicants set out to genetically modify primary cells, which are particularly challenging to transfect and transduce. Primary immune cells are often not accessible for genetic manipulation due to delivery challenges, short viability terms in culture, or both. For example, innate immune dendritic cells (DCs) specialize in pathogen detection and initiation of appropriate immune responses by rapid cytokine and antigen presentation to adaptive immune cells (Mellman, I. & Steinman, R. M. Cell 106, 255-8 (2001)). Since existing cell lines do not mimic DC biology well, many studies are performed with primary cells derived *ex vivo* from precursors isolated from the bone marrow (BMDCs), which retain many critical characteristics of DCs *in vivo* (Amit, I. et al. Science 326, 257-63 (2009); Chevrier, N. et al. Cell 147, 853-67 (2011); Garber, M. et al. Mol. Cell 47, 810-22 (2012); Shalek, A. K. et al. Nature 498, 236-40 (2013)). Short hairpin RNA (shRNA)-mediated genetic perturbations, mainly by lentiviral delivery, have been used successfully and in a highly multiplexed fashion to perturb gene expression and dissect regulatory and signaling pathways of DCs (Amit, I. et al. Science 326, 257-63 (2009); Chevrier, N. et al. Cell 147, 853-67 (2011)). However, knockdown is often inefficient and requires extensive validation due to potential off-target effects.

While genome editing approaches are compelling, applying them to BMDCs is challenging, since BMDCs are difficult to transfect and can be kept in culture for only a few days. Indeed, introducing Cas9 to the cells was a significant technical hurdle; lentiviral vectors containing Cas9 are necessarily large resulting in low viral titers that critically reduce the applicability of this approach. Many other primary cells, especially immune cells, are comparably or more challenging (Shalek, A. K. et al. Nano Lett. 12, 6498-504 (2012)). Applicants thus reasoned that constitutive expression of Cas9 in cells derived from the constitutively Cas9 expressing mouse may facilitate such applications, since all that will be required for gene editing is additional transduction of sgRNA, which can be efficiently packaged in lentivirus.

In this work Applicants introduce Cre-dependent and constitutively Cas9 expressing mice, which further the reach of Cas9-mediated genome editing to include specific cell types *in vivo* and *ex vivo.* Applicants have demonstrated that Cas9 mice can be used to modify genes in the brain, restrict Cas9 expression to cellular subtypes, specifically edit endothelial cells by nanoparticle-mediated sgRNA delivery, and also edit genes in primary immune cells leading to an altered immune response. Each of these applications broadens the utility of Cas9 and address significant challenges facing its *in vivo* and *ex vivo* application. The flexibility for inducing Cas9 expression and delivering sgRNAs was demonstrated using a wide variety of targeting methods including AAV and lentiviral transduction, nanoparticle-mediated delivery, and genetic crosses with Cre-driver lines. The example applications demonstrated here enable a broader spectrum of biological experiments involving these Cas9 transgenic mouse lines.

*Ethics Statement.* All experimental protocols were approved by the CAC (Committee on Animal Care) of MIT and were in compliance with the Guidelines for the Care and Use of Laboratory Animals (Eighth edition, National Research Council of the National Academies).

*Generation of the Cre-dependent Cas9 mouse.* Cre-dependent Cas9 knockin mice were generated by homologous recombination in R1 embryonic stem cells and implanted in C57BL6/J blastocysts using standard procedures as described previously Heyer, M. P. et al. in Genomics 211-248 (John Wiley & Sons, Ltd, 2010); doi:10.1002/9780470711675.ch10. Briefly, a codon optimized 3xFLAG-NLS-*Sp*Cas9-NLS-P2A-EGFP expression cassette was cloned into the Ai9 Roas26 targeting vector Madisen, L. et al. Nat. Neurosci. 13, 133-40 (2010) and further verified by sequencing. Linearized targeting vector was electroporated into R1 embryonic stem cells followed by G418 and DTA selection for a week. Targeted single ES cell colonies were screened by PCR with primers amplifying both recombinant arms. PCR products were sequenced to further validate correct insertion. Correctly targeted colonies were injected into C57BL6/J blastocysts for generating chimeric mice. High percentage male chimeric mice were mated with C57 BL6/J female mice (Jackson laboratory) to establish germline-transmitted founders. Genotypes of Cas9 mice were determined by amplifying a 4.5 kb product from purified mouse tail DNA (Forward primer: GCAGCCTCTGTTCCACATACAC; Reverse primer: ACCATTCTCAGTGGCTCAACAA).

*DNA vectors and sgRNA design.* The AAV vectors used for stereotaxic injection into the mouse brain were cloned between AAV serotype 2 ITRs and also included the human U6 promoter for noncoding sgRNA transcription, the ubiquitous Cbh promoter, HA-tagged Cre recombinase for recombination of loxP-SV40polyA-LoxP, WPRE, and human growth hormone polyA sequence. For nuclei sorting a similar plasmid was cloned between AAV serotype 2 ITRs and also included the human U6 promoter for noncoding sgRNA transcription, the ubiquitous hSyn promoter, HA-tagged Cre recombinase for recombination of loxP-SV40polyA-LoxP, P2A, EGFP-KASH fusion for nuclei labeling, WPRE, and human growth hormone polyA sequence. SgRNAs were designed using the CRISPRtool (crispr.mit.edu) to minimize potential off-target effects. SgRNA sequences and genomic primers are listed in supplemental **Table S1.** sgRNAs were validated *in vitro* by transient cotransfected (Lipofectamine 2000, Life Technologies) into the N2a mouse cell line with a plasmid constitutively expressing Cas9. Genomic DNA was harvested 72 hours post transfection.

*AAV1/2 production.* HEK293FT cells were transfected with the plasmid of interest, pAAV1 plasmid, pAAV2 plasmid, helper plasmid pDF6, and PEI Max (Polysciences, Inc. 24765-2) in Dulbecco's Modified Eagle Medium (DMEM from Life Technologies, 10569-010). 72 hours post transfection, the cell culture media was discarded. Then the cells were rinsed and pelleted via low speed centrifugation. Afterwards, the viruses were applied to HiTrap heparin columns (GE Biosciences 17-0406-01) and washed with a series of salt solutions with increasing molarities. During the final stages, the eluates from the heparin columns were concentrated using Amicon ultra-15 centrifugal filter units (Millipore UFC910024). Titering of viral particles was executed by quantitative PCR using custom Cre-targeted Taqman probes (Life Technologies).

*Stereotactic injection of AAV.* Before beginning surgery, pre-operative, operation, and post-operative areas were defined. Applicants ensured that all tools had been autoclaved and that reagents were sterile. Applicants administered a mixture of ketamine (100mg/kg) and xylazine (10mg/kg) intraperitoneally for anesthesia. Anesthesia levels were monitored by absence of corneal reflex, absence of foot withdrawal following pressure to the footpad, and by absence of startle from tail pinch. Applicants also administered buprenorphine HCl (0.1mg/kg) intraperitoneally as a pre-emptive analgesic.

Once subject mice were in deep anesthesia, they were immobilized in a Kopf stereotaxic apparatus using intra-aural positioning studs and a tooth bar to immobilize the skull. Heat is provided for warmth by a standard heating pad. Then Applicants proceeded with the approved craniotomy procedures. Applicants drilled a hole on the surface of the skull at 1.94 mm anterior to Bregma and 0.39 mm lateral for injection into the prefrontal cortex. Using a 33 G Nanofil needle and World Precision Instrument Nanofil syringe at a depth of -2.95 mm, Applicants injected 1 uL AAV into the right hemisphere of the brain. Injection rates were monitored by the World Precision Instruments UltraMicroPump3. After injection, Applicants closed the incision site with 6-0 Ethilon sutures (Ethicon by Johnson & Johnson). Animal were postoperatively hydrated with 1 mL lactated Ringer's solution (subcutaneous) and housed in a temperature controlled (37°C) environment until achieving ambulatory recovery. To relieve post-operative pain, Meloxicam (1-2 mg/kg) was also administered subcutaneously directly after surgery.

*Electrophysiology.* Data acquisition and analysis were performed with experimenter blinded to mice genotype as described in previous report (Han, K. et al. Nature 503, 72-7 (2013); Peça, J. et al. (2011) Nature, 472(7344), 437-42). Slices were prepared from 8-10 week old littermate-paired adult wild-type and homozygous constitutively Cas9 expressing mice. Mice were deeply anesthetized by intra-peritoneal injection of avertin (1.25%, v/v) and transcardially perfused with carbogenated (95% O2, 5% CO2) ice-cold cutting solution that contained (in mM): 30 NaCl, 194 sucrose, 4.5 KCl, 10 glucose, 26 $NaHCO_3$, 1.2 $NaH_2PO_4$, 0.2 $CaCl_2$ and 8 $MgCl_2$. Acute coronal brain slices containing typical CA1 layer of media hippocampus were sliced using vibratome (leica VT 1200S) in a chamber filled with ice-cold sucrose based cutting solution. 300 $\mu$m slices were cut in sucrose cutting solution and then transferred to recovery chamber at 32°C for 12 minutes in ACSF. The ACSF contained (in mM): 119 NaCl, 2.3 KCl, 11 glucose, 26.2 $NaHCO_3$, 1 $NaH_2PO_4$, 2.5 $CaCl_2$ and 1.3 $MgSO_4$. After recovery, slices were transferred to ACSF at room temperature (25 °C) for a least 1 hour before recording. Whole-cell recordings of CA1 pyramidal neurons were made under a DIC upright microscope (BX51 WI, Olympus, Japan) using MultiClamp 700A amplifier (Molecular Device, USA). Recording electrodes with resistance of 3-4 M$\Omega$ were pulled from glass capillary (King Precision Glass, glass type 8250) using Flaming/Brown micropipette puller (model P-97, Sutter Instrument, USA), and filled with internal solution containing (in mM): 131 K-gluconate, 17.5 KCl, 1 $MgCl_2$, 1.1 EGTA, 10 HEPES, 9 NaCl, 2 ATP, 0.2 GTP, 10 phosphocreatine at pH 7.2 and osmolarity 280-290 mOsm/L. Series resistance typically ranging between 10~20 M$\Omega$, was monitored and not compensated during the recording. Signals were sampled at frequencies of 10K Hz and filtered at 2K Hz using pClamp software (Clampex 10.2, Molecular Device) via a Digidata-1440A interface (Molecular Device). Data were presented as mean $\pm$ S.E.M. Statistical significance was tested using unpaired two-tailed Student's t-test.

*Genomic DNA extraction, captured sequencing, and indel analysis.* Genomic DNA was extracted from both cells and tissue using Quick Extract DNA extraction solution (Epicentre) following the recommended protocol. Treated cells and tissues were used as a template for PCR for both captured sequencing and SURVEYOR nuclease assay using high fidelity polymerases (Thermo Scientific) as has been previously described (Hsu, P. D. et al. Nat. Biotechnol. 31, 827-32 (2013)). Low cycle, first round PCR was performed to amplify the target site. Second round PCR was performed to add generic adapters, which were then used for a third round of PCR for sample barcoding. Samples were pooled in equal amounts and purified using QiaQuick PCR Cleanup (Qiagen), quantified using Qubit (Life Technologies) and sequenced on a MiSeq (Illumina). Indel analysis was performed using custom scripts as previously described Hsu, P. D. et al. Nat. Biotechnol. 31, 827-32 (2013). Data were presented as mean $\pm$ S.E.M. Statistical significance was tested using unpaired two-tailed Student's t-test.

*SURVEYOR nuclease assay.* PCR amplicons of target sites were purified and used as an input to the SURVEYOR nuclease assay (Transgenomics). The manufacturer's recommended protocol was followed. SURVEYOR nuclease assay-treated products were run on a 4-12% PA-TBE gel (Life Technologies), stained with Sybr Gold (Life Technologies) and imaged on a gel imager (Bio-Rad). Indel percent was quantified using relative band intensities.

*Western blot analysis.* Protein lysates were quantified using the BCA Protein Assay Kit (Pierce) and equally loaded onto a 4-20% Tris-HCL Criterion Gel (Bio-Rad). Proteins were transferred onto PVDF membrane (Bio-Rad), blocked solution (5% BLOT-QuickBlocker (G-Biosciences) in TBS-T (trisp buffered saline with 0.1% Tween-20)) and stained overnight at 4°C on an orbital shaker using the following primary antibodies: anti-FLAG (1:1000, Sigma Aldrich, F1804), HRP conjugated anti-Gapdh (1:5000, Cell Signaling Technology, 3683), anti-beta-tubulin (1:1000, Cell Signaling Technology, 2128), and anti-HA (1:1000, Cell Signaling Technology, 3724 and 2367). Membranes were washed three times with TBS-T and stained with secondary antibodies for 1 hour at room temperature. Applicants used HRP-conjugated secondary antibodies (1:10000, Cell Signaling Technology, 7074 and 7076). Stained membranes were washed three times in TBS-T and then developed using SuperSignal West Fempto Substrate (Pierce) and imaged on a gel imager (Bio-Rad)

*Immunostaining and imaging.* Mice were given a lethal dose of Ketamine/Xylazine in preparation for brain tissue collection. Mice were transcardially perfused with 0.9% saline followed by 4% paraformaldehyde using a peristaltic pump (Gilson). The collected brain tissue was fixed overnight and transferred to phosphate buffered saline the following day. Tissue sectioning was performed on a vibratome (Leica VT1000S) at a thickness of 40 μm. Sections were rinsed three times in phosphate buffered saline (PBS) with 0.1% Triton X-100 (PBS-Tx). Sections were blocked with 5% normal goat serum (NGS) (Cell Signaling Technology) in PBS-Tx for one hour. Sections were incubated with primary antibodies diluted in PBS-Tx with 5% NGS overnight at 4°C on an orbital shaker. The following is a list of primary antibodies that were utilized: anti-cleaved caspase 3 (CC3) (1:1000, Cell Signaling Technology, 9664), anti-yH2AX (1:1000, Millipore, 05-636) anti-NeuN (1:800, Cell Signaling Technology, 12943), anti-GFP (1:1600, Nacalai Tesque, GF090R), anti-parvalbumin (1:1000, Sigma Aldrich, P3088) and anti-tyrosine hydroxylase (1:1000, Immunostar, 22941). The following day, sections are washed three times in PBS-Tx. The sections are then incubated in PBS-Tx with the appropriate AlexaFluor®405, 488, 568 and/or 647 secondary antibody (1:400, Life Technologies) for three hours at room temperature on an orbital shaker. After the incubation, sections are rinsed with PBS-Tx three times and then mounted onto superfrost microscope slides (VWR). The sections are then coverslipped with VECTASHIELD HardSet Mounting Medium with DAPI (VECTOR Laboratories) and visualized under a confocal microscope (Zeiss LSM 710, Ax10 ImagerZ2, Zen 2012 Software).

*Purification and FACS of single neuronal nuclei.* For labeling neuronal nuclei Applicants generated an AAV-U6-sgRNA-hSyn-Cre-P2A-EGFP-KASH vector. Three weeks post-injection Applicants performed acute dissection of the injection site in the prefrontal cortex. Tissue was gently homogenized in 2 ml ice-cold homogenization buffer (HB) (320 mM Sucrose, 5 mM CaCl, 3 mM $Mg(Ac)_2$, 10 mM Tris pH7.8, 0.1 mM EDTA, 0.1 % NP40, 0.1 mM PMSF, 1 mM beta-mercaptoethanol) using 2 ml Dounce homogenizer (Sigma Aldrich); 25 times with pestle A, followed by 25 times with pestle B. An additional 3 ml of HB was added and the mixture was put on ice for 5 min. Gradient centrifugation was performed using 5 ml 50% OptiPrep™ density gradient medium (Sigma Aldrich) containing 5 mM CaCl, 3 mM $Mg(Ac)_2$, 10 mM Tris pH 7.8, 0.1 mM PMSF, 1 mM beta-mercaptoethanol. The nuclei-containing misture was gently added on the top of a 29% iso-osmolar OptiPrep solution in a conical 30 ml centrifuge tube (Beckman Coulter, SW28 rotor). Samples were centrifuged at 10100xg (7,500 rpm) for 30 min at 4°C. The supernatant was removed and the nuclei pellet was gently resuspended in 65 mM beta-glycerophosphate (pH 7), 2 mM $MgCl_2$, 25 mM KCl, 340 mM sucrose and 5% glycerol. Intact nuclei were labeled with Vybrant DyeCycle™ Ruby Stain (Life Technologies) and sorted using a BD FACSAria III. $EGFP^+$ nuclei were sorted into individual wells of a 96 well-plate containing 5 μl of QuickExtract DNA Extraction Solution (Epicentre).

*7C1 nanoparticle formulation.* 7C1 nanoparticles were synthesized, purified, formulated, and characterized in procedures adapted from Dahlman, J. E. et al. Nat. Nanotechnol. 9, 648-655 (2014). Specifically, 7C1 was synthesized by mixing C15 epoxide-terminated lipids to low molecular weight (MW=600) polyethyleneimine (PEI) for 48 hours in 100% EtOH. 7C1 was then purified on a silica column, before pure 7C1 was mixed with C14PEG2000 and sgRNAs in a microfluidic channel to form particles. Particle size was characterized by dynamic light scattering after diluting particles in PBS to a concentration of 0.01 mg/mL, as previously described, while particle internal structure was imaged with cryoTEM.

*Lung and heart endothelial cell isolation.* Mice were injected intravenously with 1.5 mg/kg total sgRNA on day 0, 3, 7, 10, and 13. Eight days after the final injection, pulmonary and cardiovascular endothelial cells were isolated as adapted from Dahlman, J. E. et al. Nat. Nanotechnol. 9, 648-655 (2014). More specifically, mice were immediately perfused with sterile IX PBS immediately following sacrifice, before lungs and hearts were digested in IX PBS, DNAse, Collagenase I, and Collagenase XI for 30 minutes at 37°C. Digested tissue was passed through a 70 μm cell strainer to generate a single cell suspension. Following RBC lysis, cells were stained with anti-ICAM2 (clone 3C4), anti-CD31 (clone 390) and anti-EPCAM (clone G8.8) (all from Biolegend, San Diego, USA) at 1:300 dilution in flow cytometry staining buffer (PBS containing 0.5% BSA and 2 mM EDTA) for 20 min. at 4°C. Antibodies were washed away by centrifugation at 350 g at 4°C. Cells were then stained with propidium iodide (Sigma) and sorted using BD FACSAria III. $CD31^+$ cells (~100,000) were sorted into individual tubes containing 100 μl of RNeasy lysis buffer (Qiagen) and stored at -80°C till further analysis. ICAM2 protein expression data was also collected during sorting and analyzed using FlowJo (Tree Star Inc.).

*Mouse dendritic cells.* 6-8 week old constitutively Cas9 expressing female mice were used for all DC experiments. Bone marrow cells were collected from femora and tibiae and plated at concentration of $2x10^5$/ml on non-treated tissue culture dishes in RPMI medium (Gibco, Carlsbad, CA, Invitrogen, Carlsbad, CA), supplemented with 10% FBS (Invitrogen), L-glutamine (Cellgro), penicillin/streptomycin (Cellgro), MEM non-essential amino acids (Cellgro), HEPES(Cellgro), sodium pyruvate(Cellgro), β-mercaptoethanol (Gibco), and GM-CSF (20 ng/mL; Peprotech). At day 2, cells were infected with lentiviruses encoding guide RNAs. Cells were expanded in the presence of GM-CSF. At day 7, infected cells were selected by adding puromycin (Invitrogen) at 5 μg/mL. At day 9 100 ng/ml LPS (Invivogen) was added 30 min prior to protein analysis (**Fig. 46c**) or 3 hours prior to mRNA expression profiling (**Fig. 46c,e**). Flow cytometry for GFP detection was performed with BD Accuri C6. Western blot was done using anti-Myd88 (R&D Systems AF3109) and anti-actin (Abcam, ab6276) antibodies.

*Nanostring nCounter expression measurement.* DCs were processed and analyzed as previously described (Amit, I. et al. Science 326, 257-63. Briefly, $5x10^4$ cells were lysed in TCL buffer (Qiagen) supplemented with β-mercaptoethanol. 5% of the lysate was hybridized for 16 hours with a previously described Nanostring Gene Expression CodeSet (Geiss, G. K. et al. Nat. Biotechnol. 26, 317-25 (2008)) and loaded into the nCounter Prep Station followed by quantification using the nCounter Digital Analyzer. Counts were normalized using control genes as previously described Amit, I. et al. Science 326, 257-63 (2009) and fold changes were calculated relative to cells transduced with sgRNA targeting GFP and non-targeted controls. Heat maps were created using GENE-E (http://www.broadinstitute.org/cancer/software/GENE-E/).

Supplementary Table S1 | List of oligos used in this Example

| Oligo | Sequence (5'-3') | | |
|---|---|---|---|
| sgNeuN | GTCGGGGTCCCTGAACCGGA | sgRNA | AAV/stereotactic injection |
| sgICAM2-1 | GCTTACCTGGGCTGTAGAAC | sgRNA | in vitro validation and 7C1/systemic injection |
| sgICAM2-2 | CTTATCCTGTTCTACAGCCC | sgRNA | in vitro validation |
| sgICAM2-3 | AGATGTCTTCTTTTGCTTGC | sgRNA | in vitro validation |
| sgICAM2-4 | GTCTCCAGGCCGCCCATGTC | sgRNA | in vitro validation |
| sgICAM2-5 | CATGTCTGGGGCTGCGCAGT | sgRNA | in vitro validation |
| sgICAM2-6 | AGCCCCAGACATGGGCGGCC | sgRNA | in vitro validation |
| sgICAM2-7 | TTTTATTCGTGGGCGTCTCC | sgRNA | in vitro validation |
| sgICAM2-8 | GCTTTCCCGAACACGTGAAA | sgRNA | in vitro validation |
| sgICAM2-9 | CTTTTGCCATTTCACGTGTT | sgRNA | in vitro validation |
| sgICAM2-10 | GCAGCCGCCTCGGGTGTTTG | sgRNA | in vitro validation |
| sgICAM2-11 | CAGCCGCCTCGGGTGTTTGT | sgRNA | in vitro validation |
| sgICAM2-12 | AGGGGACACCGTGCACTCAA | sgRNA | in vitro validation |
| sgICAM2-13 | GAGCTGGATCTACGGCCCCA | sgRNA | in vitro validation |
| sgICAM2-14 | GCGGATGATATACCCACCAT | sgRNA | in vitro validation |
| sgICAM2-15 | TGGATCTACGGCCCCATGGT | sgRNA | in vitro validation |
| sgICAM2-16 | TCACACGGGACGTGCCCGAA | sgRNA | in vitro validation |
| sgICAM2-17 | CGGGACGTGCCCGAAAGGCT | sgRNA | in vitro validation |
| sgICAM2-18 | GGACGTGCCCGAAAGGCTCG | sgRNA | in vitro validation |
| sgICAM2-19 | GGGACGTGCCCGAAAGGCTC | sgRNA | in vitro validation |
| sgICAM2-20 | AAGACGGACAGGCACCTACG | sgRNA | in vitro validation and 7C1/systemic injection |
| sgLacZ | TGCGAATACGCCCACGCGAT | sgRNA | AAV/stereotactic and 7C1/system injections (control) |
| sgMyd88-1 | CCCTTGGTCGCGCTTAACGT | sgRNA | Lenti/DC infection |
| sgMyd88-2 | CCCACGTTAAGCGCGACCAA | sgRNA | Lenti/DC infection |

(continued)

| Oligo | Sequence (5'-3') | | |
|---|---|---|---|
| sgA20-1 | CTCCAGCGTGTATCGGTGCA | sgRNA | Lenti/DC infection |
| sgA20-2 | GGATGATCTCTCGGAACTGT | sgRNA | Lenti/DC infection |
| sgGFP-1 | CGTCGCCGTCCAGCTCGACC | sgRNA | Lenti/DC infection (indel analysis/ western/nanostring control) |
| sgGFP-2 | CAGGGTCAGCTTGCCGTAGG | sgRNA | Lenti/DC infection (indel analysis/ western/nanostring control) |
| sgLacZ-1 | TGTTCGCATTATCCGAACCAT | sgRNA | Lenti/DC infection (indel analysis/ western control) |
| sgLacZ-2 | CGCGATCGTAATCACCCGAGT | sgRNA | Lenti/DC infection (indel analysis/ western control) |
| sgCTR-1 | CTCAGTTCCAGTACGGCTCCA | sgRNA | Lenti/DC infection (nanostring control) |
| sgCTR-2 | GCTTCAAGTGGGAGCGCGTGA | sgRNA | Lenti/DC infection (nanostring control) |
| sgCTR-3 | ACGTCTATATCATGGCCGACA | sgRNA | Lenti/DC infection (nanostring control) |
| sgCTR-4 | CCCGACCACATGAAGCAGCAC | sgRNA | Lenti/DC infection (nanostring control) |
| NeuN-F | CAGCAGCCCAAACGACTACAT | Primer | AAV/stereotactic injection |
| NeuN-R | CTAAGGAATCTCGGCTGGAGGT | Primer | AAV/stereotactic injection |
| ICAM2 exon1-F | TCACTGGCACAGAGGAGATTGT | Primer | in vitro validation (SURVEYOR assay) |
| ICAM2 exon1-R | TGGAGTCTCCCATTTACCCACT | Primer | in vitro validation (SURVEYOR assay) |
| ICAM2 exon2-F | GACCCAGGAGAAGTCACTGAGC | Primer | in vitro validation (SURVEYOR assay) |
| ICAM2 exon2-R | TGGTAATAGAGAGCCCCAAGGA | Primer | in vitro validation (SURVEYOR assay) |
| ICAM2 exon3-F | CACGCTCACTTCCTCTTTCTCA | Primer | in vitro validation (SURVEYOR assay) |
| ICAM2 exon3-R | CCTAGGAGTGAGGCTCGTGAGT | Primer | in vitro validation (SURVEYOR assay) |
| ICAM2 exon4-F<br>ICAM2 exon4-R | TGGCTTAGAACATAGGCCACCT<br>TACCTTCCCTGGCATAGGTGTT | Primer<br>Primer | in vitro validation (SURVEYOR assay)<br>in vitro validation (SURVEYOR assay) |
| ICAM2-Target1-F | TAGACTCCACAGACCCCACAGA | Primer | 7C1/systemic injection (indel analysis) |
| ICAM2-Target1-R | AGGAGTATTAGTACTTCCCACCCTAA | Primer | 7C1/systemic injection (indel analysis) |
| ICAM2-Target2-F | CGTGGTGGTGTCAATACTGCT | Primer | 7C1/systemic injection (indel analysis) |
| ICAM2-Target2-R | TGAAGGTGCCATGTTGAGTTC | Primer | 7C1/systemic injection (indel analysis) |
| Myd88-F | TGCCATG TCTGCGGGAGA | Primer | Lenti/DC infection (indel analysis) |
| Myd88-R | GGCGGAGGAGATGGGCTTCGAG | Primer | Lenti/DC infection (indel analysis) |
| A20-F | CGAGAGAGAACCCCAGAAGAC | Primer | Lenti/DC infection (indel analysis) |
| A20-R | AGAAGTGTCCAGGCTTCCCT | Primer | Lenti/DC infection (indel analysis) |
| shMyd88 | GCGACTGATTCCTATTAAATA | shRNA | Lenti/DC infection (nanostring control) |
| shA20 | AGCTATCACTCATGGATATAA | shRNA | Lenti/DC infection (nanostring control) |

Supplementary Table S2 | Electrophysiological properties of wild-type and constitutively Cas9 expressing neurons

| Parameter | Wild-type | Cas9 |
|---|---|---|
| Rheobase current (pA) | 213.6 ± 16.64 | 210.0 ± 22.99 |

(continued)

| Parameter | Wild-type | Cas9 |
|---|---|---|
| Input resistance (MΩ) | 107.7 ± 7.773 | 110.8 ± 10.47 |
| Whole-cell capacitance (pF) | 234.8 ± 11.78 | 235.6 ± 16.12 |
| Membrane resting potential (mV) | -73.62 ± 1.381 | -72.83 ± 1.468 |

*Example 28: Particle-mediated delivery of CRISPR-Cas9 components into hematopoietic stem cells (HSCs)*

Applicants have demonstrated that Cas9 can be delivered to cells via particles. In this instance, in that many nucleic therapeutics may require the delivery of both the one or more sgRNA and the Cas9 nuclease concurrently, we demonstrated the ability to deliver in this fashion.

The sgRNA was pre-complexed with the Cas9 protein, before formulating the entire complex in a particle. Twenty different particle formulations were generated and tested for CRISPR-Cas9 delivery efficiency into cells. Each formulation was made with a different molar ratio of four components known to promote delivery of nucleic acids into cells: 1,2-dioleoyl-3-trimethylammonium-propane (DOTAP), 1,2-ditetradecanoyl-*sn*-glycero-3-phosphocholine (DMPC), polyethylene glycol (PEG), and cholesterol (e.g. DOTAP : DMPC : PEG : Cholesterol Molar Ratios; Formulation number 1 = DOTAP 100, DMPC 0, PEG 0, Cholesterol 0; Formulation number 2 = DOTAP 90, DMPC 0, PEG 10, Cholesterol 0; Formulation number 3 = DOTAP 90, DMPC 0, PEG 5, Cholesterol 5).

Particles were formed using an efficient, multistep process. First, Cas9 protein and sgRNA targeting the gene EMX1 or the control gene LacZ were mixed together at a 1:1 molar ratio at room temperature for 30 minutes in sterile, nuclease free IX PBS. Separately, DOTAP, DMPC, PEG, and cholesterol were dissolved in 100% ethanol. The two solutions were mixed together to form particles containing the Cas9-sgRNA complexes. After the particles were formed, HSCs in 96 well plates were transfected with 15ug Cas9 protein per well. Three days after transfection, HSCs were harvested, and the number of insertions and deletions (indels) at the EMX1 locus were quantified.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention.

Further features of the invention are set forth in the following numbered clauses.

1. A composition comprising a delivery particle formulation comprising a CRISPR-Cas system RNA polynucleotide sequence, wherein the polynucleotide sequence comprises:

   (a) a guide sequence capable of hybridizing to a target sequence in a eukaryotic cell,
   (b) a tracr mate sequence, and
   (c) a tracr sequence

   wherein (a), (b) and (c) are arranged in a 5' to 3' orientation,
   wherein when transcribed, the tracr mate sequence hybridizes to the tracr sequence and the guide sequence directs sequence-specific binding of a CRISPR complex to the target sequence.

2. The composition of clause 1 further comprising a polynucleotide sequence encoding a CRISPR enzyme, optionally comprising at least one or more nuclear localization sequences, wherein the CRISPR complex comprises the CRISPR enzyme complexed with (1) the guide sequence that is hybridized to the target sequence, and (2) the tracr mate sequence that is hybridized to the tracr sequence and the polynucleotide sequence encoding a CRISPR enzyme is DNA or RNA.
3. The composition of clause 2, wherein the polynucleotide sequence is comprised within a vector system comprising one or more vectors.
4. The composition of clause 2, wherein the CRISPR enzyme is a Cas 9.
5. The composition of clause 1, wherein the guide sequence, tracr mate sequence or tracr sequence is/are RNA and comprised within a vector system comprising one or more vectors
6. The composition of clause 1, wherein the delivery particle formulation comprises liposomes, nanoparticles, exosomes, microvesicles, or a gene gun.
7. The composition of clause 6, wherein the delivery particles are less than 500 nm in diameter.
8. The composition of clause 6, wherein the delivery particles are less than 250 nm in diameter.
9. The composition of clause 6, wherein the delivery particles are less than 100 nm in diameter.

10. The composition of clause 6, wherein the delivery particles are about 35 nm to about 60 nm in diameter.

11. The composition of clause 6 wherein the delivery particle formulation is a nanoparticle formulation.

12. The composition of clause 11, wherein the nanoparticle formulation comprises a lipid-based nanoparticle.

13. The composition of clause 12, wherein the lipid-based nanoparticle comprises an epoxide-modified lipid polymer.

14. The composition of clause 1 comprising two or more of (a), (b), and/or (c).

15. The composition of clause 1, wherein the target sequence is an endothelial target sequence.

16. The composition of clause 1, wherein the target sequence is a lung or skin target sequence.

17. The composition of clause 1, wherein the target sequence is a cardiac or muscle target sequence.

18. The composition of clause 1, wherein the delivery particle formulation is provided as a pharmaceutical composition.

19. The composition of clause 2, wherein the delivery particle formulation is provided as a pharmaceutical composition.

20. The composition of clause 18 further comprising pharmaceutically acceptable excipients and or carriers for *ex vivo* and/or *in vivo* administration.

21. The composition of clause 19 further comprising pharmaceutically acceptable excipients and or carriers for *ex vivo* and/or *in vivo* administration.

22. A composition comprising a delivery particle formulation comprising:

I. polynucleotides comprising:

(a) a guide sequence capable of hybridizing to a target sequence in a eukaryotic cell, and
(b) at least one or more tracr mate sequences,

II. a polynucleotide sequence encoding a CRISPR enzyme, and
III. a polynucleotide sequence comprising a tracr sequence,

wherein when transcribed, the tracr mate sequence hybridizes to the tracr sequence and the guide sequence directs sequence-specific binding of a CRISPR complex to the target sequence,
wherein the CRISPR complex comprises the CRISPR enzyme complexed with (1) the guide sequence that is hybridized to the target sequence, and (2) the tracr mate sequence that is hybridized to the tracr sequence, and the polynucleotide sequence encoding a CRISPR enzyme is DNA or RNA, and
wherein the delivery particles are less than 250 nm in diameter.

23. The composition of clause 22, wherein the deliver particle is a formulation comprises a liposome, a nanoparticle, an exosome, a microvesicle, or a gene gun.

24. The composition of clause 22, wherein the target sequence is an endothelial target sequence.

25. A method of modifying an organism or a non-human organism by manipulation of a target sequence in a genomic locus of interest comprising administering a composition comprising administering a delivery particle formulation comprising a CRISPR-Cas system RNA polynucleotide sequence, wherein the polynucleotide sequence comprises:

(a) a guide sequence capable of hybridizing to a target sequence in a eukaryotic cell,
(b) a tracr mate sequence, and
(c) a tracr sequence

wherein (a), (b) and (c) are arranged in a 5' to 3' orientation,
wherein when transcribed, the tracr mate sequence hybridizes to the tracr sequence and the guide sequence directs sequence-specific binding of a CRISPR complex to the target sequence.

26. The method of clause 25, further comprising administering a polynucleotide sequence encoding a CRISPR enzyme, optionally comprising at least one or more nuclear localization sequences, wherein the CRISPR complex comprises the CRISPR enzyme complexed with (1) the guide sequence that is hybridized to the target sequence, and (2) the tracr mate sequence that is hybridized to the tracr sequence and the polynucleotide sequence encoding a CRISPR enzyme is DNA or RNA.

27. The method of clause 26, wherein the the CRISPR enzyme is a Cas9.

28. The method of clause 26, wherein the polynucleotide or enzyme coding sequence encoding the CRISPR enzyme is delivered to the cell by delivering mRNA encoding the CRISPR enzyme to the cell.

29. The method of clause 25, wherein the delivery particle formulation comprises a liposome, a nanoparticle, an exosome, a microvesicle, or a gene gun.

30. The method of clause 29, wherein the delivery particles are less than 250 nm in diameter.

31. The method of clause 29, wherein the delivery particles are less than 100 nm in diameter.

32. The method of clause 29, wherein the delivery particles are about 35 nm to about 60 nm in diameter.
33. The method of clause 29, wherein the delivery particle formulation is a nanoparticle formulation.
34. The method of clause 33, wherein the nanoparticle formulation comprises a lipid-based nanoparticle.
35. The method of clause 34, wherein the lipid-based nanoparticle comprises an epoxide-modified lipid polymer.
36. The method of clause 26, wherein the method is carried out *in vitro, ex vivo,* and/or *in vivo.*
37. The method of clauses 26, wherein the organism or subject is a eukaryote.
38. The method of any of clause 26, wherein the organism or subject is a mammal or a non-human mammal.
39. The method of clause 26, wherein the modification comprises functional gene silencing.
40. The method of clause 39, wherein the functional gene silencing comprises a reduction in mRNA and/or protein levels of a targeted gene.
41. The method of clause 40, wherein the targeted gene is expressed in endothelial cells.
42. The method of clause 40, wherein the targeted gene is expressed in lung or skin.
43. The method of clause 40, wherein the targeted gene is expressed in heart or muscle.
44. The method of clause 40, wherein the functional gene silencing provides a therapeutic benefit.
45. A method of modifying an organism or a non-human organism by manipulation of a target sequence in a genomic locus of interest comprising administering a composition comprising a delivery particle formulation comprising:

I. polynucleotides comprising:

(a) a guide sequence capable of hybridizing to a target sequence in a eukaryotic cell, and
(b) at least one or more tracr mate sequences,

II. a polynucleotide sequence encoding a CRISPR enzyme, and
III. a polynucleotide sequence comprising a tracr sequence,

wherein when transcribed, the tracr mate sequence hybridizes to the tracr sequence and the guide sequence directs sequence-specific binding of a CRISPR complex to the target sequence,
wherein the CRISPR complex comprises the CRISPR enzyme complexed with (1) the guide sequence that is hybridized to the target sequence, and (2) the tracr mate sequence that is hybridized to the tracr sequence, and the polynucleotide sequence encoding a CRISPR enzyme is DNA or RNA, and
wherein the delivery particles are less than 250 nm in diameter.
46. The method of clause 45, wherein the delivery particle formulation comprises a liposome, a nanoparticle, an exosomes, a microvesicle, or a gene gun.
47. The method of clause 45, wherein the target sequence is an endothelial target sequence.
48. A method of preparing an sgRNA-and-Cas9 protein containing particle comprising admixing an sgRNA and Cas9 protein mixture with a mixture comprising or consisting essentially of or consisting of surfactant, phospholipid, biodegradable polymer, lipoprotein and alcohol.
49. An sgRNA-and-Cas9 protein containing particle from the method of clause 48.
50. Use of the particle of clause 49 in a method of modifying a genomic locus of interest, or an organism or a non-human organism by manipulation of a target sequence in a genomic locus of interest, comprising contacting a cell containing the genomic locus of interest with the particle wherein the sgRNA targets the genomic locus of interest; or a method of modifying a genomic locus of interest, or an organism or a non-human organism by manipulation of a target sequence in a genomic locus of interest, comprising contacting a cell containing the genomic locus of interest with the particle wherein the sgRNA targets the genomic locus of interest.

### Claims

1. A method of preparing an sgRNA-and-Cas9 protein containing particle comprising admixing an sgRNA and Cas9 protein mixture with a mixture comprising surfactant, phospholipid, biodegradable polymer, lipoprotein and alcohol.

2. An sgRNA-and-Cas9 protein containing particle obtained from the method of claim 1.

3. Use of the particle of claim 2 in a method of modifying a genomic locus of interest, or a non-human organism by manipulation of a target sequence in a genomic locus of interest, comprising contacting a cell containing the genomic locus of interest with the particle wherein the sgRNA targets the genomic locus of interest.

4. The method of claim 1, the particle of claim 2, or the use of claim 3, wherein:

(i) the surfactant comprises a cationic lipid, preferably 1,2-dioleoyl-3-trimethylammonium-propane (DOTAP);
(ii) the phospholipid comprises 1,2-ditetradecanoyl-sn-glycero-3-phosphocholine (DMPC);
(iii) the biodegradable polymer comprises an ethylene-glycol polymer, preferably polyethylene glycol (PEG); and/or
(iv) the lipoprotein comprises a low-density lipoprotein, preferably cholesterol.

5. A delivery particle formulation comprising a particle containing a Cas protein and a guide RNA, wherein the particle comprises cationic lipid and biodegradable polymer.

6. The delivery particle formulation of claim 5, wherein the Cas protein and the guide RNA forms a CRISPR-Cas complex.

7. The delivery particle formulation of any one of claims 5 to 6, wherein the cationic lipid comprises 1,2-dioleoyl-3-trimethylammonium-propane (DOTAP).

8. The delivery particle formulation of any one of claims 5 to 7, wherein the biodegradable polymer comprises ethylene glycol or polyethylene glycol.

9. The delivery particle formulation of any one of claims 5 to 8, wherein the particle further comprises a lipoprotein, preferably cholesterol.

10. The delivery particle formulation of any one of claims 5 to 9, wherein the particle further comprises a phospholipid, preferably 1,2-ditetradecanoyl-sn-glycero-3-phosphocholine (DMPC).

11. The delivery particle formulation of any one of claims 5 to 10, wherein the guide RNA is a sgRNA comprising:

(a) a guide sequence capable of hybridizing to a target sequence in a eukaryotic cell,
(b) a tracr mate sequence, and
(c) a tracr sequence.

12. The delivery particle formulation of any one of claims 5 to 11, wherein the Cas protein is Cas9.

13. The delivery particle formulation of claim 12, wherein the Cas9 is Streptococcus pyogenes Cas9 (SpCas9) or Staphylococcus aureus Cas9 (SaCas9).

14. The delivery particle formulation of any one of claims 12 to 13, wherein the Cas9 is a nickase, or is mutated, or has reduced or abolished nuclease activity.

15. The delivery particle formulation of any one of claims 12 to 14, wherein the Cas9 includes one or more heterologous protein domains.

16. The delivery particle formulation of claim 5, wherein:

preferably, the cationic lipid comprises DOTAP;
preferably, the biodegradable polymer comprises ethylene glycol or polyethylene glycol;
the particle further comprises a lipoprotein, preferably cholesterol; and
the particle further comprises a phospholipid, preferably DMPC.

17. The delivery particle formulation of claim 16, wherein:

the Cas protein is Cas9; and
the guide RNA is a sgRNA comprising (a) a guide sequence capable of hybridizing to a target sequence in a eukaryotic cell, (b) a tracr mate sequence, and (c) a tracr sequence.

**18.** The delivery particle formulation of claim 17, wherein the particle or a component thereof comprises a barcode.

**19.** A method of delivering a CRISPR-Cas complex to a cell, or method of modifying expression of a polynucleotide in a eukaryotic cell, said method comprising contacting the cell with the delivery particle formulation of any one of claims 5 to 18.

**20.** The method of claim 1, wherein the sgRNA is pre-complexed with the Cas9 protein before formulating the complex in a particle.

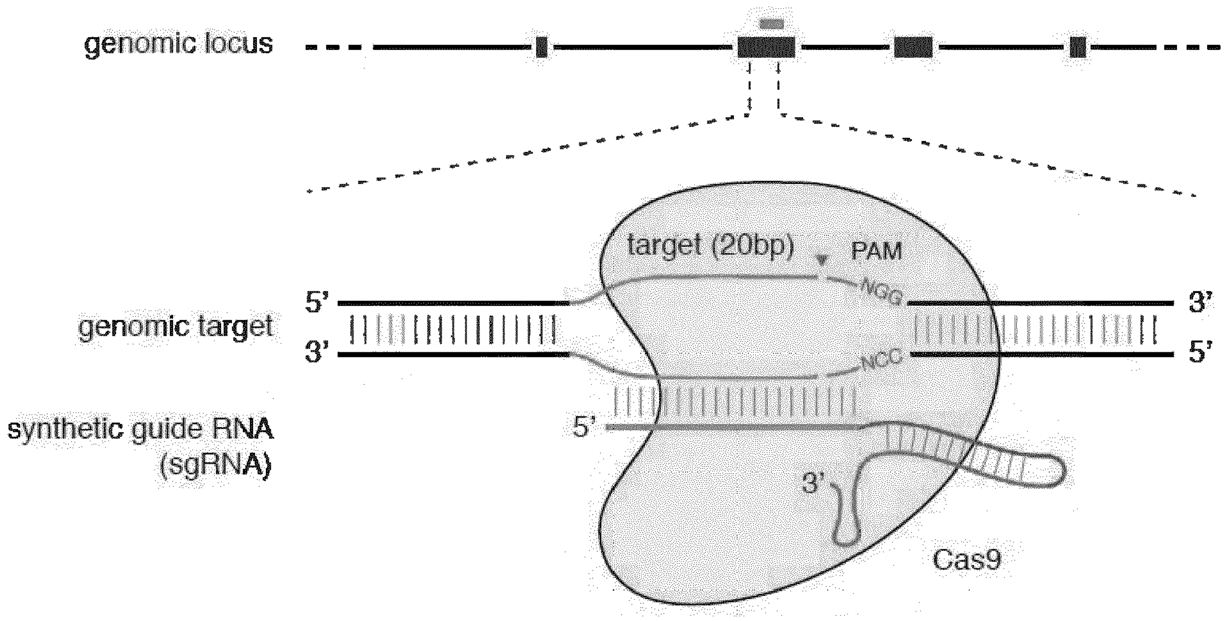
genomic locus
target (20bp)
PAM
NGG
genomic target
5'
3'
3'
5'
NCC
synthetic guide RNA
(sgRNA)
5'
3'
Cas9

FIG. 1

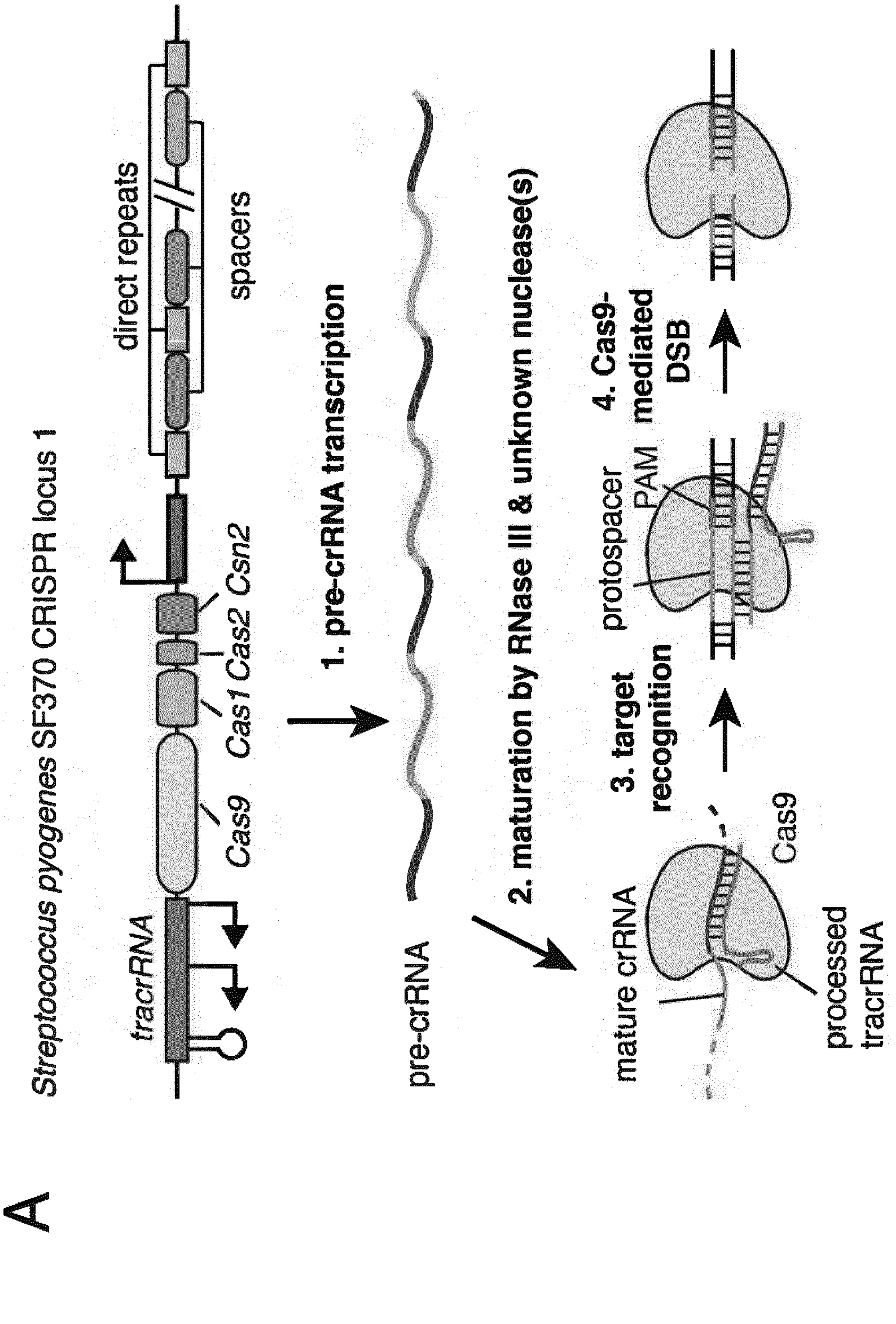
A
Streptococcus pyogenes SF370 CRISPR locus 1
tracrRNA
Cas9
Cas1
Cas2
Csn2
direct repeats
spacers
1. pre-crRNA transcription
pre-crRNA
2. maturation by RNase III & unknown nuclease(s)
mature crRNA
Cas9
processed tracrRNA
3. target recognition
protospacer
PAM
4. Cas9-mediated DSB

FIG. 2A

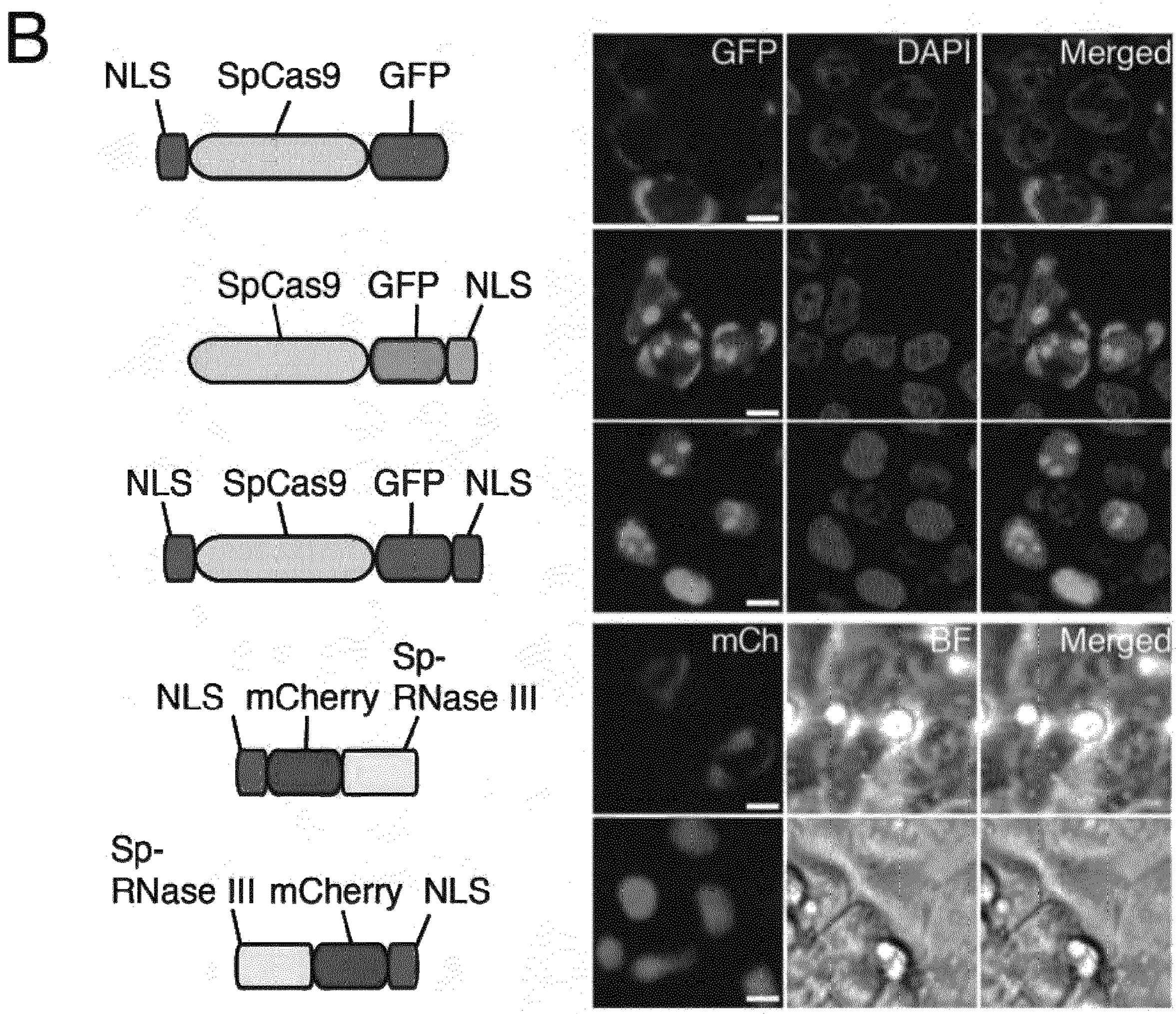
B
NLS
SpCas9
GFP
SpCas9
GFP
NLS
NLS
SpCas9
GFP
NLS
Sp-
NLS
mCherry
RNase III
Sp-
RNase III
mCherry
NLS
GFP
DAPI
Merged
mCh
BF
Merged

FIG. 2B

C

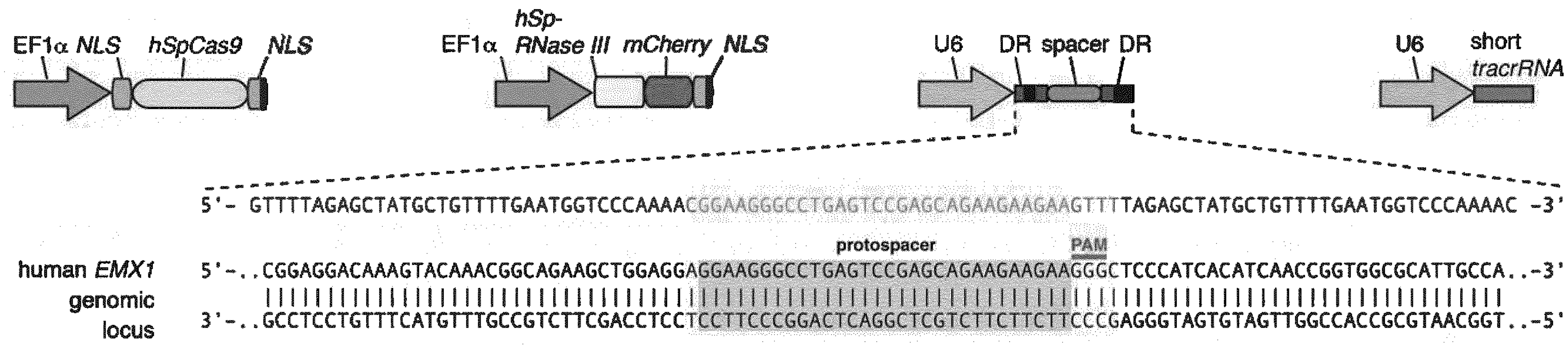
EF1α NLS
hSpCas9
NLS
EF1α
hSp-
RNase III
mCherry
NLS
U6
DR
spacer
DR
U6
short
tracrRNA
5'- GTTTTAGAGCTATGCTGTTTTGAATGGTCCCAAAACGGAAGGGCCTGAGTCCGAGCAGAAGAAGAAGTTTTAGAGCTATGCTGTTTTGAATGGTCCCAAAAC -3'
protospacer
PAM
human EMX1
genomic
locus
5'-..CGGAGGACAAAGTACAAACGGCAGAAGCTGGAGGAGGAAGGGCCTGAGTCCGAGCAGAAGAAGAAGGGCTCCCATCACATCAACCGGTGGCGCATTGCCA..-3'
3'-..GCCTCCTGTTTCATGTTTGCCGTCTTCGACCTCCTCCTTCCCGGACTCAGGCTCGTCTTCTTCTTCCCGAGGGTAGTGTAGTTGGCCACCGCGTAACGGT..-5'

FIG. 2C

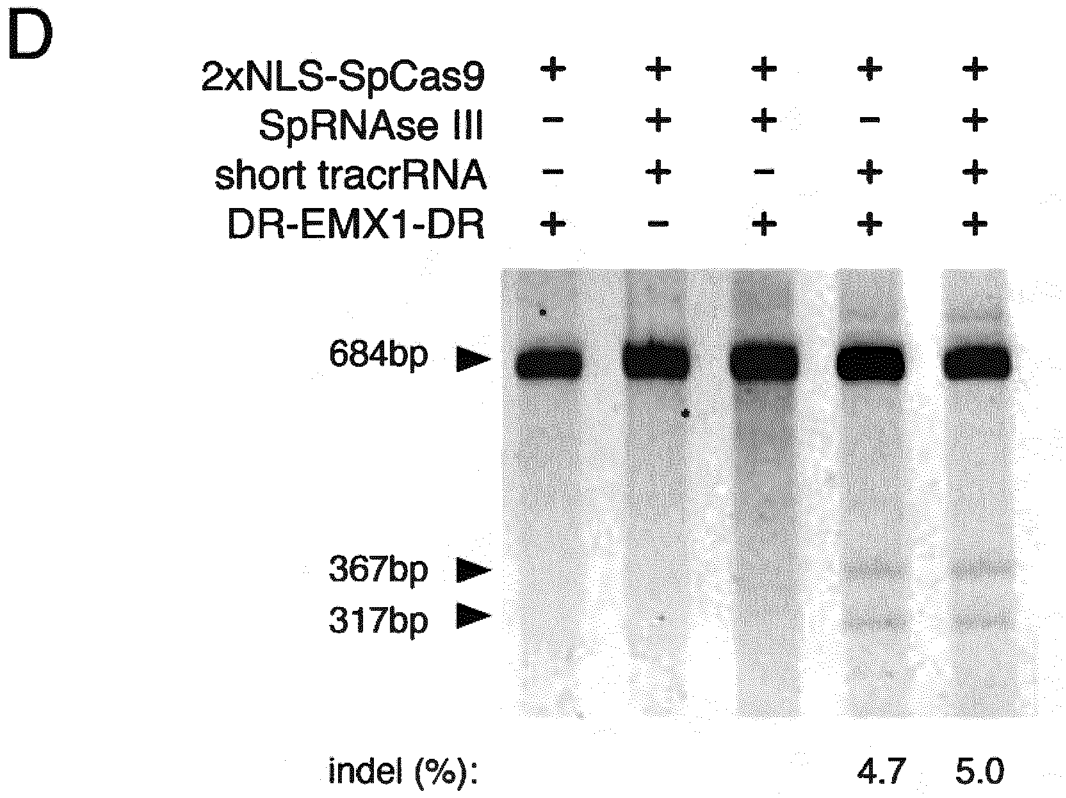
D
2xNLS-SpCas9 + + + + +
SpRNAse III − + + − +
short tracrRNA − + − + +
DR-EMX1-DR + − + + +
684bp
367bp
317bp
indel (%): 4.7 5.0

FIG. 2D

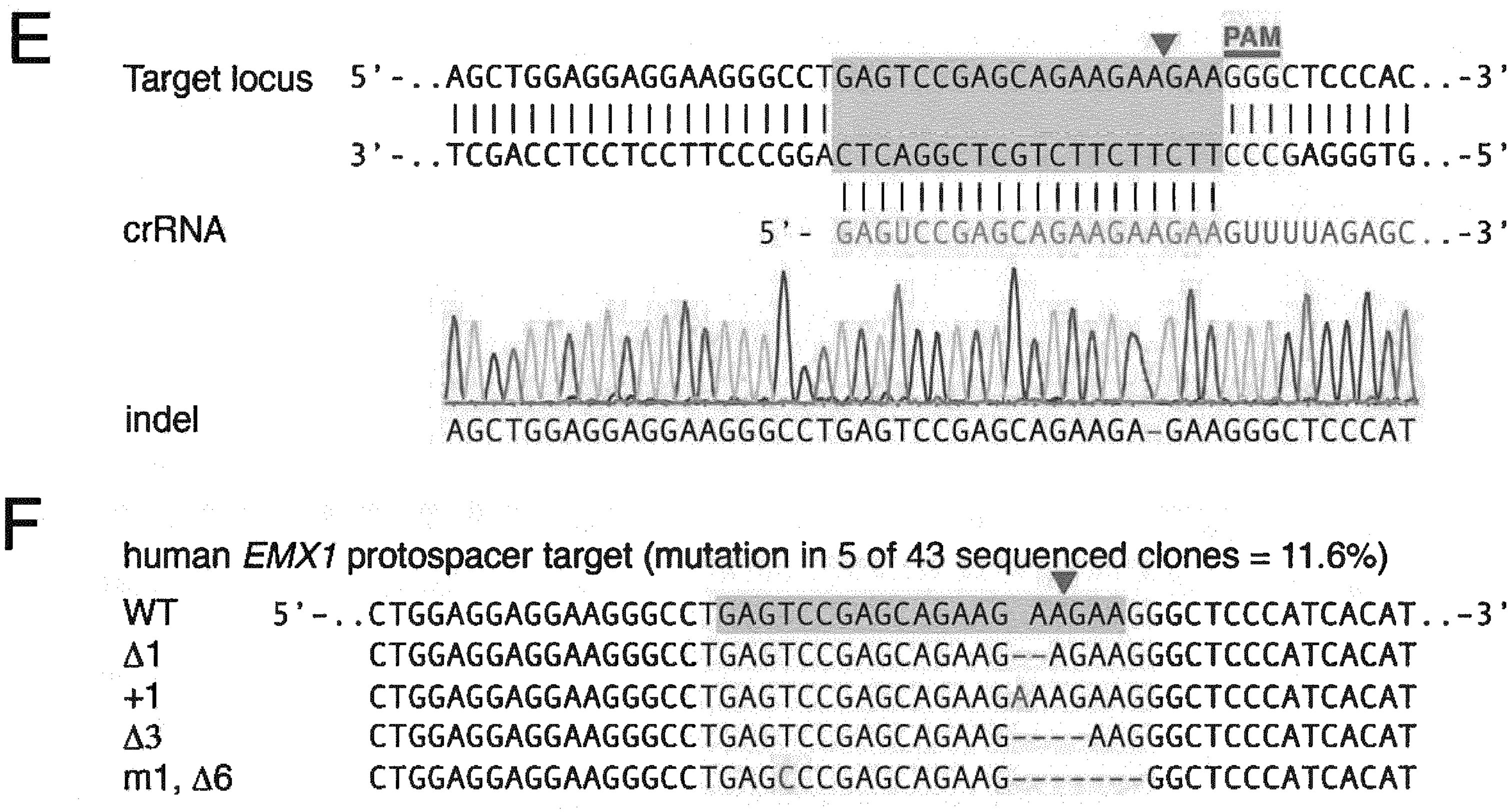
E
PAM
Target locus 5'-..AGCTGGAGGAGGAAGGGCCTGAGTCCGAGCAGAAGAAGAAGGGCTCCCAC..-3'
3'-..TCGACCTCCTCCTTCCCGGACTCAGGCTCGTCTTCTTCTTCCCGAGGGTG..-5'
crRNA 5'- GAGUCCGAGCAGAAGAAGAAGUUUUAGAGC..-3'
indel AGCTGGAGGAGGAAGGGCCTGAGTCCGAGCAGAAGA-GAAGGGCTCCCAT
F
human EMX1 protospacer target (mutation in 5 of 43 sequenced clones = 11.6%)
WT 5'-..CTGGAGGAGGAAGGGCCTGAGTCCGAGCAGAAG AAGAAGGGCTCCCATCACAT..-3'
Δ1 CTGGAGGAGGAAGGGCCTGAGTCCGAGCAGAAG--AGAAGGGCTCCCATCACAT
+1 CTGGAGGAGGAAGGGCCTGAGTCCGAGCAGAAGAAAGAAGGGCTCCCATCACAT
Δ3 CTGGAGGAGGAAGGGCCTGAGTCCGAGCAGAAG----AAGGGCTCCCATCACAT
m1, Δ6 CTGGAGGAGGAAGGGCCTGAGCCCGAGCAGAAG-------GGCTCCCATCACAT

FIG. 2E-F

**A**

protospacer (1) ▼ PAM

human *EMX1* locus
5'-..CTGGAGGAGGAAGGGCCTGAGTCCGAGCAGAAGAAGAAGGGCTCCCAT..-3'
3'-..GACCTCCTCCTTCCCGGACTCAGGCTCGTCTTCTTCTTCCCGAGGGTA..-5'

wt crRNA 5'- GAGUCCGAGCAGAAGAAGAAGUUUUAGAGC..-3'

mismatch-containing guide sequences
m1 GAGUCCGAGCAGAAGAAGAU
m2 GAGUCCGAGCAGAAGAAGUA
m3 GAGUCCGAGCAGAAGAACAA
m4 GAGUCCGAGCAGAAGAUGAA
m5 GAGUCCGAGCAGAAGUAGAA
m7 GAGUCCGAGCAGAUGAAGAA
m9 GAGUCCGAGCACAAGAAGAA
m11 GAGUCCGAGGAGAAGAAGAA
m13 GAGUCCGUGCAGAAGAAGAA
m15 GAGUCGGAGCAGAAGAAGAA
m17 GAGACCGAGCAGAAGAAGAA

**B**

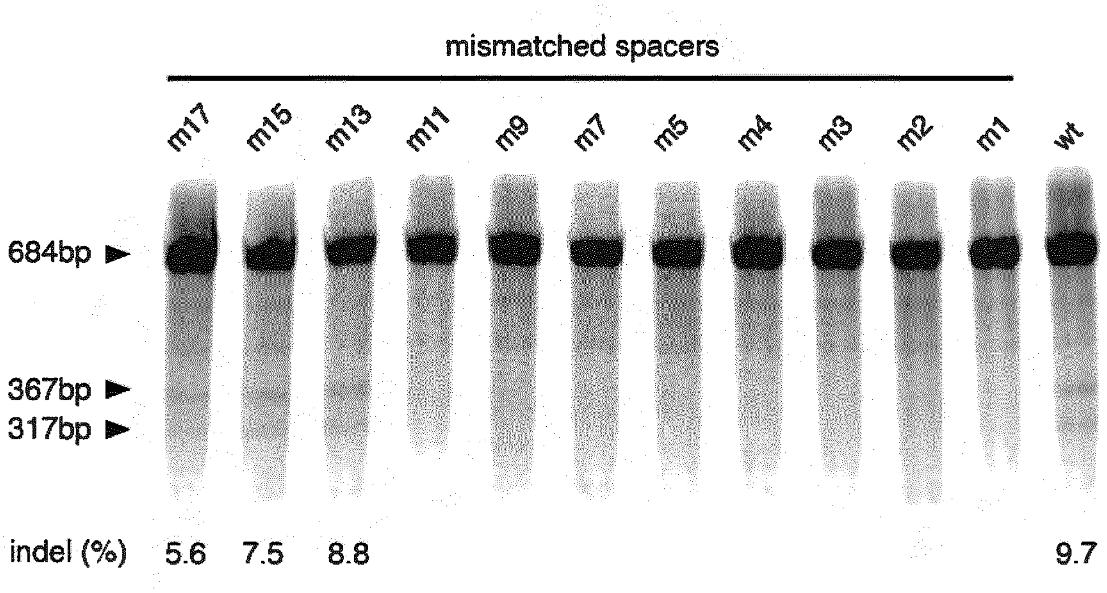


**C**

left TALEN binding site protospacer (1) ▼ PAM

human *EMX1* locus
5'-..CTGGAGGAGGAAGGGCCTGAGTCCGAGCAGAAGAAGAAGGGCTCCCAT..-3'
3'-..GACCTCCTCCTTCCCGGACTCAGGCTCGTCTTCTTCTTCCCGAGGGTA..-5'

right TALEN binding site

**FIG. 3A-C**

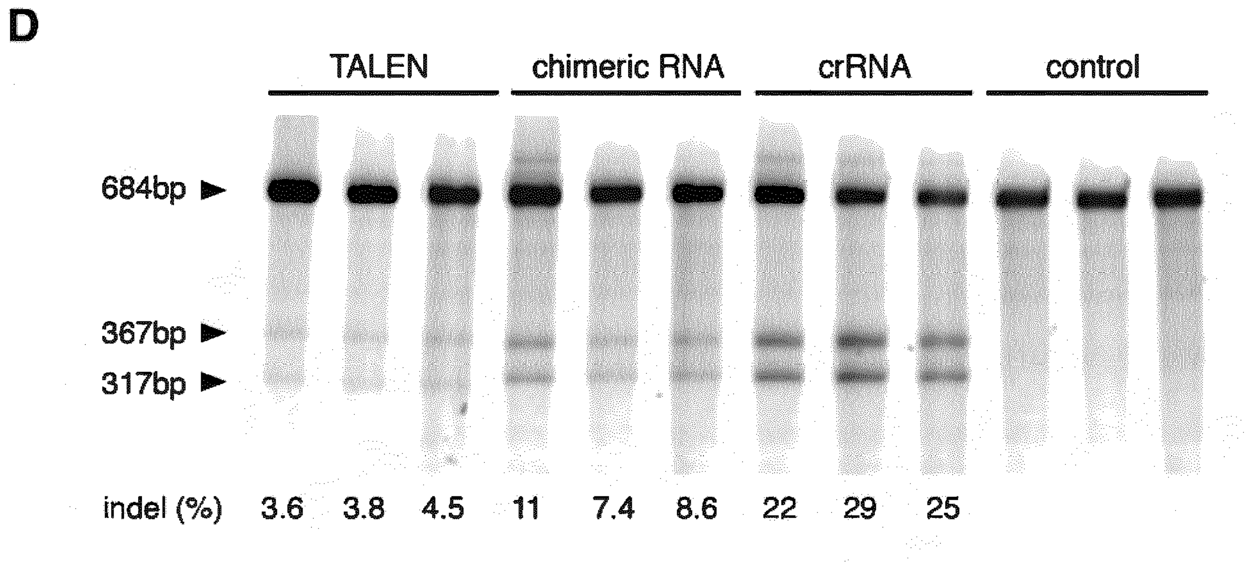
D
TALEN
chimeric RNA
crRNA
control
684bp
367bp
317bp
indel (%)
3.6
3.8
4.5
11
7.4
8.6
22
29
25

FIG. 3D

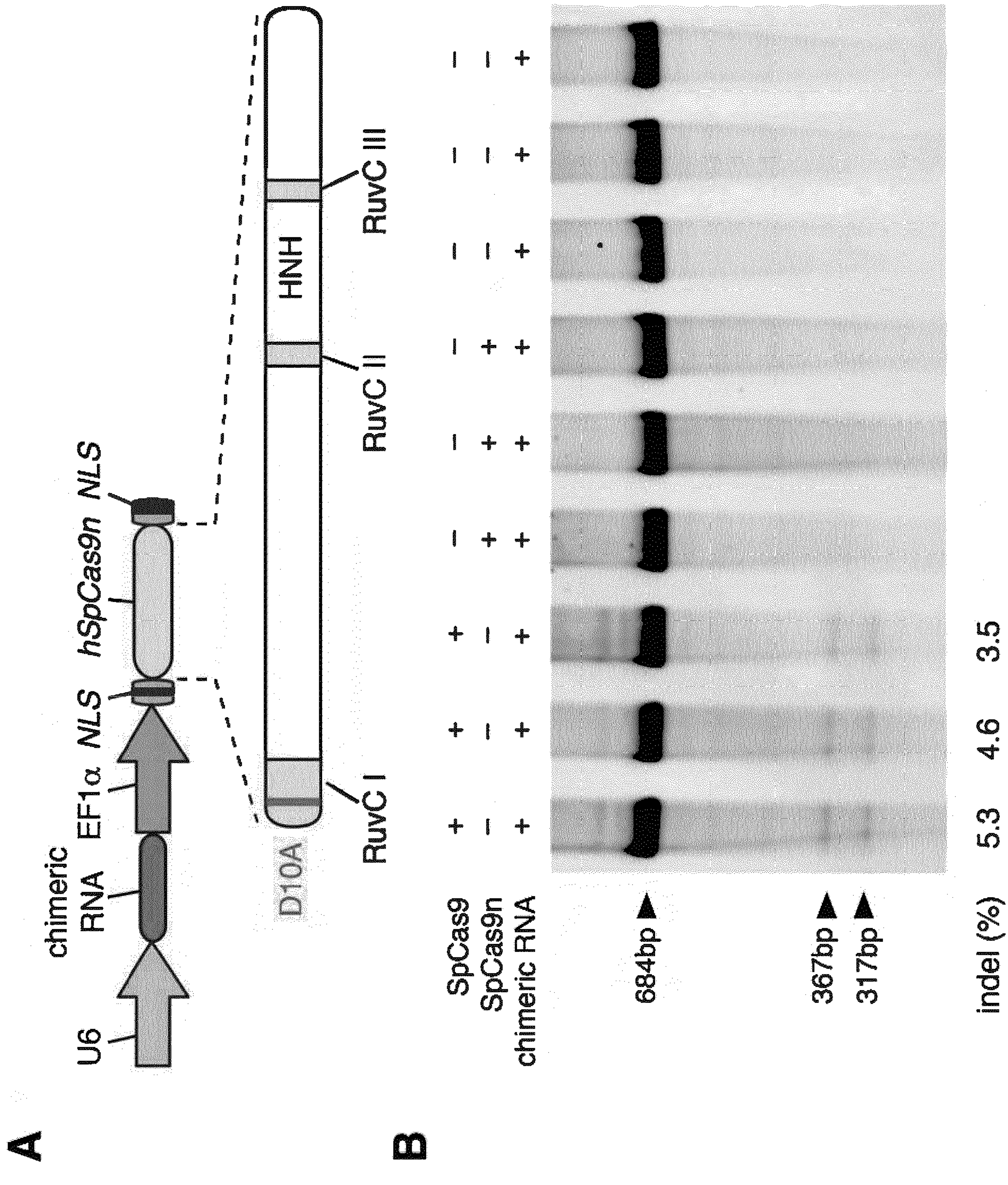
A
U6
chimeric RNA
EF1α
NLS
hSpCas9n
NLS
D10A
RuvC I
RuvC II
HNH
RuvC III
B
SpCas9
SpCas9n
chimeric RNA
684bp
367bp
317bp
indel (%)
5.3
4.6
3.5

FIG. 4A-B

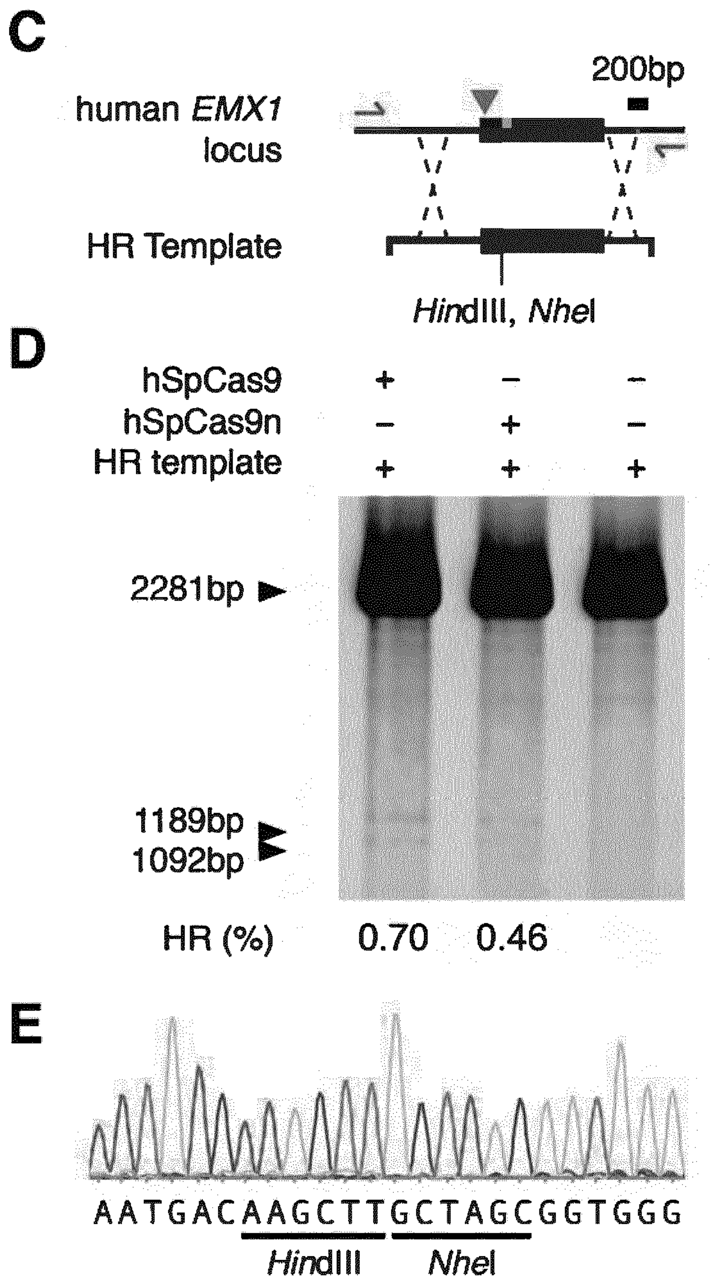
C
200bp
human EMX1
locus
HR Template
HindIII, NheI
D
hSpCas9 + − −
hSpCas9n − + −
HR template + + +
2281bp
1189bp
1092bp
HR (%) 0.70 0.46
E
AATGACAAGCTTGCTAGCGGTGGG
HindIII
NheI

FIG. 4C-E

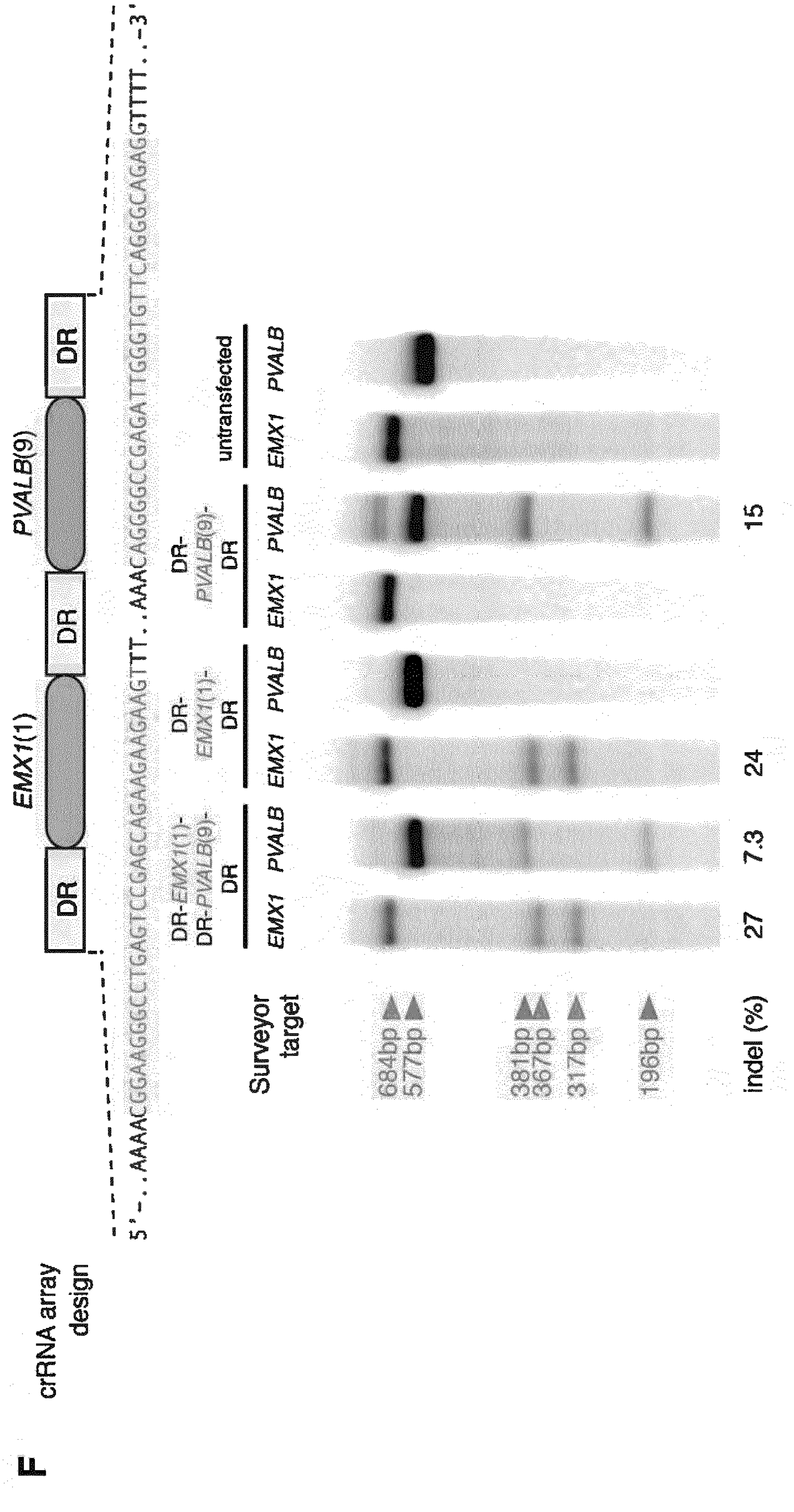
F
crRNA array design
5'-..AAAACGGAAGGGCCTGAGTCCGAGCAGAAGAAGAAGTTT..AAACAGGGGCCGAGATTGGGTGTTCAGGGCAGAGGTTTT..-3'
DR
EMX1(1)
DR
PVALB(9)
DR
Surveyor target
DR-EMX1(1)-DR-PVALB(9)-DR
DR-EMX1(1)-DR
DR-PVALB(9)-DR
untransfected
EMX1
PVALB
EMX1
PVALB
EMX1
PVALB
EMX1
PVALB
684bp
577bp
381bp
367bp
317bp
196bp
indel (%)
27
7.3
24
15

FIG. 4F

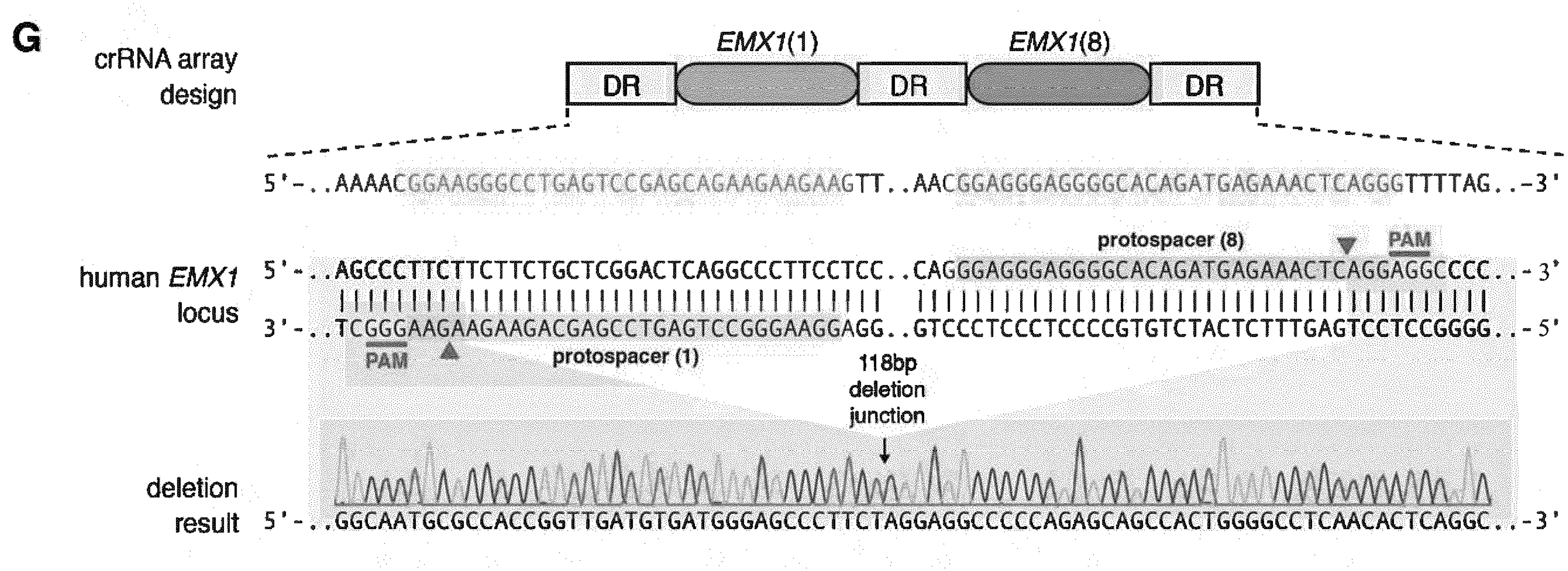
G
crRNA array design
EMX1(1)
EMX1(8)
DR
DR
DR
5'-..AAAACGGAAGGGCCTGAGTCCGAGCAGAAGAAGAAGTT..AACGGAGGGAGGGGCACAGATGAGAAACTCAGGGTTTTAG..-3'
human EMX1 locus
protospacer (8)
PAM
5'-..AGCCCTTCTTCTTCTGCTCGGACTCAGGCCCTTCCTCC..CAGGGAGGGAGGGGCACAGATGAGAAACTCAGGAGGCCCC..-3'
3'-..TCGGGAAGAAGAAGACGAGCCTGAGTCCGGGAAGGAGG..GTCCCTCCCTCCCCGTGTCTACTCTTTGAGTCCTCCGGGG..-5'
PAM
protospacer (1)
118bp deletion junction
deletion result
5'-..GGCAATGCGCCACCGGTTGATGTGATGGGAGCCCTTCTAGGAGGCCCCCAGAGCAGCCACTGGGGCCTCAACACTCAGGC..-3'

FIG. 4G

| Cas9 | target species | gene | protospacer ID | protospacer sequence (5' to 3') | PAM | strand | cell line tested | % indel (pre-crRNA + tracrRNA) | % indel (chimeric RNA) |
|---|---|---|---|---|---|---|---|---|---|
| *S. pyogenes SF370 type II CRISPR* | *Homo sapiens* | *EMX1* | 1 | GGAAGGGCCTGAGTCCGAGCAGAAGAAGAA | GGG | + | 293FT | 20 ± 1.8 | 6.7 ± 0.62 |
| | | *EMX1* | 2 | CATTGGAGGTGACATCGATGTCCTCCCCAT | TGG | – | 293FT | 2.1 ± 0.31 | N.D. |
| | | *EMX1* | 3 | GGACATCGATGTCACCTCCAATGACTAGGG | TGG | + | 293FT | 14 ± 1.1 | N.D. |
| | | *EMX1* | 4 | CATCGATGTCCTCCCCATTGGCCTGCTTCG | TGG | – | 293FT | 11 ± 1.7 | N.D. |
| | | *EMX1* | 5 | TTCGTGGCAATGCGCCACCGGTTGATGTGA | TGG | – | 293FT | 4.3 ± 0.46 | 2.1 ± 0.51 |
| | | *EMX1* | 6 | TCGTGGCAATGCGCCACCGGTTGATGTGAT | GGG | – | 293FT | 4.0 ± 0.66 | 0.41 ± 0.25 |
| | | *EMX1* | 7 | TCCAGCTTCTGCCGTTTGTACTTTGTCCTC | CGG | – | 293FT | 1.5 ± 0.12 | N.D. |
| | | *EMX1* | 8 | GGAGGGAGGGGCACAGATGAGAAACTCAGG | AGG | – | 293FT | 7.8 ± 0.83 | 2.3 ± 1.2 |
| | *Homo sapiens* | *PVALB* | 9 | AGGGGCCGAGATTGGGTGTTCAGGGCAGAG | AGG | + | 293FT | 21 ± 2.6 | 6.5 ± 0.32 |
| | | *PVALB* | 10 | ATGCAGGAGGGTGGCGAGAGGGGCCGAGAT | TGG | + | 293FT | N.D. | N.D. |
| | | *PVALB* | 11 | GGTGGCGAGAGGGGCCGAGATTGGGTGTTC | AGG | + | 293FT | N.D. | N.D. |
| | *Mus musculus* | *Th* | 12 | CAAGCACTGAGTGCCATTAGCTAAATGCAT | AGG | – | Neuro2A | 27 ± 4.3 | 4.1 ± 2.2 |
| | | *Th* | 13 | AATGCATAGGGTACCACCCACAGGTGCCAG | GGG | – | Neuro2A | 4.8 ± 1.2 | N.D. |
| | | *Th* | 14 | ACACACATGGGAAAGCCTCTGGGCCAGGAA | AGG | + | Neuro2A | 11.3 ± 1.3 | N.D. |
| *S. thermophilus LMD-9 CRISPR1* | *Homo sapiens* | *EMX1* | 15 | GGAGGAGGTAGTATACAGAAACACAGAGAA | GTAGAAT | – | 293FT | 14 ± 0.88 | N.T. |
| | | *EMX1* | 16 | AGAATGTAGAGGAGTCACAGAAACTCAGCA | CTAGAAA | – | 293FT | 7.8 ± 0.77 | N.T. |

FIG. 5

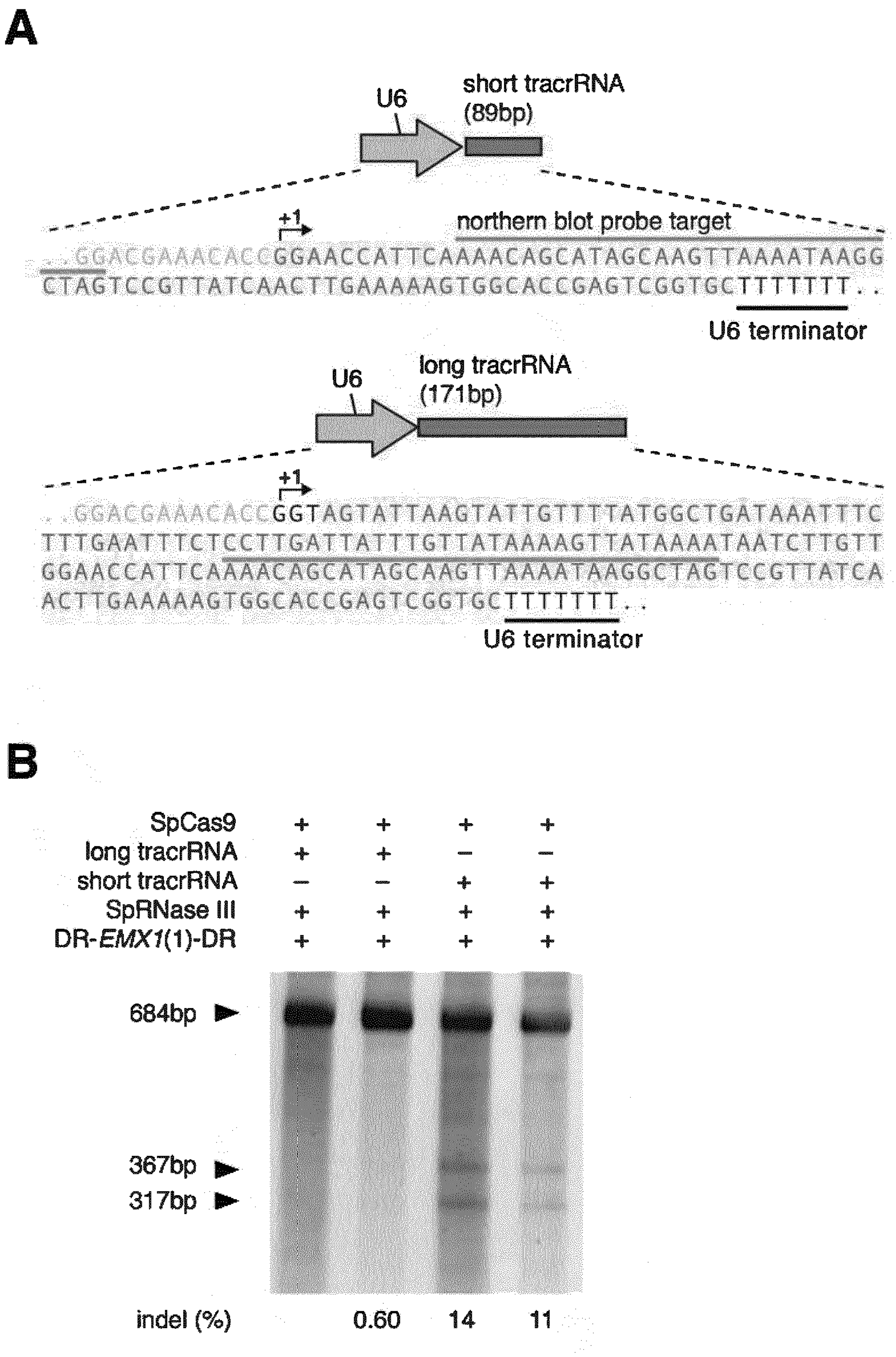
A
U6
short tracrRNA
(89bp)
+1
northern blot probe target
..GGACGAAACACCGGAACCATTCAAAACAGCATAGCAAGTTAAAATAAGG
CTAGTCCGTTATCAACTTGAAAAAGTGGCACCGAGTCGGTGCTTTTTTT..
U6 terminator
U6
long tracrRNA
(171bp)
+1
..GGACGAAACACCGGTAGTATTAAGTATTGTTTTATGGCTGATAAATTTC
TTTGAATTTCTCCTTGATTATTTGTTATAAAAGTTATAAAATAATCTTGTT
GGAACCATTCAAAACAGCATAGCAAGTTAAAATAAGGCTAGTCCGTTATCA
ACTTGAAAAAGTGGCACCGAGTCGGTGCTTTTTTT..
U6 terminator
B
SpCas9 + + + +
long tracrRNA + + − −
short tracrRNA − − + +
SpRNase III + + + +
DR-EMX1(1)-DR + + + +
684bp
367bp
317bp
indel (%) 0.60 14 11

FIG. 6A-B

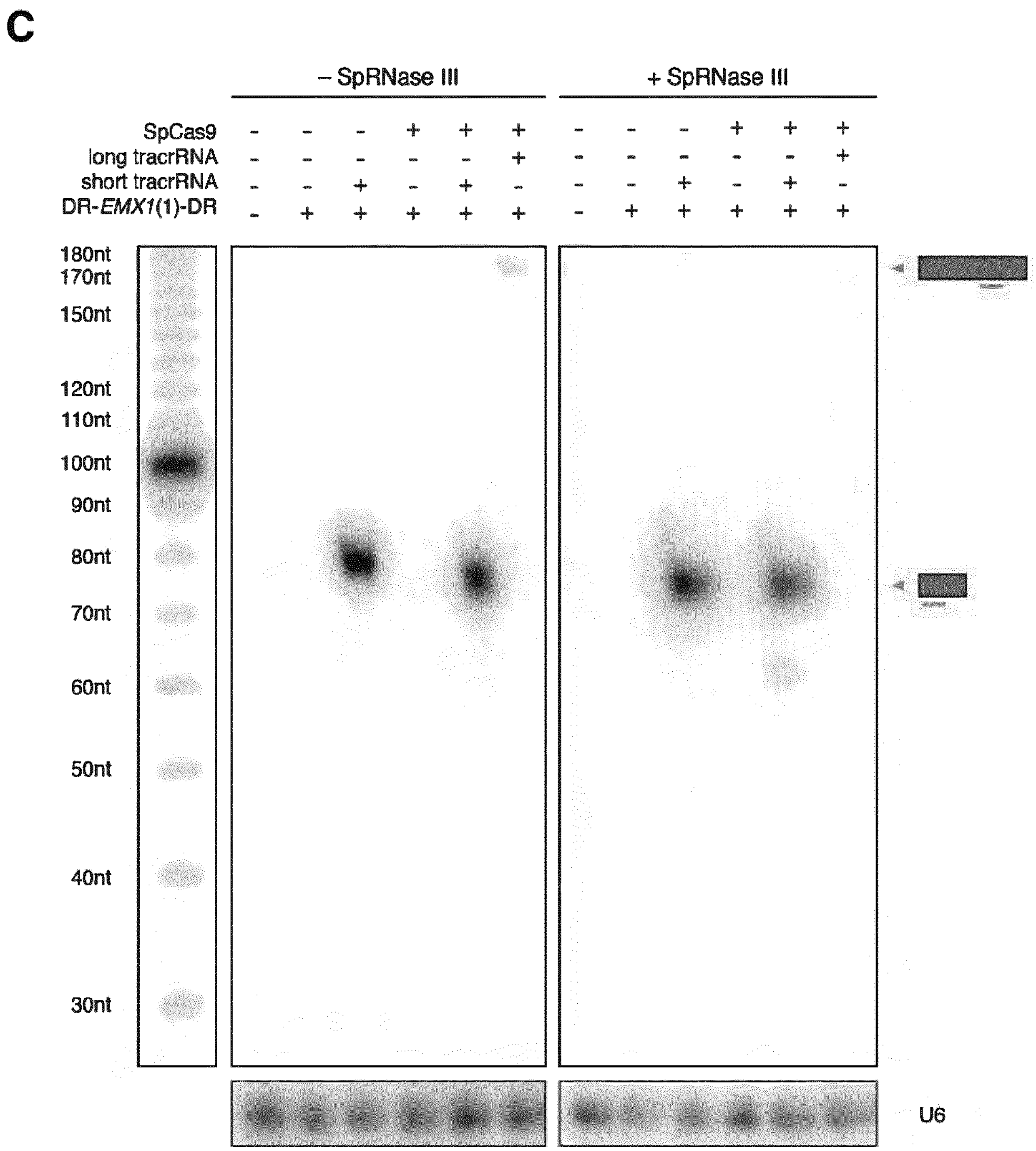
C
– SpRNase III
+ SpRNase III
SpCas9 - - - + + + - - - + + +
long tracrRNA - - - - - + - - - - - +
short tracrRNA - - + - + - - - + - + -
DR-*EMX1*(1)-DR - + + + + + - + + + + +
180nt
170nt
150nt
120nt
110nt
100nt
90nt
80nt
70nt
60nt
50nt
40nt
30nt
U6

FIG. 6C

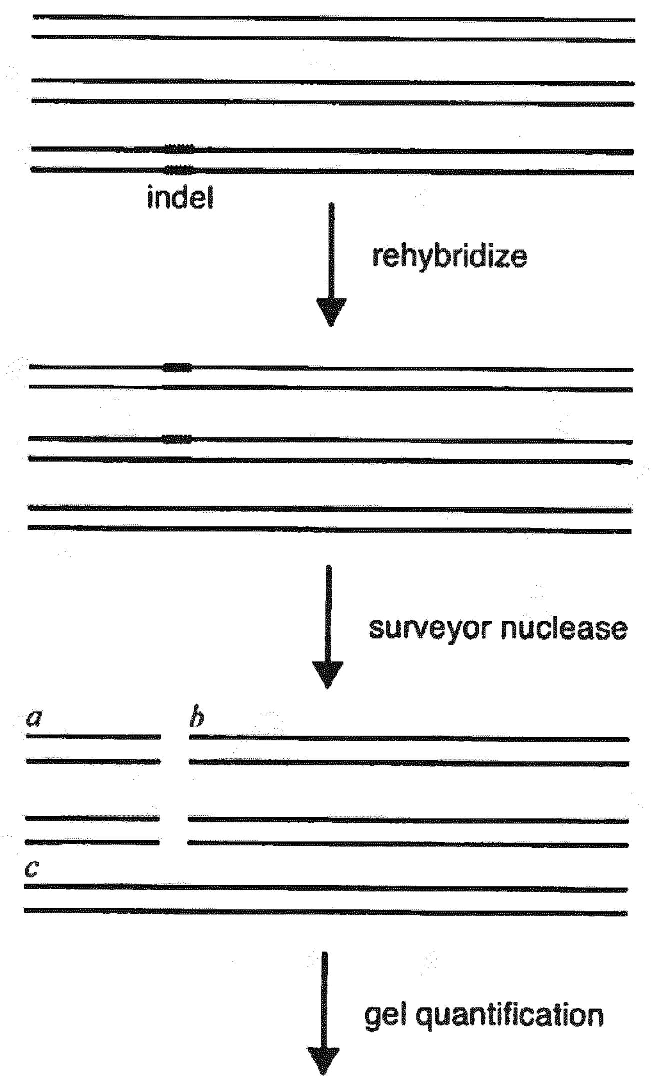


$$\% \text{ indel} = \left(1-\sqrt{1-(a+b)/(a+b+c)}\right)*100$$

FIG. 7

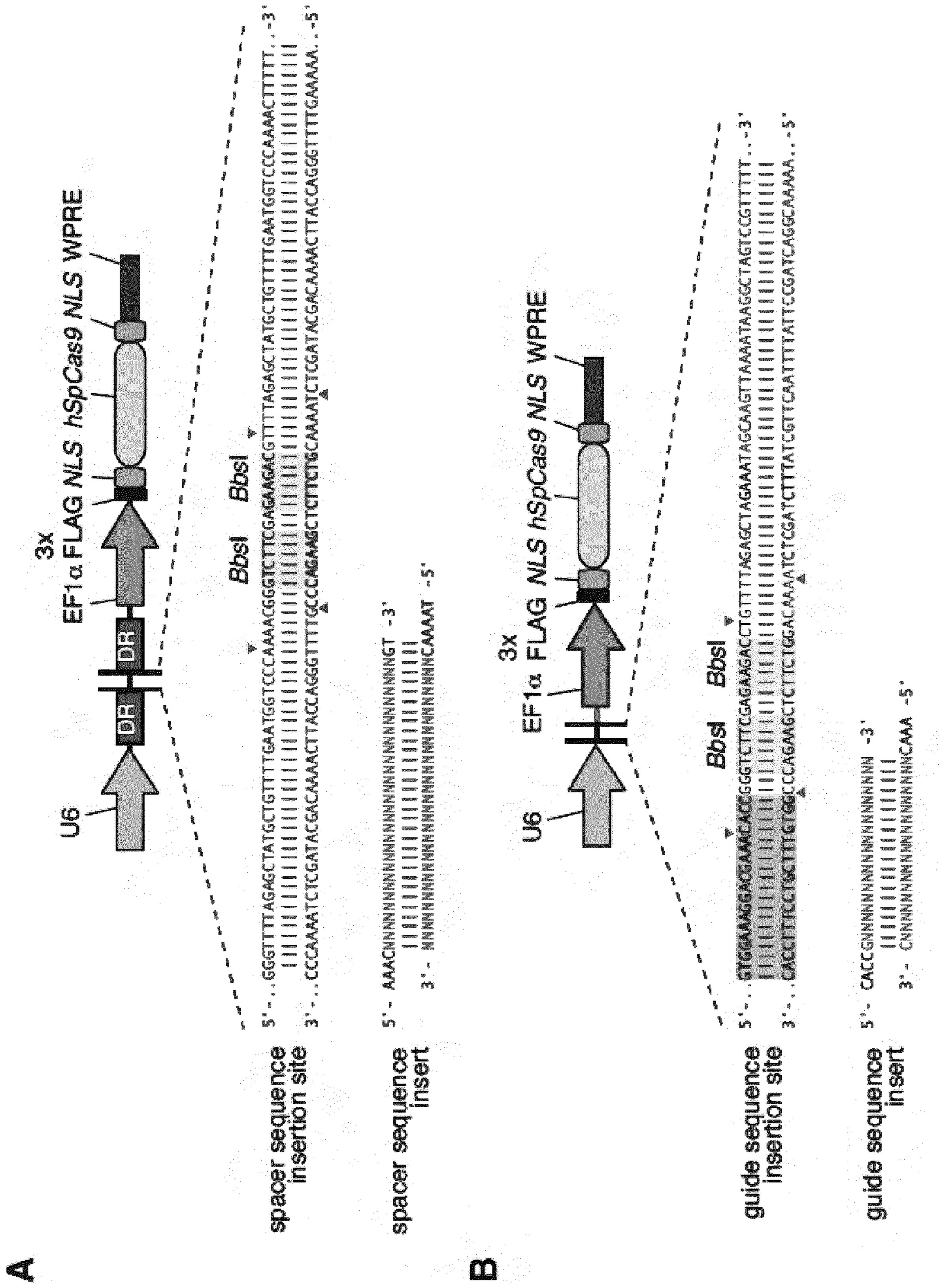
A
U6
DR
DR
EF1α
3x
FLAG
NLS
hSpCas9
NLS
WPRE
BbsI
BbsI
spacer sequence insertion site
spacer sequence insert
B
U6
EF1α
3x
FLAG
NLS
hSpCas9
NLS
WPRE
BbsI
BbsI
guide sequence insertion site
guide sequence insert

FIG. 8A-B

A

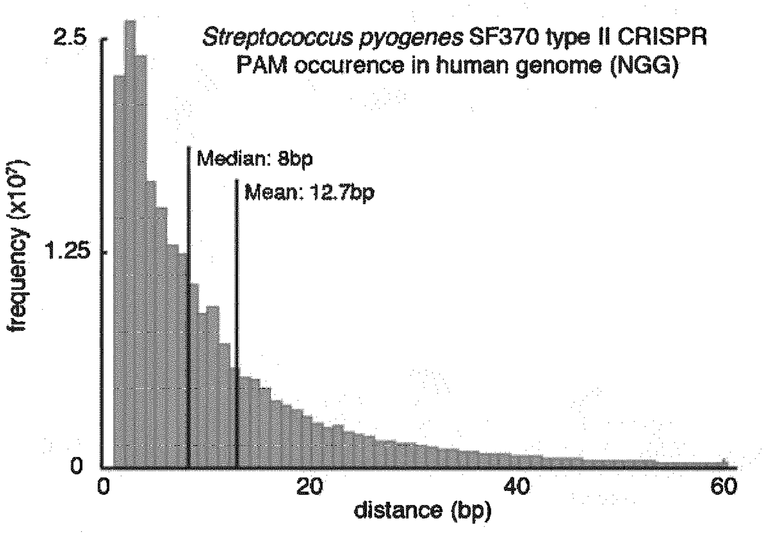


B

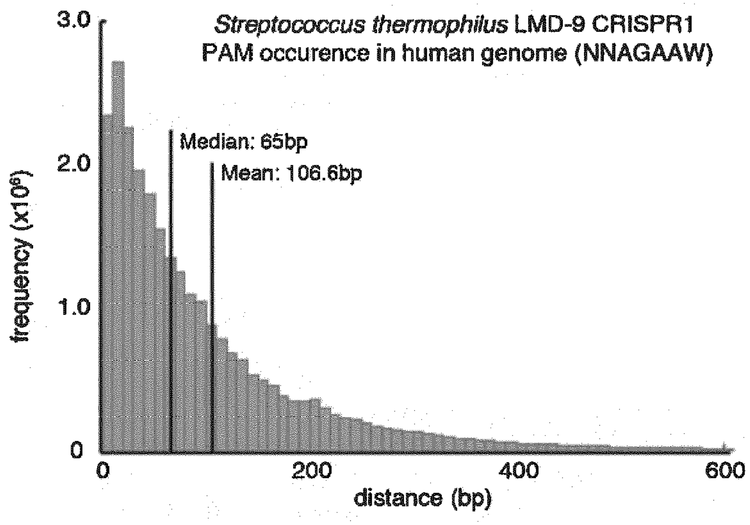


C

| | NGG | | NNAGAAW | |
|---|---|---|---|---|
| Chr | median | mean | median | mean |
| 1 | 7 | 12.8 | 67 | 115.8 |
| 2 | 8 | 12.7 | 64 | 100.8 |
| 3 | 8 | 13.0 | 63 | 98.5 |
| 4 | 9 | 14.0 | 61 | 94.5 |
| 5 | 8 | 13.1 | 63 | 97.9 |
| 6 | 8 | 13.1 | 63 | 98.5 |
| 7 | 8 | 12.4 | 64 | 102.9 |
| 8 | 8 | 12.8 | 64 | 100.9 |
| 9 | 7 | 13.9 | 65 | 120.5 |
| 10 | 7 | 12.1 | 66 | 107.0 |
| 11 | 7 | 12.0 | 65 | 105.8 |
| 12 | 8 | 12.4 | 65 | 103.5 |
| 13 | 8 | 13.6 | 62 | 94.6 |
| 14 | 8 | 12.0 | 65 | 101.5 |
| 15 | 7 | 11.5 | 68 | 107.7 |
| 16 | 7 | 11.7 | 74 | 136.8 |
| 17 | 6 | 10.3 | 76 | 127.9 |
| 18 | 8 | 13.4 | 63 | 101.8 |
| 19 | 6 | 9.4 | 82 | 145.4 |
| 20 | 7 | 11.1 | 72 | 121.8 |
| 21 | 7 | 13.4 | 64 | 111.4 |
| 22 | 6 | 9.2 | 85 | 140.3 |
| X | 8 | 13.2 | 63 | 99.0 |
| Y | 8 | 29.2 | 62 | 223.7 |

FIG. 9A-C

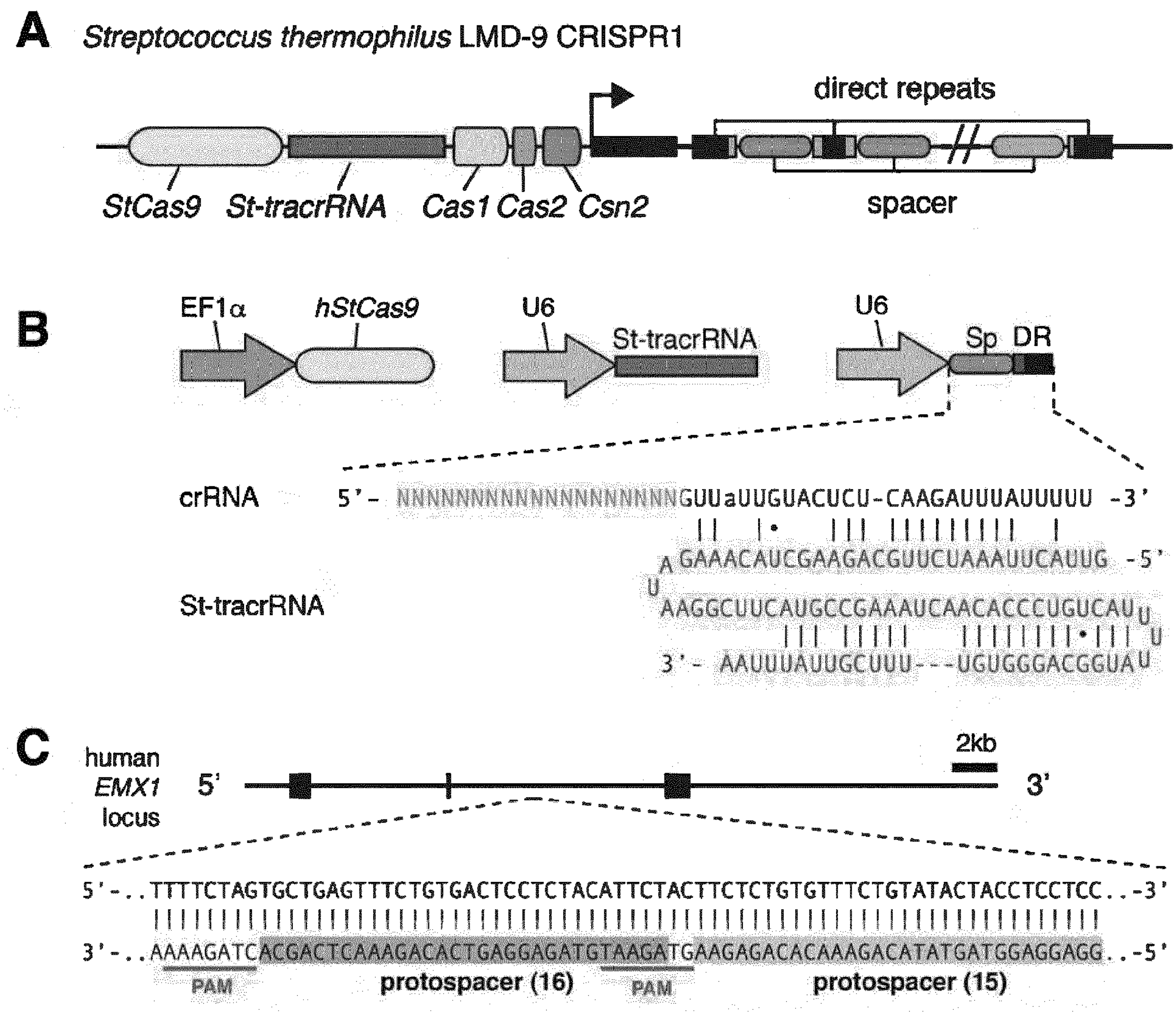
A
Streptococcus thermophilus LMD-9 CRISPR1
direct repeats
StCas9
St-tracrRNA
Cas1
Cas2
Csn2
spacer
B
EF1α
hStCas9
U6
St-tracrRNA
U6
Sp
DR
crRNA
5'- NNNNNNNNNNNNNNNNNNNNGUUaUUGUACUCU-CAAGAUUUAUUUUU -3'
AGAAACAUCGAAGACGUUCUAAAUUCAUUG -5'
U
St-tracrRNA
AAGGCUUCAUGCCGAAAUCAACACCCUGUCAUU
U
3'- AAUUUAUUGCUUU---UGUGGGACGGUAU
C
human
EMX1
locus
5'
2kb
3'
5'-..TTTTCTAGTGCTGAGTTTCTGTGACTCCTCTACATTCTACTTCTCTGTGTTTCTGTATACTACCTCCTCC..-3'
3'-..AAAAGATCACGACTCAAAGACACTGAGGAGATGTAAGATGAAGAGACACAAAGACATATGATGGAGGAGG..-5'
PAM
protospacer (16)
PAM
protospacer (15)

FIG. 10A-C

D

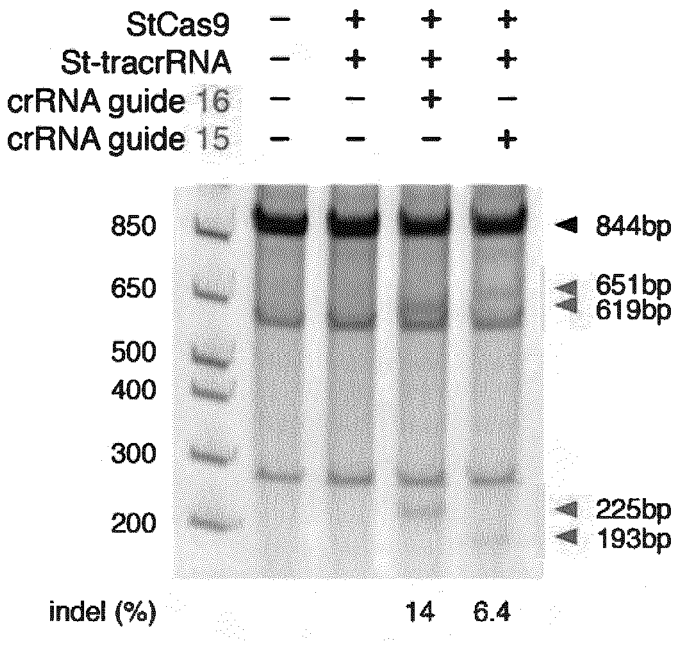
StCas9 − + + +
St-tracrRNA − + + +
crRNA guide 16 − − + −
crRNA guide 15 − − − +
850
650
500
400
300
200
844bp
651bp
619bp
225bp
193bp
indel (%)
14
6.4

FIG. 10D

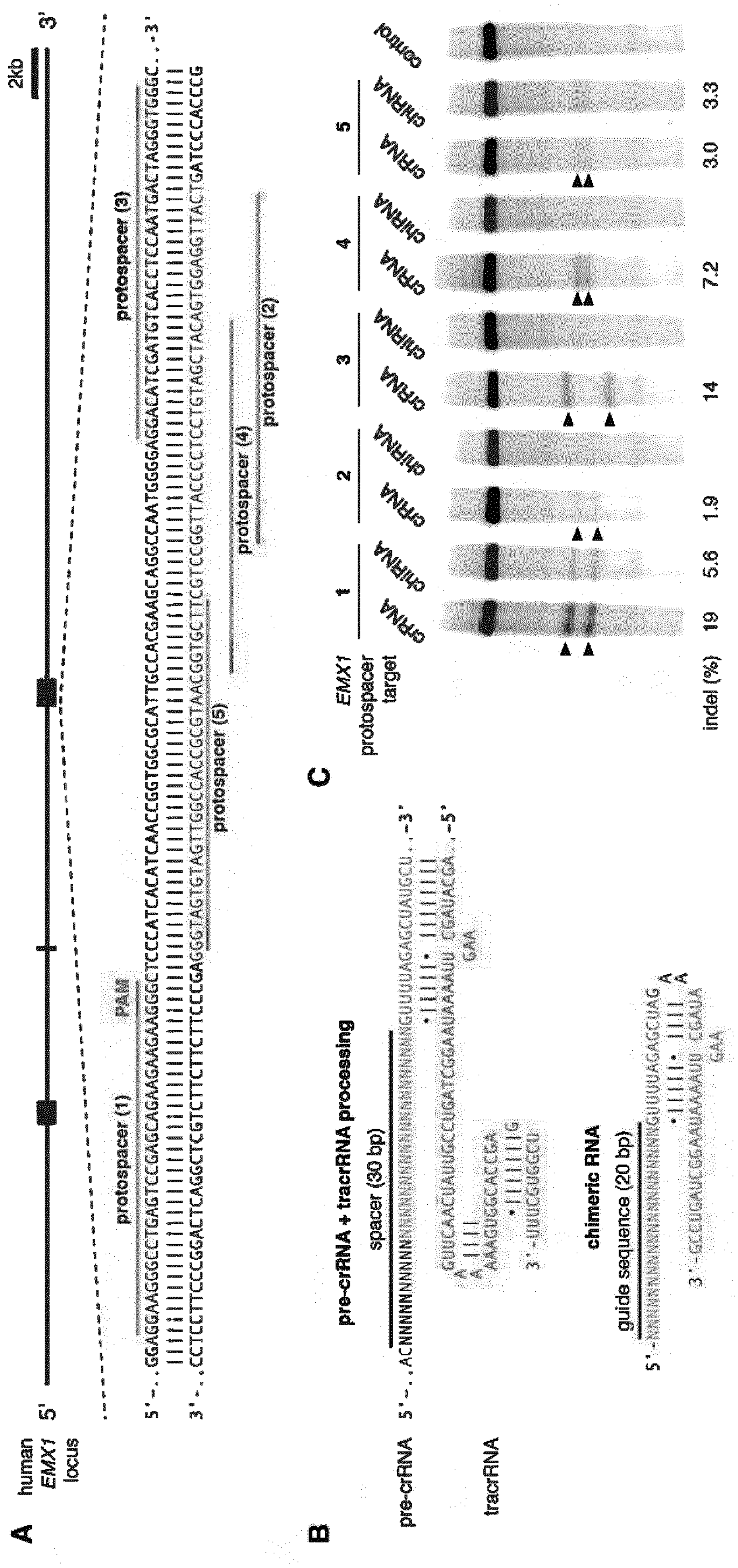
A
human EMX1 locus
5'
3'
2kb
protospacer (1)
PAM
protospacer (3)
protospacer (5)
protospacer (4)
protospacer (2)
B
pre-crRNA + tracrRNA processing
spacer (30 bp)
pre-crRNA
tracrRNA
chimeric RNA
guide sequence (20 bp)
C
EMX1 protospacer target
1
2
3
4
5
crRNA
chiRNA
control
indel (%)
19
5.6
1.9
14
7.2
3.0
3.3

FIG. 11A-C

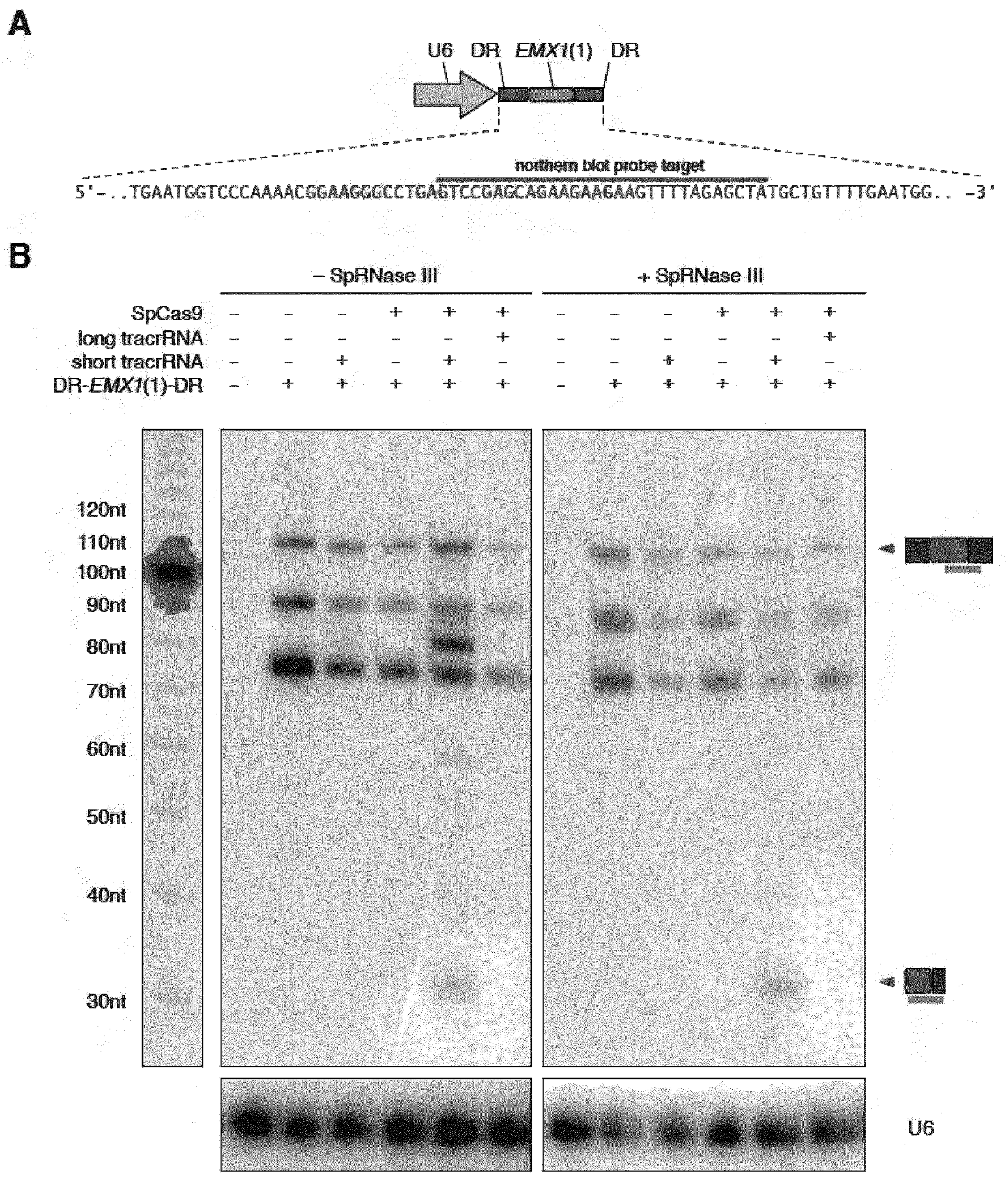
A
U6
DR
EMX1(1)
DR
northern blot probe target
5'-..TGAATGGTCCCAAAACGGAAGGGCCTGAGTCCGAGCAGAAGAAGAAGTTTTAGAGCTATGCTGTTTTGAATGG.. -3'
B
– SpRNase III
+ SpRNase III
SpCas9
long tracrRNA
short tracrRNA
DR-EMX1(1)-DR
120nt
110nt
100nt
90nt
80nt
70nt
60nt
50nt
40nt
30nt
U6

FIG. 12A-B

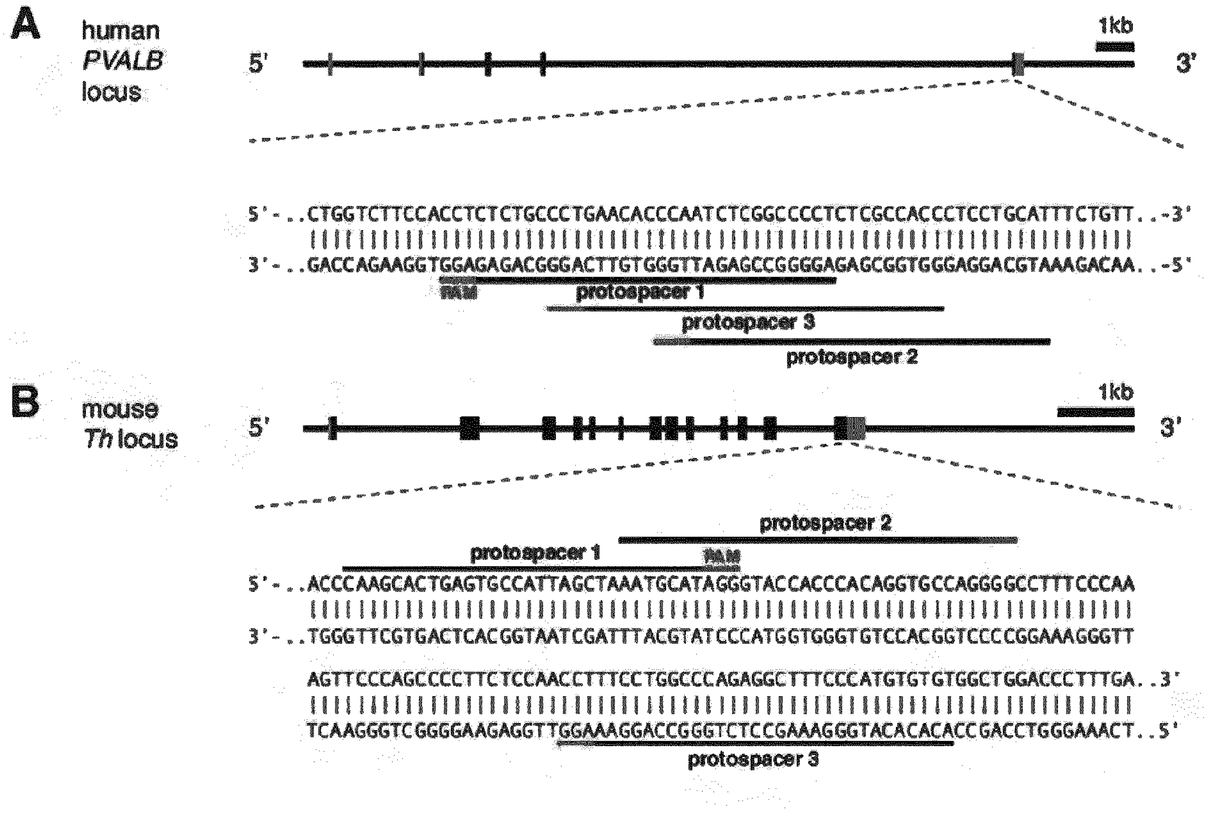
A
human
PVALB
locus
1kb
5'
3'
5'-..CTGGTCTTCCACCTCTCTGCCCTGAACACCCAATCTCGGCCCCTCTCGCCACCCTCCTGCATTTCTGTT..-3'
3'-..GACCAGAAGGTGGAGAGACGGGACTTGTGGGTTAGAGCCGGGGAGAGCGGTGGGAGGACGTAAAGACAA..-5'
PAM
protospacer 1
protospacer 3
protospacer 2
B
mouse
Th locus
1kb
5'
3'
protospacer 2
protospacer 1
PAM
5'-..ACCCAAGCACTGAGTGCCATTAGCTAAATGCATAGGGTACCACCCACAGGTGCCAGGGGCCTTTCCCAA
3'-..TGGGTTCGTGACTCACGGTAATCGATTTACGTATCCCATGGTGGGTGTCCACGGTCCCCGGAAAGGGTT
AGTTCCCAGCCCCTTCTCCAACCTTTCCTGGCCCAGAGGCTTTCCCATGTGTGTGGCTGGACCCTTTGA..3'
TCAAGGGTCGGGGAAGAGGTTGGAAAGGACCGGGTCTCCGAAAGGGTACACACACCGACCTGGGAAACT..5'
protospacer 3

FIG. 13A-B

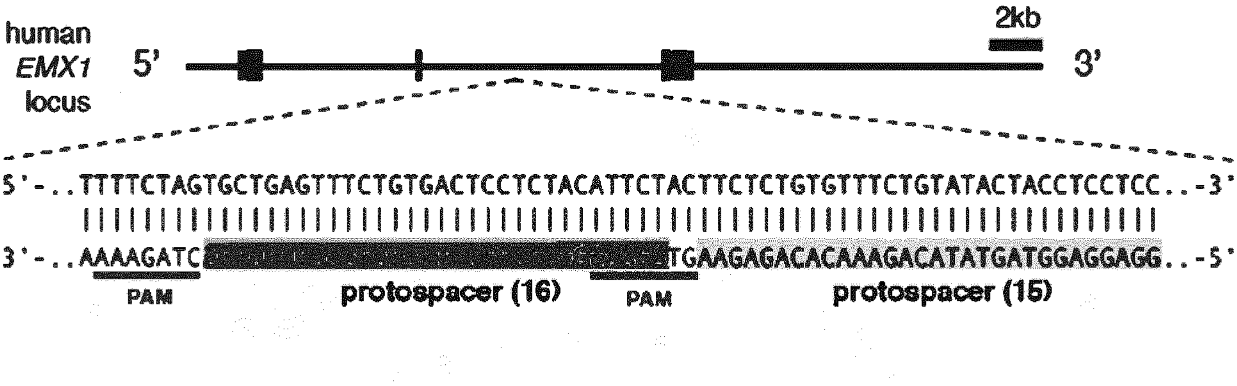
human
EMX1
locus
5'
3'
2kb
5'-..TTTTCTAGTGCTGAGTTTCTGTGACTCCTCTACATTCTACTTCTCTGTGTTTCTGTATACTACCTCCTCC..-3'
3'-..AAAAGATC
TGAAGAGACACAAAGACATATGATGGAGGAGG..-5'
PAM
protospacer (16)
PAM
protospacer (15)

FIG. 14

| Primer name | Assay | Genomic Target | Primer sequence |
|---|---|---|---|
| Sp-EMX1-F | SURVEYOR assay, sequencing | *EMX1* | AAAACCACCCTTCTCTCTGGC |
| Sp-EMX1-R | SURVEYOR assay, sequencing | *EMX1* | GGAGATTGGAGACACGGAGAG |
| Sp-PVALB-F | SURVEYOR assay, sequencing | *PVALB* | CTGGAAAGCCAATGCCTGAC |
| Sp-PVALB-R | SURVEYOR assay, sequencing | *PVALB* | GGCAGCAAACTCCTTGTCCT |
| Sp-Th-F | SURVEYOR assay, sequencing | *Th* | GTGCTTTGCAGAGGCCTACC |
| Sp-Th-R | SURVEYOR assay, sequencing | *Th* | CCTGGAGCGCATGCAGTAGT |
| St-EMX1-F | SURVEYOR assay, sequencing | *EMX1* | ACCTTCTGTGTTTCCACCATTC |
| St-EMX1-R | SURVEYOR assay, sequencing | *EMX1* | TTGGGGAGTGCACAGACTTC |
| Sp-EMX1-RFLP-F | RFLP, sequencing | *EMX1* | GGCTCCCTGGGTTCAAAGTA |
| Sp-EMX1-RFLP-R | RFLP, sequencing | *EMX1* | AGAGGGGTCTGGATGTCGTAA |
| Pb_EMX1_sp1 | Northern Blot Probe | Not applicable | TAGCTCTAAAACTTCTTCTTCTGCTCGGAC |
| Pb_tracrRNA | Northern Blot Probe | Not applicable | CTAGCCTTATTTTAACTTGCTATGCTGTTT |

**FIG. 15**

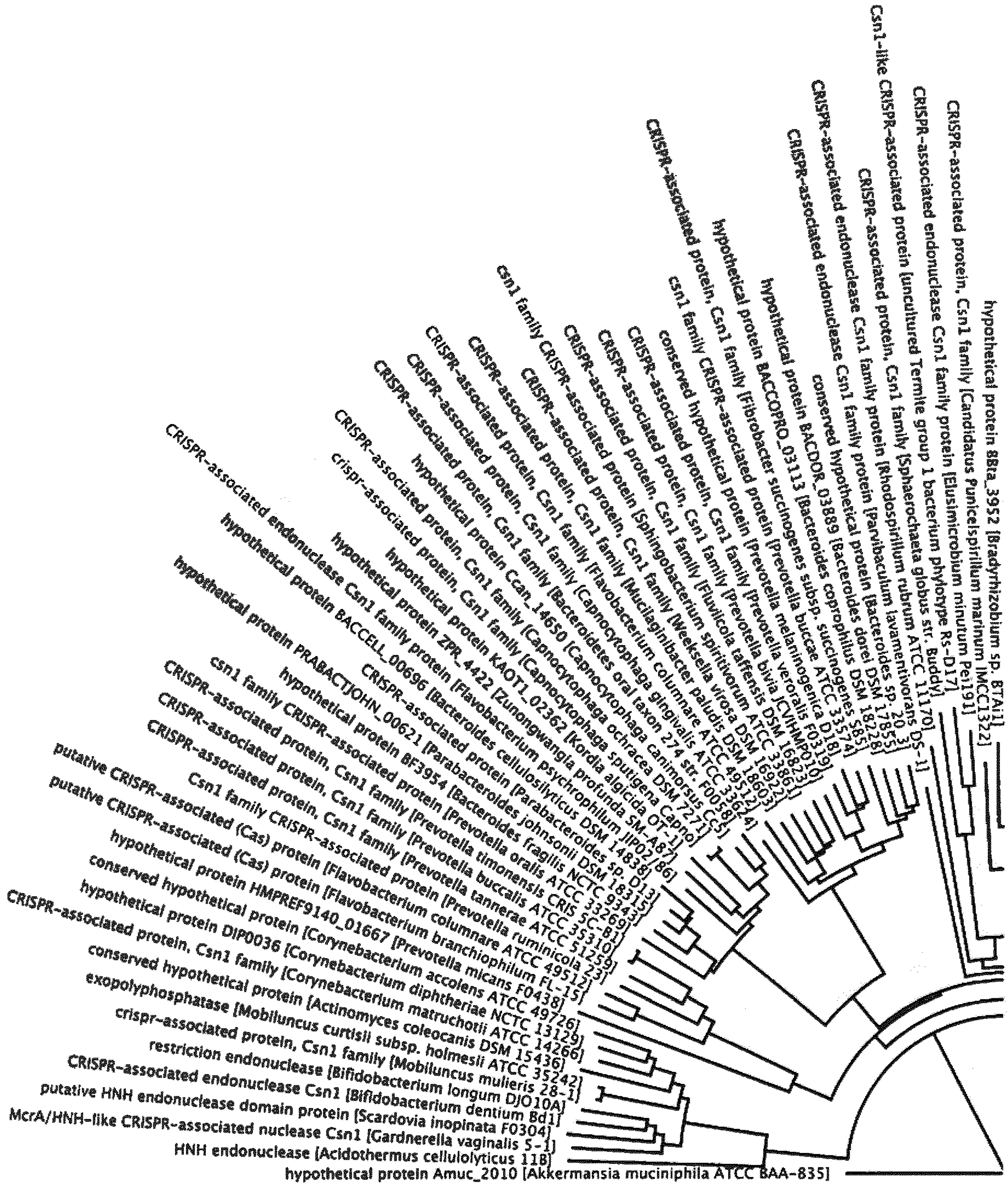
hypothetical protein BBta_3952 [Bradyrhizobium sp. BTAi1]
CRISPR-associated protein, Csn1 family [Candidatus Puniceispirillum marinum IMCC1322]
CRISPR-associated endonuclease Csn1 family protein [Elusimicrobium minutum Pei191]
CRISPR-associated protein [uncultured Termite group 1 bacterium phylotype Rs-D17]
Csn1-like CRISPR-associated protein, Csn1 family [Sphaerochaeta globus str. Buddy]
CRISPR-associated protein, Csn1 family [Rhodospirillum rubrum ATCC 11170]
CRISPR-associated endonuclease Csn1 family protein [Parvibaculum lavamentivorans DS-1]
CRISPR-associated endonuclease Csn1 family protein [Bacteroides sp. 20_3]
conserved hypothetical protein [Bacteroides dorei DSM 17855]
hypothetical protein BACDOR_03889 [Bacteroides coprophilus DSM 18228]
hypothetical protein BACCOPRO_03113 [Bacteroides coprophilus DSM 18228]
hypothetical protein BACCOPRO_03113 [Bacteroides coprophilus DSM 18228]
HNH endonuclease [Acidothermus cellulolyticus 11B]
hypothetical protein Amuc_2010 [Akkermansia muciniphila ATCC BAA-835]

FIG. 16A

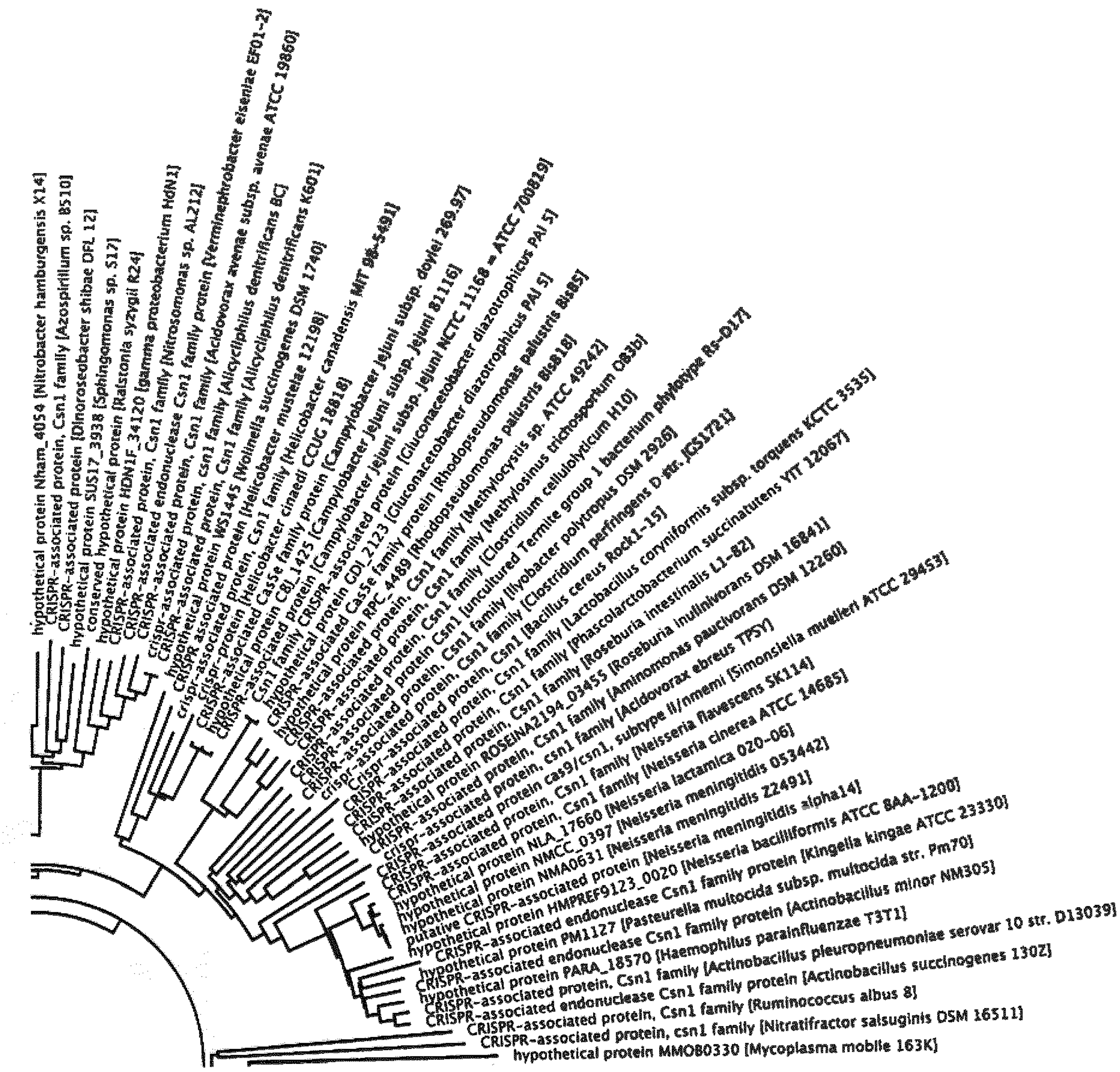
hypothetical protein Nham_4054 [Nitrobacter hamburgensis X14]
CRISPR-associated protein, Csn1 family [Azospirillum sp. B510]
hypothetical protein [Dinoroseobacter shibae DFL 12]
conserved hypothetical protein SUS17_3938 [Sphingomonas sp. S17]
hypothetical protein [Ralstonia syzygii R24]
CRISPR-associated protein HDN1F_34120 [gamma proteobacterium HdN1]
crispr-associated protein, Csn1 family [Nitrosomonas sp. AL212]
CRISPR-associated endonuclease Csn1 family protein [Verminephrobacter eiseniae EF01-2]
CRISPR-associated protein, Csn1 family [Acidovorax avenae subsp. avenae ATCC 19860]
hypothetical protein, csn1 family [Alicycliphilus denitrificans BC]
crispr-associated protein WS1445 [Wolinella succinogenes DSM 1740]
CRISPR associated protein, Csn1 family [Alicycliphilus denitrificans K601]
crispr-protein [Helicobacter mustelae 12198]
hypothetical protein CBJ_1425 [Helicobacter cinaedi CCUG 18818]
CRISPR-associated Cas5e family protein [Helicobacter canadensis MIT 98-5491]
Csn1 family CRISPR-associated protein [Campylobacter jejuni subsp. doylei 269.97]
hypothetical protein [Campylobacter jejuni subsp. jejuni 81116]
CRISPR-associated protein GDI_2123 [Campylobacter jejuni subsp. jejuni NCTC 11168 = ATCC 700819]
hypothetical protein Cas5e family protein [Gluconacetobacter diazotrophicus PAl 5]
CRISPR-associated protein RPC_4489 [Gluconacetobacter diazotrophicus PAl 5]
CRISPR-associated protein, Csn1 family [Rhodopseudomonas palustris BisB18]
CRISPR-associated protein, Csn1 family [Rhodopseudomonas palustris BisB5]
crispr-associated protein, Csn1 family [Methylocystis sp. ATCC 49242]
Crispr-associated protein, Csn1 family [Methylosinus trichosporium OB3b]
CRISPR-associated protein, Csn1 family [Clostridium cellulolyticum H10]
crispr-associated protein, Csn1 family [uncultured Termite group 1 bacterium phylotype Rs-D17]
CRISPR-associated protein, Csn1 family [Ilyobacter polytropus DSM 2926]
hypothetical protein ROSEINA2194_03455 [Clostridium perfringens D str. JGS1721]
CRISPR-associated protein, Csn1 family [Bacillus cereus Rock1-15]
crispr-associated protein, Csn1 family [Lactobacillus coryniformis subsp. torquens KCTC 3535]
CRISPR-associated protein cas9/csn1, subtype II/nmemi [Phascolarctobacterium succinatutens YIT 12067]
CRISPR-associated protein, Csn1 family [Roseburia intestinalis L1-82]
hypothetical protein NLA_17660 [Roseburia inulinivorans DSM 16841]
CRISPR-associated protein, Csn1 family [Aminomonas paucivorans DSM 12260]
hypothetical protein NMCC_0397 [Acidovorax ebreus TPSY]
hypothetical protein NMA0631 [Simonsiella muelleri ATCC 29453]
hypothetical CRISPR-associated protein [Neisseria flavescens SK114]
putative CRISPR-associated protein HMPREF9123_0020 [Neisseria cinerea ATCC 14685]
hypothetical protein PM1127 [Neisseria lactamica 020-06]
CRISPR-associated endonuclease Csn1 family protein [Neisseria meningitidis 053442]
hypothetical protein [Neisseria meningitidis Z2491]
CRISPR-associated endonuclease [Neisseria meningitidis alpha14]
hypothetical protein PARA_18570 [Neisseria bacilliformis ATCC BAA-1200]
CRISPR-associated protein, Csn1 family protein [Kingella kingae ATCC 23330]
CRISPR-associated endonuclease Csn1 family protein [Pasteurella multocida subsp. multocida str. Pm70]
[Actinobacillus minor NM305]
[Haemophilus parainfluenzae T3T1]
[Actinobacillus pleuropneumoniae serovar 10 str. D13039]
[Actinobacillus succinogenes 130Z]
[Ruminococcus albus 8]
CRISPR-associated protein, Csn1 family [Nitratifractor salsuginis DSM 16511]
CRISPR-associated protein, csn1 family
hypothetical protein MMO80330 [Mycoplasma mobile 163K]

FIG. 16B

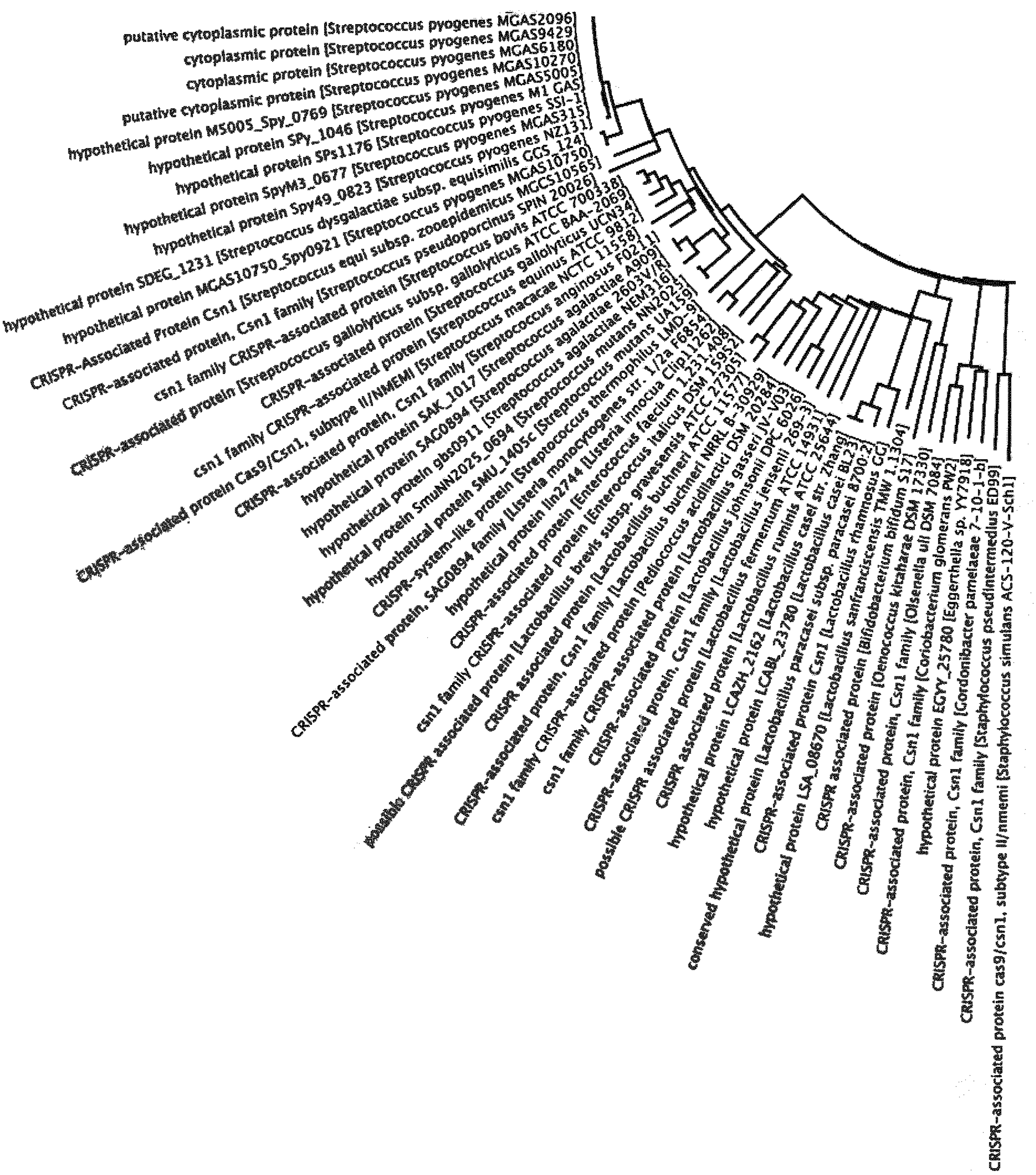

FIG. 16C

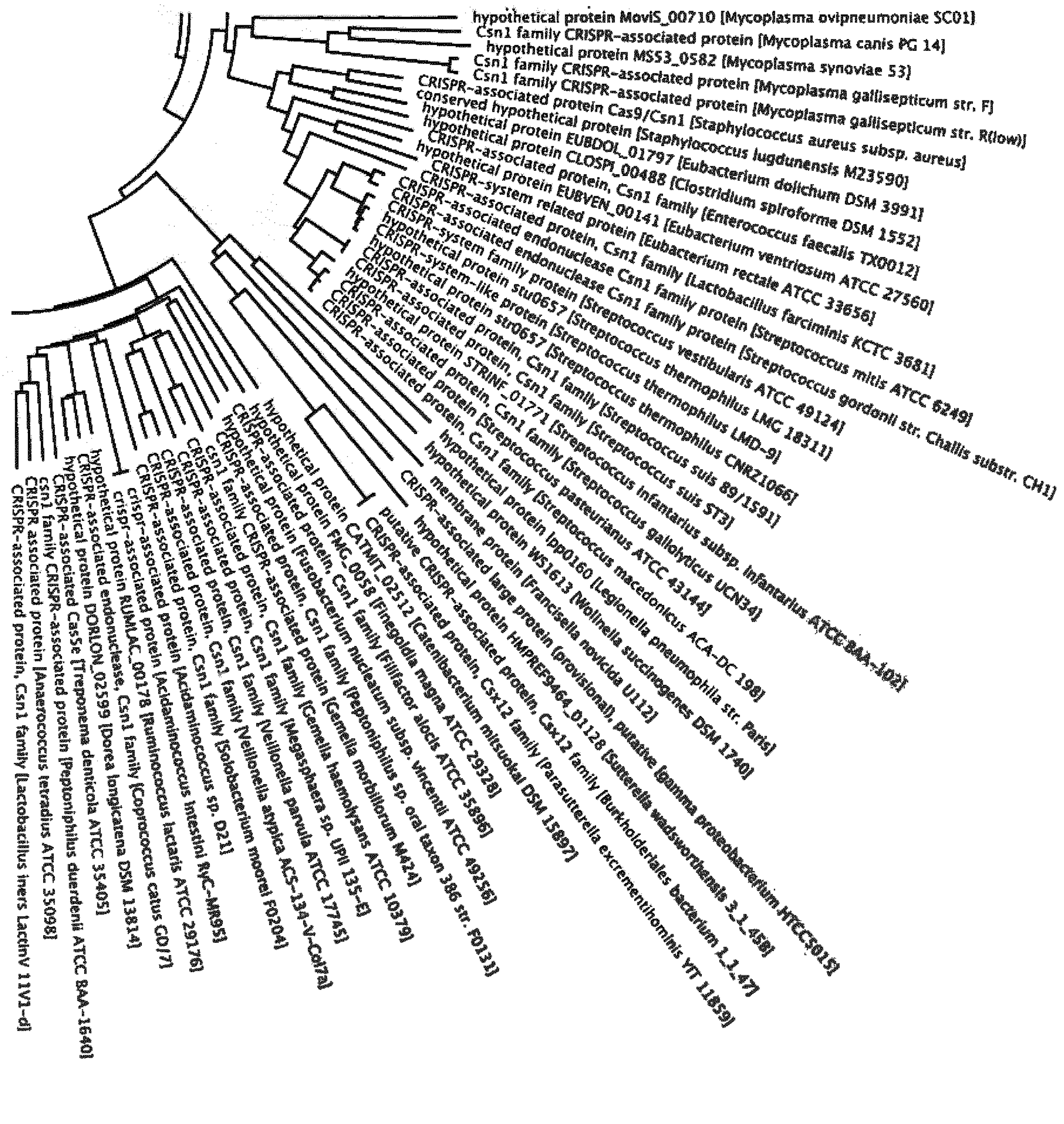

FIG. 16D

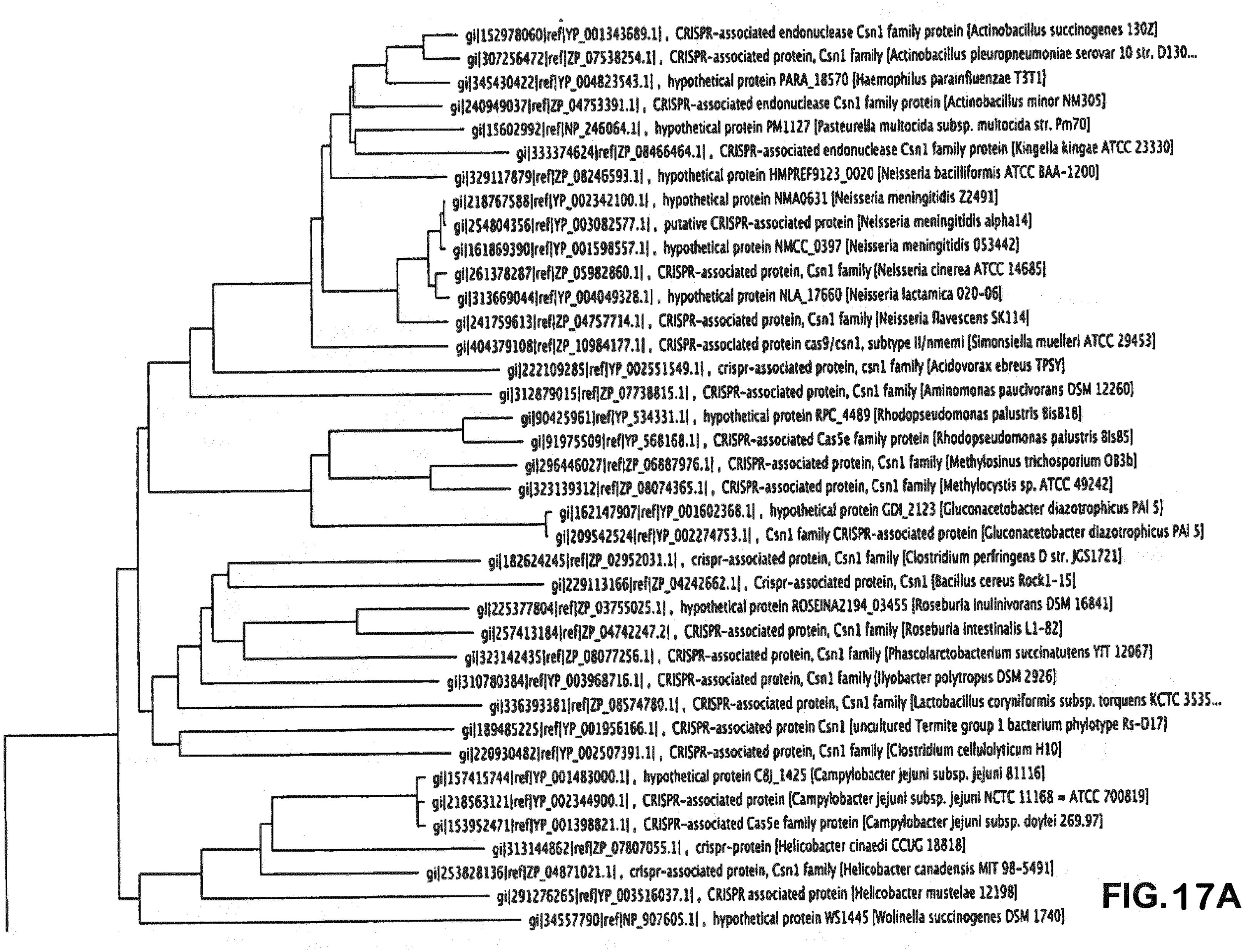
gi|152978060|ref|YP_001343689.1| , CRISPR-associated endonuclease Csn1 family protein [Actinobacillus succinogenes 130Z]
gi|307256472|ref|ZP_07538254.1| , CRISPR-associated protein, Csn1 family [Actinobacillus pleuropneumoniae serovar 10 str. D130...
gi|345430422|ref|YP_004823543.1| , hypothetical protein PARA_18570 [Haemophilus parainfluenzae T3T1]
gi|240949037|ref|ZP_04753391.1| , CRISPR-associated endonuclease Csn1 family protein [Actinobacillus minor NM305]
gi|15602992|ref|NP_246064.1| , hypothetical protein PM1127 [Pasteurella multocida subsp. multocida str. Pm70]
gi|333374624|ref|ZP_08466464.1| , CRISPR-associated endonuclease Csn1 family protein [Kingella kingae ATCC 23330]
gi|329117879|ref|ZP_08246593.1| , hypothetical protein HMPREF9123_0020 [Neisseria bacilliformis ATCC BAA-1200]
gi|218767588|ref|YP_002342100.1| , hypothetical protein NMA0631 [Neisseria meningitidis Z2491]
gi|254804356|ref|YP_003082577.1| , putative CRISPR-associated protein [Neisseria meningitidis alpha14]
gi|161869390|ref|YP_001598557.1| , hypothetical protein NMCC_0397 [Neisseria meningitidis 053442]
gi|261378287|ref|ZP_05982860.1| , CRISPR-associated protein, Csn1 family [Neisseria cinerea ATCC 14685]
gi|313669044|ref|YP_004049328.1| , hypothetical protein NLA_17660 [Neisseria lactamica 020-06]
gi|241759613|ref|ZP_04757714.1| , CRISPR-associated protein, Csn1 family [Neisseria flavescens SK114]
gi|404379108|ref|ZP_10984177.1| , CRISPR-associated protein cas9/csn1, subtype II/nmemi [Simonsiella muelleri ATCC 29453]
gi|222109285|ref|YP_002551549.1| , crispr-associated protein, csn1 family [Acidovorax ebreus TPSY]
gi|312879015|ref|ZP_07738815.1| , CRISPR-associated protein, Csn1 family [Aminomonas paucivorans DSM 12260]
gi|90425961|ref|YP_534331.1| , hypothetical protein RPC_4489 [Rhodopseudomonas palustris BisB18]
gi|91975509|ref|YP_568168.1| , CRISPR-associated Cas5e family protein [Rhodopseudomonas palustris BisB5]
gi|296446027|ref|ZP_06887976.1| , CRISPR-associated protein, Csn1 family [Methylosinus trichosporium OB3b]
gi|323139312|ref|ZP_08074365.1| , CRISPR-associated protein, Csn1 family [Methylocystis sp. ATCC 49242]
gi|162147907|ref|YP_001602368.1| , hypothetical protein GDI_2123 [Gluconacetobacter diazotrophicus PAl 5]
gi|209542524|ref|YP_002274753.1| , Csn1 family CRISPR-associated protein [Gluconacetobacter diazotrophicus PAl 5]
gi|182624245|ref|ZP_02952031.1| , crispr-associated protein, Csn1 family [Clostridium perfringens D str. JGS1721]
gi|229113166|ref|ZP_04242662.1| , Crispr-associated protein, Csn1 [Bacillus cereus Rock1-15]
gi|225377804|ref|ZP_03755025.1| , hypothetical protein ROSEINA2194_03455 [Roseburia inulinivorans DSM 16841]
gi|257413184|ref|ZP_04742247.2| , CRISPR-associated protein, Csn1 family [Roseburia intestinalis L1-82]
gi|323142435|ref|ZP_08077256.1| , CRISPR-associated protein, Csn1 family [Phascolarctobacterium succinatutens YIT 12067]
gi|310780384|ref|YP_003968716.1| , CRISPR-associated protein, Csn1 family [Ilyobacter polytropus DSM 2926]
gi|336393381|ref|ZP_08574780.1| , CRISPR-associated protein, Csn1 family [Lactobacillus coryniformis subsp. torquens KCTC 3535...
gi|189485225|ref|YP_001956166.1| , CRISPR-associated protein Csn1 [uncultured Termite group 1 bacterium phylotype Rs-D17]
gi|220930482|ref|YP_002507391.1| , CRISPR-associated protein, Csn1 family [Clostridium cellulolyticum H10]
gi|157415744|ref|YP_001483000.1| , hypothetical protein C8J_1425 [Campylobacter jejuni subsp. jejuni 81116]
gi|218563121|ref|YP_002344900.1| , CRISPR-associated protein [Campylobacter jejuni subsp. jejuni NCTC 11168 = ATCC 700819]
gi|153952471|ref|YP_001398821.1| , CRISPR-associated Cas5e family protein [Campylobacter jejuni subsp. doylei 269.97]
gi|313144862|ref|ZP_07807055.1| , crispr-protein [Helicobacter cinaedi CCUG 18818]
gi|253828136|ref|ZP_04871021.1| , crispr-associated protein, Csn1 family [Helicobacter canadensis MIT 98-5491]
gi|291276265|ref|YP_003516037.1| , CRISPR associated protein [Helicobacter mustelae 12198]
gi|34557790|ref|NP_907605.1| , hypothetical protein WS1445 [Wolinella succinogenes DSM 1740]

FIG.17A

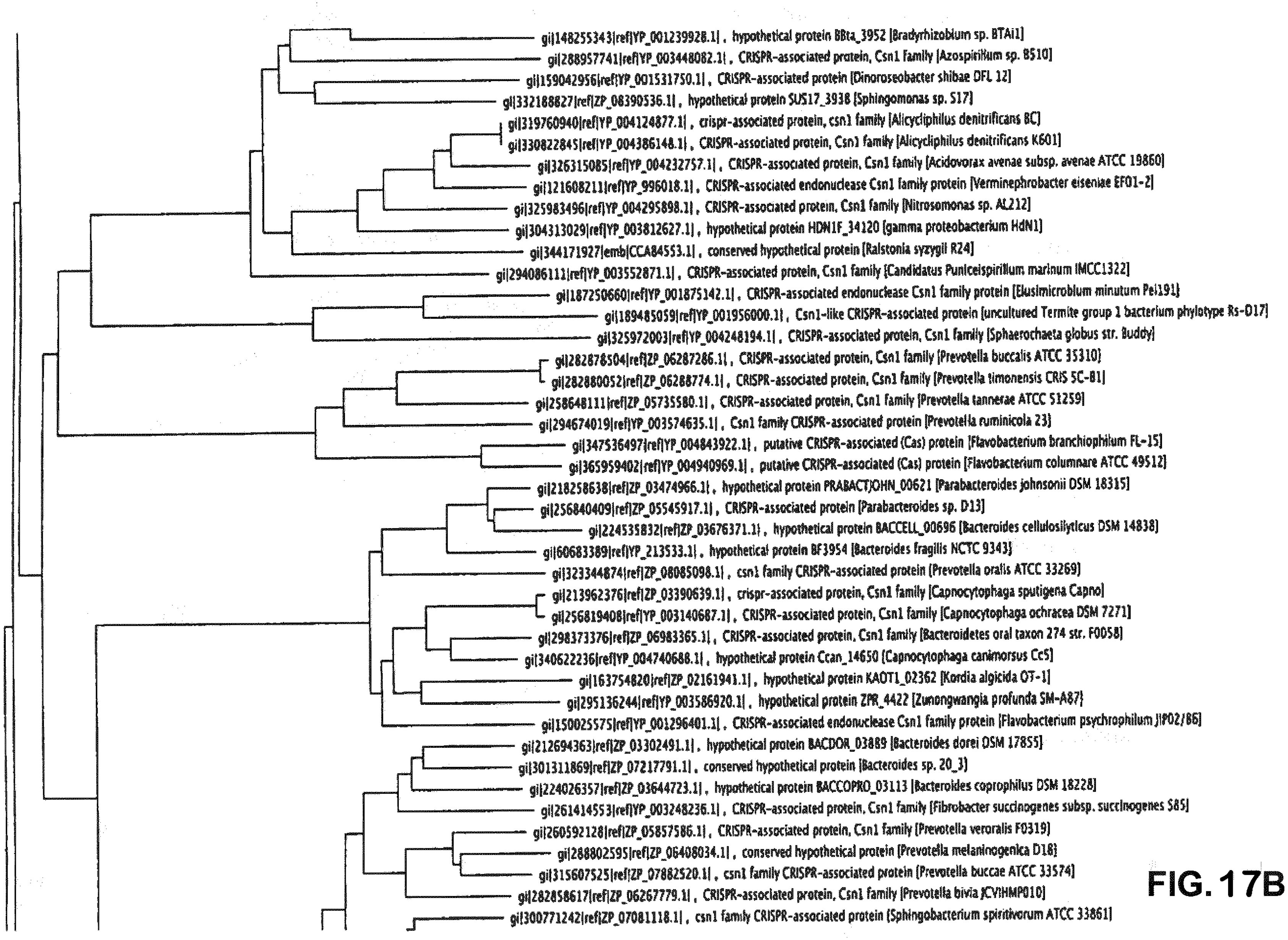
gi|148255343|ref|YP_001239928.1| , hypothetical protein BBta_3952 [Bradyrhizobium sp. BTAi1]
gi|288957741|ref|YP_003448082.1| , CRISPR-associated protein, Csn1 family [Azospirillum sp. B510]
gi|159042956|ref|YP_001531750.1| , CRISPR-associated protein [Dinoroseobacter shibae DFL 12]
gi|332188827|ref|ZP_08390536.1| , hypothetical protein SUS17_3938 [Sphingomonas sp. S17]
gi|319760940|ref|YP_004124877.1| , crispr-associated protein, csn1 family [Alicycliphilus denitrificans BC]
gi|330822845|ref|YP_004386148.1| , CRISPR-associated protein, Csn1 family [Alicycliphilus denitrificans K601]
gi|326315085|ref|YP_004232757.1| , CRISPR-associated protein, Csn1 family [Acidovorax avenae subsp. avenae ATCC 19860]
gi|121608211|ref|YP_996018.1| , CRISPR-associated endonuclease Csn1 family protein [Verminephrobacter eiseniae EF01-2]
gi|325983496|ref|YP_004295898.1| , CRISPR-associated protein, Csn1 family [Nitrosomonas sp. AL212]
gi|304313029|ref|YP_003812627.1| , hypothetical protein HDN1F_34120 [gamma proteobacterium HdN1]
gi|344171927|emb|CCA84553.1| , conserved hypothetical protein [Ralstonia syzygii R24]
gi|294086111|ref|YP_003552871.1| , CRISPR-associated protein, Csn1 family [Candidatus Puniceispirillum marinum IMCC1322]
gi|187250660|ref|YP_001875142.1| , CRISPR-associated endonuclease Csn1 family protein [Elusimicrobium minutum Pei191]
gi|189485059|ref|YP_001956000.1| , Csn1-like CRISPR-associated protein [uncultured Termite group 1 bacterium phylotype Rs-D17]
gi|325972003|ref|YP_004248194.1| , CRISPR-associated protein, Csn1 family [Sphaerochaeta globus str. Buddy]
gi|282878504|ref|ZP_06287286.1| , CRISPR-associated protein, Csn1 family [Prevotella buccalis ATCC 35310]
gi|282880052|ref|ZP_06288774.1| , CRISPR-associated protein, Csn1 family [Prevotella timonensis CRIS 5C-B1]
gi|258648111|ref|ZP_05735580.1| , CRISPR-associated protein, Csn1 family [Prevotella tannerae ATCC 51259]
gi|294674019|ref|YP_003574635.1| , Csn1 family CRISPR-associated protein [Prevotella ruminicola 23]
gi|347536497|ref|YP_004843922.1| , putative CRISPR-associated (Cas) protein [Flavobacterium branchiophilum FL-15]
gi|365959402|ref|YP_004940969.1| , putative CRISPR-associated (Cas) protein [Flavobacterium columnare ATCC 49512]
gi|218258638|ref|ZP_03474966.1| , hypothetical protein PRABACTJOHN_00621 [Parabacteroides johnsonii DSM 18315]
gi|256840409|ref|ZP_05545917.1| , CRISPR-associated protein [Parabacteroides sp. D13]
gi|224535832|ref|ZP_03676371.1| , hypothetical protein BACCELL_00696 [Bacteroides cellulosilyticus DSM 14838]
gi|60683389|ref|YP_213533.1| , hypothetical protein BF3954 [Bacteroides fragilis NCTC 9343]
gi|323344874|ref|ZP_08085098.1| , csn1 family CRISPR-associated protein [Prevotella oralis ATCC 33269]
gi|213962376|ref|ZP_03390639.1| , crispr-associated protein, Csn1 family [Capnocytophaga sputigena Capno]
gi|256819408|ref|YP_003140687.1| , CRISPR-associated protein, Csn1 family [Capnocytophaga ochracea DSM 7271]
gi|298373376|ref|ZP_06983365.1| , CRISPR-associated protein, Csn1 family [Bacteroidetes oral taxon 274 str. F0058]
gi|340622236|ref|YP_004740688.1| , hypothetical protein Ccan_14650 [Capnocytophaga canimorsus Cc5]
gi|163754820|ref|ZP_02161941.1| , hypothetical protein KAOT1_02362 [Kordia algicida OT-1]
gi|295136244|ref|YP_003586920.1| , hypothetical protein ZPR_4422 [Zunongwangia profunda SM-A87]
gi|150025575|ref|YP_001296401.1| , CRISPR-associated endonuclease Csn1 family protein [Flavobacterium psychrophilum JIP02/86]
gi|212694363|ref|ZP_03302491.1| , hypothetical protein BACDOR_03889 [Bacteroides dorei DSM 17855]
gi|301311869|ref|ZP_07217791.1| , conserved hypothetical protein [Bacteroides sp. 20_3]
gi|224026357|ref|ZP_03644723.1| , hypothetical protein BACCOPRO_03113 [Bacteroides coprophilus DSM 18228]
gi|261414553|ref|YP_003248236.1| , CRISPR-associated protein, Csn1 family [Fibrobacter succinogenes subsp. succinogenes S85]
gi|260592128|ref|ZP_05857586.1| , CRISPR-associated protein, Csn1 family [Prevotella veroralis F0319]
gi|288802595|ref|ZP_06408034.1| , conserved hypothetical protein [Prevotella melaninogenica D18]
gi|315607525|ref|ZP_07882520.1| , csn1 family CRISPR-associated protein [Prevotella buccae ATCC 33574]
gi|282858617|ref|ZP_06267779.1| , CRISPR-associated protein, Csn1 family [Prevotella bivia JCVIHMP010]
gi|300771242|ref|ZP_07081118.1| , csn1 family CRISPR-associated protein [Sphingobacterium spiritivorum ATCC 33861]

FIG. 17B

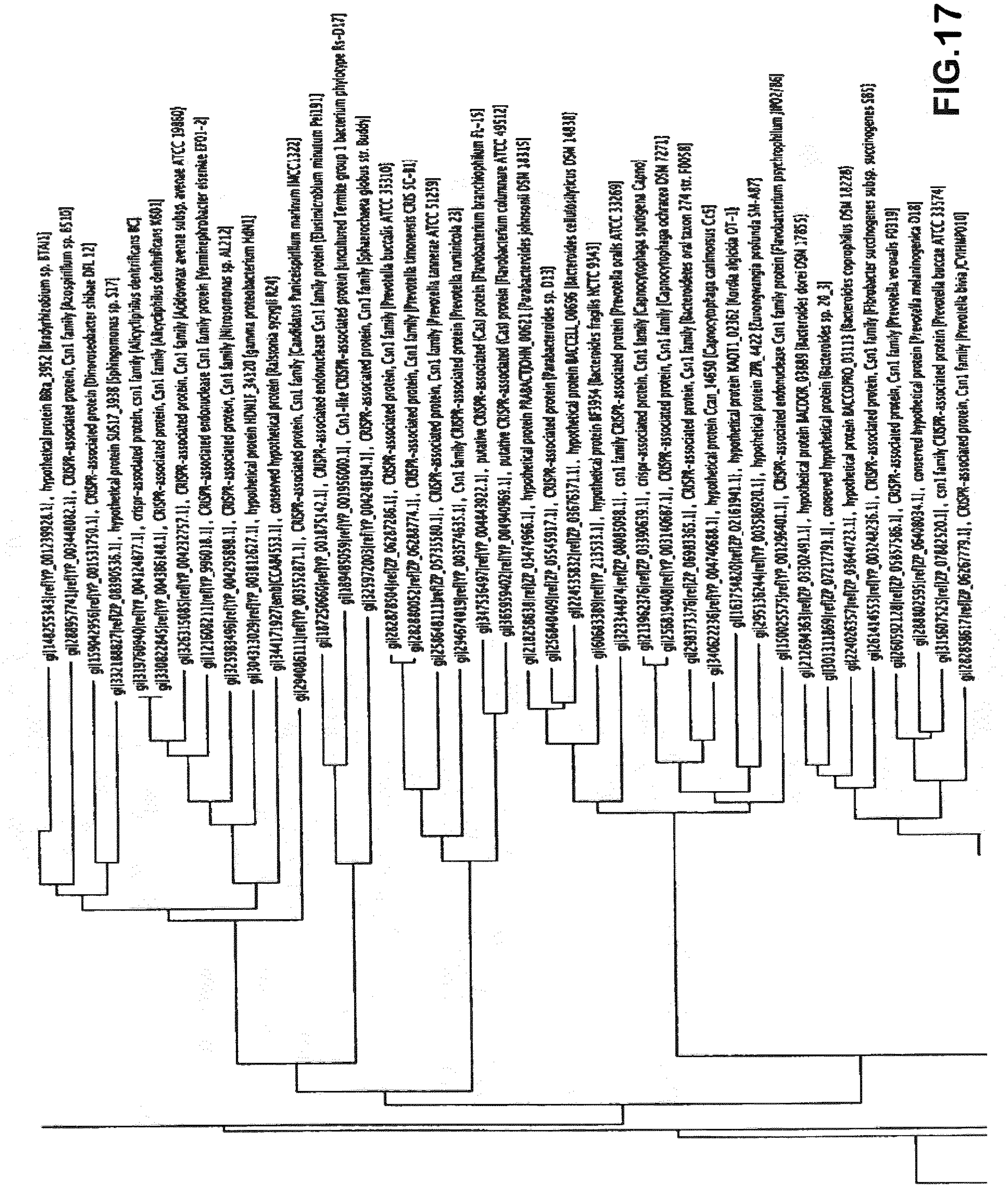

FIG.17C

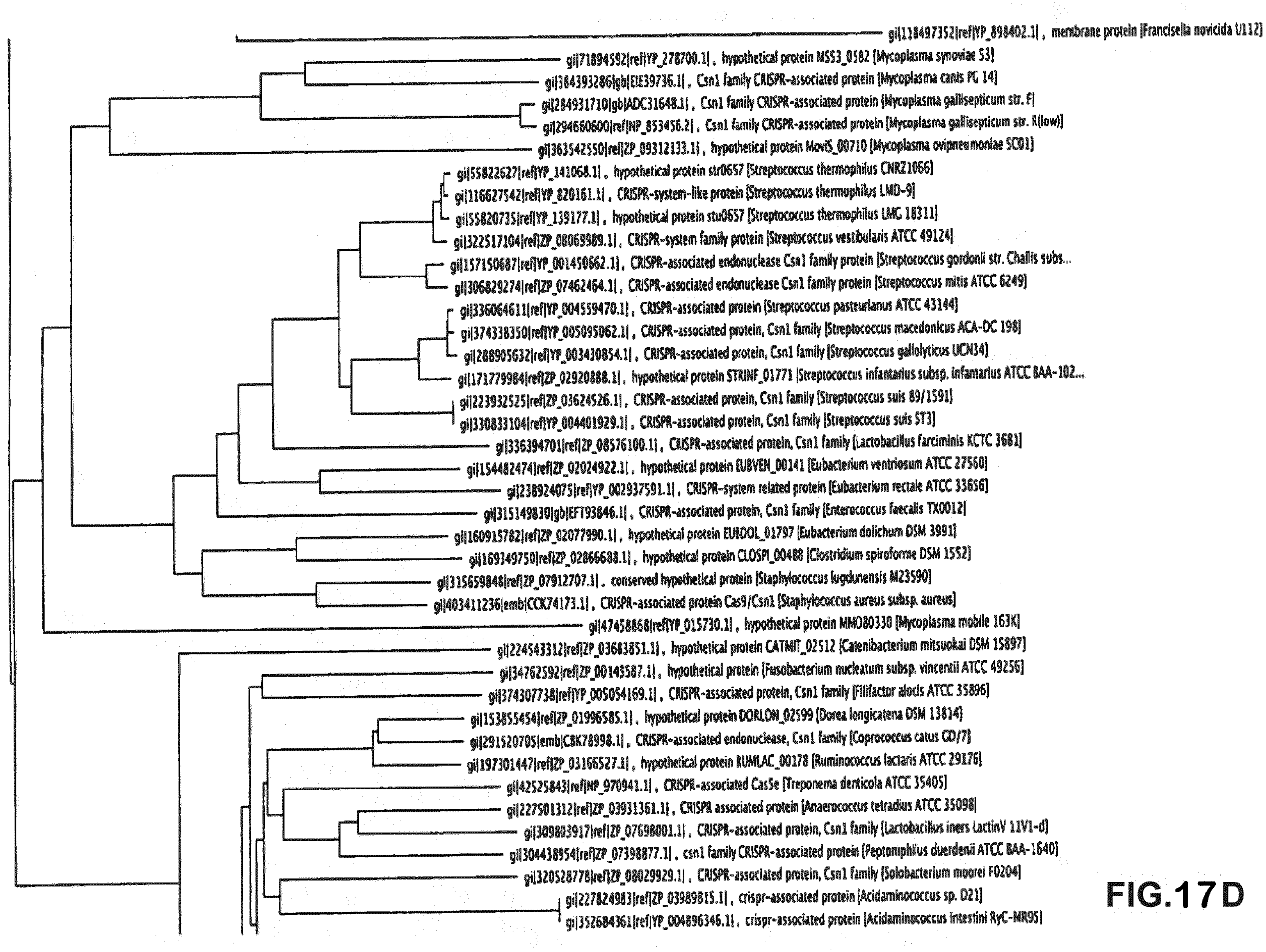
gi|118497352|ref|YP_898402.1| , membrane protein [Francisella novicida U112]
gi|71894592|ref|YP_278700.1| , hypothetical protein MS53_0582 [Mycoplasma synoviae 53]
gi|384393286|gb|EIE39736.1| , Csn1 family CRISPR-associated protein [Mycoplasma canis PG 14]
gi|284931710|gb|ADC31648.1| , Csn1 family CRISPR-associated protein [Mycoplasma gallisepticum str. F]
gi|294660600|ref|NP_853456.2| , Csn1 family CRISPR-associated protein [Mycoplasma gallisepticum str. R(low)]
gi|363542550|ref|ZP_09312133.1| , hypothetical protein MovS_00710 [Mycoplasma ovipneumoniae SC01]
gi|55822627|ref|YP_141068.1| , hypothetical protein str0657 [Streptococcus thermophilus CNRZ1066]
gi|116627542|ref|YP_820161.1| , CRISPR-system-like protein [Streptococcus thermophilus LMD-9]
gi|55820735|ref|YP_139177.1| , hypothetical protein stu0657 [Streptococcus thermophilus LMG 18311]
gi|322517104|ref|ZP_08069989.1| , CRISPR-system family protein [Streptococcus vestibularis ATCC 49124]
gi|157150687|ref|YP_001450662.1| , CRISPR-associated endonuclease Csn1 family protein [Streptococcus gordonii str. Challis subs...
gi|306829274|ref|ZP_07462464.1| , CRISPR-associated endonuclease Csn1 family protein [Streptococcus mitis ATCC 6249]
gi|336064611|ref|YP_004559470.1| , CRISPR-associated protein [Streptococcus pasteurianus ATCC 43144]
gi|374338350|ref|YP_005095062.1| , CRISPR-associated protein, Csn1 family [Streptococcus macedonicus ACA-DC 198]
gi|288905632|ref|YP_003430854.1| , CRISPR-associated protein, Csn1 family [Streptococcus gallolyticus UCN34]
gi|171779984|ref|ZP_02920888.1| , hypothetical protein STRINF_01771 [Streptococcus infantarius subsp. infantarius ATCC BAA-102...
gi|223932525|ref|ZP_03624526.1| , CRISPR-associated protein, Csn1 family [Streptococcus suis 89/1591]
gi|330833104|ref|YP_004401929.1| , CRISPR-associated protein, Csn1 family [Streptococcus suis ST3]
gi|336394701|ref|ZP_08576100.1| , CRISPR-associated protein, Csn1 family [Lactobacillus farciminis KCTC 3681]
gi|154482474|ref|ZP_02024922.1| , hypothetical protein EUBVEN_00141 [Eubacterium ventriosum ATCC 27560]
gi|238924075|ref|YP_002937591.1| , CRISPR-system related protein [Eubacterium rectale ATCC 33656]
gi|315149830|gb|EFT93846.1| , CRISPR-associated protein, Csn1 family [Enterococcus faecalis TX0012]
gi|160915782|ref|ZP_02077990.1| , hypothetical protein EUBDOL_01797 [Eubacterium dolichum DSM 3991]
gi|169349750|ref|ZP_02866688.1| , hypothetical protein CLOSPI_00488 [Clostridium spiroforme DSM 1552]
gi|315659848|ref|ZP_07912707.1| , conserved hypothetical protein [Staphylococcus lugdunensis M23590]
gi|403411236|emb|CCK74173.1| , CRISPR-associated protein Cas9/Csn1 [Staphylococcus aureus subsp. aureus]
gi|47458868|ref|YP_015730.1| , hypothetical protein MMOB0330 [Mycoplasma mobile 163K]
gi|224543312|ref|ZP_03683851.1| , hypothetical protein CATMIT_02512 [Catenibacterium mitsuokai DSM 15897]
gi|34762592|ref|ZP_00143587.1| , hypothetical protein [Fusobacterium nucleatum subsp. vincentii ATCC 49256]
gi|374307738|ref|YP_005054169.1| , CRISPR-associated protein, Csn1 family [Filifactor alocis ATCC 35896]
gi|153855454|ref|ZP_01996585.1| , hypothetical protein DORLON_02599 [Dorea longicatena DSM 13814]
gi|291520705|emb|CBK78998.1| , CRISPR-associated endonuclease, Csn1 family [Coprococcus catus GD/7]
gi|197301447|ref|ZP_03166527.1| , hypothetical protein RUMLAC_00178 [Ruminococcus lactaris ATCC 29176]
gi|42525843|ref|NP_970941.1| , CRISPR-associated Cas5e [Treponema denticola ATCC 35405]
gi|227501312|ref|ZP_03931361.1| , CRISPR associated protein [Anaerococcus tetradius ATCC 35098]
gi|309803917|ref|ZP_07698001.1| , CRISPR-associated protein, Csn1 family [Lactobacillus iners LactinV 11V1-d]
gi|304438954|ref|ZP_07398877.1| , csn1 family CRISPR-associated protein [Peptoniphilus duerdenii ATCC BAA-1640]
gi|320528778|ref|ZP_08029929.1| , CRISPR-associated protein, Csn1 family [Solobacterium moorei F0204]
gi|227824983|ref|ZP_03989815.1| , crispr-associated protein [Acidaminococcus sp. D21]
gi|352684361|ref|YP_004896346.1| , crispr-associated protein [Acidaminococcus intestini RyC-MR95]

FIG.17D

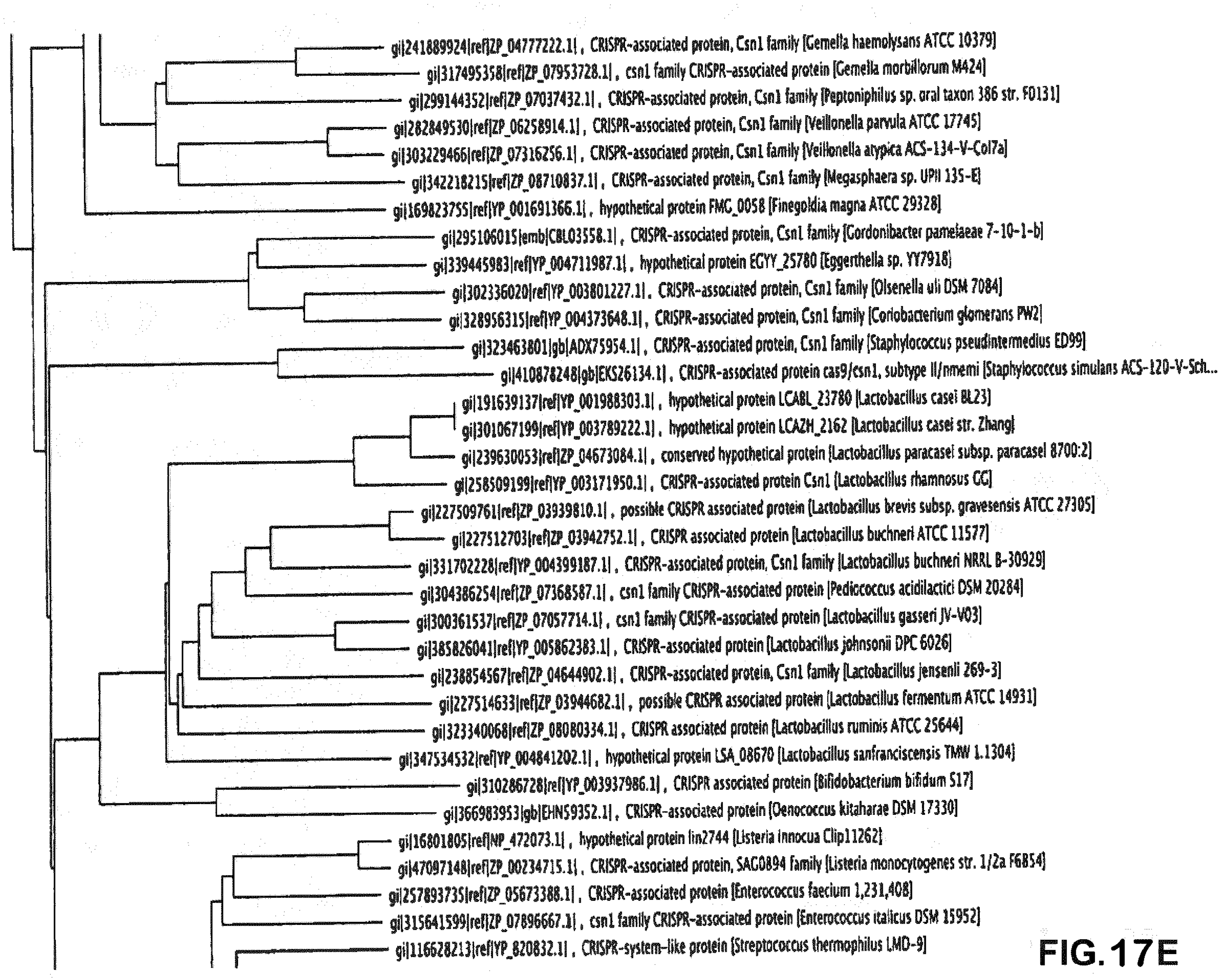
gi|241889924|ref|ZP_04777222.1| , CRISPR-associated protein, Csn1 family [Gemella haemolysans ATCC 10379]
gi|317495358|ref|ZP_07953728.1| , csn1 family CRISPR-associated protein [Gemella morbillorum M424]
gi|299144352|ref|ZP_07037432.1| , CRISPR-associated protein, Csn1 family [Peptoniphilus sp. oral taxon 386 str. F0131]
gi|282849530|ref|ZP_06258914.1| , CRISPR-associated protein, Csn1 family [Veillonella parvula ATCC 17745]
gi|303229466|ref|ZP_07316256.1| , CRISPR-associated protein, Csn1 family [Veillonella atypica ACS-134-V-Col7a]
gi|342218215|ref|ZP_08710837.1| , CRISPR-associated protein, Csn1 family [Megasphaera sp. UPII 135-E]
gi|169823755|ref|YP_001691366.1| , hypothetical protein FMG_0058 [Finegoldia magna ATCC 29328]
gi|295106015|emb|CBL03558.1| , CRISPR-associated protein, Csn1 family [Gordonibacter pamelaeae 7-10-1-b]
gi|339445983|ref|YP_004711987.1| , hypothetical protein EGYY_25780 [Eggerthella sp. YY7918]
gi|302336020|ref|YP_003801227.1| , CRISPR-associated protein, Csn1 family [Olsenella uli DSM 7084]
gi|328956315|ref|YP_004373648.1| , CRISPR-associated protein, Csn1 family [Coriobacterium glomerans PW2]
gi|323463801|gb|ADX75954.1| , CRISPR-associated protein, Csn1 family [Staphylococcus pseudintermedius ED99]
gi|410878248|gb|EKS26134.1| , CRISPR-associated protein cas9/csn1, subtype II/nmemi [Staphylococcus simulans ACS-120-V-Sch...
gi|191639137|ref|YP_001988303.1| , hypothetical protein LCABL_23780 [Lactobacillus casei BL23]
gi|301067199|ref|YP_003789222.1| , hypothetical protein LCAZH_2162 [Lactobacillus casei str. Zhang]
gi|239630053|ref|ZP_04673084.1| , conserved hypothetical protein [Lactobacillus paracasei subsp. paracasei 8700:2]
gi|258509199|ref|YP_003171950.1| , CRISPR-associated protein Csn1 [Lactobacillus rhamnosus GG]
gi|227509761|ref|ZP_03939810.1| , possible CRISPR associated protein [Lactobacillus brevis subsp. gravesensis ATCC 27305]
gi|227512703|ref|ZP_03942752.1| , CRISPR associated protein [Lactobacillus buchneri ATCC 11577]
gi|331702228|ref|YP_004399187.1| , CRISPR-associated protein, Csn1 family [Lactobacillus buchneri NRRL B-30929]
gi|304386254|ref|ZP_07368587.1| , csn1 family CRISPR-associated protein [Pediococcus acidilactici DSM 20284]
gi|300361537|ref|ZP_07057714.1| , csn1 family CRISPR-associated protein [Lactobacillus gasseri JV-V03]
gi|385826041|ref|YP_005862383.1| , CRISPR-associated protein [Lactobacillus johnsonii DPC 6026]
gi|238854567|ref|ZP_04644902.1| , CRISPR-associated protein, Csn1 family [Lactobacillus jensenii 269-3]
gi|227514633|ref|ZP_03944682.1| , possible CRISPR associated protein [Lactobacillus fermentum ATCC 14931]
gi|323340068|ref|ZP_08080334.1| , CRISPR associated protein [Lactobacillus ruminis ATCC 25644]
gi|347534532|ref|YP_004841202.1| , hypothetical protein LSA_08670 [Lactobacillus sanfranciscensis TMW 1.1304]
gi|310286728|ref|YP_003937986.1| , CRISPR associated protein [Bifidobacterium bifidum S17]
gi|366983953|gb|EHN59352.1| , CRISPR-associated protein [Oenococcus kitaharae DSM 17330]
gi|16801805|ref|NP_472073.1| , hypothetical protein lin2744 [Listeria innocua Clip11262]
gi|47097148|ref|ZP_00234715.1| , CRISPR-associated protein, SAG0894 family [Listeria monocytogenes str. 1/2a F6854]
gi|257893735|ref|ZP_05673388.1| , CRISPR-associated protein [Enterococcus faecium 1,231,408]
gi|315641599|ref|ZP_07896667.1| , csn1 family CRISPR-associated protein [Enterococcus italicus DSM 15952]
gi|116628213|ref|YP_820832.1| , CRISPR-system-like protein [Streptococcus thermophilus LMD-9]

FIG.17E

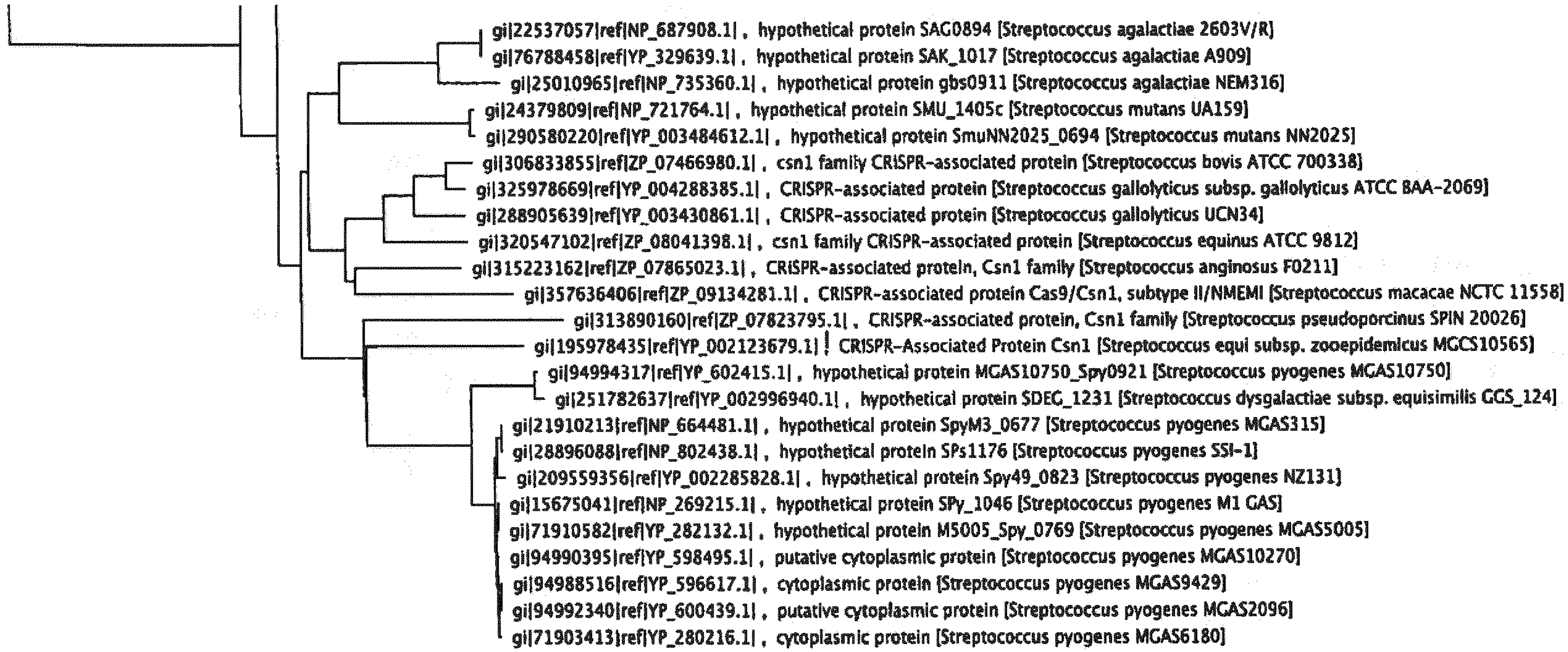
gi|22537057|ref|NP_687908.1| , hypothetical protein SAG0894 [Streptococcus agalactiae 2603V/R]
gi|76788458|ref|YP_329639.1| , hypothetical protein SAK_1017 [Streptococcus agalactiae A909]
gi|25010965|ref|NP_735360.1| , hypothetical protein gbs0911 [Streptococcus agalactiae NEM316]
gi|24379809|ref|NP_721764.1| , hypothetical protein SMU_1405c [Streptococcus mutans UA159]
gi|290580220|ref|YP_003484612.1| , hypothetical protein SmuNN2025_0694 [Streptococcus mutans NN2025]
gi|306833855|ref|ZP_07466980.1| , csn1 family CRISPR-associated protein [Streptococcus bovis ATCC 700338]
gi|325978669|ref|YP_004288385.1| , CRISPR-associated protein [Streptococcus gallolyticus subsp. gallolyticus ATCC BAA-2069]
gi|288905639|ref|YP_003430861.1| , CRISPR-associated protein [Streptococcus gallolyticus UCN34]
gi|320547102|ref|ZP_08041398.1| , csn1 family CRISPR-associated protein [Streptococcus equinus ATCC 9812]
gi|315223162|ref|ZP_07865023.1| , CRISPR-associated protein, Csn1 family [Streptococcus anginosus F0211]
gi|357636406|ref|ZP_09134281.1| , CRISPR-associated protein Cas9/Csn1, subtype II/NMEMI [Streptococcus macacae NCTC 11558]
gi|313890160|ref|ZP_07823795.1| , CRISPR-associated protein, Csn1 family [Streptococcus pseudoporcinus SPIN 20026]
gi|195978435|ref|YP_002123679.1| CRISPR-Associated Protein Csn1 [Streptococcus equi subsp. zooepidemicus MGCS10565]
gi|94994317|ref|YP_602415.1| , hypothetical protein MGAS10750_Spy0921 [Streptococcus pyogenes MGAS10750]
gi|251782637|ref|YP_002996940.1| , hypothetical protein SDEG_1231 [Streptococcus dysgalactiae subsp. equisimilis GGS_124]
gi|21910213|ref|NP_664481.1| , hypothetical protein SpyM3_0677 [Streptococcus pyogenes MGAS315]
gi|28896088|ref|NP_802438.1| , hypothetical protein SPs1176 [Streptococcus pyogenes SSI-1]
gi|209559356|ref|YP_002285828.1| , hypothetical protein Spy49_0823 [Streptococcus pyogenes NZ131]
gi|15675041|ref|NP_269215.1| , hypothetical protein SPy_1046 [Streptococcus pyogenes M1 GAS]
gi|71910582|ref|YP_282132.1| , hypothetical protein M5005_Spy_0769 [Streptococcus pyogenes MGAS5005]
gi|94990395|ref|YP_598495.1| , putative cytoplasmic protein [Streptococcus pyogenes MGAS10270]
gi|94988516|ref|YP_596617.1| , cytoplasmic protein [Streptococcus pyogenes MGAS9429]
gi|94992340|ref|YP_600439.1| , putative cytoplasmic protein [Streptococcus pyogenes MGAS2096]
gi|71903413|ref|YP_280216.1| , cytoplasmic protein [Streptococcus pyogenes MGAS6180]

FIG.17F

a

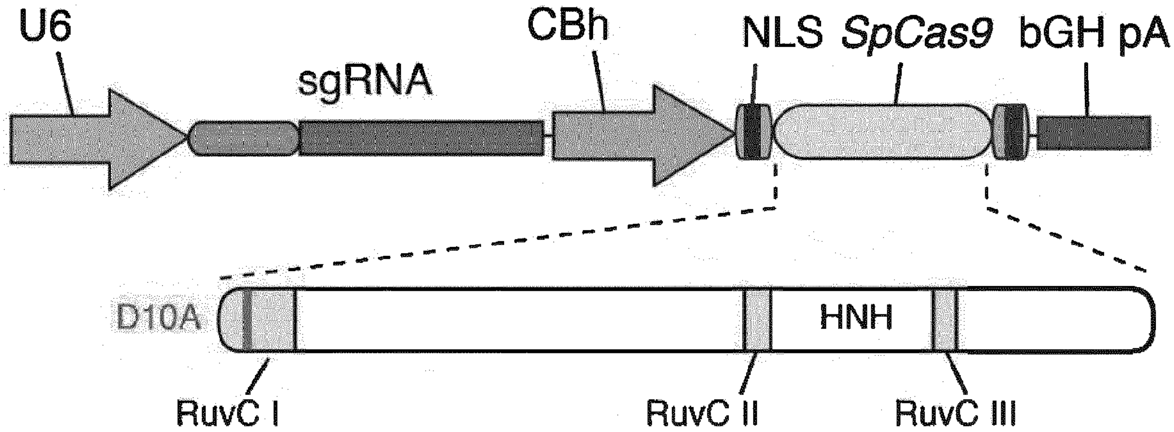


FIG. 18A

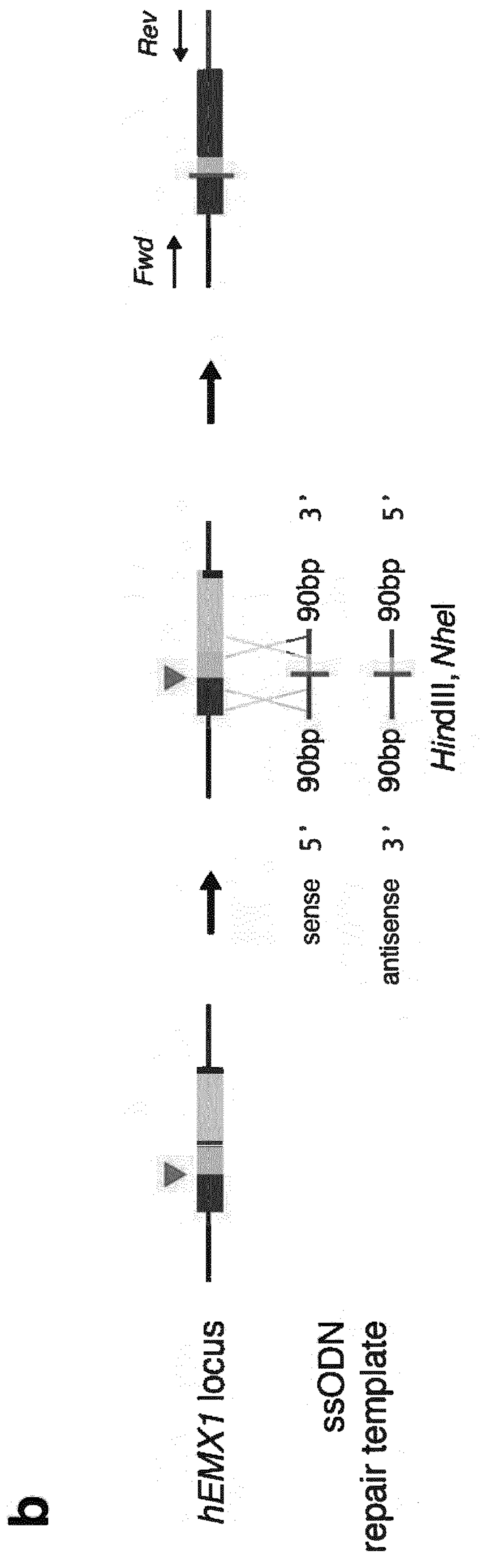
b
hEMX1 locus
ssODN
repair template
sense 5' 90bp 90bp 3'
antisense 3' 90bp 90bp 5'
HindIII, NheI
Fwd
Rev

FIG. 18B

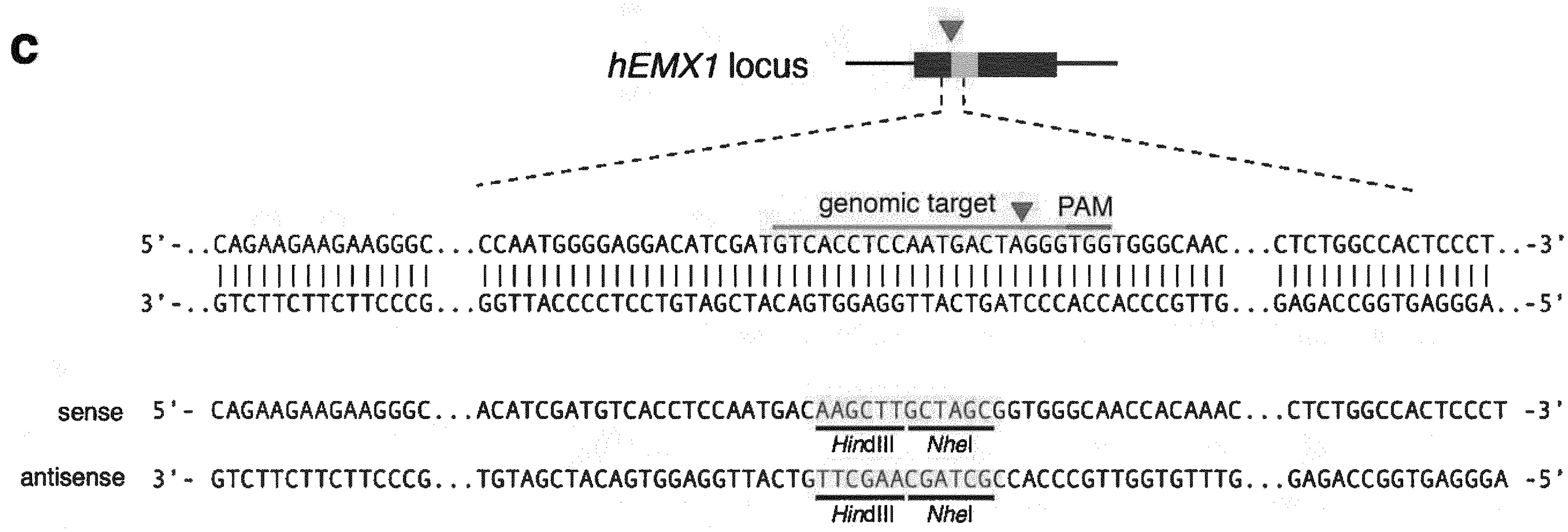
c
hEMX1 locus
genomic target
PAM
5'-..CAGAAGAAGAAGGGC...CCAATGGGGAGGACATCGATGTCACCTCCAATGACTAGGGTGGTGGGCAAC...CTCTGGCCACTCCCT..-3'
3'-..GTCTTCTTCTTCCCG...GGTTACCCCTCCTGTAGCTACAGTGGAGGTTACTGATCCCACCACCCGTTG...GAGACCGGTGAGGGA..-5'
sense
5'- CAGAAGAAGAAGGGC...ACATCGATGTCACCTCCAATGACAAGCTTGCTAGCGGTGGGCAACCACAAAC...CTCTGGCCACTCCCT -3'
HindIII
NheI
antisense
3'- GTCTTCTTCTTCCCG...TGTAGCTACAGTGGAGGTTACTGTTCGAACGATCGCCACCCGTTGGTGTTTG...GAGACCGGTGAGGGA -5'
HindIII
NheI

FIG. 18C

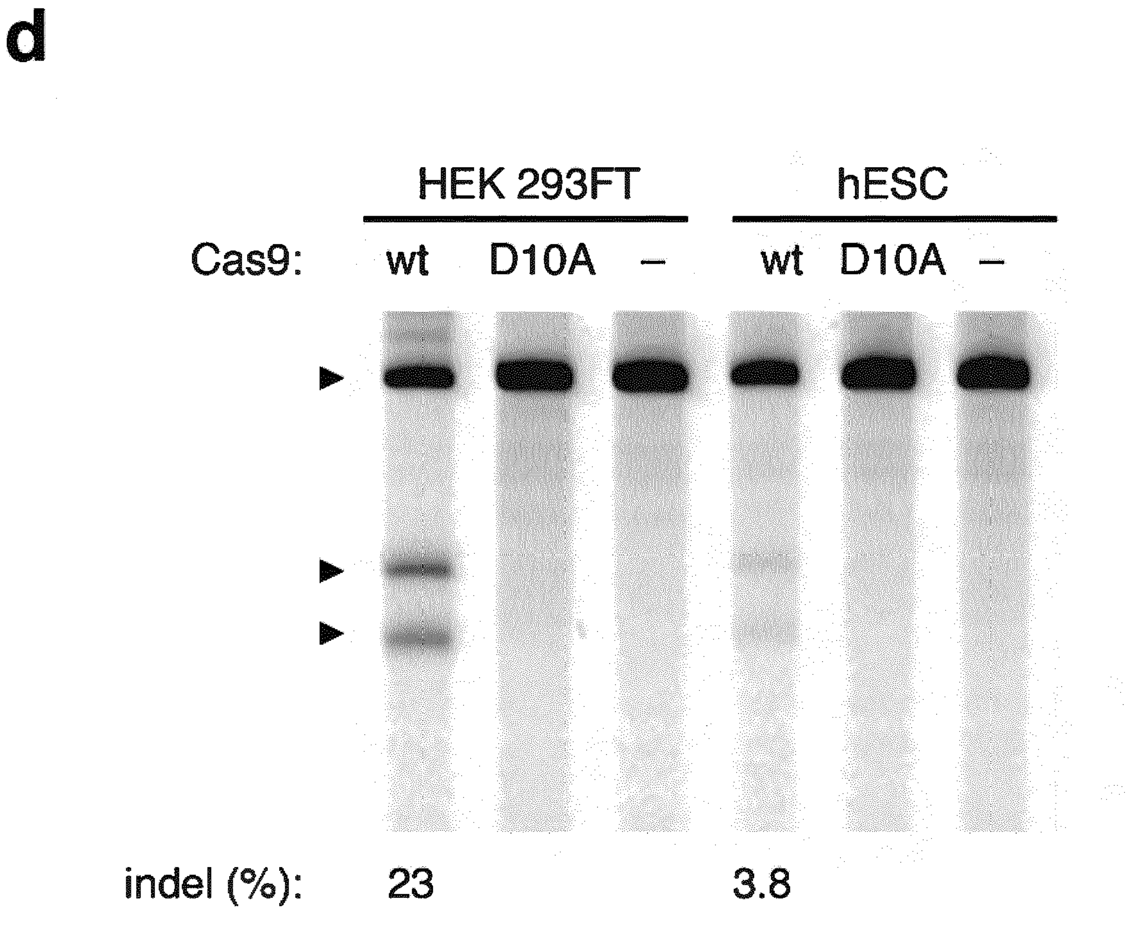


FIG. 18D

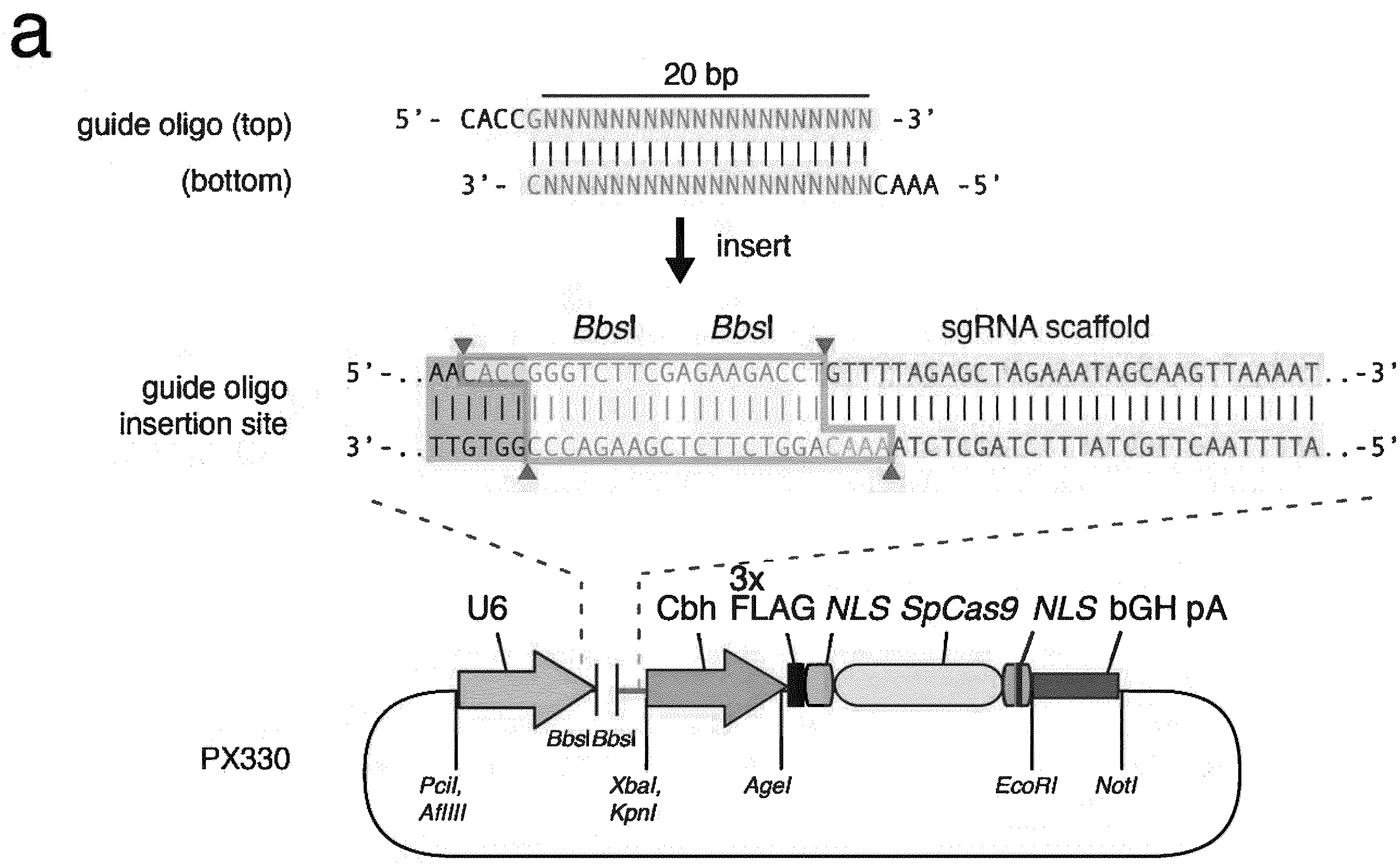
a
20 bp
guide oligo (top)
5'- CACCGNNNNNNNNNNNNNNNNNNN -3'
(bottom)
3'- CNNNNNNNNNNNNNNNNNNNCAAA -5'
insert
BbsI
BbsI
sgRNA scaffold
guide oligo insertion site
5'-..AACACCGGGTCTTCGAGAAGACCTGTTTTAGAGCTAGAAATAGCAAGTTAAAAT..-3'
3'-..TTGTGGCCCAGAAGCTCTTCTGGACAAAATCTCGATCTTTATCGTTCAATTTTA..-5'
U6
Cbh
3x FLAG
NLS
SpCas9
NLS
bGH pA
PX330
BbsI BbsI
PciI, AflIII
XbaI, KpnI
AgeI
EcoRI
NotI

FIG. 19A

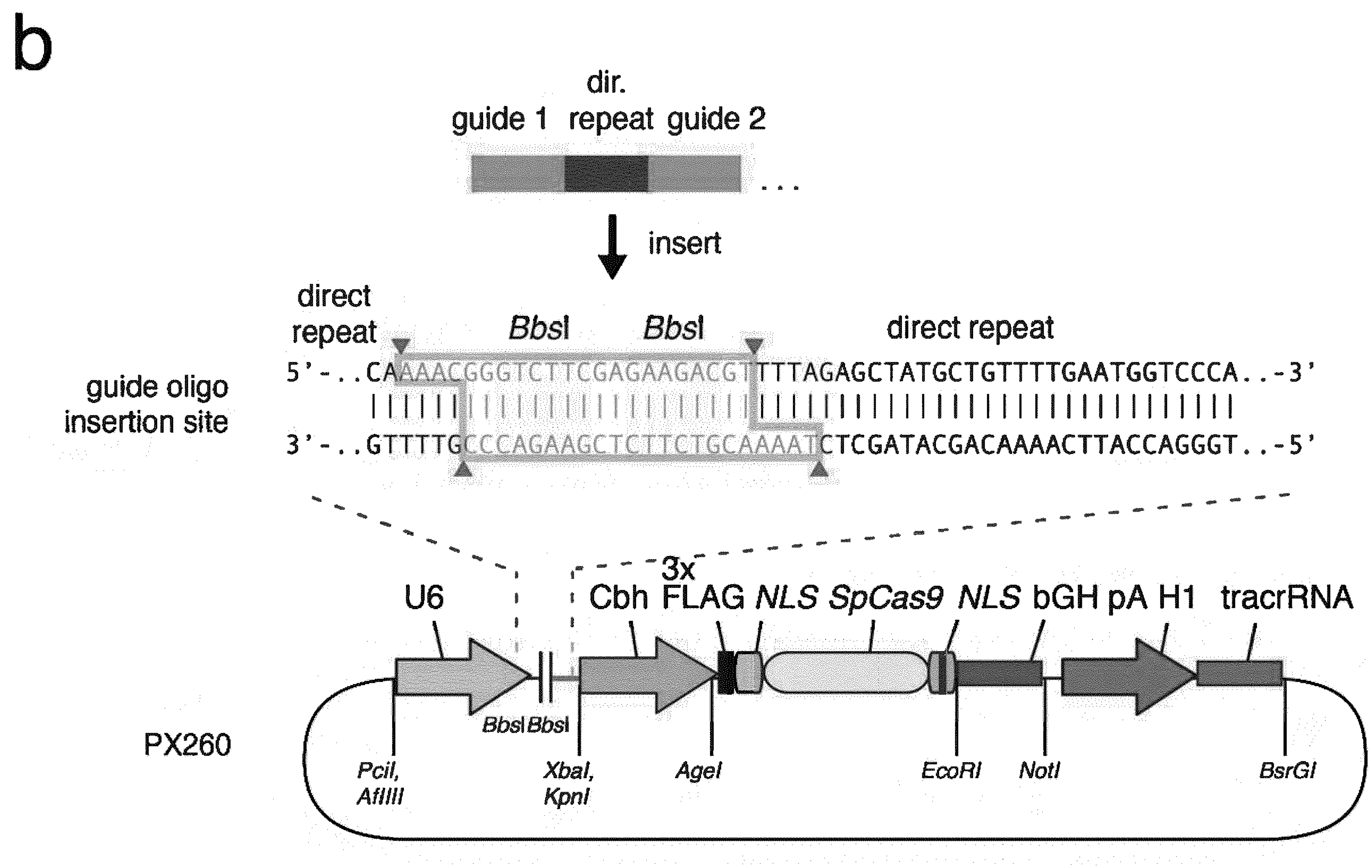
b
dir.
guide 1 repeat guide 2
insert
direct repeat
BbsI
BbsI
direct repeat
guide oligo insertion site
5'-..CAAAACGGGTCTTCGAGAAGACGTTTTAGAGCTATGCTGTTTTGAATGGTCCCA..-3'
3'-..GTTTTGCCCAGAAGCTCTTCTGCAAAATCTCGATACGACAAAACTTACCAGGGT..-5'
PX260
U6
Cbh
3x FLAG
NLS
SpCas9
NLS
bGH pA
H1
tracrRNA
BbsI BbsI
PciI, AflIII
XbaI, KpnI
AgeI
EcoRI
NotI
BsrGI

FIG.19B

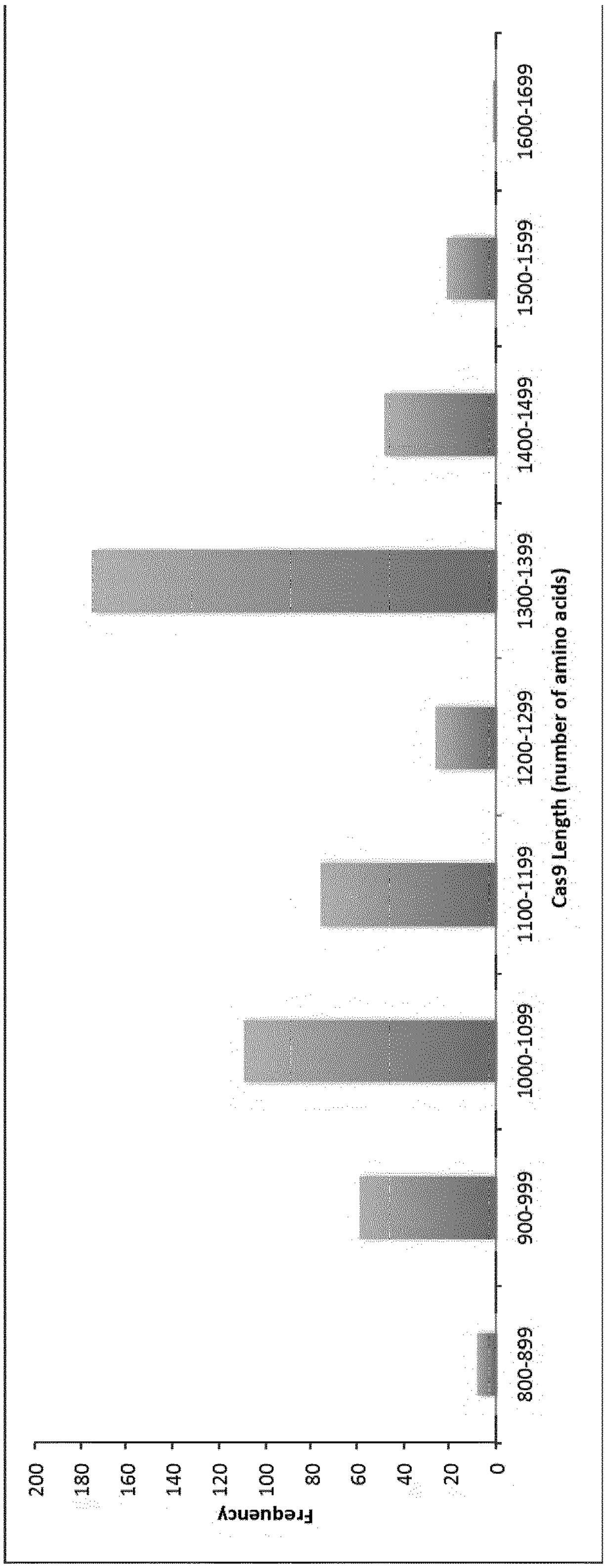
Frequency
200
180
160
140
120
100
80
60
40
20
0
800-899
900-999
1000-1099
1100-1199
1200-1299
1300-1399
1400-1499
1500-1599
1600-1699
Cas9 Length (number of amino acids)

FIG. 20

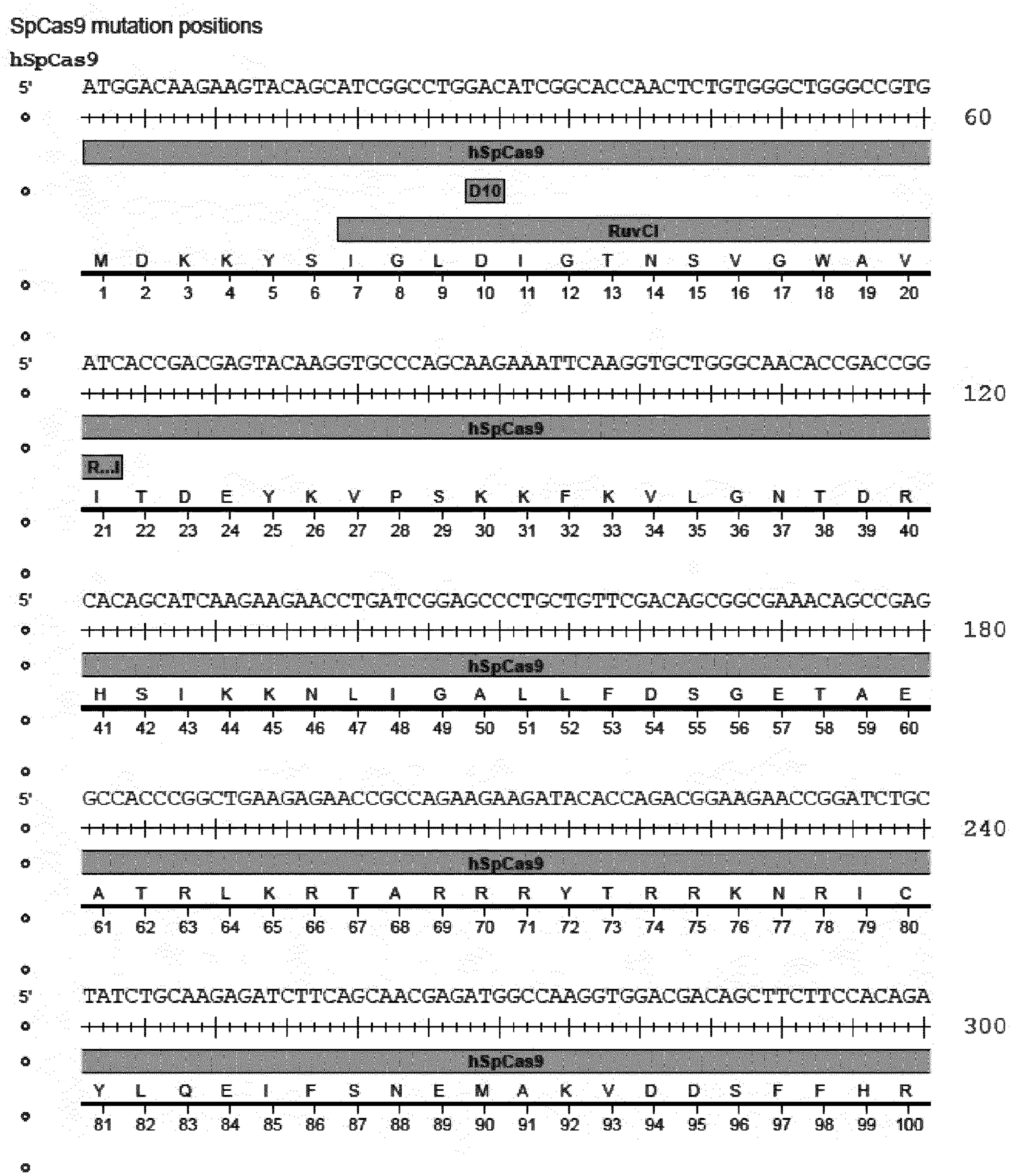
SpCas9 mutation positions
hSpCas9
5' ATGGACAAGAAGTACAGCATCGGCCTGGACATCGGCACCAACTCTGTGGGCTGGGCCGTG 60
hSpCas9
D10
RuvCI
M D K K Y S I G L D I G T N S V G W A V
1 2 3 4 5 6 7 8 9 10 11 12 13 14 15 16 17 18 19 20
5' ATCACCGACGAGTACAAGGTGCCCAGCAAGAAATTCAAGGTGCTGGGCAACACCGACCGG 120
hSpCas9
R...I
I T D E Y K V P S K K F K V L G N T D R
21 22 23 24 25 26 27 28 29 30 31 32 33 34 35 36 37 38 39 40
5' CACAGCATCAAGAAGAACCTGATCGGAGCCCTGCTGTTCGACAGCGGCGAAACAGCCGAG 180
hSpCas9
H S I K K N L I G A L L F D S G E T A E
41 42 43 44 45 46 47 48 49 50 51 52 53 54 55 56 57 58 59 60
5' GCCACCCGGCTGAAGAGAACCGCCAGAAGAAGATACACCAGACGGAAGAACCGGATCTGC 240
hSpCas9
A T R L K R T A R R R Y T R R K N R I C
61 62 63 64 65 66 67 68 69 70 71 72 73 74 75 76 77 78 79 80
5' TATCTGCAAGAGATCTTCAGCAACGAGATGGCCAAGGTGGACGACAGCTTCTTCCACAGA 300
hSpCas9
Y L Q E I F S N E M A K V D D S F F H R
81 82 83 84 85 86 87 88 89 90 91 92 93 94 95 96 97 98 99 100

FIG. 21A

hSpCas9

5' CTGGAAGAGTCCTTCCTGGTGGAAGAGGATAAGAAGCACGAGCGGCACCCCATCTTCGGC 360

hSpCas9

L E E S F L V E E D K K H E R H P I F G

101 102 103 104 105 106 107 108 109 110 111 112 113 114 115 116 117 118 119 120

5' AACATCGTGGACGAGGTGGCCTACCACGAGAAGTACCCCACCATCTACCACCTGAGAAAG 420

hSpCas9

N I V D E V A Y H E K Y P T I Y H L R K

121 122 123 124 125 126 127 128 129 130 131 132 133 134 135 136 137 138 139 140

5' AAACTGGTGGACAGCACCGACAAGGCCGACCTGCGGCTGATCTATCTGGCCCTGGCCCAC 480

hSpCas9

K L V D S T D K A D L R L I Y L A L A H

141 142 143 144 145 146 147 148 149 150 151 152 153 154 155 156 157 158 159 160

5' ATGATCAAGTTCCGGGGCCACTTCCTGATCGAGGGCGACCTGAACCCCGACAACAGCGAC 540

hSpCas9

M I K F R G H F L I E G D L N P D N S D

161 162 163 164 165 166 167 168 169 170 171 172 173 174 175 176 177 178 179 180

5' GTGGACAAGCTGTTCATCCAGCTGGTGCAGACCTACAACCAGCTGTTCGAGGAAAACCCC 600

hSpCas9

V D K L F I Q L V Q T Y N Q L F E E N P

181 182 183 184 185 186 187 188 189 190 191 192 193 194 195 196 197 198 199 200

5' ATCAACGCCAGCGGCGTGGACGCCAAGGCCATCCTGTCTGCCAGACTGAGCAAGAGCAGA 660

hSpCas9

I N A S G V D A K A I L S A R L S K S R

201 202 203 204 205 206 207 208 209 210 211 212 213 214 215 216 217 218 219 220

FIG. 21B

**hSpCas9**

5' CGGCTGGAAAATCTGATCGCCCAGCTGCCCGGCGAGAAGAAGAATGGCCTGTTCGGCAAC 720

hSpCas9

R L E N L I A Q L P G E K K N G L F G N

221 222 223 224 225 226 227 228 229 230 231 232 233 234 235 236 237 238 239 240

5' CTGATTGCCCTGAGCCTGGGCCTGACCCCCAACTTCAAGAGCAACTTCGACCTGGCCGAG 780

hSpCas9

L I A L S L G L T P N F K S N F D L A E

241 242 243 244 245 246 247 248 249 250 251 252 253 254 255 256 257 258 259 260

5' GATGCCAAACTGCAGCTGAGCAAGGACACCTACGACGACGACCTGGACAACCTGCTGGCC 840

hSpCas9

D A K L Q L S K D T Y D D D L D N L L A

261 262 263 264 265 266 267 268 269 270 271 272 273 274 275 276 277 278 279 280

5' CAGATCGGCGACCAGTACGCCGACCTGTTTCTGGCCGCCAAGAACCTGTCCGACGCCATC 900

hSpCas9

Q I G D Q Y A D L F L A A K N L S D A I

281 282 283 284 285 286 287 288 289 290 291 292 293 294 295 296 297 298 299 300

5' CTGCTGAGCGACATCCTGAGAGTGAACACCGAGATCACCAAGGCCCCCCTGAGCGCCTCT 960

hSpCas9

L L S D I L R V N T E I T K A P L S A S

301 302 303 304 305 306 307 308 309 310 311 312 313 314 315 316 317 318 319 320

5' ATGATCAAGAGATACGACGAGCACCACCAGGACCTGACCCTGCTGAAAGCTCTCGTGCGG 1020

hSpCas9

M I K R Y D E H H Q D L T L L K A L V R

321 322 323 324 325 326 327 328 329 330 331 332 333 334 335 336 337 338 339 340

FIG. 21C

**hSpCas9**

5' CAGCAGCTGCCTGAGAAGTACAAAGAGATTTTCTTCGACCAGAGCAAGAACGGCTACGCC 1080

hSpCas9

Q Q L P E K Y K E I F F D Q S K N G Y A

341 342 343 344 345 346 347 348 349 350 351 352 353 354 355 356 357 358 359 360

5' GGCTACATTGACGGCGGAGCCAGCCAGGAAGAGTTCTACAAGTTCATCAAGCCCATCCTG 1140

hSpCas9

G Y I D G G A S Q E E F Y K F I K P I L

361 362 363 364 365 366 367 368 369 370 371 372 373 374 375 376 377 378 379 380

5' GAAAAGATGGACGGCACCGAGGAACTGCTCGTGAAGCTGAACAGAGAGGACCTGCTGCGG 1200

hSpCas9

E K M D G T E E L L V K L N R E D L L R

381 382 383 384 385 386 387 388 389 390 391 392 393 394 395 396 397 398 399 400

5' AAGCAGCGGACCTTCGACAACGGCAGCATCCCCCACCAGATCCACCTGGGAGAGCTGCAC 1260

hSpCas9

K Q R T F D N G S I P H Q I H L G E L H

401 402 403 404 405 406 407 408 409 410 411 412 413 414 415 416 417 418 419 420

5' GCCATTCTGCGGCGGCAGGAAGATTTTTACCCATTCCTGAAGGACAACCGGGAAAAGATC 1320

hSpCas9

A I L R R Q E D F Y P F L K D N R E K I

421 422 423 424 425 426 427 428 429 430 431 432 433 434 435 436 437 438 439 440

5' GAGAAGATCCTGACCTTCCGCATCCCCTACTACGTGGGCCCTCTGGCCAGGGGAAACAGC 1380

hSpCas9

E K I L T F R I P Y Y V G P L A R G N S

441 442 443 444 445 446 447 448 449 450 451 452 453 454 455 456 457 458 459 460

FIG. 21D

**hSpCas9**

5' AGATTCGCCTGGATGACCAGAAAGAGCGAGGAAACCATCACCCCCTGGAACTTCGAGGAA 1440

hSpCas9

R F A W M T R K S E E T I T P W N F E E

461 462 463 464 465 466 467 468 469 470 471 472 473 474 475 476 477 478 479 480

5' GTGGTGGACAAGGGCGCTTCCGCCCAGAGCTTCATCGAGCGGATGACCAACTTCGATAAG 1500

hSpCas9

V V D K G A S A Q S F I E R M T N F D K

481 482 483 484 485 486 487 488 489 490 491 492 493 494 495 496 497 498 499 500

5' AACCTGCCCAACGAGAAGGTGCTGCCCAAGCACAGCCTGCTGTACGAGTACTTCACCGTG 1560

hSpCas9

N L P N E K V L P K H S L L Y E Y F T V

501 502 503 504 505 506 507 508 509 510 511 512 513 514 515 516 517 518 519 520

5' TATAACGAGCTGACCAAAGTGAAATACGTGACCGAGGGAATGAGAAAGCCCGCCTTCCTG 1620

hSpCas9

Y N E L T K V K Y V T E G M R K P A F L

521 522 523 524 525 526 527 528 529 530 531 532 533 534 535 536 537 538 539 540

5' AGCGGCGAGCAGAAAAAGGCCATCGTGGACCTGCTGTTCAAGACCAACCGGAAAGTGACC 1680

hSpCas9

S G E Q K K A I V D L L F K T N R K V T

541 542 543 544 545 546 547 548 549 550 551 552 553 554 555 556 557 558 559 560

5' GTGAAGCAGCTGAAAGAGGACTACTTCAAGAAAATCGAGTGCTTCGACTCCGTGGAAATC 1740

hSpCas9

V K Q L K E D Y F K K I E C F D S V E I

561 562 563 564 565 566 567 568 569 570 571 572 573 574 575 576 577 578 579 580

FIG. 21E

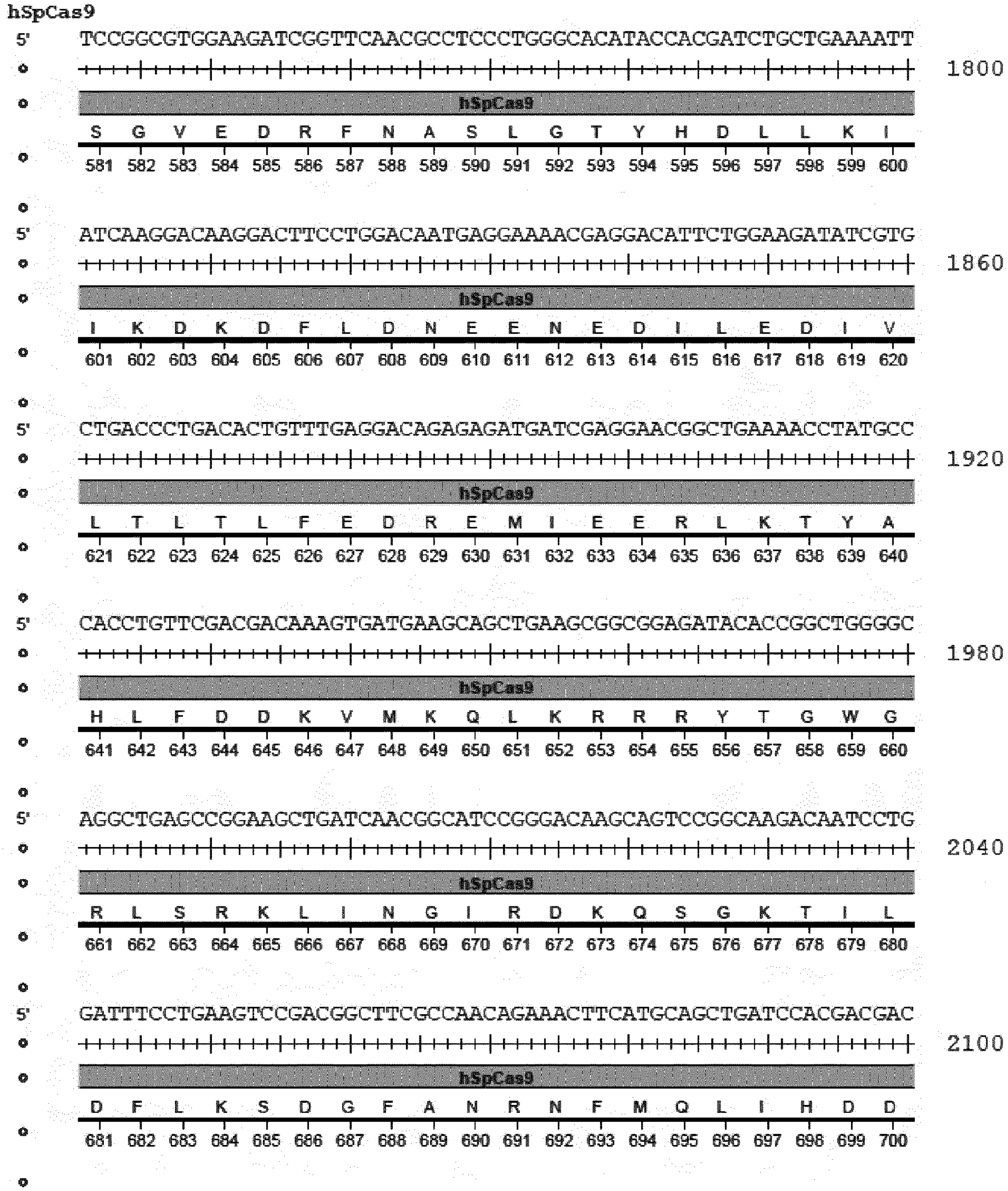
hSpCas9
5' TCCGGCGTGGAAGATCGGTTCAACGCCTCCCTGGGCACATACCACGATCTGCTGAAAATT 1800
hSpCas9
S G V E D R F N A S L G T Y H D L L K I
581 582 583 584 585 586 587 588 589 590 591 592 593 594 595 596 597 598 599 600
5' ATCAAGGACAAGGACTTCCTGGACAATGAGGAAAACGAGGACATTCTGGAAGATATCGTG 1860
hSpCas9
I K D K D F L D N E E N E D I L E D I V
601 602 603 604 605 606 607 608 609 610 611 612 613 614 615 616 617 618 619 620
5' CTGACCCTGACACTGTTTGAGGACAGAGAGATGATCGAGGAACGGCTGAAAACCTATGCC 1920
hSpCas9
L T L T L F E D R E M I E E R L K T Y A
621 622 623 624 625 626 627 628 629 630 631 632 633 634 635 636 637 638 639 640
5' CACCTGTTCGACGACAAAGTGATGAAGCAGCTGAAGCGGCGGAGATACACCGGCTGGGGC 1980
hSpCas9
H L F D D K V M K Q L K R R R Y T G W G
641 642 643 644 645 646 647 648 649 650 651 652 653 654 655 656 657 658 659 660
5' AGGCTGAGCCGGAAGCTGATCAACGGCATCCGGGACAAGCAGTCCGGCAAGACAATCCTG 2040
hSpCas9
R L S R K L I N G I R D K Q S G K T I L
661 662 663 664 665 666 667 668 669 670 671 672 673 674 675 676 677 678 679 680
5' GATTTCCTGAAGTCCGACGGCTTCGCCAACAGAAACTTCATGCAGCTGATCCACGACGAC 2100
hSpCas9
D F L K S D G F A N R N F M Q L I H D D
681 682 683 684 685 686 687 688 689 690 691 692 693 694 695 696 697 698 699 700

FIG. 21F

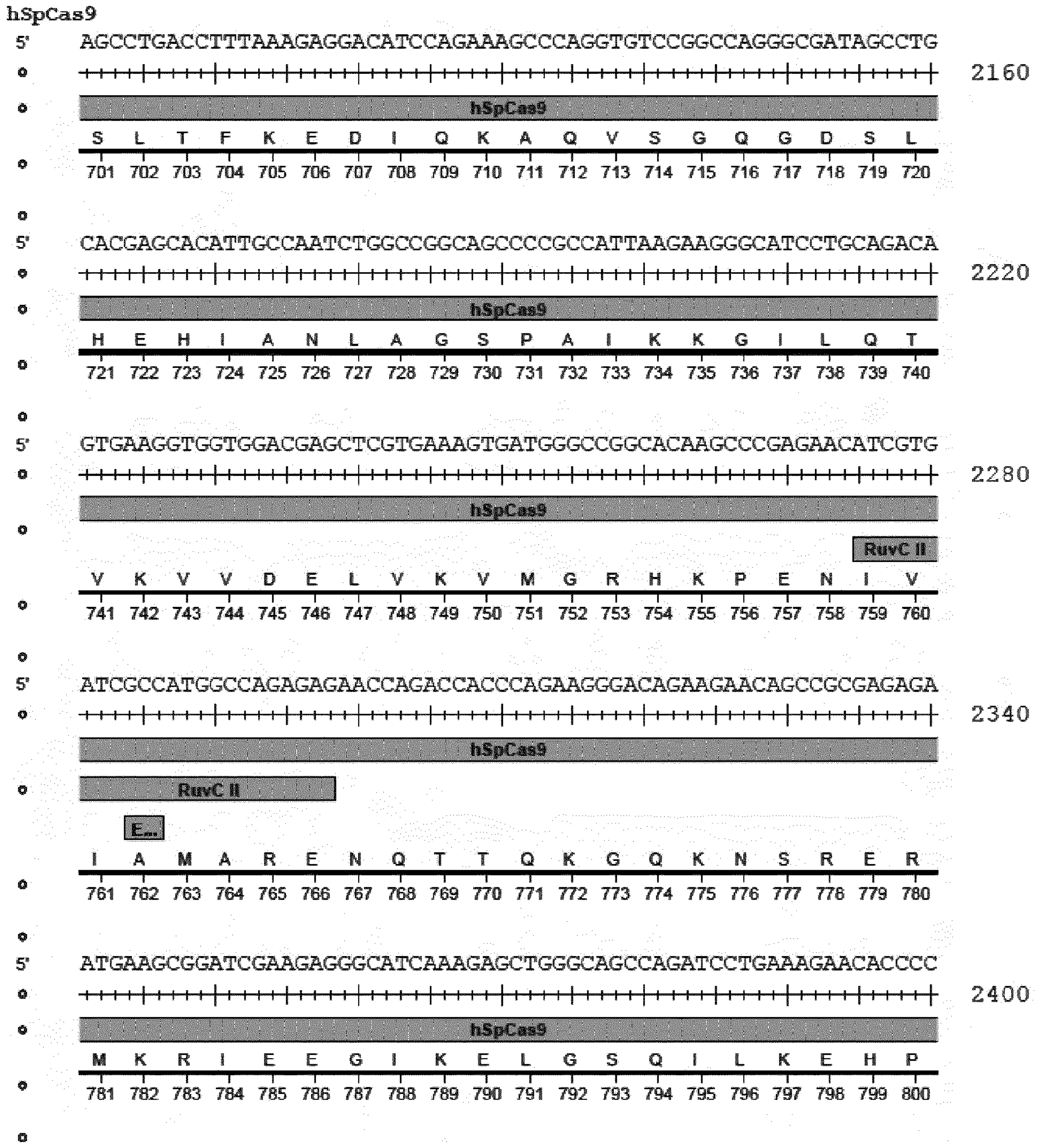
hSpCas9
5' AGCCTGACCTTTAAAGAGGACATCCAGAAAGCCCAGGTGTCCGGCCAGGGCGATAGCCTG 2160
hSpCas9
S L T F K E D I Q K A Q V S G Q G D S L
701 702 703 704 705 706 707 708 709 710 711 712 713 714 715 716 717 718 719 720
5' CACGAGCACATTGCCAATCTGGCCGGCAGCCCCGCCATTAAGAAGGGCATCCTGCAGACA 2220
hSpCas9
H E H I A N L A G S P A I K K G I L Q T
721 722 723 724 725 726 727 728 729 730 731 732 733 734 735 736 737 738 739 740
5' GTGAAGGTGGTGGACGAGCTCGTGAAAGTGATGGGCCGGCACAAGCCCGAGAACATCGTG 2280
hSpCas9
RuvC II
V K V V D E L V K V M G R H K P E N I V
741 742 743 744 745 746 747 748 749 750 751 752 753 754 755 756 757 758 759 760
5' ATCGCCATGGCCAGAGAGAACCAGACCACCCAGAAGGGACAGAAGAACAGCCGCGAGAGA 2340
hSpCas9
RuvC II
E...
I A M A R E N Q T T Q K G Q K N S R E R
761 762 763 764 765 766 767 768 769 770 771 772 773 774 775 776 777 778 779 780
5' ATGAAGCGGATCGAAGAGGGCATCAAAGAGCTGGGCAGCCAGATCCTGAAAGAACACCCC 2400
hSpCas9
M K R I E E G I K E L G S Q I L K E H P
781 782 783 784 785 786 787 788 789 790 791 792 793 794 795 796 797 798 799 800

FIG. 21G

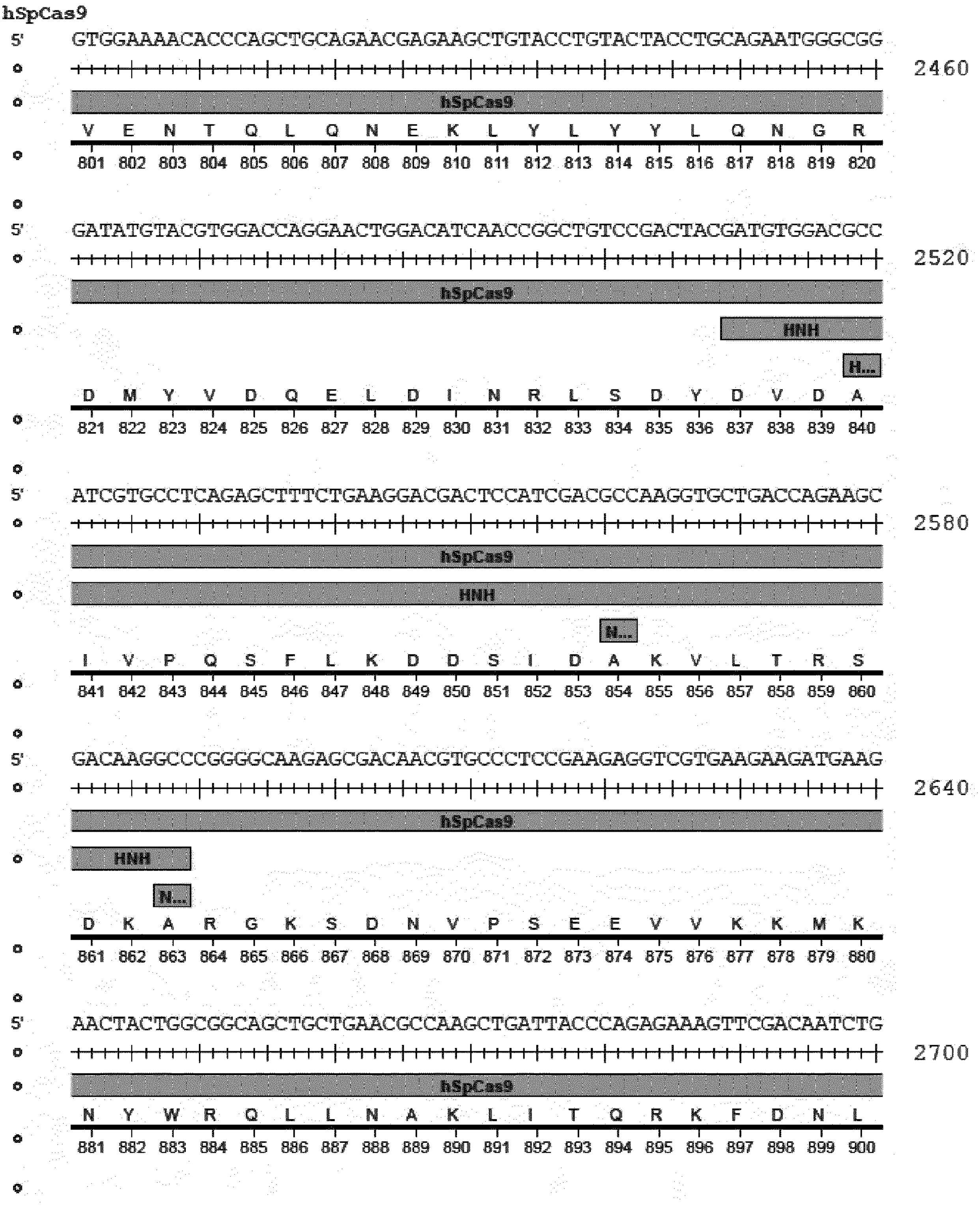
hSpCas9
5' GTGGAAAACACCCAGCTGCAGAACGAGAAGCTGTACCTGTACTACCTGCAGAATGGGCGG 2460
hSpCas9
V E N T Q L Q N E K L Y L Y Y L Q N G R
801 802 803 804 805 806 807 808 809 810 811 812 813 814 815 816 817 818 819 820
5' GATATGTACGTGGACCAGGAACTGGACATCAACCGGCTGTCCGACTACGATGTGGACGCC 2520
hSpCas9
HNH
H...
D M Y V D Q E L D I N R L S D Y D V D A
821 822 823 824 825 826 827 828 829 830 831 832 833 834 835 836 837 838 839 840
5' ATCGTGCCTCAGAGCTTTCTGAAGGACGACTCCATCGACGCCAAGGTGCTGACCAGAAGC 2580
hSpCas9
HNH
N...
I V P Q S F L K D D S I D A K V L T R S
841 842 843 844 845 846 847 848 849 850 851 852 853 854 855 856 857 858 859 860
5' GACAAGGCCCGGGGCAAGAGCGACAACGTGCCCTCCGAAGAGGTCGTGAAGAAGATGAAG 2640
hSpCas9
HNH
N...
D K A R G K S D N V P S E E V V K K M K
861 862 863 864 865 866 867 868 869 870 871 872 873 874 875 876 877 878 879 880
5' AACTACTGGCGGCAGCTGCTGAACGCCAAGCTGATTACCCAGAGAAAGTTCGACAATCTG 2700
hSpCas9
N Y W R Q L L N A K L I T Q R K F D N L
881 882 883 884 885 886 887 888 889 890 891 892 893 894 895 896 897 898 899 900

FIG. 21H

**hSpCas9**

5' ACCAAGGCCGAGAGAGGCGGCCTGAGCGAACTGGATAAGGCCGGCTTCATCAAGAGACAG 2760

hSpCas9

T K A E R G G L S E L D K A G F I K R Q

901 902 903 904 905 906 907 908 909 910 911 912 913 914 915 916 917 918 919 920

5' CTGGTGGAAACCCGGCAGATCACAAAGCACGTGGCACAGATCCTGGACTCCCGGATGAAC 2820

hSpCas9

L V E T R Q I T K H V A Q I L D S R M N

921 922 923 924 925 926 927 928 929 930 931 932 933 934 935 936 937 938 939 940

5' ACTAAGTACGACGAGAATGACAAGCTGATCCGGGAAGTGAAAGTGATCACCCTGAAGTCC 2880

hSpCas9

T K Y D E N D K L I R E V K V I T L K S

941 942 943 944 945 946 947 948 949 950 951 952 953 954 955 956 957 958 959 960

5' AAGCTGGTGTCCGATTTCCGGAAGGATTTCCAGTTTTACAAAGTGCGCGAGATCAACAAC 2940

hSpCas9

K L V S D F R K D F Q F Y K V R E I N N

961 962 963 964 965 966 967 968 969 970 971 972 973 974 975 976 977 978 979 980

5' TACCACCACGCCCACGCCGCCTACCTGAACGCCGTCGTGGGAACCGCCCTGATCAAAAAG 3000

hSpCas9

RuvC III

D...

Y H H A H A A Y L N A V V G T A L I K K

981 982 983 984 985 986 987 988 989 990 991 992 993 994 995 996 997 998 999 1000

FIG. 21I

hSpCas9

5' TACCCTAAGCTGGAAAGCGAGTTCGTGTACGGCGACTACAAGGTGTACGACGTGCGGAAG 3060

hSpCas9

Y P K L E S E F V Y G D Y K V Y D V R K

1001 1002 1003 1004 1005 1006 1007 1008 1009 1010 1011 1012 1013 1014 1015 1016 1017 1018 1019 1020

5' ATGATCGCCAAGAGCGAGCAGGAAATCGGCAAGGCTACCGCCAAGTACTTCTTCTACAGC 3120

hSpCas9

M I A K S E Q E I G K A T A K Y F F Y S

1021 1022 1023 1024 1025 1026 1027 1028 1029 1030 1031 1032 1033 1034 1035 1036 1037 1038 1039 1040

5' AACATCATGAACTTTTTCAAGACCGAGATTACCCTGGCCAACGGCGAGATCCGGAAGCGG 3180

hSpCas9

N I M N F F K T E I T L A N G E I R K R

1041 1042 1043 1044 1045 1046 1047 1048 1049 1050 1051 1052 1053 1054 1055 1056 1057 1058 1059 1060

5' CCTCTGATCGAGACAAACGGCGAAACCGGGGAGATCGTGTGGGATAAGGGCCGGGATTTT 3240

hSpCas9

P L I E T N G E T G E I V W D K G R D F

1061 1062 1063 1064 1065 1066 1067 1068 1069 1070 1071 1072 1073 1074 1075 1076 1077 1078 1079 1080

5' GCCACCGTGCGGAAAGTGCTGAGCATGCCCCAAGTGAATATCGTGAAAAAGACCGAGGTG 3300

hSpCas9

A T V R K V L S M P Q V N I V K K T E V

1081 1082 1083 1084 1085 1086 1087 1088 1089 1090 1091 1092 1093 1094 1095 1096 1097 1098 1099 1100

5' CAGACAGGCGGCTTCAGCAAAGAGTCTATCCTGCCCAAGAGGAACAGCGATAAGCTGATC 3360

hSpCas9

Q T G G F S K E S I L P K R N S D K L I

1101 1102 1103 1104 1105 1106 1107 1108 1109 1110 1111 1112 1113 1114 1115 1116 1117 1118 1119 1120

FIG. 21J

hSpCas9

5' GCCAGAAAGAAGGACTGGGACCCTAAGAAGTACGGCGGCTTCGACAGCCCCACCGTGGCC 3420

hSpCas9

A R K K D W D P K K Y G G F D S P T V A

1121 1122 1123 1124 1125 1126 1127 1128 1129 1130 1131 1132 1133 1134 1135 1136 1137 1138 1139 1140

5' TATTCTGTGCTGGTGGTGGCCAAAGTGGAAAAGGGCAAGTCCAAGAAACTGAAGAGTGTG 3480

hSpCas9

Y S V L V V A K V E K G K S K K L K S V

1141 1142 1143 1144 1145 1146 1147 1148 1149 1150 1151 1152 1153 1154 1155 1156 1157 1158 1159 1160

5' AAAGAGCTGCTGGGGATCACCATCATGGAAAGAAGCAGCTTCGAGAAGAATCCCATCGAC 3540

hSpCas9

K E L L G I T I M E R S S F E K N P I D

1161 1162 1163 1164 1165 1166 1167 1168 1169 1170 1171 1172 1173 1174 1175 1176 1177 1178 1179 1180

5' TTTCTGGAAGCCAAGGGCTACAAAGAAGTGAAAAAGGACCTGATCATCAAGCTGCCTAAG 3600

hSpCas9

F L E A K G Y K E V K K D L I I K L P K

1181 1182 1183 1184 1185 1186 1187 1188 1189 1190 1191 1192 1193 1194 1195 1196 1197 1198 1199 1200

5' TACTCCCTGTTCGAGCTGGAAAACGGCCGGAAGAGAATGCTGGCCTCTGCCGGCGAACTG 3660

hSpCas9

Y S L F E L E N G R K R M L A S A G E L

1201 1202 1203 1204 1205 1206 1207 1208 1209 1210 1211 1212 1213 1214 1215 1216 1217 1218 1219 1220

5' CAGAAGGGAAACGAACTGGCCCTGCCCTCCAAATATGTGAACTTCCTGTACCTGGCCAGC 3720

hSpCas9

Q K G N E L A L P S K Y V N F L Y L A S

1221 1222 1223 1224 1225 1226 1227 1228 1229 1230 1231 1232 1233 1234 1235 1236 1237 1238 1239 1240

FIG. 21K

hSpCas9

5' CACTATGAGAAGCTGAAGGGCTCCCCCGAGGATAATGAGCAGAAACAGCTGTTTGTGGAA 3780

hSpCas9

H Y E K L K G S P E D N E Q K Q L F V E

1241 1242 1243 1244 1245 1246 1247 1248 1249 1250 1251 1252 1253 1254 1255 1256 1257 1258 1259 1260

5' CAGCACAAGCACTACCTGGACGAGATCATCGAGCAGATCAGCGAGTTCTCCAAGAGAGTG 3840

hSpCas9

Q H K H Y L D E I I E Q I S E F S K R V

1261 1262 1263 1264 1265 1266 1267 1268 1269 1270 1271 1272 1273 1274 1275 1276 1277 1278 1279 1280

5' ATCCTGGCCGACGCTAATCTGGACAAAGTGCTGTCCGCCTACAACAAGCACCGGGATAAG 3900

hSpCas9

I L A D A N L D K V L S A Y N K H R D K

1281 1282 1283 1284 1285 1286 1287 1288 1289 1290 1291 1292 1293 1294 1295 1296 1297 1298 1299 1300

5' CCCATCAGAGAGCAGGCCGAGAATATCATCCACCTGTTTACCCTGACCAATCTGGGAGCC 3960

hSpCas9

P I R E Q A E N I I H L F T L T N L G A

1301 1302 1303 1304 1305 1306 1307 1308 1309 1310 1311 1312 1313 1314 1315 1316 1317 1318 1319 1320

5' CCTGCCGCCTTCAAGTACTTTGACACCACCATCGACCGGAAGAGGTACACCAGCACCAAA 4020

hSpCas9

P A A F K Y F D T T I D R K R Y T S T K

1321 1322 1323 1324 1325 1326 1327 1328 1329 1330 1331 1332 1333 1334 1335 1336 1337 1338 1339 1340

5' GAGGTGCTGGACGCCACCCTGATCCACCAGAGCATCACCGGCCTGTACGAGACACGGATC 4080

hSpCas9

E V L D A T L I H Q S I T G L Y E T R I

1341 1342 1343 1344 1345 1346 1347 1348 1349 1350 1351 1352 1353 1354 1355 1356 1357 1358 1359 1360

FIG. 21L

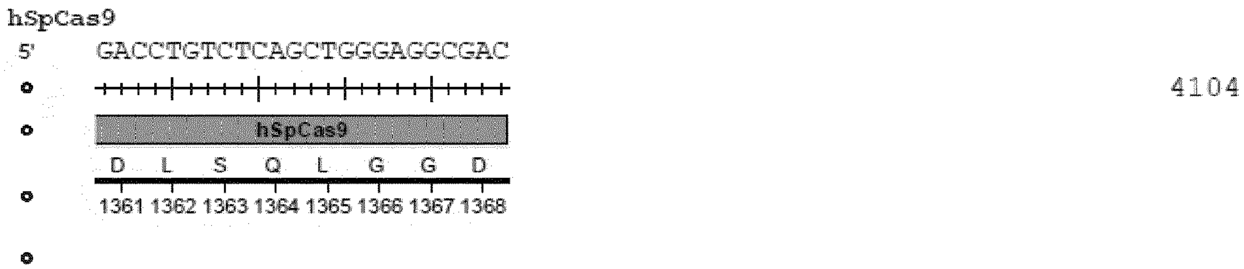
hSpCas9
5'
GACCTGTCTCAGCTGGGAGGCGAC
4104
hSpCas9
D L S Q L G G D
1361 1362 1363 1364 1365 1366 1367 1368

FIG. 21M

Conditional Cas9, Rosa26 targeting vector map

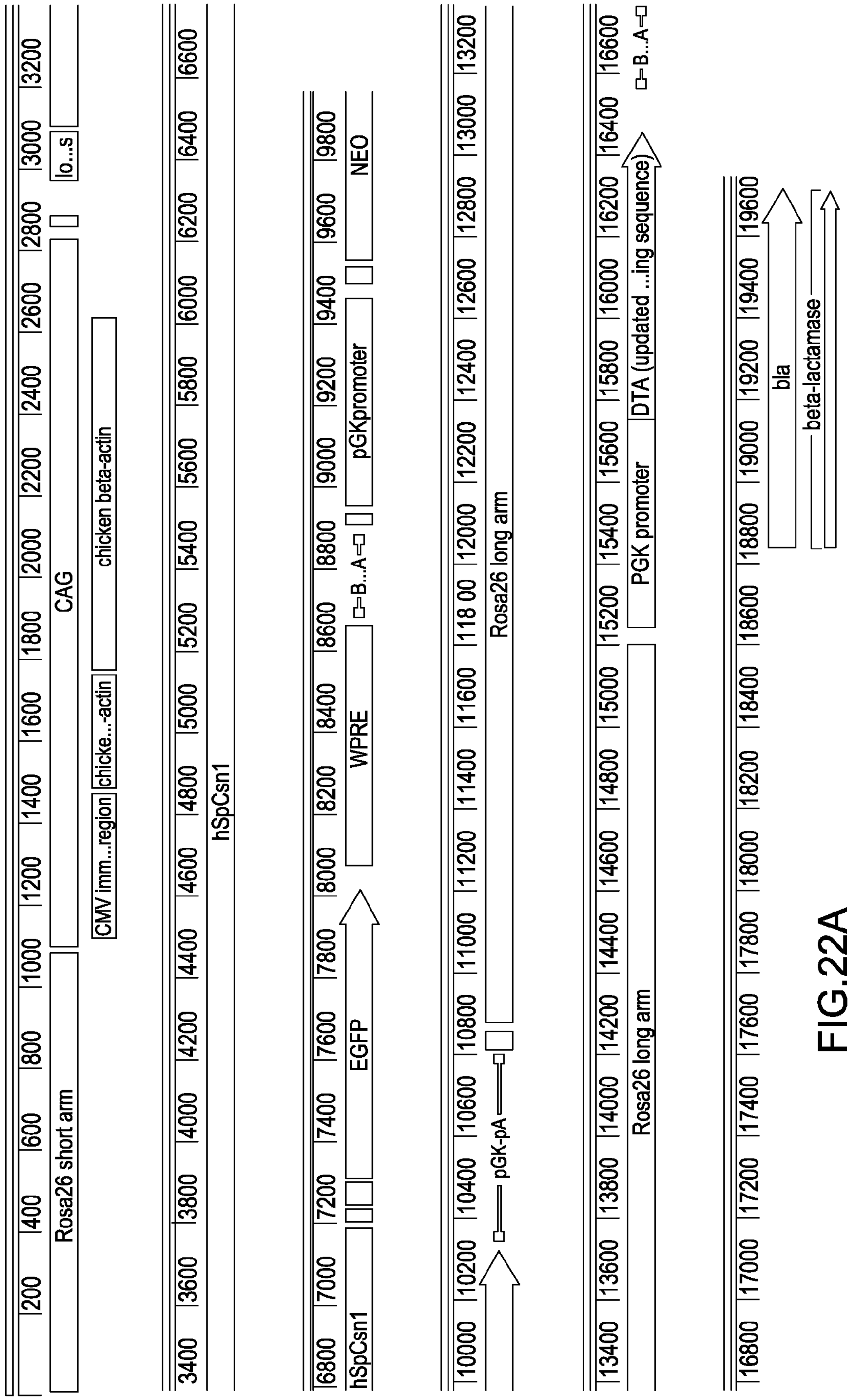


FIG.22A

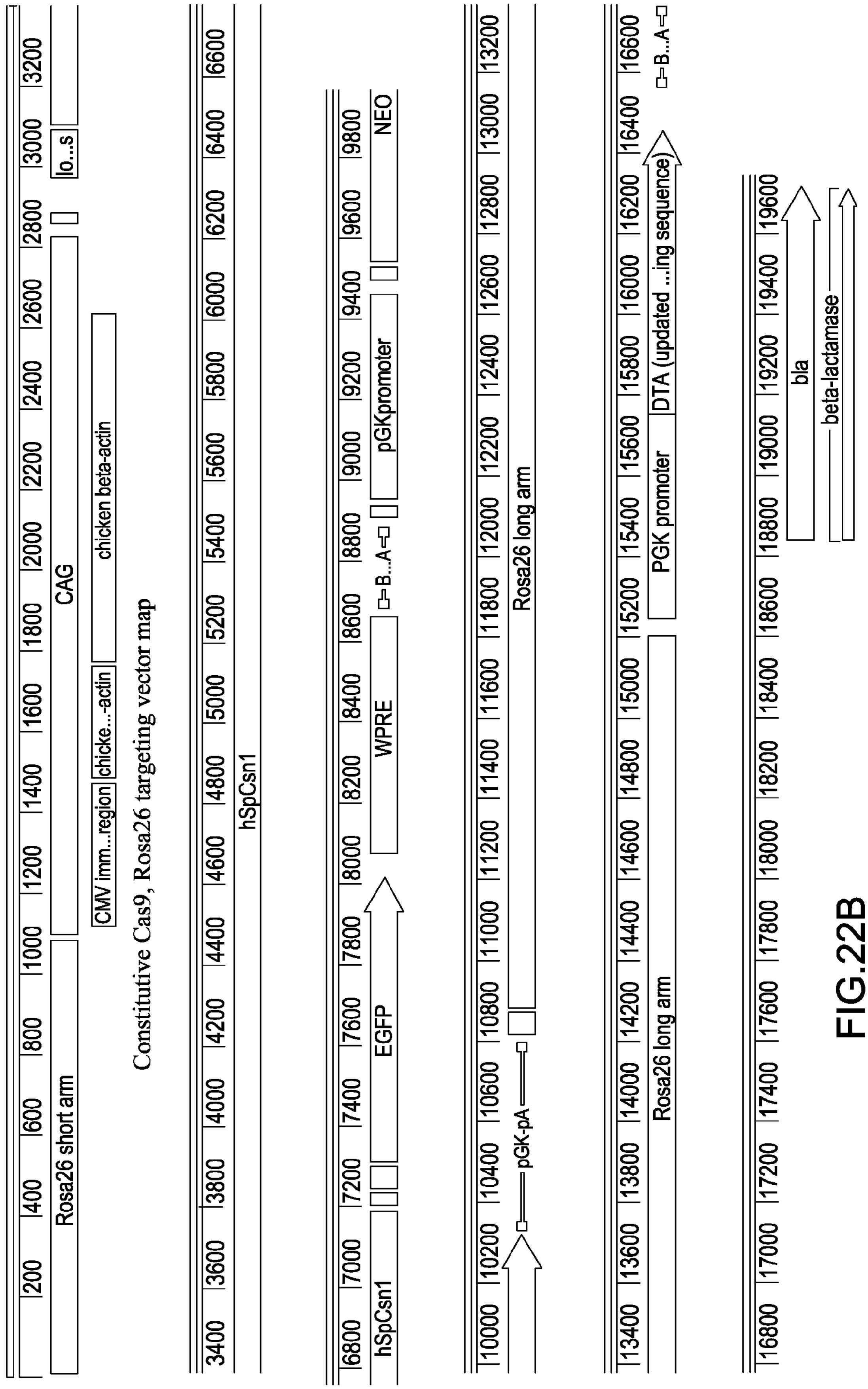
Constitutive Cas9, Rosa26 targeting vector map
Rosa26 short arm
CAG
lo...s
CMV imm...region
chicke...-actin
chicken beta-actin
hSpCsn1
EGFP
WPRE
B...A
pGKpromoter
NEO
pGK-pA
Rosa26 long arm
PGK promoter
DTA (updated ...ing sequence)
bla
beta-lactamase

FIG.22B

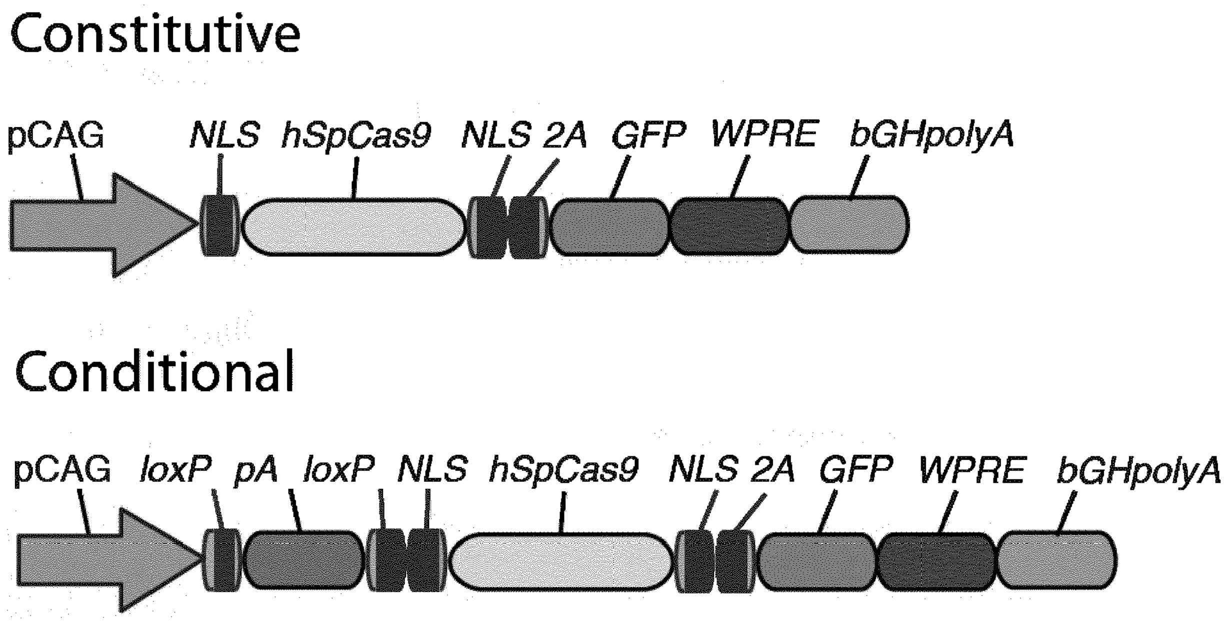
Constitutive
pCAG
NLS
hSpCas9
NLS
2A
GFP
WPRE
bGHpolyA
Conditional
pCAG
loxP
pA
loxP
NLS
hSpCas9
NLS
2A
GFP
WPRE
bGHpolyA

FIG. 23

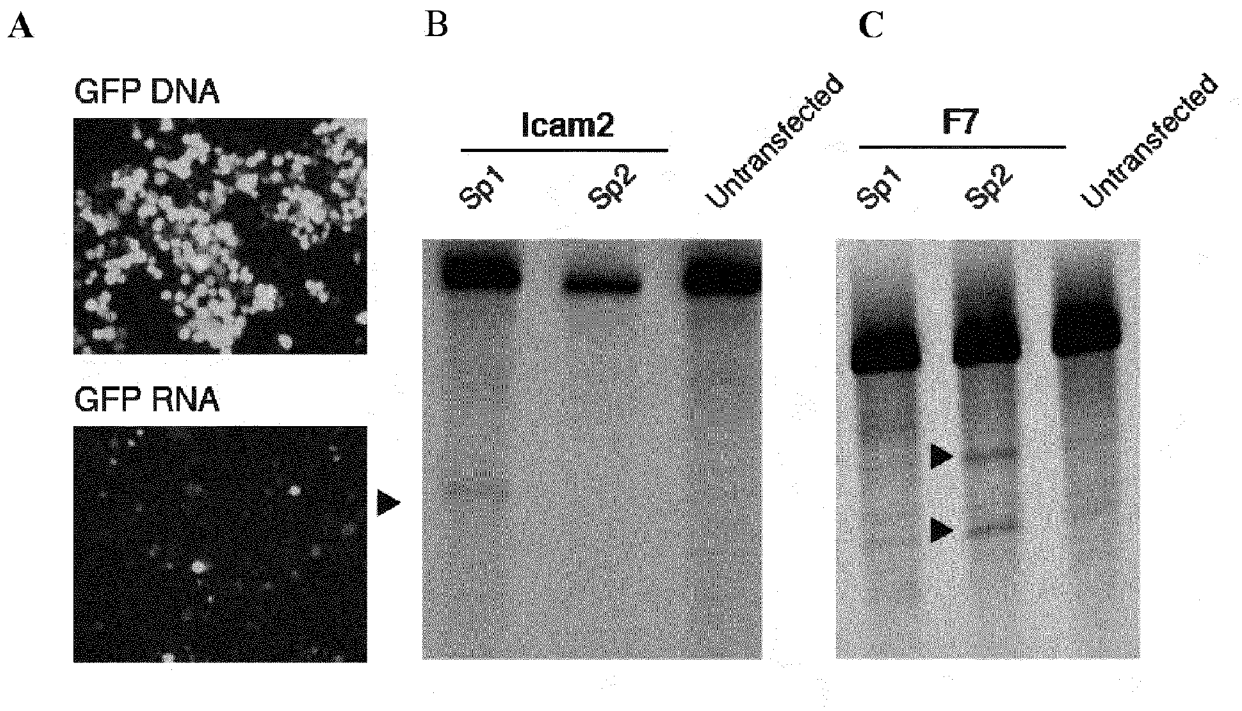
A
B
C
GFP DNA
GFP RNA
Icam2
Sp1
Sp2
Untransfected
F7
Sp1
Sp2
Untransfected

FIG. 24A-C

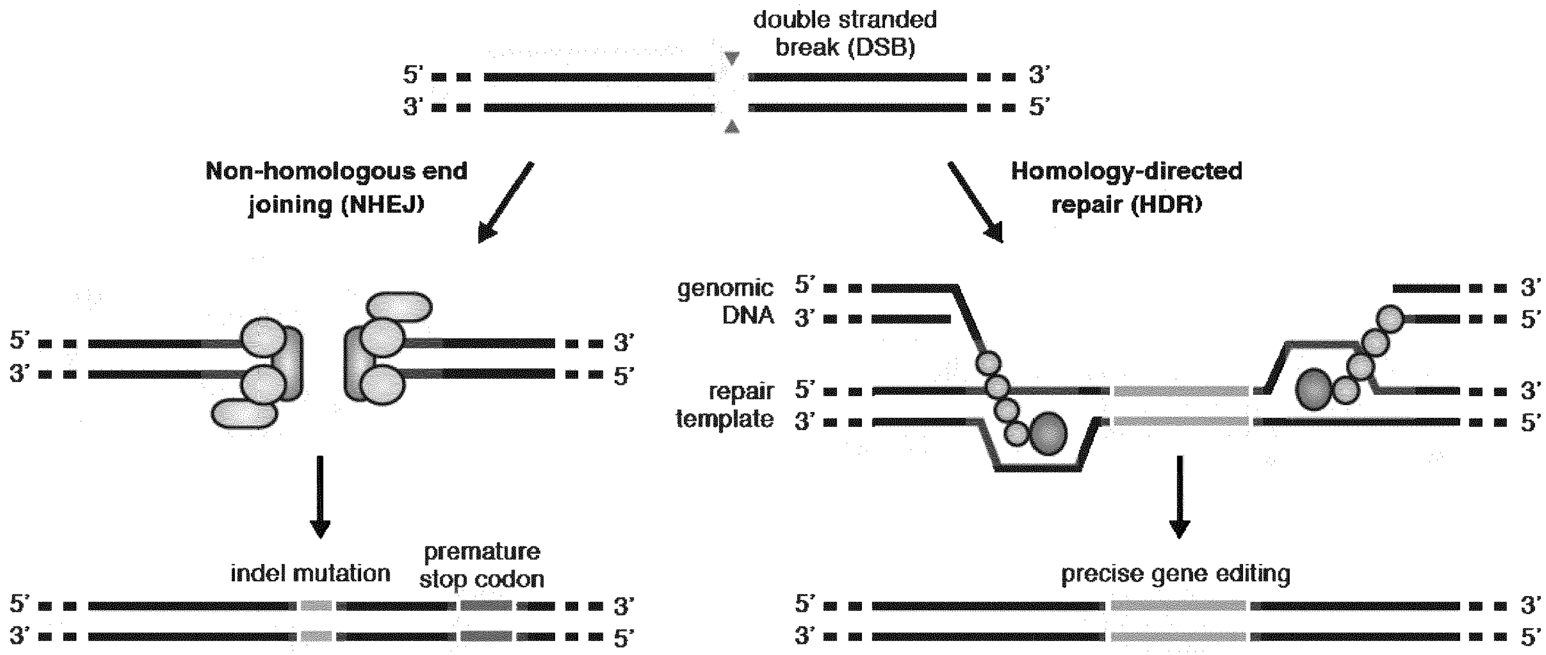
double stranded
break (DSB)
5'
3'
3'
5'
Non-homologous end
joining (NHEJ)
Homology-directed
repair (HDR)
genomic
DNA
repair
template
indel mutation
premature
stop codon
precise gene editing

FIG. 25

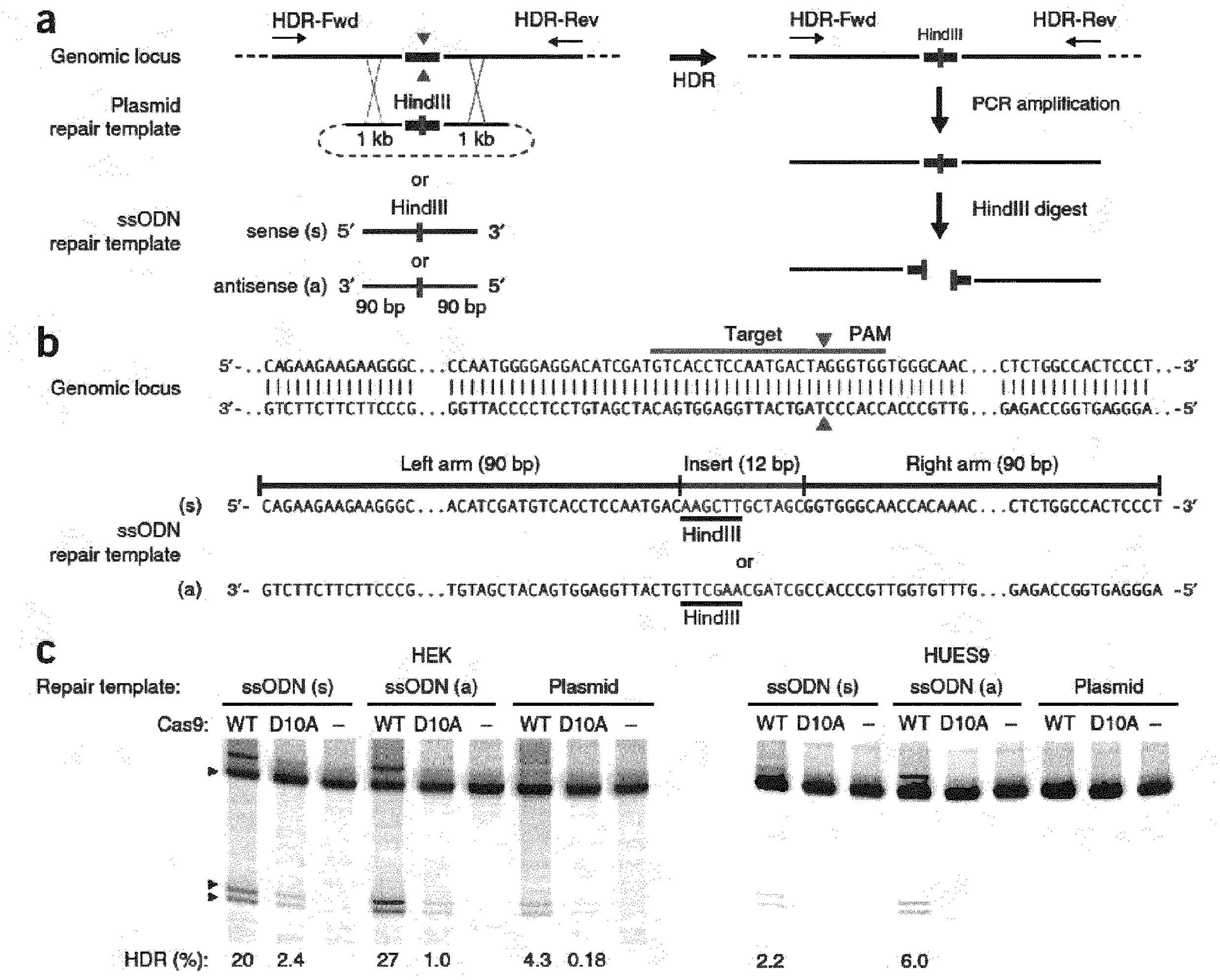
a
HDR-Fwd
HDR-Rev
Genomic locus
Plasmid repair template
HindIII
1 kb
1 kb
or
ssODN repair template
sense (s) 5′
3′
antisense (a) 3′
5′
90 bp
90 bp
HDR
HindIII
PCR amplification
HindIII digest
b
Target
PAM
Genomic locus
5′-..CAGAAGAAGAAGGGC...CCAATGGGGAGGACATCGATGTCACCTCCAATGACTAGGGTGGTGGGCAAC...CTCTGGCCACTCCCT..-3′
3′-..GTCTTCTTCTTCCCG...GGTTACCCCTCCTGTAGCTACAGTGGAGGTTACTGATCCCACCACCCGTTG...GAGACCGGTGAGGGA..-5′
Left arm (90 bp)
Insert (12 bp)
Right arm (90 bp)
(s) 5′- CAGAAGAAGAAGGGC...ACATCGATGTCACCTCCAATGACAAGCTTGCTAGCGGTGGGCAACCACAAAC...CTCTGGCCACTCCCT -3′
ssODN repair template
HindIII
or
(a) 3′- GTCTTCTTCTTCCCG...TGTAGCTACAGTGGAGGTTACTGTTCGAACGATCGCCACCCGTTGGTGTTTG...GAGACCGGTGAGGGA -5′
HindIII
c
HEK
HUES9
Repair template:
ssODN (s)
ssODN (a)
Plasmid
Cas9:
WT D10A –
HDR (%):
20 2.4
27 1.0
4.3 0.18
2.2
6.0

FIG. 26A-C

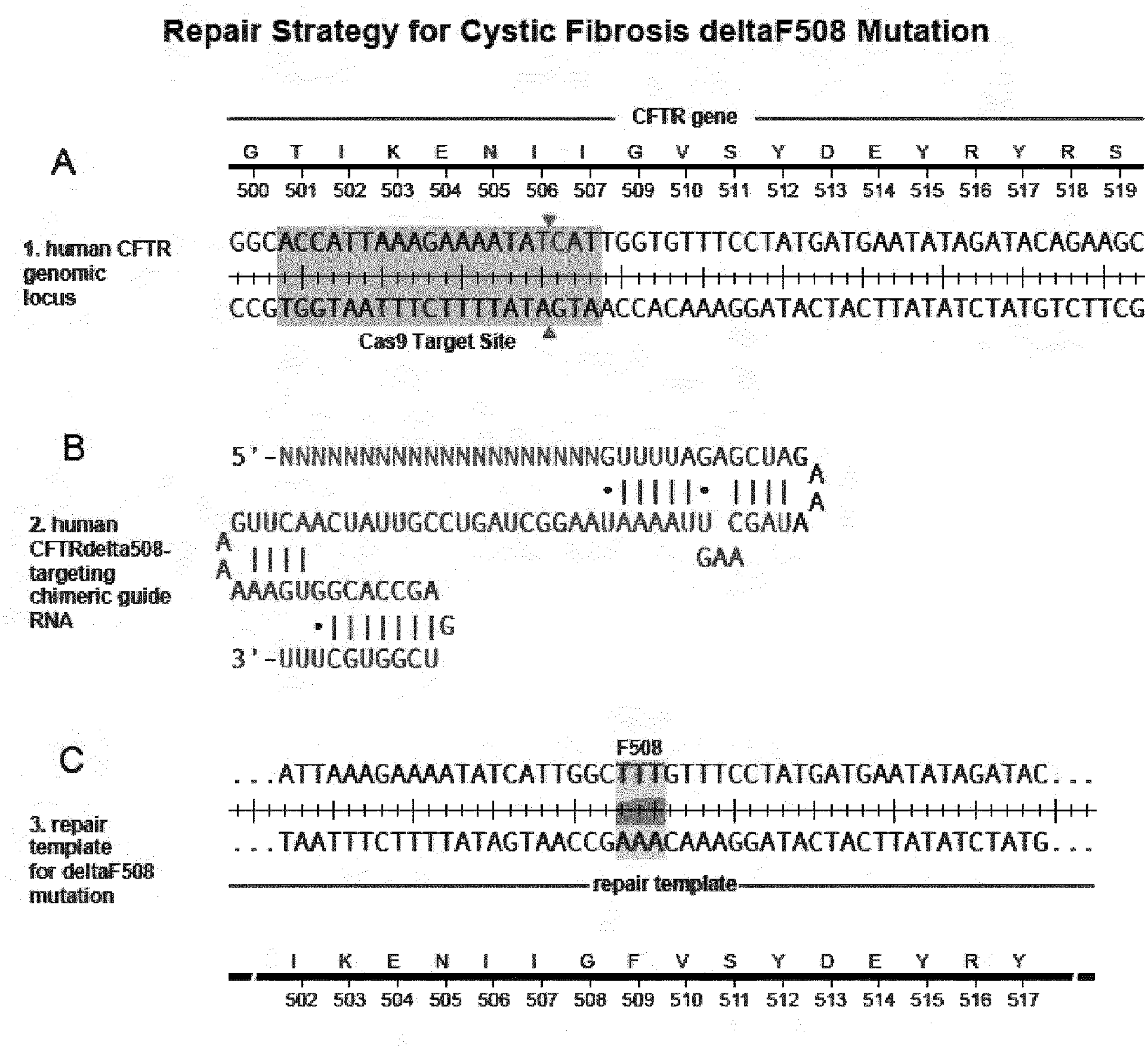
Repair Strategy for Cystic Fibrosis deltaF508 Mutation
A
CFTR gene
G T I K E N I I G V S Y D E Y R Y R S
500 501 502 503 504 505 506 507 509 510 511 512 513 514 515 516 517 518 519
1. human CFTR genomic locus
GGCACCATTAAAGAAAATATCATTGGTGTTTCCTATGATGAATATAGATACAGAAGC
CCGTGGTAATTTCTTTTATAGTAACCACAAAGGATACTACTTATATCTATGTCTTCG
Cas9 Target Site
B
2. human CFTRdelta508-targeting chimeric guide RNA
5'-NNNNNNNNNNNNNNNNNNNNGUUUUAGAGCUAG
GUUCAACUAUUGCCUGAUCGGAAUAAAAUU CGAUA
GAA
AAAGUGGCACCGA
3'-UUUCGUGGCU
C
3. repair template for deltaF508 mutation
F508
...ATTAAAGAAAATATCATTGGCTTTGTTTCCTATGATGAATATAGATAC...
...TAATTTCTTTTATAGTAACCGAAACAAAGGATACTACTTATATCTATG...
repair template
I K E N I I G F V S Y D E Y R Y
502 503 504 505 506 507 508 509 510 511 512 513 514 515 516 517

FIG. 27A-C

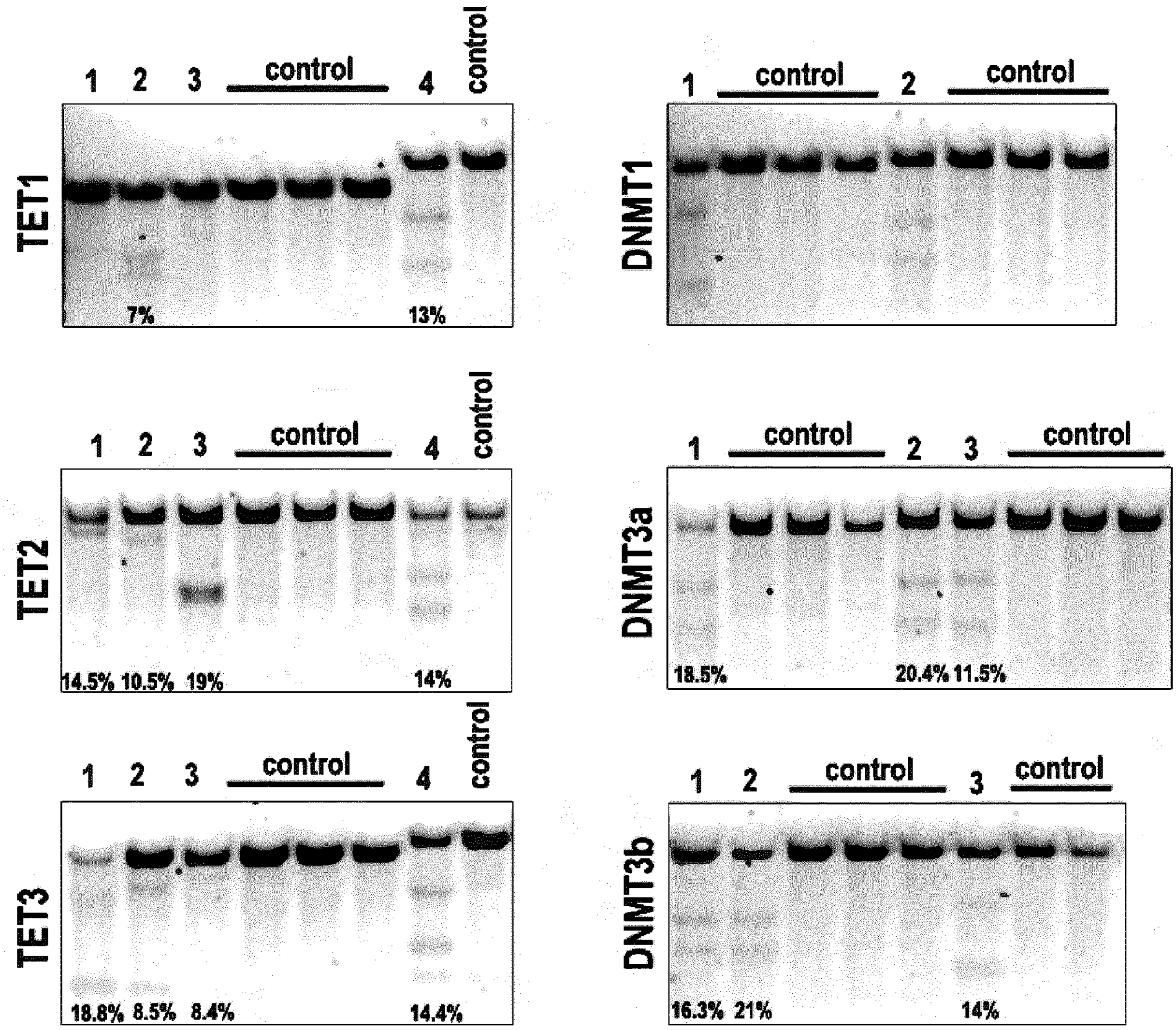
1 2 3 control 4 control
TET1
7% 13%
1 control 2 control
DNMT1
1 2 3 control 4 control
TET2
14.5% 10.5% 19% 14%
1 control 2 3 control
DNMT3a
18.5% 20.4% 11.5%
1 2 3 control 4 control
TET3
18.8% 8.5% 8.4% 14.4%
1 2 control 3 control
DNMT3b
16.3% 21% 14%

FIG. 28

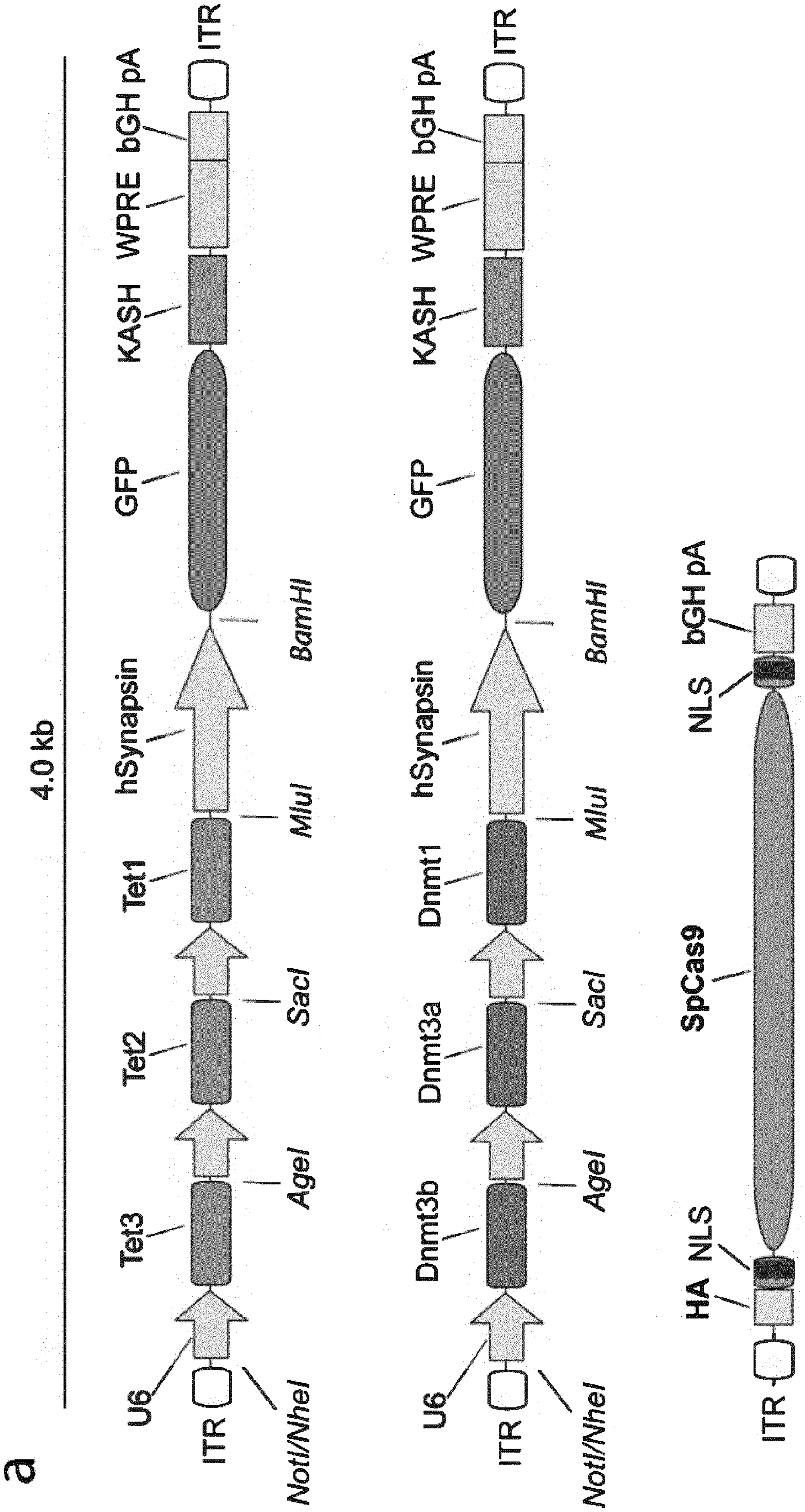
a
4.0 kb
ITR
U6
NotI/NheI
Tet3
AgeI
Tet2
SacI
Tet1
MluI
hSynapsin
BamHI
GFP
KASH
WPRE
bGH pA
ITR
ITR
U6
NotI/NheI
Dnmt3b
AgeI
Dnmt3a
SacI
Dnmt1
MluI
hSynapsin
BamHI
GFP
KASH
WPRE
bGH pA
ITR
ITR
HA
NLS
SpCas9
NLS
bGH pA

FIG. 29

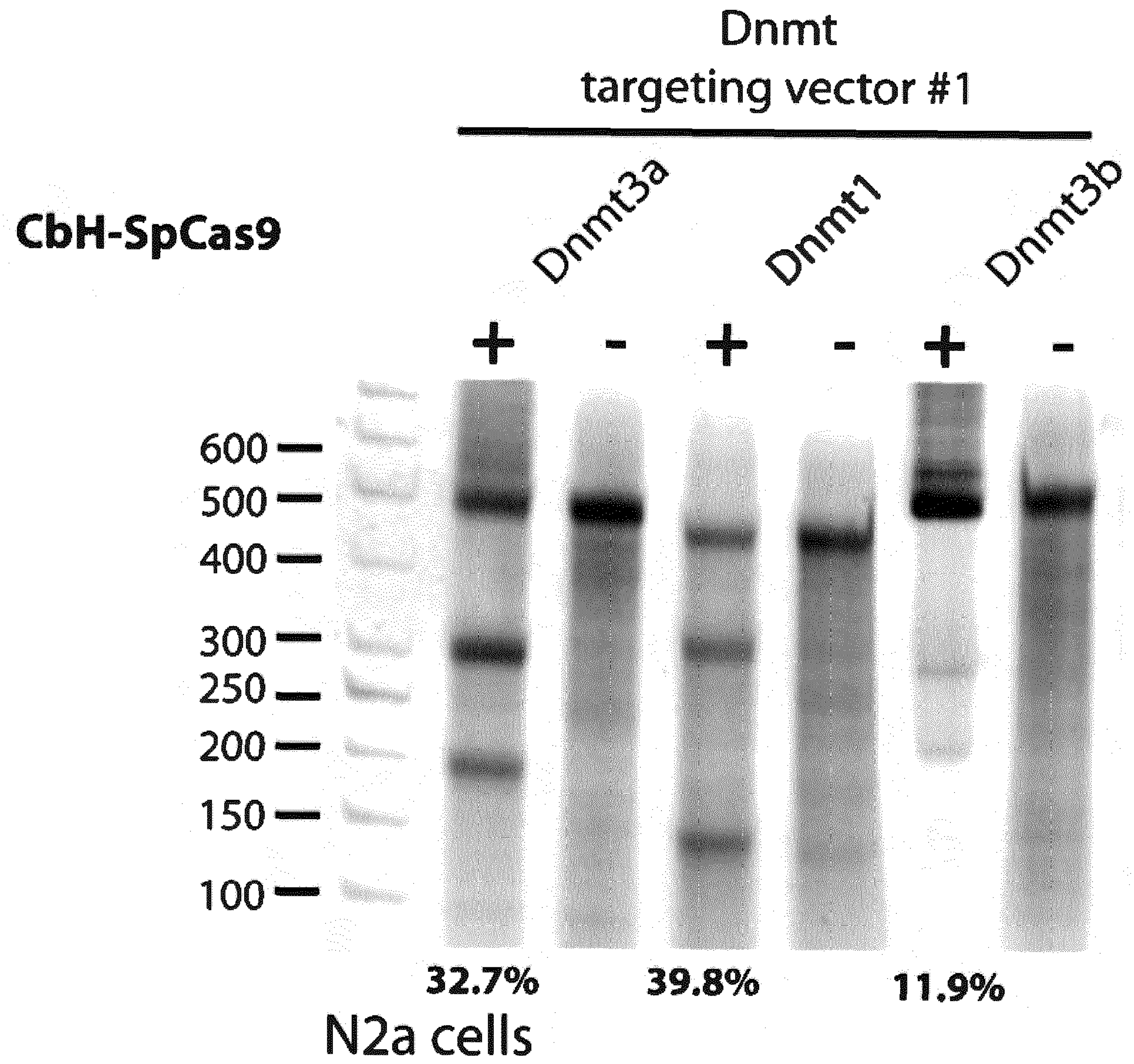
Dnmt
targeting vector #1
CbH-SpCas9
Dnmt3a
Dnmt1
Dnmt3b
+
-
+
-
+
-
600
500
400
300
250
200
150
100
32.7%
39.8%
11.9%
N2a cells

FIG. 30

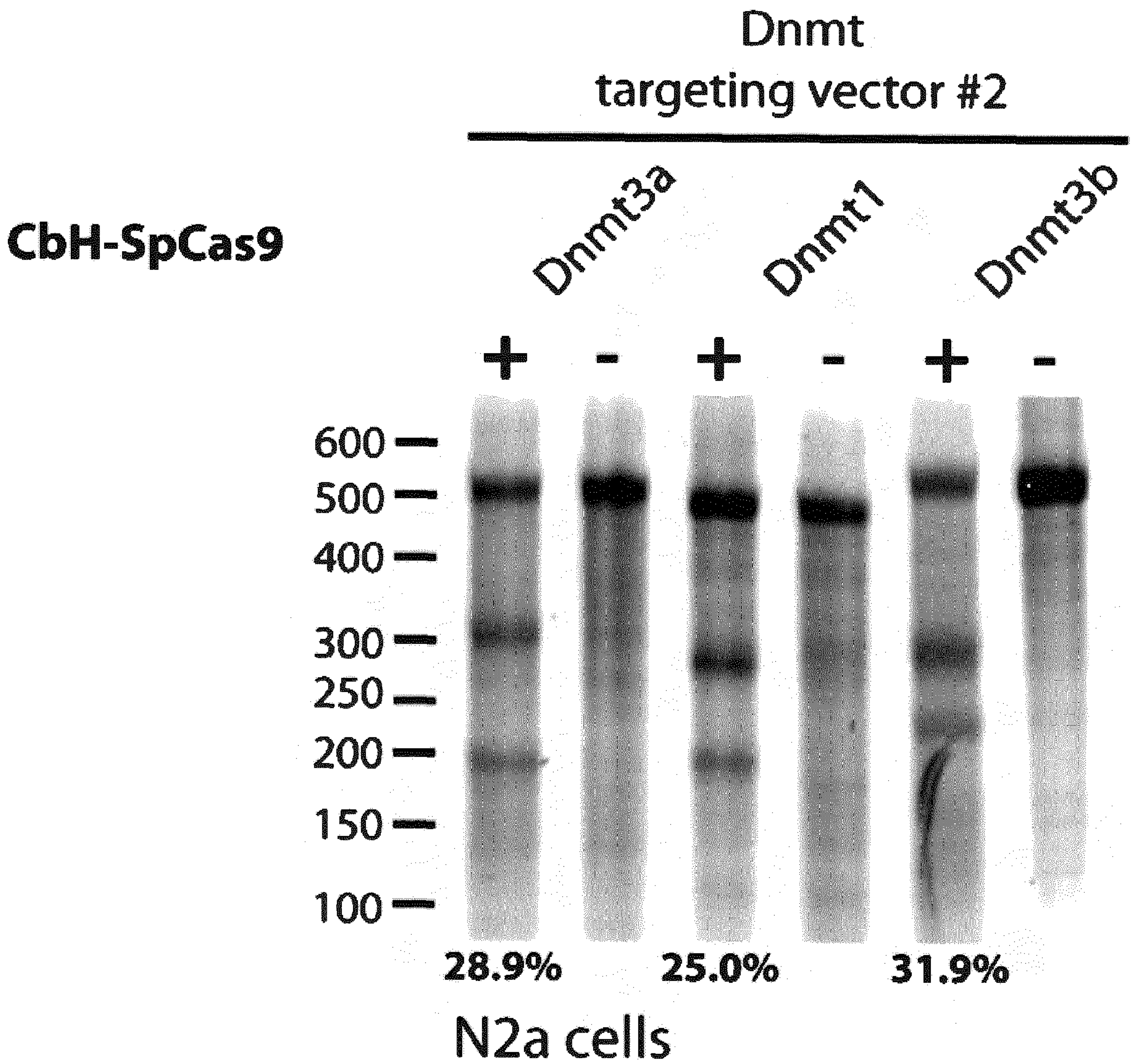
Dnmt
targeting vector #2
CbH-SpCas9
Dnmt3a
Dnmt1
Dnmt3b
+
-
+
-
+
-
600
500
400
300
250
200
150
100
28.9%
25.0%
31.9%
N2a cells

FIG. 31

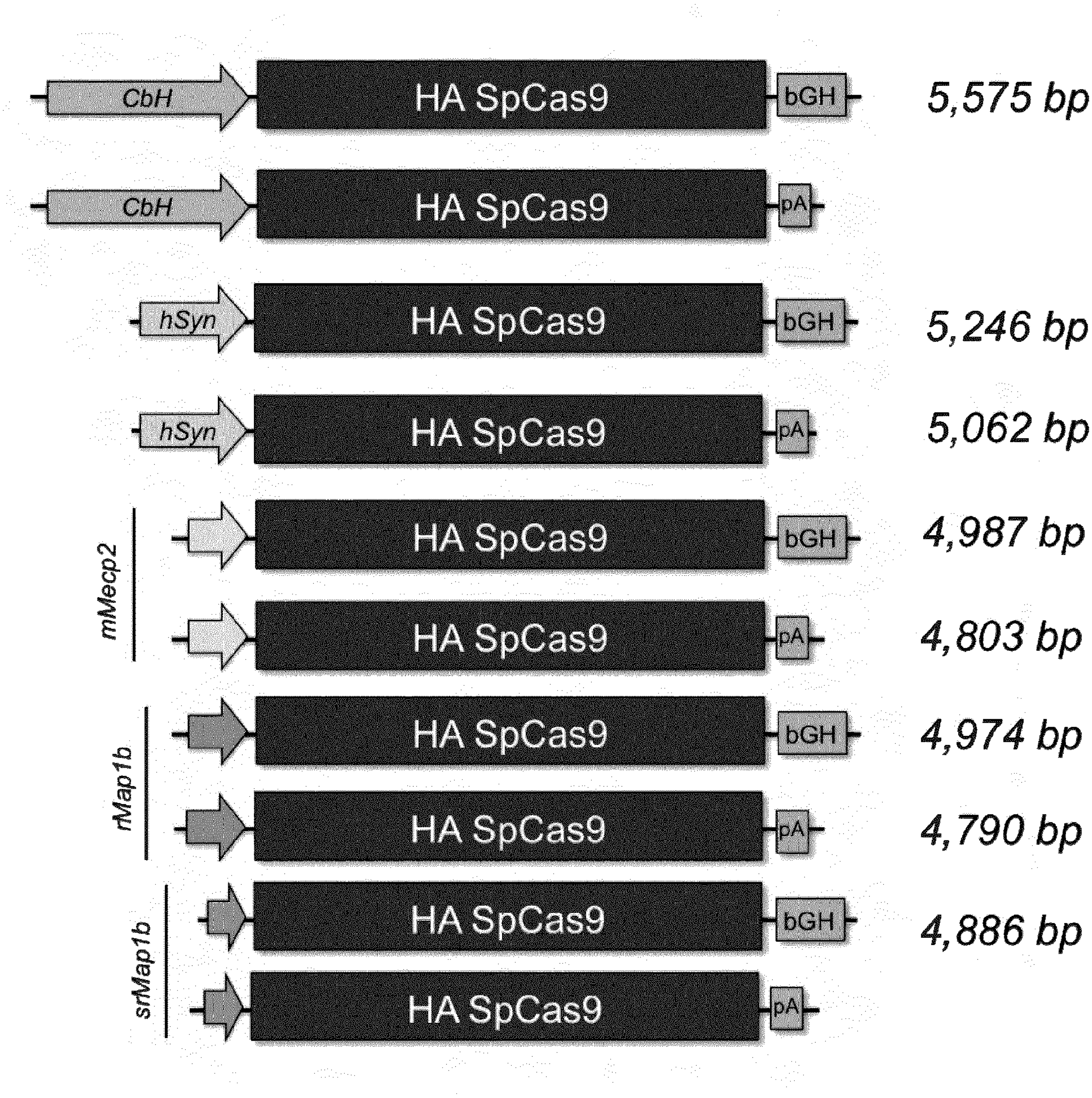
CbH
HA SpCas9
bGH
5,575 bp
CbH
HA SpCas9
pA
hSyn
HA SpCas9
bGH
5,246 bp
hSyn
HA SpCas9
pA
5,062 bp
mMecp2
HA SpCas9
bGH
4,987 bp
HA SpCas9
pA
4,803 bp
rMap1b
HA SpCas9
bGH
4,974 bp
HA SpCas9
pA
4,790 bp
srMap1b
HA SpCas9
bGH
4,886 bp
HA SpCas9
pA

FIG. 32

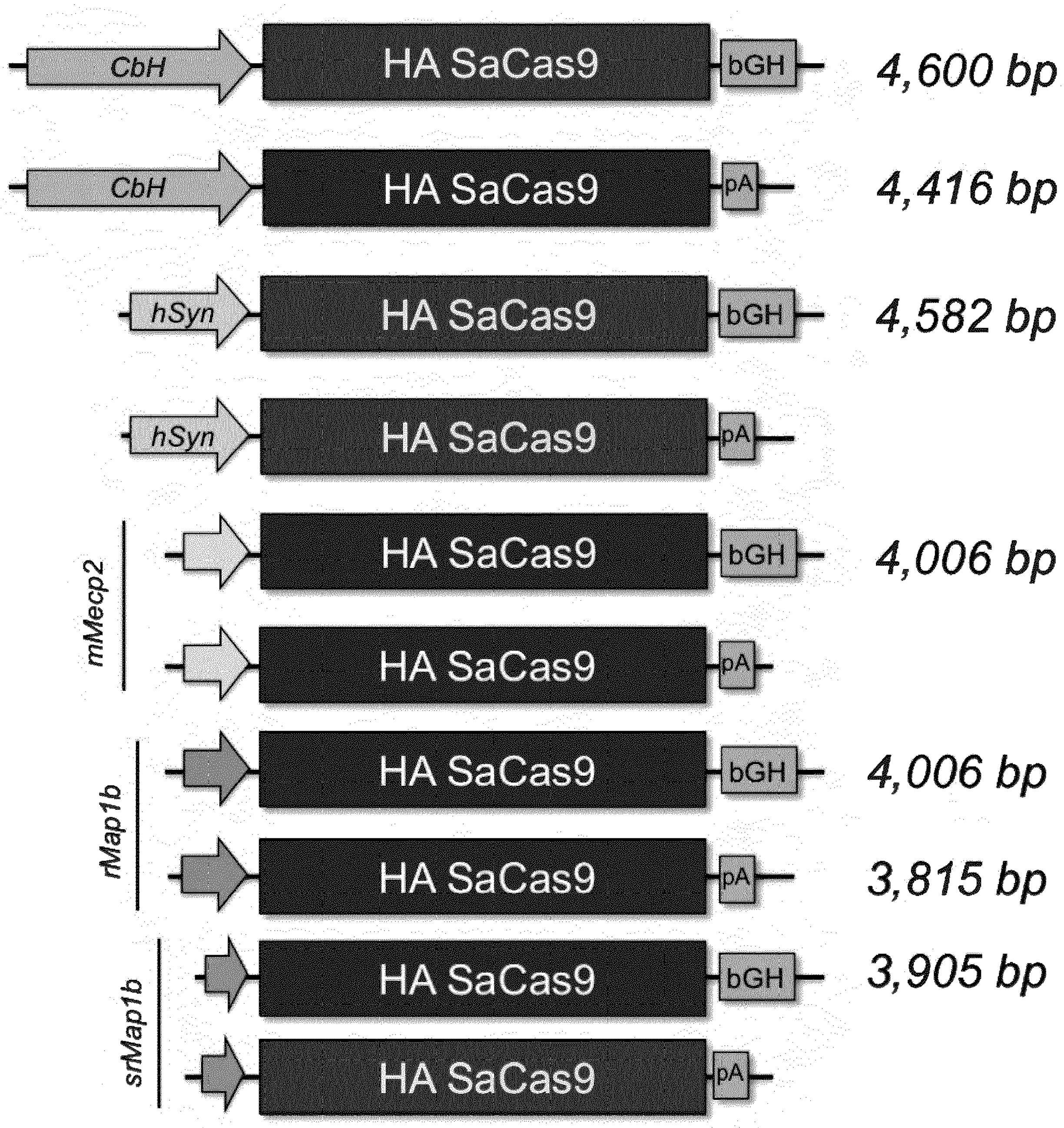
CbH
HA SaCas9
bGH
4,600 bp
CbH
HA SaCas9
pA
4,416 bp
hSyn
HA SaCas9
bGH
4,582 bp
hSyn
HA SaCas9
pA
mMecp2
HA SaCas9
bGH
4,006 bp
HA SaCas9
pA
rMap1b
HA SaCas9
bGH
4,006 bp
HA SaCas9
pA
3,815 bp
srMap1b
HA SaCas9
bGH
3,905 bp
HA SaCas9
pA

FIG. 33

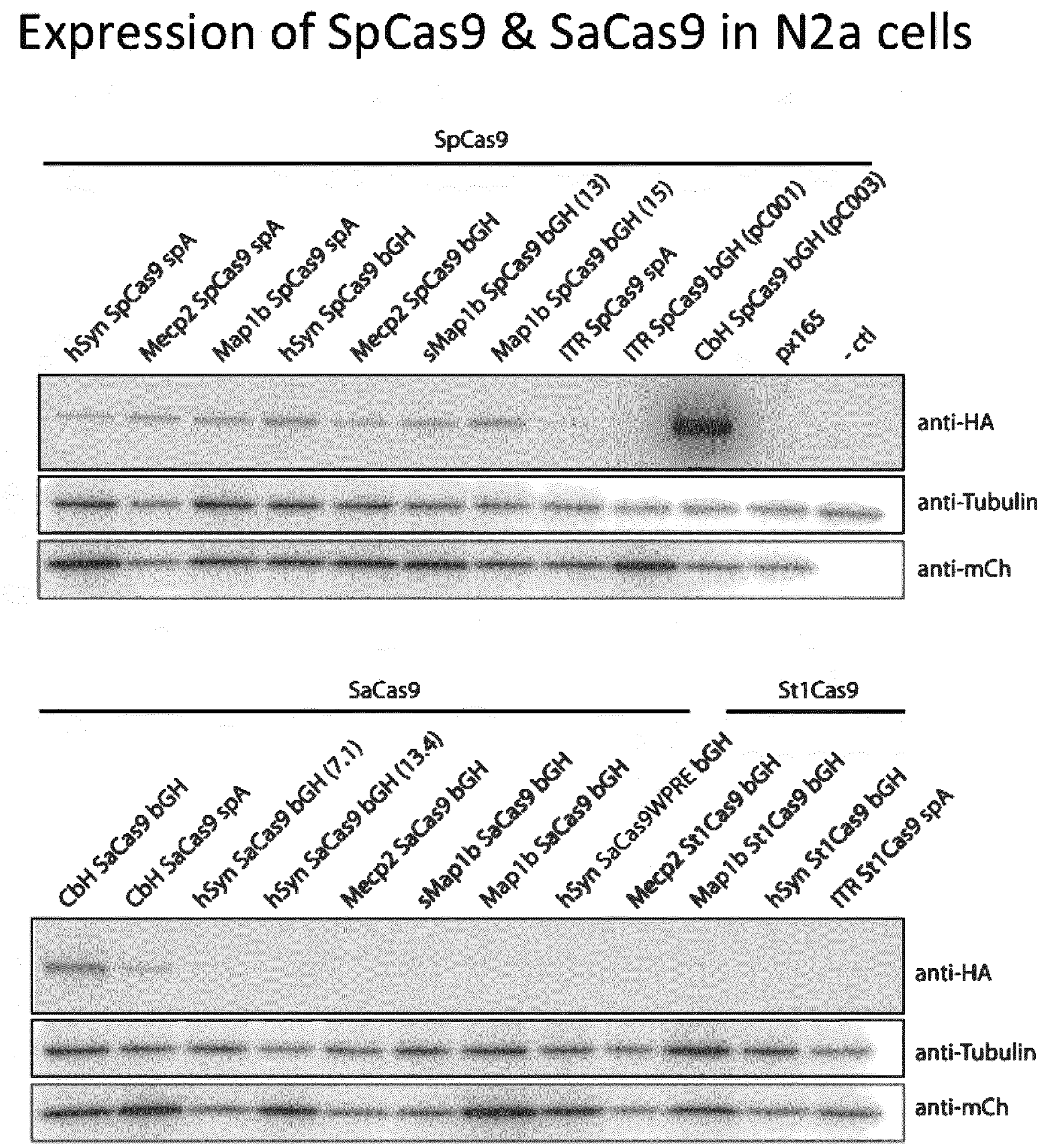
Expression of SpCas9 & SaCas9 in N2a cells
SpCas9
hSyn SpCas9 spA
Mecp2 SpCas9 spA
Map1b SpCas9 spA
hSyn SpCas9 bGH
Mecp2 SpCas9 bGH
sMap1b SpCas9 bGH (13)
Map1b SpCas9 bGH (15)
ITR SpCas9 spA
ITR SpCas9 bGH (pC001)
CbH SpCas9 bGH (pC003)
px165
- ctl
anti-HA
anti-Tubulin
anti-mCh
SaCas9
St1Cas9
CbH SaCas9 bGH
CbH SaCas9 spA
hSyn SaCas9 bGH (7.1)
hSyn SaCas9 bGH (13.4)
Mecp2 SaCas9 bGH
sMap1b SaCas9 bGH
Map1b SaCas9 bGH
hSyn SaCas9WPRE bGH
Mecp2 St1Cas9 bGH
Map1b St1Cas9 bGH
hSyn St1Cas9 bGH
ITR St1Cas9 spA
anti-HA
anti-Tubulin
anti-mCh

FIG. 34

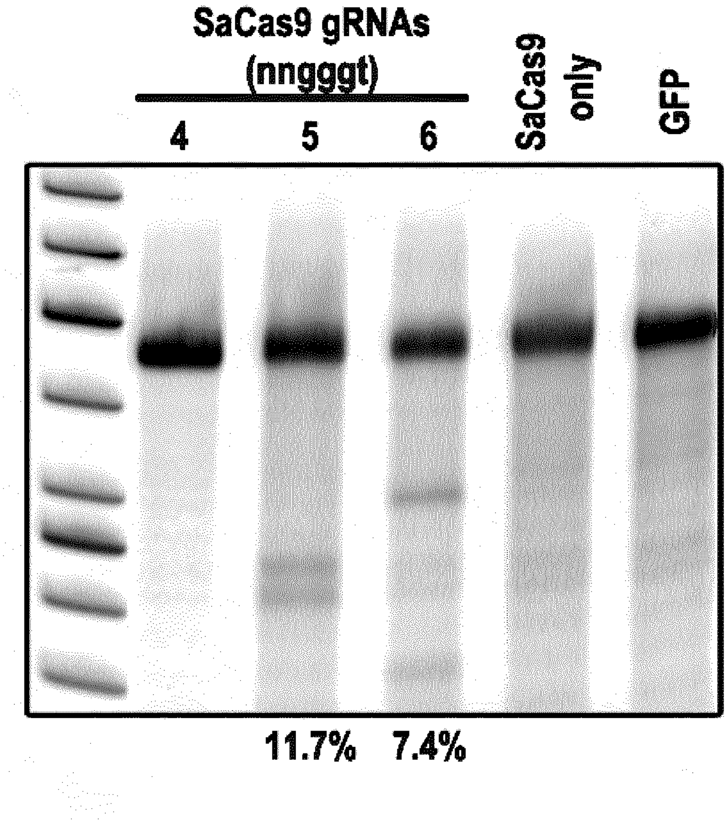


FIG. 35

CA1region of hippocampus

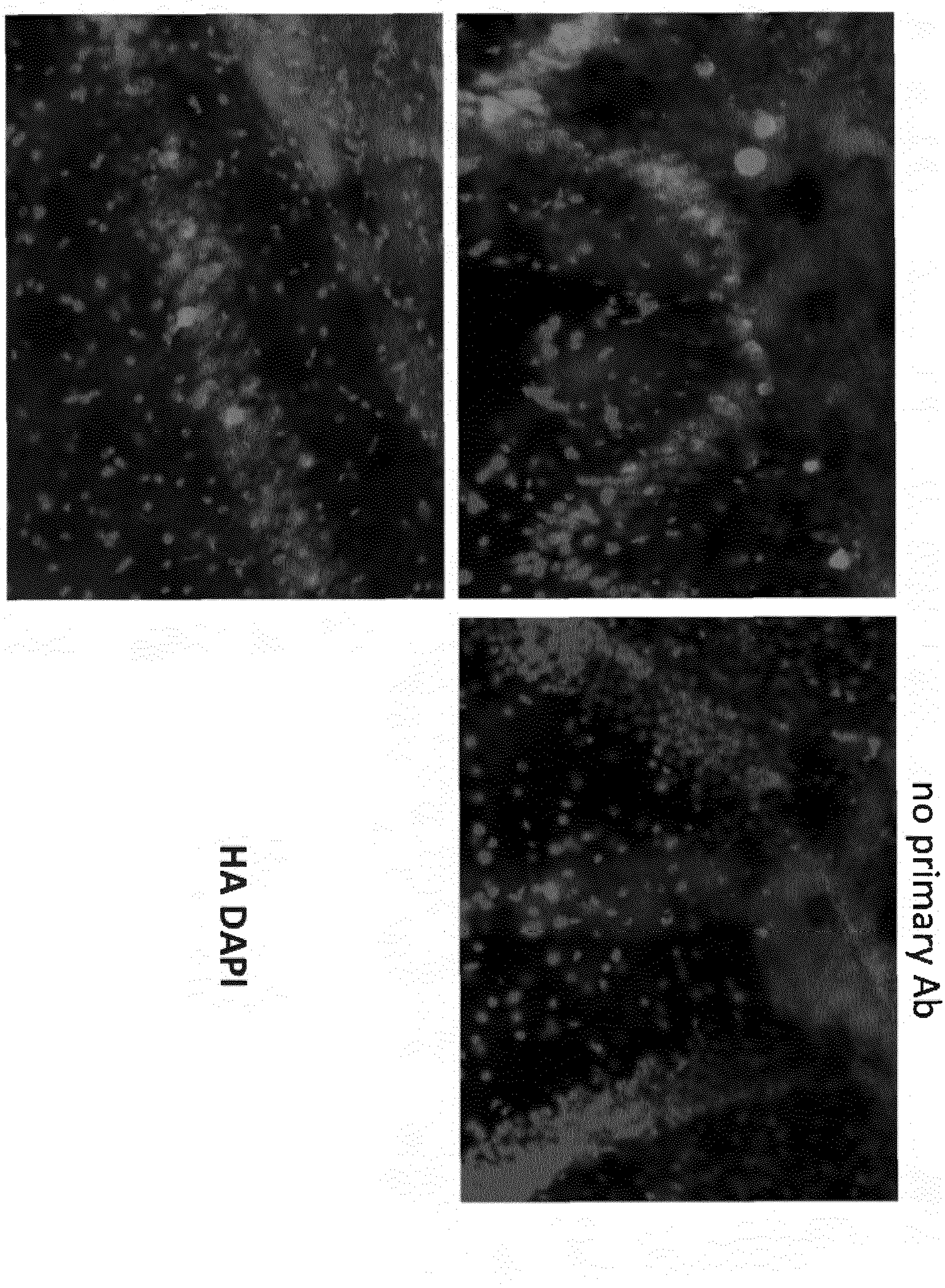


FIG. 36

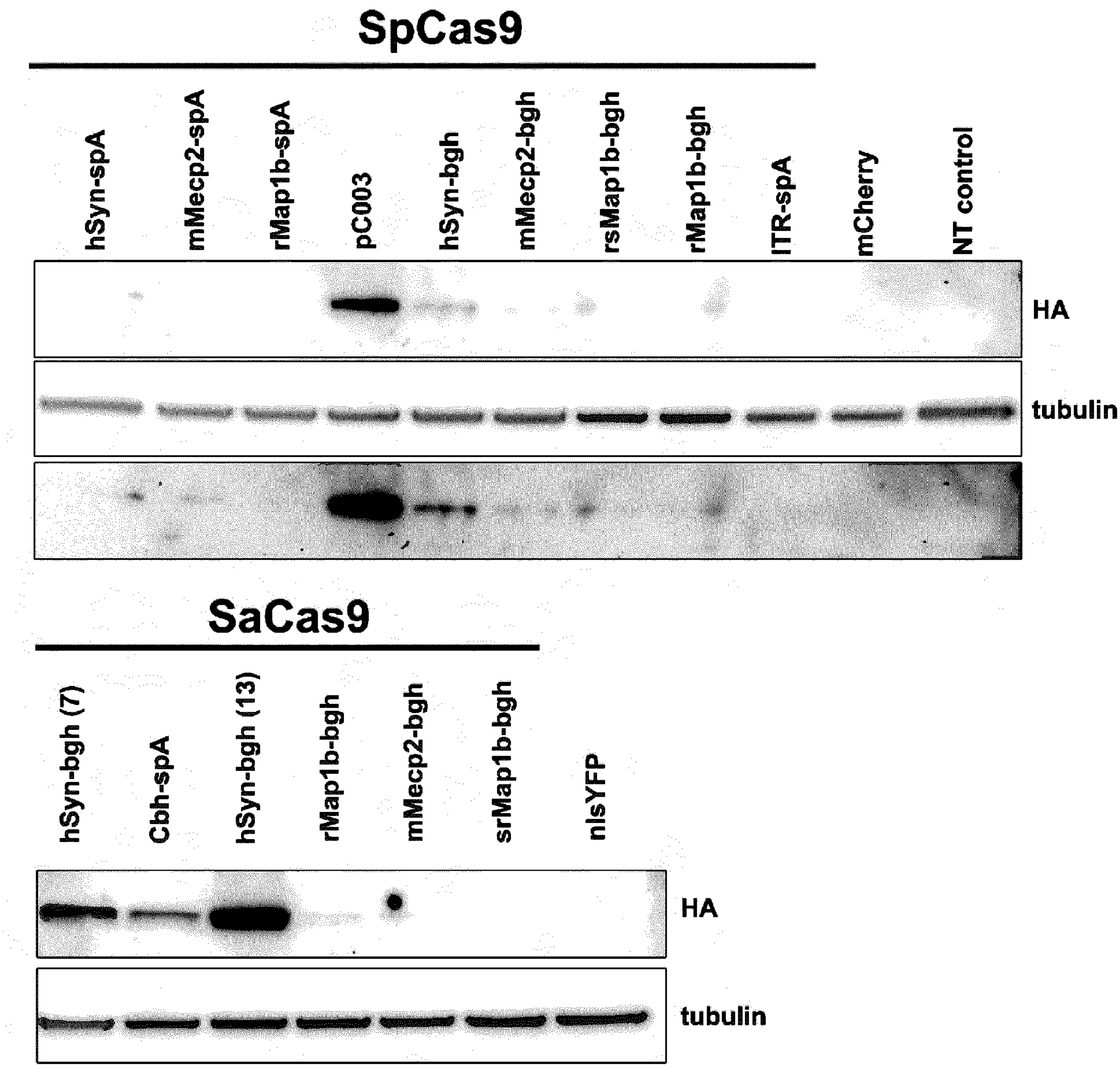
SpCas9
hSyn-spA
mMecp2-spA
rMap1b-spA
pC003
hSyn-bgh
mMecp2-bgh
rsMap1b-bgh
rMap1b-bgh
ITR-spA
mCherry
NT control
HA
tubulin
SaCas9
hSyn-bgh (7)
Cbh-spA
hSyn-bgh (13)
rMap1b-bgh
mMecp2-bgh
srMap1b-bgh
nlsYFP
HA
tubulin

FIG. 37

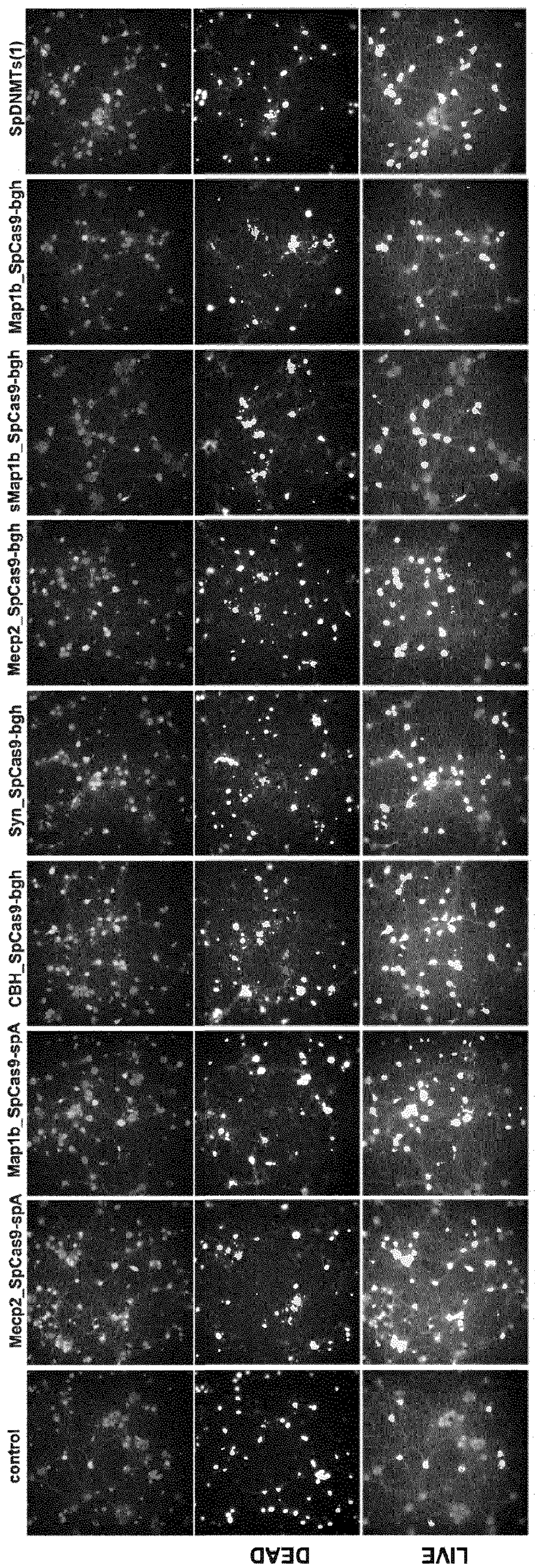
control
Mecp2_SpCas9-spA
Map1b_SpCas9-spA
CBH_SpCas9-bgh
Syn_SpCas9-bgh
Mecp2_SpCas9-bgh
sMap1b_SpCas9-bgh
Map1b_SpCas9-bgh
SpDNMTs(1)
DEAD
LIVE

FIG. 38

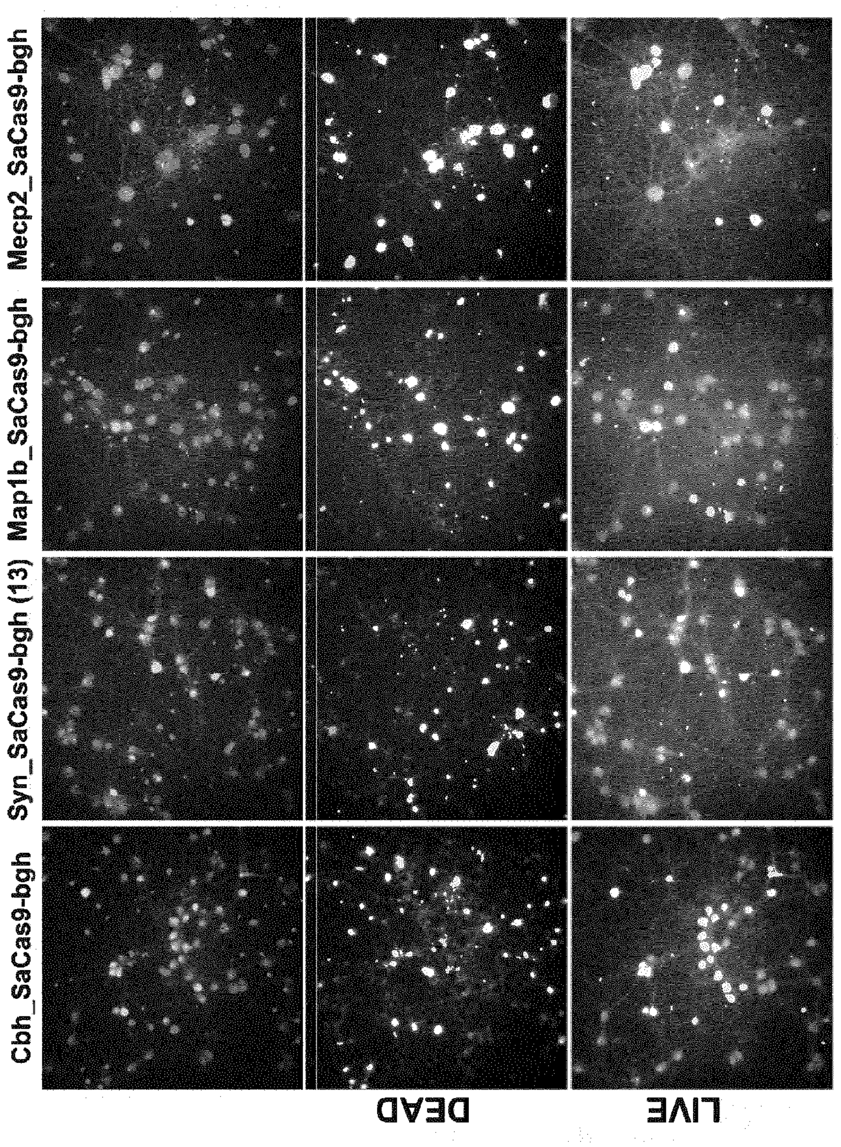

Mecp2_SaCas9-bgh
Map1b_SaCas9-bgh
Syn_SaCas9-bgh (13)
Cbh_SaCas9-bgh
DEAD
LIVE


FIG. 39

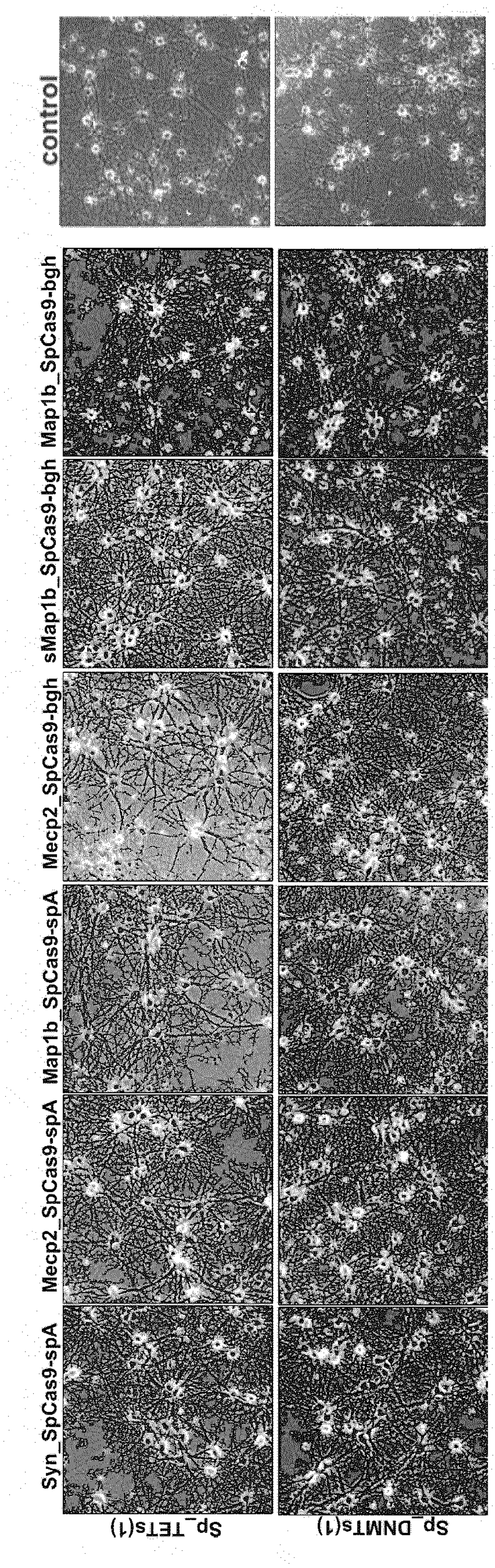
control
Map1b_SpCas9-bgh
sMap1b_SpCas9-bgh
Mecp2_SpCas9-bgh
Map1b_SpCas9-spA
Mecp2_SpCas9-spA
Syn_SpCas9-spA
Sp_TETs(1)
Sp_DNMTs(1)

FIG. 40

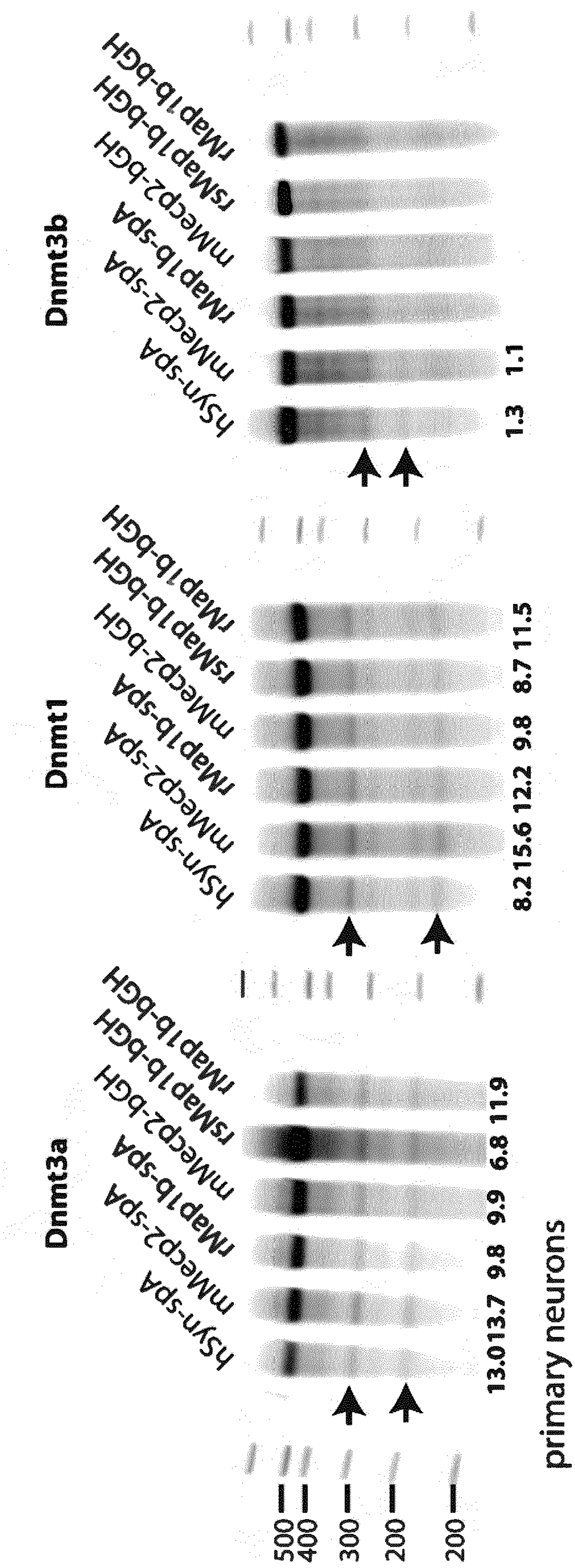
Dnmt3a
Dnmt1
Dnmt3b
hSyn-spA
mMecp2-spA
rMap1b-spA
mMecp2-bGH
rsMap1b-bGH
rMap1b-bGH
13.0
13.7
9.8
9.9
6.8
11.9
8.2
15.6
12.2
9.8
8.7
11.5
1.3
1.1
primary neurons
500
400
300
200
200

FIG. 41

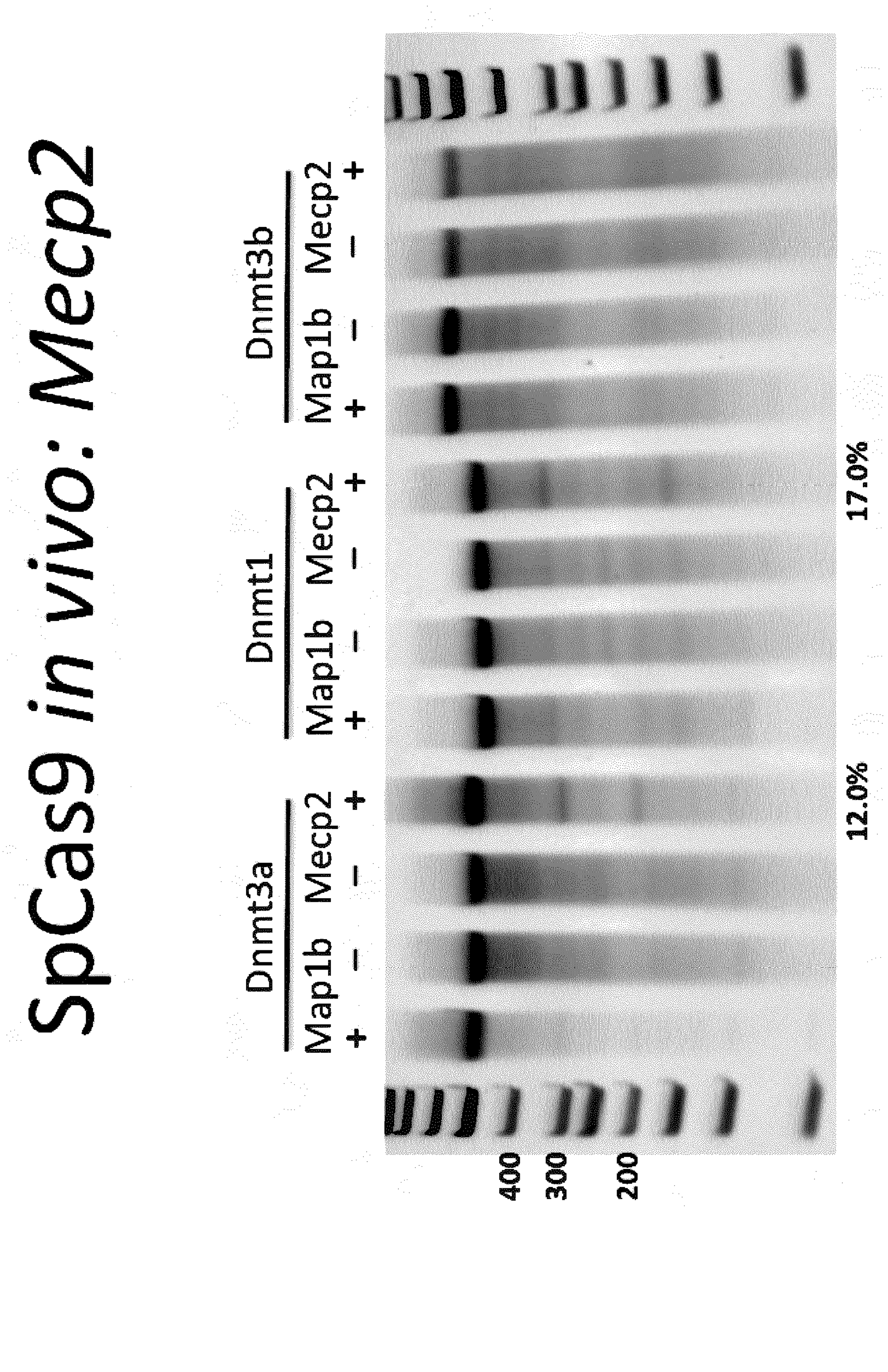
SpCas9 in vivo: Mecp2
Dnmt3a
Dnmt1
Dnmt3b
Map1b
Mecp2
+
–
12.0%
17.0%
400
300
200

FIG. 42

# Purification of cell nuclei from brain

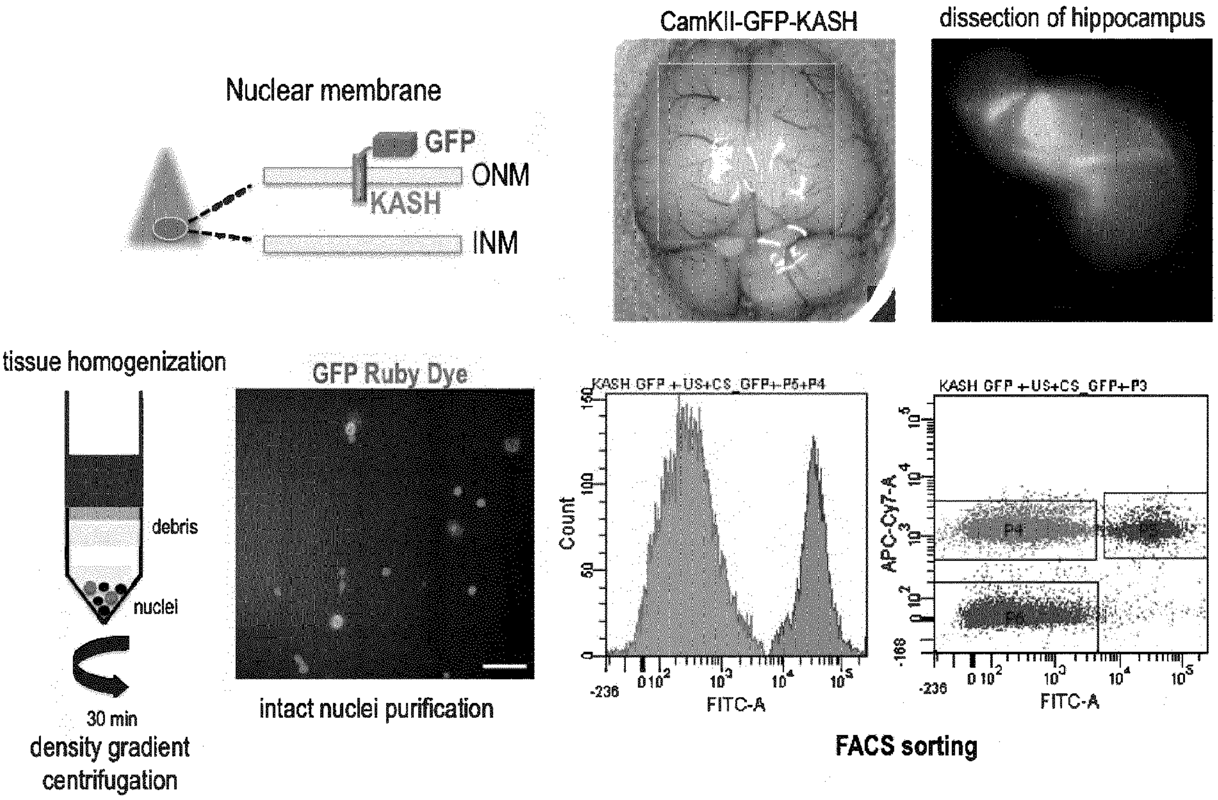


FIG. 43

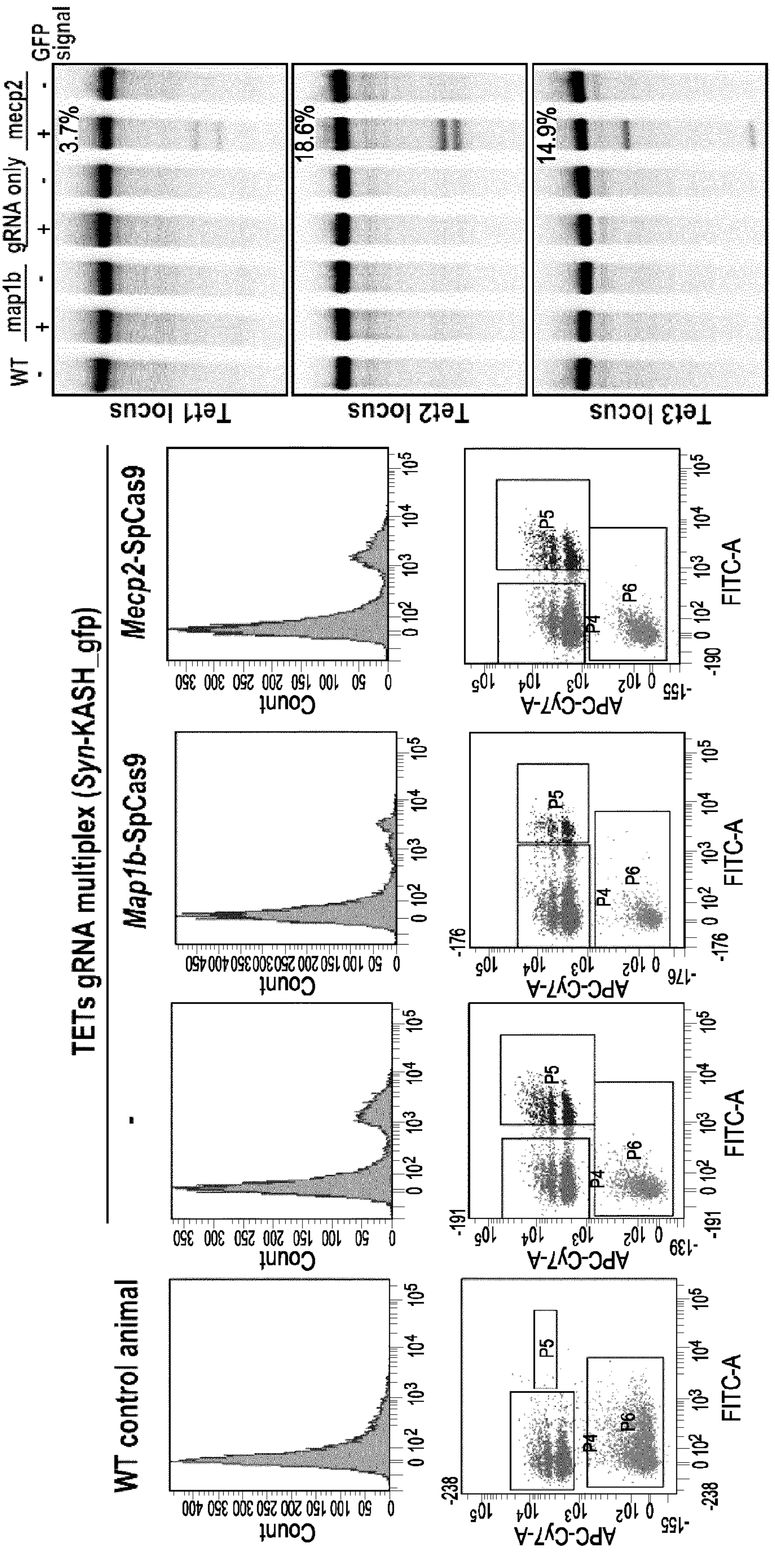
WT control animal
TETs gRNA multiplex (Syn-KASH_gfp)
-
Map1b-SpCas9
Mecp2-SpCas9
Count
APC-Cy7-A
FITC-A
P4
P5
P6
WT
map1b
gRNA only
mecp2
GFP signal
Tet1 locus
Tet2 locus
Tet3 locus
3.7%
18.6%
14.9%

FIG.44

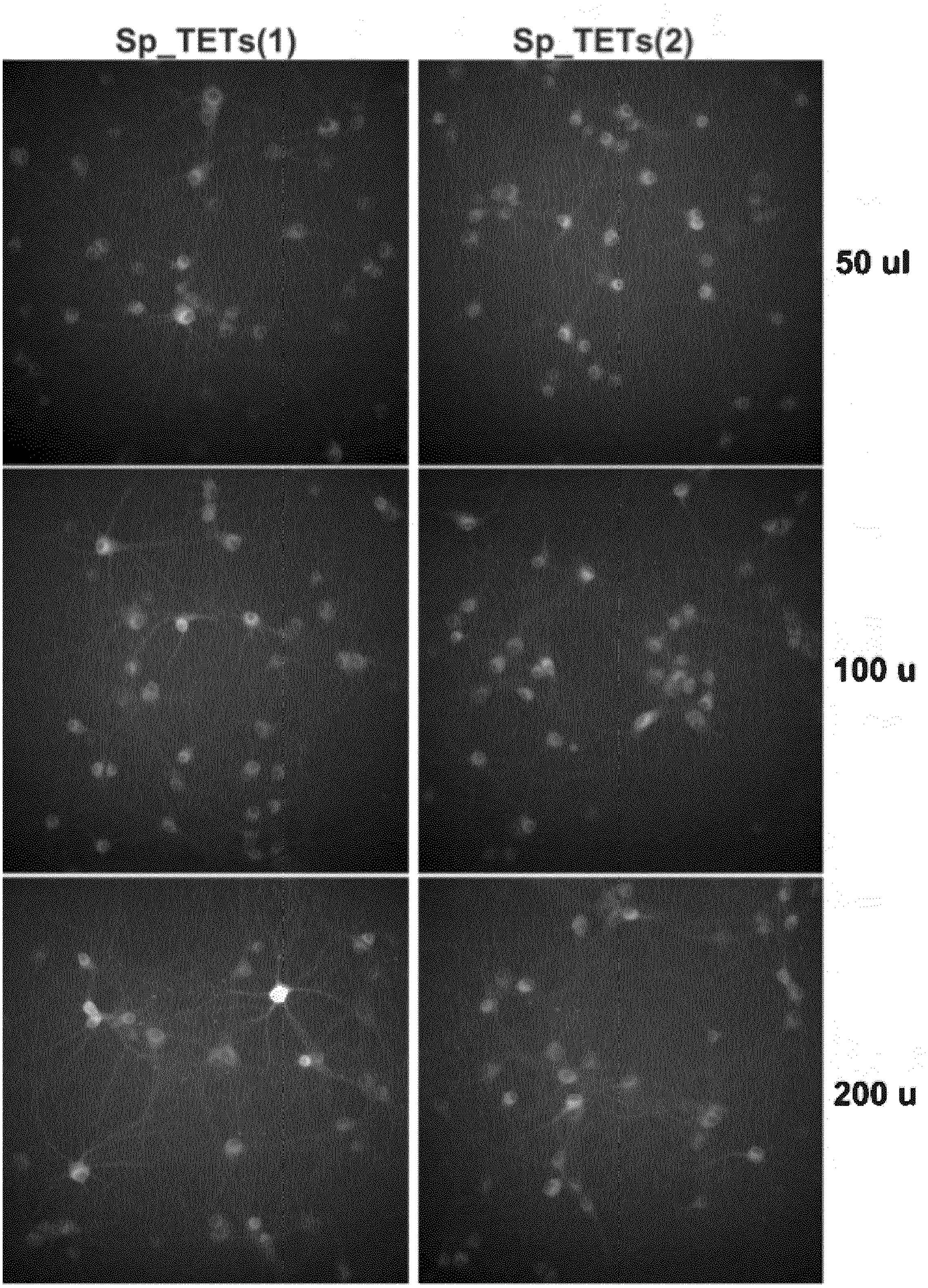


FIG. 45

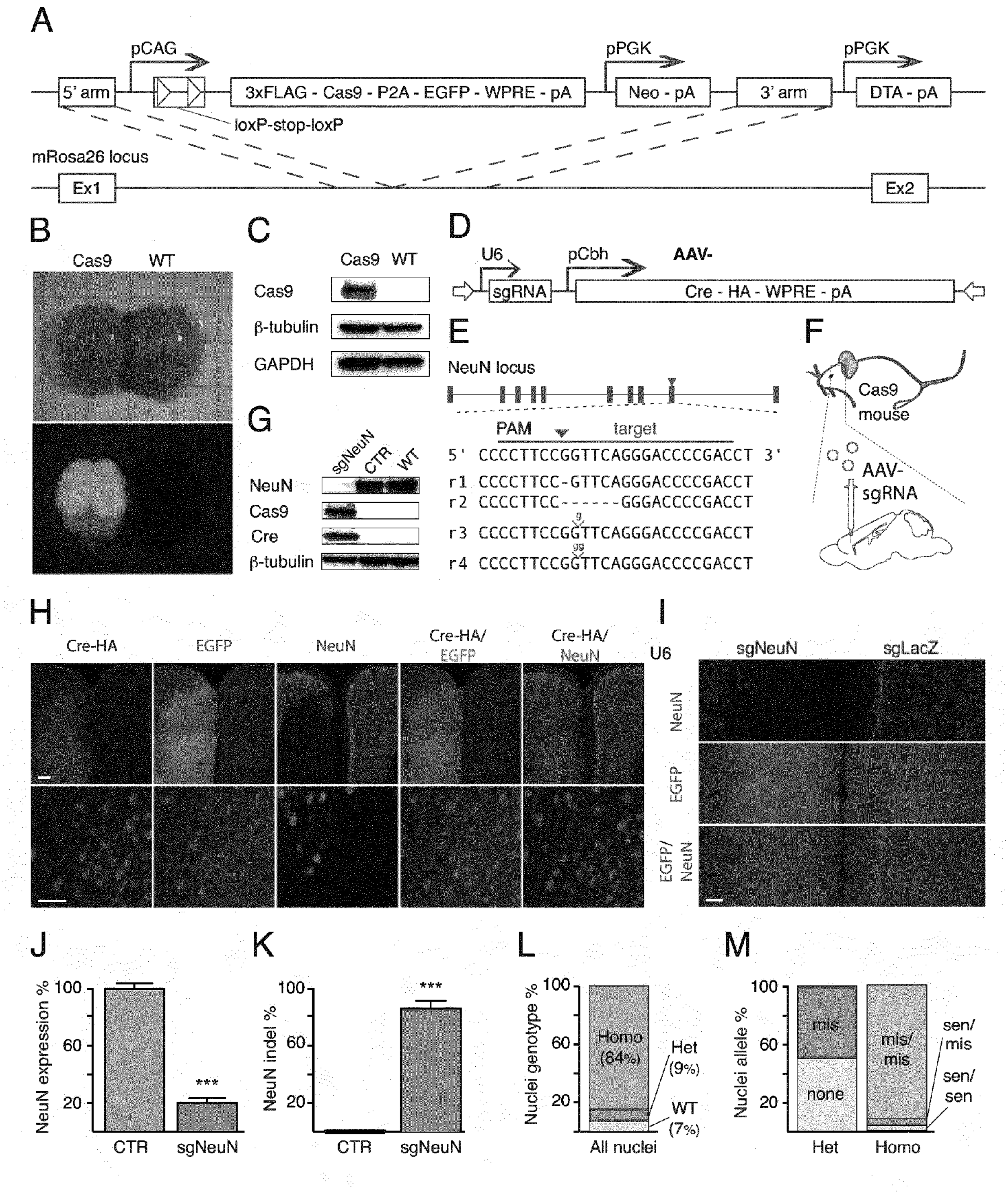

A
pCAG
pPGK
pPGK
5' arm
3xFLAG - Cas9 - P2A - EGFP - WPRE - pA
Neo - pA
3' arm
DTA - pA
loxP-stop-loxP
mRosa26 locus
Ex1
Ex2
B
Cas9
WT
C
Cas9
WT
Cas9
β-tubulin
GAPDH
D
U6
pCbh
AAV-
sgRNA
Cre - HA - WPRE - pA
E
NeuN locus
PAM
target
5' CCCCTTCCGGTTCAGGGACCCCGACCT 3'
r1 CCCCTTCC-GTTCAGGGACCCCGACCT
r2 CCCCTTCC------GGGACCCCGACCT
r3 CCCCTTCCGGTTCAGGGACCCCGACCT
r4 CCCCTTCCGGTTCAGGGACCCCGACCT
F
Cas9 mouse
AAV-sgRNA
G
sgNeuN
CTR
WT
NeuN
Cas9
Cre
β-tubulin
H
Cre-HA
EGFP
NeuN
Cre-HA/EGFP
Cre-HA/NeuN
I
U6
sgNeuN
sgLacZ
NeuN
EGFP
EGFP/NeuN
J
NeuN expression %
CTR
sgNeuN
***
K
NeuN indel %
CTR
sgNeuN
***
L
Nuclei genotype %
Homo (84%)
Het (9%)
WT (7%)
All nuclei
M
Nuclei allele %
mis
none
mis/mis
sen/mis
sen/sen
Het
Homo


FIG. 46A-M

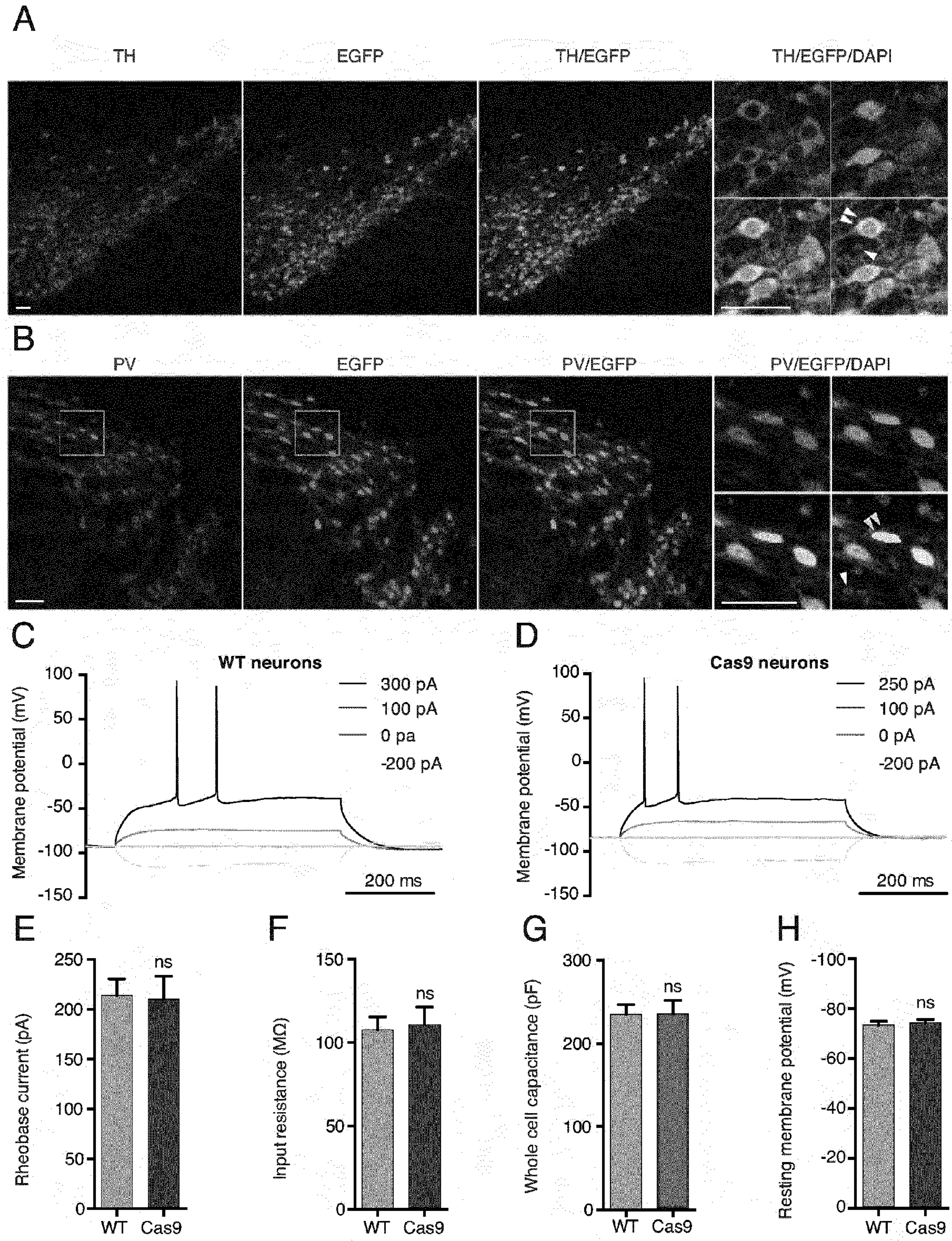
A
TH
EGFP
TH/EGFP
TH/EGFP/DAPI
B
PV
EGFP
PV/EGFP
PV/EGFP/DAPI
C
WT neurons
Membrane potential (mV)
300 pA
100 pA
0 pa
-200 pA
200 ms
D
Cas9 neurons
Membrane potential (mV)
250 pA
100 pA
0 pA
-200 pA
200 ms
E
Rheobase current (pA)
ns
WT
Cas9
F
Input resistance (MΩ)
ns
WT
Cas9
G
Whole cell capacitance (pF)
ns
WT
Cas9
H
Resting membrane potential (mV)
ns
WT
Cas9

FIG. 47A-H

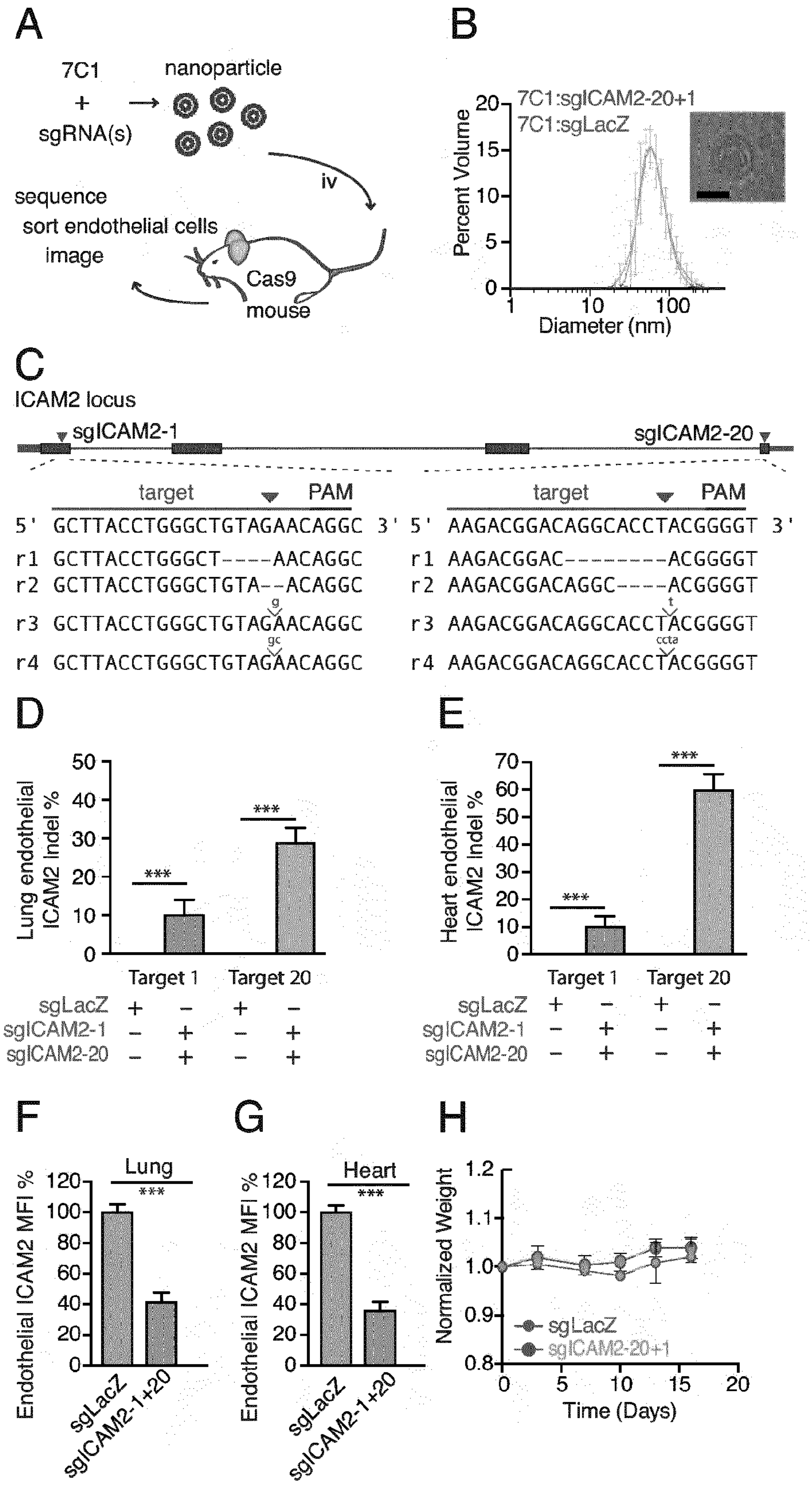
A
7C1
+
sgRNA(s)
nanoparticle
iv
sequence
sort endothelial cells
image
Cas9
mouse
B
7C1:sgICAM2-20+1
7C1:sgLacZ
Percent Volume
Diameter (nm)
C
ICAM2 locus
sgICAM2-1
sgICAM2-20
target
PAM
5' GCTTACCTGGGCTGTAGAACAGGC 3'
r1 GCTTACCTGGGCT----AACAGGC
r2 GCTTACCTGGGCTGTA--ACAGGC
r3 GCTTACCTGGGCTGTAGAACAGGC
r4 GCTTACCTGGGCTGTAGAACAGGC
5' AAGACGGACAGGCACCTACGGGGT 3'
r1 AAGACGGAC--------ACGGGGT
r2 AAGACGGACAGGC----ACGGGGT
r3 AAGACGGACAGGCACCTACGGGGT
r4 AAGACGGACAGGCACCTACGGGGT
D
Lung endothelial ICAM2 Indel %
E
Heart endothelial ICAM2 Indel %
Target 1
Target 20
sgLacZ
sgICAM2-1
sgICAM2-20
F
Lung
Endothelial ICAM2 MFI %
sgLacZ
sgICAM2-1+20
G
Heart
H
Normalized Weight
Time (Days)
sgLacZ
sgICAM2-20+1

FIG. 48A-H

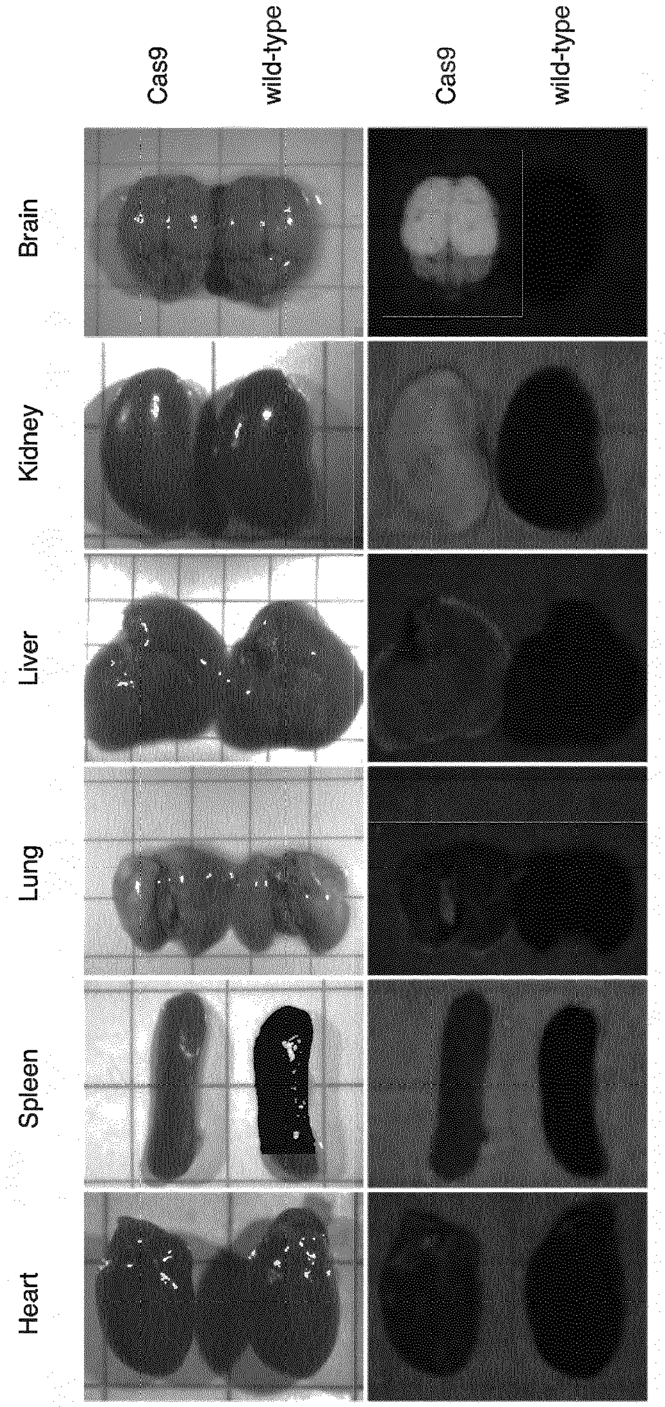

Cas9
wild-type
Cas9
wild-type
Brain
Kidney
Liver
Lung
Spleen
Heart


FIG. 49

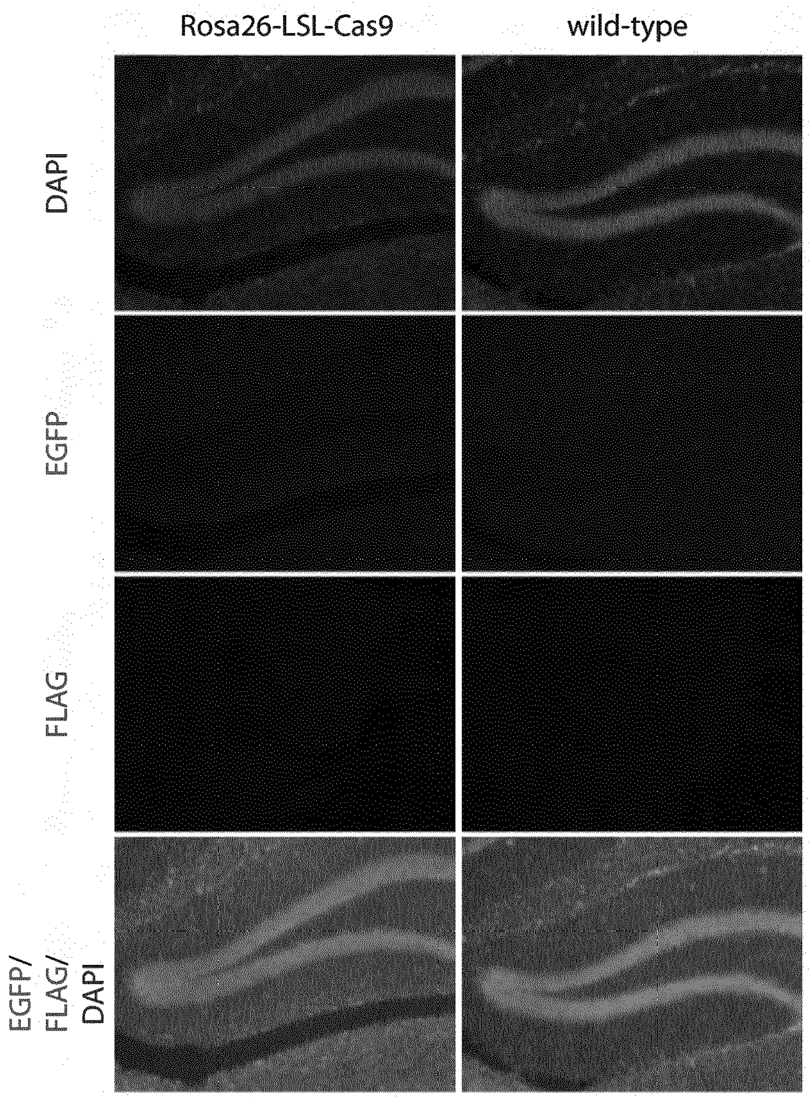


FIG. 50

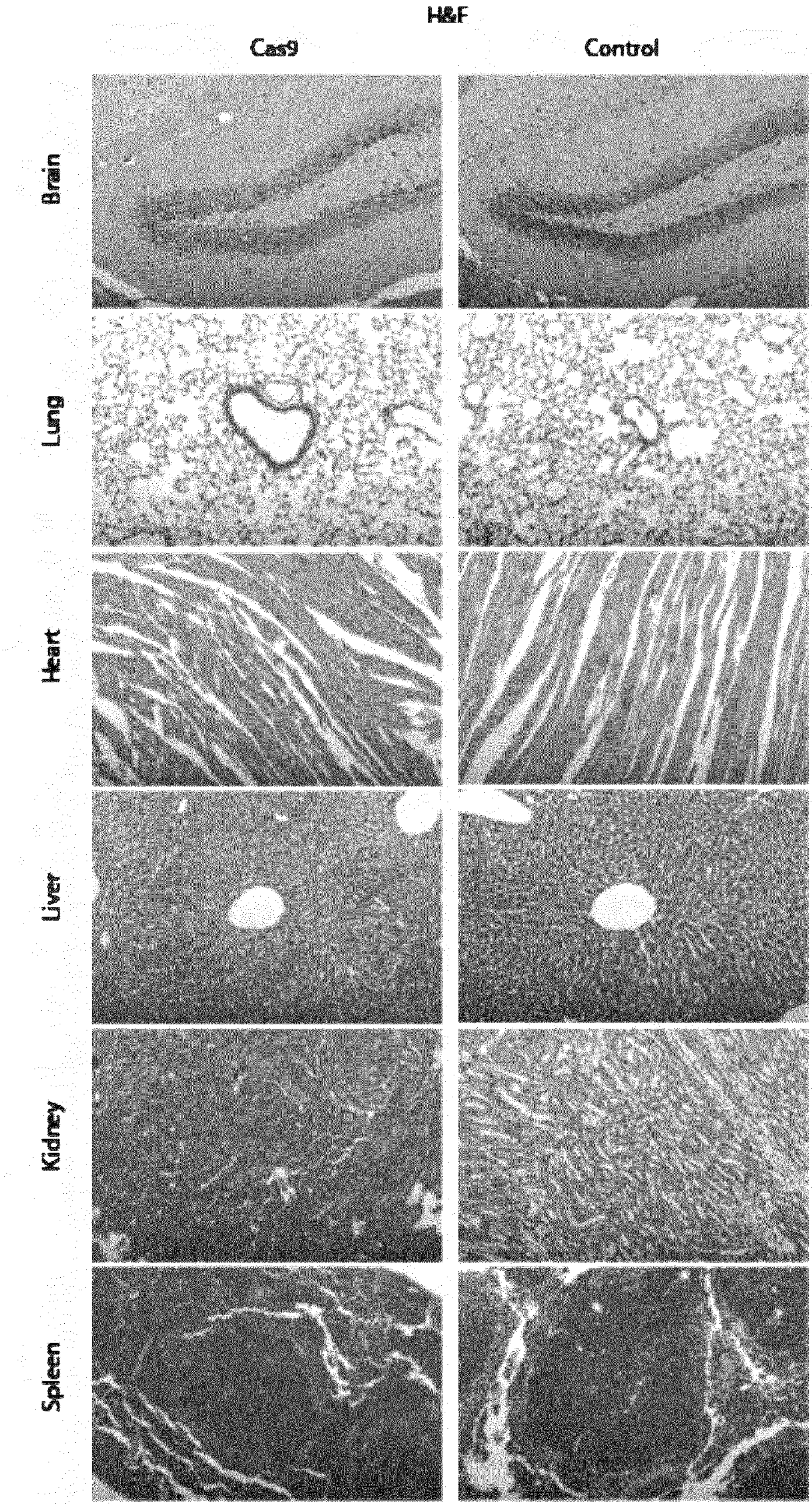


FIG. 51

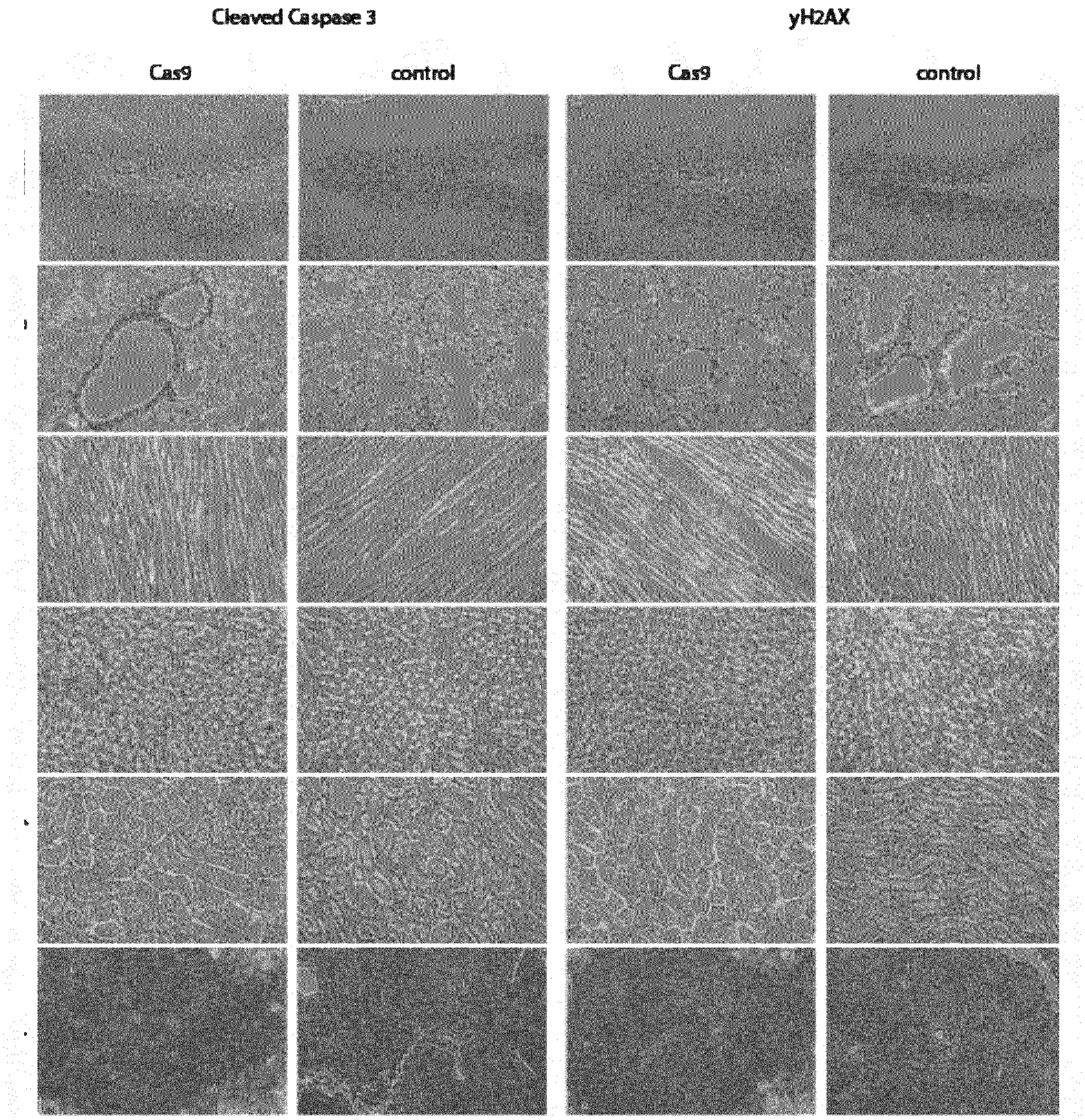


FIG. 52

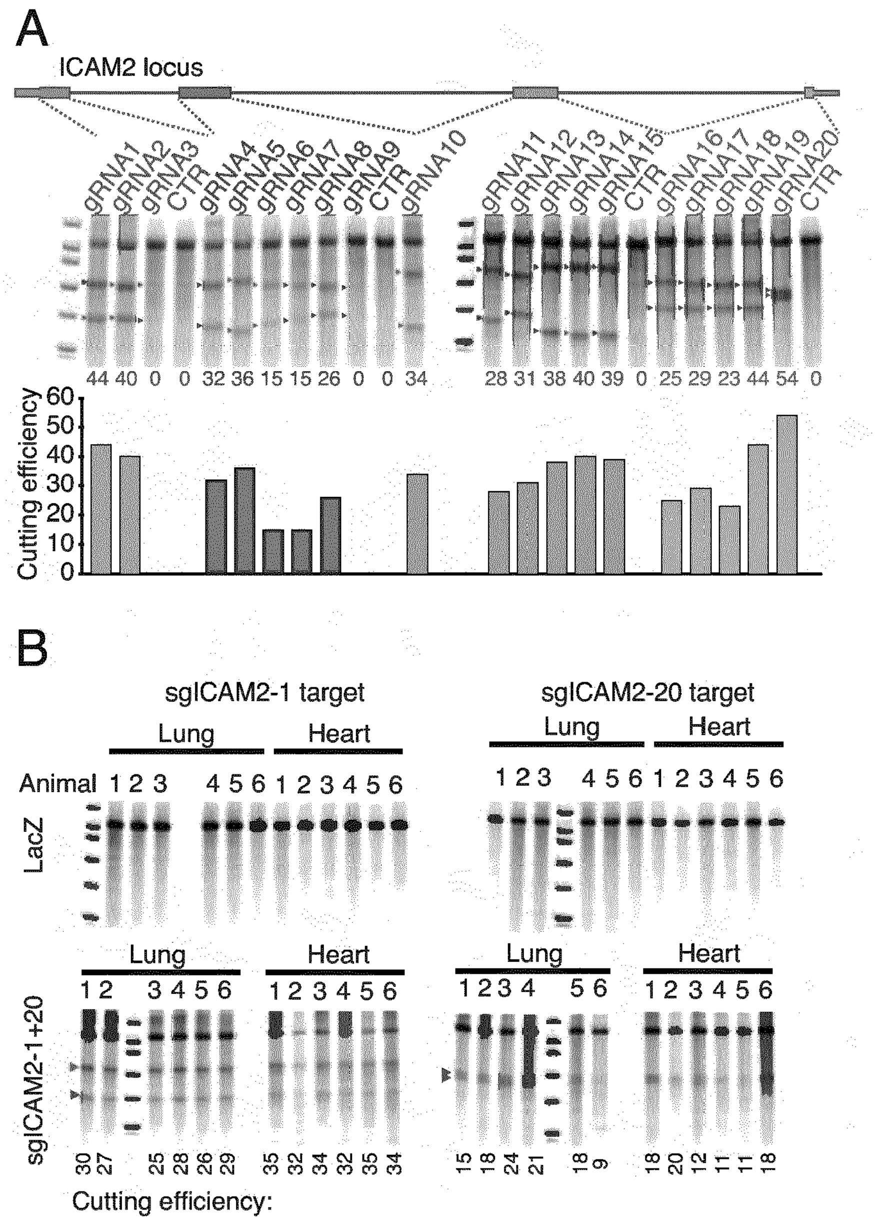
A
ICAM2 locus
gRNA1 gRNA2 gRNA3 CTR gRNA4 gRNA5 gRNA6 gRNA7 gRNA8 gRNA9 CTR gRNA10
gRNA11 gRNA12 gRNA13 gRNA14 gRNA15 CTR gRNA16 gRNA17 gRNA18 gRNA19 gRNA20 CTR
44 40 0 0 32 36 15 15 26 0 0 34
28 31 38 40 39 0 25 29 23 44 54 0
Cutting efficiency
0 10 20 30 40 50 60
B
sgICAM2-1 target
sgICAM2-20 target
Lung
Heart
Animal 1 2 3 4 5 6 1 2 3 4 5 6
LacZ
sgICAM2-1+20
30 27 25 28 26 29 35 32 34 32 35 34
15 18 24 21 18 9 18 20 12 11 11 18
Cutting efficiency:

FIG. 53A-B

Europäisches Patentamt
European Patent Office
Office européen des brevets

# EUROPEAN SEARCH REPORT

Application Number
EP 18 19 9415

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2013/155572 A1 (COMMW SCIENT IND RES ORG [AU]; MAT MALTA ADVANCED TECHNOLOGIES LTD [MT) 24 October 2013 (2013-10-24) * paragraphs [0001] - [0016], [0081], [0101] - [0102]; claims 1-9 * ----- | 1-20 | INV. A61K48/00 C12N15/10 C12N9/22 C12N15/63 |
| X | US 2010/233084 A1 (NARASIMHASWAMY MANJUNATH [US] ET AL) 16 September 2010 (2010-09-16) * paragraphs [0020] - [0021], [0122] - [0124] * ----- | 1-20 | |

TECHNICAL FIELDS SEARCHED (IPC)

A61K
C12N

The present search report has been drawn up for all claims

2

| Place of search | Date of completion of the search | Examiner |
|---|---|---|
| Munich | 25 January 2019 | Surdej, Patrick |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 18 19 9415

This annex lists the patent family members relating to the patent documents cited in the above-mentioned European search report. The members are as contained in the European Patent Office EDP file on
The European Patent Office is in no way liable for these particulars which are merely given for the purpose of information.

25-01-2019

| Patent document cited in search report | Publication date | Patent family member(s) | Publication date |
|---|---|---|---|
| WO 2013155572 A1 | 24-10-2013 | AU 2013204327 A1 | 07-11-2013 |
| | | AU 2013248945 A1 | 30-10-2014 |
| | | BR 112014026186 A2 | 18-07-2017 |
| | | CN 104619848 A | 13-05-2015 |
| | | EP 2839016 A1 | 25-02-2015 |
| | | JP 2015514404 A | 21-05-2015 |
| | | KR 20150014446 A | 06-02-2015 |
| | | US 2015072064 A1 | 12-03-2015 |
| | | WO 2013155572 A1 | 24-10-2013 |
| US 2010233084 A1 | 16-09-2010 | US 2010233084 A1 | 16-09-2010 |
| | | US 2014294727 A1 | 02-10-2014 |
| | | US 2018028677 A1 | 01-02-2018 |
| | | WO 2008054544 A2 | 08-05-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62054490 B
- US 62010441 B
- US 61915118 B
- US 61915215 B
- US 61915148 B
- WO 2013138585 A1
- US 8697359 B
- US 8771945 B
- US 8795965 B
- US 8865406 B
- US 8871445 B
- US 8889356 B
- US 8889418 B
- US 8895308 B
- US 20140310830 A
- US 105031 A
- US 20140287938 A1
- US 213991 A
- US 20140273234 A1
- US 293674 A
- US 20140273232 A1
- US 290575 A
- US 20140273231 A
- US 259420 A
- US 20140256046 A1
- US 226274 A
- US 20140248702 A1
- US 258458 A
- US 20140242700 A1
- US 222930 A
- US 20140242699 A1
- US 183512 A
- US 20140242664 A1
- US 104990 A
- US 20140234972 A1
- US 183471 A
- US 20140227787 A1
- US 256912 A
- US 20140189896 A1
- US 105035 A
- US 20140186958 A
- US 105017 A
- US 20140186919 A1
- US 104977 A
- US 20140186843 A1
- US 104900 A
- US 20140179770 A1
- US 104837 A
- US 20140179006 A1
- US 183486 A
- US 20140170753 A
- US 183429 A
- EP 2771468 A
- EP 13818570 A
- EP 2764103 A
- EP 13824232 A
- EP 2784162 A
- EP 14170383 A
- WO 2014093661 A
- US 2013074743 W
- WO 2014093694 PCT
- US 2013074790 W
- WO 2014093595 PCT
- US 2013074611 W
- WO 2014093718 A
- US 2013074825 W
- WO 2014093709 PCT
- US 2013074812 W
- WO 2014093622 A
- US 2013074667 W
- WO 2014093635 PCT
- US 2013074691 W
- WO 2014093655 A
- US 2013074736 W
- WO 2014093712 PCT
- US 2013074819 W
- WO 2014093701 A
- US 2013074800 W
- WO 2014018423 PCT
- US 2013051418 W
- US 61758468 B
- US 61802174 B
- US 61806375 B
- US 61814263 B
- US 61819803 B
- US 61828130 B
- US 61836123 B
- US 61835931 B
- US 61835936 B
- US 61836127 B
- US 61836101 A
- US 61836080 B
- US 61835973 B
- US 61862468 B
- US 61862355 B
- US 61871301 B

- US 61960777 B
- US 61961980 B
- US 2014041803 W
- US 2014041800 W
- US 2014041809 W
- US 2014041804 W
- US 2014041806 W
- US 2014041808 W
- US 201462558 W
- US 61915251 A
- US 61915301 B
- US 61915260 B
- US 61930214 B
- US 62010329 B
- US 61939228 B
- US 61939242 B
- US 61980012 B
- US 62038358 B
- US 62055484 B
- US 62055460 B
- US 62055487 B
- US 62069243 B
- US 61915150 A
- US 62010888 B
- US 62010879 B
- US 8454972 B
- US 8404658 B
- US 5846946 A
- US 20120017290 A
- US 20110265198 A
- US 20130236946 A
- US 20110293703 A
- US 0110293703 B
- US 20130302401 A
- US 8709843 B
- US 6007845 A
- US 5855913 A
- US 5985309 A
- US 5543158 A
- WO 2012135025 A
- US 20120251560 A
- US 7982027 B
- US 7799565 B
- US 8058069 B
- US 8283333 B
- US 7901708 B
- US 7745651 B
- US 7803397 B
- US 8101741 B
- US 8188263 B
- US 7915399 B
- US 8236943 B
- US 7838658 B
- EP 1766035 A
- EP 1519714 A
- EP 1781593 A
- EP 1664316 A
- US 20130252281 A
- US 20130245107 A
- US 20130244279 A
- US 20120251618 A
- US 20050019923 A
- US 20080267903 A
- US 61871301 A
- US 20130171732 A
- US 20100291048 A
- US 20130137104 A
- US 20130122591 A
- US 20100146651 A
- US 20110016540 A
- WO 2013163628 A2
- US 20130145487 A
- WO 2013126794 A
- US 20110225664 A
- US 20110091441 A
- US 20100229252 A
- US 20090271881 A
- US 20090222937 A
- US 20110182867 A
- US 20120328580 A
- US 20060030837 A, McKenna
- US 7206639 B, Jacobsen
- US 20070093878 A, Edge
- US 20050287127 A, Li
- US 11953797 B, Li
- US 2007084654 W, Edge
- US 20110142917 A
- US 5593972 A
- US 5589466 A
- US 5580859 A
- US 20120204282 A
- US 20130183282 A
- US 20130202678 A
- US 20120159653 A
- US 20110023139 A
- US 20130253040 A
- WO 9703211 A
- WO 9639154 A
- US 81573004 A
- US 20040171156 A1
- US 491026 A
- WO 2011028929 A
- US 511940 A
- US 4873316 A
- EP 264166 A
- US 6750059 B
- US 092085 A
- US 7776321 B
- US 20110059502 A
- US 61736465 B
- US 61721283 B
- US 5049386 A
- US 4946787 A
- US 4897355 A
- WO 9117424 A, Felgner
- WO 9116024 A

- US 4186183 A
- US 4217344 A
- US 4235871 A
- US 4261975 A
- US 4485054 A
- US 4501728 A
- US 4774085 A
- US 4837028 A
- US 9405700 W
- US 20120164118 A
- US 4797368 A
- WO 9324641 A
- US 5173414 A
- US 20030087817 A
- US 20110230839 A
- US 5210015 A
- US 5445934 A
- US 61736527 B
- US 61748427 B
- US 20110158957 A
- US 20100311124 A
- US 20110023145 A
- US 20110023153 A
- US 20110023146 A
- US 20110023144 A
- US 6603061 B
- US 7868149 B
- US 20090100536 A

**Non-patent literature cited in the description**


- **DAHLMAN, J. E. et al.** *Nat. Nanotechnol.,* 2014, vol. 9, 648-655
- **CONG, L. ; RAN, F.A. ; COX, D. ; LIN, S. ; BARRETTO, R. ; HABIB, N. ; HSU, P.D. ; WU, X. ; JIANG, W. ; MARRAFFINI, L.A.** Multiplex genome engineering using CRISPR/Cas systems. *Science,* 15 February 2013, vol. 339 (6121), 819-23
- **JIANG W. ; BIKARD D. ; COX D. ; ZHANG F ; MARRAFFINI LA.** RNA-guided editing of bacterial genomes using CRISPR-Cas systems. *Nat Biotechnol,* March 2013, vol. 31 (3), 233-9
- **WANG H. ; YANG H. ; SHIVALILA CS. ; DAWLATY MM. ; CHENG AW. ; ZHANG F. ; JAENISCH R.** One-Step Generation of Mice Carrying Mutations in Multiple Genes by CRISPR/Cas-Mediated Genome Engineering. *Cell,* 09 May 2013, vol. 153 (4), 910-8
- **KONERMANN S ; BRIGHAM MD ; TREVINO AE ; HSU PD ; HEIDENREICH M ; CONG L ; PLATT RJ ; SCOTT DA ; CHURCH GM ; ZHANG F.** Optical control of mammalian endogenous transcription and epigenetic states. *Nature,* 22 August 2013, vol. 500 (7463), 472-6
- **RAN, FA. ; HSU, PD. ; LIN, CY. ; GOOTENBERG, JS. ; KONERMANN, S. ; TREVINO, AE. ; SCOTT, DA. ; INOUE, A. ; MATOBA, S. ; ZHANG, Y.** Double Nicking by RNA-Guided CRISPR Cas9 for Enhanced Genome Editing Specificity. *Cell,* 28 August 2013, S0092-8674
- **HSU, P. ; SCOTT, D. ; WEINSTEIN, J. ; RAN, FA. ; KONERMANN, S. ; AGARWALA, V. ; LI, Y. ; FINE, E. ; WU, X. ; SHALEM, O.** DNA targeting specificity of RNA-guided Cas9 nucleases. *Nat Biotechnol,* 2013
- **RAN, FA. ; HSU, PD. ; WRIGHT, J. ; AGARWALA, V. ; SCOTT, DA. ; ZHANG, F.** Genome engineering using the CRISPR-Cas9 system. *Nature Protocols,* November 2013, vol. 8 (11), 2281-308
- **SHALEM, O. ; SANJANA, NE. ; HARTENIAN, E. ; SHI, X. ; SCOTT, DA. ; MIKKELSON, T. ; HECKL, D. ; EBERT, BL. ; ROOT, DE. ; DOENCH, JG.** Genome-Scale CRISPR-Cas9 Knockout Screening in Human Cells. *Science,* 12 December 2013
- **NISHIMASU, H. ; RAN, FA. ; HSU, PD. ; KONERMANN, S. ; SHEHATA, SI. ; DOHMAE, N. ; ISHITANI, R. ; ZHANG, F. ; NUREKI, O.** Crystal structure of cas9 in complex with guide RNA and target DNA. *Cell,* 27 February 2014, vol. 156 (5), 935-49
- **WU X. ; SCOTT DA. ; KRIZ AJ. ; CHIU AC. ; HSU PD. ; DADON DB. ; CHENG AW. ; TREVINO AE. ; KONERMANN S. ; CHEN S.** Genome-wide binding of the CRISPR endonuclease Cas9 in mammalian cells. *Nat Biotechnol.,* 20 April 2014
- **PLATT et al.** CRISPR-Cas9 Knockin Mice for Genome Editing and Cancer Modeling. *Cell,* 2014, vol. 159 (2), 440-455
- **HSU et al.** Development and Applications of CRISPR-Cas9 for Genome Engineering. *Cell,* 05 June 2014, vol. 157, 1262-1278
- **WANG et al.** Genetic screens in human cells using the CRISPR/Cas9 system. *Science,* 03 January 2014, vol. 343 (6166), 80-84
- **DOENCH et al.** Rational design of highly active sgRNAs for CRISPR-Cas9-mediated gene inactivation. *Nature Biotechnology,* 03 September 2014
- **SWIECH et al.** In vivo interrogation of gene function in the mammalian brain using CRISPR-Cas9. *Nature Biotechnology,* 19 October 2014
- **ZUKER ; STIEGLER.** *Nucleic Acids Res.,* 1981, vol. 9, 133-148
- **A.R. GRUBER et al.** *Cell,* 2008, vol. 106 (1), 23-24
- **PA CARR ; GM CHURCH.** *Nature Biotechnology,* 2009, vol. 27 (12), 1151-62
- **KARGINOV ; HANNON.** The CRISPR system: small RNA-guided defence in bacteria and archaea. *Mole Cell,* 15 January 2010, vol. 37 (1), 7

- **NAKAMURA, Y. et al.** Codon usage tabulated from the international DNA sequence databases: status for the year 2000. *Nucl. Acids Res.,* 2000, vol. 28, 292
- **SU X ; FRICKE J ; KAVANAGH DG ; IRVINE DJ.** In vitro and in vivo mRNA delivery using lipid-enveloped pH-responsive polymer nanoparticles. *Mol Pharm.,* 06 June 2011, vol. 8 (3), 774-87
- **MAZZA, M. et al.** *ACSNano,* 2013, vol. 7 (2), 1016-1026
- **SIEW, A. et al.** *Mol Pharm,* 2012, vol. 9 (1), 14-28
- **LALATSA, A. et al.** *J Contr Rel,* 2012, vol. 161 (2), 523-36
- **LALATSA, A. et al.** *Mol Pharm,* 2012, vol. 9 (6), 1665-80
- **LALATSA, A. et al.** *Mol Pharm,* 2012, vol. 9 (6), 1764-74
- **GARRETT, N.L. et al.** *J Biophotonics,* 2012, vol. 5 (5-6), 458-68
- **GARRETT, N.L. et al.** *J Raman Spect,* 2012, vol. 43 (5), 681-688
- **AHMAD, S. et al.** *J Royal Soc Interface,* 2010, vol. 7, 423-33
- **UCHEGBU, I.F.** *Expert Opin Drug Deliv,* 2006, vol. 3 (5), 629-40
- **QU, X.** *Biomacromolecules,* 2006, vol. 7 (12), 3452-9
- **UCHEGBU, I.F. et al.** *Int J Pharm,* 2001, vol. 224, 185-199
- **ALABI et al.** *Proc Natl Acad Sci U S A.,* 06 August 2013, vol. 110 (32), 12881-6
- **ZHANG et al.** *Adv Mater.,* 06 September 2013, vol. 25 (33), 4641-5
- **JIANG et al.** *Nano Lett.,* 13 March 2013, vol. 13 (3), 1059-64
- **KARAGIANNIS et al.** *ACS Nano.,* 23 October 2012, vol. 6 (10), 8484-7
- **WHITEHEAD et al.** *ACS Nano.,* 28 August 2012, vol. 6 (8), 6922-9
- **LEE et al.** *Nat Nanotechnol.,* 03 June 2012, vol. 7 (6), 389-93
- **COELHO et al.** *N Engl J Med,* 2013, vol. 369, 819-29
- **TABERNERO et al.** *Cancer Discovery,* April 2013, vol. 3 (4), 363-470
- **ROSIN et al.** *Molecular Therapy,* December 2011, vol. 19 (12), 1286-2200
- **CUTLER et al.** *J. Am. Chem. Soc.,* 2011, vol. 133, 9254-9257
- **HAO et al.** *Small.,* 2011, vol. 7, 3158-3162
- **ZHANG et al.** *ACS Nano.,* 2011, vol. 5, 6962-6970
- **CUTLER et al.** *J. Am. Chem. Soc.,* 2012, vol. 134, 1376-1391
- **YOUNG et al.** *Nano Lett.,* 2012, vol. 12, 3867-71
- **ZHENG et al.** *Proc. Natl. Acad. Sci. USA.,* 2012, vol. 109, 11975-80
- **MIRKIN.** *Nanomedicine,* 2012, vol. 7, 635-638
- **ZHANG et al.** *J. Am. Chem. Soc.,* 2012, vol. 134, 16488-1691
- **WEINTRAUB.** *Nature,* 2013, vol. 495, S14-S16
- **CHOI et al.** *Proc. Natl. Acad. Sci. USA.,* 2013, vol. 110 (19), 7625-7630
- **JENSEN et al.** *Sci. Transl. Med.,* 2013, vol. 5, 209ra152
- **MIRKIN et al.** *Small*
- **SCHIFFELERS et al.** *Nucleic Acids Research,* 2004, vol. 32 (19
- **BARTLETT et al.** *PNAS,* 25 September 2007, vol. 104 (39
- **DAVIS et al.** *Nature,* 15 April 2010, vol. 464
- **KANASTY ; ANDERSON.** *Nature Materials,* November 2013, vol. 12
- **JAMES E. DAHLMAN ; CARMEN BARNES et al.** *Nature Nanotechnology,* 11 May 2014
- **ALVAREZ-ERVITI et al.** *Nat Biotechnol,* 2011, vol. 29, 341
- **EL-ANDALOUSSI et al.** *Nature Protocols,* 2012, vol. 7, 2112-2126
- **WAHLGREN et al.** *Nucleic Acids Research,* 2012, vol. 40 (17), e130
- **SPUCH ; NAVARRO.** *Journal of Drug Delivery,* 2011, vol. 2011, 12
- **MORRISSEY et al.** *Nature Biotechnology,* August 2005, vol. 23 (8
- **ZIMMERMAN et al.** *Nature Letters,* 04 May 2006, vol. 441
- **LI.** *Gene Therapy,* 2012, vol. 19, 775-780
- **GEISBERT et al.** *Lancet,* 2010, vol. 375, 1896-905
- **JUDGE.** *J. Clin. Invest.,* 2009, vol. 119, 661-673
- **BARROS ; GOLLOB.** *Alnylam Pharmaceuticals*
- *Advanced Drug Delivery Reviews,* 2012, vol. 64, 1730-1737
- **SEMPLE et al.** *Nature Niotechnology,* February 2010, vol. 28 (2), 172-177
- **JAYARAMAN.** *Angew. Chem. Int. Ed.,* 2012, vol. 51, 8529-8533
- **MICHAEL S D KORMANN et al.** Expression of therapeutic proteins after delivery of chemically modified mRNA in mice. *Nature Biotechnology,* 09 January 2011, vol. 29, 154-157
- **NOVOBRANTSEVA.** *Molecular Therapy-Nucleic Acids,* 2012, vol. 1, e4
- **SCHRUM et al.** *Delivery and Formulation of Engineered Nucleic Acids*
- **MAZZA et al.** *ACS Nano.,* 26 February 2013, vol. 7 (2), 1016-26
- **UCHEGBU ; SIEW.** *J Pharm Sci.,* 2013, vol. 102 (2), 305-10

- **LALATSA et al.** *J Control Release.,* 20 July 2012, vol. 161 (2), 523-36
- **RAN et al.** *Cell.,* 12 September 2013, vol. 154 (6), 1380-9
- **BAILEY et al.** *J Mol Med (Berl).,* January 1999, vol. 77 (1), 244-9
- **SATO et al.** *Nature Biotechnology,* April 2008, vol. 26 (4), 431-442
- **ROZEMA et al.** *PNAS,* 07 August 2007, vol. 104 (32
- **OAKES ; LIEBERMAN.** *Clin Orthop Relat Res.,* October 2000, vol. 379, 101-12
- **XIA CF ; BOADO RJ ; PARDRIDGE WM.** Antibody-mediated targeting of siRNA via the human insulin receptor using avidin-biotin technology. *Mol Pharm.,* May 2009, vol. 6 (3), 747-51
- **ZHANG et al.** *Mol Ther.,* January 2003, vol. 7 (1), 11-8
- **CHO, S. ; GOLDBERG, M. ; SON, S. ; XU, Q. ; YANG, F. ; MEI, Y. ; BOGATYREV, S. ; LANGER, R. ; ANDERSON, D.** Lipid-like nanoparticles for small interfering RNA delivery to endothelial cells. *Advanced Functional Materials,* 2010, vol. 19, 3112-3118
- **SCHROEDER, A. ; LEVINS, C. ; CORTEZ, C. ; LANGER, R. ; ANDERSON, D.** Lipid-based nanotherapeutics for siRNA delivery. *Journal of Internal Medicine,* 2010, vol. 267, 9-21
- **EL-ANDALOUSSI S et al.** Exosome-mediated delivery of siRNA in vitro and in vivo. *Nat Protoc.,* December 2012, vol. 7 (12), 2112-26
- **UNO et al.** *HUMAN GENE THERAPY,* June 2011, vol. 22, 711-719
- **ZOU et al.** *HUMAN GENE THERAPY,* April 2011, vol. 22, 465-475
- **XIA et al.** *Nature Medicine,* August 2004, vol. 10 (8
- **TANGRI S et al.** Rationally engineered therapeutic proteins with reduced immunogenicity. *J Immunol.,* 15 March 2005, vol. 174 (6), 3187-96
- **CAVAZZANA-CALVO et al.** *Annu. Rev. Med.,* 2005, vol. 56, 585-602
- **FISCHER et al.** *Immunol. Rev.,* 2005, vol. 203, 98-109
- **GIBLETT et al.** *Lancet,* 1972, vol. 2, 1067-1069
- **NOGUCHI et al.** *Cell,* 1993, vol. 73, 147-157
- **MACCHI et al.** *Nature,* 1995, vol. 377, 65-68
- **HIRSCHHORN et al.** *Nat. Genet.,* 1996, vol. 13, 290-295
- **STEPHAN et al.** *N. Engl. J. Med.,* 1996, vol. 335, 1563-1567
- **BOUSSO et al.** *Proc. Natl., Acad. Sci. USA,* 2000, vol. 97, 274-278
- **WADA et al.** *Proc. Natl. Acad. Sci. USA,* 2001, vol. 98, 8697-8702
- **NISHIKOMORI et al.** *Blood,* 2004, vol. 103, 4565-4572
- **CANDOTTI et al.** *Blood,* 1996, vol. 87, 3097-3102
- **CAVAZZANA-CALVO et al.** *Blood,* 1996
- *Blood,* vol. 88, 3901-3909
- **TAYLOR et al.** *Blood,* 1996, vol. 87, 3103-3107
- **HACEIN-BEY et al.** *Blood,* 1998, vol. 92, 4090-4097
- **SOUDAIS et al.** *Blood,* 2000, vol. 95, 3071-3077
- **TSAI et al.** *Blood,* 2002, vol. 100, 72-79
- **BUNTING et al.** *Nat. Med.,* 1998, vol. 4, 58-64
- **BUNTING et al.** *Hum. Gene Ther.,* 2000, vol. 11, 2353-2364
- **YATES et al.** *Blood,* 2002, vol. 100, 3942-3949
- **CAVAZZANA-CALVO et al.** *Science,* 2000, vol. 288, 669-672
- **AIUTI et al.** *Nat. Med.,* 2002, vol. 8, 423-425
- **GASPAR et al.** *Lancet,* 2004, vol. 364, 2181-2187
- **BAUER et al.** *Science,* 11 October 2013, vol. 342 (6155), 253-257
- **XU et al.** *Science,* 18 November 2011, vol. 334 (6058), 993-996
- **SALT ; PLONTKE.** *Drug Discovery Today,* 2005, vol. 10, 1299-1306
- **TAKAHASHI et al.** *Cell,* 2007, vol. 131 (5), 861-872
- **TAKAHASHI ; YAMANAKA.** *Cell,* 2006, vol. 126, 663-76
- **OKITA et al.** *Nature,* 2007, vol. 448, 260-262
- **YU, J. et al.** *Science,* 2007, vol. 318 (5858), 1917-1920
- **NAKAGAWA et al.** *Nat. Biotechnol.,* 2008, vol. 26, 101-106
- **ZAEHRES ; SCHOLER.** *Cell,* 2007, vol. 131 (5), 834-835
- **SHEN et al.** *FEBS Let.,* 2003, vol. 539, 111-114
- **XIA et al.** *Nat. Biotech.,* 2002, vol. 20, 1006-1010
- **REICH et al.** *Mol. Vision.,* 2003, vol. 9, 210-216
- **SORENSEN et al.** *J. Mol. Biol.,* 2003, vol. 327, 761-766
- **LEWIS et al.** *Nat. Gen.,* 2002, vol. 32, 107-108
- **SIMEONI et al.** *NAR,* 2003, vol. 31 (11), 2717-2724
- **TOLENTINO et al.** *Retina,* vol. 24 (4), 660
- **QI et al.** *Gene Therapy,* 2013, 1-9
- **REJALI et al.** *Hear Res.,* June 2007, vol. 228 (1-2), 180-7
- **MUKHERJEA et al.** *Antioxidants & Redox Signaling,* 2010, vol. 13 (5
- **JUNG et al.** *Molecular Therapy,* April 2013, vol. 21 (4), 834-841

- Genetic Diseases of the Eye. Oxford University Press, 2012
- **BALAGAAN.** J Gene Med 2006. Wiley InterScience, 21 November 2005, vol. 8, 275-285
- **BINLEY et al.** *HUMAN GENE THERAPY,* September 2012, vol. 23, 980-991
- **CAMPOCHIARO et al.** *Human Gene Therapy,* February 2006, vol. 17, 167-176
- **MILLINGTON-WARD et al.** *Molecular Therapy,* April 2011, vol. 19 (4), 642-649
- **DALKARA et al.** *Sci Transl Med,* 2013, vol. 5, 189ra76
- **WU.** *Cell Stem Cell,* 2013, vol. 13, 659-62
- **LIN-YANGA et al.** *PNAS,* 10 March 2009, vol. 106 (10
- **EULALIO et al.** *Nature,* 2012, vol. 492, 376
- **SOMASUNTHARAM et al.** *Biomaterials,* 2013, vol. 34, 7790
- Delivery Methods to Target RNAs in the Kidney. **CSABA RÉVÉSZ ; PÉTER HAMAR.** Gene Therapy Applications. InTech, 2011
- **LARSON et al.** *Surgery,* August 2007, vol. 142 (2), 262-269
- **HAMAR et al.** *Proc Natl Acad Sci,* October 2004, vol. 101 (41), 14883-14888
- **ZHENG et al.** *Am J Pathol,* October 2008, vol. 173 (4), 973-980
- **FENG et al.** *Transplantation,* May 2009, vol. 87 (9), 1283-1289
- **XIE ; GUO.** *Am Soc Nephrol,* December 2006, vol. 17 (12), 3336-3346
- **Q. ZHANG et al.** *PloS ONE,* July 2010, vol. 5 (7), 1-13
- **KUSHIBIKIA et al.** *J Controlled Release,* July 2005, vol. 105 (3), 318-331
- **WANG et al.** *Gene Therapy,* July 2006, vol. 13 (14), 1097-1103
- **KOBAYASHI et al.** *Journal of Pharmacology and Experimental Therapeutics,* February 2004, vol. 308 (2), 688-693
- **WOLFRUM et al.** *Nature Biotechnology,* September 2007, vol. 25 (10), 1149-1157
- **MOLITORIS et al.** *J Am Soc Nephrol,* August 2009, vol. 20 (8), 1754-1764
- **MIKHAYLOVA et al.** *Cancer Gene Therapy,* March 2011, vol. 16 (3), 217-226
- **YUAN et al.** *Am J Physiol Renal Physiol,* June 2008, vol. 295, F605-F617
- **Y. ZHANG et al.** *J Am Soc Nephrol,* April 2006, vol. 17 (4), 1090-1101
- **SINGHAL et al.** *Cancer Res,* May 2009, vol. 69 (10), 4244-4251
- **MALEK et al.** *Toxicology and Applied Pharmacology,* April 2009, vol. 236 (1), 97-108
- **SHIMIZU et al.** *J Am Soc Nephrology,* April 2010, vol. 21 (4), 622-633
- **JIANG et al.** *Molecular Pharmaceutics,* May 2009, vol. 6 (3), 727-737
- **CAO et al.** *J Controlled Release,* June 2010, vol. 144 (2), 203-212
- **NINICHUK et al.** *Am J Pathol,* March 2008, vol. 172 (3), 628-637
- **PURSCHKE et al.** *Proc Natl Acad Sci,* March 2006, vol. 103 (13), 5173-5178
- **YUAN et al.** *Am J Physiol Renal Physiol,* 2008, vol. 295, F605-F617
- **MOLITORIS et al.** *J Am Soc Nephrol,* 2009, vol. 20, 1754-1764
- **THOMPSON et al.** *Nucleic Acid Therapeutics,* 2012, vol. 22 (4
- **SHIMIZU et al.** *J Am Soc Nephrol,* 2010, vol. 21, 622-633
- **LI et al.** *Molecular Therapy,* December 2009, vol. 17 (12), 2067-2077
- **ZAMORA et al.** *Am J Respir Crit Care Med,* 2011, vol. 183, 531-538
- **SCHWANK et al.** *Cell Stem Cell,* 2013, vol. 13, 653-58
- **BORTOLANZA et al.** *Molecular Therapy,* November 2011, vol. 19 (11), 2055-2064
- **DUMONCEAUX et al.** *Molecular Therapy,* May 2010, vol. 18 (5), 881-887
- **KINOUCHI et al.** *Gene Therapy,* 2008, vol. 15, 1126-1130
- **HAGSTROM et al.** *Molecular Therapy,* August 2004, vol. 10 (2
- **HICKERSON et al.** *Molecular Therapy-Nucleic Acids,* 2013, vol. 2, e129
- **LEACHMAN et al.** *Molecular Therapy,* February 2010, vol. 18 (2), 442-446
- **ZHENG et al.** *PNAS,* 24 July 2012, vol. 109 (30), 11975-11980
- **GRIMM et al.** *Nature,* 26 May 2006, vol. 441
- **CHEN et al.** *Gene Therapy,* 2007, vol. 14, 11-19
- **WOODDELL et al.** *Molecular Therapy,* May 2013, vol. 21 (5), 973-985
- **ROELVINKI et al.** *Molecular Therapy,* September 2012, vol. 20 (9), 1737-1749
- **MCBRIDE et al.** *Molecular Therapy,* December 2011, vol. 19 (12), 2152-2162
- **DIFIGLIA et al.** *PNAS,* 23 October 2007, vol. 104 (43), 17204-17209
- **BOUDREAU et al.** *Molecular Therapy,* June 2009, vol. 17 (6
- **YU et al.** *Cell,* 31 August 2012, vol. 150, 895-908
- **STILES et al.** *Experimental Neurology,* 2012, vol. 233, 463-471
- Overview of principles of hybridization and the strategy of nucleic acid probe assay. **TIJSSEN.** Laboratory Techniques In Biochemistry And Molecular Biology-Hybridization With Nucleic Acid Probes. Elsevier, 1993
- **DEVEREUX et al.** *Nucleic Acids Research,* 1984, vol. 12, 387

- **ATSCHUL et al.** *J. Mol. Biol.,* 1990, 403-410
- **DEVEREUX et al.** *Nuc. Acids Research,* 1984, vol. 12, 387
- **AUSUBEL et al.** Short Protocols in Molecular Biology. 1999
- **ALTSCHUL et al.** *J. Mol. Biol.,* 1990, 403-410
- **AUSUBEL et al.** *Short Protocols in Molecular Biology,* 1999, 7-58, 7-60
- *FEMS Microbiol Lett.,* 1999, vol. 174 (2), 247-50
- *FEMS Microbiol Lett.,* 1999, vol. 177 (1), 187-8
- **HIGGINS DG ; SHARP PM.** *Gene,* 1988, vol. 73 (1), 237-244
- **LIVINGSTONE C.D. ; BARTON G.J.** Protein sequence alignments: a strategy for the hierarchical analysis of residue conservation. *Comput. Appl. Biosci.,* 1993, vol. 9, 745-756
- **TAYLOR W.R.** The classification of amino acid conservation. *J. Theor. Biol.,* 1986, vol. 119, 205-218
- **SIMON RJ et al.** *PNAS,* 1992, vol. 89 (20), 9367-9371
- **HORWELL DC.** *Trends Biotechnol.,* 1995, vol. 13 (4), 132-134
- **SAMBROOK ; FRITSCH ; MANIATIS.** MOLECULAR CLONING: A LABORATORY MANUAL. 1989
- CURRENT PROTOCOLS IN MOLECULAR BIOLOGY. 1987
- PCR 2: A PRACTICAL APPROACH. METHODS IN ENZYMOLOGY. Academic Press, Inc, 1995
- ANTIBODIES, A LABORATORY MANUAL. 1988
- ANIMAL CELL CULTURE. 1987
- METHODS IN ENZYMOLOGY. **GOEDDEL.** GENE EXPRESSION TECHNOLOGY. Academic Press, 1990, 185
- **BOSHART et al.** *Cell,* 1985, vol. 41, 521-530
- *Mol. Cell. Biol.,* 1988, vol. 8 (1), 466-472
- *Proc. Natl. Acad. Sci. USA.,* 1981, vol. 78 (3), 1527-31
- **SMITH ; JOHNSON.** Gene. Pharmacia Biotech Inc, 1988, vol. 67, 31-40
- **AMRANN et al.** *Gene,* 1988, vol. 69, 301-315
- METHODS IN ENZYMOLOGY. **STUDIER et al.** GENE EXPRESSION TECHNOLOGY. Academic Press, 1990, vol. 185, 60-89
- **BALDARI et al.** *EMBO J.,* 1987, vol. 6, 229-234
- **KUIJAN ; HERSKOWITZ.** *Cell,* 1982, vol. 30, 933-943
- **SCHULTZ et al.** *Gene,* 1987, vol. 54, 113-123
- **SMITH et al.** *Mol. Cell. Biol.,* 1983, vol. 3, 2156-2165
- **LUCKLOW ; SUMMERS.** *Virology,* 1989, vol. 170, 31-39
- **SEED.** *Nature,* 1987, vol. 329, 840
- **KAUFMAN et al.** *EMBO J.,* 1987, vol. 6, 187-195
- **SAMBROOK et al.** MOLECULAR CLONING: A LABORATORY MANUAL. Cold Spring Harbor Laboratory Press, 1989
- **PINKERT et al.** *Genes Dev.,* 1987, vol. 1, 268-277
- **CALAME ; EATON.** *Adv. Immunol.,* 1988, vol. 43, 235-275
- **WINOTO ; BALTIMORE.** *EMBO J.,* 1989, vol. 8, 729-733
- **BANEIJI et al.** *Cell,* 1983, vol. 33, 729-740
- **QUEEN ; BALTIMORE.** *Cell,* 1983, vol. 33, 741-748
- **BYRNE ; RUDDLE.** *Proc. Natl. Acad. Sci. USA,* 1989, vol. 86, 5473-5477
- **EDLUND et al.** *Science,* 1985, vol. 230, 912-916
- **KESSEL ; GRUSS.** *Science,* 1990, vol. 249, 374-379
- **CAMPES ; TILGHMAN.** *Genes Dev.,* 1989, vol. 3, 537-546
- **ISHINO et al.** *J. Bacteriol.,* 1987, vol. 169, 5429-5433
- **NAKATA et al.** *J. Bacteriol.,* 1989, vol. 171, 3553-3556
- **GROENEN et al.** *Mol. Microbiol.,* 1993, vol. 10, 1057-1065
- **HOE et al.** *Emerg. Infect. Dis.,* 1999, vol. 5, 254-263
- **MASEPOHL et al.** *Biochim. Biophys. Acta,* 1996, vol. 1307, 26-30
- **MOJICA et al.** *Mol. Microbiol.,* 1995, vol. 17, 85-93
- **JANSSEN et al.** *OMICS J. Integ. Biol.,* 2002, vol. 6, 23-33
- **MOJICA et al.** *Mol. Microbiol.,* 2000, vol. 36, 244-246
- **VAN EMBDEN et al.** *J. Bacteriol.,* 2000, vol. 182, 2393-2401
- **JANSEN et al.** *Mol. Microbiol.,* 2002, vol. 43, 1565-1575
- **SAPRANAUSKAS et al.** *Nucleic Acis Research,* 2011, vol. 39, 9275
- **GASIUNAS et al.** *Proc. Natl. Acad. Sci. USA,* 2012, vol. 109, E2579
- *Codon Usage Database,* 09 July 2002, www.kazusa.orjp/codon
- **ANDERSON.** *Science,* 1992, vol. 256, 808-813
- **NABEL ; FELGNER.** *TIBTECH,* 1993, vol. 11, 211-217
- **MITANI ; CASKEY.** *TIBTECH,* 1993, vol. 11, 162-166
- **DILLON.** *TIBTECH,* 1993, vol. 11, 167-175
- **MILLER.** *Nature,* 1992, vol. 357, 455-460
- **VAN BRUNT.** *Biotechnology,* 1988, vol. 6 (10), 1149-1154

- **VIGNE.** *Restorative Neurology and Neuroscience,* 1995, vol. 8, 35-36
- **KREMER ; PERRICAUDET.** *British Medical Bulletin,* 1995, vol. 51 (1), 31-44
- **HADDADA et al.** Current Topics in Microbiology and Immunology. 1995
- **YU et al.** *Gene Therapy,* 1994, vol. 1, 13-26
- **CRYSTAL.** *Science,* 1995, vol. 270, 404-410
- **BLAESE et al.** *Cancer Gene Ther.,* 1995, vol. 2, 291-297
- **BEHR et al.** *Bioconjugate Chem.,* 1994, vol. 5, 382-389
- **REMY et al.** *Bioconjugate Chem.,* 1994, vol. 5, 647-654
- **GAO et al.** *Gene Therapy,* 1995, vol. 2, 710-722
- **AHMAD et al.** *Cancer Res.,* 1992, vol. 52, 4817-4820
- **BUCHSCHER et al.** *J. Virol.,* 1992, vol. 66, 2731-2739
- **JOHANN et al.** *J. Virol.,* 1992, vol. 66, 1635-1640
- **SOMMNERFELT et al.** *Virol.,* 1990, vol. 176, 58-59
- **WILSON et al.** *J. Virol.,* 1989, vol. 63, 2374-2378
- **MILLER et al.** *J. Virol.,* 1991, vol. 65, 2220-2224
- **JONKERS et al.** *Am. J. Vet. Res.,* 1964, vol. 25, 236-242
- **TRAVASSOS DA ROSA et al.** *Am. J. Tropical Med. & Hygiene,* 1984, vol. 33, 999-1006
- **WEST et al.** *Virology,* 1987, vol. 160, 38-47
- **KOTIN.** *Human Gene Therapy,* 1994, vol. 5, 793-801
- **MUZYCZKA.** *J. Clin. Invest.,* 1994, vol. 94, 1351
- **TRATSCHIN et al.** *Mol. Cell. Biol.,* 1985, vol. 5, 3251-3260
- **TRATSCHIN et al.** *Mol. Cell. Biol.,* 1984, vol. 4, 2072-2081
- **HERMONAT ; MUZYCZKA.** *PNAS,* 1984, vol. 81, 6466-6470
- **SAMULSKI et al.** *J. Virol.,* 1989, vol. 63, 03822-3828
- **GRIMM, D. et al.** *J. Virol.,* 2008, vol. 82, 5887-5911
- **SAMBROOK et al.** Nonradioactive In Situ Hybridization Application Manual. 1989
- **CHAN-HUI et al.** *Clinical Immunology,* 2003, vol. 111, 162-174
- **ROBERT D. WELLS ; TETSUO ASHIZAWA.** Genetic Instabilities and Neurological Diseases. Academic Press, 13 October 2011
- **MCIVOR EI ; POLAK U ; NAPIERALA M.** New insights into repeat instability: role of RNA•DNA hybrids. *RNA Biol.,* September 2010, vol. 7 (5), 551-8
- Genetics of Epilepsy and Genetic Epilepsies. Mariani Foundation Paediatric Neurology, 2009, 20
- **FREITAG et al.** *Eur. Child. Adolesc. Psychiatry,* 2010, vol. 19, 169-178
- **BUCAN et al.** *PLoS Genetics,* 2009, vol. 5, e1000536
- **WARING et al.** *Arch. Neurol.,* 2008, vol. 65, 329-334
- **MORRELL et al.** Crop genomics:advances and applications. *Nat Rev Genet.,* 29 December 2011, vol. 13 (2), 85-96
- **GRUBER et al.** *Nucleic Acids Research,* 2008, vol. 36, W70
- **SAPRANAUSAKS et al.** *Nucleic Acids Resch,* 2011, vol. 39, 9275
- **JINEK et al.** *Science,* 2012, vol. 337, 816
- **MAKAROVA et al.** *Nat Rev Microbiol,* 2011, vol. 9, 467
- **PLOSKER GL et al.** Fluvastatin: a review of its pharmacology and use in the management of hypercholesterolaemia. *Drugs,* 1996, vol. 51 (3), 433-459
- **TRAPANI et al.** Potential role of nonstatin cholesterol lowering agents. *IUBMB Life,* November 2011, vol. 63 (11), 964-971
- **BIRCH AM et al.** DGAT1 inhibitors as anti-obesity and anti-diabetic agents. *Current Opinion in Drug Discovery & Development,* 2010, vol. 13 (4), 489-496
- **FUCHS et al.** Killing of leukemic cells with a BCR/ABL fusion gene by RNA interference (RNAi). *Oncogene,* 2002, vol. 21 (37), 5716-5724
- **MCMANAMAN JL.** Perilipin-2 Null Mice are Protected Against Diet-Induced Obesity, Adipose Inflammation and Fatty Liver Disease. *THE JOURNAL OF LIPID RESEARCH,* 12 February 2013
- **TANG J et al.** Inhibition of SREBP by a Small Molecule, Betulin, Improves Hyperlipidemia and Insulin Resistance and Reduces Atherosclerotic Plaques. *Cell Metabolism,* 05 January 2011, vol. 13 (1), 44-56
- **JOAO et al.** Transthyretin mutations in health and disease. *Human Mutation,* 1995, vol. 5 (3), 191-196
- **SHALEK et al.** *Nano Letters,* 2012
- **PARDRIDGE et al.** *Cold Spring Harb Protoc,* 2010
- **XUE, W. et al.** *Nature,* 2014
- **YIN, H. et al.** *Nat. Biotechnol.,* 2014, vol. 32, 551-3
- **HECKL, D. et al.** *Nat. Biotechnol.,* 2014
- **PEREZ, E. E. et al.** *Nat. Biotechnol.,* 2008, vol. 26, 808-16
- **MILLER, J. C. et al.** *Nat. Biotechnol.,* 2011, vol. 29, 143-8
- **CONG, L. et al.** *Science,* 2013
- **MALI, P. et al.** *Science,* 2013, vol. 339, 823-6

- **DELTCHEVA, E. et al.** *Nature,* 2011, vol. 471, 602-7
- **JINEK, M. et al.** *Science,* 2012, vol. 337, 816-21
- **GASIUNAS, G. et al.** *Proc. Natl. Acad. Sci. U. S. A.,* 2012, vol. 109, E2579-86
- **XIAO, A. et al.** *Nucleic Acids Res.,* 2013, vol. 41, e141
- **SHALEM, O. et al.** *Science,* 2014, vol. 343, 84-7
- **SANJANA, N. E. et al.** *Nat. Methods,* 2014, vol. 11, 783-784
- **KUMAR, M. et al.** *Hum. Gene Ther.,* 2001, vol. 12, 1893-905
- **LEWANDOSKI, M. et al.** *Cold Spring Harb. Symp. Quant. Biol.,* 1997, vol. 62, 159-68
- **OKADA, S. et al.** *J. Cell. Physiol.,* 2011, vol. 226, 552-8
- **MELLMAN, I. ; STEINMAN, R. M.** *Cell,* 2001, vol. 106, 255-8
- **AMIT, I. et al.** *Science,* 2009, vol. 326, 257-63
- **CHEVRIER, N. et al.** *Cell,* 2011, vol. 147, 853-67
- **GARBER, M. et al.** *Mol. Cell,* 2012, vol. 47, 810-22
- **SHALEK, A. K. et al.** *Nature,* 2013, vol. 498, 236-40
- **SHALEK, A. K. et al.** *Nano Lett.,* 2012, vol. 12, 6498-504
- Care and Use of Laboratory Animals. National Research Council of the National Academies
- **HEYER, M. P. et al.** Genomics. John Wiley & Sons, Ltd, 2010, 211-248
- **MADISEN, L. et al.** *Nat. Neurosci.,* 2010, vol. 13, 133-40
- **HAN, K. et al.** *Nature,* 2013, vol. 503, 72-7
- **PEÇA, J. et al.** *Nature,* 2011, vol. 472 (7344), 437-42
- **HSU, P. D. et al.** *Nat. Biotechnol.,* 2013, vol. 31, 827-32
- **AMIT, I. et al.** *Science,* vol. 326, 257-63
- **GEISS, G. K. et al.** *Nat. Biotechnol.,* 2008, vol. 26, 317-25

SEQUENCE LISTING

<110> THE BROAD INSTITUTE INC.
MASSACHUSETTS INSTITUTE OF TECHNOLOGY

<120> DELIVERY, USE AND THERAPEUTIC APPLICATIONS OF THE CRISPR-CAS SYSTEMS AND COMPOSITIONS FOR TARGETING DISORDERS AND DISEASES USING PARTICLE DELIVERY COMPONENTS

<130> 47627.99.2060

<140> PCT/US2014/070057
<141> 2014-12-12

<150> 62/054,490
<151> 2014-09-24

<150> 62/010,441
<151> 2014-06-10

<150> 61/915,118
<151> 2013-12-12

<150> 61/915,215
<151> 2013-12-12

<150> 61/915,148
<151> 2013-12-12

<160> 317

<170> PatentIn version 3.5

<210> 1
<211> 23
<212> DNA
<213> Artificial Sequence

<220>
<221> source
<223> /note="Description of Artificial Sequence: Synthetic oligonucleotide"

<220>
<221> modified_base
<222> (1)..(20)
<223> a, c, t or g

<220>
<221> modified_base
<222> (21)..(21)
<223> a, c, t, g, unknown or other

<400> 1
nnnnnnnnnn nnnnnnnnnn ngg 23

<210> 2
<211> 15
<212> DNA
<213> Artificial Sequence

<220>
<221> source
<223> /note="Description of Artificial Sequence: Synthetic
oligonucleotide"

<220>
<221> modified_base
<222> (1)..(12)
<223> a, c, t or g

<220>
<221> modified_base
<222> (13)..(13)
<223> a, c, t, g, unknown or other

<400> 2
nnnnnnnnnn nnngg 15

<210> 3
<211> 23
<212> DNA
<213> Artificial Sequence

<220>
<221> source
<223> /note="Description of Artificial Sequence: Synthetic
oligonucleotide"

<220>
<221> modified_base
<222> (1)..(20)
<223> a, c, t or g

<220>
<221> modified_base
<222> (21)..(21)
<223> a, c, t, g, unknown or other

<400> 3
nnnnnnnnnn nnnnnnnnnn ngg 23

<210> 4
<211> 14
<212> DNA
<213> Artificial Sequence

<220>
<221> source
<223> /note="Description of Artificial Sequence: Synthetic
oligonucleotide"

<220>
<221> modified_base
<222> (1)..(11)
<223> a, c, t or g

<220>
<221> modified_base

<222> (12)..(12)
<223> a, c, t, g, unknown or other

<400> 4
nnnnnnnnnn nngg 14

<210> 5
<211> 27
<212> DNA
<213> Artificial Sequence

<220>
<221> source
<223> /note="Description of Artificial Sequence: Synthetic
oligonucleotide"

<220>
<221> modified_base
<222> (1)..(20)
<223> a, c, t or g

<220>
<221> modified_base
<222> (21)..(22)
<223> a, c, t, g, unknown or other

<400> 5
nnnnnnnnnn nnnnnnnnnn nnagaaw 27

<210> 6
<211> 19
<212> DNA
<213> Artificial Sequence

<220>
<221> source
<223> /note="Description of Artificial Sequence: Synthetic
oligonucleotide"

<220>
<221> modified_base
<222> (1)..(12)
<223> a, c, t or g

<220>
<221> modified_base
<222> (13)..(14)
<223> a, c, t, g, unknown or other

<400> 6
nnnnnnnnnn nnnnagaaw 19

<210> 7
<211> 27
<212> DNA
<213> Artificial Sequence

<220>

<221> source
<223> /note="Description of Artificial Sequence: Synthetic oligonucleotide"

<220>
<221> modified_base
<222> (1)..(20)
<223> a, c, t or g

<220>
<221> modified_base
<222> (21)..(22)
<223> a, c, t, g, unknown or other

<400> 7
nnnnnnnnnn nnnnnnnnnn nnagaaw 27

<210> 8
<211> 18
<212> DNA
<213> Artificial Sequence

<220>
<221> source
<223> /note="Description of Artificial Sequence: Synthetic oligonucleotide"

<220>
<221> modified_base
<222> (1)..(11)
<223> a, c, t or g

<220>
<221> modified_base
<222> (12)..(13)
<223> a, c, t, g, unknown or other

<400> 8
nnnnnnnnnn nnnagaaw 18

<210> 9
<211> 25
<212> DNA
<213> Artificial Sequence

<220>
<221> source
<223> /note="Description of Artificial Sequence: Synthetic oligonucleotide"

<220>
<221> modified_base
<222> (1)..(20)
<223> a, c, t or g

<220>
<221> modified_base
<222> (21)..(21)

<223> a, c, t, g, unknown or other

<220>
<221> modified_base
<222> (24)..(24)
<223> a, c, t, g, unknown or other

<400> 9
nnnnnnnnnn nnnnnnnnnn nggng 25

<210> 10
<211> 17
<212> DNA
<213> Artificial Sequence

<220>
<221> source
<223> /note="Description of Artificial Sequence: Synthetic
oligonucleotide"

<220>
<221> modified_base
<222> (1)..(12)
<223> a, c, t or g

<220>
<221> modified_base
<222> (13)..(13)
<223> a, c, t, g, unknown or other

<220>
<221> modified_base
<222> (16)..(16)
<223> a, c, t, g, unknown or other

<400> 10
nnnnnnnnnn nnnggng 17

<210> 11
<211> 25
<212> DNA
<213> Artificial Sequence

<220>
<221> source
<223> /note="Description of Artificial Sequence: Synthetic
oligonucleotide"

<220>
<221> modified_base
<222> (1)..(20)
<223> a, c, t or g

<220>
<221> modified_base
<222> (21)..(21)
<223> a, c, t, g, unknown or other

<220>

<221> modified_base
<222> (24)..(24)
<223> a, c, t, g, unknown or other

<400> 11
nnnnnnnnnn nnnnnnnnnn nggng 25

<210> 12
<211> 16
<212> DNA
<213> Artificial Sequence

<220>
<221> source
<223> /note="Description of Artificial Sequence: Synthetic oligonucleotide"

<220>
<221> modified_base
<222> (1)..(11)
<223> a, c, t or g

<220>
<221> modified_base
<222> (12)..(12)
<223> a, c, t, g, unknown or other

<220>
<221> modified_base
<222> (15)..(15)
<223> a, c, t, g, unknown or other

<400> 12
nnnnnnnnnn nnggng 16

<210> 13
<211> 137
<212> DNA
<213> Artificial Sequence

<220>
<221> source
<223> /note="Description of Artificial Sequence: Synthetic polynucleotide"

<220>
<221> modified_base
<222> (1)..(20)
<223> a, c, t, g, unknown or other

<400> 13
nnnnnnnnnn nnnnnnnnnn gtttttgtac tctcaagatt tagaaataaa tcttgcagaa 60

gctacaaaga taaggcttca tgccgaaatc aacaccctgt cattttatgg cagggtgttt 120

tcgttattta atttttt 137

<210> 14

<211> 123
<212> DNA
<213> Artificial Sequence

<220>
<221> source
<223> /note="Description of Artificial Sequence: Synthetic
polynucleotide"

<220>
<221> modified_base
<222> (1)..(20)
<223> a, c, t, g, unknown or other

<400> 14
nnnnnnnnnn nnnnnnnnnn gtttttgtac tctcagaaat gcagaagcta caaagataag 60

gcttcatgcc gaaatcaaca ccctgtcatt ttatggcagg gtgttttcgt tatttaattt 120

ttt 123

<210> 15
<211> 110
<212> DNA
<213> Artificial Sequence

<220>
<221> source
<223> /note="Description of Artificial Sequence: Synthetic
polynucleotide"

<220>
<221> modified_base
<222> (1)..(20)
<223> a, c, t, g, unknown or other

<400> 15
nnnnnnnnnn nnnnnnnnnn gtttttgtac tctcagaaat gcagaagcta caaagataag 60

gcttcatgcc gaaatcaaca ccctgtcatt ttatggcagg gtgttttttt 110

<210> 16
<211> 102
<212> DNA
<213> Artificial Sequence

<220>
<221> source
<223> /note="Description of Artificial Sequence: Synthetic
polynucleotide"

<220>
<221> modified_base
<222> (1)..(20)
<223> a, c, t, g, unknown or other

<400> 16
nnnnnnnnnn nnnnnnnnnn gttttagagc tagaaatagc aagttaaaat aaggctagtc 60

cgttatcaac ttgaaaaagt ggcaccgagt cggtgctttt tt 102

<210> 17
<211> 88
<212> DNA
<213> Artificial Sequence

<220>
<221> source
<223> /note="Description of Artificial Sequence: Synthetic oligonucleotide"

<220>
<221> modified_base
<222> (1)..(20)
<223> a, c, t, g, unknown or other

<400> 17
nnnnnnnnnn nnnnnnnnnn gttttagagc tagaaatagc aagttaaaat aaggctagtc 60

cgttatcaac ttgaaaaagt gttttttt 88

<210> 18
<211> 76
<212> DNA
<213> Artificial Sequence

<220>
<221> source
<223> /note="Description of Artificial Sequence: Synthetic oligonucleotide"

<220>
<221> modified_base
<222> (1)..(20)
<223> a, c, t, g, unknown or other

<400> 18
nnnnnnnnnn nnnnnnnnnn gttttagagc tagaaatagc aagttaaaat aaggctagtc 60

cgttatcatt tttttt 76

<210> 19
<211> 20
<212> DNA
<213> Homo sapiens

<400> 19
gagtccgagc agaagaagaa 20

<210> 20
<211> 20
<212> DNA
<213> Homo sapiens

<400> 20

gagtcctagc aggagaagaa 20

<210> 21
<211> 20
<212> DNA
<213> Homo sapiens

<400> 21
gagtctaagc agaagaagaa 20

<210> 22
<211> 7
<212> PRT
<213> Unknown

<220>
<221> source
<223> /note="Description of Unknown:
Simian virus 40"

<400> 22
Pro Lys Lys Lys Arg Lys Val
1 5

<210> 23
<211> 16
<212> PRT
<213> Unknown

<220>
<221> source
<223> /note="Description of Unknown:
Nucleoplasmin bipartite NLS sequence"

<400> 23
Lys Arg Pro Ala Ala Thr Lys Lys Ala Gly Gln Ala Lys Lys Lys Lys
1 5 10 15

<210> 24
<211> 9
<212> PRT
<213> Unknown

<220>
<221> source
<223> /note="Description of Unknown:
C-myc NLS sequence"

<400> 24
Pro Ala Ala Lys Arg Val Lys Leu Asp
1 5

<210> 25
<211> 11
<212> PRT
<213> Unknown

<220>

<221> source
<223> /note="Description of Unknown:
C-myc NLS sequence"

<400> 25
Arg Gln Arg Arg Asn Glu Leu Lys Arg Ser Pro
1               5                   10

<210> 26
<211> 38
<212> PRT
<213> Homo sapiens

<400> 26
Asn Gln Ser Ser Asn Phe Gly Pro Met Lys Gly Gly Asn Phe Gly Gly
1               5                   10                  15

Arg Ser Ser Gly Pro Tyr Gly Gly Gly Gly Gln Tyr Phe Ala Lys Pro
            20                  25                  30

Arg Asn Gln Gly Gly Tyr
        35

<210> 27
<211> 42
<212> PRT
<213> Unknown

<220>
<221> source
<223> /note="Description of Unknown:
IBB domain from importin-alpha sequence"

<400> 27
Arg Met Arg Ile Glx Phe Lys Asn Lys Gly Lys Asp Thr Ala Glu Leu
1               5                   10                  15

Arg Arg Arg Arg Val Glu Val Ser Val Glu Leu Arg Lys Ala Lys Lys
            20                  25                  30

Asp Glu Gln Ile Leu Lys Arg Arg Asn Val
        35                  40

<210> 28
<211> 8
<212> PRT
<213> Unknown

<220>
<221> source
<223> /note="Description of Unknown:
Myoma T protein sequence"

<400> 28
Val Ser Arg Lys Arg Pro Arg Pro
1               5

<210> 29
<211> 8
<212> PRT
<213> Unknown

<220>
<221> source
<223> /note="Description of Unknown:
Myoma T protein sequence"

<400> 29
Pro Pro Lys Lys Ala Arg Glu Asp
1 5

<210> 30
<211> 8
<212> PRT
<213> Homo sapiens

<400> 30
Pro Gln Pro Lys Lys Lys Pro Leu
1 5

<210> 31
<211> 12
<212> PRT
<213> Mus musculus

<400> 31
Ser Ala Leu Ile Lys Lys Lys Lys Lys Met Ala Pro
1 5 10

<210> 32
<211> 5
<212> PRT
<213> Influenza virus

<400> 32
Asp Arg Leu Arg Arg
1 5

<210> 33
<211> 7
<212> PRT
<213> Influenza virus

<400> 33
Pro Lys Gln Lys Lys Arg Lys
1 5

<210> 34
<211> 10
<212> PRT
<213> Hepatitis delta virus

<400> 34

Arg Lys Leu Lys Lys Lys Ile Lys Lys Leu
1               5                   10

<210> 35
<211> 10
<212> PRT
<213> Mus musculus

<400> 35
Arg Glu Lys Lys Lys Phe Leu Lys Arg Arg
1               5                   10

<210> 36
<211> 20
<212> PRT
<213> Homo sapiens

<400> 36
Lys Arg Lys Gly Asp Glu Val Asp Gly Val Asp Glu Val Ala Lys Lys
1               5                   10                  15

Lys Ser Lys Lys
            20

<210> 37
<211> 17
<212> PRT
<213> Homo sapiens

<400> 37
Arg Lys Cys Leu Gln Ala Gly Met Asn Leu Glu Ala Arg Lys Thr Lys
1               5                   10                  15

Lys

<210> 38
<211> 60
<212> RNA
<213> Artificial Sequence

<220>
<221> source
<223> /note="Description of Artificial Sequence: Synthetic oligonucleotide"

<220>
<221> modified_base
<222> (1)..(20)
<223> a, c, u, g, unknown or other

<220>
<221> modified_base
<222> (23)..(44)
<223> a, c, u, g, unknown or other

<220>
<221> modified_base
<222> (47)..(60)
<223> a, c, u, g, unknown or other

<400> 38
nnnnnnnnnn nnnnnnnnnn ccnnnnnnnn nnnnnnnnnn nnnnggnnnn nnnnnnnnnn 60

<210> 39
<211> 60
<212> RNA
<213> Artificial Sequence

<220>
<221> source
<223> /note="Description of Artificial Sequence: Synthetic oligonucleotide"

<220>
<221> modified_base
<222> (1)..(14)
<223> a, c, u, g, unknown or other

<220>
<221> modified_base
<222> (17)..(38)
<223> a, c, u, g, unknown or other

<220>
<221> modified_base
<222> (41)..(60)
<223> a, c, u, g, unknown or other

<400> 39
nnnnnnnnnn nnnnccnnnn nnnnnnnnnn nnnnnnnngg nnnnnnnnnn nnnnnnnnnn 60

<210> 40
<211> 60
<212> RNA
<213> Artificial Sequence

<220>
<221> source
<223> /note="Description of Artificial Sequence: Synthetic oligonucleotide"

<220>
<221> modified_base
<222> (1)..(20)
<223> a, c, u, g, unknown or other

<220>
<221> modified_base
<222> (23)..(43)
<223> a, c, u, g, unknown or other

<220>
<221> modified_base
<222> (46)..(60)

<223> a, c, u, g, unknown or other

<400> 40
nnnnnnnnnn nnnnnnnnnn ccnnnnnnnn nnnnnnnnnn nnnggnnnnn nnnnnnnnnn 60

<210> 41
<211> 60
<212> RNA
<213> Artificial Sequence

<220>
<221> source
<223> /note="Description of Artificial Sequence: Synthetic
oligonucleotide"

<220>
<221> modified_base
<222> (1)..(15)
<223> a, c, u, g, unknown or other

<220>
<221> modified_base
<222> (18)..(38)
<223> a, c, u, g, unknown or other

<220>
<221> modified_base
<222> (41)..(60)
<223> a, c, u, g, unknown or other

<400> 41
nnnnnnnnnn nnnnnccnnn nnnnnnnnnn nnnnnnnngg nnnnnnnnnn nnnnnnnnnn 60

<210> 42
<211> 60
<212> RNA
<213> Artificial Sequence

<220>
<221> source
<223> /note="Description of Artificial Sequence: Synthetic
oligonucleotide"

<220>
<221> modified_base
<222> (1)..(20)
<223> a, c, u, g, unknown or other

<220>
<221> modified_base
<222> (23)..(42)
<223> a, c, u, g, unknown or other

<220>
<221> modified_base
<222> (45)..(60)
<223> a, c, u, g, unknown or other

<400> 42

nnnnnnnnnn nnnnnnnnnn ccnnnnnnnn nnnnnnnnnn nnggnnnnnn nnnnnnnnnn 60

<210> 43
<211> 60
<212> RNA
<213> Artificial Sequence

<220>
<221> source
<223> /note="Description of Artificial Sequence: Synthetic oligonucleotide"

<220>
<221> modified_base
<222> (1)..(15)
<223> a, c, u, g, unknown or other

<220>
<221> modified_base
<222> (18)..(38)
<223> a, c, u, g, unknown or other

<220>
<221> modified_base
<222> (41)..(60)
<223> a, c, u, g, unknown or other

<400> 43
nnnnnnnnnn nnnnnccnnn nnnnnnnnnn nnnnnnnngg nnnnnnnnnn nnnnnnnnnn 60

<210> 44
<211> 60
<212> RNA
<213> Artificial Sequence

<220>
<221> source
<223> /note="Description of Artificial Sequence: Synthetic oligonucleotide"

<220>
<221> modified_base
<222> (1)..(20)
<223> a, c, u, g, unknown or other

<220>
<221> modified_base
<222> (23)..(41)
<223> a, c, u, g, unknown or other

<220>
<221> modified_base
<222> (44)..(60)
<223> a, c, u, g, unknown or other

<400> 44
nnnnnnnnnn nnnnnnnnnn ccnnnnnnnn nnnnnnnnnn nggnnnnnnn nnnnnnnnnn 60

<210> 45
<211> 60
<212> RNA
<213> Artificial Sequence

<220>
<221> source
<223> /note="Description of Artificial Sequence: Synthetic oligonucleotide"

<220>
<221> modified_base
<222> (1)..(17)
<223> a, c, u, g, unknown or other

<220>
<221> modified_base
<222> (20)..(38)
<223> a, c, u, g, unknown or other

<220>
<221> modified_base
<222> (41)..(60)
<223> a, c, u, g, unknown or other

<400> 45
nnnnnnnnnn nnnnnnnccn nnnnnnnnnn nnnnnnnngg nnnnnnnnnn nnnnnnnnnn 60

<210> 46
<211> 60
<212> RNA
<213> Artificial Sequence

<220>
<221> source
<223> /note="Description of Artificial Sequence: Synthetic oligonucleotide"

<220>
<221> modified_base
<222> (1)..(20)
<223> a, c, u, g, unknown or other

<220>
<221> modified_base
<222> (23)..(40)
<223> a, c, u, g, unknown or other

<220>
<221> modified_base
<222> (43)..(60)
<223> a, c, u, g, unknown or other

<400> 46
nnnnnnnnnn nnnnnnnnnn ccnnnnnnnn nnnnnnnnnn ggnnnnnnnn nnnnnnnnnn 60

<210> 47
<211> 60
<212> RNA

<213> Artificial Sequence

<220>
<221> source
<223> /note="Description of Artificial Sequence: Synthetic oligonucleotide"

<220>
<221> modified_base
<222> (1)..(18)
<223> a, c, u, g, unknown or other

<220>
<221> modified_base
<222> (21)..(38)
<223> a, c, u, g, unknown or other

<220>
<221> modified_base
<222> (41)..(60)
<223> a, c, u, g, unknown or other

<400> 47
nnnnnnnnnn nnnnnnnncc nnnnnnnnnn nnnnnnnngg nnnnnnnnnn nnnnnnnnnn 60

<210> 48
<211> 60
<212> RNA
<213> Artificial Sequence

<220>
<221> source
<223> /note="Description of Artificial Sequence: Synthetic oligonucleotide"

<220>
<221> modified_base
<222> (1)..(20)
<223> a, c, u, g, unknown or other

<220>
<221> modified_base
<222> (23)..(39)
<223> a, c, u, g, unknown or other

<220>
<221> modified_base
<222> (42)..(60)
<223> a, c, u, g, unknown or other

<400> 48
nnnnnnnnnn nnnnnnnnnn ccnnnnnnnn nnnnnnnnng gnnnnnnnnn nnnnnnnnnn 60

<210> 49
<211> 60
<212> RNA
<213> Artificial Sequence

<220>

<221> source
<223> /note="Description of Artificial Sequence: Synthetic oligonucleotide"

<220>
<221> modified_base
<222> (1)..(19)
<223> a, c, u, g, unknown or other

<220>
<221> modified_base
<222> (22)..(38)
<223> a, c, u, g, unknown or other

<220>
<221> modified_base
<222> (41)..(60)
<223> a, c, u, g, unknown or other

<400> 49
nnnnnnnnnn nnnnnnnnnc cnnnnnnnnn nnnnnnnngg nnnnnnnnnn nnnnnnnnnn 60

<210> 50
<211> 60
<212> RNA
<213> Artificial Sequence

<220>
<221> source
<223> /note="Description of Artificial Sequence: Synthetic oligonucleotide"

<220>
<221> modified_base
<222> (1)..(20)
<223> a, c, u, g, unknown or other

<220>
<221> modified_base
<222> (23)..(38)
<223> a, c, u, g, unknown or other

<220>
<221> modified_base
<222> (41)..(60)
<223> a, c, u, g, unknown or other

<400> 50
nnnnnnnnnn nnnnnnnnnn ccnnnnnnnn nnnnnnnngg nnnnnnnnnn nnnnnnnnnn 60

<210> 51
<211> 60
<212> RNA
<213> Artificial Sequence

<220>
<221> source
<223> /note="Description of Artificial Sequence: Synthetic oligonucleotide"

<220>
<221> modified_base
<222> (1)..(20)
<223> a, c, u, g, unknown or other

<220>
<221> modified_base
<222> (23)..(38)
<223> a, c, u, g, unknown or other

<220>
<221> modified_base
<222> (41)..(60)
<223> a, c, u, g, unknown or other

<400> 51
nnnnnnnnnn nnnnnnnnnn ccnnnnnnnn nnnnnnnngg nnnnnnnnnn nnnnnnnnnn 60

<210> 52
<211> 60
<212> RNA
<213> Artificial Sequence

<220>
<221> source
<223> /note="Description of Artificial Sequence: Synthetic oligonucleotide"

<220>
<221> modified_base
<222> (1)..(20)
<223> a, c, u, g, unknown or other

<220>
<221> modified_base
<222> (23)..(37)
<223> a, c, u, g, unknown or other

<220>
<221> modified_base
<222> (40)..(60)
<223> a, c, u, g, unknown or other

<400> 52
nnnnnnnnnn nnnnnnnnnn ccnnnnnnnn nnnnnnnggn nnnnnnnnnn nnnnnnnnnn 60

<210> 53
<211> 60
<212> RNA
<213> Artificial Sequence

<220>
<221> source
<223> /note="Description of Artificial Sequence: Synthetic oligonucleotide"

<220>

<221> modified_base
<222> (1)..(21)
<223> a, c, u, g, unknown or other

<220>
<221> modified_base
<222> (24)..(38)
<223> a, c, u, g, unknown or other

<220>
<221> modified_base
<222> (41)..(60)
<223> a, c, u, g, unknown or other

<400> 53
nnnnnnnnnn nnnnnnnnnn nccnnnnnnn nnnnnnnngg nnnnnnnnnn nnnnnnnnnn 60

<210> 54
<211> 60
<212> RNA
<213> Artificial Sequence

<220>
<221> source
<223> /note="Description of Artificial Sequence: Synthetic oligonucleotide"

<220>
<221> modified_base
<222> (1)..(20)
<223> a, c, u, g, unknown or other

<220>
<221> modified_base
<222> (23)..(36)
<223> a, c, u, g, unknown or other

<220>
<221> modified_base
<222> (39)..(60)
<223> a, c, u, g, unknown or other

<400> 54
nnnnnnnnnn nnnnnnnnnn ccnnnnnnnn nnnnnnggnn nnnnnnnnnn nnnnnnnnnn 60

<210> 55
<211> 60
<212> RNA
<213> Artificial Sequence

<220>
<221> source
<223> /note="Description of Artificial Sequence: Synthetic oligonucleotide"

<220>
<221> modified_base
<222> (1)..(22)
<223> a, c, u, g, unknown or other

<220>
<221> modified_base
<222> (25)..(38)
<223> a, c, u, g, unknown or other

<220>
<221> modified_base
<222> (41)..(60)
<223> a, c, u, g, unknown or other

<400> 55
nnnnnnnnnn nnnnnnnnnn nnccnnnnnn nnnnnnnngg nnnnnnnnnn nnnnnnnnnn 60

<210> 56
<211> 60
<212> RNA
<213> Artificial Sequence

<220>
<221> source
<223> /note="Description of Artificial Sequence: Synthetic oligonucleotide"

<220>
<221> modified_base
<222> (1)..(20)
<223> a, c, u, g, unknown or other

<220>
<221> modified_base
<222> (23)..(35)
<223> a, c, u, g, unknown or other

<220>
<221> modified_base
<222> (38)..(60)
<223> a, c, u, g, unknown or other

<400> 56
nnnnnnnnnn nnnnnnnnnn ccnnnnnnnn nnnnnggnnn nnnnnnnnnn nnnnnnnnnn 60

<210> 57
<211> 60
<212> RNA
<213> Artificial Sequence

<220>
<221> source
<223> /note="Description of Artificial Sequence: Synthetic oligonucleotide"

<220>
<221> modified_base
<222> (1)..(23)
<223> a, c, u, g, unknown or other

<220>
<221> modified_base

<222> (26)..(38)
<223> a, c, u, g, unknown or other

<220>
<221> modified_base
<222> (41)..(60)
<223> a, c, u, g, unknown or other

<400> 57
nnnnnnnnnn nnnnnnnnnn nnnccnnnnn nnnnnnnngg nnnnnnnnnn nnnnnnnnnn 60

<210> 58
<211> 60
<212> RNA
<213> Artificial Sequence

<220>
<221> source
<223> /note="Description of Artificial Sequence: Synthetic oligonucleotide"

<220>
<221> modified_base
<222> (1)..(20)
<223> a, c, u, g, unknown or other

<220>
<221> modified_base
<222> (23)..(34)
<223> a, c, u, g, unknown or other

<220>
<221> modified_base
<222> (37)..(60)
<223> a, c, u, g, unknown or other

<400> 58
nnnnnnnnnn nnnnnnnnnn ccnnnnnnnn nnnnggnnnn nnnnnnnnnn nnnnnnnnnn 60

<210> 59
<211> 60
<212> RNA
<213> Artificial Sequence

<220>
<221> source
<223> /note="Description of Artificial Sequence: Synthetic oligonucleotide"

<220>
<221> modified_base
<222> (1)..(24)
<223> a, c, u, g, unknown or other

<220>
<221> modified_base
<222> (27)..(38)
<223> a, c, u, g, unknown or other

<220>
<221> modified_base
<222> (41)..(60)
<223> a, c, u, g, unknown or other

<400> 59
nnnnnnnnnn nnnnnnnnnn nnnnccnnnn nnnnnnnngg nnnnnnnnnn nnnnnnnnnn 60

<210> 60
<211> 60
<212> RNA
<213> Artificial Sequence

<220>
<221> source
<223> /note="Description of Artificial Sequence: Synthetic oligonucleotide"

<220>
<221> modified_base
<222> (1)..(20)
<223> a, c, u, g, unknown or other

<220>
<221> modified_base
<222> (23)..(33)
<223> a, c, u, g, unknown or other

<220>
<221> modified_base
<222> (36)..(60)
<223> a, c, u, g, unknown or other

<400> 60
nnnnnnnnnn nnnnnnnnnn ccnnnnnnnn nnnggnnnnn nnnnnnnnnn nnnnnnnnnn 60

<210> 61
<211> 60
<212> RNA
<213> Artificial Sequence

<220>
<221> source
<223> /note="Description of Artificial Sequence: Synthetic oligonucleotide"

<220>
<221> modified_base
<222> (1)..(25)
<223> a, c, u, g, unknown or other

<220>
<221> modified_base
<222> (28)..(38)
<223> a, c, u, g, unknown or other

<220>
<221> modified_base
<222> (41)..(60)

<223> a, c, u, g, unknown or other

<400> 61
nnnnnnnnnn nnnnnnnnnn nnnnnccnnn nnnnnnnngg nnnnnnnnnn nnnnnnnnnn 60

<210> 62
<211> 60
<212> RNA
<213> Artificial Sequence

<220>
<221> source
<223> /note="Description of Artificial Sequence: Synthetic oligonucleotide"

<220>
<221> modified_base
<222> (1)..(20)
<223> a, c, u, g, unknown or other

<220>
<221> modified_base
<222> (23)..(32)
<223> a, c, u, g, unknown or other

<220>
<221> modified_base
<222> (35)..(60)
<223> a, c, u, g, unknown or other

<400> 62
nnnnnnnnnn nnnnnnnnnn ccnnnnnnnn nnggnnnnnn nnnnnnnnnn nnnnnnnnnn 60

<210> 63
<211> 60
<212> RNA
<213> Artificial Sequence

<220>
<221> source
<223> /note="Description of Artificial Sequence: Synthetic oligonucleotide"

<220>
<221> modified_base
<222> (1)..(26)
<223> a, c, u, g, unknown or other

<220>
<221> modified_base
<222> (29)..(38)
<223> a, c, u, g, unknown or other

<220>
<221> modified_base
<222> (41)..(60)
<223> a, c, u, g, unknown or other

<400> 63

nnnnnnnnnn nnnnnnnnnn nnnnnnccnn nnnnnnnngg nnnnnnnnnn nnnnnnnnnn 60

<210> 64
<211> 60
<212> RNA
<213> Artificial Sequence

<220>
<221> source
<223> /note="Description of Artificial Sequence: Synthetic oligonucleotide"

<220>
<221> modified_base
<222> (1)..(20)
<223> a, c, u, g, unknown or other

<220>
<221> modified_base
<222> (23)..(31)
<223> a, c, u, g, unknown or other

<220>
<221> modified_base
<222> (34)..(60)
<223> a, c, u, g, unknown or other

<400> 64
nnnnnnnnnn nnnnnnnnnn ccnnnnnnnn nggnnnnnnn nnnnnnnnnn nnnnnnnnnn 60

<210> 65
<211> 60
<212> RNA
<213> Artificial Sequence

<220>
<221> source
<223> /note="Description of Artificial Sequence: Synthetic oligonucleotide"

<220>
<221> modified_base
<222> (1)..(27)
<223> a, c, u, g, unknown or other

<220>
<221> modified_base
<222> (30)..(38)
<223> a, c, u, g, unknown or other

<220>
<221> modified_base
<222> (41)..(60)
<223> a, c, u, g, unknown or other

<400> 65
nnnnnnnnnn nnnnnnnnnn nnnnnnnccn nnnnnnnngg nnnnnnnnnn nnnnnnnnnn 60

<210> 66
<211> 60
<212> RNA
<213> Artificial Sequence

<220>
<221> source
<223> /note="Description of Artificial Sequence: Synthetic oligonucleotide"

<220>
<221> modified_base
<222> (1)..(20)
<223> a, c, u, g, unknown or other

<220>
<221> modified_base
<222> (23)..(30)
<223> a, c, u, g, unknown or other

<220>
<221> modified_base
<222> (33)..(60)
<223> a, c, u, g, unknown or other

<400> 66
nnnnnnnnnn nnnnnnnnnn ccnnnnnnnn ggnnnnnnnn nnnnnnnnnn nnnnnnnnnn 60

<210> 67
<211> 60
<212> RNA
<213> Artificial Sequence

<220>
<221> source
<223> /note="Description of Artificial Sequence: Synthetic oligonucleotide"

<220>
<221> modified_base
<222> (1)..(28)
<223> a, c, u, g, unknown or other

<220>
<221> modified_base
<222> (31)..(38)
<223> a, c, u, g, unknown or other

<220>
<221> modified_base
<222> (41)..(60)
<223> a, c, u, g, unknown or other

<400> 67
nnnnnnnnnn nnnnnnnnnn nnnnnnnncc nnnnnnnngg nnnnnnnnnn nnnnnnnnnn 60

<210> 68
<211> 60
<212> RNA

<213> Artificial Sequence

<220>
<221> source
<223> /note="Description of Artificial Sequence: Synthetic
oligonucleotide"

<220>
<221> modified_base
<222> (1)..(20)
<223> a, c, u, g, unknown or other

<220>
<221> modified_base
<222> (23)..(29)
<223> a, c, u, g, unknown or other

<220>
<221> modified_base
<222> (32)..(60)
<223> a, c, u, g, unknown or other

<400> 68
nnnnnnnnnn nnnnnnnnnn ccnnnnnnng gnnnnnnnnn nnnnnnnnnn nnnnnnnnnn 60

<210> 69
<211> 60
<212> RNA
<213> Artificial Sequence

<220>
<221> source
<223> /note="Description of Artificial Sequence: Synthetic
oligonucleotide"

<220>
<221> modified_base
<222> (1)..(29)
<223> a, c, u, g, unknown or other

<220>
<221> modified_base
<222> (32)..(38)
<223> a, c, u, g, unknown or other

<220>
<221> modified_base
<222> (41)..(60)
<223> a, c, u, g, unknown or other

<400> 69
nnnnnnnnnn nnnnnnnnnn nnnnnnnnnc cnnnnnnngg nnnnnnnnnn nnnnnnnnnn 60

<210> 70
<211> 60
<212> RNA
<213> Artificial Sequence

<220>

<221> source
<223> /note="Description of Artificial Sequence: Synthetic oligonucleotide"

<220>
<221> modified_base
<222> (1)..(20)
<223> a, c, u, g, unknown or other

<220>
<221> modified_base
<222> (23)..(28)
<223> a, c, u, g, unknown or other

<220>
<221> modified_base
<222> (31)..(60)
<223> a, c, u, g, unknown or other

<400> 70
nnnnnnnnnn nnnnnnnnnn ccnnnnnngg nnnnnnnnnn nnnnnnnnnn nnnnnnnnnn 60

<210> 71
<211> 60
<212> RNA
<213> Artificial Sequence

<220>
<221> source
<223> /note="Description of Artificial Sequence: Synthetic oligonucleotide"

<220>
<221> modified_base
<222> (1)..(30)
<223> a, c, u, g, unknown or other

<220>
<221> modified_base
<222> (33)..(38)
<223> a, c, u, g, unknown or other

<220>
<221> modified_base
<222> (41)..(60)
<223> a, c, u, g, unknown or other

<400> 71
nnnnnnnnnn nnnnnnnnnn nnnnnnnnnn ccnnnnnngg nnnnnnnnnn nnnnnnnnnn 60

<210> 72
<211> 60
<212> RNA
<213> Artificial Sequence

<220>
<221> source
<223> /note="Description of Artificial Sequence: Synthetic oligonucleotide"

<220>
<221> modified_base
<222> (1)..(20)
<223> a, c, u, g, unknown or other

<220>
<221> modified_base
<222> (23)..(27)
<223> a, c, u, g, unknown or other

<220>
<221> modified_base
<222> (30)..(60)
<223> a, c, u, g, unknown or other

<400> 72
nnnnnnnnnn nnnnnnnnnn ccnnnnnggn nnnnnnnnnn nnnnnnnnnn nnnnnnnnnn 60

<210> 73
<211> 60
<212> RNA
<213> Artificial Sequence

<220>
<221> source
<223> /note="Description of Artificial Sequence: Synthetic oligonucleotide"

<220>
<221> modified_base
<222> (1)..(31)
<223> a, c, u, g, unknown or other

<220>
<221> modified_base
<222> (34)..(38)
<223> a, c, u, g, unknown or other

<220>
<221> modified_base
<222> (41)..(60)
<223> a, c, u, g, unknown or other

<400> 73
nnnnnnnnnn nnnnnnnnnn nnnnnnnnnn nccnnnnngg nnnnnnnnnn nnnnnnnnnn 60

<210> 74
<211> 60
<212> RNA
<213> Artificial Sequence

<220>
<221> source
<223> /note="Description of Artificial Sequence: Synthetic oligonucleotide"

<220>

<221> modified_base
<222> (1)..(20)
<223> a, c, u, g, unknown or other

<220>
<221> modified_base
<222> (23)..(26)
<223> a, c, u, g, unknown or other

<220>
<221> modified_base
<222> (29)..(60)
<223> a, c, u, g, unknown or other

<400> 74
nnnnnnnnnn nnnnnnnnnn ccnnnnggnn nnnnnnnnnn nnnnnnnnnn nnnnnnnnnn 60

<210> 75
<211> 60
<212> RNA
<213> Artificial Sequence

<220>
<221> source
<223> /note="Description of Artificial Sequence: Synthetic
oligonucleotide"

<220>
<221> modified_base
<222> (1)..(32)
<223> a, c, u, g, unknown or other

<220>
<221> modified_base
<222> (35)..(38)
<223> a, c, u, g, unknown or other

<220>
<221> modified_base
<222> (41)..(60)
<223> a, c, u, g, unknown or other

<400> 75
nnnnnnnnnn nnnnnnnnnn nnnnnnnnnn nnccnnnngg nnnnnnnnnn nnnnnnnnnn 60

<210> 76
<211> 60
<212> RNA
<213> Artificial Sequence

<220>
<221> source
<223> /note="Description of Artificial Sequence: Synthetic
oligonucleotide"

<220>
<221> modified_base
<222> (1)..(20)
<223> a, c, u, g, unknown or other

<220>
<221> modified_base
<222> (23)..(25)
<223> a, c, u, g, unknown or other

<220>
<221> modified_base
<222> (28)..(60)
<223> a, c, u, g, unknown or other

<400> 76
nnnnnnnnnn nnnnnnnnnn ccnnnggnnn nnnnnnnnnn nnnnnnnnnn nnnnnnnnnn 60

<210> 77
<211> 60
<212> RNA
<213> Artificial Sequence

<220>
<221> source
<223> /note="Description of Artificial Sequence: Synthetic oligonucleotide"

<220>
<221> modified_base
<222> (1)..(33)
<223> a, c, u, g, unknown or other

<220>
<221> modified_base
<222> (36)..(38)
<223> a, c, u, g, unknown or other

<220>
<221> modified_base
<222> (41)..(60)
<223> a, c, u, g, unknown or other

<400> 77
nnnnnnnnnn nnnnnnnnnn nnnnnnnnnn nnnccnnngg nnnnnnnnnn nnnnnnnnnn 60

<210> 78
<211> 60
<212> RNA
<213> Artificial Sequence

<220>
<221> source
<223> /note="Description of Artificial Sequence: Synthetic oligonucleotide"

<220>
<221> modified_base
<222> (1)..(20)
<223> a, c, u, g, unknown or other

<220>
<221> modified_base

<222> (23)..(24)
<223> a, c, u, g, unknown or other

<220>
<221> modified_base
<222> (27)..(60)
<223> a, c, u, g, unknown or other

<400> 78
nnnnnnnnnn nnnnnnnnnn ccnnggnnnn nnnnnnnnnn nnnnnnnnnn nnnnnnnnnn 60

<210> 79
<211> 60
<212> RNA
<213> Artificial Sequence

<220>
<221> source
<223> /note="Description of Artificial Sequence: Synthetic oligonucleotide"

<220>
<221> modified_base
<222> (1)..(34)
<223> a, c, u, g, unknown or other

<220>
<221> modified_base
<222> (37)..(38)
<223> a, c, u, g, unknown or other

<220>
<221> modified_base
<222> (41)..(60)
<223> a, c, u, g, unknown or other

<400> 79
nnnnnnnnnn nnnnnnnnnn nnnnnnnnnn nnnnccnngg nnnnnnnnnn nnnnnnnnnn 60

<210> 80
<211> 60
<212> RNA
<213> Artificial Sequence

<220>
<221> source
<223> /note="Description of Artificial Sequence: Synthetic oligonucleotide"

<220>
<221> modified_base
<222> (1)..(20)
<223> a, c, u, g, unknown or other

<220>
<221> modified_base
<222> (23)..(23)
<223> a, c, u, g, unknown or other

<220>
<221> modified_base
<222> (26)..(60)
<223> a, c, u, g, unknown or other

<400> 80
nnnnnnnnnn nnnnnnnnnn ccnggnnnnn nnnnnnnnnn nnnnnnnnnn nnnnnnnnnn 60

<210> 81
<211> 60
<212> RNA
<213> Artificial Sequence

<220>
<221> source
<223> /note="Description of Artificial Sequence: Synthetic
oligonucleotide"

<220>
<221> modified_base
<222> (1)..(35)
<223> a, c, u, g, unknown or other

<220>
<221> modified_base
<222> (38)..(38)
<223> a, c, u, g, unknown or other

<220>
<221> modified_base
<222> (41)..(60)
<223> a, c, u, g, unknown or other

<400> 81
nnnnnnnnnn nnnnnnnnnn nnnnnnnnnn nnnnnccngg nnnnnnnnnn nnnnnnnnnn 60

<210> 82
<211> 60
<212> RNA
<213> Artificial Sequence

<220>
<221> source
<223> /note="Description of Artificial Sequence: Synthetic
oligonucleotide"

<220>
<221> modified_base
<222> (1)..(21)
<223> a, c, u, g, unknown or other

<220>
<221> modified_base
<222> (26)..(60)
<223> a, c, u, g, unknown or other

<400> 82
nnnnnnnnnn nnnnnnnnnn nccggnnnnn nnnnnnnnnn nnnnnnnnnn nnnnnnnnnn 60

<210> 83
<211> 60
<212> RNA
<213> Artificial Sequence

<220>
<221> source
<223> /note="Description of Artificial Sequence: Synthetic
oligonucleotide"

<220>
<221> modified_base
<222> (1)..(35)
<223> a, c, u, g, unknown or other

<220>
<221> modified_base
<222> (40)..(60)
<223> a, c, u, g, unknown or other

<400> 83
nnnnnnnnnn nnnnnnnnnn nnnnnnnnnn nnnnnccggn nnnnnnnnnn nnnnnnnnnn 60

<210> 84
<211> 60
<212> RNA
<213> Artificial Sequence

<220>
<221> source
<223> /note="Description of Artificial Sequence: Synthetic
oligonucleotide"

<220>
<221> modified_base
<222> (1)..(23)
<223> a, c, u, g, unknown or other

<220>
<221> modified_base
<222> (26)..(60)
<223> a, c, u, g, unknown or other

<400> 84
nnnnnnnnnn nnnnnnnnnn nnnggnnnnn nnnnnnnnnn nnnnnnnnnn nnnnnnnnnn 60

<210> 85
<211> 60
<212> RNA
<213> Artificial Sequence

<220>
<221> source
<223> /note="Description of Artificial Sequence: Synthetic
oligonucleotide"

<220>

<221> modified_base
<222> (1)..(33)
<223> a, c, u, g, unknown or other

<220>
<221> modified_base
<222> (38)..(60)
<223> a, c, u, g, unknown or other

<400> 85
nnnnnnnnnn nnnnnnnnnn nnnnnnnnnn nnnggccnnn nnnnnnnnnn nnnnnnnnnn 60

<210> 86
<211> 60
<212> RNA
<213> Artificial Sequence

<220>
<221> source
<223> /note="Description of Artificial Sequence: Synthetic oligonucleotide"

<220>
<221> modified_base
<222> (1)..(22)
<223> a, c, u, g, unknown or other

<220>
<221> modified_base
<222> (26)..(60)
<223> a, c, u, g, unknown or other

<400> 86
nnnnnnnnnn nnnnnnnnnn nncggnnnnn nnnnnnnnnn nnnnnnnnnn nnnnnnnnnn 60

<210> 87
<211> 60
<212> RNA
<213> Artificial Sequence

<220>
<221> source
<223> /note="Description of Artificial Sequence: Synthetic oligonucleotide"

<220>
<221> modified_base
<222> (1)..(32)
<223> a, c, u, g, unknown or other

<220>
<221> modified_base
<222> (35)..(35)
<223> a, c, u, g, unknown or other

<220>
<221> modified_base
<222> (38)..(60)
<223> a, c, u, g, unknown or other

<400> 87
nnnnnnnnnn nnnnnnnnnn nnnnnnnnnn nnggnccnnn nnnnnnnnnn nnnnnnnnnn 60

<210> 88
<211> 60
<212> RNA
<213> Artificial Sequence

<220>
<221> source
<223> /note="Description of Artificial Sequence: Synthetic oligonucleotide"

<220>
<221> modified_base
<222> (1)..(31)
<223> a, c, u, g, unknown or other

<220>
<221> modified_base
<222> (34)..(35)
<223> a, c, u, g, unknown or other

<220>
<221> modified_base
<222> (38)..(60)
<223> a, c, u, g, unknown or other

<400> 88
nnnnnnnnnn nnnnnnnnnn nnnnnnnnnn nggnnccnnn nnnnnnnnnn nnnnnnnnnn 60

<210> 89
<211> 60
<212> RNA
<213> Artificial Sequence

<220>
<221> source
<223> /note="Description of Artificial Sequence: Synthetic oligonucleotide"

<220>
<221> modified_base
<222> (1)..(30)
<223> a, c, u, g, unknown or other

<220>
<221> modified_base
<222> (33)..(35)
<223> a, c, u, g, unknown or other

<220>
<221> modified_base
<222> (38)..(60)
<223> a, c, u, g, unknown or other

<400> 89
nnnnnnnnnn nnnnnnnnnn nnnnnnnnnn ggnnnccnnn nnnnnnnnnn nnnnnnnnnn 60

<210> 90
<211> 60
<212> RNA
<213> Artificial Sequence

<220>
<221> source
<223> /note="Description of Artificial Sequence: Synthetic oligonucleotide"

<220>
<221> modified_base
<222> (1)..(29)
<223> a, c, u, g, unknown or other

<220>
<221> modified_base
<222> (32)..(35)
<223> a, c, u, g, unknown or other

<220>
<221> modified_base
<222> (38)..(60)
<223> a, c, u, g, unknown or other

<400> 90
nnnnnnnnnn nnnnnnnnnn nnnnnnnnng gnnnnccnnn nnnnnnnnnn nnnnnnnnnn 60

<210> 91
<211> 60
<212> RNA
<213> Artificial Sequence

<220>
<221> source
<223> /note="Description of Artificial Sequence: Synthetic oligonucleotide"

<220>
<221> modified_base
<222> (1)..(28)
<223> a, c, u, g, unknown or other

<220>
<221> modified_base
<222> (31)..(35)
<223> a, c, u, g, unknown or other

<220>
<221> modified_base
<222> (38)..(60)
<223> a, c, u, g, unknown or other

<400> 91
nnnnnnnnnn nnnnnnnnnn nnnnnnnngg nnnnnccnnn nnnnnnnnnn nnnnnnnnnn 60

<210> 92

<211> 9
<212> PRT
<213> Unknown

<220>
<223> Description of Unknown:
'LAGLIDADG' family motif peptide

<400> 92
Leu Ala Gly Leu Ile Asp Ala Asp Gly
1 5

<210> 93
<211> 12
<212> RNA
<213> Artificial Sequence

<220>
<221> source
<223> /note="Description of Artificial Sequence: Synthetic
oligonucleotide"

<400> 93
guuuuagagc ua 12

<210> 94
<211> 12
<212> DNA
<213> Artificial Sequence

<220>
<221> source
<223> /note="Description of Artificial Sequence: Synthetic
oligonucleotide"

<400> 94
gttttagagc ta 12

<210> 95
<211> 31
<212> DNA
<213> Artificial Sequence

<220>
<221> source
<223> /note="Description of Artificial Sequence: Synthetic
oligonucleotide"

<400> 95
tagcaagtta aaataaggct agtccgtttt t 31

<210> 96
<211> 32
<212> DNA
<213> Artificial Sequence

<220>
<221> source
<223> /note="Description of Artificial Sequence: Synthetic

primer"

<400> 96
aaactctaga gagggcctat ttcccatgat tc 32

<210> 97
<211> 153
<212> DNA
<213> Artificial Sequence

<220>
<221> source
<223> /note="Description of Artificial Sequence: Synthetic primer"

<400> 97
acctctagaa aaaaagcacc gactcggtgc cactttttca agttgataac ggactagcct 60

tattttaact tgctatgctg ttttgtttcc aaaacagcat agctctaaaa cccctagtca 120

ttggaggtga cggtgtttcg tcctttccac aag 153

<210> 98
<211> 52
<212> DNA
<213> Artificial Sequence

<220>
<221> source
<223> /note="Description of Artificial Sequence: Synthetic primer"

<400> 98
taatacgact cactatagga agtgcgccac catggcccca aagaagaagc gg 52

<210> 99
<211> 60
<212> DNA
<213> Artificial Sequence

<220>
<221> source
<223> /note="Description of Artificial Sequence: Synthetic primer"

<400> 99
ggtttttttt tttttttttt tttttttttt ttttcttact ttttcttttt tgcctggccg 60

<210> 100
<211> 984
<212> PRT
<213> Campylobacter jejuni

<400> 100
Met Ala Arg Ile Leu Ala Phe Asp Ile Gly Ile Ser Ser Ile Gly Trp
1 5 10 15

Ala Phe Ser Glu Asn Asp Glu Leu Lys Asp Cys Gly Val Arg Ile Phe

20 25 30

Thr Lys Val Glu Asn Pro Lys Thr Gly Glu Ser Leu Ala Leu Pro Arg
35 40 45

Arg Leu Ala Arg Ser Ala Arg Lys Arg Leu Ala Arg Arg Lys Ala Arg
50 55 60

Leu Asn His Leu Lys His Leu Ile Ala Asn Glu Phe Lys Leu Asn Tyr
65 70 75 80

Glu Asp Tyr Gln Ser Phe Asp Glu Ser Leu Ala Lys Ala Tyr Lys Gly
85 90 95

Ser Leu Ile Ser Pro Tyr Glu Leu Arg Phe Arg Ala Leu Asn Glu Leu
100 105 110

Leu Ser Lys Gln Asp Phe Ala Arg Val Ile Leu His Ile Ala Lys Arg
115 120 125

Arg Gly Tyr Asp Asp Ile Lys Asn Ser Asp Asp Lys Glu Lys Gly Ala
130 135 140

Ile Leu Lys Ala Ile Lys Gln Asn Glu Glu Lys Leu Ala Asn Tyr Gln
145 150 155 160

Ser Val Gly Glu Tyr Leu Tyr Lys Glu Tyr Phe Gln Lys Phe Lys Glu
165 170 175

Asn Ser Lys Glu Phe Thr Asn Val Arg Asn Lys Lys Glu Ser Tyr Glu
180 185 190

Arg Cys Ile Ala Gln Ser Phe Leu Lys Asp Glu Leu Lys Leu Ile Phe
195 200 205

Lys Lys Gln Arg Glu Phe Gly Phe Ser Phe Ser Lys Lys Phe Glu Glu
210 215 220

Glu Val Leu Ser Val Ala Phe Tyr Lys Arg Ala Leu Lys Asp Phe Ser
225 230 235 240

His Leu Val Gly Asn Cys Ser Phe Phe Thr Asp Glu Lys Arg Ala Pro
245 250 255

Lys Asn Ser Pro Leu Ala Phe Met Phe Val Ala Leu Thr Arg Ile Ile
260 265 270

```
Asn Leu Leu Asn Asn Leu Lys Asn Thr Glu Gly Ile Leu Tyr Thr Lys
        275                 280                 285

Asp Asp Leu Asn Ala Leu Leu Asn Glu Val Leu Lys Asn Gly Thr Leu
    290                 295                 300

Thr Tyr Lys Gln Thr Lys Lys Leu Leu Gly Leu Ser Asp Asp Tyr Glu
305                 310                 315                 320

Phe Lys Gly Glu Lys Gly Thr Tyr Phe Ile Glu Phe Lys Lys Tyr Lys
                325                 330                 335

Glu Phe Ile Lys Ala Leu Gly Glu His Asn Leu Ser Gln Asp Asp Leu
            340                 345                 350

Asn Glu Ile Ala Lys Asp Ile Thr Leu Ile Lys Asp Glu Ile Lys Leu
        355                 360                 365

Lys Lys Ala Leu Ala Lys Tyr Asp Leu Asn Gln Asn Gln Ile Asp Ser
    370                 375                 380

Leu Ser Lys Leu Glu Phe Lys Asp His Leu Asn Ile Ser Phe Lys Ala
385                 390                 395                 400

Leu Lys Leu Val Thr Pro Leu Met Leu Glu Gly Lys Lys Tyr Asp Glu
                405                 410                 415

Ala Cys Asn Glu Leu Asn Leu Lys Val Ala Ile Asn Glu Asp Lys Lys
            420                 425                 430

Asp Phe Leu Pro Ala Phe Asn Glu Thr Tyr Tyr Lys Asp Glu Val Thr
        435                 440                 445

Asn Pro Val Val Leu Arg Ala Ile Lys Glu Tyr Arg Lys Val Leu Asn
    450                 455                 460

Ala Leu Leu Lys Lys Tyr Gly Lys Val His Lys Ile Asn Ile Glu Leu
465                 470                 475                 480

Ala Arg Glu Val Gly Lys Asn His Ser Gln Arg Ala Lys Ile Glu Lys
                485                 490                 495

Glu Gln Asn Glu Asn Tyr Lys Ala Lys Lys Asp Ala Glu Leu Glu Cys
            500                 505                 510

Glu Lys Leu Gly Leu Lys Ile Asn Ser Lys Asn Ile Leu Lys Leu Arg
        515                 520                 525
```

```
Leu Phe Lys Glu Gln Lys Glu Phe Cys Ala Tyr Ser Gly Glu Lys Ile
    530                 535                 540

Lys Ile Ser Asp Leu Gln Asp Glu Lys Met Leu Glu Ile Asp His Ile
545                 550                 555                 560

Tyr Pro Tyr Ser Arg Ser Phe Asp Asp Ser Tyr Met Asn Lys Val Leu
                565                 570                 575

Val Phe Thr Lys Gln Asn Gln Glu Lys Leu Asn Gln Thr Pro Phe Glu
            580                 585                 590

Ala Phe Gly Asn Asp Ser Ala Lys Trp Gln Lys Ile Glu Val Leu Ala
        595                 600                 605

Lys Asn Leu Pro Thr Lys Lys Gln Lys Arg Ile Leu Asp Lys Asn Tyr
    610                 615                 620

Lys Asp Lys Glu Gln Lys Asn Phe Lys Asp Arg Asn Leu Asn Asp Thr
625                 630                 635                 640

Arg Tyr Ile Ala Arg Leu Val Leu Asn Tyr Thr Lys Asp Tyr Leu Asp
                645                 650                 655

Phe Leu Pro Leu Ser Asp Asp Glu Asn Thr Lys Leu Asn Asp Thr Gln
            660                 665                 670

Lys Gly Ser Lys Val His Val Glu Ala Lys Ser Gly Met Leu Thr Ser
        675                 680                 685

Ala Leu Arg His Thr Trp Gly Phe Ser Ala Lys Asp Arg Asn Asn His
    690                 695                 700

Leu His His Ala Ile Asp Ala Val Ile Ile Ala Tyr Ala Asn Asn Ser
705                 710                 715                 720

Ile Val Lys Ala Phe Ser Asp Phe Lys Lys Glu Gln Glu Ser Asn Ser
                725                 730                 735

Ala Glu Leu Tyr Ala Lys Lys Ile Ser Glu Leu Asp Tyr Lys Asn Lys
            740                 745                 750

Arg Lys Phe Phe Glu Pro Phe Ser Gly Phe Arg Gln Lys Val Leu Asp
        755                 760                 765

Lys Ile Asp Glu Ile Phe Val Ser Lys Pro Glu Arg Lys Lys Pro Ser
    770                 775                 780
```

Gly Ala Leu His Glu Glu Thr Phe Arg Lys Glu Glu Glu Phe Tyr Gln
785                 790                 795                 800

Ser Tyr Gly Gly Lys Glu Gly Val Leu Lys Ala Leu Glu Leu Gly Lys
                805                 810                 815

Ile Arg Lys Val Asn Gly Lys Ile Val Lys Asn Gly Asp Met Phe Arg
            820                 825                 830

Val Asp Ile Phe Lys His Lys Lys Thr Asn Lys Phe Tyr Ala Val Pro
        835                 840                 845

Ile Tyr Thr Met Asp Phe Ala Leu Lys Val Leu Pro Asn Lys Ala Val
    850                 855                 860

Ala Arg Ser Lys Lys Gly Glu Ile Lys Asp Trp Ile Leu Met Asp Glu
865                 870                 875                 880

Asn Tyr Glu Phe Cys Phe Ser Leu Tyr Lys Asp Ser Leu Ile Leu Ile
                885                 890                 895

Gln Thr Lys Asp Met Gln Glu Pro Glu Phe Val Tyr Tyr Asn Ala Phe
            900                 905                 910

Thr Ser Ser Thr Val Ser Leu Ile Val Ser Lys His Asp Asn Lys Phe
        915                 920                 925

Glu Thr Leu Ser Lys Asn Gln Lys Ile Leu Phe Lys Asn Ala Asn Glu
    930                 935                 940

Lys Glu Val Ile Ala Lys Ser Ile Gly Ile Gln Asn Leu Lys Val Phe
945                 950                 955                 960

Glu Lys Tyr Ile Val Ser Ala Leu Gly Glu Val Thr Lys Ala Glu Phe
                965                 970                 975

Arg Gln Arg Glu Asp Phe Lys Lys
            980

<210> 101
<211> 91
<212> DNA
<213> Artificial Sequence

<220>
<221> source
<223> /note="Description of Artificial Sequence: Synthetic oligonucleotide"

<400> 101
tataatctca taagaaattt aaaaagggac taaaataaag agtttgcggg actctgcggg 60

gttacaatcc cctaaaaccg cttttaaaat t 91

<210> 102
<211> 36
<212> DNA
<213> Artificial Sequence

<220>
<221> source
<223> /note="Description of Artificial Sequence: Synthetic
oligonucleotide"

<400> 102
attttaccat aaagaaattt aaaaagggac taaaac 36

<210> 103
<211> 95
<212> RNA
<213> Artificial Sequence

<220>
<221> source
<223> /note="Description of Artificial Sequence: Synthetic
oligonucleotide"

<220>
<221> modified_base
<222> (1)..(20)
<223> a, c, u, g, unknown or other

<400> 103
nnnnnnnnnn nnnnnnnnnn guuuuagucc cgaaagggac uaaaauaaag aguuugcggg 60

acucugcggg guuacaaucc ccuaaaaccg cuuuu 95

<210> 104
<211> 1115
<212> PRT
<213> Artificial Sequence

<220>
<221> source
<223> /note="Description of Artificial Sequence: Synthetic
polypeptide"

<400> 104
Met Ser Asp Leu Val Leu Gly Leu Asp Ile Gly Ile Gly Ser Val Gly
1 5 10 15

Val Gly Ile Leu Asn Lys Val Thr Gly Glu Ile Ile His Lys Asn Ser
20 25 30

Arg Ile Phe Pro Ala Ala Gln Ala Glu Asn Asn Leu Val Arg Arg Thr

35 40 45

Asn Arg Gln Gly Arg Arg Leu Ala Arg Arg Lys Lys His Arg Arg Val
50 55 60

Arg Leu Asn Arg Leu Phe Glu Glu Ser Gly Leu Ile Thr Asp Phe Thr
65 70 75 80

Lys Ile Ser Ile Asn Leu Asn Pro Tyr Gln Leu Arg Val Lys Gly Leu
85 90 95

Thr Asp Glu Leu Ser Asn Glu Glu Leu Phe Ile Ala Leu Lys Asn Met
100 105 110

Val Lys His Arg Gly Ile Ser Tyr Leu Asp Asp Ala Ser Asp Asp Gly
115 120 125

Asn Ser Ser Val Gly Asp Tyr Ala Gln Ile Val Lys Glu Asn Ser Lys
130 135 140

Gln Leu Glu Thr Lys Thr Pro Gly Gln Ile Gln Leu Glu Arg Tyr Gln
145 150 155 160

Thr Tyr Gly Gln Leu Arg Gly Asp Phe Thr Val Glu Lys Asp Gly Lys
165 170 175

Lys His Arg Leu Ile Asn Val Phe Pro Thr Ser Ala Tyr Arg Ser Glu
180 185 190

Ala Leu Arg Ile Leu Gln Thr Gln Gln Glu Phe Asn Pro Gln Ile Thr
195 200 205

Asp Glu Phe Ile Asn Arg Tyr Leu Glu Ile Leu Thr Gly Lys Arg Lys
210 215 220

Tyr Tyr His Gly Pro Gly Asn Glu Lys Ser Arg Thr Asp Tyr Gly Arg
225 230 235 240

Tyr Arg Thr Ser Gly Glu Thr Leu Asp Asn Ile Phe Gly Ile Leu Ile
245 250 255

Gly Lys Cys Thr Phe Tyr Pro Asp Glu Phe Arg Ala Ala Lys Ala Ser
260 265 270

Tyr Thr Ala Gln Glu Phe Asn Leu Leu Asn Asp Leu Asn Asn Leu Thr
275 280 285

```
Val Pro Thr Glu Thr Lys Lys Leu Ser Lys Glu Gln Lys Asn Gln Ile
    290                 295                 300

Ile Asn Tyr Val Lys Asn Glu Lys Ala Met Gly Pro Ala Lys Leu Phe
305                 310                 315                 320

Lys Tyr Ile Ala Lys Leu Leu Ser Cys Asp Val Ala Asp Ile Lys Gly
                325                 330                 335

Tyr Arg Ile Asp Lys Ser Gly Lys Ala Glu Ile His Thr Phe Glu Ala
            340                 345                 350

Tyr Arg Lys Met Lys Thr Leu Glu Thr Leu Asp Ile Glu Gln Met Asp
        355                 360                 365

Arg Glu Thr Leu Asp Lys Leu Ala Tyr Val Leu Thr Leu Asn Thr Glu
    370                 375                 380

Arg Glu Gly Ile Gln Glu Ala Leu Glu His Glu Phe Ala Asp Gly Ser
385                 390                 395                 400

Phe Ser Gln Lys Gln Val Asp Glu Leu Val Gln Phe Arg Lys Ala Asn
                405                 410                 415

Ser Ser Ile Phe Gly Lys Gly Trp His Asn Phe Ser Val Lys Leu Met
            420                 425                 430

Met Glu Leu Ile Pro Glu Leu Tyr Glu Thr Ser Glu Glu Gln Met Thr
        435                 440                 445

Ile Leu Thr Arg Leu Gly Lys Gln Lys Thr Thr Ser Ser Ser Asn Lys
    450                 455                 460

Thr Lys Tyr Ile Asp Glu Lys Leu Leu Thr Glu Glu Ile Tyr Asn Pro
465                 470                 475                 480

Val Val Ala Lys Ser Val Arg Gln Ala Ile Lys Ile Val Asn Ala Ala
                485                 490                 495

Ile Lys Glu Tyr Gly Asp Phe Asp Asn Ile Val Ile Glu Met Ala Arg
            500                 505                 510

Glu Asn Gln Thr Thr Gln Lys Gly Gln Lys Asn Ser Arg Glu Arg Met
        515                 520                 525

Lys Arg Ile Glu Glu Gly Ile Lys Glu Leu Gly Ser Gln Ile Leu Lys
    530                 535                 540
```

Glu His Pro Val Glu Asn Thr Gln Leu Gln Asn Glu Lys Leu Tyr Leu
545 550 555 560

Tyr Tyr Leu Gln Asn Gly Arg Asp Met Tyr Val Asp Gln Glu Leu Asp
565 570 575

Ile Asn Arg Leu Ser Asp Tyr Asp Val Asp His Ile Val Pro Gln Ser
580 585 590

Phe Leu Lys Asp Asp Ser Ile Asp Asn Lys Val Leu Thr Arg Ser Asp
595 600 605

Lys Asn Arg Gly Lys Ser Asp Asn Val Pro Ser Glu Glu Val Val Lys
610 615 620

Lys Met Lys Asn Tyr Trp Arg Gln Leu Leu Asn Ala Lys Leu Ile Thr
625 630 635 640

Gln Arg Lys Phe Asp Asn Leu Thr Lys Ala Glu Arg Gly Gly Leu Ser
645 650 655

Glu Leu Asp Lys Ala Gly Phe Ile Lys Arg Gln Leu Val Glu Thr Arg
660 665 670

Gln Ile Thr Lys His Val Ala Gln Ile Leu Asp Ser Arg Met Asn Thr
675 680 685

Lys Tyr Asp Glu Asn Asp Lys Leu Ile Arg Glu Val Lys Val Ile Thr
690 695 700

Leu Lys Ser Lys Leu Val Ser Asp Phe Arg Lys Asp Phe Gln Phe Tyr
705 710 715 720

Lys Val Arg Glu Ile Asn Asn Tyr His His Ala His Asp Ala Tyr Leu
725 730 735

Asn Ala Val Val Gly Thr Ala Leu Ile Lys Lys Tyr Pro Lys Leu Glu
740 745 750

Ser Glu Phe Val Tyr Gly Asp Tyr Lys Val Tyr Asp Val Arg Lys Met
755 760 765

Ile Ala Lys Ser Glu Gln Glu Ile Gly Lys Ala Thr Ala Lys Tyr Phe
770 775 780

Phe Tyr Ser Asn Ile Met Asn Phe Phe Lys Thr Glu Ile Thr Leu Ala
785 790 795 800

Asn Gly Glu Ile Arg Lys Arg Pro Leu Ile Glu Thr Asn Gly Glu Thr
805 810 815

Gly Glu Ile Val Trp Asp Lys Gly Arg Asp Phe Ala Thr Val Arg Lys
820 825 830

Val Leu Ser Met Pro Gln Val Asn Ile Val Lys Lys Thr Glu Val Gln
835 840 845

Thr Gly Gly Phe Ser Lys Glu Ser Ile Leu Pro Lys Arg Asn Ser Asp
850 855 860

Lys Leu Ile Ala Arg Lys Lys Asp Trp Asp Pro Lys Lys Tyr Gly Gly
865 870 875 880

Phe Asp Ser Pro Thr Val Ala Tyr Ser Val Leu Val Val Ala Lys Val
885 890 895

Glu Lys Gly Lys Ser Lys Lys Leu Lys Ser Val Lys Glu Leu Leu Gly
900 905 910

Ile Thr Ile Met Glu Arg Ser Ser Phe Glu Lys Asn Pro Ile Asp Phe
915 920 925

Leu Glu Ala Lys Gly Tyr Lys Glu Val Lys Lys Asp Leu Ile Ile Lys
930 935 940

Leu Pro Lys Tyr Ser Leu Phe Glu Leu Glu Asn Gly Arg Lys Arg Met
945 950 955 960

Leu Ala Ser Ala Gly Glu Leu Gln Lys Gly Asn Glu Leu Ala Leu Pro
965 970 975

Ser Lys Tyr Val Asn Phe Leu Tyr Leu Ala Ser His Tyr Glu Lys Leu
980 985 990

Lys Gly Ser Pro Glu Asp Asn Glu Gln Lys Gln Leu Phe Val Glu Gln
995 1000 1005

His Lys His Tyr Leu Asp Glu Ile Ile Glu Gln Ile Ser Glu Phe
1010 1015 1020

Ser Lys Arg Val Ile Leu Ala Asp Ala Asn Leu Asp Lys Val Leu
1025 1030 1035

Ser Ala Tyr Asn Lys His Arg Asp Lys Pro Ile Arg Glu Gln Ala

1040 1045 1050

Glu Asn Ile Ile His Leu Phe Thr Leu Thr Asn Leu Gly Ala Pro
1055 1060 1065

Ala Ala Phe Lys Tyr Phe Asp Thr Thr Ile Asp Arg Lys Arg Tyr
1070 1075 1080

Thr Ser Thr Lys Glu Val Leu Asp Ala Thr Leu Ile His Gln Ser
1085 1090 1095

Ile Thr Gly Leu Tyr Glu Thr Arg Ile Asp Leu Ser Gln Leu Gly
1100 1105 1110

Gly Asp
1115

<210> 105
<211> 1374
<212> PRT
<213> Artificial Sequence

<220>
<221> source
<223> /note="Description of Artificial Sequence: Synthetic polypeptide"

<400> 105
Met Asp Lys Lys Tyr Ser Ile Gly Leu Asp Ile Gly Thr Asn Ser Val
1 5 10 15

Gly Trp Ala Val Ile Thr Asp Glu Tyr Lys Val Pro Ser Lys Lys Phe
20 25 30

Lys Val Leu Gly Asn Thr Asp Arg His Ser Ile Lys Lys Asn Leu Ile
35 40 45

Gly Ala Leu Leu Phe Asp Ser Gly Glu Thr Ala Glu Ala Thr Arg Leu
50 55 60

Lys Arg Thr Ala Arg Arg Arg Tyr Thr Arg Arg Lys Asn Arg Ile Cys
65 70 75 80

Tyr Leu Gln Glu Ile Phe Ser Asn Glu Met Ala Lys Val Asp Asp Ser
85 90 95

Phe Phe His Arg Leu Glu Glu Ser Phe Leu Val Glu Glu Asp Lys Lys
100 105 110

His Glu Arg His Pro Ile Phe Gly Asn Ile Val Asp Glu Val Ala Tyr

115 120 125

His Glu Lys Tyr Pro Thr Ile Tyr His Leu Arg Lys Lys Leu Val Asp
130 135 140

Ser Thr Asp Lys Ala Asp Leu Arg Leu Ile Tyr Leu Ala Leu Ala His
145 150 155 160

Met Ile Lys Phe Arg Gly His Phe Leu Ile Glu Gly Asp Leu Asn Pro
165 170 175

Asp Asn Ser Asp Val Asp Lys Leu Phe Ile Gln Leu Val Gln Thr Tyr
180 185 190

Asn Gln Leu Phe Glu Glu Asn Pro Ile Asn Ala Ser Gly Val Asp Ala
195 200 205

Lys Ala Ile Leu Ser Ala Arg Leu Ser Lys Ser Arg Arg Leu Glu Asn
210 215 220

Leu Ile Ala Gln Leu Pro Gly Glu Lys Lys Asn Gly Leu Phe Gly Asn
225 230 235 240

Leu Ile Ala Leu Ser Leu Gly Leu Thr Pro Asn Phe Lys Ser Asn Phe
245 250 255

Asp Leu Ala Glu Asp Ala Lys Leu Gln Leu Ser Lys Asp Thr Tyr Asp
260 265 270

Asp Asp Leu Asp Asn Leu Leu Ala Gln Ile Gly Asp Gln Tyr Ala Asp
275 280 285

Leu Phe Leu Ala Ala Lys Asn Leu Ser Asp Ala Ile Leu Leu Ser Asp
290 295 300

Ile Leu Arg Val Asn Thr Glu Ile Thr Lys Ala Pro Leu Ser Ala Ser
305 310 315 320

Met Ile Lys Arg Tyr Asp Glu His His Gln Asp Leu Thr Leu Leu Lys
325 330 335

Ala Leu Val Arg Gln Gln Leu Pro Glu Lys Tyr Lys Glu Ile Phe Phe
340 345 350

Asp Gln Ser Lys Asn Gly Tyr Ala Gly Tyr Ile Asp Gly Gly Ala Ser
355 360 365

```
Gln Glu Glu Phe Tyr Lys Phe Ile Lys Pro Ile Leu Glu Lys Met Asp
    370                 375                 380

Gly Thr Glu Glu Leu Leu Val Lys Leu Asn Arg Glu Asp Leu Leu Arg
385                 390                 395                 400

Lys Gln Arg Thr Phe Asp Asn Gly Ser Ile Pro His Gln Ile His Leu
                405                 410                 415

Gly Glu Leu His Ala Ile Leu Arg Arg Gln Glu Asp Phe Tyr Pro Phe
            420                 425                 430

Leu Lys Asp Asn Arg Glu Lys Ile Glu Lys Ile Leu Thr Phe Arg Ile
        435                 440                 445

Pro Tyr Tyr Val Gly Pro Leu Ala Arg Gly Asn Ser Arg Phe Ala Trp
    450                 455                 460

Met Thr Arg Lys Ser Glu Glu Thr Ile Thr Pro Trp Asn Phe Glu Glu
465                 470                 475                 480

Val Val Asp Lys Gly Ala Ser Ala Gln Ser Phe Ile Glu Arg Met Thr
                485                 490                 495

Asn Phe Asp Lys Asn Leu Pro Asn Glu Lys Val Leu Pro Lys His Ser
            500                 505                 510

Leu Leu Tyr Glu Tyr Phe Thr Val Tyr Asn Glu Leu Thr Lys Val Lys
        515                 520                 525

Tyr Val Thr Glu Gly Met Arg Lys Pro Ala Phe Leu Ser Gly Glu Gln
    530                 535                 540

Lys Lys Ala Ile Val Asp Leu Leu Phe Lys Thr Asn Arg Lys Val Thr
545                 550                 555                 560

Val Lys Gln Leu Lys Glu Asp Tyr Phe Lys Lys Ile Glu Cys Phe Asp
                565                 570                 575

Ser Val Glu Ile Ser Gly Val Glu Asp Arg Phe Asn Ala Ser Leu Gly
            580                 585                 590

Thr Tyr His Asp Leu Leu Lys Ile Ile Lys Asp Lys Asp Phe Leu Asp
        595                 600                 605

Asn Glu Glu Asn Glu Asp Ile Leu Glu Asp Ile Val Leu Thr Leu Thr
    610                 615                 620
```

Leu Phe Glu Asp Arg Glu Met Ile Glu Glu Arg Leu Lys Thr Tyr Ala
625 630 635 640

His Leu Phe Asp Asp Lys Val Met Lys Gln Leu Lys Arg Arg Arg Tyr
645 650 655

Thr Gly Trp Gly Arg Leu Ser Arg Lys Leu Ile Asn Gly Ile Arg Asp
660 665 670

Lys Gln Ser Gly Lys Thr Ile Leu Asp Phe Leu Lys Ser Asp Gly Phe
675 680 685

Ala Asn Arg Asn Phe Met Gln Leu Ile His Asp Asp Ser Leu Thr Phe
690 695 700

Lys Glu Asp Ile Gln Lys Ala Gln Val Ser Gly Gln Gly Asp Ser Leu
705 710 715 720

His Glu His Ile Ala Asn Leu Ala Gly Ser Pro Ala Ile Lys Lys Gly
725 730 735

Ile Leu Gln Thr Val Lys Val Val Asp Glu Leu Val Lys Val Met Gly
740 745 750

Arg His Lys Pro Glu Asn Ile Val Ile Glu Met Ala Arg Glu Thr Asn
755 760 765

Glu Asp Asp Glu Lys Lys Ala Ile Gln Lys Ile Gln Lys Ala Asn Lys
770 775 780

Asp Glu Lys Asp Ala Ala Met Leu Lys Ala Ala Asn Gln Tyr Asn Gly
785 790 795 800

Lys Ala Glu Leu Pro His Ser Val Phe His Gly His Lys Gln Leu Ala
805 810 815

Thr Lys Ile Arg Leu Trp His Gln Gln Gly Glu Arg Cys Leu Tyr Thr
820 825 830

Gly Lys Thr Ile Ser Ile His Asp Leu Ile Asn Asn Ser Asn Gln Phe
835 840 845

Glu Val Asp His Ile Leu Pro Leu Ser Ile Thr Phe Asp Asp Ser Leu
850 855 860

Ala Asn Lys Val Leu Val Tyr Ala Thr Ala Asn Gln Glu Lys Gly Gln
865 870 875 880

Arg Thr Pro Tyr Gln Ala Leu Asp Ser Met Asp Asp Ala Trp Ser Phe
885 890 895

Arg Glu Leu Lys Ala Phe Val Arg Glu Ser Lys Thr Leu Ser Asn Lys
900 905 910

Lys Lys Glu Tyr Leu Leu Thr Glu Glu Asp Ile Ser Lys Phe Asp Val
915 920 925

Arg Lys Lys Phe Ile Glu Arg Asn Leu Val Asp Thr Arg Tyr Ala Ser
930 935 940

Arg Val Val Leu Asn Ala Leu Gln Glu His Phe Arg Ala His Lys Ile
945 950 955 960

Asp Thr Lys Val Ser Val Val Arg Gly Gln Phe Thr Ser Gln Leu Arg
965 970 975

Arg His Trp Gly Ile Glu Lys Thr Arg Asp Thr Tyr His His His Ala
980 985 990

Val Asp Ala Leu Ile Ile Ala Ala Ser Ser Gln Leu Asn Leu Trp Lys
995 1000 1005

Lys Gln Lys Asn Thr Leu Val Ser Tyr Ser Glu Asp Gln Leu Leu
1010 1015 1020

Asp Ile Glu Thr Gly Glu Leu Ile Ser Asp Asp Glu Tyr Lys Glu
1025 1030 1035

Ser Val Phe Lys Ala Pro Tyr Gln His Phe Val Asp Thr Leu Lys
1040 1045 1050

Ser Lys Glu Phe Glu Asp Ser Ile Leu Phe Ser Tyr Gln Val Asp
1055 1060 1065

Ser Lys Phe Asn Arg Lys Ile Ser Asp Ala Thr Ile Tyr Ala Thr
1070 1075 1080

Arg Gln Ala Lys Val Gly Lys Asp Lys Ala Asp Glu Thr Tyr Val
1085 1090 1095

Leu Gly Lys Ile Lys Asp Ile Tyr Thr Gln Asp Gly Tyr Asp Ala
1100 1105 1110

Phe Met Lys Ile Tyr Lys Lys Asp Lys Ser Lys Phe Leu Met Tyr

1115 1120 1125

Arg His Asp Pro Gln Thr Phe Glu Lys Val Ile Glu Pro Ile Leu
1130 1135 1140

Glu Asn Tyr Pro Asn Lys Gln Ile Asn Glu Lys Gly Lys Glu Val
1145 1150 1155

Pro Cys Asn Pro Phe Leu Lys Tyr Lys Glu Glu His Gly Tyr Ile
1160 1165 1170

Arg Lys Tyr Ser Lys Lys Gly Asn Gly Pro Glu Ile Lys Ser Leu
1175 1180 1185

Lys Tyr Tyr Asp Ser Lys Leu Gly Asn His Ile Asp Ile Thr Pro
1190 1195 1200

Lys Asp Ser Asn Asn Lys Val Val Leu Gln Ser Val Ser Pro Trp
1205 1210 1215

Arg Ala Asp Val Tyr Phe Asn Lys Thr Thr Gly Lys Tyr Glu Ile
1220 1225 1230

Leu Gly Leu Lys Tyr Ala Asp Leu Gln Phe Glu Lys Gly Thr Gly
1235 1240 1245

Thr Tyr Lys Ile Ser Gln Glu Lys Tyr Asn Asp Ile Lys Lys Lys
1250 1255 1260

Glu Gly Val Asp Ser Asp Ser Glu Phe Lys Phe Thr Leu Tyr Lys
1265 1270 1275

Asn Asp Leu Leu Leu Val Lys Asp Thr Glu Thr Lys Glu Gln Gln
1280 1285 1290

Leu Phe Arg Phe Leu Ser Arg Thr Met Pro Lys Gln Lys His Tyr
1295 1300 1305

Val Glu Leu Lys Pro Tyr Asp Lys Gln Lys Phe Glu Gly Gly Glu
1310 1315 1320

Ala Leu Ile Lys Val Leu Gly Asn Val Ala Asn Ser Gly Gln Cys
1325 1330 1335

Lys Lys Gly Leu Gly Lys Ser Asn Ile Ser Ile Tyr Lys Val Arg
1340 1345 1350

```
Thr Asp  Val Leu Gly Asn Gln  His Ile Ile Lys Asn  Glu Gly Asp
    1355                 1360                 1365


Lys Pro  Lys Leu Asp Phe
    1370


<210> 106
<211> 6
<212> PRT
<213> Artificial Sequence

<220>
<221> source
<223> /note="Description of Artificial Sequence: Synthetic
      6xHis tag"

<400> 106
His His His His His His
1               5


<210> 107
<211> 15
<212> PRT
<213> Artificial Sequence

<220>
<221> source
<223> /note="Description of Artificial Sequence: Synthetic
      peptide"

<400> 107
Gly Gly Gly Gly Ser Gly Gly Gly Gly Ser Gly Gly Gly Gly Ser
1               5                   10                  15


<210> 108
<211> 15
<212> PRT
<213> Artificial Sequence

<220>
<221> source
<223> /note="Description of Artificial Sequence: Synthetic
      peptide"

<400> 108
Glu Ala Ala Ala Lys Glu Ala Ala Ala Lys Glu Ala Ala Ala Lys
1               5                   10                  15


<210> 109
<211> 18
<212> PRT
<213> Artificial Sequence

<220>
<221> source
<223> /note="Description of Artificial Sequence: Synthetic
      peptide"
```

<400> 109
Gly Gly Gly Gly Gly Ser Gly Gly Gly Gly Gly Ser Gly Gly Gly Gly
1               5                   10                  15

Gly Ser

<210> 110
<211> 23
<212> DNA
<213> Artificial Sequence

<220>
<221> source
<223> /note="Description of Artificial Sequence: Synthetic oligonucleotide"

<400> 110
gccaaattgg acgaccctcg cgg 23

<210> 111
<211> 23
<212> DNA
<213> Artificial Sequence

<220>
<221> source
<223> /note="Description of Artificial Sequence: Synthetic oligonucleotide"

<400> 111
cgaggagacc cccgtttcgg tgg 23

<210> 112
<211> 23
<212> DNA
<213> Artificial Sequence

<220>
<221> source
<223> /note="Description of Artificial Sequence: Synthetic oligonucleotide"

<400> 112
cccgccgccg ccgtggctcg agg 23

<210> 113
<211> 23
<212> DNA
<213> Artificial Sequence

<220>
<221> source
<223> /note="Description of Artificial Sequence: Synthetic oligonucleotide"

<400> 113
tgagctctac gagatccaca agg 23

<210> 114
<211> 23
<212> DNA
<213> Artificial Sequence

<220>
<221> source
<223> /note="Description of Artificial Sequence: Synthetic oligonucleotide"

<400> 114
ctcaaaattc ataccggttg tgg 23

<210> 115
<211> 23
<212> DNA
<213> Artificial Sequence

<220>
<221> source
<223> /note="Description of Artificial Sequence: Synthetic oligonucleotide"

<400> 115
cgttaaacaa caaccggact tgg 23

<210> 116
<211> 23
<212> DNA
<213> Artificial Sequence

<220>
<221> source
<223> /note="Description of Artificial Sequence: Synthetic oligonucleotide"

<400> 116
ttcaccccgc ggcgctgaat ggg 23

<210> 117
<211> 23
<212> DNA
<213> Artificial Sequence

<220>
<221> source
<223> /note="Description of Artificial Sequence: Synthetic oligonucleotide"

<400> 117
accactacca gtccgtccac agg 23

<210> 118
<211> 23
<212> DNA
<213> Artificial Sequence

<220>
<221> source
<223> /note="Description of Artificial Sequence: Synthetic
oligonucleotide"

<400> 118
agcctttctg aacacatgca cgg 23

<210> 119
<211> 39
<212> DNA
<213> Homo sapiens

<400> 119
cctgccatca atgtggccat gcatgtgttc agaaaggct 39

<210> 120
<211> 39
<212> DNA
<213> Homo sapiens

<400> 120
cctgccatca atgtggccgt gcatgtgttc agaaaggct 39

<210> 121
<211> 23
<212> DNA
<213> Artificial Sequence

<220>
<221> source
<223> /note="Description of Artificial Sequence: Synthetic
oligonucleotide"

<400> 121
cactgcttaa gcctcgctcg agg 23

<210> 122
<211> 23
<212> DNA
<213> Artificial Sequence

<220>
<221> source
<223> /note="Description of Artificial Sequence: Synthetic
oligonucleotide"

<400> 122
tcaccagcaa tattcgctcg agg 23

<210> 123
<211> 23
<212> DNA
<213> Artificial Sequence

<220>
<221> source
<223> /note="Description of Artificial Sequence: Synthetic

oligonucleotide"

<400> 123
caccagcaat attccgctcg agg 23

<210> 124
<211> 23
<212> DNA
<213> Artificial Sequence

<220>
<221> source
<223> /note="Description of Artificial Sequence: Synthetic oligonucleotide"

<400> 124
tagcaacaga catacgctcg agg 23

<210> 125
<211> 23
<212> DNA
<213> Artificial Sequence

<220>
<221> source
<223> /note="Description of Artificial Sequence: Synthetic oligonucleotide"

<400> 125
gggcagtagt aatacgctcg agg 23

<210> 126
<211> 23
<212> DNA
<213> Artificial Sequence

<220>
<221> source
<223> /note="Description of Artificial Sequence: Synthetic oligonucleotide"

<400> 126
ccaattccca tacattattg tac 23

<210> 127
<211> 26
<212> DNA
<213> Homo sapiens

<400> 127
ccgtgccggg cgggagaccg ccatgg 26

<210> 128
<211> 22
<212> DNA
<213> Homo sapiens

<400> 128

ggcccggctg tggctgagga gc 22

<210> 129
<211> 20
<212> DNA
<213> Homo sapiens

<400> 129
cggtctcccg cccggcacgg 20

<210> 130
<211> 26
<212> DNA
<213> Homo sapiens

<400> 130
gctcctcagc cacagccggg ccgggt 26

<210> 131
<211> 12
<212> DNA
<213> Homo sapiens

<400> 131
cgaccctgga aa 12

<210> 132
<211> 14
<212> DNA
<213> Homo sapiens

<400> 132
ccccgccgcc accc 14

<210> 133
<211> 18
<212> DNA
<213> Homo sapiens

<400> 133
tttccagggt cgccatgg 18

<210> 134
<211> 10
<212> DNA
<213> Homo sapiens

<400> 134
ggcggcgggg 10

<210> 135
<211> 23
<212> DNA
<213> Artificial Sequence

<220>

<221> source
<223> /note="Description of Artificial Sequence: Synthetic primer"

<400> 135
gagggcctat ttcccatgat tcc 23

<210> 136
<211> 126
<212> DNA
<213> Artificial Sequence

<220>
<221> source
<223> /note="Description of Artificial Sequence: Synthetic primer"

<220>
<221> modified_base
<222> (84)..(102)
<223> a, c, t, g, unknown or other

<400> 136
aaaaaaagca ccgactcggt gccacttttt caagttgata acggactagc cttattttaa 60

cttgctattt ctagctctaa aacnnnnnnn nnnnnnnnnn nnccggtgtt tcgtcctttc 120

cacaag 126

<210> 137
<211> 24
<212> DNA
<213> Artificial Sequence

<220>
<221> source
<223> /note="Description of Artificial Sequence: Synthetic primer"

<220>
<221> modified_base
<222> (6)..(24)
<223> a, c, t, g, unknown or other

<400> 137
caccgnnnnn nnnnnnnnnn nnnn 24

<210> 138
<211> 24
<212> DNA
<213> Artificial Sequence

<220>
<221> source
<223> /note="Description of Artificial Sequence: Synthetic primer"

<220>
<221> modified_base
<222> (5)..(23)
<223> a, c, t, g, unknown or other

<400> 138
aaacnnnnnn nnnnnnnnnn nnnc 24

<210> 139
<211> 126
<212> DNA
<213> Artificial Sequence

<220>
<221> source
<223> /note="Description of Artificial Sequence: Synthetic primer"

<400> 139
aaaaaaagca ccgactcggt gccacttttt caagttgata acggactagc cttattttaa 60

cttgctattt ctagctctaa aacccctagt cattggaggt gaccggtgtt tcgtcctttc 120

cacaag 126

<210> 140
<211> 24
<212> DNA
<213> Artificial Sequence

<220>
<221> source
<223> /note="Description of Artificial Sequence: Synthetic primer"

<400> 140
caccgtcacc tccaatgact aggg 24

<210> 141
<211> 24
<212> DNA
<213> Artificial Sequence

<220>
<221> source
<223> /note="Description of Artificial Sequence: Synthetic primer"

<400> 141
aaacccctag tcattggagg tgac 24

<210> 142
<211> 192
<212> DNA
<213> Artificial Sequence

<220>
<221> source
<223> /note="Description of Artificial Sequence: Synthetic

primer"

<400> 142
cagaagaaga agggctccca tcacatcaac cggtggcgca ttgccacgaa gcaggccaat 60

ggggaggaca tcgatgtcac ctccaatgac aagcttgcta gcggtgggca accacaaacc 120

cacgagggca gagtgctgct tgctgctggc caggcccctg cgtgggccca agctggactc 180

tggccactcc ct 192

<210> 143
<211> 192
<212> DNA
<213> Artificial Sequence

<220>
<221> source
<223> /note="Description of Artificial Sequence: Synthetic primer"

<400> 143
agggagtggc cagagtccag cttgggccca cgcaggggcc tggccagcag caagcagcac 60

tctgccctcg tgggtttgtg gttgcccacc gctagcaagc ttgtcattgg aggtgacatc 120

gatgtcctcc ccattggcct gcttcgtggc aatgcgccac cggttgatgt gatgggagcc 180

cttcttcttc tg 192

<210> 144
<211> 20
<212> DNA
<213> Artificial Sequence

<220>
<221> source
<223> /note="Description of Artificial Sequence: Synthetic primer"

<400> 144
ccatcccctt ctgtgaatgt 20

<210> 145
<211> 20
<212> DNA
<213> Artificial Sequence

<220>
<221> source
<223> /note="Description of Artificial Sequence: Synthetic primer"

<400> 145
ggagattgga gacacggaga 20

<210> 146
<211> 20
<212> DNA

<213> Artificial Sequence

<220>
<221> source
<223> /note="Description of Artificial Sequence: Synthetic primer"

<400> 146
ggctccctgg gttcaaagta 20

<210> 147
<211> 21
<212> DNA
<213> Artificial Sequence

<220>
<221> source
<223> /note="Description of Artificial Sequence: Synthetic primer"

<400> 147
agaggggtct ggatgtcgta a 21

<210> 148
<211> 24
<212> DNA
<213> Artificial Sequence

<220>
<221> source
<223> /note="Description of Artificial Sequence: Synthetic primer"

<400> 148
cgccagggtt ttcccagtca cgac 24

<210> 149
<211> 51
<212> DNA
<213> Artificial Sequence

<220>
<221> source
<223> /note="Description of Artificial Sequence: Synthetic primer"

<400> 149
gagggtctcg tccttgcggc cgcgctagcg agggcctatt tcccatgatt c 51

<210> 150
<211> 133
<212> DNA
<213> Artificial Sequence

<220>
<221> source
<223> /note="Description of Artificial Sequence: Synthetic primer"

<220>
<221> modified_base
<222> (95)..(114)
<223> a, c, t, g, unknown or other

<400> 150
ctcggtctcg gtaaaaaagc accgactcgg tgccactttt tcaagttgat aacggactag 60

ccttatttta acttgctatt tctagctcta aaacnnnnnn nnnnnnnnnn nnnnggtgtt 120

tcgtcctttc cac 133

<210> 151
<211> 41
<212> DNA
<213> Artificial Sequence

<220>
<221> source
<223> /note="Description of Artificial Sequence: Synthetic primer"

<400> 151
gagggtctct ttaccggtga gggcctattt cccatgattc c 41

<210> 152
<211> 133
<212> DNA
<213> Artificial Sequence

<220>
<221> source
<223> /note="Description of Artificial Sequence: Synthetic primer"

<220>
<221> modified_base
<222> (95)..(114)
<223> a, c, t, g, unknown or other

<400> 152
ctcggtctcc tcaaaaaagc accgactcgg tgccactttt tcaagttgat aacggactag 60

ccttatttta acttgctatt tctagctcta aaacnnnnnn nnnnnnnnnn nnnnggtgtt 120

tcgtcctttc cac 133

<210> 153
<211> 40
<212> DNA
<213> Artificial Sequence

<220>
<221> source
<223> /note="Description of Artificial Sequence: Synthetic primer"

<400> 153

gagggtctct ttgagctcga gggcctattt cccatgattc 40

<210> 154
<211> 133
<212> DNA
<213> Artificial Sequence

<220>
<221> source
<223> /note="Description of Artificial Sequence: Synthetic
primer"

<220>
<221> modified_base
<222> (96)..(115)
<223> a, c, t, g, unknown or other

<400> 154
ctcggtctcg cgtaaaaaag caccgactcg gtgccacttt ttcaagttga taacggacta 60

gccttatttt aacttgctat ttctagctct aaaacnnnnn nnnnnnnnnn nnnnnggtgt 120

ttcgtccttt cca 133

<210> 155
<211> 27
<212> DNA
<213> Artificial Sequence

<220>
<221> source
<223> /note="Description of Artificial Sequence: Synthetic
primer"

<400> 155
gagggtctct tacgcgtgtg tctagac 27

<210> 156
<211> 98
<212> DNA
<213> Artificial Sequence

<220>
<221> source
<223> /note="Description of Artificial Sequence: Synthetic
primer"

<400> 156
ctcggtctca aggacaggga agggagcagt ggttcacgcc tgtaatccca gcaatttggg 60

aggccaaggt gggtagatca cctgagatta ggagttgc 98

<210> 157
<211> 30
<212> DNA
<213> Artificial Sequence

<220>

<221> source
<223> /note="Description of Artificial Sequence: Synthetic primer"

<400> 157
cctgtccttg cggccgcgct agcgagggcc 30

<210> 158
<211> 31
<212> DNA
<213> Artificial Sequence

<220>
<221> source
<223> /note="Description of Artificial Sequence: Synthetic primer"

<400> 158
cacgcggccg caaggacagg gaagggagca g 31

<210> 159
<211> 327
<212> PRT
<213> Artificial Sequence

<220>
<221> source
<223> /note="Description of Artificial Sequence: Synthetic polypeptide"

<400> 159
Met Val Ser Lys Gly Glu Glu Leu Phe Thr Gly Val Val Pro Ile Leu
1 5 10 15

Val Glu Leu Asp Gly Asp Val Asn Gly His Lys Phe Ser Val Ser Gly
20 25 30

Glu Gly Glu Gly Asp Ala Thr Tyr Gly Lys Leu Thr Leu Lys Phe Ile
35 40 45

Cys Thr Thr Gly Lys Leu Pro Val Pro Trp Pro Thr Leu Val Thr Thr
50 55 60

Leu Thr Tyr Gly Val Gln Cys Phe Ser Arg Tyr Pro Asp His Met Lys
65 70 75 80

Gln His Asp Phe Phe Lys Ser Ala Met Pro Glu Gly Tyr Val Gln Glu
85 90 95

Arg Thr Ile Phe Phe Lys Asp Asp Gly Asn Tyr Lys Thr Arg Ala Glu
100 105 110

Val Lys Phe Glu Gly Asp Thr Leu Val Asn Arg Ile Glu Leu Lys Gly
115 120 125

```
Ile Asp Phe Lys Glu Asp Gly Asn Ile Leu Gly His Lys Leu Glu Tyr
    130                 135                 140

Asn Tyr Asn Ser His Asn Val Tyr Ile Met Ala Asp Lys Gln Lys Asn
145                 150                 155                 160

Gly Ile Lys Val Asn Phe Lys Ile Arg His Asn Ile Glu Asp Gly Ser
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Thr Gln Ser Ala Leu
        195                 200                 205

Ser Lys Asp Pro Asn Glu Lys Arg Asp His Met Val Leu Leu Glu Phe
    210                 215                 220

Val Thr Ala Ala Gly Ile Thr Leu Gly Met Asp Glu Leu Tyr Lys Ser
225                 230                 235                 240

Gly Leu Arg Ser Arg Glu Glu Glu Glu Glu Thr Asp Ser Arg Met Pro
                245                 250                 255

His Leu Asp Ser Pro Gly Ser Ser Gln Pro Arg Arg Ser Phe Leu Ser
            260                 265                 270

Arg Val Ile Arg Ala Ala Leu Pro Leu Gln Leu Leu Leu Leu Leu Leu
        275                 280                 285

Leu Leu Leu Ala Cys Leu Leu Pro Ala Ser Glu Asp Asp Tyr Ser Cys
    290                 295                 300

Thr Gln Ala Asn Asn Phe Ala Arg Ser Phe Tyr Pro Met Leu Arg Tyr
305                 310                 315                 320

Thr Asn Gly Pro Pro Pro Thr
                325
```

<210> 160
<211> 22
<212> DNA
<213> Artificial Sequence

<220>
<221> source
<223> /note="Description of Artificial Sequence: Synthetic primer"

<400> 160
gcagcctctg ttccacatac ac 22

<210> 161
<211> 22
<212> DNA
<213> Artificial Sequence

<220>
<221> source
<223> /note="Description of Artificial Sequence: Synthetic primer"

<400> 161
accattctca gtggctcaac aa 22

<210> 162
<211> 20
<212> DNA
<213> Artificial Sequence

<220>
<221> source
<223> /note="Description of Artificial Sequence: Synthetic oligonucleotide"

<400> 162
gtcggggtcc ctgaaccgga 20

<210> 163
<211> 20
<212> DNA
<213> Artificial Sequence

<220>
<221> source
<223> /note="Description of Artificial Sequence: Synthetic oligonucleotide"

<400> 163
gcttacctgg gctgtagaac 20

<210> 164
<211> 20
<212> DNA
<213> Artificial Sequence

<220>
<221> source
<223> /note="Description of Artificial Sequence: Synthetic oligonucleotide"

<400> 164
cttatcctgt tctacagccc 20

<210> 165
<211> 20

<212> DNA
<213> Artificial Sequence

<220>
<221> source
<223> /note="Description of Artificial Sequence: Synthetic oligonucleotide"

<400> 165
agatgtcttc ttttgcttgc 20

<210> 166
<211> 20
<212> DNA
<213> Artificial Sequence

<220>
<221> source
<223> /note="Description of Artificial Sequence: Synthetic oligonucleotide"

<400> 166
gtctccaggc cgcccatgtc 20

<210> 167
<211> 20
<212> DNA
<213> Artificial Sequence

<220>
<221> source
<223> /note="Description of Artificial Sequence: Synthetic oligonucleotide"

<400> 167
catgtctggg gctgcgcagt 20

<210> 168
<211> 20
<212> DNA
<213> Artificial Sequence

<220>
<221> source
<223> /note="Description of Artificial Sequence: Synthetic oligonucleotide"

<400> 168
agccccagac atgggcggcc 20

<210> 169
<211> 20
<212> DNA
<213> Artificial Sequence

<220>
<221> source
<223> /note="Description of Artificial Sequence: Synthetic oligonucleotide"

<400> 169
ttttattcgt gggcgtctcc 20

<210> 170
<211> 20
<212> DNA
<213> Artificial Sequence

<220>
<221> source
<223> /note="Description of Artificial Sequence: Synthetic oligonucleotide"

<400> 170
gctttcccga acacgtgaaa 20

<210> 171
<211> 20
<212> DNA
<213> Artificial Sequence

<220>
<221> source
<223> /note="Description of Artificial Sequence: Synthetic oligonucleotide"

<400> 171
cttttgccat ttcacgtgtt 20

<210> 172
<211> 20
<212> DNA
<213> Artificial Sequence

<220>
<221> source
<223> /note="Description of Artificial Sequence: Synthetic oligonucleotide"

<400> 172
gcagccgcct cgggtgtttg 20

<210> 173
<211> 20
<212> DNA
<213> Artificial Sequence

<220>
<221> source
<223> /note="Description of Artificial Sequence: Synthetic oligonucleotide"

<400> 173
cagccgcctc gggtgtttgt 20

<210> 174
<211> 20

<212> DNA
<213> Artificial Sequence

<220>
<221> source
<223> /note="Description of Artificial Sequence: Synthetic oligonucleotide"

<400> 174
aggggacacc gtgcactcaa 20

<210> 175
<211> 20
<212> DNA
<213> Artificial Sequence

<220>
<221> source
<223> /note="Description of Artificial Sequence: Synthetic oligonucleotide"

<400> 175
gagctggatc tacggcccca 20

<210> 176
<211> 20
<212> DNA
<213> Artificial Sequence

<220>
<221> source
<223> /note="Description of Artificial Sequence: Synthetic oligonucleotide"

<400> 176
gcggatgata tacccaccat 20

<210> 177
<211> 20
<212> DNA
<213> Artificial Sequence

<220>
<221> source
<223> /note="Description of Artificial Sequence: Synthetic oligonucleotide"

<400> 177
tggatctacg gccccatggt 20

<210> 178
<211> 20
<212> DNA
<213> Artificial Sequence

<220>
<221> source
<223> /note="Description of Artificial Sequence: Synthetic oligonucleotide"

<400> 178
tcacacggga cgtgcccgaa 20

<210> 179
<211> 20
<212> DNA
<213> Artificial Sequence

<220>
<221> source
<223> /note="Description of Artificial Sequence: Synthetic oligonucleotide"

<400> 179
cgggacgtgc ccgaaaggct 20

<210> 180
<211> 20
<212> DNA
<213> Artificial Sequence

<220>
<221> source
<223> /note="Description of Artificial Sequence: Synthetic oligonucleotide"

<400> 180
ggacgtgccc gaaaggctcg 20

<210> 181
<211> 20
<212> DNA
<213> Artificial Sequence

<220>
<221> source
<223> /note="Description of Artificial Sequence: Synthetic oligonucleotide"

<400> 181
gggacgtgcc cgaaaggctc 20

<210> 182
<211> 20
<212> DNA
<213> Artificial Sequence

<220>
<221> source
<223> /note="Description of Artificial Sequence: Synthetic oligonucleotide"

<400> 182
aagacggaca ggcacctacg 20

<210> 183
<211> 20

<212> DNA
<213> Artificial Sequence

<220>
<221> source
<223> /note="Description of Artificial Sequence: Synthetic oligonucleotide"

<400> 183
tgcgaatacg cccacgcgat 20

<210> 184
<211> 20
<212> DNA
<213> Artificial Sequence

<220>
<221> source
<223> /note="Description of Artificial Sequence: Synthetic oligonucleotide"

<400> 184
cccttggtcg cgcttaacgt 20

<210> 185
<211> 20
<212> DNA
<213> Artificial Sequence

<220>
<221> source
<223> /note="Description of Artificial Sequence: Synthetic oligonucleotide"

<400> 185
cccacgttaa gcgcgaccaa 20

<210> 186
<211> 20
<212> DNA
<213> Artificial Sequence

<220>
<221> source
<223> /note="Description of Artificial Sequence: Synthetic oligonucleotide"

<400> 186
ctccagcgtg tatcggtgca 20

<210> 187
<211> 20
<212> DNA
<213> Artificial Sequence

<220>
<221> source
<223> /note="Description of Artificial Sequence: Synthetic oligonucleotide"

<400> 187
ggatgatctc tcggaactgt 20

<210> 188
<211> 20
<212> DNA
<213> Artificial Sequence

<220>
<221> source
<223> /note="Description of Artificial Sequence: Synthetic oligonucleotide"

<400> 188
cgtcgccgtc cagctcgacc 20

<210> 189
<211> 20
<212> DNA
<213> Artificial Sequence

<220>
<221> source
<223> /note="Description of Artificial Sequence: Synthetic oligonucleotide"

<400> 189
cagggtcagc ttgccgtagg 20

<210> 190
<211> 21
<212> DNA
<213> Artificial Sequence

<220>
<221> source
<223> /note="Description of Artificial Sequence: Synthetic oligonucleotide"

<400> 190
tgttcgcatt atccgaacca t 21

<210> 191
<211> 21
<212> DNA
<213> Artificial Sequence

<220>
<221> source
<223> /note="Description of Artificial Sequence: Synthetic oligonucleotide"

<400> 191
cgcgatcgta atcacccgag t 21

<210> 192
<211> 21

<212> DNA
<213> Artificial Sequence

<220>
<221> source
<223> /note="Description of Artificial Sequence: Synthetic oligonucleotide"

<400> 192
ctcagttcca gtacggctcc a 21

<210> 193
<211> 21
<212> DNA
<213> Artificial Sequence

<220>
<221> source
<223> /note="Description of Artificial Sequence: Synthetic oligonucleotide"

<400> 193
gcttcaagtg ggagcgcgtg a 21

<210> 194
<211> 21
<212> DNA
<213> Artificial Sequence

<220>
<221> source
<223> /note="Description of Artificial Sequence: Synthetic oligonucleotide"

<400> 194
acgtctatat catggccgac a 21

<210> 195
<211> 21
<212> DNA
<213> Artificial Sequence

<220>
<221> source
<223> /note="Description of Artificial Sequence: Synthetic oligonucleotide"

<400> 195
cccgaccaca tgaagcagca c 21

<210> 196
<211> 21
<212> DNA
<213> Artificial Sequence

<220>
<221> source
<223> /note="Description of Artificial Sequence: Synthetic oligonucleotide"

<400> 196
cagcagccca aacgactaca t 21

<210> 197
<211> 22
<212> DNA
<213> Artificial Sequence

<220>
<221> source
<223> /note="Description of Artificial Sequence: Synthetic oligonucleotide"

<400> 197
ctaaggaatc tcggctggag gt 22

<210> 198
<211> 22
<212> DNA
<213> Artificial Sequence

<220>
<221> source
<223> /note="Description of Artificial Sequence: Synthetic oligonucleotide"

<400> 198
tcactggcac agaggagatt gt 22

<210> 199
<211> 22
<212> DNA
<213> Artificial Sequence

<220>
<221> source
<223> /note="Description of Artificial Sequence: Synthetic oligonucleotide"

<400> 199
tggagtctcc catttaccca ct 22

<210> 200
<211> 22
<212> DNA
<213> Artificial Sequence

<220>
<221> source
<223> /note="Description of Artificial Sequence: Synthetic oligonucleotide"

<400> 200
gacccaggag aagtcactga gc 22

<210> 201
<211> 22

<212> DNA
<213> Artificial Sequence

<220>
<221> source
<223> /note="Description of Artificial Sequence: Synthetic
oligonucleotide"

<400> 201
tggtaataga gagccccaag ga 22

<210> 202
<211> 22
<212> DNA
<213> Artificial Sequence

<220>
<221> source
<223> /note="Description of Artificial Sequence: Synthetic
oligonucleotide"

<400> 202
cacgctcact tcctctttct ca 22

<210> 203
<211> 22
<212> DNA
<213> Artificial Sequence

<220>
<221> source
<223> /note="Description of Artificial Sequence: Synthetic
oligonucleotide"

<400> 203
cctaggagtg aggctcgtga gt 22

<210> 204
<211> 22
<212> DNA
<213> Artificial Sequence

<220>
<221> source
<223> /note="Description of Artificial Sequence: Synthetic
oligonucleotide"

<400> 204
tggcttagaa cataggccac ct 22

<210> 205
<211> 22
<212> DNA
<213> Artificial Sequence

<220>
<221> source
<223> /note="Description of Artificial Sequence: Synthetic
oligonucleotide"

<400> 205
taccttccct ggcataggtg tt 22

<210> 206
<211> 22
<212> DNA
<213> Artificial Sequence

<220>
<221> source
<223> /note="Description of Artificial Sequence: Synthetic oligonucleotide"

<400> 206
tagactccac agaccccaca ga 22

<210> 207
<211> 26
<212> DNA
<213> Artificial Sequence

<220>
<221> source
<223> /note="Description of Artificial Sequence: Synthetic oligonucleotide"

<400> 207
aggagtatta gtacttccca ccctaa 26

<210> 208
<211> 21
<212> DNA
<213> Artificial Sequence

<220>
<221> source
<223> /note="Description of Artificial Sequence: Synthetic oligonucleotide"

<400> 208
cgtggtggtg tcaatactgc t 21

<210> 209
<211> 21
<212> DNA
<213> Artificial Sequence

<220>
<221> source
<223> /note="Description of Artificial Sequence: Synthetic oligonucleotide"

<400> 209
tgaaggtgcc atgttgagtt c 21

<210> 210
<211> 18

<212> DNA
<213> Artificial Sequence

<220>
<221> source
<223> /note="Description of Artificial Sequence: Synthetic oligonucleotide"

<400> 210
tgccatgtct gcgggaga 18

<210> 211
<211> 22
<212> DNA
<213> Artificial Sequence

<220>
<221> source
<223> /note="Description of Artificial Sequence: Synthetic oligonucleotide"

<400> 211
ggcggaggag atgggcttcg ag 22

<210> 212
<211> 21
<212> DNA
<213> Artificial Sequence

<220>
<221> source
<223> /note="Description of Artificial Sequence: Synthetic oligonucleotide"

<400> 212
cgagagagaa ccccagaaga c 21

<210> 213
<211> 20
<212> DNA
<213> Artificial Sequence

<220>
<221> source
<223> /note="Description of Artificial Sequence: Synthetic oligonucleotide"

<400> 213
agaagtgtcc aggcttccct 20

<210> 214
<211> 21
<212> DNA
<213> Artificial Sequence

<220>
<221> source
<223> /note="Description of Artificial Sequence: Synthetic oligonucleotide"

<400> 214
gcgactgatt cctattaaat a 21

<210> 215
<211> 21
<212> DNA
<213> Artificial Sequence

<220>
<221> source
<223> /note="Description of Artificial Sequence: Synthetic oligonucleotide"

<400> 215
agctatcact catggatata a 21

<210> 216
<211> 102
<212> DNA
<213> Artificial Sequence

<220>
<221> source
<223> /note="Description of Artificial Sequence: Synthetic polynucleotide"

<400> 216
gttttagagc tatgctgttt tgaatggtcc caaaacggaa gggcctgagt ccgagcagaa 60

gaagaagttt tagagctatg ctgttttgaa tggtcccaaa ac 102

<210> 217
<211> 100
<212> DNA
<213> Homo sapiens

<400> 217
cggaggacaa agtacaaacg gcagaagctg gaggaggaag ggcctgagtc cgagcagaag 60

aagaagggct cccatcacat caaccggtgg cgcattgcca 100

<210> 218
<211> 50
<212> DNA
<213> Homo sapiens

<400> 218
agctggagga ggaagggcct gagtccgagc agaagaagaa gggctcccac 50

<210> 219
<211> 30
<212> RNA
<213> Artificial Sequence

<220>
<221> source
<223> /note="Description of Artificial Sequence: Synthetic

oligonucleotide"

<400> 219
gaguccgagc agaagaagaa guuuuagagc 30

<210> 220
<211> 49
<212> DNA
<213> Artificial Sequence

<220>
<221> source
<223> /note="Description of Artificial Sequence: Synthetic oligonucleotide"

<400> 220
agctggagga ggaagggcct gagtccgagc agaagagaag ggctcccat 49

<210> 221
<211> 53
<212> DNA
<213> Homo sapiens

<400> 221
ctggaggagg aagggcctga gtccgagcag aagaagaagg gctcccatca cat 53

<210> 222
<211> 52
<212> DNA
<213> Homo sapiens

<400> 222
ctggaggagg aagggcctga gtccgagcag aagagaaggg ctcccatcac at 52

<210> 223
<211> 54
<212> DNA
<213> Homo sapiens

<400> 223
ctggaggagg aagggcctga gtccgagcag aagaaagaag ggctcccatc acat 54

<210> 224
<211> 50
<212> DNA
<213> Homo sapiens

<400> 224
ctggaggagg aagggcctga gtccgagcag aagaagggct cccatcacat 50

<210> 225
<211> 47
<212> DNA
<213> Homo sapiens

<400> 225
ctggaggagg aagggcctga gcccgagcag aagggctccc atcacat 47

<210> 226
<211> 48
<212> DNA
<213> Homo sapiens

<400> 226
ctggaggagg aagggcctga gtccgagcag aagaagaagg gctcccat 48

<210> 227
<211> 20
<212> RNA
<213> Artificial Sequence

<220>
<221> source
<223> /note="Description of Artificial Sequence: Synthetic oligonucleotide"

<400> 227
gaguccgagc agaagaagau 20

<210> 228
<211> 20
<212> RNA
<213> Artificial Sequence

<220>
<221> source
<223> /note="Description of Artificial Sequence: Synthetic oligonucleotide"

<400> 228
gaguccgagc agaagaagua 20

<210> 229
<211> 20
<212> RNA
<213> Artificial Sequence

<220>
<221> source
<223> /note="Description of Artificial Sequence: Synthetic oligonucleotide"

<400> 229
gaguccgagc agaagaacaa 20

<210> 230
<211> 20
<212> RNA
<213> Artificial Sequence

<220>
<221> source
<223> /note="Description of Artificial Sequence: Synthetic oligonucleotide"

<400> 230
gaguccgagc agaagaugaa 20

<210> 231
<211> 20
<212> RNA
<213> Artificial Sequence

<220>
<221> source
<223> /note="Description of Artificial Sequence: Synthetic oligonucleotide"

<400> 231
gaguccgagc agaaguagaa 20

<210> 232
<211> 20
<212> RNA
<213> Artificial Sequence

<220>
<221> source
<223> /note="Description of Artificial Sequence: Synthetic oligonucleotide"

<400> 232
gaguccgagc agaugaagaa 20

<210> 233
<211> 20
<212> RNA
<213> Artificial Sequence

<220>
<221> source
<223> /note="Description of Artificial Sequence: Synthetic oligonucleotide"

<400> 233
gaguccgagc acaagaagaa 20

<210> 234
<211> 20
<212> RNA
<213> Artificial Sequence

<220>
<221> source
<223> /note="Description of Artificial Sequence: Synthetic oligonucleotide"

<400> 234
gaguccgagg agaagaagaa 20

<210> 235
<211> 20
<212> RNA

<213> Artificial Sequence

<220>
<221> source
<223> /note="Description of Artificial Sequence: Synthetic oligonucleotide"

<400> 235
gaguccgugc agaagaagaa 20

<210> 236
<211> 20
<212> RNA
<213> Artificial Sequence

<220>
<221> source
<223> /note="Description of Artificial Sequence: Synthetic oligonucleotide"

<400> 236
gagucggagc agaagaagaa 20

<210> 237
<211> 20
<212> RNA
<213> Artificial Sequence

<220>
<221> source
<223> /note="Description of Artificial Sequence: Synthetic oligonucleotide"

<400> 237
gagaccgagc agaagaagaa 20

<210> 238
<211> 24
<212> DNA
<213> Artificial Sequence

<220>
<221> source
<223> /note="Description of Artificial Sequence: Synthetic oligonucleotide"

<400> 238
aatgacaagc ttgctagcgg tggg 24

<210> 239
<211> 39
<212> DNA
<213> Artificial Sequence

<220>
<221> source
<223> /note="Description of Artificial Sequence: Synthetic oligonucleotide"

<400> 239
aaaacggaag ggcctgagtc cgagcagaag aagaagttt 39

<210> 240
<211> 39
<212> DNA
<213> Artificial Sequence

<220>
<221> source
<223> /note="Description of Artificial Sequence: Synthetic oligonucleotide"

<400> 240
aaacaggggc cgagattggg tgttcagggc agaggtttt 39

<210> 241
<211> 38
<212> DNA
<213> Artificial Sequence

<220>
<221> source
<223> /note="Description of Artificial Sequence: Synthetic oligonucleotide"

<400> 241
aaaacggaag ggcctgagtc cgagcagaag aagaagtt 38

<210> 242
<211> 40
<212> DNA
<213> Artificial Sequence

<220>
<221> source
<223> /note="Description of Artificial Sequence: Synthetic oligonucleotide"

<400> 242
aacggaggga ggggcacaga tgagaaactc agggttttag 40

<210> 243
<211> 38
<212> DNA
<213> Homo sapiens

<400> 243
agcccttctt cttctgctcg gactcaggcc cttcctcc 38

<210> 244
<211> 40
<212> DNA
<213> Homo sapiens

<400> 244
cagggaggga ggggcacaga tgagaaactc aggaggcccc 40

<210> 245
<211> 80
<212> DNA
<213> Artificial Sequence

<220>
<221> source
<223> /note="Description of Artificial Sequence: Synthetic oligonucleotide"

<400> 245
ggcaatgcgc caccggttga tgtgatggga gcccttctag gaggccccca gagcagccac 60

tggggcctca acactcaggc 80

<210> 246
<211> 33
<212> DNA
<213> Homo sapiens

<400> 246
ggaagggcct gagtccgagc agaagaagaa ggg 33

<210> 247
<211> 33
<212> DNA
<213> Homo sapiens

<400> 247
cattggaggt gacatcgatg tcctccccat tgg 33

<210> 248
<211> 33
<212> DNA
<213> Homo sapiens

<400> 248
ggacatcgat gtcacctcca atgactaggg tgg 33

<210> 249
<211> 33
<212> DNA
<213> Homo sapiens

<400> 249
catcgatgtc ctccccattg gcctgcttcg tgg 33

<210> 250
<211> 33
<212> DNA
<213> Homo sapiens

<400> 250
ttcgtggcaa tgcgccaccg gttgatgtga tgg 33

<210> 251

<211> 33
<212> DNA
<213> Homo sapiens

<400> 251
tcgtggcaat gcgccaccgg ttgatgtgat ggg 33

<210> 252
<211> 33
<212> DNA
<213> Homo sapiens

<400> 252
tccagcttct gccgtttgta ctttgtcctc cgg 33

<210> 253
<211> 33
<212> DNA
<213> Homo sapiens

<400> 253
ggagggaggg gcacagatga gaaactcagg agg 33

<210> 254
<211> 33
<212> DNA
<213> Homo sapiens

<400> 254
aggggccgag attgggtgtt cagggcagag agg 33

<210> 255
<211> 33
<212> DNA
<213> Homo sapiens

<400> 255
atgcaggagg gtggcgagag gggccgagat tgg 33

<210> 256
<211> 33
<212> DNA
<213> Homo sapiens

<400> 256
ggtggcgaga ggggccgaga ttgggtgttc agg 33

<210> 257
<211> 33
<212> DNA
<213> Mus musculus

<400> 257
caagcactga gtgccattag ctaaatgcat agg 33

<210> 258

<211> 33
<212> DNA
<213> Mus musculus

<400> 258
aatgcatagg gtaccaccca caggtgccag ggg 33

<210> 259
<211> 33
<212> DNA
<213> Mus musculus

<400> 259
acacacatgg gaaagcctct gggccaggaa agg 33

<210> 260
<211> 37
<212> DNA
<213> Homo sapiens

<400> 260
ggaggaggta gtatacagaa acacagagaa gtagaat 37

<210> 261
<211> 37
<212> DNA
<213> Homo sapiens

<400> 261
agaatgtaga ggagtcacag aaactcagca ctagaaa 37

<210> 262
<211> 98
<212> DNA
<213> Artificial Sequence

<220>
<221> source
<223> /note="Description of Artificial Sequence: Synthetic oligonucleotide"

<400> 262
ggacgaaaca ccggaaccat tcaaaacagc atagcaagtt aaaataaggc tagtccgtta 60

tcaacttgaa aaagtggcac cgagtcggtg ctttttt 98

<210> 263
<211> 186
<212> DNA
<213> Artificial Sequence

<220>
<221> source
<223> /note="Description of Artificial Sequence: Synthetic polynucleotide"

<400> 263
ggacgaaaca ccggtagtat taagtattgt tttatggctg ataaatttct ttgaatttct 60

ccttgattat ttgttataaa agttataaaa taatcttgtt ggaaccattc aaaacagcat 120

agcaagttaa aataaggcta gtccgttatc aacttgaaaa agtggcaccg agtcggtgct 180

tttttt 186

<210> 264
<211> 95
<212> DNA
<213> Artificial Sequence

<220>
<221> source
<223> /note="Description of Artificial Sequence: Synthetic oligonucleotide"

<400> 264
gggttttaga gctatgctgt tttgaatggt cccaaaacgg gtcttcgaga agacgtttta 60

gagctatgct gttttgaatg gtcccaaaac ttttt 95

<210> 265
<211> 36
<212> DNA
<213> Artificial Sequence

<220>
<221> source
<223> /note="Description of Artificial Sequence: Synthetic oligonucleotide"

<220>
<221> modified_base
<222> (5)..(34)
<223> a, c, t, g, unknown or other

<400> 265
aaacnnnnnn nnnnnnnnnn nnnnnnnnnn nnnngt 36

<210> 266
<211> 36
<212> DNA
<213> Artificial Sequence

<220>
<221> source
<223> /note="Description of Artificial Sequence: Synthetic oligonucleotide"

<220>
<221> modified_base
<222> (7)..(36)
<223> a, c, t, g, unknown or other

<400> 266
taaaacnnnn nnnnnnnnnn nnnnnnnnnn nnnnnn 36

<210> 267
<211> 84
<212> DNA
<213> Artificial Sequence

<220>
<221> source
<223> /note="Description of Artificial Sequence: Synthetic oligonucleotide"

<400> 267
gtggaaagga cgaaacaccg ggtcttcgag aagacctgtt ttagagctag aaatagcaag 60

ttaaaataag gctagtccgt tttt 84

<210> 268
<211> 46
<212> RNA
<213> Artificial Sequence

<220>
<221> source
<223> /note="Description of Artificial Sequence: Synthetic oligonucleotide"

<220>
<221> modified_base
<222> (1)..(19)
<223> a, c, u, g, unknown or other

<400> 268
nnnnnnnnnn nnnnnnnnng uuauuguacu cucaagauuu auuuuu 46

<210> 269
<211> 91
<212> RNA
<213> Artificial Sequence

<220>
<221> source
<223> /note="Description of Artificial Sequence: Synthetic oligonucleotide"

<400> 269
guuacuuaaa ucuugcagaa gcuacaaaga uaaggcuuca ugccgaaauc aacacccugu 60

cauuuuaugg cagggguguuu ucguuauuua a 91

<210> 270
<211> 70
<212> DNA
<213> Homo sapiens

<400> 270
ttttctagtg ctgagtttct gtgactcctc tacattctac ttctctgtgt ttctgtatac 60

tacctcctcc 70

<210> 271
<211> 122
<212> DNA
<213> Homo sapiens

<400> 271
ggaggaaggg cctgagtccg agcagaagaa gaagggctcc catcacatca accggtggcg 60

cattgccacg aagcaggcca atggggagga catcgatgtc acctccaatg actagggtgg 120

gc 122

<210> 272
<211> 48
<212> RNA
<213> Artificial Sequence

<220>
<221> source
<223> /note="Description of Artificial Sequence: Synthetic oligonucleotide"

<220>
<221> modified_base
<222> (3)..(32)
<223> a, c, u, g, unknown or other

<400> 272
acnnnnnnnn nnnnnnnnnn nnnnnnnnnn nnguuuuaga gcuaugcu 48

<210> 273
<211> 67
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 273
agcauagcaa guuaaaauaa ggctaguccg uuaucaacuu gaaaaagugg caccgagucg 60

gugcuuu 67

<210> 274
<211> 62
<212> RNA
<213> Artificial Sequence

<220>
<221> source
<223> /note="Description of Artificial Sequence: Synthetic

oligonucleotide"

<220>
<221> modified_base
<222> (1)..(20)
<223> a, c, u, g, unknown or other

<400> 274
nnnnnnnnnn nnnnnnnnnn guuuuagagc uagaaauagc aaguuaaaau aaggcuaguc 60

cg 62

<210> 275
<211> 73
<212> DNA
<213> Artificial Sequence

<220>
<221> source
<223> /note="Description of Artificial Sequence: Synthetic oligonucleotide"

<400> 275
tgaatggtcc caaaacggaa gggcctgagt ccgagcagaa gaagaagttt tagagctatg 60

ctgttttgaa tgg 73

<210> 276
<211> 69
<212> DNA
<213> Homo sapiens

<400> 276
ctggtcttcc acctctctgc cctgaacacc caatctcggc ccctctcgcc accctcctgc 60

atttctgtt 69

<210> 277
<211> 138
<212> DNA
<213> Mus musculus

<400> 277
acccaagcac tgagtgccat tagctaaatg catagggtac cacccacagg tgccaggggc 60

ctttcccaaa gttcccagcc ccttctccaa cctttcctgg cccagaggct ttcccatgtg 120

tgtggctgga ccctttga 138

<210> 278
<211> 21
<212> DNA
<213> Artificial Sequence

<220>
<221> source
<223> /note="Description of Artificial Sequence: Synthetic primer"

<400> 278
aaaaccaccc ttctctctgg c 21

<210> 279
<211> 21
<212> DNA
<213> Artificial Sequence

<220>
<221> source
<223> /note="Description of Artificial Sequence: Synthetic primer"

<400> 279
ggagattgga gacacggaga g 21

<210> 280
<211> 20
<212> DNA
<213> Artificial Sequence

<220>
<221> source
<223> /note="Description of Artificial Sequence: Synthetic primer"

<400> 280
ctggaaagcc aatgcctgac 20

<210> 281
<211> 20
<212> DNA
<213> Artificial Sequence

<220>
<221> source
<223> /note="Description of Artificial Sequence: Synthetic primer"

<400> 281
ggcagcaaac tccttgtcct 20

<210> 282
<211> 20
<212> DNA
<213> Artificial Sequence

<220>
<221> source
<223> /note="Description of Artificial Sequence: Synthetic primer"

<400> 282
gtgctttgca gaggcctacc 20

<210> 283
<211> 20

<212> DNA
<213> Artificial Sequence

<220>
<221> source
<223> /note="Description of Artificial Sequence: Synthetic primer"

<400> 283
cctggagcgc atgcagtagt 20

<210> 284
<211> 22
<212> DNA
<213> Artificial Sequence

<220>
<221> source
<223> /note="Description of Artificial Sequence: Synthetic primer"

<400> 284
accttctgtg tttccaccat tc 22

<210> 285
<211> 20
<212> DNA
<213> Artificial Sequence

<220>
<221> source
<223> /note="Description of Artificial Sequence: Synthetic primer"

<400> 285
ttggggagtg cacagacttc 20

<210> 286
<211> 30
<212> DNA
<213> Artificial Sequence

<220>
<221> source
<223> /note="Description of Artificial Sequence: Synthetic probe"

<400> 286
tagctctaaa acttcttctt ctgctcggac 30

<210> 287
<211> 30
<212> DNA
<213> Artificial Sequence

<220>
<221> source
<223> /note="Description of Artificial Sequence: Synthetic probe"

<400> 287
ctagccttat tttaacttgc tatgctgttt 30

<210> 288
<211> 15
<212> DNA
<213> Homo sapiens

<400> 288
cagaagaaga agggc 15

<210> 289
<211> 51
<212> DNA
<213> Homo sapiens

<400> 289
ccaatgggga ggacatcgat gtcacctcca atgactaggg tggtgggcaa c 51

<210> 290
<211> 15
<212> DNA
<213> Homo sapiens

<400> 290
ctctggccac tccct 15

<210> 291
<211> 52
<212> DNA
<213> Homo sapiens

<400> 291
acatcgatgt cacctccaat gacaagcttg ctagcggtgg gcaaccacaa ac 52

<210> 292
<211> 25
<212> DNA
<213> Artificial Sequence

<220>
<221> source
<223> /note="Description of Artificial Sequence: Synthetic oligonucleotide"

<220>
<221> modified_base
<222> (6)..(25)
<223> a, c, t, g, unknown or other

<400> 292
caccgnnnnn nnnnnnnnnn nnnnn 25

<210> 293
<211> 25

<212> DNA
<213> Artificial Sequence

<220>
<221> source
<223> /note="Description of Artificial Sequence: Synthetic oligonucleotide"

<220>
<221> modified_base
<222> (5)..(24)
<223> a, c, t, g, unknown or other

<400> 293
aaacnnnnnn nnnnnnnnnn nnnnc 25

<210> 294
<211> 54
<212> DNA
<213> Artificial Sequence

<220>
<221> source
<223> /note="Description of Artificial Sequence: Synthetic oligonucleotide"

<400> 294
aacaccgggt cttcgagaag acctgtttta gagctagaaa tagcaagtta aaat 54

<210> 295
<211> 54
<212> DNA
<213> Artificial Sequence

<220>
<221> source
<223> /note="Description of Artificial Sequence: Synthetic oligonucleotide"

<400> 295
caaaacgggt cttcgagaag acgttttaga gctatgctgt tttgaatggt ccca 54

<210> 296
<211> 4104
<212> DNA
<213> Homo sapiens

<220>
<221> CDS
<222> (1)..(4104)

<400> 296
atg gac aag aag tac agc atc ggc ctg gac atc ggc acc aac tct gtg 48
Met Asp Lys Lys Tyr Ser Ile Gly Leu Asp Ile Gly Thr Asn Ser Val
1 5 10 15

ggc tgg gcc gtg atc acc gac gag tac aag gtg ccc agc aag aaa ttc 96
Gly Trp Ala Val Ile Thr Asp Glu Tyr Lys Val Pro Ser Lys Lys Phe

20 25 30

aag gtg ctg ggc aac acc gac cgg cac agc atc aag aag aac ctg atc 144
Lys Val Leu Gly Asn Thr Asp Arg His Ser Ile Lys Lys Asn Leu Ile
35 40 45

gga gcc ctg ctg ttc gac agc ggc gaa aca gcc gag gcc acc cgg ctg 192
Gly Ala Leu Leu Phe Asp Ser Gly Glu Thr Ala Glu Ala Thr Arg Leu
50 55 60

aag aga acc gcc aga aga aga tac acc aga cgg aag aac cgg atc tgc 240
Lys Arg Thr Ala Arg Arg Arg Tyr Thr Arg Arg Lys Asn Arg Ile Cys
65 70 75 80

tat ctg caa gag atc ttc agc aac gag atg gcc aag gtg gac gac agc 288
Tyr Leu Gln Glu Ile Phe Ser Asn Glu Met Ala Lys Val Asp Asp Ser
85 90 95

ttc ttc cac aga ctg gaa gag tcc ttc ctg gtg gaa gag gat aag aag 336
Phe Phe His Arg Leu Glu Glu Ser Phe Leu Val Glu Glu Asp Lys Lys
100 105 110

cac gag cgg cac ccc atc ttc ggc aac atc gtg gac gag gtg gcc tac 384
His Glu Arg His Pro Ile Phe Gly Asn Ile Val Asp Glu Val Ala Tyr
115 120 125

cac gag aag tac ccc acc atc tac cac ctg aga aag aaa ctg gtg gac 432
His Glu Lys Tyr Pro Thr Ile Tyr His Leu Arg Lys Lys Leu Val Asp
130 135 140

agc acc gac aag gcc gac ctg cgg ctg atc tat ctg gcc ctg gcc cac 480
Ser Thr Asp Lys Ala Asp Leu Arg Leu Ile Tyr Leu Ala Leu Ala His
145 150 155 160

atg atc aag ttc cgg ggc cac ttc ctg atc gag ggc gac ctg aac ccc 528
Met Ile Lys Phe Arg Gly His Phe Leu Ile Glu Gly Asp Leu Asn Pro
165 170 175

gac aac agc gac gtg gac aag ctg ttc atc cag ctg gtg cag acc tac 576
Asp Asn Ser Asp Val Asp Lys Leu Phe Ile Gln Leu Val Gln Thr Tyr
180 185 190

aac cag ctg ttc gag gaa aac ccc atc aac gcc agc ggc gtg gac gcc 624
Asn Gln Leu Phe Glu Glu Asn Pro Ile Asn Ala Ser Gly Val Asp Ala
195 200 205

aag gcc atc ctg tct gcc aga ctg agc aag agc aga cgg ctg gaa aat 672
Lys Ala Ile Leu Ser Ala Arg Leu Ser Lys Ser Arg Arg Leu Glu Asn
210 215 220

ctg atc gcc cag ctg ccc ggc gag aag aag aat ggc ctg ttc ggc aac 720
Leu Ile Ala Gln Leu Pro Gly Glu Lys Lys Asn Gly Leu Phe Gly Asn
225 230 235 240

ctg att gcc ctg agc ctg ggc ctg acc ccc aac ttc aag agc aac ttc 768
Leu Ile Ala Leu Ser Leu Gly Leu Thr Pro Asn Phe Lys Ser Asn Phe
245 250 255

gac ctg gcc gag gat gcc aaa ctg cag ctg agc aag gac acc tac gac 816
Asp Leu Ala Glu Asp Ala Lys Leu Gln Leu Ser Lys Asp Thr Tyr Asp
260 265 270

gac gac ctg gac aac ctg ctg gcc cag atc ggc gac cag tac gcc gac 864

```
Asp Asp Leu Asp Asn Leu Leu Ala Gln Ile Gly Asp Gln Tyr Ala Asp
        275                 280                 285

ctg ttt ctg gcc gcc aag aac ctg tcc gac gcc atc ctg ctg agc gac      912
Leu Phe Leu Ala Ala Lys Asn Leu Ser Asp Ala Ile Leu Leu Ser Asp
    290                 295                 300

atc ctg aga gtg aac acc gag atc acc aag gcc ccc ctg agc gcc tct      960
Ile Leu Arg Val Asn Thr Glu Ile Thr Lys Ala Pro Leu Ser Ala Ser
305                 310                 315                 320

atg atc aag aga tac gac gag cac cac cag gac ctg acc ctg ctg aaa     1008
Met Ile Lys Arg Tyr Asp Glu His His Gln Asp Leu Thr Leu Leu Lys
                325                 330                 335

gct ctc gtg cgg cag cag ctg cct gag aag tac aaa gag att ttc ttc     1056
Ala Leu Val Arg Gln Gln Leu Pro Glu Lys Tyr Lys Glu Ile Phe Phe
            340                 345                 350

gac cag agc aag aac ggc tac gcc ggc tac att gac ggc gga gcc agc     1104
Asp Gln Ser Lys Asn Gly Tyr Ala Gly Tyr Ile Asp Gly Gly Ala Ser
        355                 360                 365

cag gaa gag ttc tac aag ttc atc aag ccc atc ctg gaa aag atg gac     1152
Gln Glu Glu Phe Tyr Lys Phe Ile Lys Pro Ile Leu Glu Lys Met Asp
    370                 375                 380

ggc acc gag gaa ctg ctc gtg aag ctg aac aga gag gac ctg ctg cgg     1200
Gly Thr Glu Glu Leu Leu Val Lys Leu Asn Arg Glu Asp Leu Leu Arg
385                 390                 395                 400

aag cag cgg acc ttc gac aac ggc agc atc ccc cac cag atc cac ctg     1248
Lys Gln Arg Thr Phe Asp Asn Gly Ser Ile Pro His Gln Ile His Leu
                405                 410                 415

gga gag ctg cac gcc att ctg cgg cgg cag gaa gat ttt tac cca ttc     1296
Gly Glu Leu His Ala Ile Leu Arg Arg Gln Glu Asp Phe Tyr Pro Phe
            420                 425                 430

ctg aag gac aac cgg gaa aag atc gag aag atc ctg acc ttc cgc atc     1344
Leu Lys Asp Asn Arg Glu Lys Ile Glu Lys Ile Leu Thr Phe Arg Ile
        435                 440                 445

ccc tac tac gtg ggc cct ctg gcc agg gga aac agc aga ttc gcc tgg     1392
Pro Tyr Tyr Val Gly Pro Leu Ala Arg Gly Asn Ser Arg Phe Ala Trp
    450                 455                 460

atg acc aga aag agc gag gaa acc atc acc ccc tgg aac ttc gag gaa     1440
Met Thr Arg Lys Ser Glu Glu Thr Ile Thr Pro Trp Asn Phe Glu Glu
465                 470                 475                 480

gtg gtg gac aag ggc gct tcc gcc cag agc ttc atc gag cgg atg acc     1488
Val Val Asp Lys Gly Ala Ser Ala Gln Ser Phe Ile Glu Arg Met Thr
                485                 490                 495

aac ttc gat aag aac ctg ccc aac gag aag gtg ctg ccc aag cac agc     1536
Asn Phe Asp Lys Asn Leu Pro Asn Glu Lys Val Leu Pro Lys His Ser
            500                 505                 510

ctg ctg tac gag tac ttc acc gtg tat aac gag ctg acc aaa gtg aaa     1584
Leu Leu Tyr Glu Tyr Phe Thr Val Tyr Asn Glu Leu Thr Lys Val Lys
        515                 520                 525
```

```
tac gtg acc gag gga atg aga aag ccc gcc ttc ctg agc ggc gag cag        1632
Tyr Val Thr Glu Gly Met Arg Lys Pro Ala Phe Leu Ser Gly Glu Gln
    530                 535                 540

aaa aag gcc atc gtg gac ctg ctg ttc aag acc aac cgg aaa gtg acc        1680
Lys Lys Ala Ile Val Asp Leu Leu Phe Lys Thr Asn Arg Lys Val Thr
545                 550                 555                 560

gtg aag cag ctg aaa gag gac tac ttc aag aaa atc gag tgc ttc gac        1728
Val Lys Gln Leu Lys Glu Asp Tyr Phe Lys Lys Ile Glu Cys Phe Asp
                565                 570                 575

tcc gtg gaa atc tcc ggc gtg gaa gat cgg ttc aac gcc tcc ctg ggc        1776
Ser Val Glu Ile Ser Gly Val Glu Asp Arg Phe Asn Ala Ser Leu Gly
            580                 585                 590

aca tac cac gat ctg ctg aaa att atc aag gac aag gac ttc ctg gac        1824
Thr Tyr His Asp Leu Leu Lys Ile Ile Lys Asp Lys Asp Phe Leu Asp
        595                 600                 605

aat gag gaa aac gag gac att ctg gaa gat atc gtg ctg acc ctg aca        1872
Asn Glu Glu Asn Glu Asp Ile Leu Glu Asp Ile Val Leu Thr Leu Thr
    610                 615                 620

ctg ttt gag gac aga gag atg atc gag gaa cgg ctg aaa acc tat gcc        1920
Leu Phe Glu Asp Arg Glu Met Ile Glu Glu Arg Leu Lys Thr Tyr Ala
625                 630                 635                 640

cac ctg ttc gac gac aaa gtg atg aag cag ctg aag cgg cgg aga tac        1968
His Leu Phe Asp Asp Lys Val Met Lys Gln Leu Lys Arg Arg Arg Tyr
                645                 650                 655

acc ggc tgg ggc agg ctg agc cgg aag ctg atc aac ggc atc cgg gac        2016
Thr Gly Trp Gly Arg Leu Ser Arg Lys Leu Ile Asn Gly Ile Arg Asp
            660                 665                 670

aag cag tcc ggc aag aca atc ctg gat ttc ctg aag tcc gac ggc ttc        2064
Lys Gln Ser Gly Lys Thr Ile Leu Asp Phe Leu Lys Ser Asp Gly Phe
        675                 680                 685

gcc aac aga aac ttc atg cag ctg atc cac gac gac agc ctg acc ttt        2112
Ala Asn Arg Asn Phe Met Gln Leu Ile His Asp Asp Ser Leu Thr Phe
    690                 695                 700

aaa gag gac atc cag aaa gcc cag gtg tcc ggc cag ggc gat agc ctg        2160
Lys Glu Asp Ile Gln Lys Ala Gln Val Ser Gly Gln Gly Asp Ser Leu
705                 710                 715                 720

cac gag cac att gcc aat ctg gcc ggc agc ccc gcc att aag aag ggc        2208
His Glu His Ile Ala Asn Leu Ala Gly Ser Pro Ala Ile Lys Lys Gly
                725                 730                 735

atc ctg cag aca gtg aag gtg gtg gac gag ctc gtg aaa gtg atg ggc        2256
Ile Leu Gln Thr Val Lys Val Val Asp Glu Leu Val Lys Val Met Gly
            740                 745                 750

cgg cac aag ccc gag aac atc gtg atc gcc atg gcc aga gag aac cag        2304
Arg His Lys Pro Glu Asn Ile Val Ile Ala Met Ala Arg Glu Asn Gln
        755                 760                 765

acc acc cag aag gga cag aag aac agc cgc gag aga atg aag cgg atc        2352
Thr Thr Gln Lys Gly Gln Lys Asn Ser Arg Glu Arg Met Lys Arg Ile
    770                 775                 780
```

```
gaa gag ggc atc aaa gag ctg ggc agc cag atc ctg aaa gaa cac ccc        2400
Glu Glu Gly Ile Lys Glu Leu Gly Ser Gln Ile Leu Lys Glu His Pro
785                 790                 795                 800

gtg gaa aac acc cag ctg cag aac gag aag ctg tac ctg tac tac ctg        2448
Val Glu Asn Thr Gln Leu Gln Asn Glu Lys Leu Tyr Leu Tyr Tyr Leu
                805                 810                 815

cag aat ggg cgg gat atg tac gtg gac cag gaa ctg gac atc aac cgg        2496
Gln Asn Gly Arg Asp Met Tyr Val Asp Gln Glu Leu Asp Ile Asn Arg
            820                 825                 830

ctg tcc gac tac gat gtg gac gcc atc gtg cct cag agc ttt ctg aag        2544
Leu Ser Asp Tyr Asp Val Asp Ala Ile Val Pro Gln Ser Phe Leu Lys
        835                 840                 845

gac gac tcc atc gac gcc aag gtg ctg acc aga agc gac aag gcc cgg        2592
Asp Asp Ser Ile Asp Ala Lys Val Leu Thr Arg Ser Asp Lys Ala Arg
    850                 855                 860

ggc aag agc gac aac gtg ccc tcc gaa gag gtc gtg aag aag atg aag        2640
Gly Lys Ser Asp Asn Val Pro Ser Glu Glu Val Val Lys Lys Met Lys
865                 870                 875                 880

aac tac tgg cgg cag ctg ctg aac gcc aag ctg att acc cag aga aag        2688
Asn Tyr Trp Arg Gln Leu Leu Asn Ala Lys Leu Ile Thr Gln Arg Lys
                885                 890                 895

ttc gac aat ctg acc aag gcc gag aga ggc ggc ctg agc gaa ctg gat        2736
Phe Asp Asn Leu Thr Lys Ala Glu Arg Gly Gly Leu Ser Glu Leu Asp
            900                 905                 910

aag gcc ggc ttc atc aag aga cag ctg gtg gaa acc cgg cag atc aca        2784
Lys Ala Gly Phe Ile Lys Arg Gln Leu Val Glu Thr Arg Gln Ile Thr
        915                 920                 925

aag cac gtg gca cag atc ctg gac tcc cgg atg aac act aag tac gac        2832
Lys His Val Ala Gln Ile Leu Asp Ser Arg Met Asn Thr Lys Tyr Asp
    930                 935                 940

gag aat gac aag ctg atc cgg gaa gtg aaa gtg atc acc ctg aag tcc        2880
Glu Asn Asp Lys Leu Ile Arg Glu Val Lys Val Ile Thr Leu Lys Ser
945                 950                 955                 960

aag ctg gtg tcc gat ttc cgg aag gat ttc cag ttt tac aaa gtg cgc        2928
Lys Leu Val Ser Asp Phe Arg Lys Asp Phe Gln Phe Tyr Lys Val Arg
                965                 970                 975

gag atc aac aac tac cac cac gcc cac gcc gcc tac ctg aac gcc gtc        2976
Glu Ile Asn Asn Tyr His His Ala His Ala Ala Tyr Leu Asn Ala Val
            980                 985                 990

gtg gga acc gcc ctg atc aaa aag  tac cct aag ctg gaa  agc gag ttc      3024
Val Gly Thr Ala Leu Ile Lys Lys  Tyr Pro Lys Leu Glu  Ser Glu Phe
        995                 1000                1005

gtg tac  ggc gac tac aag gtg  tac gac gtg cgg aag  atg atc gcc        3069
Val Tyr  Gly Asp Tyr Lys Val  Tyr Asp Val Arg Lys  Met Ile Ala
    1010                1015                1020

aag agc  gag cag gaa atc ggc  aag gct acc gcc aag  tac ttc ttc        3114
Lys Ser  Glu Gln Glu Ile Gly  Lys Ala Thr Ala Lys  Tyr Phe Phe
```

1025 1030 1035

tac agc aac atc atg aac ttt ttc aag acc gag att acc ctg gcc 3159
Tyr Ser Asn Ile Met Asn Phe Phe Lys Thr Glu Ile Thr Leu Ala
1040 1045 1050

aac ggc gag atc cgg aag cgg cct ctg atc gag aca aac ggc gaa 3204
Asn Gly Glu Ile Arg Lys Arg Pro Leu Ile Glu Thr Asn Gly Glu
1055 1060 1065

acc ggg gag atc gtg tgg gat aag ggc cgg gat ttt gcc acc gtg 3249
Thr Gly Glu Ile Val Trp Asp Lys Gly Arg Asp Phe Ala Thr Val
1070 1075 1080

cgg aaa gtg ctg agc atg ccc caa gtg aat atc gtg aaa aag acc 3294
Arg Lys Val Leu Ser Met Pro Gln Val Asn Ile Val Lys Lys Thr
1085 1090 1095

gag gtg cag aca ggc ggc ttc agc aaa gag tct atc ctg ccc aag 3339
Glu Val Gln Thr Gly Gly Phe Ser Lys Glu Ser Ile Leu Pro Lys
1100 1105 1110

agg aac agc gat aag ctg atc gcc aga aag aag gac tgg gac cct 3384
Arg Asn Ser Asp Lys Leu Ile Ala Arg Lys Lys Asp Trp Asp Pro
1115 1120 1125

aag aag tac ggc ggc ttc gac agc ccc acc gtg gcc tat tct gtg 3429
Lys Lys Tyr Gly Gly Phe Asp Ser Pro Thr Val Ala Tyr Ser Val
1130 1135 1140

ctg gtg gtg gcc aaa gtg gaa aag ggc aag tcc aag aaa ctg aag 3474
Leu Val Val Ala Lys Val Glu Lys Gly Lys Ser Lys Lys Leu Lys
1145 1150 1155

agt gtg aaa gag ctg ctg ggg atc acc atc atg gaa aga agc agc 3519
Ser Val Lys Glu Leu Leu Gly Ile Thr Ile Met Glu Arg Ser Ser
1160 1165 1170

ttc gag aag aat ccc atc gac ttt ctg gaa gcc aag ggc tac aaa 3564
Phe Glu Lys Asn Pro Ile Asp Phe Leu Glu Ala Lys Gly Tyr Lys
1175 1180 1185

gaa gtg aaa aag gac ctg atc atc aag ctg cct aag tac tcc ctg 3609
Glu Val Lys Lys Asp Leu Ile Ile Lys Leu Pro Lys Tyr Ser Leu
1190 1195 1200

ttc gag ctg gaa aac ggc cgg aag aga atg ctg gcc tct gcc ggc 3654
Phe Glu Leu Glu Asn Gly Arg Lys Arg Met Leu Ala Ser Ala Gly
1205 1210 1215

gaa ctg cag aag gga aac gaa ctg gcc ctg ccc tcc aaa tat gtg 3699
Glu Leu Gln Lys Gly Asn Glu Leu Ala Leu Pro Ser Lys Tyr Val
1220 1225 1230

aac ttc ctg tac ctg gcc agc cac tat gag aag ctg aag ggc tcc 3744
Asn Phe Leu Tyr Leu Ala Ser His Tyr Glu Lys Leu Lys Gly Ser
1235 1240 1245

ccc gag gat aat gag cag aaa cag ctg ttt gtg gaa cag cac aag 3789
Pro Glu Asp Asn Glu Gln Lys Gln Leu Phe Val Glu Gln His Lys
1250 1255 1260

cac tac ctg gac gag atc atc gag cag atc agc gag ttc tcc aag 3834

```
His Tyr  Leu Asp Glu Ile Ile  Glu Gln Ile Ser Glu  Phe Ser Lys
    1265                 1270                 1275

aga gtg  atc ctg gcc gac gct  aat ctg gac aaa gtg  ctg tcc gcc         3879
Arg Val  Ile Leu Ala Asp Ala  Asn Leu Asp Lys Val  Leu Ser Ala
    1280                 1285                 1290

tac aac  aag cac cgg gat aag  ccc atc aga gag cag  gcc gag aat         3924
Tyr Asn  Lys His Arg Asp Lys  Pro Ile Arg Glu Gln  Ala Glu Asn
    1295                 1300                 1305

atc atc  cac ctg ttt acc ctg  acc aat ctg gga gcc  cct gcc gcc         3969
Ile Ile  His Leu Phe Thr Leu  Thr Asn Leu Gly Ala  Pro Ala Ala
    1310                 1315                 1320

ttc aag  tac ttt gac acc acc  atc gac cgg aag agg  tac acc agc         4014
Phe Lys  Tyr Phe Asp Thr Thr  Ile Asp Arg Lys Arg  Tyr Thr Ser
    1325                 1330                 1335

acc aaa  gag gtg ctg gac gcc  acc ctg atc cac cag  agc atc acc         4059
Thr Lys  Glu Val Leu Asp Ala  Thr Leu Ile His Gln  Ser Ile Thr
    1340                 1345                 1350

ggc ctg  tac gag aca cgg atc  gac ctg tct cag ctg  gga ggc gac         4104
Gly Leu  Tyr Glu Thr Arg Ile  Asp Leu Ser Gln Leu  Gly Gly Asp
    1355                 1360                 1365


<210> 297
<211> 1368
<212> PRT
<213> Homo sapiens

<400> 297
Met Asp Lys Lys Tyr Ser Ile Gly Leu Asp Ile Gly Thr Asn Ser Val
1               5                   10                  15


Gly Trp Ala Val Ile Thr Asp Glu Tyr Lys Val Pro Ser Lys Lys Phe
            20                  25                  30


Lys Val Leu Gly Asn Thr Asp Arg His Ser Ile Lys Lys Asn Leu Ile
        35                  40                  45


Gly Ala Leu Leu Phe Asp Ser Gly Glu Thr Ala Glu Ala Thr Arg Leu
    50                  55                  60


Lys Arg Thr Ala Arg Arg Arg Tyr Thr Arg Arg Lys Asn Arg Ile Cys
65                  70                  75                  80


Tyr Leu Gln Glu Ile Phe Ser Asn Glu Met Ala Lys Val Asp Asp Ser
                85                  90                  95


Phe Phe His Arg Leu Glu Glu Ser Phe Leu Val Glu Glu Asp Lys Lys
            100                 105                 110


His Glu Arg His Pro Ile Phe Gly Asn Ile Val Asp Glu Val Ala Tyr
```

115 120 125

His Glu Lys Tyr Pro Thr Ile Tyr His Leu Arg Lys Lys Leu Val Asp
130 135 140

Ser Thr Asp Lys Ala Asp Leu Arg Leu Ile Tyr Leu Ala Leu Ala His
145 150 155 160

Met Ile Lys Phe Arg Gly His Phe Leu Ile Glu Gly Asp Leu Asn Pro
165 170 175

Asp Asn Ser Asp Val Asp Lys Leu Phe Ile Gln Leu Val Gln Thr Tyr
180 185 190

Asn Gln Leu Phe Glu Glu Asn Pro Ile Asn Ala Ser Gly Val Asp Ala
195 200 205

Lys Ala Ile Leu Ser Ala Arg Leu Ser Lys Ser Arg Arg Leu Glu Asn
210 215 220

Leu Ile Ala Gln Leu Pro Gly Glu Lys Lys Asn Gly Leu Phe Gly Asn
225 230 235 240

Leu Ile Ala Leu Ser Leu Gly Leu Thr Pro Asn Phe Lys Ser Asn Phe
245 250 255

Asp Leu Ala Glu Asp Ala Lys Leu Gln Leu Ser Lys Asp Thr Tyr Asp
260 265 270

Asp Asp Leu Asp Asn Leu Leu Ala Gln Ile Gly Asp Gln Tyr Ala Asp
275 280 285

Leu Phe Leu Ala Ala Lys Asn Leu Ser Asp Ala Ile Leu Leu Ser Asp
290 295 300

Ile Leu Arg Val Asn Thr Glu Ile Thr Lys Ala Pro Leu Ser Ala Ser
305 310 315 320

Met Ile Lys Arg Tyr Asp Glu His His Gln Asp Leu Thr Leu Leu Lys
325 330 335

Ala Leu Val Arg Gln Gln Leu Pro Glu Lys Tyr Lys Glu Ile Phe Phe
340 345 350

Asp Gln Ser Lys Asn Gly Tyr Ala Gly Tyr Ile Asp Gly Gly Ala Ser
355 360 365

Gln Glu Glu Phe Tyr Lys Phe Ile Lys Pro Ile Leu Glu Lys Met Asp
370 375 380

Gly Thr Glu Glu Leu Leu Val Lys Leu Asn Arg Glu Asp Leu Leu Arg
385 390 395 400

Lys Gln Arg Thr Phe Asp Asn Gly Ser Ile Pro His Gln Ile His Leu
405 410 415

Gly Glu Leu His Ala Ile Leu Arg Arg Gln Glu Asp Phe Tyr Pro Phe
420 425 430

Leu Lys Asp Asn Arg Glu Lys Ile Glu Lys Ile Leu Thr Phe Arg Ile
435 440 445

Pro Tyr Tyr Val Gly Pro Leu Ala Arg Gly Asn Ser Arg Phe Ala Trp
450 455 460

Met Thr Arg Lys Ser Glu Glu Thr Ile Thr Pro Trp Asn Phe Glu Glu
465 470 475 480

Val Val Asp Lys Gly Ala Ser Ala Gln Ser Phe Ile Glu Arg Met Thr
485 490 495

Asn Phe Asp Lys Asn Leu Pro Asn Glu Lys Val Leu Pro Lys His Ser
500 505 510

Leu Leu Tyr Glu Tyr Phe Thr Val Tyr Asn Glu Leu Thr Lys Val Lys
515 520 525

Tyr Val Thr Glu Gly Met Arg Lys Pro Ala Phe Leu Ser Gly Glu Gln
530 535 540

Lys Lys Ala Ile Val Asp Leu Leu Phe Lys Thr Asn Arg Lys Val Thr
545 550 555 560

Val Lys Gln Leu Lys Glu Asp Tyr Phe Lys Lys Ile Glu Cys Phe Asp
565 570 575

Ser Val Glu Ile Ser Gly Val Glu Asp Arg Phe Asn Ala Ser Leu Gly
580 585 590

Thr Tyr His Asp Leu Leu Lys Ile Ile Lys Asp Lys Asp Phe Leu Asp
595 600 605

Asn Glu Glu Asn Glu Asp Ile Leu Glu Asp Ile Val Leu Thr Leu Thr
610 615 620

Leu Phe Glu Asp Arg Glu Met Ile Glu Glu Arg Leu Lys Thr Tyr Ala
625 630 635 640

His Leu Phe Asp Asp Lys Val Met Lys Gln Leu Lys Arg Arg Arg Tyr
645 650 655

Thr Gly Trp Gly Arg Leu Ser Arg Lys Leu Ile Asn Gly Ile Arg Asp
660 665 670

Lys Gln Ser Gly Lys Thr Ile Leu Asp Phe Leu Lys Ser Asp Gly Phe
675 680 685

Ala Asn Arg Asn Phe Met Gln Leu Ile His Asp Asp Ser Leu Thr Phe
690 695 700

Lys Glu Asp Ile Gln Lys Ala Gln Val Ser Gly Gln Gly Asp Ser Leu
705 710 715 720

His Glu His Ile Ala Asn Leu Ala Gly Ser Pro Ala Ile Lys Lys Gly
725 730 735

Ile Leu Gln Thr Val Lys Val Val Asp Glu Leu Val Lys Val Met Gly
740 745 750

Arg His Lys Pro Glu Asn Ile Val Ile Ala Met Ala Arg Glu Asn Gln
755 760 765

Thr Thr Gln Lys Gly Gln Lys Asn Ser Arg Glu Arg Met Lys Arg Ile
770 775 780

Glu Glu Gly Ile Lys Glu Leu Gly Ser Gln Ile Leu Lys Glu His Pro
785 790 795 800

Val Glu Asn Thr Gln Leu Gln Asn Glu Lys Leu Tyr Leu Tyr Tyr Leu
805 810 815

Gln Asn Gly Arg Asp Met Tyr Val Asp Gln Glu Leu Asp Ile Asn Arg
820 825 830

Leu Ser Asp Tyr Asp Val Asp Ala Ile Val Pro Gln Ser Phe Leu Lys
835 840 845

Asp Asp Ser Ile Asp Ala Lys Val Leu Thr Arg Ser Asp Lys Ala Arg
850 855 860

Gly Lys Ser Asp Asn Val Pro Ser Glu Glu Val Val Lys Lys Met Lys
865 870 875 880

```
Asn Tyr Trp Arg Gln Leu Leu Asn Ala Lys Leu Ile Thr Gln Arg Lys
                885                 890                 895

Phe Asp Asn Leu Thr Lys Ala Glu Arg Gly Gly Leu Ser Glu Leu Asp
            900                 905                 910

Lys Ala Gly Phe Ile Lys Arg Gln Leu Val Glu Thr Arg Gln Ile Thr
        915                 920                 925

Lys His Val Ala Gln Ile Leu Asp Ser Arg Met Asn Thr Lys Tyr Asp
    930                 935                 940

Glu Asn Asp Lys Leu Ile Arg Glu Val Lys Val Ile Thr Leu Lys Ser
945                 950                 955                 960

Lys Leu Val Ser Asp Phe Arg Lys Asp Phe Gln Phe Tyr Lys Val Arg
                965                 970                 975

Glu Ile Asn Asn Tyr His His Ala His Ala Ala Tyr Leu Asn Ala Val
            980                 985                 990

Val Gly Thr Ala Leu Ile Lys Lys  Tyr Pro Lys Leu Glu  Ser Glu Phe
        995                 1000                1005

Val Tyr  Gly Asp Tyr Lys Val  Tyr Asp Val Arg Lys  Met Ile Ala
    1010                 1015                 1020

Lys Ser  Glu Gln Glu Ile Gly  Lys Ala Thr Ala Lys  Tyr Phe Phe
    1025                 1030                 1035

Tyr Ser  Asn Ile Met Asn Phe  Phe Lys Thr Glu Ile  Thr Leu Ala
    1040                 1045                 1050

Asn Gly  Glu Ile Arg Lys Arg  Pro Leu Ile Glu Thr  Asn Gly Glu
    1055                 1060                 1065

Thr Gly  Glu Ile Val Trp Asp  Lys Gly Arg Asp Phe  Ala Thr Val
    1070                 1075                 1080

Arg Lys  Val Leu Ser Met Pro  Gln Val Asn Ile Val  Lys Lys Thr
    1085                 1090                 1095

Glu Val  Gln Thr Gly Gly Phe  Ser Lys Glu Ser Ile  Leu Pro Lys
    1100                 1105                 1110

Arg Asn  Ser Asp Lys Leu Ile  Ala Arg Lys Lys Asp  Trp Asp Pro
```

1115 1120 1125

Lys Lys Tyr Gly Gly Phe Asp Ser Pro Thr Val Ala Tyr Ser Val
1130 1135 1140

Leu Val Val Ala Lys Val Glu Lys Gly Lys Ser Lys Lys Leu Lys
1145 1150 1155

Ser Val Lys Glu Leu Leu Gly Ile Thr Ile Met Glu Arg Ser Ser
1160 1165 1170

Phe Glu Lys Asn Pro Ile Asp Phe Leu Glu Ala Lys Gly Tyr Lys
1175 1180 1185

Glu Val Lys Lys Asp Leu Ile Ile Lys Leu Pro Lys Tyr Ser Leu
1190 1195 1200

Phe Glu Leu Glu Asn Gly Arg Lys Arg Met Leu Ala Ser Ala Gly
1205 1210 1215

Glu Leu Gln Lys Gly Asn Glu Leu Ala Leu Pro Ser Lys Tyr Val
1220 1225 1230

Asn Phe Leu Tyr Leu Ala Ser His Tyr Glu Lys Leu Lys Gly Ser
1235 1240 1245

Pro Glu Asp Asn Glu Gln Lys Gln Leu Phe Val Glu Gln His Lys
1250 1255 1260

His Tyr Leu Asp Glu Ile Ile Glu Gln Ile Ser Glu Phe Ser Lys
1265 1270 1275

Arg Val Ile Leu Ala Asp Ala Asn Leu Asp Lys Val Leu Ser Ala
1280 1285 1290

Tyr Asn Lys His Arg Asp Lys Pro Ile Arg Glu Gln Ala Glu Asn
1295 1300 1305

Ile Ile His Leu Phe Thr Leu Thr Asn Leu Gly Ala Pro Ala Ala
1310 1315 1320

Phe Lys Tyr Phe Asp Thr Thr Ile Asp Arg Lys Arg Tyr Thr Ser
1325 1330 1335

Thr Lys Glu Val Leu Asp Ala Thr Leu Ile His Gln Ser Ile Thr
1340 1345 1350

Gly Leu Tyr Glu Thr Arg Ile Asp Leu Ser Gln Leu Gly Gly Asp
    1355                1360                1365

<210> 298
<211> 57
<212> DNA
<213> Homo sapiens

<220>
<221> CDS
<222> (1)..(57)

<400> 298
ggc acc att aaa gaa aat atc att ggt gtt tcc tat gat gaa tat aga 48
Gly Thr Ile Lys Glu Asn Ile Ile Gly Val Ser Tyr Asp Glu Tyr Arg
1               5                   10                  15

tac aga agc 57
Tyr Arg Ser

<210> 299
<211> 19
<212> PRT
<213> Homo sapiens

<400> 299
Gly Thr Ile Lys Glu Asn Ile Ile Gly Val Ser Tyr Asp Glu Tyr Arg
1               5                   10                  15

Tyr Arg Ser

<210> 300
<211> 99
<212> RNA
<213> Artificial Sequence

<220>
<221> source
<223> /note="Description of Artificial Sequence: Synthetic oligonucleotide"

<220>
<221> modified_base
<222> (1)..(20)
<223> a, c, u, g, unknown or other

<400> 300
nnnnnnnnnn nnnnnnnnnn guuuuagagc uagaaauagc aaguuaaaau aaggcuaguc 60

cguuaucaac uugaaaaagu ggcaccgagu cggugcuuu 99

<210> 301
<211> 48
<212> DNA

<213> Artificial Sequence

<220>
<221> source
<223> /note="Description of Artificial Sequence: Synthetic oligonucleotide"

<220>
<221> CDS
<222> (1)..(48)

<400> 301
att aaa gaa aat atc att ggc ttt gtt tcc tat gat gaa tat aga tac 48
Ile Lys Glu Asn Ile Ile Gly Phe Val Ser Tyr Asp Glu Tyr Arg Tyr
1 5 10 15

<210> 302
<211> 16
<212> PRT
<213> Artificial Sequence

<220>
<221> source
<223> /note="Description of Artificial Sequence: Synthetic peptide"

<400> 302
Ile Lys Glu Asn Ile Ile Gly Phe Val Ser Tyr Asp Glu Tyr Arg Tyr
1 5 10 15

<210> 303
<211> 27
<212> DNA
<213> Artificial Sequence

<220>
<221> source
<223> /note="Description of Artificial Sequence: Synthetic oligonucleotide"

<400> 303
ccccttccgg ttcagggacc ccgacct 27

<210> 304
<211> 26
<212> DNA
<213> Artificial Sequence

<220>
<221> source
<223> /note="Description of Artificial Sequence: Synthetic oligonucleotide"

<400> 304
ccccttccgt tcagggaccc cgacct 26

<210> 305
<211> 21

<212> DNA
<213> Artificial Sequence

<220>
<221> source
<223> /note="Description of Artificial Sequence: Synthetic oligonucleotide"

<400> 305
ccccttccgg gaccccgacc t 21

<210> 306
<211> 28
<212> DNA
<213> Artificial Sequence

<220>
<221> source
<223> /note="Description of Artificial Sequence: Synthetic oligonucleotide"

<400> 306
ccccttccgg gttcagggac cccgacct 28

<210> 307
<211> 29
<212> DNA
<213> Artificial Sequence

<220>
<221> source
<223> /note="Description of Artificial Sequence: Synthetic oligonucleotide"

<400> 307
ccccttccgg ggttcaggga ccccgacct 29

<210> 308
<211> 24
<212> DNA
<213> Artificial Sequence

<220>
<221> source
<223> /note="Description of Artificial Sequence: Synthetic oligonucleotide"

<400> 308
gcttacctgg gctgtagaac aggc 24

<210> 309
<211> 20
<212> DNA
<213> Artificial Sequence

<220>
<221> source
<223> /note="Description of Artificial Sequence: Synthetic oligonucleotide"

<400> 309
gcttacctgg gctaacaggc 20

<210> 310
<211> 22
<212> DNA
<213> Artificial Sequence

<220>
<221> source
<223> /note="Description of Artificial Sequence: Synthetic oligonucleotide"

<400> 310
gcttacctgg gctgtaacag gc 22

<210> 311
<211> 25
<212> DNA
<213> Artificial Sequence

<220>
<221> source
<223> /note="Description of Artificial Sequence: Synthetic oligonucleotide"

<400> 311
gcttacctgg gctgtaggaa caggc 25

<210> 312
<211> 26
<212> DNA
<213> Artificial Sequence

<220>
<221> source
<223> /note="Description of Artificial Sequence: Synthetic oligonucleotide"

<400> 312
gcttacctgg gctgtaggca acaggc 26

<210> 313
<211> 24
<212> DNA
<213> Artificial Sequence

<220>
<221> source
<223> /note="Description of Artificial Sequence: Synthetic oligonucleotide"

<400> 313
aagacggaca ggcacctacg gggt 24

<210> 314
<211> 16

<212> DNA
<213> Artificial Sequence

<220>
<221> source
<223> /note="Description of Artificial Sequence: Synthetic oligonucleotide"

<400> 314
aagacggaca cggggt 16

<210> 315
<211> 20
<212> DNA
<213> Artificial Sequence

<220>
<221> source
<223> /note="Description of Artificial Sequence: Synthetic oligonucleotide"

<400> 315
aagacggaca ggcacggggt 20

<210> 316
<211> 25
<212> DNA
<213> Artificial Sequence

<220>
<221> source
<223> /note="Description of Artificial Sequence: Synthetic oligonucleotide"

<400> 316
aagacggaca ggcaccttac ggggt 25

<210> 317
<211> 28
<212> DNA
<213> Artificial Sequence

<220>
<221> source
<223> /note="Description of Artificial Sequence: Synthetic oligonucleotide"

<400> 317
aagacggaca ggcacctcct aacggggt 28

SEQUENCE LISTING

<110> THE BROAD INSTITUTE INC.
MASSACHUSETTS INSTITUTE OF TECHNOLOGY

<120> DELIVERY, USE AND THERAPEUTIC APPLICATIONS OF THE CRISPR-CAS SYSTEMS AND COMPOSITIONS FOR TARGETING DISORDERS AND DISEASES USING PARTICLE DELIVERY COMPONENTS

<130> 47627.99.2060

<140> PCT/US2014/070057
<141> 2014-12-12

<150> 62/054,490
<151> 2014-09-24

<150> 62/010,441
<151> 2014-06-10

<150> 61/915,118
<151> 2013-12-12

<150> 61/915,215
<151> 2013-12-12

<150> 61/915,148
<151> 2013-12-12

<160> 317

<170> PatentIn version 3.5

<210> 1
<211> 23
<212> DNA
<213> Artificial Sequence

<220>
<221> source
<223> /note="Description of Artificial Sequence: Synthetic oligonucleotide"

<220>
<221> modified_base
<222> (1)..(20)
<223> a, c, t or g

<220>
<221> modified_base
<222> (21)..(21)
<223> a, c, t, g, unknown or other

<400> 1
nnnnnnnnnn nnnnnnnnnn ngg 23

<210> 2
<211> 15
<212> DNA
<213> Artificial Sequence

<220>
<221> source
<223> /note="Description of Artificial Sequence: Synthetic
oligonucleotide"

<220>
<221> modified_base
<222> (1)..(12)
<223> a, c, t or g

<220>
<221> modified_base
<222> (13)..(13)
<223> a, c, t, g, unknown or other

<400> 2
nnnnnnnnnn nnngg 15

<210> 3
<211> 23
<212> DNA
<213> Artificial Sequence

<220>
<221> source
<223> /note="Description of Artificial Sequence: Synthetic
oligonucleotide"

<220>
<221> modified_base
<222> (1)..(20)
<223> a, c, t or g

<220>
<221> modified_base
<222> (21)..(21)
<223> a, c, t, g, unknown or other

<400> 3
nnnnnnnnnn nnnnnnnnnn ngg 23

<210> 4
<211> 14
<212> DNA
<213> Artificial Sequence

<220>
<221> source
<223> /note="Description of Artificial Sequence: Synthetic
oligonucleotide"

<220>
<221> modified_base
<222> (1)..(11)
<223> a, c, t or g

<220>
<221> modified_base

<222> (12)..(12)
<223> a, c, t, g, unknown or other

<400> 4
nnnnnnnnnn nngg 14

<210> 5
<211> 27
<212> DNA
<213> Artificial Sequence

<220>
<221> source
<223> /note="Description of Artificial Sequence: Synthetic oligonucleotide"

<220>
<221> modified_base
<222> (1)..(20)
<223> a, c, t or g

<220>
<221> modified_base
<222> (21)..(22)
<223> a, c, t, g, unknown or other

<400> 5
nnnnnnnnnn nnnnnnnnnn nnagaaw 27

<210> 6
<211> 19
<212> DNA
<213> Artificial Sequence

<220>
<221> source
<223> /note="Description of Artificial Sequence: Synthetic oligonucleotide"

<220>
<221> modified_base
<222> (1)..(12)
<223> a, c, t or g

<220>
<221> modified_base
<222> (13)..(14)
<223> a, c, t, g, unknown or other

<400> 6
nnnnnnnnnn nnnnagaaw 19

<210> 7
<211> 27
<212> DNA
<213> Artificial Sequence

<220>

<221> source
<223> /note="Description of Artificial Sequence: Synthetic oligonucleotide"

<220>
<221> modified_base
<222> (1)..(20)
<223> a, c, t or g

<220>
<221> modified_base
<222> (21)..(22)
<223> a, c, t, g, unknown or other

<400> 7
nnnnnnnnnn nnnnnnnnnn nnagaaw 27

<210> 8
<211> 18
<212> DNA
<213> Artificial Sequence

<220>
<221> source
<223> /note="Description of Artificial Sequence: Synthetic oligonucleotide"

<220>
<221> modified_base
<222> (1)..(11)
<223> a, c, t or g

<220>
<221> modified_base
<222> (12)..(13)
<223> a, c, t, g, unknown or other

<400> 8
nnnnnnnnnn nnnagaaw 18

<210> 9
<211> 25
<212> DNA
<213> Artificial Sequence

<220>
<221> source
<223> /note="Description of Artificial Sequence: Synthetic oligonucleotide"

<220>
<221> modified_base
<222> (1)..(20)
<223> a, c, t or g

<220>
<221> modified_base
<222> (21)..(21)

```
<223> a, c, t, g, unknown or other

<220>
<221> modified_base
<222> (24)..(24)
<223> a, c, t, g, unknown or other

<400> 9
nnnnnnnnnn nnnnnnnnnn nggng                                            25


<210> 10
<211> 17
<212> DNA
<213> Artificial Sequence

<220>
<221> source
<223> /note="Description of Artificial Sequence: Synthetic
      oligonucleotide"


<220>
<221> modified_base
<222> (1)..(12)
<223> a, c, t or g

<220>
<221> modified_base
<222> (13)..(13)
<223> a, c, t, g, unknown or other

<220>
<221> modified_base
<222> (16)..(16)
<223> a, c, t, g, unknown or other

<400> 10
nnnnnnnnnn nnnggng                                                     17


<210> 11
<211> 25
<212> DNA
<213> Artificial Sequence

<220>
<221> source
<223> /note="Description of Artificial Sequence: Synthetic
      oligonucleotide"


<220>
<221> modified_base
<222> (1)..(20)
<223> a, c, t or g

<220>
<221> modified_base
<222> (21)..(21)
<223> a, c, t, g, unknown or other

<220>
```

<221> modified_base
<222> (24)..(24)
<223> a, c, t, g, unknown or other

<400> 11
nnnnnnnnnn nnnnnnnnnn nggng 25

<210> 12
<211> 16
<212> DNA
<213> Artificial Sequence

<220>
<221> source
<223> /note="Description of Artificial Sequence: Synthetic oligonucleotide"

<220>
<221> modified_base
<222> (1)..(11)
<223> a, c, t or g

<220>
<221> modified_base
<222> (12)..(12)
<223> a, c, t, g, unknown or other

<220>
<221> modified_base
<222> (15)..(15)
<223> a, c, t, g, unknown or other

<400> 12
nnnnnnnnnn nnggng 16

<210> 13
<211> 137
<212> DNA
<213> Artificial Sequence

<220>
<221> source
<223> /note="Description of Artificial Sequence: Synthetic polynucleotide"

<220>
<221> modified_base
<222> (1)..(20)
<223> a, c, t, g, unknown or other

<400> 13
nnnnnnnnnn nnnnnnnnnn gtttttgtac tctcaagatt tagaaataaa tcttgcagaa 60

gctacaaaga taaggcttca tgccgaaatc aacaccctgt cattttatgg cagggtgttt 120

tcgttattta atttttt 137

<210> 14

<211> 123
<212> DNA
<213> Artificial Sequence

<220>
<221> source
<223> /note="Description of Artificial Sequence: Synthetic
polynucleotide"

<220>
<221> modified_base
<222> (1)..(20)
<223> a, c, t, g, unknown or other

<400> 14
nnnnnnnnnn nnnnnnnnnn gtttttgtac tctcagaaat gcagaagcta caaagataag 60

gcttcatgcc gaaatcaaca ccctgtcatt ttatggcagg gtgttttcgt tatttaattt 120

ttt 123

<210> 15
<211> 110
<212> DNA
<213> Artificial Sequence

<220>
<221> source
<223> /note="Description of Artificial Sequence: Synthetic
polynucleotide"

<220>
<221> modified_base
<222> (1)..(20)
<223> a, c, t, g, unknown or other

<400> 15
nnnnnnnnnn nnnnnnnnnn gtttttgtac tctcagaaat gcagaagcta caaagataag 60

gcttcatgcc gaaatcaaca ccctgtcatt ttatggcagg gtgttttttt 110

<210> 16
<211> 102
<212> DNA
<213> Artificial Sequence

<220>
<221> source
<223> /note="Description of Artificial Sequence: Synthetic
polynucleotide"

<220>
<221> modified_base
<222> (1)..(20)
<223> a, c, t, g, unknown or other

<400> 16
nnnnnnnnnn nnnnnnnnnn gttttagagc tagaaatagc aagttaaaat aaggctagtc 60

cgttatcaac ttgaaaaagt ggcaccgagt cggtgctttt tt 102

<210> 17
<211> 88
<212> DNA
<213> Artificial Sequence

<220>
<221> source
<223> /note="Description of Artificial Sequence: Synthetic
oligonucleotide"

<220>
<221> modified_base
<222> (1)..(20)
<223> a, c, t, g, unknown or other

<400> 17
nnnnnnnnnn nnnnnnnnnn gttttagagc tagaaatagc aagttaaaat aaggctagtc 60

cgttatcaac ttgaaaaagt gttttttt 88

<210> 18
<211> 76
<212> DNA
<213> Artificial Sequence

<220>
<221> source
<223> /note="Description of Artificial Sequence: Synthetic
oligonucleotide"

<220>
<221> modified_base
<222> (1)..(20)
<223> a, c, t, g, unknown or other

<400> 18
nnnnnnnnnn nnnnnnnnnn gttttagagc tagaaatagc aagttaaaat aaggctagtc 60

cgttatcatt tttttt 76

<210> 19
<211> 20
<212> DNA
<213> Homo sapiens

<400> 19
gagtccgagc agaagaagaa 20

<210> 20
<211> 20
<212> DNA
<213> Homo sapiens

<400> 20

gagtcctagc aggagaagaa 20

<210> 21
<211> 20
<212> DNA
<213> Homo sapiens

<400> 21
gagtctaagc agaagaagaa 20

<210> 22
<211> 7
<212> PRT
<213> Unknown

<220>
<221> source
<223> /note="Description of Unknown:
Simian virus 40"

<400> 22
Pro Lys Lys Lys Arg Lys Val
1 5

<210> 23
<211> 16
<212> PRT
<213> Unknown

<220>
<221> source
<223> /note="Description of Unknown:
Nucleoplasmin bipartite NLS sequence"

<400> 23
Lys Arg Pro Ala Ala Thr Lys Lys Ala Gly Gln Ala Lys Lys Lys Lys
1 5 10 15

<210> 24
<211> 9
<212> PRT
<213> Unknown

<220>
<221> source
<223> /note="Description of Unknown:
C-myc NLS sequence"

<400> 24
Pro Ala Ala Lys Arg Val Lys Leu Asp
1 5

<210> 25
<211> 11
<212> PRT
<213> Unknown

<220>

<221> source
<223> /note="Description of Unknown:
C-myc NLS sequence"

<400> 25
Arg Gln Arg Arg Asn Glu Leu Lys Arg Ser Pro
1 5 10

<210> 26
<211> 38
<212> PRT
<213> Homo sapiens

<400> 26
Asn Gln Ser Ser Asn Phe Gly Pro Met Lys Gly Gly Asn Phe Gly Gly
1 5 10 15

Arg Ser Ser Gly Pro Tyr Gly Gly Gly Gly Gln Tyr Phe Ala Lys Pro
20 25 30

Arg Asn Gln Gly Gly Tyr
35

<210> 27
<211> 42
<212> PRT
<213> Unknown

<220>
<221> source
<223> /note="Description of Unknown:
IBB domain from importin-alpha sequence"

<400> 27
Arg Met Arg Ile Glx Phe Lys Asn Lys Gly Lys Asp Thr Ala Glu Leu
1 5 10 15

Arg Arg Arg Arg Val Glu Val Ser Val Glu Leu Arg Lys Ala Lys Lys
20 25 30

Asp Glu Gln Ile Leu Lys Arg Arg Asn Val
35 40

<210> 28
<211> 8
<212> PRT
<213> Unknown

<220>
<221> source
<223> /note="Description of Unknown:
Myoma T protein sequence"

<400> 28
Val Ser Arg Lys Arg Pro Arg Pro
1 5

<210> 29
<211> 8
<212> PRT
<213> Unknown

<220>
<221> source
<223> /note="Description of Unknown:
Myoma T protein sequence"

<400> 29
Pro Pro Lys Lys Ala Arg Glu Asp
1 5

<210> 30
<211> 8
<212> PRT
<213> Homo sapiens

<400> 30
Pro Gln Pro Lys Lys Lys Pro Leu
1 5

<210> 31
<211> 12
<212> PRT
<213> Mus musculus

<400> 31
Ser Ala Leu Ile Lys Lys Lys Lys Lys Met Ala Pro
1 5 10

<210> 32
<211> 5
<212> PRT
<213> Influenza virus

<400> 32
Asp Arg Leu Arg Arg
1 5

<210> 33
<211> 7
<212> PRT
<213> Influenza virus

<400> 33
Pro Lys Gln Lys Lys Arg Lys
1 5

<210> 34
<211> 10
<212> PRT
<213> Hepatitis delta virus

<400> 34

Arg Lys Leu Lys Lys Lys Ile Lys Lys Leu
1 5 10

<210> 35
<211> 10
<212> PRT
<213> Mus musculus

<400> 35
Arg Glu Lys Lys Lys Phe Leu Lys Arg Arg
1 5 10

<210> 36
<211> 20
<212> PRT
<213> Homo sapiens

<400> 36
Lys Arg Lys Gly Asp Glu Val Asp Gly Val Asp Glu Val Ala Lys Lys
1 5 10 15

Lys Ser Lys Lys
20

<210> 37
<211> 17
<212> PRT
<213> Homo sapiens

<400> 37
Arg Lys Cys Leu Gln Ala Gly Met Asn Leu Glu Ala Arg Lys Thr Lys
1 5 10 15

Lys

<210> 38
<211> 60
<212> RNA
<213> Artificial Sequence

<220>
<221> source
<223> /note="Description of Artificial Sequence: Synthetic oligonucleotide"

<220>
<221> modified_base
<222> (1)..(20)
<223> a, c, u, g, unknown or other

<220>
<221> modified_base
<222> (23)..(44)
<223> a, c, u, g, unknown or other

<220>
<221> modified_base
<222> (47)..(60)
<223> a, c, u, g, unknown or other

<400> 38
nnnnnnnnnn nnnnnnnnnn ccnnnnnnnn nnnnnnnnnn nnnnggnnnn nnnnnnnnnn 60

<210> 39
<211> 60
<212> RNA
<213> Artificial Sequence

<220>
<221> source
<223> /note="Description of Artificial Sequence: Synthetic oligonucleotide"

<220>
<221> modified_base
<222> (1)..(14)
<223> a, c, u, g, unknown or other

<220>
<221> modified_base
<222> (17)..(38)
<223> a, c, u, g, unknown or other

<220>
<221> modified_base
<222> (41)..(60)
<223> a, c, u, g, unknown or other

<400> 39
nnnnnnnnnn nnnnccnnnn nnnnnnnnnn nnnnnnnngg nnnnnnnnnn nnnnnnnnnn 60

<210> 40
<211> 60
<212> RNA
<213> Artificial Sequence

<220>
<221> source
<223> /note="Description of Artificial Sequence: Synthetic oligonucleotide"

<220>
<221> modified_base
<222> (1)..(20)
<223> a, c, u, g, unknown or other

<220>
<221> modified_base
<222> (23)..(43)
<223> a, c, u, g, unknown or other

<220>
<221> modified_base
<222> (46)..(60)

<223> a, c, u, g, unknown or other

<400> 40
nnnnnnnnnn nnnnnnnnnn ccnnnnnnnn nnnnnnnnnn nnnggnnnnn nnnnnnnnnn 60

<210> 41
<211> 60
<212> RNA
<213> Artificial Sequence

<220>
<221> source
<223> /note="Description of Artificial Sequence: Synthetic oligonucleotide"

<220>
<221> modified_base
<222> (1)..(15)
<223> a, c, u, g, unknown or other

<220>
<221> modified_base
<222> (18)..(38)
<223> a, c, u, g, unknown or other

<220>
<221> modified_base
<222> (41)..(60)
<223> a, c, u, g, unknown or other

<400> 41
nnnnnnnnnn nnnnnccnnn nnnnnnnnnn nnnnnnnngg nnnnnnnnnn nnnnnnnnnn 60

<210> 42
<211> 60
<212> RNA
<213> Artificial Sequence

<220>
<221> source
<223> /note="Description of Artificial Sequence: Synthetic oligonucleotide"

<220>
<221> modified_base
<222> (1)..(20)
<223> a, c, u, g, unknown or other

<220>
<221> modified_base
<222> (23)..(42)
<223> a, c, u, g, unknown or other

<220>
<221> modified_base
<222> (45)..(60)
<223> a, c, u, g, unknown or other

<400> 42

nnnnnnnnnn nnnnnnnnnn ccnnnnnnnn nnnnnnnnnn nnggnnnnnn nnnnnnnnnn 60

<210> 43
<211> 60
<212> RNA
<213> Artificial Sequence

<220>
<221> source
<223> /note="Description of Artificial Sequence: Synthetic oligonucleotide"

<220>
<221> modified_base
<222> (1)..(15)
<223> a, c, u, g, unknown or other

<220>
<221> modified_base
<222> (18)..(38)
<223> a, c, u, g, unknown or other

<220>
<221> modified_base
<222> (41)..(60)
<223> a, c, u, g, unknown or other

<400> 43
nnnnnnnnnn nnnnnccnnn nnnnnnnnnn nnnnnnnngg nnnnnnnnnn nnnnnnnnnn 60

<210> 44
<211> 60
<212> RNA
<213> Artificial Sequence

<220>
<221> source
<223> /note="Description of Artificial Sequence: Synthetic oligonucleotide"

<220>
<221> modified_base
<222> (1)..(20)
<223> a, c, u, g, unknown or other

<220>
<221> modified_base
<222> (23)..(41)
<223> a, c, u, g, unknown or other

<220>
<221> modified_base
<222> (44)..(60)
<223> a, c, u, g, unknown or other

<400> 44
nnnnnnnnnn nnnnnnnnnn ccnnnnnnnn nnnnnnnnnn nggnnnnnnn nnnnnnnnnn 60

<210> 45
<211> 60
<212> RNA
<213> Artificial Sequence

<220>
<221> source
<223> /note="Description of Artificial Sequence: Synthetic oligonucleotide"

<220>
<221> modified_base
<222> (1)..(17)
<223> a, c, u, g, unknown or other

<220>
<221> modified_base
<222> (20)..(38)
<223> a, c, u, g, unknown or other

<220>
<221> modified_base
<222> (41)..(60)
<223> a, c, u, g, unknown or other

<400> 45
nnnnnnnnnn nnnnnnnccn nnnnnnnnnn nnnnnnnngg nnnnnnnnnn nnnnnnnnnn 60

<210> 46
<211> 60
<212> RNA
<213> Artificial Sequence

<220>
<221> source
<223> /note="Description of Artificial Sequence: Synthetic oligonucleotide"

<220>
<221> modified_base
<222> (1)..(20)
<223> a, c, u, g, unknown or other

<220>
<221> modified_base
<222> (23)..(40)
<223> a, c, u, g, unknown or other

<220>
<221> modified_base
<222> (43)..(60)
<223> a, c, u, g, unknown or other

<400> 46
nnnnnnnnnn nnnnnnnnnn ccnnnnnnnn nnnnnnnnnn ggnnnnnnnn nnnnnnnnnn 60

<210> 47
<211> 60
<212> RNA

<213> Artificial Sequence

<220>
<221> source
<223> /note="Description of Artificial Sequence: Synthetic oligonucleotide"

<220>
<221> modified_base
<222> (1)..(18)
<223> a, c, u, g, unknown or other

<220>
<221> modified_base
<222> (21)..(38)
<223> a, c, u, g, unknown or other

<220>
<221> modified_base
<222> (41)..(60)
<223> a, c, u, g, unknown or other

<400> 47
nnnnnnnnnn nnnnnnnncc nnnnnnnnnn nnnnnnnngg nnnnnnnnnn nnnnnnnnnn 60

<210> 48
<211> 60
<212> RNA
<213> Artificial Sequence

<220>
<221> source
<223> /note="Description of Artificial Sequence: Synthetic oligonucleotide"

<220>
<221> modified_base
<222> (1)..(20)
<223> a, c, u, g, unknown or other

<220>
<221> modified_base
<222> (23)..(39)
<223> a, c, u, g, unknown or other

<220>
<221> modified_base
<222> (42)..(60)
<223> a, c, u, g, unknown or other

<400> 48
nnnnnnnnnn nnnnnnnnnn ccnnnnnnnn nnnnnnnnng gnnnnnnnnn nnnnnnnnnn 60

<210> 49
<211> 60
<212> RNA
<213> Artificial Sequence

<220>

<221> source
<223> /note="Description of Artificial Sequence: Synthetic oligonucleotide"

<220>
<221> modified_base
<222> (1)..(19)
<223> a, c, u, g, unknown or other

<220>
<221> modified_base
<222> (22)..(38)
<223> a, c, u, g, unknown or other

<220>
<221> modified_base
<222> (41)..(60)
<223> a, c, u, g, unknown or other

<400> 49
nnnnnnnnnn nnnnnnnnnc cnnnnnnnnn nnnnnnnngg nnnnnnnnnn nnnnnnnnnn 60

<210> 50
<211> 60
<212> RNA
<213> Artificial Sequence

<220>
<221> source
<223> /note="Description of Artificial Sequence: Synthetic oligonucleotide"

<220>
<221> modified_base
<222> (1)..(20)
<223> a, c, u, g, unknown or other

<220>
<221> modified_base
<222> (23)..(38)
<223> a, c, u, g, unknown or other

<220>
<221> modified_base
<222> (41)..(60)
<223> a, c, u, g, unknown or other

<400> 50
nnnnnnnnnn nnnnnnnnnn ccnnnnnnnn nnnnnnnngg nnnnnnnnnn nnnnnnnnnn 60

<210> 51
<211> 60
<212> RNA
<213> Artificial Sequence

<220>
<221> source
<223> /note="Description of Artificial Sequence: Synthetic oligonucleotide"

<220>
<221> modified_base
<222> (1)..(20)
<223> a, c, u, g, unknown or other

<220>
<221> modified_base
<222> (23)..(38)
<223> a, c, u, g, unknown or other

<220>
<221> modified_base
<222> (41)..(60)
<223> a, c, u, g, unknown or other

<400> 51
nnnnnnnnnn nnnnnnnnnn ccnnnnnnnn nnnnnnnngg nnnnnnnnnn nnnnnnnnnn 60

<210> 52
<211> 60
<212> RNA
<213> Artificial Sequence

<220>
<221> source
<223> /note="Description of Artificial Sequence: Synthetic oligonucleotide"

<220>
<221> modified_base
<222> (1)..(20)
<223> a, c, u, g, unknown or other

<220>
<221> modified_base
<222> (23)..(37)
<223> a, c, u, g, unknown or other

<220>
<221> modified_base
<222> (40)..(60)
<223> a, c, u, g, unknown or other

<400> 52
nnnnnnnnnn nnnnnnnnnn ccnnnnnnnn nnnnnnnggn nnnnnnnnnn nnnnnnnnnn 60

<210> 53
<211> 60
<212> RNA
<213> Artificial Sequence

<220>
<221> source
<223> /note="Description of Artificial Sequence: Synthetic oligonucleotide"

<220>

<221> modified_base
<222> (1)..(21)
<223> a, c, u, g, unknown or other

<220>
<221> modified_base
<222> (24)..(38)
<223> a, c, u, g, unknown or other

<220>
<221> modified_base
<222> (41)..(60)
<223> a, c, u, g, unknown or other

<400> 53
nnnnnnnnnn nnnnnnnnnn nccnnnnnnn nnnnnnnngg nnnnnnnnnn nnnnnnnnnn 60

<210> 54
<211> 60
<212> RNA
<213> Artificial Sequence

<220>
<221> source
<223> /note="Description of Artificial Sequence: Synthetic
oligonucleotide"

<220>
<221> modified_base
<222> (1)..(20)
<223> a, c, u, g, unknown or other

<220>
<221> modified_base
<222> (23)..(36)
<223> a, c, u, g, unknown or other

<220>
<221> modified_base
<222> (39)..(60)
<223> a, c, u, g, unknown or other

<400> 54
nnnnnnnnnn nnnnnnnnnn ccnnnnnnnn nnnnnnggnn nnnnnnnnnn nnnnnnnnnn 60

<210> 55
<211> 60
<212> RNA
<213> Artificial Sequence

<220>
<221> source
<223> /note="Description of Artificial Sequence: Synthetic
oligonucleotide"

<220>
<221> modified_base
<222> (1)..(22)
<223> a, c, u, g, unknown or other

<220>
<221> modified_base
<222> (25)..(38)
<223> a, c, u, g, unknown or other

<220>
<221> modified_base
<222> (41)..(60)
<223> a, c, u, g, unknown or other

<400> 55
nnnnnnnnnn nnnnnnnnnn nnccnnnnnn nnnnnnnngg nnnnnnnnnn nnnnnnnnnn 60

<210> 56
<211> 60
<212> RNA
<213> Artificial Sequence

<220>
<221> source
<223> /note="Description of Artificial Sequence: Synthetic oligonucleotide"

<220>
<221> modified_base
<222> (1)..(20)
<223> a, c, u, g, unknown or other

<220>
<221> modified_base
<222> (23)..(35)
<223> a, c, u, g, unknown or other

<220>
<221> modified_base
<222> (38)..(60)
<223> a, c, u, g, unknown or other

<400> 56
nnnnnnnnnn nnnnnnnnnn ccnnnnnnnn nnnnnggnnn nnnnnnnnnn nnnnnnnnnn 60

<210> 57
<211> 60
<212> RNA
<213> Artificial Sequence

<220>
<221> source
<223> /note="Description of Artificial Sequence: Synthetic oligonucleotide"

<220>
<221> modified_base
<222> (1)..(23)
<223> a, c, u, g, unknown or other

<220>
<221> modified_base

<222> (26)..(38)
<223> a, c, u, g, unknown or other

<220>
<221> modified_base
<222> (41)..(60)
<223> a, c, u, g, unknown or other

<400> 57
nnnnnnnnnn nnnnnnnnnn nnnccnnnnn nnnnnnnngg nnnnnnnnnn nnnnnnnnnn 60

<210> 58
<211> 60
<212> RNA
<213> Artificial Sequence

<220>
<221> source
<223> /note="Description of Artificial Sequence: Synthetic oligonucleotide"

<220>
<221> modified_base
<222> (1)..(20)
<223> a, c, u, g, unknown or other

<220>
<221> modified_base
<222> (23)..(34)
<223> a, c, u, g, unknown or other

<220>
<221> modified_base
<222> (37)..(60)
<223> a, c, u, g, unknown or other

<400> 58
nnnnnnnnnn nnnnnnnnnn ccnnnnnnnn nnnnggnnnn nnnnnnnnnn nnnnnnnnnn 60

<210> 59
<211> 60
<212> RNA
<213> Artificial Sequence

<220>
<221> source
<223> /note="Description of Artificial Sequence: Synthetic oligonucleotide"

<220>
<221> modified_base
<222> (1)..(24)
<223> a, c, u, g, unknown or other

<220>
<221> modified_base
<222> (27)..(38)
<223> a, c, u, g, unknown or other

<220>
<221> modified_base
<222> (41)..(60)
<223> a, c, u, g, unknown or other

<400> 59
nnnnnnnnnn nnnnnnnnnn nnnnccnnnn nnnnnnnngg nnnnnnnnnn nnnnnnnnnn 60

<210> 60
<211> 60
<212> RNA
<213> Artificial Sequence

<220>
<221> source
<223> /note="Description of Artificial Sequence: Synthetic oligonucleotide"

<220>
<221> modified_base
<222> (1)..(20)
<223> a, c, u, g, unknown or other

<220>
<221> modified_base
<222> (23)..(33)
<223> a, c, u, g, unknown or other

<220>
<221> modified_base
<222> (36)..(60)
<223> a, c, u, g, unknown or other

<400> 60
nnnnnnnnnn nnnnnnnnnn ccnnnnnnnn nnnggnnnnn nnnnnnnnnn nnnnnnnnnn 60

<210> 61
<211> 60
<212> RNA
<213> Artificial Sequence

<220>
<221> source
<223> /note="Description of Artificial Sequence: Synthetic oligonucleotide"

<220>
<221> modified_base
<222> (1)..(25)
<223> a, c, u, g, unknown or other

<220>
<221> modified_base
<222> (28)..(38)
<223> a, c, u, g, unknown or other

<220>
<221> modified_base
<222> (41)..(60)

<223> a, c, u, g, unknown or other

<400> 61
nnnnnnnnnn nnnnnnnnnn nnnnnccnnn nnnnnnnngg nnnnnnnnnn nnnnnnnnnn 60

<210> 62
<211> 60
<212> RNA
<213> Artificial Sequence

<220>
<221> source
<223> /note="Description of Artificial Sequence: Synthetic
oligonucleotide"

<220>
<221> modified_base
<222> (1)..(20)
<223> a, c, u, g, unknown or other

<220>
<221> modified_base
<222> (23)..(32)
<223> a, c, u, g, unknown or other

<220>
<221> modified_base
<222> (35)..(60)
<223> a, c, u, g, unknown or other

<400> 62
nnnnnnnnnn nnnnnnnnnn ccnnnnnnnn nnggnnnnnn nnnnnnnnnn nnnnnnnnnn 60

<210> 63
<211> 60
<212> RNA
<213> Artificial Sequence

<220>
<221> source
<223> /note="Description of Artificial Sequence: Synthetic
oligonucleotide"

<220>
<221> modified_base
<222> (1)..(26)
<223> a, c, u, g, unknown or other

<220>
<221> modified_base
<222> (29)..(38)
<223> a, c, u, g, unknown or other

<220>
<221> modified_base
<222> (41)..(60)
<223> a, c, u, g, unknown or other

<400> 63

nnnnnnnnnn nnnnnnnnnn nnnnnnccnn nnnnnnnngg nnnnnnnnnn nnnnnnnnnn 60

<210> 64
<211> 60
<212> RNA
<213> Artificial Sequence

<220>
<221> source
<223> /note="Description of Artificial Sequence: Synthetic oligonucleotide"

<220>
<221> modified_base
<222> (1)..(20)
<223> a, c, u, g, unknown or other

<220>
<221> modified_base
<222> (23)..(31)
<223> a, c, u, g, unknown or other

<220>
<221> modified_base
<222> (34)..(60)
<223> a, c, u, g, unknown or other

<400> 64
nnnnnnnnnn nnnnnnnnnn ccnnnnnnnn nggnnnnnnn nnnnnnnnnn nnnnnnnnnn 60

<210> 65
<211> 60
<212> RNA
<213> Artificial Sequence

<220>
<221> source
<223> /note="Description of Artificial Sequence: Synthetic oligonucleotide"

<220>
<221> modified_base
<222> (1)..(27)
<223> a, c, u, g, unknown or other

<220>
<221> modified_base
<222> (30)..(38)
<223> a, c, u, g, unknown or other

<220>
<221> modified_base
<222> (41)..(60)
<223> a, c, u, g, unknown or other

<400> 65
nnnnnnnnnn nnnnnnnnnn nnnnnnnccn nnnnnnnngg nnnnnnnnnn nnnnnnnnnn 60

<210> 66
<211> 60
<212> RNA
<213> Artificial Sequence

<220>
<221> source
<223> /note="Description of Artificial Sequence: Synthetic oligonucleotide"

<220>
<221> modified_base
<222> (1)..(20)
<223> a, c, u, g, unknown or other

<220>
<221> modified_base
<222> (23)..(30)
<223> a, c, u, g, unknown or other

<220>
<221> modified_base
<222> (33)..(60)
<223> a, c, u, g, unknown or other

<400> 66
nnnnnnnnnn nnnnnnnnnn ccnnnnnnnn ggnnnnnnnn nnnnnnnnnn nnnnnnnnnn 60

<210> 67
<211> 60
<212> RNA
<213> Artificial Sequence

<220>
<221> source
<223> /note="Description of Artificial Sequence: Synthetic oligonucleotide"

<220>
<221> modified_base
<222> (1)..(28)
<223> a, c, u, g, unknown or other

<220>
<221> modified_base
<222> (31)..(38)
<223> a, c, u, g, unknown or other

<220>
<221> modified_base
<222> (41)..(60)
<223> a, c, u, g, unknown or other

<400> 67
nnnnnnnnnn nnnnnnnnnn nnnnnnnncc nnnnnnnngg nnnnnnnnnn nnnnnnnnnn 60

<210> 68
<211> 60
<212> RNA

<213> Artificial Sequence

<220>
<221> source
<223> /note="Description of Artificial Sequence: Synthetic
oligonucleotide"

<220>
<221> modified_base
<222> (1)..(20)
<223> a, c, u, g, unknown or other

<220>
<221> modified_base
<222> (23)..(29)
<223> a, c, u, g, unknown or other

<220>
<221> modified_base
<222> (32)..(60)
<223> a, c, u, g, unknown or other

<400> 68
nnnnnnnnnn nnnnnnnnnn ccnnnnnnng gnnnnnnnnn nnnnnnnnnn nnnnnnnnnn 60

<210> 69
<211> 60
<212> RNA
<213> Artificial Sequence

<220>
<221> source
<223> /note="Description of Artificial Sequence: Synthetic
oligonucleotide"

<220>
<221> modified_base
<222> (1)..(29)
<223> a, c, u, g, unknown or other

<220>
<221> modified_base
<222> (32)..(38)
<223> a, c, u, g, unknown or other

<220>
<221> modified_base
<222> (41)..(60)
<223> a, c, u, g, unknown or other

<400> 69
nnnnnnnnnn nnnnnnnnnn nnnnnnnnnc cnnnnnnngg nnnnnnnnnn nnnnnnnnnn 60

<210> 70
<211> 60
<212> RNA
<213> Artificial Sequence

<220>

<221> source
<223> /note="Description of Artificial Sequence: Synthetic oligonucleotide"

<220>
<221> modified_base
<222> (1)..(20)
<223> a, c, u, g, unknown or other

<220>
<221> modified_base
<222> (23)..(28)
<223> a, c, u, g, unknown or other

<220>
<221> modified_base
<222> (31)..(60)
<223> a, c, u, g, unknown or other

<400> 70
nnnnnnnnnn nnnnnnnnnn ccnnnnnngg nnnnnnnnnn nnnnnnnnnn nnnnnnnnnn 60

<210> 71
<211> 60
<212> RNA
<213> Artificial Sequence

<220>
<221> source
<223> /note="Description of Artificial Sequence: Synthetic oligonucleotide"

<220>
<221> modified_base
<222> (1)..(30)
<223> a, c, u, g, unknown or other

<220>
<221> modified_base
<222> (33)..(38)
<223> a, c, u, g, unknown or other

<220>
<221> modified_base
<222> (41)..(60)
<223> a, c, u, g, unknown or other

<400> 71
nnnnnnnnnn nnnnnnnnnn nnnnnnnnnn ccnnnnnngg nnnnnnnnnn nnnnnnnnnn 60

<210> 72
<211> 60
<212> RNA
<213> Artificial Sequence

<220>
<221> source
<223> /note="Description of Artificial Sequence: Synthetic oligonucleotide"

<220>
<221> modified_base
<222> (1)..(20)
<223> a, c, u, g, unknown or other

<220>
<221> modified_base
<222> (23)..(27)
<223> a, c, u, g, unknown or other

<220>
<221> modified_base
<222> (30)..(60)
<223> a, c, u, g, unknown or other

<400> 72
nnnnnnnnnn nnnnnnnnnn ccnnnnnggn nnnnnnnnnn nnnnnnnnnn nnnnnnnnnn 60

<210> 73
<211> 60
<212> RNA
<213> Artificial Sequence

<220>
<221> source
<223> /note="Description of Artificial Sequence: Synthetic oligonucleotide"

<220>
<221> modified_base
<222> (1)..(31)
<223> a, c, u, g, unknown or other

<220>
<221> modified_base
<222> (34)..(38)
<223> a, c, u, g, unknown or other

<220>
<221> modified_base
<222> (41)..(60)
<223> a, c, u, g, unknown or other

<400> 73
nnnnnnnnnn nnnnnnnnnn nnnnnnnnnn nccnnnnngg nnnnnnnnnn nnnnnnnnnn 60

<210> 74
<211> 60
<212> RNA
<213> Artificial Sequence

<220>
<221> source
<223> /note="Description of Artificial Sequence: Synthetic oligonucleotide"

<220>

<221> modified_base
<222> (1)..(20)
<223> a, c, u, g, unknown or other

<220>
<221> modified_base
<222> (23)..(26)
<223> a, c, u, g, unknown or other

<220>
<221> modified_base
<222> (29)..(60)
<223> a, c, u, g, unknown or other

<400> 74
nnnnnnnnnn nnnnnnnnnn ccnnnnggnn nnnnnnnnnn nnnnnnnnnn nnnnnnnnnn 60

<210> 75
<211> 60
<212> RNA
<213> Artificial Sequence

<220>
<221> source
<223> /note="Description of Artificial Sequence: Synthetic oligonucleotide"

<220>
<221> modified_base
<222> (1)..(32)
<223> a, c, u, g, unknown or other

<220>
<221> modified_base
<222> (35)..(38)
<223> a, c, u, g, unknown or other

<220>
<221> modified_base
<222> (41)..(60)
<223> a, c, u, g, unknown or other

<400> 75
nnnnnnnnnn nnnnnnnnnn nnnnnnnnnn nnccnnnngg nnnnnnnnnn nnnnnnnnnn 60

<210> 76
<211> 60
<212> RNA
<213> Artificial Sequence

<220>
<221> source
<223> /note="Description of Artificial Sequence: Synthetic oligonucleotide"

<220>
<221> modified_base
<222> (1)..(20)
<223> a, c, u, g, unknown or other

<220>
<221> modified_base
<222> (23)..(25)
<223> a, c, u, g, unknown or other

<220>
<221> modified_base
<222> (28)..(60)
<223> a, c, u, g, unknown or other

<400> 76
nnnnnnnnnn nnnnnnnnnn ccnnnggnnn nnnnnnnnnn nnnnnnnnnn nnnnnnnnnn 60

<210> 77
<211> 60
<212> RNA
<213> Artificial Sequence

<220>
<221> source
<223> /note="Description of Artificial Sequence: Synthetic oligonucleotide"

<220>
<221> modified_base
<222> (1)..(33)
<223> a, c, u, g, unknown or other

<220>
<221> modified_base
<222> (36)..(38)
<223> a, c, u, g, unknown or other

<220>
<221> modified_base
<222> (41)..(60)
<223> a, c, u, g, unknown or other

<400> 77
nnnnnnnnnn nnnnnnnnnn nnnnnnnnnn nnnccnnngg nnnnnnnnnn nnnnnnnnnn 60

<210> 78
<211> 60
<212> RNA
<213> Artificial Sequence

<220>
<221> source
<223> /note="Description of Artificial Sequence: Synthetic oligonucleotide"

<220>
<221> modified_base
<222> (1)..(20)
<223> a, c, u, g, unknown or other

<220>
<221> modified_base

<222> (23)..(24)
<223> a, c, u, g, unknown or other

<220>
<221> modified_base
<222> (27)..(60)
<223> a, c, u, g, unknown or other

<400> 78
nnnnnnnnnn nnnnnnnnnn ccnnggnnnn nnnnnnnnnn nnnnnnnnnn nnnnnnnnnn 60

<210> 79
<211> 60
<212> RNA
<213> Artificial Sequence

<220>
<221> source
<223> /note="Description of Artificial Sequence: Synthetic oligonucleotide"

<220>
<221> modified_base
<222> (1)..(34)
<223> a, c, u, g, unknown or other

<220>
<221> modified_base
<222> (37)..(38)
<223> a, c, u, g, unknown or other

<220>
<221> modified_base
<222> (41)..(60)
<223> a, c, u, g, unknown or other

<400> 79
nnnnnnnnnn nnnnnnnnnn nnnnnnnnnn nnnnccnngg nnnnnnnnnn nnnnnnnnnn 60

<210> 80
<211> 60
<212> RNA
<213> Artificial Sequence

<220>
<221> source
<223> /note="Description of Artificial Sequence: Synthetic oligonucleotide"

<220>
<221> modified_base
<222> (1)..(20)
<223> a, c, u, g, unknown or other

<220>
<221> modified_base
<222> (23)..(23)
<223> a, c, u, g, unknown or other

<220>
<221> modified_base
<222> (26)..(60)
<223> a, c, u, g, unknown or other

<400> 80
nnnnnnnnnn nnnnnnnnnn ccnggnnnnn nnnnnnnnnn nnnnnnnnnn nnnnnnnnnn 60

<210> 81
<211> 60
<212> RNA
<213> Artificial Sequence

<220>
<221> source
<223> /note="Description of Artificial Sequence: Synthetic oligonucleotide"

<220>
<221> modified_base
<222> (1)..(35)
<223> a, c, u, g, unknown or other

<220>
<221> modified_base
<222> (38)..(38)
<223> a, c, u, g, unknown or other

<220>
<221> modified_base
<222> (41)..(60)
<223> a, c, u, g, unknown or other

<400> 81
nnnnnnnnnn nnnnnnnnnn nnnnnnnnnn nnnnnccngg nnnnnnnnnn nnnnnnnnnn 60

<210> 82
<211> 60
<212> RNA
<213> Artificial Sequence

<220>
<221> source
<223> /note="Description of Artificial Sequence: Synthetic oligonucleotide"

<220>
<221> modified_base
<222> (1)..(21)
<223> a, c, u, g, unknown or other

<220>
<221> modified_base
<222> (26)..(60)
<223> a, c, u, g, unknown or other

<400> 82
nnnnnnnnnn nnnnnnnnnn nccggnnnnn nnnnnnnnnn nnnnnnnnnn nnnnnnnnnn 60

<210> 83
<211> 60
<212> RNA
<213> Artificial Sequence

<220>
<221> source
<223> /note="Description of Artificial Sequence: Synthetic oligonucleotide"

<220>
<221> modified_base
<222> (1)..(35)
<223> a, c, u, g, unknown or other

<220>
<221> modified_base
<222> (40)..(60)
<223> a, c, u, g, unknown or other

<400> 83
nnnnnnnnnn nnnnnnnnnn nnnnnnnnnn nnnnnccggn nnnnnnnnnn nnnnnnnnnn 60

<210> 84
<211> 60
<212> RNA
<213> Artificial Sequence

<220>
<221> source
<223> /note="Description of Artificial Sequence: Synthetic oligonucleotide"

<220>
<221> modified_base
<222> (1)..(23)
<223> a, c, u, g, unknown or other

<220>
<221> modified_base
<222> (26)..(60)
<223> a, c, u, g, unknown or other

<400> 84
nnnnnnnnnn nnnnnnnnnn nnnggnnnnn nnnnnnnnnn nnnnnnnnnn nnnnnnnnnn 60

<210> 85
<211> 60
<212> RNA
<213> Artificial Sequence

<220>
<221> source
<223> /note="Description of Artificial Sequence: Synthetic oligonucleotide"

<220>

<221> modified_base
<222> (1)..(33)
<223> a, c, u, g, unknown or other

<220>
<221> modified_base
<222> (38)..(60)
<223> a, c, u, g, unknown or other

<400> 85
nnnnnnnnnn nnnnnnnnnn nnnnnnnnnn nnnggccnnn nnnnnnnnnn nnnnnnnnnn 60

<210> 86
<211> 60
<212> RNA
<213> Artificial Sequence

<220>
<221> source
<223> /note="Description of Artificial Sequence: Synthetic
oligonucleotide"

<220>
<221> modified_base
<222> (1)..(22)
<223> a, c, u, g, unknown or other

<220>
<221> modified_base
<222> (26)..(60)
<223> a, c, u, g, unknown or other

<400> 86
nnnnnnnnnn nnnnnnnnnn nncggnnnnn nnnnnnnnnn nnnnnnnnnn nnnnnnnnnn 60

<210> 87
<211> 60
<212> RNA
<213> Artificial Sequence

<220>
<221> source
<223> /note="Description of Artificial Sequence: Synthetic
oligonucleotide"

<220>
<221> modified_base
<222> (1)..(32)
<223> a, c, u, g, unknown or other

<220>
<221> modified_base
<222> (35)..(35)
<223> a, c, u, g, unknown or other

<220>
<221> modified_base
<222> (38)..(60)
<223> a, c, u, g, unknown or other

<400> 87
nnnnnnnnnn nnnnnnnnnn nnnnnnnnnn nnggnccnnn nnnnnnnnnn nnnnnnnnnn 60

<210> 88
<211> 60
<212> RNA
<213> Artificial Sequence

<220>
<221> source
<223> /note="Description of Artificial Sequence: Synthetic oligonucleotide"

<220>
<221> modified_base
<222> (1)..(31)
<223> a, c, u, g, unknown or other

<220>
<221> modified_base
<222> (34)..(35)
<223> a, c, u, g, unknown or other

<220>
<221> modified_base
<222> (38)..(60)
<223> a, c, u, g, unknown or other

<400> 88
nnnnnnnnnn nnnnnnnnnn nnnnnnnnnn nggnnccnnn nnnnnnnnnn nnnnnnnnnn 60

<210> 89
<211> 60
<212> RNA
<213> Artificial Sequence

<220>
<221> source
<223> /note="Description of Artificial Sequence: Synthetic oligonucleotide"

<220>
<221> modified_base
<222> (1)..(30)
<223> a, c, u, g, unknown or other

<220>
<221> modified_base
<222> (33)..(35)
<223> a, c, u, g, unknown or other

<220>
<221> modified_base
<222> (38)..(60)
<223> a, c, u, g, unknown or other

<400> 89
nnnnnnnnnn nnnnnnnnnn nnnnnnnnnn ggnnnccnnn nnnnnnnnnn nnnnnnnnnn 60

<210> 90
<211> 60
<212> RNA
<213> Artificial Sequence

<220>
<221> source
<223> /note="Description of Artificial Sequence: Synthetic oligonucleotide"

<220>
<221> modified_base
<222> (1)..(29)
<223> a, c, u, g, unknown or other

<220>
<221> modified_base
<222> (32)..(35)
<223> a, c, u, g, unknown or other

<220>
<221> modified_base
<222> (38)..(60)
<223> a, c, u, g, unknown or other

<400> 90
nnnnnnnnnn nnnnnnnnnn nnnnnnnnng gnnnnccnnn nnnnnnnnnn nnnnnnnnnn 60

<210> 91
<211> 60
<212> RNA
<213> Artificial Sequence

<220>
<221> source
<223> /note="Description of Artificial Sequence: Synthetic oligonucleotide"

<220>
<221> modified_base
<222> (1)..(28)
<223> a, c, u, g, unknown or other

<220>
<221> modified_base
<222> (31)..(35)
<223> a, c, u, g, unknown or other

<220>
<221> modified_base
<222> (38)..(60)
<223> a, c, u, g, unknown or other

<400> 91
nnnnnnnnnn nnnnnnnnnn nnnnnnnngg nnnnnccnnn nnnnnnnnnn nnnnnnnnnn 60

<210> 92

<211> 9
<212> PRT
<213> Unknown

<220>
<223> Description of Unknown:
'LAGLIDADG' family motif peptide

<400> 92
Leu Ala Gly Leu Ile Asp Ala Asp Gly
1 5

<210> 93
<211> 12
<212> RNA
<213> Artificial Sequence

<220>
<221> source
<223> /note="Description of Artificial Sequence: Synthetic
oligonucleotide"

<400> 93
guuuuagagc ua 12

<210> 94
<211> 12
<212> DNA
<213> Artificial Sequence

<220>
<221> source
<223> /note="Description of Artificial Sequence: Synthetic
oligonucleotide"

<400> 94
gttttagagc ta 12

<210> 95
<211> 31
<212> DNA
<213> Artificial Sequence

<220>
<221> source
<223> /note="Description of Artificial Sequence: Synthetic
oligonucleotide"

<400> 95
tagcaagtta aaataaggct agtccgtttt t 31

<210> 96
<211> 32
<212> DNA
<213> Artificial Sequence

<220>
<221> source
<223> /note="Description of Artificial Sequence: Synthetic

primer"

<400> 96
aaactctaga gagggcctat ttcccatgat tc 32

<210> 97
<211> 153
<212> DNA
<213> Artificial Sequence

<220>
<221> source
<223> /note="Description of Artificial Sequence: Synthetic
primer"

<400> 97
acctctagaa aaaaagcacc gactcggtgc cactttttca agttgataac ggactagcct 60

tattttaact tgctatgctg ttttgtttcc aaaacagcat agctctaaaa cccctagtca 120

ttggaggtga cggtgtttcg tcctttccac aag 153

<210> 98
<211> 52
<212> DNA
<213> Artificial Sequence

<220>
<221> source
<223> /note="Description of Artificial Sequence: Synthetic
primer"

<400> 98
taatacgact cactatagga agtgcgccac catggcccca aagaagaagc gg 52

<210> 99
<211> 60
<212> DNA
<213> Artificial Sequence

<220>
<221> source
<223> /note="Description of Artificial Sequence: Synthetic
primer"

<400> 99
ggtttttttt tttttttttt tttttttttt ttttcttact ttttcttttt tgcctggccg 60

<210> 100
<211> 984
<212> PRT
<213> Campylobacter jejuni

<400> 100
Met Ala Arg Ile Leu Ala Phe Asp Ile Gly Ile Ser Ser Ile Gly Trp
1 5 10 15

Ala Phe Ser Glu Asn Asp Glu Leu Lys Asp Cys Gly Val Arg Ile Phe

20 25 30

Thr Lys Val Glu Asn Pro Lys Thr Gly Glu Ser Leu Ala Leu Pro Arg
35 40 45

Arg Leu Ala Arg Ser Ala Arg Lys Arg Leu Ala Arg Arg Lys Ala Arg
50 55 60

Leu Asn His Leu Lys His Leu Ile Ala Asn Glu Phe Lys Leu Asn Tyr
65 70 75 80

Glu Asp Tyr Gln Ser Phe Asp Glu Ser Leu Ala Lys Ala Tyr Lys Gly
85 90 95

Ser Leu Ile Ser Pro Tyr Glu Leu Arg Phe Arg Ala Leu Asn Glu Leu
100 105 110

Leu Ser Lys Gln Asp Phe Ala Arg Val Ile Leu His Ile Ala Lys Arg
115 120 125

Arg Gly Tyr Asp Asp Ile Lys Asn Ser Asp Asp Lys Glu Lys Gly Ala
130 135 140

Ile Leu Lys Ala Ile Lys Gln Asn Glu Glu Lys Leu Ala Asn Tyr Gln
145 150 155 160

Ser Val Gly Glu Tyr Leu Tyr Lys Glu Tyr Phe Gln Lys Phe Lys Glu
165 170 175

Asn Ser Lys Glu Phe Thr Asn Val Arg Asn Lys Lys Glu Ser Tyr Glu
180 185 190

Arg Cys Ile Ala Gln Ser Phe Leu Lys Asp Glu Leu Lys Leu Ile Phe
195 200 205

Lys Lys Gln Arg Glu Phe Gly Phe Ser Phe Ser Lys Lys Phe Glu Glu
210 215 220

Glu Val Leu Ser Val Ala Phe Tyr Lys Arg Ala Leu Lys Asp Phe Ser
225 230 235 240

His Leu Val Gly Asn Cys Ser Phe Phe Thr Asp Glu Lys Arg Ala Pro
245 250 255

Lys Asn Ser Pro Leu Ala Phe Met Phe Val Ala Leu Thr Arg Ile Ile
260 265 270

```
Asn Leu Leu Asn Asn Leu Lys Asn Thr Glu Gly Ile Leu Tyr Thr Lys
        275                 280                 285

Asp Asp Leu Asn Ala Leu Leu Asn Glu Val Leu Lys Asn Gly Thr Leu
    290                 295                 300

Thr Tyr Lys Gln Thr Lys Lys Leu Leu Gly Leu Ser Asp Asp Tyr Glu
305                 310                 315                 320

Phe Lys Gly Glu Lys Gly Thr Tyr Phe Ile Glu Phe Lys Lys Tyr Lys
                325                 330                 335

Glu Phe Ile Lys Ala Leu Gly Glu His Asn Leu Ser Gln Asp Asp Leu
            340                 345                 350

Asn Glu Ile Ala Lys Asp Ile Thr Leu Ile Lys Asp Glu Ile Lys Leu
        355                 360                 365

Lys Lys Ala Leu Ala Lys Tyr Asp Leu Asn Gln Asn Gln Ile Asp Ser
    370                 375                 380

Leu Ser Lys Leu Glu Phe Lys Asp His Leu Asn Ile Ser Phe Lys Ala
385                 390                 395                 400

Leu Lys Leu Val Thr Pro Leu Met Leu Glu Gly Lys Lys Tyr Asp Glu
                405                 410                 415

Ala Cys Asn Glu Leu Asn Leu Lys Val Ala Ile Asn Glu Asp Lys Lys
            420                 425                 430

Asp Phe Leu Pro Ala Phe Asn Glu Thr Tyr Tyr Lys Asp Glu Val Thr
        435                 440                 445

Asn Pro Val Val Leu Arg Ala Ile Lys Glu Tyr Arg Lys Val Leu Asn
    450                 455                 460

Ala Leu Leu Lys Lys Tyr Gly Lys Val His Lys Ile Asn Ile Glu Leu
465                 470                 475                 480

Ala Arg Glu Val Gly Lys Asn His Ser Gln Arg Ala Lys Ile Glu Lys
                485                 490                 495

Glu Gln Asn Glu Asn Tyr Lys Ala Lys Lys Asp Ala Glu Leu Glu Cys
            500                 505                 510

Glu Lys Leu Gly Leu Lys Ile Asn Ser Lys Asn Ile Leu Lys Leu Arg
        515                 520                 525
```

```
Leu Phe Lys Glu Gln Lys Glu Phe Cys Ala Tyr Ser Gly Glu Lys Ile
    530                 535                 540

Lys Ile Ser Asp Leu Gln Asp Glu Lys Met Leu Glu Ile Asp His Ile
545                 550                 555                 560

Tyr Pro Tyr Ser Arg Ser Phe Asp Asp Ser Tyr Met Asn Lys Val Leu
                565                 570                 575

Val Phe Thr Lys Gln Asn Gln Glu Lys Leu Asn Gln Thr Pro Phe Glu
            580                 585                 590

Ala Phe Gly Asn Asp Ser Ala Lys Trp Gln Lys Ile Glu Val Leu Ala
        595                 600                 605

Lys Asn Leu Pro Thr Lys Lys Gln Lys Arg Ile Leu Asp Lys Asn Tyr
    610                 615                 620

Lys Asp Lys Glu Gln Lys Asn Phe Lys Asp Arg Asn Leu Asn Asp Thr
625                 630                 635                 640

Arg Tyr Ile Ala Arg Leu Val Leu Asn Tyr Thr Lys Asp Tyr Leu Asp
                645                 650                 655

Phe Leu Pro Leu Ser Asp Asp Glu Asn Thr Lys Leu Asn Asp Thr Gln
            660                 665                 670

Lys Gly Ser Lys Val His Val Glu Ala Lys Ser Gly Met Leu Thr Ser
        675                 680                 685

Ala Leu Arg His Thr Trp Gly Phe Ser Ala Lys Asp Arg Asn Asn His
    690                 695                 700

Leu His His Ala Ile Asp Ala Val Ile Ile Ala Tyr Ala Asn Asn Ser
705                 710                 715                 720

Ile Val Lys Ala Phe Ser Asp Phe Lys Lys Glu Gln Glu Ser Asn Ser
                725                 730                 735

Ala Glu Leu Tyr Ala Lys Lys Ile Ser Glu Leu Asp Tyr Lys Asn Lys
            740                 745                 750

Arg Lys Phe Phe Glu Pro Phe Ser Gly Phe Arg Gln Lys Val Leu Asp
        755                 760                 765

Lys Ile Asp Glu Ile Phe Val Ser Lys Pro Glu Arg Lys Lys Pro Ser
    770                 775                 780
```

```
Gly Ala Leu His Glu Glu Thr Phe Arg Lys Glu Glu Glu Phe Tyr Gln
785                 790                 795                 800

Ser Tyr Gly Gly Lys Glu Gly Val Leu Lys Ala Leu Glu Leu Gly Lys
                805                 810                 815

Ile Arg Lys Val Asn Gly Lys Ile Val Lys Asn Gly Asp Met Phe Arg
            820                 825                 830

Val Asp Ile Phe Lys His Lys Lys Thr Asn Lys Phe Tyr Ala Val Pro
        835                 840                 845

Ile Tyr Thr Met Asp Phe Ala Leu Lys Val Leu Pro Asn Lys Ala Val
    850                 855                 860

Ala Arg Ser Lys Lys Gly Glu Ile Lys Asp Trp Ile Leu Met Asp Glu
865                 870                 875                 880

Asn Tyr Glu Phe Cys Phe Ser Leu Tyr Lys Asp Ser Leu Ile Leu Ile
                885                 890                 895

Gln Thr Lys Asp Met Gln Glu Pro Glu Phe Val Tyr Tyr Asn Ala Phe
            900                 905                 910

Thr Ser Ser Thr Val Ser Leu Ile Val Ser Lys His Asp Asn Lys Phe
        915                 920                 925

Glu Thr Leu Ser Lys Asn Gln Lys Ile Leu Phe Lys Asn Ala Asn Glu
    930                 935                 940

Lys Glu Val Ile Ala Lys Ser Ile Gly Ile Gln Asn Leu Lys Val Phe
945                 950                 955                 960

Glu Lys Tyr Ile Val Ser Ala Leu Gly Glu Val Thr Lys Ala Glu Phe
                965                 970                 975

Arg Gln Arg Glu Asp Phe Lys Lys
            980
```

<210> 101
<211> 91
<212> DNA
<213> Artificial Sequence

<220>
<221> source
<223> /note="Description of Artificial Sequence: Synthetic oligonucleotide"

<400> 101
tataatctca taagaaattt aaaaagggac taaaataaag agtttgcggg actctgcggg 60

gttacaatcc cctaaaaccg cttttaaaat t 91

<210> 102
<211> 36
<212> DNA
<213> Artificial Sequence

<220>
<221> source
<223> /note="Description of Artificial Sequence: Synthetic
oligonucleotide"

<400> 102
attttaccat aaagaaattt aaaaagggac taaaac 36

<210> 103
<211> 95
<212> RNA
<213> Artificial Sequence

<220>
<221> source
<223> /note="Description of Artificial Sequence: Synthetic
oligonucleotide"

<220>
<221> modified_base
<222> (1)..(20)
<223> a, c, u, g, unknown or other

<400> 103
nnnnnnnnnn nnnnnnnnnn guuuuagucc cgaaagggac uaaaauaaag aguuugcggg 60

acucugcggg guuacaaucc ccuaaaaccg cuuuu 95

<210> 104
<211> 1115
<212> PRT
<213> Artificial Sequence

<220>
<221> source
<223> /note="Description of Artificial Sequence: Synthetic
polypeptide"

<400> 104
Met Ser Asp Leu Val Leu Gly Leu Asp Ile Gly Ile Gly Ser Val Gly
1 5 10 15

Val Gly Ile Leu Asn Lys Val Thr Gly Glu Ile Ile His Lys Asn Ser
20 25 30

Arg Ile Phe Pro Ala Ala Gln Ala Glu Asn Asn Leu Val Arg Arg Thr

```
        35                  40                  45

Asn Arg Gln Gly Arg Arg Leu Ala Arg Arg Lys Lys His Arg Arg Val
    50                  55                  60

Arg Leu Asn Arg Leu Phe Glu Glu Ser Gly Leu Ile Thr Asp Phe Thr
65                  70                  75                  80

Lys Ile Ser Ile Asn Leu Asn Pro Tyr Gln Leu Arg Val Lys Gly Leu
                85                  90                  95

Thr Asp Glu Leu Ser Asn Glu Glu Leu Phe Ile Ala Leu Lys Asn Met
            100                 105                 110

Val Lys His Arg Gly Ile Ser Tyr Leu Asp Asp Ala Ser Asp Asp Gly
        115                 120                 125

Asn Ser Ser Val Gly Asp Tyr Ala Gln Ile Val Lys Glu Asn Ser Lys
    130                 135                 140

Gln Leu Glu Thr Lys Thr Pro Gly Gln Ile Gln Leu Glu Arg Tyr Gln
145                 150                 155                 160

Thr Tyr Gly Gln Leu Arg Gly Asp Phe Thr Val Glu Lys Asp Gly Lys
                165                 170                 175

Lys His Arg Leu Ile Asn Val Phe Pro Thr Ser Ala Tyr Arg Ser Glu
            180                 185                 190

Ala Leu Arg Ile Leu Gln Thr Gln Gln Glu Phe Asn Pro Gln Ile Thr
        195                 200                 205

Asp Glu Phe Ile Asn Arg Tyr Leu Glu Ile Leu Thr Gly Lys Arg Lys
    210                 215                 220

Tyr Tyr His Gly Pro Gly Asn Glu Lys Ser Arg Thr Asp Tyr Gly Arg
225                 230                 235                 240

Tyr Arg Thr Ser Gly Glu Thr Leu Asp Asn Ile Phe Gly Ile Leu Ile
                245                 250                 255

Gly Lys Cys Thr Phe Tyr Pro Asp Glu Phe Arg Ala Ala Lys Ala Ser
            260                 265                 270

Tyr Thr Ala Gln Glu Phe Asn Leu Leu Asn Asp Leu Asn Asn Leu Thr
        275                 280                 285
```

Val Pro Thr Glu Thr Lys Lys Leu Ser Lys Glu Gln Lys Asn Gln Ile
    290                 295                 300

Ile Asn Tyr Val Lys Asn Glu Lys Ala Met Gly Pro Ala Lys Leu Phe
305                 310                 315                 320

Lys Tyr Ile Ala Lys Leu Leu Ser Cys Asp Val Ala Asp Ile Lys Gly
                325                 330                 335

Tyr Arg Ile Asp Lys Ser Gly Lys Ala Glu Ile His Thr Phe Glu Ala
            340                 345                 350

Tyr Arg Lys Met Lys Thr Leu Glu Thr Leu Asp Ile Glu Gln Met Asp
        355                 360                 365

Arg Glu Thr Leu Asp Lys Leu Ala Tyr Val Leu Thr Leu Asn Thr Glu
    370                 375                 380

Arg Glu Gly Ile Gln Glu Ala Leu Glu His Glu Phe Ala Asp Gly Ser
385                 390                 395                 400

Phe Ser Gln Lys Gln Val Asp Glu Leu Val Gln Phe Arg Lys Ala Asn
                405                 410                 415

Ser Ser Ile Phe Gly Lys Gly Trp His Asn Phe Ser Val Lys Leu Met
            420                 425                 430

Met Glu Leu Ile Pro Glu Leu Tyr Glu Thr Ser Glu Glu Gln Met Thr
        435                 440                 445

Ile Leu Thr Arg Leu Gly Lys Gln Lys Thr Thr Ser Ser Ser Asn Lys
    450                 455                 460

Thr Lys Tyr Ile Asp Glu Lys Leu Leu Thr Glu Glu Ile Tyr Asn Pro
465                 470                 475                 480

Val Val Ala Lys Ser Val Arg Gln Ala Ile Lys Ile Val Asn Ala Ala
                485                 490                 495

Ile Lys Glu Tyr Gly Asp Phe Asp Asn Ile Val Ile Glu Met Ala Arg
            500                 505                 510

Glu Asn Gln Thr Thr Gln Lys Gly Gln Lys Asn Ser Arg Glu Arg Met
        515                 520                 525

Lys Arg Ile Glu Glu Gly Ile Lys Glu Leu Gly Ser Gln Ile Leu Lys
    530                 535                 540

```
Glu His Pro Val Glu Asn Thr Gln Leu Gln Asn Glu Lys Leu Tyr Leu
545                 550                 555                 560

Tyr Tyr Leu Gln Asn Gly Arg Asp Met Tyr Val Asp Gln Glu Leu Asp
                565                 570                 575

Ile Asn Arg Leu Ser Asp Tyr Asp Val Asp His Ile Val Pro Gln Ser
            580                 585                 590

Phe Leu Lys Asp Asp Ser Ile Asp Asn Lys Val Leu Thr Arg Ser Asp
        595                 600                 605

Lys Asn Arg Gly Lys Ser Asp Asn Val Pro Ser Glu Glu Val Val Lys
    610                 615                 620

Lys Met Lys Asn Tyr Trp Arg Gln Leu Leu Asn Ala Lys Leu Ile Thr
625                 630                 635                 640

Gln Arg Lys Phe Asp Asn Leu Thr Lys Ala Glu Arg Gly Gly Leu Ser
                645                 650                 655

Glu Leu Asp Lys Ala Gly Phe Ile Lys Arg Gln Leu Val Glu Thr Arg
            660                 665                 670

Gln Ile Thr Lys His Val Ala Gln Ile Leu Asp Ser Arg Met Asn Thr
        675                 680                 685

Lys Tyr Asp Glu Asn Asp Lys Leu Ile Arg Glu Val Lys Val Ile Thr
    690                 695                 700

Leu Lys Ser Lys Leu Val Ser Asp Phe Arg Lys Asp Phe Gln Phe Tyr
705                 710                 715                 720

Lys Val Arg Glu Ile Asn Asn Tyr His His Ala His Asp Ala Tyr Leu
                725                 730                 735

Asn Ala Val Val Gly Thr Ala Leu Ile Lys Lys Tyr Pro Lys Leu Glu
            740                 745                 750

Ser Glu Phe Val Tyr Gly Asp Tyr Lys Val Tyr Asp Val Arg Lys Met
        755                 760                 765

Ile Ala Lys Ser Glu Gln Glu Ile Gly Lys Ala Thr Ala Lys Tyr Phe
    770                 775                 780

Phe Tyr Ser Asn Ile Met Asn Phe Phe Lys Thr Glu Ile Thr Leu Ala
785                 790                 795                 800
```

Asn Gly Glu Ile Arg Lys Arg Pro Leu Ile Glu Thr Asn Gly Glu Thr
805 810 815

Gly Glu Ile Val Trp Asp Lys Gly Arg Asp Phe Ala Thr Val Arg Lys
820 825 830

Val Leu Ser Met Pro Gln Val Asn Ile Val Lys Lys Thr Glu Val Gln
835 840 845

Thr Gly Gly Phe Ser Lys Glu Ser Ile Leu Pro Lys Arg Asn Ser Asp
850 855 860

Lys Leu Ile Ala Arg Lys Lys Asp Trp Asp Pro Lys Lys Tyr Gly Gly
865 870 875 880

Phe Asp Ser Pro Thr Val Ala Tyr Ser Val Leu Val Val Ala Lys Val
885 890 895

Glu Lys Gly Lys Ser Lys Lys Leu Lys Ser Val Lys Glu Leu Leu Gly
900 905 910

Ile Thr Ile Met Glu Arg Ser Ser Phe Glu Lys Asn Pro Ile Asp Phe
915 920 925

Leu Glu Ala Lys Gly Tyr Lys Glu Val Lys Lys Asp Leu Ile Ile Lys
930 935 940

Leu Pro Lys Tyr Ser Leu Phe Glu Leu Glu Asn Gly Arg Lys Arg Met
945 950 955 960

Leu Ala Ser Ala Gly Glu Leu Gln Lys Gly Asn Glu Leu Ala Leu Pro
965 970 975

Ser Lys Tyr Val Asn Phe Leu Tyr Leu Ala Ser His Tyr Glu Lys Leu
980 985 990

Lys Gly Ser Pro Glu Asp Asn Glu Gln Lys Gln Leu Phe Val Glu Gln
995 1000 1005

His Lys His Tyr Leu Asp Glu Ile Ile Glu Gln Ile Ser Glu Phe
1010 1015 1020

Ser Lys Arg Val Ile Leu Ala Asp Ala Asn Leu Asp Lys Val Leu
1025 1030 1035

Ser Ala Tyr Asn Lys His Arg Asp Lys Pro Ile Arg Glu Gln Ala

1040 1045 1050

Glu Asn Ile Ile His Leu Phe Thr Leu Thr Asn Leu Gly Ala Pro
1055 1060 1065

Ala Ala Phe Lys Tyr Phe Asp Thr Thr Ile Asp Arg Lys Arg Tyr
1070 1075 1080

Thr Ser Thr Lys Glu Val Leu Asp Ala Thr Leu Ile His Gln Ser
1085 1090 1095

Ile Thr Gly Leu Tyr Glu Thr Arg Ile Asp Leu Ser Gln Leu Gly
1100 1105 1110

Gly Asp
1115

<210> 105
<211> 1374
<212> PRT
<213> Artificial Sequence

<220>
<221> source
<223> /note="Description of Artificial Sequence: Synthetic polypeptide"

<400> 105
Met Asp Lys Lys Tyr Ser Ile Gly Leu Asp Ile Gly Thr Asn Ser Val
1 5 10 15

Gly Trp Ala Val Ile Thr Asp Glu Tyr Lys Val Pro Ser Lys Lys Phe
20 25 30

Lys Val Leu Gly Asn Thr Asp Arg His Ser Ile Lys Lys Asn Leu Ile
35 40 45

Gly Ala Leu Leu Phe Asp Ser Gly Glu Thr Ala Glu Ala Thr Arg Leu
50 55 60

Lys Arg Thr Ala Arg Arg Arg Tyr Thr Arg Arg Lys Asn Arg Ile Cys
65 70 75 80

Tyr Leu Gln Glu Ile Phe Ser Asn Glu Met Ala Lys Val Asp Asp Ser
85 90 95

Phe Phe His Arg Leu Glu Glu Ser Phe Leu Val Glu Glu Asp Lys Lys
100 105 110

His Glu Arg His Pro Ile Phe Gly Asn Ile Val Asp Glu Val Ala Tyr

115 120 125

His Glu Lys Tyr Pro Thr Ile Tyr His Leu Arg Lys Lys Leu Val Asp
130 135 140

Ser Thr Asp Lys Ala Asp Leu Arg Leu Ile Tyr Leu Ala Leu Ala His
145 150 155 160

Met Ile Lys Phe Arg Gly His Phe Leu Ile Glu Gly Asp Leu Asn Pro
165 170 175

Asp Asn Ser Asp Val Asp Lys Leu Phe Ile Gln Leu Val Gln Thr Tyr
180 185 190

Asn Gln Leu Phe Glu Glu Asn Pro Ile Asn Ala Ser Gly Val Asp Ala
195 200 205

Lys Ala Ile Leu Ser Ala Arg Leu Ser Lys Ser Arg Arg Leu Glu Asn
210 215 220

Leu Ile Ala Gln Leu Pro Gly Glu Lys Lys Asn Gly Leu Phe Gly Asn
225 230 235 240

Leu Ile Ala Leu Ser Leu Gly Leu Thr Pro Asn Phe Lys Ser Asn Phe
245 250 255

Asp Leu Ala Glu Asp Ala Lys Leu Gln Leu Ser Lys Asp Thr Tyr Asp
260 265 270

Asp Asp Leu Asp Asn Leu Leu Ala Gln Ile Gly Asp Gln Tyr Ala Asp
275 280 285

Leu Phe Leu Ala Ala Lys Asn Leu Ser Asp Ala Ile Leu Leu Ser Asp
290 295 300

Ile Leu Arg Val Asn Thr Glu Ile Thr Lys Ala Pro Leu Ser Ala Ser
305 310 315 320

Met Ile Lys Arg Tyr Asp Glu His His Gln Asp Leu Thr Leu Leu Lys
325 330 335

Ala Leu Val Arg Gln Gln Leu Pro Glu Lys Tyr Lys Glu Ile Phe Phe
340 345 350

Asp Gln Ser Lys Asn Gly Tyr Ala Gly Tyr Ile Asp Gly Gly Ala Ser
355 360 365

```
Gln Glu Glu Phe Tyr Lys Phe Ile Lys Pro Ile Leu Glu Lys Met Asp
    370                 375                 380

Gly Thr Glu Glu Leu Leu Val Lys Leu Asn Arg Glu Asp Leu Leu Arg
385                 390                 395                 400

Lys Gln Arg Thr Phe Asp Asn Gly Ser Ile Pro His Gln Ile His Leu
                405                 410                 415

Gly Glu Leu His Ala Ile Leu Arg Arg Gln Glu Asp Phe Tyr Pro Phe
            420                 425                 430

Leu Lys Asp Asn Arg Glu Lys Ile Glu Lys Ile Leu Thr Phe Arg Ile
        435                 440                 445

Pro Tyr Tyr Val Gly Pro Leu Ala Arg Gly Asn Ser Arg Phe Ala Trp
    450                 455                 460

Met Thr Arg Lys Ser Glu Glu Thr Ile Thr Pro Trp Asn Phe Glu Glu
465                 470                 475                 480

Val Val Asp Lys Gly Ala Ser Ala Gln Ser Phe Ile Glu Arg Met Thr
                485                 490                 495

Asn Phe Asp Lys Asn Leu Pro Asn Glu Lys Val Leu Pro Lys His Ser
            500                 505                 510

Leu Leu Tyr Glu Tyr Phe Thr Val Tyr Asn Glu Leu Thr Lys Val Lys
        515                 520                 525

Tyr Val Thr Glu Gly Met Arg Lys Pro Ala Phe Leu Ser Gly Glu Gln
    530                 535                 540

Lys Lys Ala Ile Val Asp Leu Leu Phe Lys Thr Asn Arg Lys Val Thr
545                 550                 555                 560

Val Lys Gln Leu Lys Glu Asp Tyr Phe Lys Lys Ile Glu Cys Phe Asp
                565                 570                 575

Ser Val Glu Ile Ser Gly Val Glu Asp Arg Phe Asn Ala Ser Leu Gly
            580                 585                 590

Thr Tyr His Asp Leu Leu Lys Ile Ile Lys Asp Lys Asp Phe Leu Asp
        595                 600                 605

Asn Glu Glu Asn Glu Asp Ile Leu Glu Asp Ile Val Leu Thr Leu Thr
    610                 615                 620
```

Leu Phe Glu Asp Arg Glu Met Ile Glu Glu Arg Leu Lys Thr Tyr Ala
625 630 635 640

His Leu Phe Asp Asp Lys Val Met Lys Gln Leu Lys Arg Arg Arg Tyr
645 650 655

Thr Gly Trp Gly Arg Leu Ser Arg Lys Leu Ile Asn Gly Ile Arg Asp
660 665 670

Lys Gln Ser Gly Lys Thr Ile Leu Asp Phe Leu Lys Ser Asp Gly Phe
675 680 685

Ala Asn Arg Asn Phe Met Gln Leu Ile His Asp Asp Ser Leu Thr Phe
690 695 700

Lys Glu Asp Ile Gln Lys Ala Gln Val Ser Gly Gln Gly Asp Ser Leu
705 710 715 720

His Glu His Ile Ala Asn Leu Ala Gly Ser Pro Ala Ile Lys Lys Gly
725 730 735

Ile Leu Gln Thr Val Lys Val Val Asp Glu Leu Val Lys Val Met Gly
740 745 750

Arg His Lys Pro Glu Asn Ile Val Ile Glu Met Ala Arg Glu Thr Asn
755 760 765

Glu Asp Asp Glu Lys Lys Ala Ile Gln Lys Ile Gln Lys Ala Asn Lys
770 775 780

Asp Glu Lys Asp Ala Ala Met Leu Lys Ala Ala Asn Gln Tyr Asn Gly
785 790 795 800

Lys Ala Glu Leu Pro His Ser Val Phe His Gly His Lys Gln Leu Ala
805 810 815

Thr Lys Ile Arg Leu Trp His Gln Gln Gly Glu Arg Cys Leu Tyr Thr
820 825 830

Gly Lys Thr Ile Ser Ile His Asp Leu Ile Asn Asn Ser Asn Gln Phe
835 840 845

Glu Val Asp His Ile Leu Pro Leu Ser Ile Thr Phe Asp Asp Ser Leu
850 855 860

Ala Asn Lys Val Leu Val Tyr Ala Thr Ala Asn Gln Glu Lys Gly Gln
865 870 875 880

Arg Thr Pro Tyr Gln Ala Leu Asp Ser Met Asp Asp Ala Trp Ser Phe
885 890 895

Arg Glu Leu Lys Ala Phe Val Arg Glu Ser Lys Thr Leu Ser Asn Lys
900 905 910

Lys Lys Glu Tyr Leu Leu Thr Glu Glu Asp Ile Ser Lys Phe Asp Val
915 920 925

Arg Lys Lys Phe Ile Glu Arg Asn Leu Val Asp Thr Arg Tyr Ala Ser
930 935 940

Arg Val Val Leu Asn Ala Leu Gln Glu His Phe Arg Ala His Lys Ile
945 950 955 960

Asp Thr Lys Val Ser Val Val Arg Gly Gln Phe Thr Ser Gln Leu Arg
965 970 975

Arg His Trp Gly Ile Glu Lys Thr Arg Asp Thr Tyr His His His Ala
980 985 990

Val Asp Ala Leu Ile Ile Ala Ala Ser Ser Gln Leu Asn Leu Trp Lys
995 1000 1005

Lys Gln Lys Asn Thr Leu Val Ser Tyr Ser Glu Asp Gln Leu Leu
1010 1015 1020

Asp Ile Glu Thr Gly Glu Leu Ile Ser Asp Asp Glu Tyr Lys Glu
1025 1030 1035

Ser Val Phe Lys Ala Pro Tyr Gln His Phe Val Asp Thr Leu Lys
1040 1045 1050

Ser Lys Glu Phe Glu Asp Ser Ile Leu Phe Ser Tyr Gln Val Asp
1055 1060 1065

Ser Lys Phe Asn Arg Lys Ile Ser Asp Ala Thr Ile Tyr Ala Thr
1070 1075 1080

Arg Gln Ala Lys Val Gly Lys Asp Lys Ala Asp Glu Thr Tyr Val
1085 1090 1095

Leu Gly Lys Ile Lys Asp Ile Tyr Thr Gln Asp Gly Tyr Asp Ala
1100 1105 1110

Phe Met Lys Ile Tyr Lys Lys Asp Lys Ser Lys Phe Leu Met Tyr

1115 1120 1125

Arg His Asp Pro Gln Thr Phe Glu Lys Val Ile Glu Pro Ile Leu
1130 1135 1140

Glu Asn Tyr Pro Asn Lys Gln Ile Asn Glu Lys Gly Lys Glu Val
1145 1150 1155

Pro Cys Asn Pro Phe Leu Lys Tyr Lys Glu Glu His Gly Tyr Ile
1160 1165 1170

Arg Lys Tyr Ser Lys Lys Gly Asn Gly Pro Glu Ile Lys Ser Leu
1175 1180 1185

Lys Tyr Tyr Asp Ser Lys Leu Gly Asn His Ile Asp Ile Thr Pro
1190 1195 1200

Lys Asp Ser Asn Asn Lys Val Val Leu Gln Ser Val Ser Pro Trp
1205 1210 1215

Arg Ala Asp Val Tyr Phe Asn Lys Thr Thr Gly Lys Tyr Glu Ile
1220 1225 1230

Leu Gly Leu Lys Tyr Ala Asp Leu Gln Phe Glu Lys Gly Thr Gly
1235 1240 1245

Thr Tyr Lys Ile Ser Gln Glu Lys Tyr Asn Asp Ile Lys Lys Lys
1250 1255 1260

Glu Gly Val Asp Ser Asp Ser Glu Phe Lys Phe Thr Leu Tyr Lys
1265 1270 1275

Asn Asp Leu Leu Leu Val Lys Asp Thr Glu Thr Lys Glu Gln Gln
1280 1285 1290

Leu Phe Arg Phe Leu Ser Arg Thr Met Pro Lys Gln Lys His Tyr
1295 1300 1305

Val Glu Leu Lys Pro Tyr Asp Lys Gln Lys Phe Glu Gly Gly Glu
1310 1315 1320

Ala Leu Ile Lys Val Leu Gly Asn Val Ala Asn Ser Gly Gln Cys
1325 1330 1335

Lys Lys Gly Leu Gly Lys Ser Asn Ile Ser Ile Tyr Lys Val Arg
1340 1345 1350

Thr Asp Val Leu Gly Asn Gln His Ile Ile Lys Asn Glu Gly Asp
1355 1360 1365

Lys Pro Lys Leu Asp Phe
1370

<210> 106
<211> 6
<212> PRT
<213> Artificial Sequence

<220>
<221> source
<223> /note="Description of Artificial Sequence: Synthetic 6xHis tag"

<400> 106
His His His His His His
1 5

<210> 107
<211> 15
<212> PRT
<213> Artificial Sequence

<220>
<221> source
<223> /note="Description of Artificial Sequence: Synthetic peptide"

<400> 107
Gly Gly Gly Gly Ser Gly Gly Gly Gly Ser Gly Gly Gly Gly Ser
1 5 10 15

<210> 108
<211> 15
<212> PRT
<213> Artificial Sequence

<220>
<221> source
<223> /note="Description of Artificial Sequence: Synthetic peptide"

<400> 108
Glu Ala Ala Ala Lys Glu Ala Ala Ala Lys Glu Ala Ala Ala Lys
1 5 10 15

<210> 109
<211> 18
<212> PRT
<213> Artificial Sequence

<220>
<221> source
<223> /note="Description of Artificial Sequence: Synthetic peptide"

<400> 109
Gly Gly Gly Gly Gly Ser Gly Gly Gly Gly Gly Ser Gly Gly Gly Gly
1               5                   10                  15

Gly Ser

<210> 110
<211> 23
<212> DNA
<213> Artificial Sequence

<220>
<221> source
<223> /note="Description of Artificial Sequence: Synthetic oligonucleotide"

<400> 110
gccaaattgg acgaccctcg cgg 23

<210> 111
<211> 23
<212> DNA
<213> Artificial Sequence

<220>
<221> source
<223> /note="Description of Artificial Sequence: Synthetic oligonucleotide"

<400> 111
cgaggagacc cccgtttcgg tgg 23

<210> 112
<211> 23
<212> DNA
<213> Artificial Sequence

<220>
<221> source
<223> /note="Description of Artificial Sequence: Synthetic oligonucleotide"

<400> 112
cccgccgccg ccgtggctcg agg 23

<210> 113
<211> 23
<212> DNA
<213> Artificial Sequence

<220>
<221> source
<223> /note="Description of Artificial Sequence: Synthetic oligonucleotide"

<400> 113
tgagctctac gagatccaca agg 23

<210> 114
<211> 23
<212> DNA
<213> Artificial Sequence

<220>
<221> source
<223> /note="Description of Artificial Sequence: Synthetic oligonucleotide"

<400> 114
ctcaaaattc ataccggttg tgg 23

<210> 115
<211> 23
<212> DNA
<213> Artificial Sequence

<220>
<221> source
<223> /note="Description of Artificial Sequence: Synthetic oligonucleotide"

<400> 115
cgttaaacaa caaccggact tgg 23

<210> 116
<211> 23
<212> DNA
<213> Artificial Sequence

<220>
<221> source
<223> /note="Description of Artificial Sequence: Synthetic oligonucleotide"

<400> 116
ttcaccccgc ggcgctgaat ggg 23

<210> 117
<211> 23
<212> DNA
<213> Artificial Sequence

<220>
<221> source
<223> /note="Description of Artificial Sequence: Synthetic oligonucleotide"

<400> 117
accactacca gtccgtccac agg 23

<210> 118
<211> 23
<212> DNA
<213> Artificial Sequence

<220>
<221> source
<223> /note="Description of Artificial Sequence: Synthetic
oligonucleotide"

<400> 118
agcctttctg aacacatgca cgg 23

<210> 119
<211> 39
<212> DNA
<213> Homo sapiens

<400> 119
cctgccatca atgtggccat gcatgtgttc agaaaggct 39

<210> 120
<211> 39
<212> DNA
<213> Homo sapiens

<400> 120
cctgccatca atgtggccgt gcatgtgttc agaaaggct 39

<210> 121
<211> 23
<212> DNA
<213> Artificial Sequence

<220>
<221> source
<223> /note="Description of Artificial Sequence: Synthetic
oligonucleotide"

<400> 121
cactgcttaa gcctcgctcg agg 23

<210> 122
<211> 23
<212> DNA
<213> Artificial Sequence

<220>
<221> source
<223> /note="Description of Artificial Sequence: Synthetic
oligonucleotide"

<400> 122
tcaccagcaa tattcgctcg agg 23

<210> 123
<211> 23
<212> DNA
<213> Artificial Sequence

<220>
<221> source
<223> /note="Description of Artificial Sequence: Synthetic

oligonucleotide"

<400> 123
caccagcaat attccgctcg agg 23

<210> 124
<211> 23
<212> DNA
<213> Artificial Sequence

<220>
<221> source
<223> /note="Description of Artificial Sequence: Synthetic oligonucleotide"

<400> 124
tagcaacaga catacgctcg agg 23

<210> 125
<211> 23
<212> DNA
<213> Artificial Sequence

<220>
<221> source
<223> /note="Description of Artificial Sequence: Synthetic oligonucleotide"

<400> 125
gggcagtagt aatacgctcg agg 23

<210> 126
<211> 23
<212> DNA
<213> Artificial Sequence

<220>
<221> source
<223> /note="Description of Artificial Sequence: Synthetic oligonucleotide"

<400> 126
ccaattccca tacattattg tac 23

<210> 127
<211> 26
<212> DNA
<213> Homo sapiens

<400> 127
ccgtgccggg cgggagaccg ccatgg 26

<210> 128
<211> 22
<212> DNA
<213> Homo sapiens

<400> 128

ggcccggctg tggctgagga gc 22

<210> 129
<211> 20
<212> DNA
<213> Homo sapiens

<400> 129
cggtctcccg cccggcacgg 20

<210> 130
<211> 26
<212> DNA
<213> Homo sapiens

<400> 130
gctcctcagc cacagccggg ccgggt 26

<210> 131
<211> 12
<212> DNA
<213> Homo sapiens

<400> 131
cgaccctgga aa 12

<210> 132
<211> 14
<212> DNA
<213> Homo sapiens

<400> 132
ccccgccgcc accc 14

<210> 133
<211> 18
<212> DNA
<213> Homo sapiens

<400> 133
tttccagggt cgccatgg 18

<210> 134
<211> 10
<212> DNA
<213> Homo sapiens

<400> 134
ggcggcgggg 10

<210> 135
<211> 23
<212> DNA
<213> Artificial Sequence

<220>

<221> source
<223> /note="Description of Artificial Sequence: Synthetic primer"

<400> 135
gagggcctat ttcccatgat tcc 23

<210> 136
<211> 126
<212> DNA
<213> Artificial Sequence

<220>
<221> source
<223> /note="Description of Artificial Sequence: Synthetic primer"

<220>
<221> modified_base
<222> (84)..(102)
<223> a, c, t, g, unknown or other

<400> 136
aaaaaaagca ccgactcggt gccacttttt caagttgata acggactagc cttattttaa 60

cttgctattt ctagctctaa aacnnnnnnn nnnnnnnnnn nnccggtgtt tcgtcctttc 120

cacaag 126

<210> 137
<211> 24
<212> DNA
<213> Artificial Sequence

<220>
<221> source
<223> /note="Description of Artificial Sequence: Synthetic primer"

<220>
<221> modified_base
<222> (6)..(24)
<223> a, c, t, g, unknown or other

<400> 137
caccgnnnnn nnnnnnnnnn nnnn 24

<210> 138
<211> 24
<212> DNA
<213> Artificial Sequence

<220>
<221> source
<223> /note="Description of Artificial Sequence: Synthetic primer"

<220>
<221> modified_base
<222> (5)..(23)
<223> a, c, t, g, unknown or other

<400> 138
aaacnnnnnn nnnnnnnnnn nnnc 24

<210> 139
<211> 126
<212> DNA
<213> Artificial Sequence

<220>
<221> source
<223> /note="Description of Artificial Sequence: Synthetic primer"

<400> 139
aaaaaaagca ccgactcggt gccacttttt caagttgata acggactagc cttattttaa 60

cttgctattt ctagctctaa aacccctagt cattggaggt gaccggtgtt tcgtcctttc 120

cacaag 126

<210> 140
<211> 24
<212> DNA
<213> Artificial Sequence

<220>
<221> source
<223> /note="Description of Artificial Sequence: Synthetic primer"

<400> 140
caccgtcacc tccaatgact aggg 24

<210> 141
<211> 24
<212> DNA
<213> Artificial Sequence

<220>
<221> source
<223> /note="Description of Artificial Sequence: Synthetic primer"

<400> 141
aaacccctag tcattggagg tgac 24

<210> 142
<211> 192
<212> DNA
<213> Artificial Sequence

<220>
<221> source
<223> /note="Description of Artificial Sequence: Synthetic

primer"

<400> 142
cagaagaaga agggctccca tcacatcaac cggtggcgca ttgccacgaa gcaggccaat 60

ggggaggaca tcgatgtcac ctccaatgac aagcttgcta gcggtgggca accacaaacc 120

cacgagggca gagtgctgct tgctgctggc caggcccctg cgtgggccca agctggactc 180

tggccactcc ct 192

<210> 143
<211> 192
<212> DNA
<213> Artificial Sequence

<220>
<221> source
<223> /note="Description of Artificial Sequence: Synthetic primer"

<400> 143
agggagtggc cagagtccag cttgggccca cgcaggggcc tggccagcag caagcagcac 60

tctgccctcg tgggtttgtg gttgcccacc gctagcaagc ttgtcattgg aggtgacatc 120

gatgtcctcc ccattggcct gcttcgtggc aatgcgccac cggttgatgt gatgggagcc 180

cttcttcttc tg 192

<210> 144
<211> 20
<212> DNA
<213> Artificial Sequence

<220>
<221> source
<223> /note="Description of Artificial Sequence: Synthetic primer"

<400> 144
ccatcccctt ctgtgaatgt 20

<210> 145
<211> 20
<212> DNA
<213> Artificial Sequence

<220>
<221> source
<223> /note="Description of Artificial Sequence: Synthetic primer"

<400> 145
ggagattgga gacacggaga 20

<210> 146
<211> 20
<212> DNA

<213> Artificial Sequence

<220>
<221> source
<223> /note="Description of Artificial Sequence: Synthetic primer"

<400> 146
ggctccctgg gttcaaagta 20

<210> 147
<211> 21
<212> DNA
<213> Artificial Sequence

<220>
<221> source
<223> /note="Description of Artificial Sequence: Synthetic primer"

<400> 147
agaggggtct ggatgtcgta a 21

<210> 148
<211> 24
<212> DNA
<213> Artificial Sequence

<220>
<221> source
<223> /note="Description of Artificial Sequence: Synthetic primer"

<400> 148
cgccagggtt ttcccagtca cgac 24

<210> 149
<211> 51
<212> DNA
<213> Artificial Sequence

<220>
<221> source
<223> /note="Description of Artificial Sequence: Synthetic primer"

<400> 149
gagggtctcg tccttgcggc cgcgctagcg agggcctatt tcccatgatt c 51

<210> 150
<211> 133
<212> DNA
<213> Artificial Sequence

<220>
<221> source
<223> /note="Description of Artificial Sequence: Synthetic primer"

<220>
<221> modified_base
<222> (95)..(114)
<223> a, c, t, g, unknown or other

<400> 150
ctcggtctcg gtaaaaaagc accgactcgg tgccactttt tcaagttgat aacggactag 60

ccttatttta acttgctatt tctagctcta aaacnnnnnn nnnnnnnnnn nnnnggtgtt 120

tcgtcctttc cac 133

<210> 151
<211> 41
<212> DNA
<213> Artificial Sequence

<220>
<221> source
<223> /note="Description of Artificial Sequence: Synthetic primer"

<400> 151
gagggtctct ttaccggtga gggcctattt cccatgattc c 41

<210> 152
<211> 133
<212> DNA
<213> Artificial Sequence

<220>
<221> source
<223> /note="Description of Artificial Sequence: Synthetic primer"

<220>
<221> modified_base
<222> (95)..(114)
<223> a, c, t, g, unknown or other

<400> 152
ctcggtctcc tcaaaaaagc accgactcgg tgccactttt tcaagttgat aacggactag 60

ccttatttta acttgctatt tctagctcta aaacnnnnnn nnnnnnnnnn nnnnggtgtt 120

tcgtcctttc cac 133

<210> 153
<211> 40
<212> DNA
<213> Artificial Sequence

<220>
<221> source
<223> /note="Description of Artificial Sequence: Synthetic primer"

<400> 153

gagggtctct ttgagctcga gggcctattt cccatgattc 40

<210> 154
<211> 133
<212> DNA
<213> Artificial Sequence

<220>
<221> source
<223> /note="Description of Artificial Sequence: Synthetic
primer"

<220>
<221> modified_base
<222> (96)..(115)
<223> a, c, t, g, unknown or other

<400> 154
ctcggtctcg cgtaaaaaag caccgactcg gtgccacttt ttcaagttga taacggacta 60

gccttatttt aacttgctat ttctagctct aaaacnnnnn nnnnnnnnnn nnnnnggtgt 120

ttcgtccttt cca 133

<210> 155
<211> 27
<212> DNA
<213> Artificial Sequence

<220>
<221> source
<223> /note="Description of Artificial Sequence: Synthetic
primer"

<400> 155
gagggtctct tacgcgtgtg tctagac 27

<210> 156
<211> 98
<212> DNA
<213> Artificial Sequence

<220>
<221> source
<223> /note="Description of Artificial Sequence: Synthetic
primer"

<400> 156
ctcggtctca aggacaggga agggagcagt ggttcacgcc tgtaatccca gcaatttggg 60

aggccaaggt gggtagatca cctgagatta ggagttgc 98

<210> 157
<211> 30
<212> DNA
<213> Artificial Sequence

<220>

<221> source
<223> /note="Description of Artificial Sequence: Synthetic
      primer"

<400> 157
cctgtccttg cggccgcgct agcgagggcc                                        30

<210> 158
<211> 31
<212> DNA
<213> Artificial Sequence

<220>
<221> source
<223> /note="Description of Artificial Sequence: Synthetic
      primer"

<400> 158
cacgcggccg caaggacagg gaagggagca g                                       31

<210> 159
<211> 327
<212> PRT
<213> Artificial Sequence

<220>
<221> source
<223> /note="Description of Artificial Sequence: Synthetic
      polypeptide"

<400> 159
Met Val Ser Lys Gly Glu Glu Leu Phe Thr Gly Val Val Pro Ile Leu
1               5                   10                  15

Val Glu Leu Asp Gly Asp Val Asn Gly His Lys Phe Ser Val Ser Gly
            20                  25                  30

Glu Gly Glu Gly Asp Ala Thr Tyr Gly Lys Leu Thr Leu Lys Phe Ile
        35                  40                  45

Cys Thr Thr Gly Lys Leu Pro Val Pro Trp Pro Thr Leu Val Thr Thr
    50                  55                  60

Leu Thr Tyr Gly Val Gln Cys Phe Ser Arg Tyr Pro Asp His Met Lys
65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125

```
Ile Asp Phe Lys Glu Asp Gly Asn Ile Leu Gly His Lys Leu Glu Tyr
    130                 135                 140

Asn Tyr Asn Ser His Asn Val Tyr Ile Met Ala Asp Lys Gln Lys Asn
145                 150                 155                 160

Gly Ile Lys Val Asn Phe Lys Ile Arg His Asn Ile Glu Asp Gly Ser
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Thr Gln Ser Ala Leu
        195                 200                 205

Ser Lys Asp Pro Asn Glu Lys Arg Asp His Met Val Leu Leu Glu Phe
    210                 215                 220

Val Thr Ala Ala Gly Ile Thr Leu Gly Met Asp Glu Leu Tyr Lys Ser
225                 230                 235                 240

Gly Leu Arg Ser Arg Glu Glu Glu Glu Glu Thr Asp Ser Arg Met Pro
                245                 250                 255

His Leu Asp Ser Pro Gly Ser Ser Gln Pro Arg Arg Ser Phe Leu Ser
            260                 265                 270

Arg Val Ile Arg Ala Ala Leu Pro Leu Gln Leu Leu Leu Leu Leu Leu
        275                 280                 285

Leu Leu Leu Ala Cys Leu Leu Pro Ala Ser Glu Asp Asp Tyr Ser Cys
    290                 295                 300

Thr Gln Ala Asn Asn Phe Ala Arg Ser Phe Tyr Pro Met Leu Arg Tyr
305                 310                 315                 320

Thr Asn Gly Pro Pro Pro Thr
                325
```

<210> 160
<211> 22
<212> DNA
<213> Artificial Sequence

<220>
<221> source
<223> /note="Description of Artificial Sequence: Synthetic primer"

<400> 160
gcagcctctg ttccacatac ac 22

<210> 161
<211> 22
<212> DNA
<213> Artificial Sequence

<220>
<221> source
<223> /note="Description of Artificial Sequence: Synthetic primer"

<400> 161
accattctca gtggctcaac aa 22

<210> 162
<211> 20
<212> DNA
<213> Artificial Sequence

<220>
<221> source
<223> /note="Description of Artificial Sequence: Synthetic oligonucleotide"

<400> 162
gtcggggtcc ctgaaccgga 20

<210> 163
<211> 20
<212> DNA
<213> Artificial Sequence

<220>
<221> source
<223> /note="Description of Artificial Sequence: Synthetic oligonucleotide"

<400> 163
gcttacctgg gctgtagaac 20

<210> 164
<211> 20
<212> DNA
<213> Artificial Sequence

<220>
<221> source
<223> /note="Description of Artificial Sequence: Synthetic oligonucleotide"

<400> 164
cttatcctgt tctacagccc 20

<210> 165
<211> 20

<212> DNA
<213> Artificial Sequence

<220>
<221> source
<223> /note="Description of Artificial Sequence: Synthetic oligonucleotide"

<400> 165
agatgtcttc ttttgcttgc 20

<210> 166
<211> 20
<212> DNA
<213> Artificial Sequence

<220>
<221> source
<223> /note="Description of Artificial Sequence: Synthetic oligonucleotide"

<400> 166
gtctccaggc cgcccatgtc 20

<210> 167
<211> 20
<212> DNA
<213> Artificial Sequence

<220>
<221> source
<223> /note="Description of Artificial Sequence: Synthetic oligonucleotide"

<400> 167
catgtctggg gctgcgcagt 20

<210> 168
<211> 20
<212> DNA
<213> Artificial Sequence

<220>
<221> source
<223> /note="Description of Artificial Sequence: Synthetic oligonucleotide"

<400> 168
agccccagac atgggcggcc 20

<210> 169
<211> 20
<212> DNA
<213> Artificial Sequence

<220>
<221> source
<223> /note="Description of Artificial Sequence: Synthetic oligonucleotide"

<400> 169
ttttattcgt gggcgtctcc 20

<210> 170
<211> 20
<212> DNA
<213> Artificial Sequence

<220>
<221> source
<223> /note="Description of Artificial Sequence: Synthetic oligonucleotide"

<400> 170
gctttcccga acacgtgaaa 20

<210> 171
<211> 20
<212> DNA
<213> Artificial Sequence

<220>
<221> source
<223> /note="Description of Artificial Sequence: Synthetic oligonucleotide"

<400> 171
cttttgccat ttcacgtgtt 20

<210> 172
<211> 20
<212> DNA
<213> Artificial Sequence

<220>
<221> source
<223> /note="Description of Artificial Sequence: Synthetic oligonucleotide"

<400> 172
gcagccgcct cgggtgtttg 20

<210> 173
<211> 20
<212> DNA
<213> Artificial Sequence

<220>
<221> source
<223> /note="Description of Artificial Sequence: Synthetic oligonucleotide"

<400> 173
cagccgcctc gggtgtttgt 20

<210> 174
<211> 20

<212> DNA
<213> Artificial Sequence

<220>
<221> source
<223> /note="Description of Artificial Sequence: Synthetic oligonucleotide"

<400> 174
aggggacacc gtgcactcaa 20

<210> 175
<211> 20
<212> DNA
<213> Artificial Sequence

<220>
<221> source
<223> /note="Description of Artificial Sequence: Synthetic oligonucleotide"

<400> 175
gagctggatc tacggcccca 20

<210> 176
<211> 20
<212> DNA
<213> Artificial Sequence

<220>
<221> source
<223> /note="Description of Artificial Sequence: Synthetic oligonucleotide"

<400> 176
gcggatgata tacccaccat 20

<210> 177
<211> 20
<212> DNA
<213> Artificial Sequence

<220>
<221> source
<223> /note="Description of Artificial Sequence: Synthetic oligonucleotide"

<400> 177
tggatctacg gccccatggt 20

<210> 178
<211> 20
<212> DNA
<213> Artificial Sequence

<220>
<221> source
<223> /note="Description of Artificial Sequence: Synthetic oligonucleotide"

<400> 178
tcacacggga cgtgcccgaa 20

<210> 179
<211> 20
<212> DNA
<213> Artificial Sequence

<220>
<221> source
<223> /note="Description of Artificial Sequence: Synthetic oligonucleotide"

<400> 179
cgggacgtgc ccgaaaggct 20

<210> 180
<211> 20
<212> DNA
<213> Artificial Sequence

<220>
<221> source
<223> /note="Description of Artificial Sequence: Synthetic oligonucleotide"

<400> 180
ggacgtgccc gaaaggctcg 20

<210> 181
<211> 20
<212> DNA
<213> Artificial Sequence

<220>
<221> source
<223> /note="Description of Artificial Sequence: Synthetic oligonucleotide"

<400> 181
gggacgtgcc cgaaaggctc 20

<210> 182
<211> 20
<212> DNA
<213> Artificial Sequence

<220>
<221> source
<223> /note="Description of Artificial Sequence: Synthetic oligonucleotide"

<400> 182
aagacggaca ggcacctacg 20

<210> 183
<211> 20

<212> DNA
<213> Artificial Sequence

<220>
<221> source
<223> /note="Description of Artificial Sequence: Synthetic oligonucleotide"

<400> 183
tgcgaatacg cccacgcgat 20

<210> 184
<211> 20
<212> DNA
<213> Artificial Sequence

<220>
<221> source
<223> /note="Description of Artificial Sequence: Synthetic oligonucleotide"

<400> 184
cccttggtcg cgcttaacgt 20

<210> 185
<211> 20
<212> DNA
<213> Artificial Sequence

<220>
<221> source
<223> /note="Description of Artificial Sequence: Synthetic oligonucleotide"

<400> 185
cccacgttaa gcgcgaccaa 20

<210> 186
<211> 20
<212> DNA
<213> Artificial Sequence

<220>
<221> source
<223> /note="Description of Artificial Sequence: Synthetic oligonucleotide"

<400> 186
ctccagcgtg tatcggtgca 20

<210> 187
<211> 20
<212> DNA
<213> Artificial Sequence

<220>
<221> source
<223> /note="Description of Artificial Sequence: Synthetic oligonucleotide"

<400> 187
ggatgatctc tcggaactgt 20

<210> 188
<211> 20
<212> DNA
<213> Artificial Sequence

<220>
<221> source
<223> /note="Description of Artificial Sequence: Synthetic oligonucleotide"

<400> 188
cgtcgccgtc cagctcgacc 20

<210> 189
<211> 20
<212> DNA
<213> Artificial Sequence

<220>
<221> source
<223> /note="Description of Artificial Sequence: Synthetic oligonucleotide"

<400> 189
cagggtcagc ttgccgtagg 20

<210> 190
<211> 21
<212> DNA
<213> Artificial Sequence

<220>
<221> source
<223> /note="Description of Artificial Sequence: Synthetic oligonucleotide"

<400> 190
tgttcgcatt atccgaacca t 21

<210> 191
<211> 21
<212> DNA
<213> Artificial Sequence

<220>
<221> source
<223> /note="Description of Artificial Sequence: Synthetic oligonucleotide"

<400> 191
cgcgatcgta atcacccgag t 21

<210> 192
<211> 21

<212> DNA
<213> Artificial Sequence

<220>
<221> source
<223> /note="Description of Artificial Sequence: Synthetic oligonucleotide"

<400> 192
ctcagttcca gtacggctcc a 21

<210> 193
<211> 21
<212> DNA
<213> Artificial Sequence

<220>
<221> source
<223> /note="Description of Artificial Sequence: Synthetic oligonucleotide"

<400> 193
gcttcaagtg ggagcgcgtg a 21

<210> 194
<211> 21
<212> DNA
<213> Artificial Sequence

<220>
<221> source
<223> /note="Description of Artificial Sequence: Synthetic oligonucleotide"

<400> 194
acgtctatat catggccgac a 21

<210> 195
<211> 21
<212> DNA
<213> Artificial Sequence

<220>
<221> source
<223> /note="Description of Artificial Sequence: Synthetic oligonucleotide"

<400> 195
cccgaccaca tgaagcagca c 21

<210> 196
<211> 21
<212> DNA
<213> Artificial Sequence

<220>
<221> source
<223> /note="Description of Artificial Sequence: Synthetic oligonucleotide"

<400> 196
cagcagccca aacgactaca t 21

<210> 197
<211> 22
<212> DNA
<213> Artificial Sequence

<220>
<221> source
<223> /note="Description of Artificial Sequence: Synthetic oligonucleotide"

<400> 197
ctaaggaatc tcggctggag gt 22

<210> 198
<211> 22
<212> DNA
<213> Artificial Sequence

<220>
<221> source
<223> /note="Description of Artificial Sequence: Synthetic oligonucleotide"

<400> 198
tcactggcac agaggagatt gt 22

<210> 199
<211> 22
<212> DNA
<213> Artificial Sequence

<220>
<221> source
<223> /note="Description of Artificial Sequence: Synthetic oligonucleotide"

<400> 199
tggagtctcc catttaccca ct 22

<210> 200
<211> 22
<212> DNA
<213> Artificial Sequence

<220>
<221> source
<223> /note="Description of Artificial Sequence: Synthetic oligonucleotide"

<400> 200
gacccaggag aagtcactga gc 22

<210> 201
<211> 22

<212> DNA
<213> Artificial Sequence

<220>
<221> source
<223> /note="Description of Artificial Sequence: Synthetic oligonucleotide"

<400> 201
tggtaataga gagccccaag ga 22

<210> 202
<211> 22
<212> DNA
<213> Artificial Sequence

<220>
<221> source
<223> /note="Description of Artificial Sequence: Synthetic oligonucleotide"

<400> 202
cacgctcact tcctctttct ca 22

<210> 203
<211> 22
<212> DNA
<213> Artificial Sequence

<220>
<221> source
<223> /note="Description of Artificial Sequence: Synthetic oligonucleotide"

<400> 203
cctaggagtg aggctcgtga gt 22

<210> 204
<211> 22
<212> DNA
<213> Artificial Sequence

<220>
<221> source
<223> /note="Description of Artificial Sequence: Synthetic oligonucleotide"

<400> 204
tggcttagaa cataggccac ct 22

<210> 205
<211> 22
<212> DNA
<213> Artificial Sequence

<220>
<221> source
<223> /note="Description of Artificial Sequence: Synthetic oligonucleotide"

<400> 205
taccttccct ggcataggtg tt 22

<210> 206
<211> 22
<212> DNA
<213> Artificial Sequence

<220>
<221> source
<223> /note="Description of Artificial Sequence: Synthetic oligonucleotide"

<400> 206
tagactccac agaccccaca ga 22

<210> 207
<211> 26
<212> DNA
<213> Artificial Sequence

<220>
<221> source
<223> /note="Description of Artificial Sequence: Synthetic oligonucleotide"

<400> 207
aggagtatta gtacttccca ccctaa 26

<210> 208
<211> 21
<212> DNA
<213> Artificial Sequence

<220>
<221> source
<223> /note="Description of Artificial Sequence: Synthetic oligonucleotide"

<400> 208
cgtggtggtg tcaatactgc t 21

<210> 209
<211> 21
<212> DNA
<213> Artificial Sequence

<220>
<221> source
<223> /note="Description of Artificial Sequence: Synthetic oligonucleotide"

<400> 209
tgaaggtgcc atgttgagtt c 21

<210> 210
<211> 18

<212> DNA
<213> Artificial Sequence

<220>
<221> source
<223> /note="Description of Artificial Sequence: Synthetic oligonucleotide"

<400> 210
tgccatgtct gcgggaga 18

<210> 211
<211> 22
<212> DNA
<213> Artificial Sequence

<220>
<221> source
<223> /note="Description of Artificial Sequence: Synthetic oligonucleotide"

<400> 211
ggcggaggag atgggcttcg ag 22

<210> 212
<211> 21
<212> DNA
<213> Artificial Sequence

<220>
<221> source
<223> /note="Description of Artificial Sequence: Synthetic oligonucleotide"

<400> 212
cgagagagaa ccccagaaga c 21

<210> 213
<211> 20
<212> DNA
<213> Artificial Sequence

<220>
<221> source
<223> /note="Description of Artificial Sequence: Synthetic oligonucleotide"

<400> 213
agaagtgtcc aggcttccct 20

<210> 214
<211> 21
<212> DNA
<213> Artificial Sequence

<220>
<221> source
<223> /note="Description of Artificial Sequence: Synthetic oligonucleotide"

<400> 214
gcgactgatt cctattaaat a 21

<210> 215
<211> 21
<212> DNA
<213> Artificial Sequence

<220>
<221> source
<223> /note="Description of Artificial Sequence: Synthetic oligonucleotide"

<400> 215
agctatcact catggatata a 21

<210> 216
<211> 102
<212> DNA
<213> Artificial Sequence

<220>
<221> source
<223> /note="Description of Artificial Sequence: Synthetic polynucleotide"

<400> 216
gttttagagc tatgctgttt tgaatggtcc caaaacggaa gggcctgagt ccgagcagaa 60

gaagaagttt tagagctatg ctgttttgaa tggtcccaaa ac 102

<210> 217
<211> 100
<212> DNA
<213> Homo sapiens

<400> 217
cggaggacaa agtacaaacg gcagaagctg gaggaggaag ggcctgagtc cgagcagaag 60

aagaagggct cccatcacat caaccggtgg cgcattgcca 100

<210> 218
<211> 50
<212> DNA
<213> Homo sapiens

<400> 218
agctggagga ggaagggcct gagtccgagc agaagaagaa gggctcccac 50

<210> 219
<211> 30
<212> RNA
<213> Artificial Sequence

<220>
<221> source
<223> /note="Description of Artificial Sequence: Synthetic

oligonucleotide"

<400> 219
gaguccgagc agaagaagaa guuuuagagc 30

<210> 220
<211> 49
<212> DNA
<213> Artificial Sequence

<220>
<221> source
<223> /note="Description of Artificial Sequence: Synthetic oligonucleotide"

<400> 220
agctggagga ggaagggcct gagtccgagc agaagagaag ggctcccat 49

<210> 221
<211> 53
<212> DNA
<213> Homo sapiens

<400> 221
ctggaggagg aagggcctga gtccgagcag aagaagaagg gctcccatca cat 53

<210> 222
<211> 52
<212> DNA
<213> Homo sapiens

<400> 222
ctggaggagg aagggcctga gtccgagcag aagagaaggg ctcccatcac at 52

<210> 223
<211> 54
<212> DNA
<213> Homo sapiens

<400> 223
ctggaggagg aagggcctga gtccgagcag aagaaagaag ggctcccatc acat 54

<210> 224
<211> 50
<212> DNA
<213> Homo sapiens

<400> 224
ctggaggagg aagggcctga gtccgagcag aagaagggct cccatcacat 50

<210> 225
<211> 47
<212> DNA
<213> Homo sapiens

<400> 225
ctggaggagg aagggcctga gcccgagcag aagggctccc atcacat 47

<210> 226
<211> 48
<212> DNA
<213> Homo sapiens

<400> 226
ctggaggagg aagggcctga gtccgagcag aagaagaagg gctcccat 48

<210> 227
<211> 20
<212> RNA
<213> Artificial Sequence

<220>
<221> source
<223> /note="Description of Artificial Sequence: Synthetic oligonucleotide"

<400> 227
gaguccgagc agaagaagau 20

<210> 228
<211> 20
<212> RNA
<213> Artificial Sequence

<220>
<221> source
<223> /note="Description of Artificial Sequence: Synthetic oligonucleotide"

<400> 228
gaguccgagc agaagaagua 20

<210> 229
<211> 20
<212> RNA
<213> Artificial Sequence

<220>
<221> source
<223> /note="Description of Artificial Sequence: Synthetic oligonucleotide"

<400> 229
gaguccgagc agaagaacaa 20

<210> 230
<211> 20
<212> RNA
<213> Artificial Sequence

<220>
<221> source
<223> /note="Description of Artificial Sequence: Synthetic oligonucleotide"

<400> 230
gaguccgagc agaagaugaa 20

<210> 231
<211> 20
<212> RNA
<213> Artificial Sequence

<220>
<221> source
<223> /note="Description of Artificial Sequence: Synthetic oligonucleotide"

<400> 231
gaguccgagc agaaguagaa 20

<210> 232
<211> 20
<212> RNA
<213> Artificial Sequence

<220>
<221> source
<223> /note="Description of Artificial Sequence: Synthetic oligonucleotide"

<400> 232
gaguccgagc agaugaagaa 20

<210> 233
<211> 20
<212> RNA
<213> Artificial Sequence

<220>
<221> source
<223> /note="Description of Artificial Sequence: Synthetic oligonucleotide"

<400> 233
gaguccgagc acaagaagaa 20

<210> 234
<211> 20
<212> RNA
<213> Artificial Sequence

<220>
<221> source
<223> /note="Description of Artificial Sequence: Synthetic oligonucleotide"

<400> 234
gaguccgagg agaagaagaa 20

<210> 235
<211> 20
<212> RNA

<213> Artificial Sequence

<220>
<221> source
<223> /note="Description of Artificial Sequence: Synthetic oligonucleotide"

<400> 235
gaguccgugc agaagaagaa 20

<210> 236
<211> 20
<212> RNA
<213> Artificial Sequence

<220>
<221> source
<223> /note="Description of Artificial Sequence: Synthetic oligonucleotide"

<400> 236
gagucggagc agaagaagaa 20

<210> 237
<211> 20
<212> RNA
<213> Artificial Sequence

<220>
<221> source
<223> /note="Description of Artificial Sequence: Synthetic oligonucleotide"

<400> 237
gagaccgagc agaagaagaa 20

<210> 238
<211> 24
<212> DNA
<213> Artificial Sequence

<220>
<221> source
<223> /note="Description of Artificial Sequence: Synthetic oligonucleotide"

<400> 238
aatgacaagc ttgctagcgg tggg 24

<210> 239
<211> 39
<212> DNA
<213> Artificial Sequence

<220>
<221> source
<223> /note="Description of Artificial Sequence: Synthetic oligonucleotide"

<400> 239
aaaacggaag ggcctgagtc cgagcagaag aagaagttt 39

<210> 240
<211> 39
<212> DNA
<213> Artificial Sequence

<220>
<221> source
<223> /note="Description of Artificial Sequence: Synthetic oligonucleotide"

<400> 240
aaacaggggc cgagattggg tgttcagggc agaggtttt 39

<210> 241
<211> 38
<212> DNA
<213> Artificial Sequence

<220>
<221> source
<223> /note="Description of Artificial Sequence: Synthetic oligonucleotide"

<400> 241
aaaacggaag ggcctgagtc cgagcagaag aagaagtt 38

<210> 242
<211> 40
<212> DNA
<213> Artificial Sequence

<220>
<221> source
<223> /note="Description of Artificial Sequence: Synthetic oligonucleotide"

<400> 242
aacggaggga ggggcacaga tgagaaactc agggttttag 40

<210> 243
<211> 38
<212> DNA
<213> Homo sapiens

<400> 243
agcccttctt cttctgctcg gactcaggcc cttcctcc 38

<210> 244
<211> 40
<212> DNA
<213> Homo sapiens

<400> 244
cagggaggga ggggcacaga tgagaaactc aggaggcccc 40

<210> 245
<211> 80
<212> DNA
<213> Artificial Sequence

<220>
<221> source
<223> /note="Description of Artificial Sequence: Synthetic oligonucleotide"

<400> 245
ggcaatgcgc caccggttga tgtgatggga gcccttctag gaggccccca gagcagccac 60

tggggcctca acactcaggc 80

<210> 246
<211> 33
<212> DNA
<213> Homo sapiens

<400> 246
ggaagggcct gagtccgagc agaagaagaa ggg 33

<210> 247
<211> 33
<212> DNA
<213> Homo sapiens

<400> 247
cattggaggt gacatcgatg tcctccccat tgg 33

<210> 248
<211> 33
<212> DNA
<213> Homo sapiens

<400> 248
ggacatcgat gtcacctcca atgactaggg tgg 33

<210> 249
<211> 33
<212> DNA
<213> Homo sapiens

<400> 249
catcgatgtc ctccccattg gcctgcttcg tgg 33

<210> 250
<211> 33
<212> DNA
<213> Homo sapiens

<400> 250
ttcgtggcaa tgcgccaccg gttgatgtga tgg 33

<210> 251

<211> 33
<212> DNA
<213> Homo sapiens

<400> 251
tcgtggcaat gcgccaccgg ttgatgtgat ggg 33

<210> 252
<211> 33
<212> DNA
<213> Homo sapiens

<400> 252
tccagcttct gccgtttgta ctttgtcctc cgg 33

<210> 253
<211> 33
<212> DNA
<213> Homo sapiens

<400> 253
ggagggaggg gcacagatga gaaactcagg agg 33

<210> 254
<211> 33
<212> DNA
<213> Homo sapiens

<400> 254
aggggccgag attgggtgtt cagggcagag agg 33

<210> 255
<211> 33
<212> DNA
<213> Homo sapiens

<400> 255
atgcaggagg gtggcgagag gggccgagat tgg 33

<210> 256
<211> 33
<212> DNA
<213> Homo sapiens

<400> 256
ggtggcgaga ggggccgaga ttgggtgttc agg 33

<210> 257
<211> 33
<212> DNA
<213> Mus musculus

<400> 257
caagcactga gtgccattag ctaaatgcat agg 33

<210> 258

<211> 33
<212> DNA
<213> Mus musculus

<400> 258
aatgcatagg gtaccaccca caggtgccag ggg 33

<210> 259
<211> 33
<212> DNA
<213> Mus musculus

<400> 259
acacacatgg gaaagcctct gggccaggaa agg 33

<210> 260
<211> 37
<212> DNA
<213> Homo sapiens

<400> 260
ggaggaggta gtatacagaa acacagagaa gtagaat 37

<210> 261
<211> 37
<212> DNA
<213> Homo sapiens

<400> 261
agaatgtaga ggagtcacag aaactcagca ctagaaa 37

<210> 262
<211> 98
<212> DNA
<213> Artificial Sequence

<220>
<221> source
<223> /note="Description of Artificial Sequence: Synthetic oligonucleotide"

<400> 262
ggacgaaaca ccggaaccat tcaaaacagc atagcaagtt aaaataaggc tagtccgtta 60

tcaacttgaa aaagtggcac cgagtcggtg cttttttt 98

<210> 263
<211> 186
<212> DNA
<213> Artificial Sequence

<220>
<221> source
<223> /note="Description of Artificial Sequence: Synthetic polynucleotide"

<400> 263
ggacgaaaca ccggtagtat taagtattgt tttatggctg ataaatttct ttgaatttct 60

ccttgattat ttgttataaa agttataaaa taatcttgtt ggaaccattc aaaacagcat 120

agcaagttaa aataaggcta gtccgttatc aacttgaaaa agtggcaccg agtcggtgct 180

tttttt 186

<210> 264
<211> 95
<212> DNA
<213> Artificial Sequence

<220>
<221> source
<223> /note="Description of Artificial Sequence: Synthetic oligonucleotide"

<400> 264
gggttttaga gctatgctgt tttgaatggt cccaaaacgg gtcttcgaga agacgtttta 60

gagctatgct gttttgaatg gtcccaaaac ttttt 95

<210> 265
<211> 36
<212> DNA
<213> Artificial Sequence

<220>
<221> source
<223> /note="Description of Artificial Sequence: Synthetic oligonucleotide"

<220>
<221> modified_base
<222> (5)..(34)
<223> a, c, t, g, unknown or other

<400> 265
aaacnnnnnn nnnnnnnnnn nnnnnnnnnn nnnngt 36

<210> 266
<211> 36
<212> DNA
<213> Artificial Sequence

<220>
<221> source
<223> /note="Description of Artificial Sequence: Synthetic oligonucleotide"

<220>
<221> modified_base
<222> (7)..(36)
<223> a, c, t, g, unknown or other

<400> 266
taaaacnnnn nnnnnnnnnn nnnnnnnnnn nnnnnn 36

<210> 267
<211> 84
<212> DNA
<213> Artificial Sequence

<220>
<221> source
<223> /note="Description of Artificial Sequence: Synthetic oligonucleotide"

<400> 267
gtggaaagga cgaaacaccg ggtcttcgag aagacctgtt ttagagctag aaatagcaag 60

ttaaaataag gctagtccgt tttt 84

<210> 268
<211> 46
<212> RNA
<213> Artificial Sequence

<220>
<221> source
<223> /note="Description of Artificial Sequence: Synthetic oligonucleotide"

<220>
<221> modified_base
<222> (1)..(19)
<223> a, c, u, g, unknown or other

<400> 268
nnnnnnnnnn nnnnnnnnng uuauuguacu cucaagauuu auuuuu 46

<210> 269
<211> 91
<212> RNA
<213> Artificial Sequence

<220>
<221> source
<223> /note="Description of Artificial Sequence: Synthetic oligonucleotide"

<400> 269
guuacuuaaa ucuugcagaa gcuacaaaga uaaggcuuca ugccgaaauc aacacccugu 60

cauuuuaugg cagggugguuu ucguuauuua a 91

<210> 270
<211> 70
<212> DNA
<213> Homo sapiens

<400> 270
ttttctagtg ctgagtttct gtgactcctc tacattctac ttctctgtgt ttctgtatac 60

tacctcctcc 70

<210> 271
<211> 122
<212> DNA
<213> Homo sapiens

<400> 271
ggaggaaggg cctgagtccg agcagaagaa gaagggctcc catcacatca accggtggcg 60

cattgccacg aagcaggcca atggggagga catcgatgtc acctccaatg actagggtgg 120

gc 122

<210> 272
<211> 48
<212> RNA
<213> Artificial Sequence

<220>
<221> source
<223> /note="Description of Artificial Sequence: Synthetic oligonucleotide"

<220>
<221> modified_base
<222> (3)..(32)
<223> a, c, u, g, unknown or other

<400> 272
acnnnnnnnn nnnnnnnnnn nnnnnnnnnn nnguuuuaga gcuaugcu 48

<210> 273
<211> 67
<212> DNA
<213> Artificial Sequence

<220>
<221> source
<223> /note="Description of Artificial Sequence: Synthetic oligonucleotide"

<220>
<221> source
<223> /note="Description of Combined DNA/RNA Molecule: Synthetic oligonucleotide"

<400> 273
agcauagcaa guuaaaauaa ggctaguccg uuaucaacuu gaaaaagugg caccgagucg 60

gugcuuu 67

<210> 274
<211> 62
<212> RNA
<213> Artificial Sequence

<220>
<221> source
<223> /note="Description of Artificial Sequence: Synthetic

oligonucleotide"

<220>
<221> modified_base
<222> (1)..(20)
<223> a, c, u, g, unknown or other

<400> 274
nnnnnnnnnn nnnnnnnnnn guuuuagagc uagaaauagc aaguuaaaau aaggcuaguc 60

cg 62

<210> 275
<211> 73
<212> DNA
<213> Artificial Sequence

<220>
<221> source
<223> /note="Description of Artificial Sequence: Synthetic
oligonucleotide"

<400> 275
tgaatggtcc caaaacggaa gggcctgagt ccgagcagaa gaagaagttt tagagctatg 60

ctgttttgaa tgg 73

<210> 276
<211> 69
<212> DNA
<213> Homo sapiens

<400> 276
ctggtcttcc acctctctgc cctgaacacc caatctcggc ccctctcgcc accctcctgc 60

atttctgtt 69

<210> 277
<211> 138
<212> DNA
<213> Mus musculus

<400> 277
acccaagcac tgagtgccat tagctaaatg catagggtac cacccacagg tgccaggggc 60

ctttcccaaa gttcccagcc ccttctccaa cctttcctgg cccagaggct ttcccatgtg 120

tgtggctgga ccctttga 138

<210> 278
<211> 21
<212> DNA
<213> Artificial Sequence

<220>
<221> source
<223> /note="Description of Artificial Sequence: Synthetic
primer"

<400> 278
aaaaccaccc ttctctctgg c 21

<210> 279
<211> 21
<212> DNA
<213> Artificial Sequence

<220>
<221> source
<223> /note="Description of Artificial Sequence: Synthetic primer"

<400> 279
ggagattgga gacacggaga g 21

<210> 280
<211> 20
<212> DNA
<213> Artificial Sequence

<220>
<221> source
<223> /note="Description of Artificial Sequence: Synthetic primer"

<400> 280
ctggaaagcc aatgcctgac 20

<210> 281
<211> 20
<212> DNA
<213> Artificial Sequence

<220>
<221> source
<223> /note="Description of Artificial Sequence: Synthetic primer"

<400> 281
ggcagcaaac tccttgtcct 20

<210> 282
<211> 20
<212> DNA
<213> Artificial Sequence

<220>
<221> source
<223> /note="Description of Artificial Sequence: Synthetic primer"

<400> 282
gtgctttgca gaggcctacc 20

<210> 283
<211> 20

<212> DNA
<213> Artificial Sequence

<220>
<221> source
<223> /note="Description of Artificial Sequence: Synthetic primer"

<400> 283
cctggagcgc atgcagtagt 20

<210> 284
<211> 22
<212> DNA
<213> Artificial Sequence

<220>
<221> source
<223> /note="Description of Artificial Sequence: Synthetic primer"

<400> 284
accttctgtg tttccaccat tc 22

<210> 285
<211> 20
<212> DNA
<213> Artificial Sequence

<220>
<221> source
<223> /note="Description of Artificial Sequence: Synthetic primer"

<400> 285
ttggggagtg cacagacttc 20

<210> 286
<211> 30
<212> DNA
<213> Artificial Sequence

<220>
<221> source
<223> /note="Description of Artificial Sequence: Synthetic probe"

<400> 286
tagctctaaa acttcttctt ctgctcggac 30

<210> 287
<211> 30
<212> DNA
<213> Artificial Sequence

<220>
<221> source
<223> /note="Description of Artificial Sequence: Synthetic probe"

<400> 287
ctagccttat tttaacttgc tatgctgttt 30

<210> 288
<211> 15
<212> DNA
<213> Homo sapiens

<400> 288
cagaagaaga agggc 15

<210> 289
<211> 51
<212> DNA
<213> Homo sapiens

<400> 289
ccaatgggga ggacatcgat gtcacctcca atgactaggg tggtgggcaa c 51

<210> 290
<211> 15
<212> DNA
<213> Homo sapiens

<400> 290
ctctggccac tccct 15

<210> 291
<211> 52
<212> DNA
<213> Homo sapiens

<400> 291
acatcgatgt cacctccaat gacaagcttg ctagcggtgg gcaaccacaa ac 52

<210> 292
<211> 25
<212> DNA
<213> Artificial Sequence

<220>
<221> source
<223> /note="Description of Artificial Sequence: Synthetic oligonucleotide"

<220>
<221> modified_base
<222> (6)..(25)
<223> a, c, t, g, unknown or other

<400> 292
caccgnnnnn nnnnnnnnnn nnnnn 25

<210> 293
<211> 25

<212> DNA
<213> Artificial Sequence

<220>
<221> source
<223> /note="Description of Artificial Sequence: Synthetic oligonucleotide"

<220>
<221> modified_base
<222> (5)..(24)
<223> a, c, t, g, unknown or other

<400> 293
aaacnnnnnn nnnnnnnnnn nnnnc 25

<210> 294
<211> 54
<212> DNA
<213> Artificial Sequence

<220>
<221> source
<223> /note="Description of Artificial Sequence: Synthetic oligonucleotide"

<400> 294
aacaccgggt cttcgagaag acctgtttta gagctagaaa tagcaagtta aaat 54

<210> 295
<211> 54
<212> DNA
<213> Artificial Sequence

<220>
<221> source
<223> /note="Description of Artificial Sequence: Synthetic oligonucleotide"

<400> 295
caaaacgggt cttcgagaag acgttttaga gctatgctgt tttgaatggt ccca 54

<210> 296
<211> 4104
<212> DNA
<213> Homo sapiens

<220>
<221> CDS
<222> (1)..(4104)

<400> 296
```
atg gac aag aag tac agc atc ggc ctg gac atc ggc acc aac tct gtg       48
Met Asp Lys Lys Tyr Ser Ile Gly Leu Asp Ile Gly Thr Asn Ser Val
1               5                   10                  15

ggc tgg gcc gtg atc acc gac gag tac aag gtg ccc agc aag aaa ttc       96
Gly Trp Ala Val Ile Thr Asp Glu Tyr Lys Val Pro Ser Lys Lys Phe
```

```
            20                  25                  30

aag gtg ctg ggc aac acc gac cgg cac agc atc aag aag aac ctg atc       144
Lys Val Leu Gly Asn Thr Asp Arg His Ser Ile Lys Lys Asn Leu Ile
        35                  40                  45

gga gcc ctg ctg ttc gac agc ggc gaa aca gcc gag gcc acc cgg ctg       192
Gly Ala Leu Leu Phe Asp Ser Gly Glu Thr Ala Glu Ala Thr Arg Leu
    50                  55                  60

aag aga acc gcc aga aga aga tac acc aga cgg aag aac cgg atc tgc       240
Lys Arg Thr Ala Arg Arg Arg Tyr Thr Arg Arg Lys Asn Arg Ile Cys
65                  70                  75                  80

tat ctg caa gag atc ttc agc aac gag atg gcc aag gtg gac gac agc       288
Tyr Leu Gln Glu Ile Phe Ser Asn Glu Met Ala Lys Val Asp Asp Ser
                85                  90                  95

ttc ttc cac aga ctg gaa gag tcc ttc ctg gtg gaa gag gat aag aag       336
Phe Phe His Arg Leu Glu Glu Ser Phe Leu Val Glu Glu Asp Lys Lys
            100                 105                 110

cac gag cgg cac ccc atc ttc ggc aac atc gtg gac gag gtg gcc tac       384
His Glu Arg His Pro Ile Phe Gly Asn Ile Val Asp Glu Val Ala Tyr
        115                 120                 125

cac gag aag tac ccc acc atc tac cac ctg aga aag aaa ctg gtg gac       432
His Glu Lys Tyr Pro Thr Ile Tyr His Leu Arg Lys Lys Leu Val Asp
    130                 135                 140

agc acc gac aag gcc gac ctg cgg ctg atc tat ctg gcc ctg gcc cac       480
Ser Thr Asp Lys Ala Asp Leu Arg Leu Ile Tyr Leu Ala Leu Ala His
145                 150                 155                 160

atg atc aag ttc cgg ggc cac ttc ctg atc gag ggc gac ctg aac ccc       528
Met Ile Lys Phe Arg Gly His Phe Leu Ile Glu Gly Asp Leu Asn Pro
                165                 170                 175

gac aac agc gac gtg gac aag ctg ttc atc cag ctg gtg cag acc tac       576
Asp Asn Ser Asp Val Asp Lys Leu Phe Ile Gln Leu Val Gln Thr Tyr
            180                 185                 190

aac cag ctg ttc gag gaa aac ccc atc aac gcc agc ggc gtg gac gcc       624
Asn Gln Leu Phe Glu Glu Asn Pro Ile Asn Ala Ser Gly Val Asp Ala
        195                 200                 205

aag gcc atc ctg tct gcc aga ctg agc aag agc aga cgg ctg gaa aat       672
Lys Ala Ile Leu Ser Ala Arg Leu Ser Lys Ser Arg Arg Leu Glu Asn
    210                 215                 220

ctg atc gcc cag ctg ccc ggc gag aag aag aat ggc ctg ttc ggc aac       720
Leu Ile Ala Gln Leu Pro Gly Glu Lys Lys Asn Gly Leu Phe Gly Asn
225                 230                 235                 240

ctg att gcc ctg agc ctg ggc ctg acc ccc aac ttc aag agc aac ttc       768
Leu Ile Ala Leu Ser Leu Gly Leu Thr Pro Asn Phe Lys Ser Asn Phe
                245                 250                 255

gac ctg gcc gag gat gcc aaa ctg cag ctg agc aag gac acc tac gac       816
Asp Leu Ala Glu Asp Ala Lys Leu Gln Leu Ser Lys Asp Thr Tyr Asp
            260                 265                 270

gac gac ctg gac aac ctg ctg gcc cag atc ggc gac cag tac gcc gac       864
```

```
Asp Asp Leu Asp Asn Leu Leu Ala Gln Ile Gly Asp Gln Tyr Ala Asp
        275                 280                 285

ctg ttt ctg gcc gcc aag aac ctg tcc gac gcc atc ctg ctg agc gac       912
Leu Phe Leu Ala Ala Lys Asn Leu Ser Asp Ala Ile Leu Leu Ser Asp
    290                 295                 300

atc ctg aga gtg aac acc gag atc acc aag gcc ccc ctg agc gcc tct       960
Ile Leu Arg Val Asn Thr Glu Ile Thr Lys Ala Pro Leu Ser Ala Ser
305                 310                 315                 320

atg atc aag aga tac gac gag cac cac cag gac ctg acc ctg ctg aaa      1008
Met Ile Lys Arg Tyr Asp Glu His His Gln Asp Leu Thr Leu Leu Lys
                325                 330                 335

gct ctc gtg cgg cag cag ctg cct gag aag tac aaa gag att ttc ttc      1056
Ala Leu Val Arg Gln Gln Leu Pro Glu Lys Tyr Lys Glu Ile Phe Phe
            340                 345                 350

gac cag agc aag aac ggc tac gcc ggc tac att gac ggc gga gcc agc      1104
Asp Gln Ser Lys Asn Gly Tyr Ala Gly Tyr Ile Asp Gly Gly Ala Ser
        355                 360                 365

cag gaa gag ttc tac aag ttc atc aag ccc atc ctg gaa aag atg gac      1152
Gln Glu Glu Phe Tyr Lys Phe Ile Lys Pro Ile Leu Glu Lys Met Asp
    370                 375                 380

ggc acc gag gaa ctg ctc gtg aag ctg aac aga gag gac ctg ctg cgg      1200
Gly Thr Glu Glu Leu Leu Val Lys Leu Asn Arg Glu Asp Leu Leu Arg
385                 390                 395                 400

aag cag cgg acc ttc gac aac ggc agc atc ccc cac cag atc cac ctg      1248
Lys Gln Arg Thr Phe Asp Asn Gly Ser Ile Pro His Gln Ile His Leu
                405                 410                 415

gga gag ctg cac gcc att ctg cgg cgg cag gaa gat ttt tac cca ttc      1296
Gly Glu Leu His Ala Ile Leu Arg Arg Gln Glu Asp Phe Tyr Pro Phe
            420                 425                 430

ctg aag gac aac cgg gaa aag atc gag aag atc ctg acc ttc cgc atc      1344
Leu Lys Asp Asn Arg Glu Lys Ile Glu Lys Ile Leu Thr Phe Arg Ile
        435                 440                 445

ccc tac tac gtg ggc cct ctg gcc agg gga aac agc aga ttc gcc tgg      1392
Pro Tyr Tyr Val Gly Pro Leu Ala Arg Gly Asn Ser Arg Phe Ala Trp
    450                 455                 460

atg acc aga aag agc gag gaa acc atc acc ccc tgg aac ttc gag gaa      1440
Met Thr Arg Lys Ser Glu Glu Thr Ile Thr Pro Trp Asn Phe Glu Glu
465                 470                 475                 480

gtg gtg gac aag ggc gct tcc gcc cag agc ttc atc gag cgg atg acc      1488
Val Val Asp Lys Gly Ala Ser Ala Gln Ser Phe Ile Glu Arg Met Thr
                485                 490                 495

aac ttc gat aag aac ctg ccc aac gag aag gtg ctg ccc aag cac agc      1536
Asn Phe Asp Lys Asn Leu Pro Asn Glu Lys Val Leu Pro Lys His Ser
            500                 505                 510

ctg ctg tac gag tac ttc acc gtg tat aac gag ctg acc aaa gtg aaa      1584
Leu Leu Tyr Glu Tyr Phe Thr Val Tyr Asn Glu Leu Thr Lys Val Lys
        515                 520                 525
```

```
tac gtg acc gag gga atg aga aag ccc gcc ttc ctg agc ggc gag cag        1632
Tyr Val Thr Glu Gly Met Arg Lys Pro Ala Phe Leu Ser Gly Glu Gln
    530                 535                 540

aaa aag gcc atc gtg gac ctg ctg ttc aag acc aac cgg aaa gtg acc        1680
Lys Lys Ala Ile Val Asp Leu Leu Phe Lys Thr Asn Arg Lys Val Thr
545                 550                 555                 560

gtg aag cag ctg aaa gag gac tac ttc aag aaa atc gag tgc ttc gac        1728
Val Lys Gln Leu Lys Glu Asp Tyr Phe Lys Lys Ile Glu Cys Phe Asp
                565                 570                 575

tcc gtg gaa atc tcc ggc gtg gaa gat cgg ttc aac gcc tcc ctg ggc        1776
Ser Val Glu Ile Ser Gly Val Glu Asp Arg Phe Asn Ala Ser Leu Gly
            580                 585                 590

aca tac cac gat ctg ctg aaa att atc aag gac aag gac ttc ctg gac        1824
Thr Tyr His Asp Leu Leu Lys Ile Ile Lys Asp Lys Asp Phe Leu Asp
        595                 600                 605

aat gag gaa aac gag gac att ctg gaa gat atc gtg ctg acc ctg aca        1872
Asn Glu Glu Asn Glu Asp Ile Leu Glu Asp Ile Val Leu Thr Leu Thr
    610                 615                 620

ctg ttt gag gac aga gag atg atc gag gaa cgg ctg aaa acc tat gcc        1920
Leu Phe Glu Asp Arg Glu Met Ile Glu Glu Arg Leu Lys Thr Tyr Ala
625                 630                 635                 640

cac ctg ttc gac gac aaa gtg atg aag cag ctg aag cgg cgg aga tac        1968
His Leu Phe Asp Asp Lys Val Met Lys Gln Leu Lys Arg Arg Arg Tyr
                645                 650                 655

acc ggc tgg ggc agg ctg agc cgg aag ctg atc aac ggc atc cgg gac        2016
Thr Gly Trp Gly Arg Leu Ser Arg Lys Leu Ile Asn Gly Ile Arg Asp
            660                 665                 670

aag cag tcc ggc aag aca atc ctg gat ttc ctg aag tcc gac ggc ttc        2064
Lys Gln Ser Gly Lys Thr Ile Leu Asp Phe Leu Lys Ser Asp Gly Phe
        675                 680                 685

gcc aac aga aac ttc atg cag ctg atc cac gac gac agc ctg acc ttt        2112
Ala Asn Arg Asn Phe Met Gln Leu Ile His Asp Asp Ser Leu Thr Phe
    690                 695                 700

aaa gag gac atc cag aaa gcc cag gtg tcc ggc cag ggc gat agc ctg        2160
Lys Glu Asp Ile Gln Lys Ala Gln Val Ser Gly Gln Gly Asp Ser Leu
705                 710                 715                 720

cac gag cac att gcc aat ctg gcc ggc agc ccc gcc att aag aag ggc        2208
His Glu His Ile Ala Asn Leu Ala Gly Ser Pro Ala Ile Lys Lys Gly
                725                 730                 735

atc ctg cag aca gtg aag gtg gtg gac gag ctc gtg aaa gtg atg ggc        2256
Ile Leu Gln Thr Val Lys Val Val Asp Glu Leu Val Lys Val Met Gly
            740                 745                 750

cgg cac aag ccc gag aac atc gtg atc gcc atg gcc aga gag aac cag        2304
Arg His Lys Pro Glu Asn Ile Val Ile Ala Met Ala Arg Glu Asn Gln
        755                 760                 765

acc acc cag aag gga cag aag aac agc cgc gag aga atg aag cgg atc        2352
Thr Thr Gln Lys Gly Gln Lys Asn Ser Arg Glu Arg Met Lys Arg Ile
    770                 775                 780
```

```
gaa gag ggc atc aaa gag ctg ggc agc cag atc ctg aaa gaa cac ccc      2400
Glu Glu Gly Ile Lys Glu Leu Gly Ser Gln Ile Leu Lys Glu His Pro
785                 790                 795                 800

gtg gaa aac acc cag ctg cag aac gag aag ctg tac ctg tac tac ctg      2448
Val Glu Asn Thr Gln Leu Gln Asn Glu Lys Leu Tyr Leu Tyr Tyr Leu
                805                 810                 815

cag aat ggg cgg gat atg tac gtg gac cag gaa ctg gac atc aac cgg      2496
Gln Asn Gly Arg Asp Met Tyr Val Asp Gln Glu Leu Asp Ile Asn Arg
            820                 825                 830

ctg tcc gac tac gat gtg gac gcc atc gtg cct cag agc ttt ctg aag      2544
Leu Ser Asp Tyr Asp Val Asp Ala Ile Val Pro Gln Ser Phe Leu Lys
        835                 840                 845

gac gac tcc atc gac gcc aag gtg ctg acc aga agc gac aag gcc cgg      2592
Asp Asp Ser Ile Asp Ala Lys Val Leu Thr Arg Ser Asp Lys Ala Arg
    850                 855                 860

ggc aag agc gac aac gtg ccc tcc gaa gag gtc gtg aag aag atg aag      2640
Gly Lys Ser Asp Asn Val Pro Ser Glu Glu Val Val Lys Lys Met Lys
865                 870                 875                 880

aac tac tgg cgg cag ctg ctg aac gcc aag ctg att acc cag aga aag      2688
Asn Tyr Trp Arg Gln Leu Leu Asn Ala Lys Leu Ile Thr Gln Arg Lys
                885                 890                 895

ttc gac aat ctg acc aag gcc gag aga ggc ggc ctg agc gaa ctg gat      2736
Phe Asp Asn Leu Thr Lys Ala Glu Arg Gly Gly Leu Ser Glu Leu Asp
            900                 905                 910

aag gcc ggc ttc atc aag aga cag ctg gtg gaa acc cgg cag atc aca      2784
Lys Ala Gly Phe Ile Lys Arg Gln Leu Val Glu Thr Arg Gln Ile Thr
        915                 920                 925

aag cac gtg gca cag atc ctg gac tcc cgg atg aac act aag tac gac      2832
Lys His Val Ala Gln Ile Leu Asp Ser Arg Met Asn Thr Lys Tyr Asp
    930                 935                 940

gag aat gac aag ctg atc cgg gaa gtg aaa gtg atc acc ctg aag tcc      2880
Glu Asn Asp Lys Leu Ile Arg Glu Val Lys Val Ile Thr Leu Lys Ser
945                 950                 955                 960

aag ctg gtg tcc gat ttc cgg aag gat ttc cag ttt tac aaa gtg cgc      2928
Lys Leu Val Ser Asp Phe Arg Lys Asp Phe Gln Phe Tyr Lys Val Arg
                965                 970                 975

gag atc aac aac tac cac cac gcc cac gcc gcc tac ctg aac gcc gtc      2976
Glu Ile Asn Asn Tyr His His Ala His Ala Ala Tyr Leu Asn Ala Val
            980                 985                 990

gtg gga acc gcc ctg atc aaa aag  tac cct aag ctg gaa  agc gag ttc    3024
Val Gly Thr Ala Leu Ile Lys Lys  Tyr Pro Lys Leu Glu  Ser Glu Phe
        995                 1000                 1005

gtg tac  ggc gac tac aag gtg  tac gac gtg cgg aag  atg atc gcc      3069
Val Tyr  Gly Asp Tyr Lys Val  Tyr Asp Val Arg Lys  Met Ile Ala
    1010                 1015                 1020

aag agc  gag cag gaa atc ggc  aag gct acc gcc aag  tac ttc ttc      3114
Lys Ser  Glu Gln Glu Ile Gly  Lys Ala Thr Ala Lys  Tyr Phe Phe
```

```
    1025                1030                1035

tac agc  aac atc atg aac ttt  ttc aag acc gag att  acc ctg gcc        3159
Tyr Ser  Asn Ile Met Asn Phe  Phe Lys Thr Glu Ile  Thr Leu Ala
    1040                1045                1050

aac ggc  gag atc cgg aag cgg  cct ctg atc gag aca  aac ggc gaa        3204
Asn Gly  Glu Ile Arg Lys Arg  Pro Leu Ile Glu Thr  Asn Gly Glu
    1055                1060                1065

acc ggg  gag atc gtg tgg gat  aag ggc cgg gat ttt  gcc acc gtg        3249
Thr Gly  Glu Ile Val Trp Asp  Lys Gly Arg Asp Phe  Ala Thr Val
    1070                1075                1080

cgg aaa  gtg ctg agc atg ccc  caa gtg aat atc gtg  aaa aag acc        3294
Arg Lys  Val Leu Ser Met Pro  Gln Val Asn Ile Val  Lys Lys Thr
    1085                1090                1095

gag gtg  cag aca ggc ggc ttc  agc aaa gag tct atc  ctg ccc aag        3339
Glu Val  Gln Thr Gly Gly Phe  Ser Lys Glu Ser Ile  Leu Pro Lys
    1100                1105                1110

agg aac  agc gat aag ctg atc  gcc aga aag aag gac  tgg gac cct        3384
Arg Asn  Ser Asp Lys Leu Ile  Ala Arg Lys Lys Asp  Trp Asp Pro
    1115                1120                1125

aag aag  tac ggc ggc ttc gac  agc ccc acc gtg gcc  tat tct gtg        3429
Lys Lys  Tyr Gly Gly Phe Asp  Ser Pro Thr Val Ala  Tyr Ser Val
    1130                1135                1140

ctg gtg  gtg gcc aaa gtg gaa  aag ggc aag tcc aag  aaa ctg aag        3474
Leu Val  Val Ala Lys Val Glu  Lys Gly Lys Ser Lys  Lys Leu Lys
    1145                1150                1155

agt gtg  aaa gag ctg ctg ggg  atc acc atc atg gaa  aga agc agc        3519
Ser Val  Lys Glu Leu Leu Gly  Ile Thr Ile Met Glu  Arg Ser Ser
    1160                1165                1170

ttc gag  aag aat ccc atc gac  ttt ctg gaa gcc aag  ggc tac aaa        3564
Phe Glu  Lys Asn Pro Ile Asp  Phe Leu Glu Ala Lys  Gly Tyr Lys
    1175                1180                1185

gaa gtg  aaa aag gac ctg atc  atc aag ctg cct aag  tac tcc ctg        3609
Glu Val  Lys Lys Asp Leu Ile  Ile Lys Leu Pro Lys  Tyr Ser Leu
    1190                1195                1200

ttc gag  ctg gaa aac ggc cgg  aag aga atg ctg gcc  tct gcc ggc        3654
Phe Glu  Leu Glu Asn Gly Arg  Lys Arg Met Leu Ala  Ser Ala Gly
    1205                1210                1215

gaa ctg  cag aag gga aac gaa  ctg gcc ctg ccc tcc  aaa tat gtg        3699
Glu Leu  Gln Lys Gly Asn Glu  Leu Ala Leu Pro Ser  Lys Tyr Val
    1220                1225                1230

aac ttc  ctg tac ctg gcc agc  cac tat gag aag ctg  aag ggc tcc        3744
Asn Phe  Leu Tyr Leu Ala Ser  His Tyr Glu Lys Leu  Lys Gly Ser
    1235                1240                1245

ccc gag  gat aat gag cag aaa  cag ctg ttt gtg gaa  cag cac aag        3789
Pro Glu  Asp Asn Glu Gln Lys  Gln Leu Phe Val Glu  Gln His Lys
    1250                1255                1260

cac tac  ctg gac gag atc atc  gag cag atc agc gag  ttc tcc aag        3834
```

His Tyr Leu Asp Glu Ile Ile Glu Gln Ile Ser Glu Phe Ser Lys
1265 1270 1275

aga gtg atc ctg gcc gac gct aat ctg gac aaa gtg ctg tcc gcc 3879
Arg Val Ile Leu Ala Asp Ala Asn Leu Asp Lys Val Leu Ser Ala
1280 1285 1290

tac aac aag cac cgg gat aag ccc atc aga gag cag gcc gag aat 3924
Tyr Asn Lys His Arg Asp Lys Pro Ile Arg Glu Gln Ala Glu Asn
1295 1300 1305

atc atc cac ctg ttt acc ctg acc aat ctg gga gcc cct gcc gcc 3969
Ile Ile His Leu Phe Thr Leu Thr Asn Leu Gly Ala Pro Ala Ala
1310 1315 1320

ttc aag tac ttt gac acc acc atc gac cgg aag agg tac acc agc 4014
Phe Lys Tyr Phe Asp Thr Thr Ile Asp Arg Lys Arg Tyr Thr Ser
1325 1330 1335

acc aaa gag gtg ctg gac gcc acc ctg atc cac cag agc atc acc 4059
Thr Lys Glu Val Leu Asp Ala Thr Leu Ile His Gln Ser Ile Thr
1340 1345 1350

ggc ctg tac gag aca cgg atc gac ctg tct cag ctg gga ggc gac 4104
Gly Leu Tyr Glu Thr Arg Ile Asp Leu Ser Gln Leu Gly Gly Asp
1355 1360 1365

<210> 297
<211> 1368
<212> PRT
<213> Homo sapiens

<400> 297
Met Asp Lys Lys Tyr Ser Ile Gly Leu Asp Ile Gly Thr Asn Ser Val
1 5 10 15

Gly Trp Ala Val Ile Thr Asp Glu Tyr Lys Val Pro Ser Lys Lys Phe
20 25 30

Lys Val Leu Gly Asn Thr Asp Arg His Ser Ile Lys Lys Asn Leu Ile
35 40 45

Gly Ala Leu Leu Phe Asp Ser Gly Glu Thr Ala Glu Ala Thr Arg Leu
50 55 60

Lys Arg Thr Ala Arg Arg Arg Tyr Thr Arg Arg Lys Asn Arg Ile Cys
65 70 75 80

Tyr Leu Gln Glu Ile Phe Ser Asn Glu Met Ala Lys Val Asp Asp Ser
85 90 95

Phe Phe His Arg Leu Glu Glu Ser Phe Leu Val Glu Glu Asp Lys Lys
100 105 110

His Glu Arg His Pro Ile Phe Gly Asn Ile Val Asp Glu Val Ala Tyr

115 120 125

His Glu Lys Tyr Pro Thr Ile Tyr His Leu Arg Lys Lys Leu Val Asp
130 135 140

Ser Thr Asp Lys Ala Asp Leu Arg Leu Ile Tyr Leu Ala Leu Ala His
145 150 155 160

Met Ile Lys Phe Arg Gly His Phe Leu Ile Glu Gly Asp Leu Asn Pro
165 170 175

Asp Asn Ser Asp Val Asp Lys Leu Phe Ile Gln Leu Val Gln Thr Tyr
180 185 190

Asn Gln Leu Phe Glu Glu Asn Pro Ile Asn Ala Ser Gly Val Asp Ala
195 200 205

Lys Ala Ile Leu Ser Ala Arg Leu Ser Lys Ser Arg Arg Leu Glu Asn
210 215 220

Leu Ile Ala Gln Leu Pro Gly Glu Lys Lys Asn Gly Leu Phe Gly Asn
225 230 235 240

Leu Ile Ala Leu Ser Leu Gly Leu Thr Pro Asn Phe Lys Ser Asn Phe
245 250 255

Asp Leu Ala Glu Asp Ala Lys Leu Gln Leu Ser Lys Asp Thr Tyr Asp
260 265 270

Asp Asp Leu Asp Asn Leu Leu Ala Gln Ile Gly Asp Gln Tyr Ala Asp
275 280 285

Leu Phe Leu Ala Ala Lys Asn Leu Ser Asp Ala Ile Leu Leu Ser Asp
290 295 300

Ile Leu Arg Val Asn Thr Glu Ile Thr Lys Ala Pro Leu Ser Ala Ser
305 310 315 320

Met Ile Lys Arg Tyr Asp Glu His His Gln Asp Leu Thr Leu Leu Lys
325 330 335

Ala Leu Val Arg Gln Gln Leu Pro Glu Lys Tyr Lys Glu Ile Phe Phe
340 345 350

Asp Gln Ser Lys Asn Gly Tyr Ala Gly Tyr Ile Asp Gly Gly Ala Ser
355 360 365

```
Gln Glu Glu Phe Tyr Lys Phe Ile Lys Pro Ile Leu Glu Lys Met Asp
    370                 375                 380

Gly Thr Glu Glu Leu Leu Val Lys Leu Asn Arg Glu Asp Leu Leu Arg
385                 390                 395                 400

Lys Gln Arg Thr Phe Asp Asn Gly Ser Ile Pro His Gln Ile His Leu
                405                 410                 415

Gly Glu Leu His Ala Ile Leu Arg Arg Gln Glu Asp Phe Tyr Pro Phe
            420                 425                 430

Leu Lys Asp Asn Arg Glu Lys Ile Glu Lys Ile Leu Thr Phe Arg Ile
        435                 440                 445

Pro Tyr Tyr Val Gly Pro Leu Ala Arg Gly Asn Ser Arg Phe Ala Trp
    450                 455                 460

Met Thr Arg Lys Ser Glu Glu Thr Ile Thr Pro Trp Asn Phe Glu Glu
465                 470                 475                 480

Val Val Asp Lys Gly Ala Ser Ala Gln Ser Phe Ile Glu Arg Met Thr
                485                 490                 495

Asn Phe Asp Lys Asn Leu Pro Asn Glu Lys Val Leu Pro Lys His Ser
            500                 505                 510

Leu Leu Tyr Glu Tyr Phe Thr Val Tyr Asn Glu Leu Thr Lys Val Lys
        515                 520                 525

Tyr Val Thr Glu Gly Met Arg Lys Pro Ala Phe Leu Ser Gly Glu Gln
    530                 535                 540

Lys Lys Ala Ile Val Asp Leu Leu Phe Lys Thr Asn Arg Lys Val Thr
545                 550                 555                 560

Val Lys Gln Leu Lys Glu Asp Tyr Phe Lys Lys Ile Glu Cys Phe Asp
                565                 570                 575

Ser Val Glu Ile Ser Gly Val Glu Asp Arg Phe Asn Ala Ser Leu Gly
            580                 585                 590

Thr Tyr His Asp Leu Leu Lys Ile Ile Lys Asp Lys Asp Phe Leu Asp
        595                 600                 605

Asn Glu Glu Asn Glu Asp Ile Leu Glu Asp Ile Val Leu Thr Leu Thr
    610                 615                 620
```

Leu Phe Glu Asp Arg Glu Met Ile Glu Glu Arg Leu Lys Thr Tyr Ala
625 630 635 640

His Leu Phe Asp Asp Lys Val Met Lys Gln Leu Lys Arg Arg Arg Tyr
645 650 655

Thr Gly Trp Gly Arg Leu Ser Arg Lys Leu Ile Asn Gly Ile Arg Asp
660 665 670

Lys Gln Ser Gly Lys Thr Ile Leu Asp Phe Leu Lys Ser Asp Gly Phe
675 680 685

Ala Asn Arg Asn Phe Met Gln Leu Ile His Asp Asp Ser Leu Thr Phe
690 695 700

Lys Glu Asp Ile Gln Lys Ala Gln Val Ser Gly Gln Gly Asp Ser Leu
705 710 715 720

His Glu His Ile Ala Asn Leu Ala Gly Ser Pro Ala Ile Lys Lys Gly
725 730 735

Ile Leu Gln Thr Val Lys Val Val Asp Glu Leu Val Lys Val Met Gly
740 745 750

Arg His Lys Pro Glu Asn Ile Val Ile Ala Met Ala Arg Glu Asn Gln
755 760 765

Thr Thr Gln Lys Gly Gln Lys Asn Ser Arg Glu Arg Met Lys Arg Ile
770 775 780

Glu Glu Gly Ile Lys Glu Leu Gly Ser Gln Ile Leu Lys Glu His Pro
785 790 795 800

Val Glu Asn Thr Gln Leu Gln Asn Glu Lys Leu Tyr Leu Tyr Tyr Leu
805 810 815

Gln Asn Gly Arg Asp Met Tyr Val Asp Gln Glu Leu Asp Ile Asn Arg
820 825 830

Leu Ser Asp Tyr Asp Val Asp Ala Ile Val Pro Gln Ser Phe Leu Lys
835 840 845

Asp Asp Ser Ile Asp Ala Lys Val Leu Thr Arg Ser Asp Lys Ala Arg
850 855 860

Gly Lys Ser Asp Asn Val Pro Ser Glu Glu Val Val Lys Lys Met Lys
865 870 875 880

Asn Tyr Trp Arg Gln Leu Leu Asn Ala Lys Leu Ile Thr Gln Arg Lys
885 890 895

Phe Asp Asn Leu Thr Lys Ala Glu Arg Gly Gly Leu Ser Glu Leu Asp
900 905 910

Lys Ala Gly Phe Ile Lys Arg Gln Leu Val Glu Thr Arg Gln Ile Thr
915 920 925

Lys His Val Ala Gln Ile Leu Asp Ser Arg Met Asn Thr Lys Tyr Asp
930 935 940

Glu Asn Asp Lys Leu Ile Arg Glu Val Lys Val Ile Thr Leu Lys Ser
945 950 955 960

Lys Leu Val Ser Asp Phe Arg Lys Asp Phe Gln Phe Tyr Lys Val Arg
965 970 975

Glu Ile Asn Asn Tyr His His Ala His Ala Ala Tyr Leu Asn Ala Val
980 985 990

Val Gly Thr Ala Leu Ile Lys Lys Tyr Pro Lys Leu Glu Ser Glu Phe
995 1000 1005

Val Tyr Gly Asp Tyr Lys Val Tyr Asp Val Arg Lys Met Ile Ala
1010 1015 1020

Lys Ser Glu Gln Glu Ile Gly Lys Ala Thr Ala Lys Tyr Phe Phe
1025 1030 1035

Tyr Ser Asn Ile Met Asn Phe Phe Lys Thr Glu Ile Thr Leu Ala
1040 1045 1050

Asn Gly Glu Ile Arg Lys Arg Pro Leu Ile Glu Thr Asn Gly Glu
1055 1060 1065

Thr Gly Glu Ile Val Trp Asp Lys Gly Arg Asp Phe Ala Thr Val
1070 1075 1080

Arg Lys Val Leu Ser Met Pro Gln Val Asn Ile Val Lys Lys Thr
1085 1090 1095

Glu Val Gln Thr Gly Gly Phe Ser Lys Glu Ser Ile Leu Pro Lys
1100 1105 1110

Arg Asn Ser Asp Lys Leu Ile Ala Arg Lys Lys Asp Trp Asp Pro

1115 1120 1125

Lys Lys Tyr Gly Gly Phe Asp Ser Pro Thr Val Ala Tyr Ser Val
1130 1135 1140

Leu Val Val Ala Lys Val Glu Lys Gly Lys Ser Lys Lys Leu Lys
1145 1150 1155

Ser Val Lys Glu Leu Leu Gly Ile Thr Ile Met Glu Arg Ser Ser
1160 1165 1170

Phe Glu Lys Asn Pro Ile Asp Phe Leu Glu Ala Lys Gly Tyr Lys
1175 1180 1185

Glu Val Lys Lys Asp Leu Ile Ile Lys Leu Pro Lys Tyr Ser Leu
1190 1195 1200

Phe Glu Leu Glu Asn Gly Arg Lys Arg Met Leu Ala Ser Ala Gly
1205 1210 1215

Glu Leu Gln Lys Gly Asn Glu Leu Ala Leu Pro Ser Lys Tyr Val
1220 1225 1230

Asn Phe Leu Tyr Leu Ala Ser His Tyr Glu Lys Leu Lys Gly Ser
1235 1240 1245

Pro Glu Asp Asn Glu Gln Lys Gln Leu Phe Val Glu Gln His Lys
1250 1255 1260

His Tyr Leu Asp Glu Ile Ile Glu Gln Ile Ser Glu Phe Ser Lys
1265 1270 1275

Arg Val Ile Leu Ala Asp Ala Asn Leu Asp Lys Val Leu Ser Ala
1280 1285 1290

Tyr Asn Lys His Arg Asp Lys Pro Ile Arg Glu Gln Ala Glu Asn
1295 1300 1305

Ile Ile His Leu Phe Thr Leu Thr Asn Leu Gly Ala Pro Ala Ala
1310 1315 1320

Phe Lys Tyr Phe Asp Thr Thr Ile Asp Arg Lys Arg Tyr Thr Ser
1325 1330 1335

Thr Lys Glu Val Leu Asp Ala Thr Leu Ile His Gln Ser Ile Thr
1340 1345 1350

Gly Leu Tyr Glu Thr Arg Ile Asp Leu Ser Gln Leu Gly Gly Asp
1355 1360 1365

<210> 298
<211> 57
<212> DNA
<213> Homo sapiens

<220>
<221> CDS
<222> (1)..(57)

<400> 298
ggc acc att aaa gaa aat atc att ggt gtt tcc tat gat gaa tat aga 48
Gly Thr Ile Lys Glu Asn Ile Ile Gly Val Ser Tyr Asp Glu Tyr Arg
1 5 10 15

tac aga agc 57
Tyr Arg Ser

<210> 299
<211> 19
<212> PRT
<213> Homo sapiens

<400> 299
Gly Thr Ile Lys Glu Asn Ile Ile Gly Val Ser Tyr Asp Glu Tyr Arg
1 5 10 15

Tyr Arg Ser

<210> 300
<211> 99
<212> RNA
<213> Artificial Sequence

<220>
<221> source
<223> /note="Description of Artificial Sequence: Synthetic oligonucleotide"

<220>
<221> modified_base
<222> (1)..(20)
<223> a, c, u, g, unknown or other

<400> 300
nnnnnnnnnn nnnnnnnnnn guuuuagagc uagaaauagc aaguuaaaau aaggcuaguc 60

cguuaucaac uugaaaaagu ggcaccgagu cggugcuuu 99

<210> 301
<211> 48
<212> DNA

<213> Artificial Sequence

<220>
<221> source
<223> /note="Description of Artificial Sequence: Synthetic oligonucleotide"

<220>
<221> CDS
<222> (1)..(48)

<400> 301
att aaa gaa aat atc att ggc ttt gtt tcc tat gat gaa tat aga tac 48
Ile Lys Glu Asn Ile Ile Gly Phe Val Ser Tyr Asp Glu Tyr Arg Tyr
1 5 10 15

<210> 302
<211> 16
<212> PRT
<213> Artificial Sequence

<220>
<221> source
<223> /note="Description of Artificial Sequence: Synthetic peptide"

<400> 302
Ile Lys Glu Asn Ile Ile Gly Phe Val Ser Tyr Asp Glu Tyr Arg Tyr
1 5 10 15

<210> 303
<211> 27
<212> DNA
<213> Artificial Sequence

<220>
<221> source
<223> /note="Description of Artificial Sequence: Synthetic oligonucleotide"

<400> 303
ccccttccgg ttcagggacc ccgacct 27

<210> 304
<211> 26
<212> DNA
<213> Artificial Sequence

<220>
<221> source
<223> /note="Description of Artificial Sequence: Synthetic oligonucleotide"

<400> 304
ccccttccgt tcagggaccc cgacct 26

<210> 305
<211> 21

<212> DNA
<213> Artificial Sequence

<220>
<221> source
<223> /note="Description of Artificial Sequence: Synthetic oligonucleotide"

<400> 305
ccccttccgg gaccccgacc t 21

<210> 306
<211> 28
<212> DNA
<213> Artificial Sequence

<220>
<221> source
<223> /note="Description of Artificial Sequence: Synthetic oligonucleotide"

<400> 306
ccccttccgg gttcagggac cccgacct 28

<210> 307
<211> 29
<212> DNA
<213> Artificial Sequence

<220>
<221> source
<223> /note="Description of Artificial Sequence: Synthetic oligonucleotide"

<400> 307
ccccttccgg ggttcaggga ccccgacct 29

<210> 308
<211> 24
<212> DNA
<213> Artificial Sequence

<220>
<221> source
<223> /note="Description of Artificial Sequence: Synthetic oligonucleotide"

<400> 308
gcttacctgg gctgtagaac aggc 24

<210> 309
<211> 20
<212> DNA
<213> Artificial Sequence

<220>
<221> source
<223> /note="Description of Artificial Sequence: Synthetic oligonucleotide"

<400> 309
gcttacctgg gctaacaggc 20

<210> 310
<211> 22
<212> DNA
<213> Artificial Sequence

<220>
<221> source
<223> /note="Description of Artificial Sequence: Synthetic oligonucleotide"

<400> 310
gcttacctgg gctgtaacag gc 22

<210> 311
<211> 25
<212> DNA
<213> Artificial Sequence

<220>
<221> source
<223> /note="Description of Artificial Sequence: Synthetic oligonucleotide"

<400> 311
gcttacctgg gctgtaggaa caggc 25

<210> 312
<211> 26
<212> DNA
<213> Artificial Sequence

<220>
<221> source
<223> /note="Description of Artificial Sequence: Synthetic oligonucleotide"

<400> 312
gcttacctgg gctgtaggca acaggc 26

<210> 313
<211> 24
<212> DNA
<213> Artificial Sequence

<220>
<221> source
<223> /note="Description of Artificial Sequence: Synthetic oligonucleotide"

<400> 313
aagacggaca ggcacctacg gggt 24

<210> 314
<211> 16

```
<212> DNA
<213> Artificial Sequence

<220>
<221> source
<223> /note="Description of Artificial Sequence: Synthetic
      oligonucleotide"

<400> 314
aagacggaca cggggt                                                    16


<210> 315
<211> 20
<212> DNA
<213> Artificial Sequence

<220>
<221> source
<223> /note="Description of Artificial Sequence: Synthetic
      oligonucleotide"

<400> 315
aagacggaca ggcacggggt                                                20


<210> 316
<211> 25
<212> DNA
<213> Artificial Sequence

<220>
<221> source
<223> /note="Description of Artificial Sequence: Synthetic
      oligonucleotide"

<400> 316
aagacggaca ggcaccttac ggggt                                          25


<210> 317
<211> 28
<212> DNA
<213> Artificial Sequence

<220>
<221> source
<223> /note="Description of Artificial Sequence: Synthetic
      oligonucleotide"

<400> 317
aagacggaca ggcacctcct aacggggt                                       28
```